# EUROPEAN PATENT APPLICATION

(11) **EP 4 624 466 A1**
(43) Date of publication of application: **01.10.2025**
(21) Application number: 23895046.3
(22) Date of filing: 22.11.2023
(51) Int. Cl.: C07D 401/14, A61K 47/55, A61K 31/506, A61K 31/5377

(54) **DEGRADER FOR DECOMPOSING CMET PROTEIN, AND PHARMACEUTICAL COMPOSITION COMPRISING SAME**

(30) Priority: 22.11.2022 KR 20220157344
(71) Applicant: Innocure Therapeutics, Inc., Seongnam-si Gyeonggi-do 13488 (KR)
(72) Inventor: YOO, Hye Dong, Seongnam-si Gyeonggi-do 13488 (KR); SHIN, Young Jun, Seongnam-si Gyeonggi-do 13488 (KR); KIM, Bo Kyoung, Seongnam-si Gyeonggi-do 13488 (KR); YANG, Dong Sik, Seongnam-si Gyeonggi-do 13488 (KR); KOOK, Seung Hoon, Seongnam-si Gyeonggi-do 13488 (KR)
(74) Representative: Potter Clarkson
(86) International application number: PCT/KR2023/018991
(87) International publication number: WO 2024/112119

(57) **Abstract**

A TPD compound is disclosed.

The present invention relates to a novel PROTAC compound binding to a cMET protein and a pharmaceutical composition comprising the same. The present invention relates to a PROTAC compound that binds to a cMET protein, and since it can bind to a cMET protein and degrade it, it can be useful for the treatment or prevention of diseases related to cMET protein. The compound of the present invention has an excellent anticancer effect and can also induce the degradation of cMET proteins, which can show a therapeutic effect on cMET and related diseases.

## Description

### [Technical Field]

The present invention relates to a novel PROTAC compound binding to a cMET protein and a pharmaceutical composition comprising the same.

### [Background Technology]

A PROTAC is a heterodimer molecule in which a ligand of a target protein and a ligand that binds to an E3 ligase are linked via a linker. PROTAC binds to both proteins simultaneously, bringing the target protein very close to E3 ligase, which recognizes the target protein as a substrate and triggers polyubiquitination and subsequent proteasome degradation. Since this principle can effectively remove specific proteins from cells, PROTAC can be used as a chemical probe to study the function of target proteins, and furthermore, it has a high potential as a treatment for diseases. The term TPD (Targeted Protein Degradation) is sometimes used instead of the term PROTAC.

In the present invention, target protein-binding moieties binding to cMET proteins and E3 ligase-binding moieties were prepared, and a E3 ligase-binding moiety and a cMET protein-targeted binding moiety were connected through a linker to complete a PROTAC compound.

### [Detailed Description of the Invention]

### [Technical Challenges]

The technical challenge intended to be achieved by the present invention relates to a PROTAC and pharmaceutical compositions thereof, wherein a novel E3 ligase binding moiety and a cMET protein target binding moiety are connected via a linker.

### [Technical Solution]

In order to accomplish the technical task, the present invention provides a TPD compound or a pharmaceutically acceptable salt thereof, represented by the following Chemical Formula 1:

[Chemical Formula 1] cMET Targeted Protein-Binding Moiety (P) - {Linker (L)}p - E3 Ligase-Binding Moiety (E)

wherein,
E3 Ligase-Binding Moiety (E) is a compound represented by the following Chemical Formulas 2 to 6; and
cMET Targeted Protein-Binding Moiety (P) is a compound represented with any of the following Chemical Formulas 7 to 10; and
p is an integer of 0 or 1 (If p is 0, P and E are directly connected): 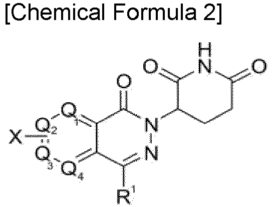 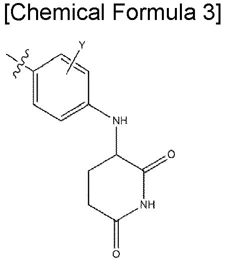 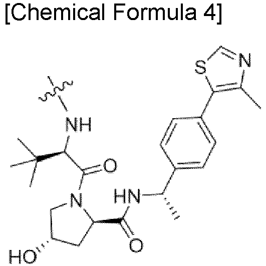 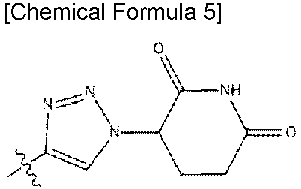 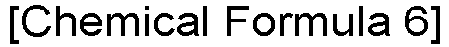 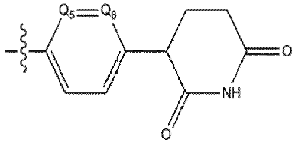 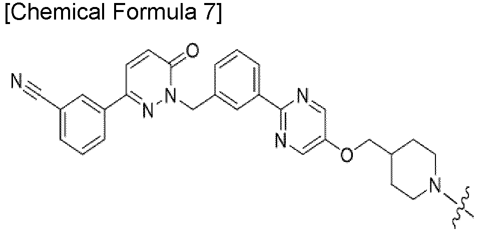 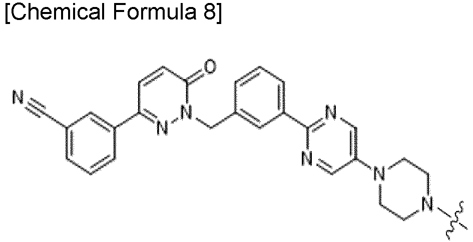 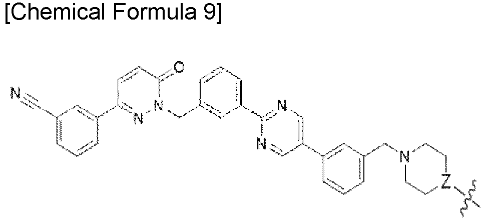 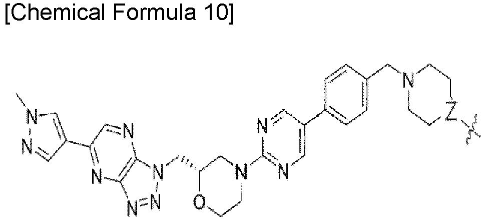 wherein,
   X is hydrogen, halogen, amino, nitro, hydroxy, C1 to C6 straight or branched alkyl, or a 4 to 8 atom heterocyclic containing oxygen or nitrogen;
   Q₁ to Q₄ are each independently C-F, C-Cl, C-H, C-X, or N, and at least any one of Q₁ to Q₄ is C-X;
   Y is hydrogen, halogen, or a C1 to C6 a straight, branched or cyclic alkyloxy unsubstituted or substituted with halogen;
   one of Q₅ and Q₆ is carbon atom, the other is nitrogen atom;
   Z is carbon atom or nitrogen atom; and
   R¹ is hydrogen, nitro, amino, carbonyl, C1 to C6 straight, branched or cyclic alkyl, or C1 to C6 straight, branched, or cyclic alkyl substituted with halogen.

In the present invention, a terminal of the linker (L) may be
(i) connected to a structure in Chemical Formula 1 by substituting X in a structure of Chemical Formula 2;
(ii) connected to a structure in Chemical Formula 1 by bonding to nitrogen or oxygen atom of X in a structure of Chemical Formula 2;
(iii) directly connected to the benzene ring of the structure of the Chemical Formula 3;
(iv) directly connected to an amino group located at the terminal of the structure of Chemical Formula 4;
(v) directly connected to the triazole ring of the structure of the Chemical Formula 5; or
(vi) directly connected to the pyridine ring of the structure of Chemical Formula 6, and the other terminal of the linker (L) may be connected to a compound linked to piperidine or piperazine located at the end of any of the structures of Chemical Formula 7 to 10.

In the present invention, a terminal of the linker (L) is 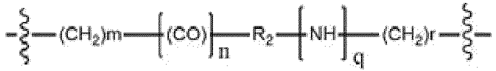 or 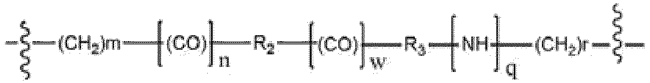 or 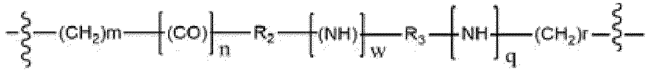 or 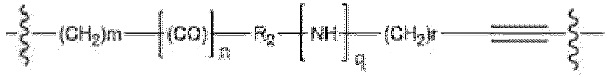 or 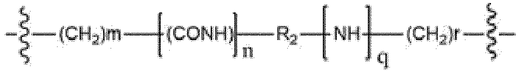 or 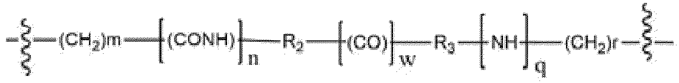 or 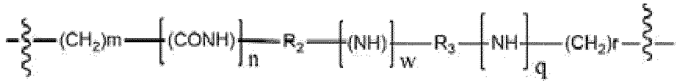 or 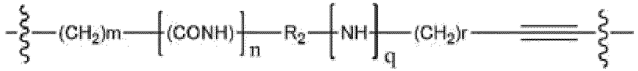 or
can have a structure selected from a group of the following formulas: 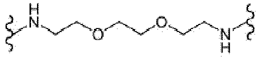 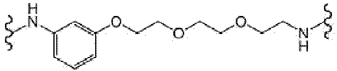 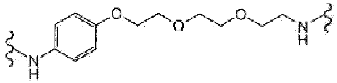 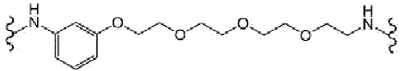 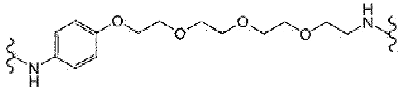 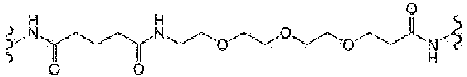
wherein, R₂ and R₃ are each independently -(CH₂)ₛ-, -(CH₂)ₜ-[O(C₂H₄)]ᵤ-, -O-(CH₂)-, - CH(OH)-piperazine, piperidine, azetidine, -(CH₂)ᵥ-piperidine, -(CH₂)ᵥ-piperazine, piperazine-(CH₂)ᵥ-piperidine, piperazine-(CH₂)ᵥ-morpholine, piperidine-(CH₂)ᵥ-morpholine, azetidine-(CH₂)ᵥ-piperazine, azetidine-(CH₂)ᵥ-piperidine, benzene-piperidine, benzene-piperazine, -(CO)-piperidine, -(CO)-piperazine, -(CO)-piperazine, benzene, triazole or pyrrolidine; and
m, n, q, r, s, t, u, v, and w are each independently an integer selected from 0 to 7.

The present invention also provides an anticancer composition comprising any compound from above.

### [Effect of the Invention]

The present invention relates to a PROTAC compound that binds to a cMET protein, and since it can bind to a cMET protein and degrade it, it can be useful for the treatment or prevention of diseases related to cMET protein. The compound of the present invention has an excellent anticancer effect and can also induce the degradation of cMET proteins, which can show a therapeutic effect on cMET related diseases.

### [Brief description of drawing]

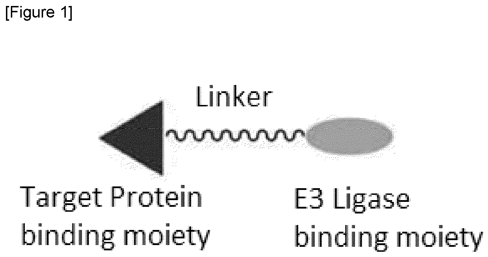

Figure 1 is a schematic of a PROTAC compound consisting of an E3 ligase-binding moiety, a linker, and a target protein-binding moiety.

### [Mode for Practicing the Invention]

The present invention is described in more detail below. However, the present invention can be implemented in various different forms, and the present invention is not limited by the embodiments described herein.

The terms used herein are used merely to describe a specific embodiment and are not intended to qualify the present invention. Expressions in the singular include plural expressions, unless the context clearly means otherwise. The 'inclusion' of a component in the specification of the present invention means that it may include more other components rather than excluding other components, unless there is a specifically contrary statement.

PROTAC according to the present invention may be a compound represented by the Chemical Formula 1:

[Chemical Formula 1] cMET Target Protein-Binding Moiety (P) - Linker (L)- E3 Ligase-Binding Moiety (E)

The basic structure of the E3 ligase binding moiety according to the present invention can be prepared by the following Reaction Equation 1 or Reaction Equation 2.

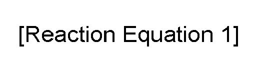

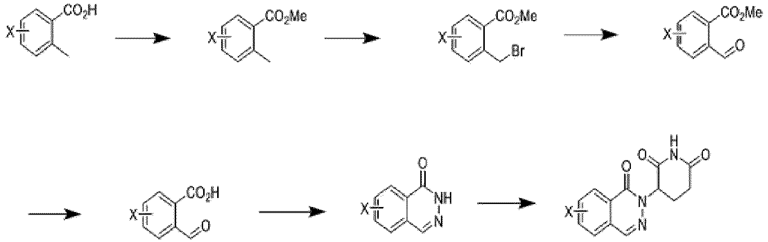

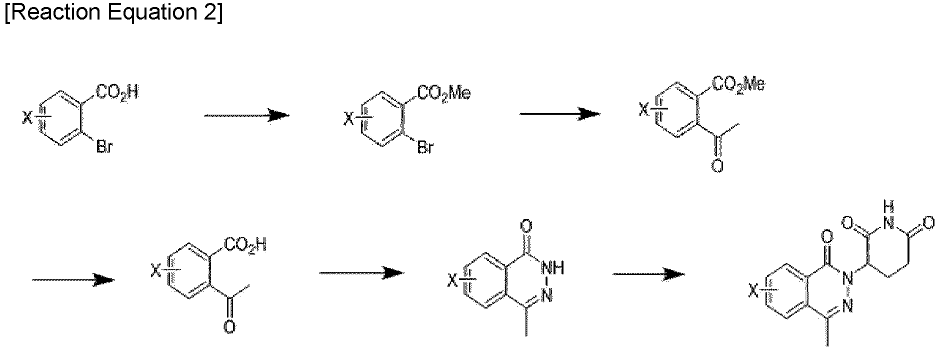

In the above Reaction Equations 1 or 2, X is hydrogen, halogen, amino, nitro, hydroxy, piperazine group or an C1 to C4 alkoxy. However, for the convenience of explanation, in the above equation, the substituent that can be bound to carbon in Q₁ to Q₄ of the compounds of Chemical Formula 1 is not shown, and only simple substituents such as hydrogen or methyl are shown among the R¹ substituents as an example.

In the following, a specific example of an E3 ligase-bound moiety compound is prepared using an embodiment.

### Example A: Preparation of an E3 ligase-coupled moiety compound

### Example A-1: Preparation of a compound

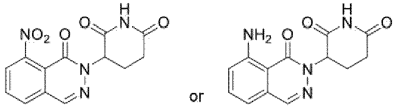

### (prepared according to Equation 1)

### 1-1) Preparation of methyl 2-methyl-6-nitrobenzoate

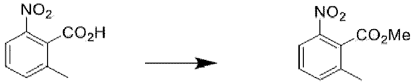

At room temperature, K2CO3 (19.1 g, 138 mmol) was added to a 2-methyl-6-nitrobenzoic acid (5 g, 27.6 mmol) solution of acetone (100 ml). After stirring for 30 minutes, methane iodide (19.6 g, 138 mmol) was added to the reactant and heated to 60oC for 6 hours. After cooling, the reactants were filtered and concentrated under reduced pressure. The product was used in the next reaction without additional purification. (5.45g, 98%) MS (ESI, m/z): [M+1]⁺ = [195.2].

### 1-2) Preparation of methyl 2-(bromomethyl)-6-nitrobenzoate

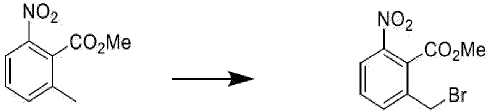

At room temperature, 1-bromomerolidine-2,5-dione (7.39 g, 41.4 mmol) and benzoyl benzene carboperoxoate (0.67 g, 7.26 mmol) were sequentially added to a solution of methyl 2-methyl-6-nitrobenzoate (5.39 g, 27.6 mmol) in ClCH₂CH₂Cl (100 ml). The reactants were heated and refluxed for 3 hours. The point when the red color disappears is the time when the reaction is complete. After cooling, the reactants were washed with water, dried with Magnesium sulfate, and concentrated under reduced pressure. The crude product was used in the next reaction without additional purification. (7.34g, 98%) MS (ESI, m/z): [M+1]⁺ = [274.4].

### 1-3) Preparation of methyl 2-formyl-6-nitrobenzoate

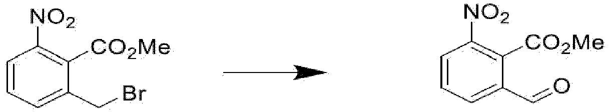

At room temperature, NMO (N-methylmorpholine N-oxide) (561 mg, 4.87 mmol) and 4Å molecular sieve were sequentially added to methyl 2-(bromomethyl)-6-nitrobenzoate (580 mg, 2.12 mmol) solution in DCM (30 ml). The reactants were stirred at room temperature for 2 hours. Molecular sieve was filtered and washed with DCM (10ml). The DCM layer was washed with water (50ml), dried with Magnesium sulfate, and concentrated under reduced pressure. The residue was purified by column chromatography, and the title compound was obtained as a white solid. (350mg, 79.07%) MS (ESI, m/z): [M+1]⁺ = [210.0].

### 1-4) Preparation of 2-formyl-6-nitrobenzoic acid

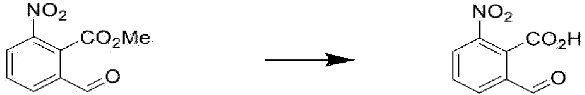

At room temperature, an aqueous solution of lithium hydroxide (1.15 g, 47.8 mmol) was added to a solution of methyl 2-formyl-6-nitrobenzoate (2 g, 9.56 mmol) in THF (10 ml). After 2 h of agitation, the reactants were concentrated under reduced pressure to dry the THF. After cooling to 0°C, the reactants were acidified with 1N HCl and the pH was adjusted to 4. The reactants were extracted twice with ethyl acetate (50ml). The combined ethyl acetate layer was dried with Magnesium sulfate, and concentrated under reduced pressure to obtain white crystals (1.50g, 80.3%). MS (ESI, m/z): [M+1]⁺ = [195.2].

### 1-5) Preparation of 8-Nitroptalazine-1(2H)-one

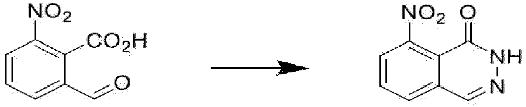

At room temperature, NH2NH2 monohydrate (417 mg, 8.33 mmol) was added to a 2-formyl-6-nitrobenzoic acid (1.2 g, 6.15 mmol) solution in methanol (10 ml). After stirring for 30 minutes, the reactants were heated to 80°C for 2 hours. The reactants were cooled to room temperature and concentrated under reduced pressure. The residue was poured into water (50ml) and extracted twice with ethyl acetate (50ml). The combined ethyl acetate layer was dried with Magnesium sulfate, concentrated under reduced pressure, and white crystals were obtained (1g, 85.07%). MS (ESI, m/z): [M+1]⁺ = [191.8].

### 1-6) Preparation of 3-(8-nitro-1-oxopthalazine-2(1H)-yl)piperidine-2,6-dione

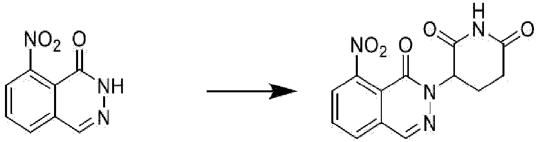

3-bromoperidin-2,6-dione (90 mg, 0.471 mmol) and K₂CO₃ (129 mg, 0.942 mmol) were sequentially added to 8-nitro-1,2-dihydrodroplasine-1-one (60 mg, 0.314 mmol) solution in DMF (1 ml). The reactants were heated to 85°C for 5 hours. After cooling, the reactants were poured into water (5 ml) and extracted twice with ethyl acetate (5 ml). The combined ethyl acetate layer was dried with Magnesium sulfate and concentrated under reduced pressure. The residue was purified by column chromatography, and the title compound was obtained as a white crystal (68 mg, 71.7%). MS (ESI, m/z): [M+1]⁺ = [302.6].

### 1-7) Preparation of 3-(8-nitro-1-oxopthalazine-2(1H)-yl)piperidine-2,6-dione

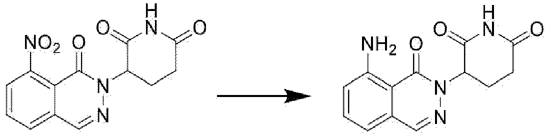

20 mg of 10% Pd/C (wet) (5 mg) of 20 mg was added to 3-(8-nitro-1-oxo-1,2-dihydrophoptalazine-2-yl-2-yl) solution of 3-(8-nitro-1-oxo-1,2-dihydrophoptalazine-2-yl) in methanol (5 ml) and stirred for 1 hour under hydrogen in the balloon. The solids were filtered, the filtrate was concentrated under reduced pressure, and the title compound was obtained at a yield of 99%. MS (ESI, m/z): [M+1]⁺ = [272.3].
[NMR] ¹H NMR (400 MHz, DMSO-d₆) δ 10.95 (s, 1H), 8.10 (s, 1H), 7.46 (t, J = 7.83 Hz, 1H), 7.22 (br s, 2H), 6.86 (d, J = 7.83 Hz, 1H), 6.81 (d, J = 7.34 Hz, 1H), 5.61 (br dd, J = 5.2, 11.92 Hz, 1H), 2.77 - 2.89 (m, 1H), 2.46 - 2.57 (m, 2H), 1.95 - 2.08 (m, 1H)

### Example A-2: Preparation of a compound

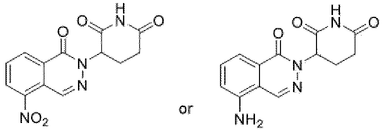

### 2-1) Preparation of methyl 2-methyl-3-nitrobenzoate

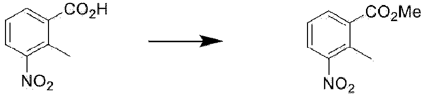

At room temperature, K₂CO₃ (19.1 g, 138 mmol) was added to a solution of 2-methyl-3-nitrobenzoic acid (5 g, 27.6 mmol) in acetone (100 ml). After stirring for 30 minutes, methane iodide (19.6 g, 138 mmol) was added to the reactant and heated to 60°C for 6 hours. After cooling, the reactants were filtered and concentrated under reduced pressure. The product was used in the next reaction without additional purification. (5.45g, 98%) MS (ESI, m/z): [M+1]⁺ = [195.3].

### 2-2) Preparation of methyl 2-(bromomethyl)-3-nitrobenzoate

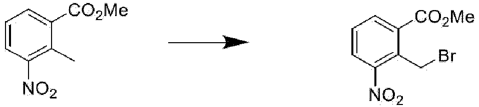

At room temperature, 1-bromophilidine-2,5-dione (7.39 g, 41.4 mmol) and benzoyl benzene carboperoxoate (0.67 g, 7.26 mmol) were sequentially added to a solution of methyl 2-methyl-3-nitrobenzoate (5.39 g, 27.6 mmol) in ClCH₂CH₂Cl (100 ml). The reactants were heated and refluxed for 3 hours. The point when the red color disappears is the time when the reaction is complete. After cooling, the reactants were washed with water, dried with Magnesium sulfate, and concentrated under reduced pressure. The crude product was used in the next reaction without additional purification. (7.34g, 98%) MS (ESI, m/z): [M+1]⁺ = [274.4].

### 2-3) Preparation of methyl 2-formyl-3-nitrobenzoate

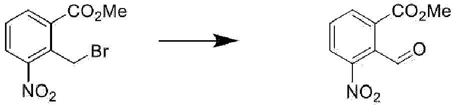

At room temperature, NMO (561mg, 4.87mmol) and 4Å molecular sieve were sequentially added to methyl 2-(bromomethyl)-3-nitrobenzoate (580mg, 2.12mmol) solution in DCM (30ml). The reactants were stirred at room temperature for 2 hours. Molecular sieve was filtered and washed with DCM (10ml). The DCM layer was washed with water (50ml), dried with Magnesium sulfate, and concentrated under reduced pressure. The residue was purified by column chromatography, and the title compound was obtained as a white solid. (350mg, 79.07%) MS (ESI, m/z): [M+1]⁺ = [210.0].

### 2-4) Preparation of 2-formyl-3-nitrobenzoic acid

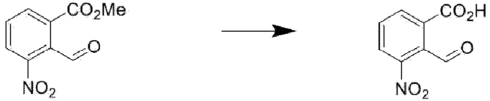

At room temperature, an aqueous solution of lithium hydroxide (1.15 g, 47.8 mmol) was added to a solution of methyl 2-formyl-3-nitrobenzoate (2 g, 9.56 mmol) in THF (10 ml). After 2 hours of agitation, the reactants were concentrated under reduce pressure to remove THF. After cooling to 0°C, the residue was acidified with 1N HCl and the pH was adjusted to 4. The reactants were extracted twice with ethyl acetate (50ml). The combined ethyl acetate layer was dried with Magnesium sulfate, and concentrated under reduced pressure to obtain white crystals (1.50g, 80.3%). MS (ESI, m/z): [M+1]⁺ = [195.2].

### 2-5) Preparation of 5-Nitroptalazine-1(2H)-one

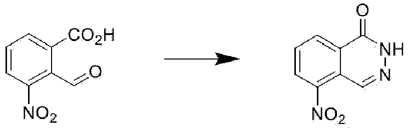

At room temperature, NH₂NH₂ monohydrate (417 mg, 8.33 mmol) was added to a 2-formyl-3-nitrobenzoic acid (1.2 g, 6.15 mmol) solution in methanol (10 ml). After stirring for 30 minutes, the reactants were heated to 80°C for 2 hours. The reactants were cooled to room temperature and concentrated under reduced pressure. The residue was poured into water (50ml) and extracted twice with ethyl acetate (50ml). The combined ethyl acetate layer was dried with Magnesium sulfate, concentrated under reduced pressure, and white crystals were obtained (1g, 85.07%). MS (ESI, m/z): [M+1]⁺ = [191.8].

### 2-6) Preparation of 3-(5-nitro-1-oxopthalazine-2(1H)-yl)piperidine-2,6-dione

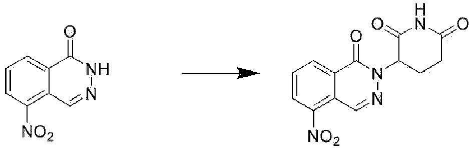

3-bromoperidin-2,6-dione (90 mg, 0.471 mmol) and K₂CO₃ (129 mg, 0.942 mmol) were sequentially added to 5-nitropthalazine-1(2H)-one (60 mg, 0.314 mmol) solution in DMF (1 ml). The reactants were heated to 85°C for 5 hours. After cooling, the reactants were poured into water (5 ml) and extracted twice with ethyl acetate (5 ml). The combined ethyl acetate layer was dried with Magnesium sulfate and concentrated under reduced pressure. The residue was purified by column chromatography, and the title compound was obtained as a white crystal (68 mg, 71.7%). MS (ESI, m/z): [M+1]⁺ = [302.4].

### 2-7) Preparation of 3-(5-amino-1-oxopthalazine-2(1H)-yl)piperidine-2,6-dione

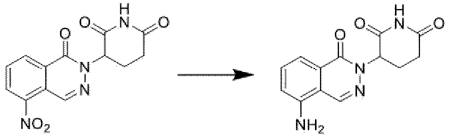

20 mg of 10% Pd/C (wet) (5 mg) of 20 mg was added to 3-(5-nitro-1-oxopthalazine-2(1H)-yl)piperidine-2,6-dione (25 mg, 0.82 mmol) solution in methanol (5 ml) and stirred for 1 hour under hydrogen in the balloon. The solids were filtered, the filtrate was concentrated under reduced pressure, and the title compound was obtained at a yield of 99%. MS (ESI, m/z): [M+1]⁺ = [272.3].
[NMR] ¹H NMR (400 MHz, DMSO-d₆) δ 10.95 (s, 1H), 8.20 -8.41 (m, 3H), 8.08 (s, 1H), 7.83 (d, J = 8.93 Hz, 1H), 6.95 (d, J = 10.5 Hz, 1H), 5.64 (br dd, J = 4.83, 11.31 Hz, 1H), 2.62 - 2.89 (m, 1H), 2.52 - 2.60 (m, 2H), 1.86 - 2.07 (m, 1H)

### Example A-3: Preparation of a compound

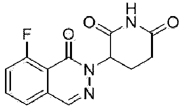

### 3-1) Preparation of methyl 2-fluoro-6-methylbenzoate

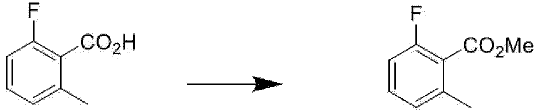

At room temperature, K₂CO₃ (44.8 g, 324 mmol) was added to a solution of 2-fluoro-6-methylbenzoic acid (10 g, 64.9 mmol) in acetone (200 ml). After stirring for 30 minutes, methane iodide (46 g, 324 mmol) was added to the reactant and heated to 60°C for 6 hours. After cooling, the reactants were filtered and concentrated under reduced pressure. The product was used in the next reaction without additional purification. (11.2g, yield 103%) MS (ESI, m/z): [M+1]⁺ = [168.3].

### 3-2) Preparation of methyl 2-(bromomethyl)-6-fluorobenzoate

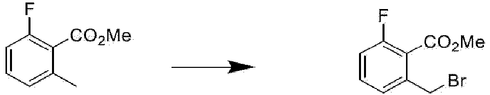

At room temperature, 1-bromomerrolidine-2,5-dione (24.7 g, 139 mmol) and benzoyl benzene carboperoxoate (1.53 g, 6.30 mmol) were sequentially added to a solution of methyl 2-fluoro-6-methylbenzoate (21.2 g, 126 mmol) in ClCH₂CH₂Cl (250 ml). The reactants were heated and refluxed for 16 hours. The point when the red color disappears is the time when the reaction is complete. After cooling, the reactants were washed with water, dried with Magnesium sulfate, and concentrated under reduced pressure. The crude products were used in the following reactions without additional purification. (35g, yield 112%) MS (ESI, m/z): [M+1]⁺ = [245.9].

### 3-3) Preparation of methyl 2-fluoro-6-formylbenzoate

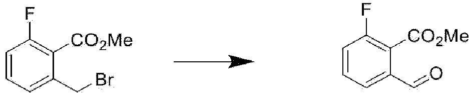

At room temperature, NMO (24.9 g, 212 mmol) was added to methyl 2-(bromomethyl)-6-fluorobenzoate (35 g, 142 mmol) solution in DCM (280 ml). The reactants were stirred at room temperature for 4 hours. The DCM layer was washed with water (200ml), dried with Magnesium sulfate, and concentrated under reduced pressure. The residue was purified by column chromatography, and the title compound was obtained as a white solid. (11.52g, yield 45%) MS (ESI, m/z): [M+1]⁺ = [183.1].

### 3-4) Preparation of 2-fluoro-6-formylbenzoic acid

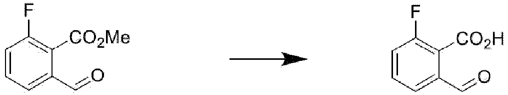

At room temperature, an aqueous solution (41 ml) of lithium hydroxide (7.57 g, 180 mmol) was added to a solution of methyl 2-fluoro-6-formylbenzoate (11.5 g, 63.2 mmol) in THF (82 ml). After 4 h of agitation, the reactants were concentrated under reduced pressure and the THF was dried. After cooling to 0°C, the reactants were acidified with 1N HCl and the pH was adjusted to 4. The reactants were extracted twice with ethyl acetate (100 ml). The combined ethyl acetate layer was dried with Magnesium sulfate, concentrated under reduced pressure, and white crystals were obtained (10.4g, 97.8%). MS (ESI, m/z): [M+1]⁺ = [168.8].

### 3-5) Preparation of 8-Fluoroptalazine-1(2H)-one

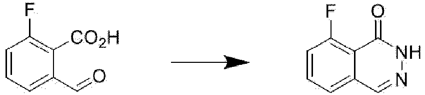

At room temperature, NH₂NH₂ monohydrate (3.72 mg, 61.9 mmol) was added to a solution of 2-fluoro-6-formylbenzoic acid (10.4 g, 61.9 mmol) in methanol (120 ml) and stirred at room temperature for 16 hours. The white sediment was filtered and washed with methanol. The obtained white crystals were triturated with 100ml of ethyl acetate (EA) and filtered to obtain white crystals (3.05g, 30%). MS (ESI, m/z): [M+1]⁺ = [164.8].

### 3-6) Preparation of 3-(8-fluoro-1-oxopthalazine-2(1H)-yl)piperidine-2,6-dione

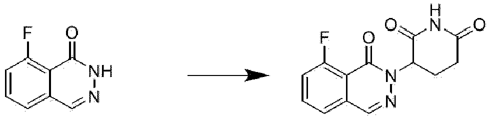

NaH (60%, 53.6mg, 1.34mmol) was added to 8-fluoroptalazine-1(2H)-one (200mg, 1.22mmol) solution in DMF (5ml) at 0°C and stirred for 30 minutes. 3-bromoperidin-2,6-dione (468 mg, 2.44 mmol) was added to the solution and stirred for 6 hours. The reaction was terminated with water and extracted twice with ethyl acetate (20ml). The mixed ethyl acetate was dried with Magnesium sulfate and concentrated under reduced pressure. The residues were purified by column chromatography, and the title compound was obtained as white crystals (70 mg, 20.8%). MS (ESI, m/z): [M+1]⁺ = [276.1].
[NMR] ¹H NMR (400 MHz, DMSO-d₆) δ 11.06 (s, 1H), 8.48 (s, 1H), 7.99 (ddd, J = 4.6, 7.2, 8.2 Hz, 1H), 7.80 (d, J = 7.2 Hz, 1H), 7.67 (dd, J = 8.2, 11.4 Hz, 1H), 5.77 (dd, J=5.3, 12.0 Hz, 1H), 2.99-2.77 (m, 1H), 2.68-2.51 (m, 2H), 2.23-2.02 (m, 1H)

### Example A-4: Preparation of a compound

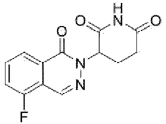

### 4-1) Preparation of methyl 5-fluoro-6-methylbenzoate

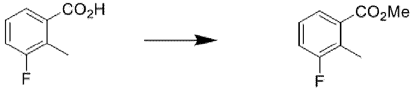

At room temperature, sulfuric acid (2 ml) was added to a solution of 3-fluoro-2-methylbenzoic acid (10 g, 64.9 mmol) in methanol (60 ml). Heated to 80°C and stirred overnight. After cooling to room temperature, the reactants were evaporated and extracted with NaHCO₃ and ethyl acetate (EA). Dried with Magnesium sulfate. The crude product was used in the next reaction without additional purification. (10.1g, yield 92%) MS (ESI, m/z): [M+1]⁺ = [168.3].

### 4-2) Preparation of methyl 2-(bromomethyl)-3-fluorobenzoate

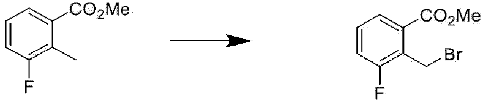

At room temperature, 1-bromomerolidin-2,5-dione (15.9 g, 89.2 mmol) and benzoyl benzene carboperoxoate (0.72 g, 2.97 mmol) were sequentially added to a solution of methyl 3-fluoro-2-methylbenzoate (10 g, 59.5 mmol) in ClCH₂CH₂Cl (250 ml). The reactants were heated and refluxed for 16 hours. The point when the red color disappears is the time when the reaction is complete. After cooling, the reactants were washed with water, dried with Magnesium sulfate, and concentrated under reduced pressure. The crude product was used in the next reaction without additional purification. (10.5g, yield 71%) MS (ESI, m/z): [M+1]⁺ = [245.9].

### 4-3) Preparation of methyl 3-fluoro-2-formylbenzoate

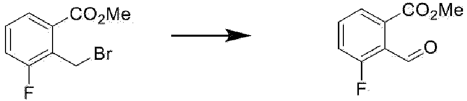

At room temperature, NMO (10.4 g, 89 mmol), 4Å molecular sieve was added to methyl 2-(bromomethyl)-3-fluorobenzoate (10 g, 40.5 mmol) solution in DCM (200 ml). The reactants were stirred at room temperature for 4 hours. The DCM layer was washed with water (200ml), dried with Magnesium sulfate, and concentrated under reduced pressure. The residue was purified by column chromatography to obtain the heading compound. (5.5g, 75%) MS (ESI, m/z): [M+1]⁺ = [183.1].

### 4-4) Preparation of 3-fluoro-2-formylbenzoic acid

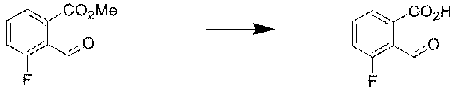

At room temperature, an aqueous solution (41 ml) of lithium hydroxide monohydrate (2.88 g, 68.6 mmol) was added to a solution of methyl 3-fluoro-2-formylbenzoate (2.5 g, 13.7 mmol) in THF (68 ml). After 4 hours of agitation, the reactants were concentrated under reduced pressure and THF was removed. After cooling to 0°C, the reactants were acidified with 1N HCl and the pH was adjusted to 4. The reactants were extracted twice with ethyl acetate (100 ml). The combined ethyl acetate layer was dried with Magnesium sulfate, concentrated under reduced pressure to obtain white crystals (2.5g, 108%). MS (ESI, m/z): [M+1]⁺ = [168.8].

### 4-5) Preparation of 5-Fluoro-1,2-dihydrophthalazine-1-one

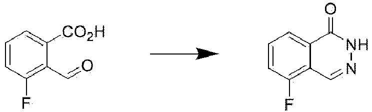

At room temperature, NH₂NH₂ monohydrate (1.22 mg, 16.4 mmol) was added to a 3-fluoro-2-formylbenzoic acid (2.5 g, 14.9 mmol) solution in THF:water=1:1 (70 ml) and stirred for 16 hours. The mixture was acidified to pH 4 and the solid was filtered and washed with hexane. Vacuum drying yielded the title compound (1.3 g, 53%). MS (ESI, m/z): [M+1]⁺ = [165.3]

### 4-6) Preparation of 3-(5-fluoro-1-oxo-1,2-dihydroptalazine-2-yl)piperidine-2,6-dione

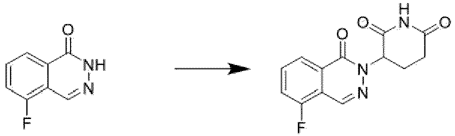

LDA (lithium diisopropylamide, 2.19 mmol) was added to 5-fluoropleptalazine-1-one (300 mg, 1.83 mmol) solution in DMF (10 ml) at 0°C and stirred for 30 minutes. 3-bromoperidin-2,6-dione (526 mg, 2.74 mmol) was added to the solution and stirred at 80°C for 6 hours. Once the reaction was completed, the reactants were cooled to room temperature, poured into water (100ml), pH was adjusted to 3~4 with 6N HCl, then extracted with ethyl acetate, dried with Magnesium sulfate, and concentrated under reduced pressure. The product was recrystallized into hexane, vacuum-dried, and the title compound was obtained. MS (ESI, m/z): [M+1]⁺ = [276.1].
[NMR] ¹H NMR (400 MHz, DMSO-d₆) δ 11.03 (s, 1H), 8.53 (s, 1H), 8.05 (d, J=7.82Hz, 1H), 7.76-7.90 (m, 2H), 5.78 (dd, J=12.23, 5.26Hz, 1H), 2.80-2.93 (m, 1H), 2.47-2.61 (m, 2H), 2.01-2.16 (m, 1H)

### Example A-5: Preparation of a compound

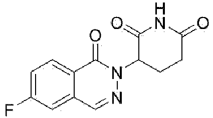

### 5-1) Preparation of methyl 4-fluoro-6-methylbenzoate

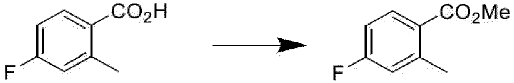

In accordance with the preparation method of Example 4-1, methyl 4-fluoro-2-methylbenzoate was prepared from 4-fluoro-2-methylbenzoate.

### 5-2) Preparation of methyl 2-(bromomethyl)-4-fluorobenzoate

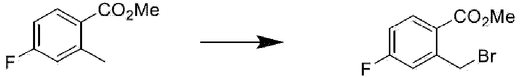

At room temperature, 1-bromophilolidin-2,5-dione (31.8 g, 178 mmol) and benzoyl benzene carboperoxoate (1.92 g, 5.95 mmol) were sequentially added to a solution of methyl 4-fluoro-2-methylbenzoate (20 g, 119 mmol) in ClCH₂CH₂Cl (100 ml). The reactants were heated and refluxed for 3 hours. The point when the red color disappears is the time when the reaction is complete. After cooling, the reactants were washed with water, dried with Magnesium sulfate, and concentrated under reduced pressure. The product was purified with MPLC (HX/EA EA 0 -> 5%) (22 g, yield 75%). MS (ESI, m/z): [M+1]⁺ = [248.4].

### 5-3) Preparation of methyl 3-fluoro-2-formylbenzoate

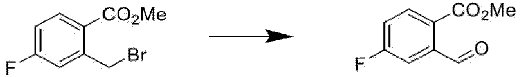

At room temperature, NMO (15.6 mg, 134 mmol) and 4Å molecular sieve were sequentially added to methyl 2-(bromomethyl)-4-fluorobenzoate (22 g, 89 mmol) solution in DCM (100 ml). The reactants were stirred at room temperature for 2 hours. The molecular sieve was filtered and washed with DCM (50ml). The DCM layer was washed with water (200ml), dried with Magnesium sulfate, and concentrated under reduced pressure. The residue was purified by column chromatography, and the title compound was obtained as a white solid. (12g, 73.98%) MS (ESI, m/z): [M+1]⁺ = [183.2].

### 5-4) Preparation of 4-fluoro-2-formylbenzoic acid

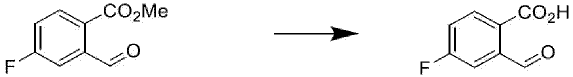

At room temperature, an aqueous solution (50 ml) of lithium hydroxide (13.8 g, 329 mmol) was added to a solution of methyl 4-fluoro-6-formylbenzoate (12 g, 65.9 mmol) in THF (50 ml). After 2 h of agitation, the reactants were concentrated under reduced pressure to dry the THF. After cooling to 0°C, the reactants were acidified with 1N HCl and the pH was adjusted to 4. The reactants were extracted twice with ethyl acetate (50ml). The combined ethyl acetate layer was dried with Magnesium sulfate, concentrated under reduced pressure, and white crystals were obtained (10g, 90.3%). MS (ESI, m/z): [M+1]⁺ = [169.2].

### 5-5) Preparation of 6-fluoro-1,2-dihydrophthalazine-1-one

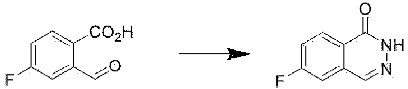

At room temperature, NH₂NH₂ monohydrate (2.02 g, 40.3 mmol) was added to a 4-fluoro-2-formylbenzoic acid (6.78 g, 40.3 mmol) solution in methanol (50 ml). After stirring for 30 minutes, the reactants were heated to 80°C for 2 hours. The reactants were cooled to room temperature and concentrated under reduced pressure. The residue was poured into water (150ml) and extracted twice with ethyl acetate (150ml). The combined ethyl acetate layer was dried with Magnesium sulfate, concentrated under reduced pressure to obtain white crystals (5.5g, 83.07%). MS (ESI, m/z): [M+1]⁺ = [165.3].

### 5-6) Preparation of 3-(6-fluoro-1-oxopthalazine-2(1H)-yl)piperidine-2,6-dione

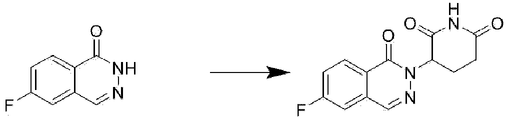

Sodium 2-methylpropane-2-oleate (175 mg, 0.914 mmol) was added to a 6-fluorophthalazine-1-one (100 mg, 0.609 mmol) solution in DMF (2 ml) at 0°C. After stirring for 30 minutes, 3-bromoperidin-2,6-dione (90 mg, 0.471 mmol) was added to the reaction mixture and stirred at room temperature for 6 hours. Water (20ml) was poured into the reaction mixture, and ethyl acetate (20ml) was extracted twice. The mixed ethyl acetate was dried with Magnesium sulfate and concentrated under reduced pressure. The residues were purified by chromatography, and the title compound was obtained as white crystals (110 mg, 65.5%). MS (ESI, m/z): [M+1]⁺ = [276.6].
[NMR] ¹H NMR (400 MHz, DMSO-d₆) δ 11.11 (s, 1H), 8.50 (s, 1H), 8.38 (dd, J=8.86, 5.44 Hz, 1H), 7.72 - 7.89 (m, 2H), 5.70 - 5.90 (m, 11H), 2.56 - 2.70 (m, 2H), 2.11 - 2.25 (m, 1H)

### Example A-6: Preparation of a compound

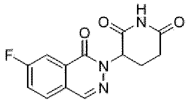

### 6-1) Preparation of methyl 4-fluoro-6-methylbenzoate

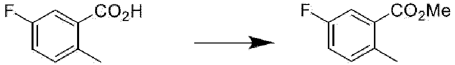

In accordance with the preparation method of Example 4-1, methyl 5-fluoro-2-methylbenzoate was prepared from 5-fluoro-2-methylbenzoate.

### 6-2) Preparation of methyl 2-(bromomethyl)-5-fluorobenzoate

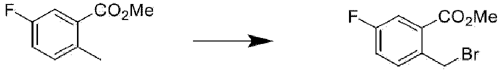

At room temperature, 1-bromophilidine-2,5-dione (31.8 g, 178 mmol) and benzoyl benzene carboperoxoate (1.92 g, 5.95 mmol) were sequentially added to a solution of methyl 5-fluoro-2-methylbenzoate (20 g, 119 mmol) in ClCH₂CH₂Cl (100 ml). The reactants were heated and refluxed for 3 hours. The point when the red color disappears is the time when the reaction is complete. After cooling, the reactants were washed with water, dried with Magnesium sulfate, and concentrated under reduced pressure. The product was purified with MPLC (HX/EA EA 0 -> 5%) (29 g, yield 98.8%). MS (ESI, m/z): [M+1]⁺ = [248.4].

### 6-3) Preparation of methyl 5-fluoro-2-formylbenzoate

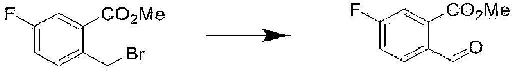

At room temperature, NMO (20.6mg, 176mmol) and 4Å molecular sieve were sequentially added to methyl 2-(bromomethyl)-5-fluorobenzoate (29g, 117mmol) solution in DCM (100ml). The reactants were stirred at room temperature for 2 hours. The molecular sieve was filtered and washed with DCM (50ml). The DCM layer was washed with water (200ml), dried with Magnesium sulfate, and concentrated under reduced pressure. The residue was purified by column chromatography, and the title compound was obtained as a white solid. (15g, yield 70.16%) MS (ESI, m/z): [M+1]⁺ = [183.2].

### 6-4) Preparation of 5-fluoro-2-formylbenzoic acid

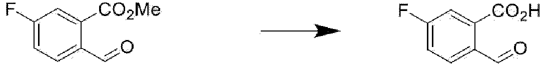

At room temperature, an aqueous solution (50 ml) of lithium hydroxide (17.3 g, 412 mmol) was added to a solution of methyl 5-fluoro-2-formylbenzoate (15 g, 82.3 mmol) in THF (50 ml). After 2 hour of agitation, the reactants were concentrated under reduced pressure to dry the THF. After cooling to 0°C, the reactants were acidified with 1N HCl and the pH was adjusted to 4. The reactants were extracted twice with ethyl acetate (50ml). The combined ethyl acetate layer was dried with Magnesium sulfate, concentrated under reduced pressure to obtain white crystals (12g, 86.67%). MS (ESI, m/z): [M+1]⁺ = [169.2].

### 6-5) Preparation of 7-Fluoro-1,2-dihydroptalazine-1-one

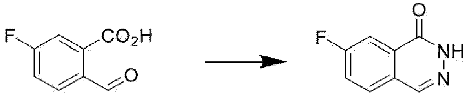

At room temperature, NH₂NH₂ monohydrate (3.74 g, 74.8 mmol) was added to a 5-fluoro-2-formylbenzoic acid (8.16 g, 48.5 mmol) solution in methanol (50 ml). After stirring for 30 minutes, the reactants were heated to 80°C for 2 hours. The reactants were cooled to room temperature and concentrated under reduced pressure. The residue was poured into water (150ml) and extracted twice with ethyl acetate (150ml). The combined ethyl acetate layer was dried with Magnesium sulfate, concentrated under reduced pressure to obtain white crystals (6.5g, 81.59%). MS (ESI, m/z): [M+1]⁺ = [165.3].

### 6-6) Preparation of 3-(7-fluoro-1-oxopthalazine-2(1H)-yl)piperidine-2,6-dione

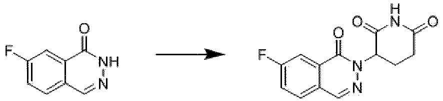

Sodium 2-methylpropane-2-oleate (586 mg, 6.09 mmol) was added to a 7-fluorine-1,2-dihydrophoptalazine-1-one (500 mg, 3.05 mmol) solution in DMF (5 ml) at 0°C. After stirring for 30 minutes, 3-bromoperidin-2,6-dione (1.05 mg, 5.48 mmol) was added to the reaction mixture and stirred at room temperature for 6 hours. The reaction mixture was poured into water (50ml) and extracted twice with ethyl acetate (50ml). The mixed ethyl acetate was dried with Magnesium sulfate and concentrated under reduced pressure. The residue was purified by column chromatography, and the title compound was obtained as a white crystal (300 mg, 35.78%). MS (ESI, m/z): [M+1]⁺ = [276.6].
[NMR] ¹H NMR (400 MHz, DMSO-d₆) δ 11.09 (s, 1H), 8.53 (s, 1H), 8.13 (dd, J=8.74, 5.20 Hz, 1H), 7.87 - 8.00 (m, 2H), 5.76 - 5.88 (m, 11H), 2.54 - 2.68 (m, 2H), 2.10 - 2.20 (m , 1H)

**Example A-7: Preparation of a compound** 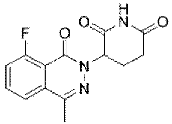 **(prepared according to Equation 2)**

### 7-1) Preparation of methyl 2-acetyl-6-fluorobenzoate

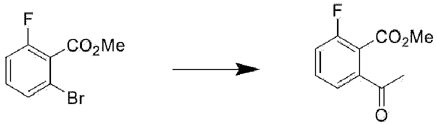

Tetrakis (triphenylphosphine)-palladium (0) (557 mg, 0.48 mmol) was added to a solution of methyl 2-acetyl-6-fluorobenzoate (1.12 g, 4.81 mmol) and tributyl (1-ethoxyvinyl) tin (1.91 g, 5.29 mmol) in toluene (20 ml) and stirred at 100°C for 16 hours. After cooling, 5 ml of 1N-HCl was added and stirred for 1 hour. The organic layer was dried with Magnesium sulfate and concentrated under reduced pressure. The residue was purified by silica column chromatography, and the heading compound was obtained as yellow oil. (820mg, yield 87%) MS (ESI, m/z): [M+1]⁺ = [196.8].

### 7-2) Preparation of 2-acetyl-6-fluorobenzoic acid

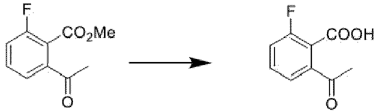

LiOH (494 mg, 20.6 mmol) was added to a solution of methyl 2-acetyl-6-fluorobenzoate (810 mg, 4.13 mmol) in THF (20 ml) and water (10 ml) and stirred for 20 hours at room temperature. The solution was acidified with 1N-HCl so that the pH was about 3. 100ml EA was applied, the organic layer was dried with Magnesium sulfate, and the title compound was obtained by reduced pressure concentration. (810mg yield 108%) MS (ESI, m/z): [M+1]⁺ = [183.1].

### 7-3) Preparation of 8-fluoro-4-methylphthalazine-1(2H)-one

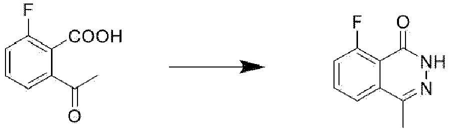

Hydrazine monohydrate (261 mg, 5.20 mmol) was added to a 2-acetyl-6-fluorobenzoic acid (790 mg, 4.34 mmol) solution in methanol (23 ml) and stirred at room temperature for 16 hours. The sediment was filtered, washed with ACN (acetonitrile), and the title compound was obtained as a white solid. (612mg, yield 79.2%) MS (ESI, m/z): [M+1]⁺ = [179.1].

### 7-4) Preparation of 3-(8-fluoro-4-methyl-1-oxopthalazine-2(1H)-yl)piperidine-2,6-dione

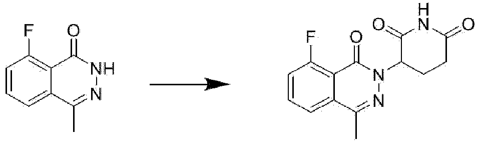

1M-Lithium diisopropylamide (3.6 ml, 3.6 mmol) was added to 8-fluoro-4-methylphthalazine-1(2H)-one (534 mg, 3 mmol) solution in THF (30 ml) at 0°C and stirred for 20 minutes. 3-brobopiperidine-2,6-dione (863 mg, 4.5 mmol) was added to the solution and stirred at 80°C for 2 hours. The reactants were under reduced pressure-concentrated and dried. Water (10 ml) was added and stirred for 1 hour, and acidified with 1N-HCl to adjust the pH to 4. The precipitate was filtered, washed with water, and the title compound was obtained as a white solid (760 mg, yield: 86.5%). MS (ESI, m/z): [M+1]⁺ = [289.8].

[NMR] ¹H NMR (400 MHz, DMSO-d₆) δ 11.05 (s, 1H), 8.04-7.94 (m, 1H), 7.79 (d, J = 7.9 Hz, 1H), 7.69 (dd, J = 7.9, 11.3 Hz, 1H), 5.71 (be dd, J = 4.9, 12.1 Hz, 1H), 3.0 - 2.83 (m, 1H), 2.64 - 2.56 (m, 2H), 2.55 (s, 3H), 2.17 - 2.05 (m, 1H).

### Example A-8: Preparation of a compound

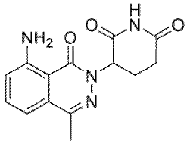

### 8-1) Preparation of 3-(8-((2,4-dimethoxybenzyl)amino)-4-methyl-1-oxopthalazine-2(1H)-yl)piperidine-2,6-dione

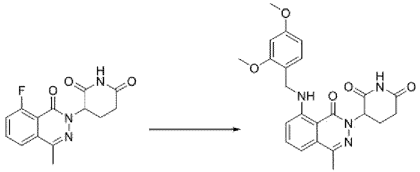

3-(8-fluoro-4-methyl-1-oxotalamine-2(1H)-yl) piperidine-2,6-dione (0.104 mmol), 2,4-dimethoxybenzylamine (0.207 mmol) and DIPEA (N,N-diisopropylethylamine) (0.312 mmol) solutions in NMP (N-methyl-pyrrolidone) (1 mL) were stimulated with microwaves for 2 hours at 120°C. The reaction mixture was purified by reversed-phase chromatography (C18, water (0.1 % FA) / ACN (0.1% FA), gradient), and 33 mg of white solid was obtained (33 mg, yield 72%). MS (ESI, m/z): [M+1]⁺ = [437.0]

### 8-2) Preparation of 3-(8-amino-4-methyl-1-oxopthalazine-2(1H)-yl)piperidine-2,6-dione

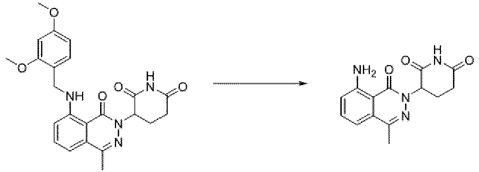

0.3ml of TFA (trifluoroacetic acid) was added to 3-(8-((2,4-dimethoxybenzyl)amino)4-methyl-1-oxopthalazine-2(1H)-yl)piperidine-2,6-dione (14mg, 0.032mmol) in toluene (0.7mL) and stirred at 80°C for 1 hour. The reaction mixture was purified by reversed-phase column chromatography (C18, water (0.1% FA) / ACN (0.1% FA), gradient) yielded 7.5 mg of white solid (7.5 mg, yield 82%). MS (ESI, m/z): [M+1]⁺ = [287.0]
[NMR] 1H NMR (400 MHz, DMSO-d6) δ10.98 (s, 1H), 7.54 (t, J = 8.0 Hz, 1H), 7.37 (brs, 2H), 6.95 (d, J = 8.2 Hz, 1H), 6.88 (d, J = 7.6 Hz, 1H), 5.62 (br dd, J = 5.3, 12.0 Hz, 1H), 3.0 - 2.82 (m, 1H), 2.66 - 2.52 (m, 2H), 2.39 (s, 3H), 2.11 - 2.02 (m, 1H)

### Example A-9: Preparation of a compound

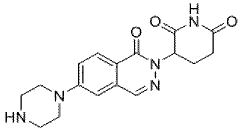

### 9-1) Preparation of tert-butyl 4-(2-(2,6-dioxopiperidine-3-yl)-1-oxo-1,2-dihydroptalazine-6-yl)piperazine-1-carboxylate

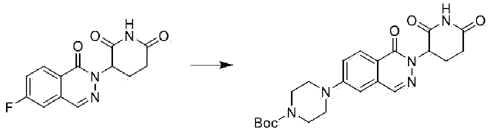

6-Fluoro-1,2-dihydroptalazine-1-one (100 mg, 0.36 mmol) and tert-butylpiperazine-1-carboxylate (81.2 mg, 0.36 mmol) were dissolved in NMP (1 mL), DIPEA (5 equivalents) was added to the reaction mixture, and stirred overnight at 120°C. The reaction of the reaction mixture was terminated with water, extracted with EA, and washed with NH₄Cl saturated aqueous solution and Brine solution. The organic layer was dried with Magnesium sulfate. The reaction mixture was loaded into silica, separated by MPLC (HX/EA 30% -> 50% for 10 min), and the oil product was obtained (110 mg, yield 66%).

### 9-2) Preparation of 3-(1-oxo-6-(piperazine-1-yl)phthalazine-2(1H)-yl)piperidine-2,6-dione

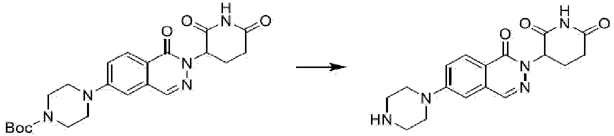

Tert-butyl 4-[2-(2,6-dioxopiperidine-3-yl)-1-oxo-1,2-dihydroptalazine-6-yl]piperazine-1-carboxylate was dissolved in a 20% TFA solution in DCM (1 ml) and reacted for 2 hours at room temperature. The reaction was terminated with an aqueous solution saturated with NaHCO3 and extracted with EA. The organic layer was dried with Magnesium sulfate and evaporated. The reaction mixture was purified with MPLC (MC/ME Me 0 -> 10%). White solid product (40 mg, yield 86%). MS (ESI, m/f): [M+1]⁺ = [342.2].
[NMR] 1H NMR (400 MHz, DMSO-d6) δ 8.24 (s, 1 H) 8.02 (d, J=9.05 Hz, 1 H) 7.47 (dd, J=8.93, 2.57 Hz, 1 H) 7.31 (s, 0.5 H) 7.23 (d, J=2.45 Hz, 1 H) 7.13 (s, 0.5 H) 5.34 (dd, J=8.93, 4.52 Hz, 1 H) 3.28 - 3.44 (m, 6 H) 2.85 - 2.95 (m, 4 H) 2.30 - 2.40 (m, 1 H) 2.16 - 2.30 (m, 2 H)

### Example A-10: Preparation of a compound

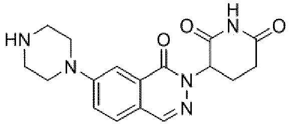

### 10-1) Preparation of tert-butyl 4-(3-(2,6-dioxopiperidine-3-yl)-4-oxo-3,4-dihydroptalazine-6-yl)piperazine-1-carboxylate

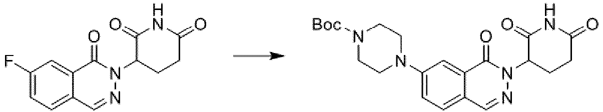

3-(7-fluoro-1-oxo-1,2-dihydroptalazine-2-yl)piperidine-2,6-dione (100 mg, 0.36 mmol) and tert-butylpiperazine-1-carboxylate (81.2 mg, 0.44 mmol) were dissolved in NMP (1 mL), DIPEA (5 equivalents) was added to the reaction mixture, and stirred overnight at 120°C. The reaction of the reaction mixture was terminated with water, extracted with EA, and washed with NH4Cl saturated aqueous solution and Brine solution. The organic layer was dried with Magnesium sulfate, the reaction mixture was loaded into silica, separated by MPLC (HX/EA 30% -> 50% for 10 min), and the product was obtained as oil (yield: 110 mg, 66%).

### 10-2) Preparation of 3-(1-oxo-7-(piperazine-1-yl)phthalazine-2(1H)-yl)piperidine-2,6-dione

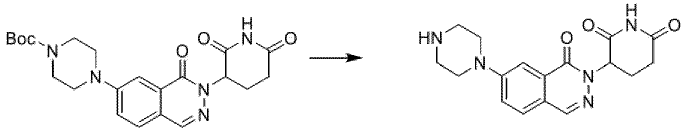

Tert-butyl 4-[3-(2,6-dioxopiperidine-3-yl)-4-oxo-3,4-dihydroptalazine-6-yl]piperazine-1-carboxylate (60 mg, 0.14 mmol) was dissolved in 20% TFA in DCM (1 ml) and reacted for 2 hours at room temperature. The reaction was terminated with an aqueous solution saturated with NaHCO₃ and extracted with EA. The organic layer was dried with Magnesium sulfate and evaporated. The reaction mixture was purified with MPLC (MC/ME Me 0 -> 10%) and a white solid was obtained as the product (yield: 40 mg/ 86%). MS (ESI, m/f): [M+1]⁺ = [342.2].
[NMR] ¹H NMR (400 MHz, DMSO-d6) δ 8.21 - 8.29 (m, 1 H) 7.76 (d, J=8.80 Hz, 1 H) 7.58 (dd, J=8.93, 2.57 Hz, 1 H) 7.47 (d, J=2.45 Hz, 1 H) 7.28 (s, 0.5 H) 7.13 (s, 0.5 H) 5.34 - 5.44 (m, 1 H) 3.26 - 3.33 (m, 4 H) 2.81 - 2.91 (m, 3 H) 2.62 - 2.69 (m, 1 H) 2.55 - 2.62 (m, 1 H) 2.31 - 2.44 (m, 1 H) 2.15 - 2.31 (m, 2 H)

### Example A-11: preparation of 3-(8-methoxy-1-oxopthalazine-2(1H)-yl)piperidine-2,6-dione

### Preparation of compound

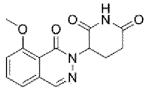

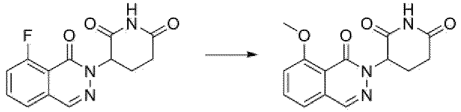

3-(6-fluoro-1-oxo-1,2-dihydroptalazine-2-yl)piperidine-2,6-dione (100 mg, 0.36 mmol) was dissolved in DMSO (1 ml), and NaOMe (19.6 mg, 0.36 mmol) was added to the reaction mixture. The reaction mixture was reacted at room temperature for 1 hour. The organic solvent was removed, and extracted with EA and water. The mixture was purified with MPLC, and the product was obtained as a white solid (yield: 80 mg / 76%). MS (ESI, m/f): [M+1]⁺ = [288.2].
[NMR] ¹H NMR (400 MHz, DMSO-d6) δ 11.04 (br. s., 1 H) 8.40 (s, 1 H) 8.14 - 8.25 (m, 1 H) 7.42 - 7.49 (m, 2 H) 5.80 (dd, J=12.23, 5.38 Hz, 1 H) 3.94 (s, 3 H) 2.86 - 2.99 (m, 1 H) 2.52 - 2.67 (m, 2 H) 2.07 - 2.17 (m, 1 H)

### Example A-12: Preparation of 3-(7-methoxy-1-oxopthalazine-2(1H)-yl)piperidine-2,6-dione

### Preparation of compound

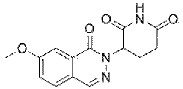

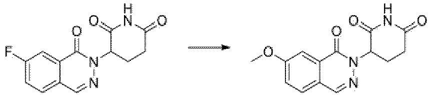

3-(7-fluoro-1-oxopthalazine-2(1H)-yl)piperidine-2,6-dione (100 mg, 0.36 mmol) was dissolved in DMSO (1 ml), and NaOMe (19.6 mg, 0.36 mmol) was added to the reaction mixture. The reaction mixture was stirred at room temperature for 1 hour. The organic solvent was removed, and extracted with EA and water. The mixture was purified with MPLC, and the product was obtained as a white solid. (Yield: 78mg / 75%) MS (ESI, m/f): [M+1]⁺ = [288.2].
[NMR] ¹H NMR (400 MHz, DMSO-d6) δ 11.01 - 11.11 (m, 1 H) 8.38 - 8.45 (m, 1 H) 7.90 - 7.96 (m, 1 H) 7.65 (d, J=2.69 Hz, 1 H) 7.56 (dd, J=8.68, 2.57 Hz, 1 H) 5.77 - 5.85 (m, 1 H) 3.93 - 3.98 (m, 3 H) 2.87 - 3.00 (m, 1 H) 2.53 - 2.70 (m, 2 H) 2.06 - 2.18 (m, 1 H)

### Example A-13: Preparation of 3-(6-methoxy-1-oxopthalazine-2(1H)-yl)piperidine-2,6-dione

### Preparation of compound

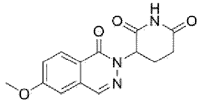

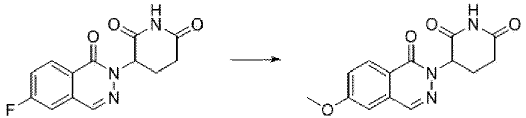

3-(6-fluoro-1-oxopthalazine-2(1H)-yl)piperidine-2,6-dione (100 mg, 0.36 mmol) was dissolved in DMSO (1 ml), and NaOMe (19.6 mg, 0.36 mmol) was added to the reaction mixture. The mixture was reacted at room temperature for 1 hour. The organic solvent was removed, and extracted with EA and water. The mixture was purified with MPLC, and the product was obtained as a white solid. (Yield: 80mg / 76%) MS (ESI, m/f): [M+1]⁺ = [288.2].
[NMR] 1H NMR (400 MHz, DMSO-d6) δ 11.01 (s, 1 H) 8.32 (s, 1 H) 7.88 (t, J=8.01 Hz, 1 H) 7.40 (d, J=8.31 Hz, 1 H) 7.44 (d, J=7.70 Hz, 1 H) 5.64 (dd, J=11.49, 4.52 Hz, 1 H) 3.90 (s, 3 H) 2.83 - 2.96 (m, 1 H) 2.52 - 2.66 (m, 2 H) 2.03 - 2.13 (m, 1 H)

### Example A-14: Preparation of 3-(5-methoxy-1-oxopthalazine-2(1H)-yl)piperidine-2,6-dione

### Preparation of compound

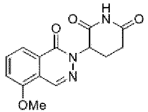

### 14-1) Preparation of Methyl 2-(Bromomethyl)-3-Methoxybenzoate

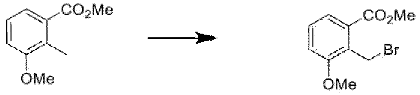

1-bromophilidine-2,5-dione (11.5 g, 64.94 mmol) and benzoyl benzene carboperoxate (786 mg, 3.24 mmol) were sequentially added to a solution of methyl 2-methyl-3-methoxybenzoate (11.7 g, 64.9 mmol) in ClCH₂CH₂Cl (250 ml) at room temperature. The reaction mixture was heated and refluxed for 3 hours. The point at which the red color disappears is the time when the reaction is completed. After cooling, the reactants were washed with water, dried with Magnesium sulfate, and concentrated under reduced pressure. The crude product was used in the next reaction without additional purification. (16.5 g, 98.2%) MS (ESI, m/z): [M+1]⁺ = [259.4].

### 14-2) Preparation of Methyl 2-Formyl-3-Methoxybenzoate

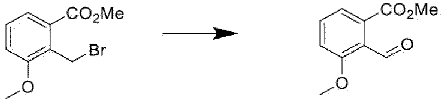

NMO (12.6 g, 108.1 mmol) and 4Å molecular sieve (50 g) were sequentially added to methyl 2-(bromomethyl)-3-methoxybenzoate (14.1 g, 54.1 mmol) solution in DCM (300 mL) at room temperature. The reactants were stirred at room temperature for 2 hours. The molecular sieve was filtered and washed with DCM (100 mL). The DCM layer was washed with water (250 mL), dried with Magnesium sulfate, and concentrated under reduced pressure. The residue was purified by column chromatography, and the title compound was obtained as a white solid. (8.3 g, 80.01%) MS (ESI, m/f): [M+1]⁺ = [195.0].

### 14-3) Preparation of 2-formyl-3-methoxybenzoic acid

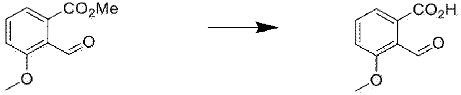

Lithium hydroxide (2.4 g, 100.5 mmol) in H2O (100 mL) was added to a solution of methyl 2-formyl-3-methoxybenzoate (6.5 g, 33.6 mmol) in THF (100 mL) at room temperature. After stirring for 2 hours, the reactants were concentrated under reduced pressure to evaporate the THF. After cooling to 0°C, the reactants were acidified with 1N HCl and the pH was adjusted to 4. The reactants were extracted twice with ethyl acetate (250 mL). The combined ethyl acetate layer was dried with Magnesium sulfate, concentrated under reduced pressure, and white crystals were obtained. (11.2g, 82.6%) MS (ESI, m/f): [M+1]⁺ = [199.2].

### 14-4) Preparation of 5-methoxyphthalazine-1(2H)-one

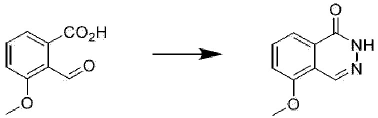

NH₂NH₂ monohydrate (10.3 g, 342 mmol) was added to a solution of 2-formyl-3-methoxybenzoic acid (8.2 g, 45.5 mmol) in MeOH (20 mL) at room temperature. After stirring for 2 hours, the reactants were concentrated under reduced pressure, poured into water (50 ml), and extracted twice with ethyl acetate (150 ml). The combined ethyl acetate layer was dried with Magnesium sulfate and concentrated under reduced pressure to obtain white crystals. (9.2g, 76.35%) MS (ESI, m/f): [M+1]⁺ = [176.9].

### 14-5) Preparation of 3-(5-methoxy-1-oxopthalazine-2(1H)-yl)piperidine-2,6-dione

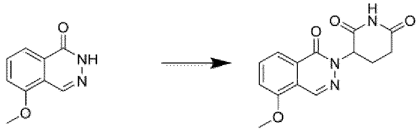

1.0 M LDA (37.4 mL) was dripped at 0°C in some suspension of 5-methoxyphthalazine-1(2H)-one (5.1 g, 28.9 mmol) in THF (250 mL). After stirring for 30 min, 3-bromoperidin-2,6-dione was partially added to the reactants. The reactants were heated to 80°C and stirred for 2 hours. Once the reaction was finished, the reactant was cooled to room temperature, poured into water (100 ml), pH was adjusted to 3~4 with 6N HCl, extracted with ethyl acetate, dried with Magnesium sulfate, and concentrated under reduced pressure. The residue was purified with MPLC and the title compound was obtained as a white crystal. (7.2g, yield 86.5%) MS (ESI, m/f): [M+1]⁺ = [288.3].

[NMR] ¹H NMR (400 MHz, DMSO-d6) δ11.06 (s, 1H), 8.51 (s, 1H), 7.87-7.77 (m, 2H), 7.54-7.51 (m, 1H), 5.85 (m, 1H), 4.0 (s, 3H), 2.98 - 2.90 (m, 1H), 2.65 - 2.55 (m, 2H), 2.14 - 2.09 (m, 1H).

### Example A-15: Preparation of 3-(6-bromo-1-oxopthalazine-2(1H)-yl)piperidine-2,6-dione

### Preparation of compound

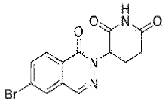

### 15-1) Preparation of 5-Bromo-3-hydroxyisobenzofuran-1(3H)-one

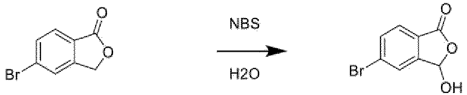

AlBN (401 mg, 2.44 mmol) was added to a mixture of 5-bromophthalide (5.2 g, 24.4 mmol) and N-bromosuccinic imide (5.6 g, 31.7 mmol) in 200 ml of ClCH₂CH₂Cl and refluxed for 8 hours. Following the completion of the reaction, TLC was carried out. The succinimide was filtered and the cake was washed with ClCH₂CH₂Cl (50 mL). The solvent was removed with vacuum, leaving 5.2g of residue, to which 50 ml of water was added. The mixture was stirred for 4 hours to reflux, the mixture was cooled, the product was filtered, neutralized washed with water, and dried, and pale yellowish-white crystals were obtained. (4.85g, yield 86.7%) MS (ESI, m/z): [M+1]⁺ = [229.6] and [230.5]

### 15-2) Preparation of 6-bromophthalazine-1(2H)-one

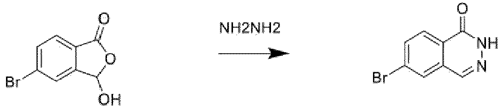

NH₂NH₂ H2O (315 mg, 9.82 mmol) was added to 5-bromo-3-hydroxy-1,3-dihydro-2-benzofuran-1-one (1.5 g, 6.55 mmol) solution in MeOH (50 mL) at room temperature and stirred for 30 minutes. The reaction was refluxed for 5 hours. The reactants were cooled to room temperature and concentrated under reduced pressure. The resulting solid was triturated with ethyl acetate to obtain white crystals. (1.31g, 5.82 mmol) MS (ESI, m/f): [M+1]⁺ = [226.3].

### 15-3) Preparation of 3-(6-bromo-1-oxopthalazine-2(1H)-yl)piperidine-2,6-dione

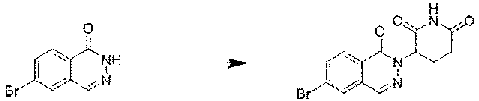

1.0 M LDA (7.33 mL) was dripped at 0°C in a 6-bromo-1,2-dihydroptalazine-1-one (1.31 g, 5.82 mmol) suspension of THF (150 mL). After stirring for 30 min, 3-bromoperidin-2,6-dione was partially added to the reactants. The reactants were heated to 80°C and stirred for 2 hours. Once the reaction was over, the reactants were cooled to room temperature, poured into water (100 ml), pH was adjusted to 3-4 with 6N HCl, extracted with ethyl acetate, dried with Magnesium sulfate, and concentrated under reduced pressure. The resulting solids were filtered, washed with ethyl acetate, and white crystals were obtained. (1.73g, 5.15 mmol) MS (ESI, m/f): [M+1]⁺ = [337.2].

[NMR] 1H NMR (400 MHz, DMSO-d6) δ11.08 (s, 1H), 8.45 (s, 1H), 8.28 (s, 1H), 8.18-8.16 (m, 1H), 8.06-8.04 (m, 1H), 5.84-5.80 (m, 1H), 2.93 - 2.90 (m, 1H), 2.65 - 2.54 (m, 2H), 2.15 - 2.12 (m, 1H).

### Example A-16: Preparation of 3-(1-oxo-8-(piperazine-1-yl)phthalazine-2(1H)-yl)piperidine-2,6-dione dihydrochloride

### Preparation of compound

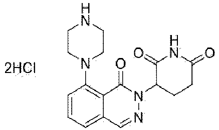

### 16-1) Preparation of 3-(1-oxo-8-(piperazine-1-yl)phthalazine-2(1H)-yl)piperidine-2,6-dione dihydrochloride

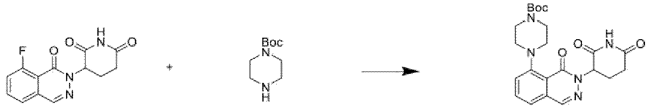

tert-butyl piperazine-1-carboxylate (244 mg, 1.13 mmol) and ethylbis (propane-2-yl)amine (423 mg, 3.24 mmol) were sequentially added to 3-(8-fluoro-1-oxo-1,2-dihydrophoptalazine-2-yl)piperidine-2,6-dione (0.3 g, 1.09 mmol) solution in DMA (4 mL). The mixture was stirred at 120°C for 5 hours. The reaction mixture was diluted with water and extracted with ethyl acetate. The organic layer was washed with brine solution, dried with Na₂SO₄, and concentrated. The residue was purified by column chromatography to obtain a pale yellowish-white oil. (415 mg, 86.2%) MS (ESI, m/f): [M+1]⁺ = [442.4]

### 16-2) Preparation of 3-(1-oxo-8-(piperazine-1-yl)phthalazine-2(1H)-yl)piperidine-2,6-dione dihydrochloride

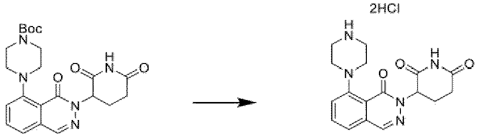

3-bromoperidin-2,6-dione (90 mg, 0.471 mmol) and K₂CO₃ (129 mg, 0.942 mmol) were sequentially added to 8-nitro-1,2-dihydrophthalazine-1-one (60 mg, 0.314 mmol) solution in DMF (1 mL). The reaction was heated to 85°C for 5 hours. After cooling, the reaction mixture was poured into water (5 mL) and extracted twice with ethyl acetate (5 mL). The mixed ethyl acetate was extracted with Magnesium sulfate and concentrated under reduced pressure. The residue was purified by column chromatography, and the title compound was obtained as white crystals. (68.0 mg, yield 71.7%) MS (ESI, m/f): [M+1]⁺ = [342.6].

[NMR] ¹H NMR (400 MHz, DMSO-d6) δ11.02 (s, 1H), 8.35 (s, 1H), 7.87-7.83 (m, 1H), 7.53-7.51 (m, 1H), 7.39-7.41 (m, 1H), 5.55 (m, 1H), 3.31-3.27 (br, 8H), 2.88 - 2.80 (m, 1H), 2.64 - 2.57 (m, 2H), 2.50 (br, 1H), 2.13 - 2.08(m, 1H).

### Example B: Preparation of CMET PROTAC based on ABN derivatives

### Example B-1: Preparation of 3-{6-[(4-{4-[(4-{2-[(2S)-2-{[5-(1-methyl-1H-pyrazole-4-yl)-1H-[1,2,3]triazolo[4,5-b]pyrazine-1-yl]methyl}morpholine-4-il]pyrimidine-5-yl}phenyl)methyl]piperazine-1-yl}-4-oxobutyl)amino]-1-oxo-1,2-dihydroptalazine-2-yl}piperidine-2,6-dione (ICT-0000922)

### Step 1) Preparation of methyl 2-(bromomethyl)-4-fluorobenzoate

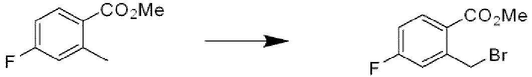

1-bromomerolidine-2,5-dione (31.8 g, 178.0 mmol) was added to a solution of methyl 4-fluoro-2-methylbenzoate (20.0 g, 119.0 mmol) in ClCH₂CH₂Cl (100 ml) at room temperature, followed by benzoyl benzene carboperoxoate (1.92 g, 5.95 mmol). The reactants were reflux heated for 3 hours. When the reaction ended, the red color disappeared. After cooling, the reactants were washed with water, dried with Magnesium sulfate, and concentrated under reduced pressure. The product was purified with MPLC. (HX/EA 0 - 5%). (yield: 22.0 g). MS (ESI, m/z): [M+H]⁺ = 248.4.

### Step 2) Preparation of 4-fluoro-2-formylbenzoate

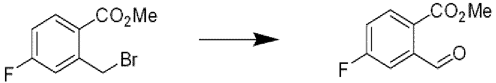

NMO (15.6 mg, 134 mmol) was added to methyl 2-(bromomethyl)-4-fluorobenzoate (22.0 g, 89 mmol) solution in DCM (100 mL) at room temperature, followed by molecular sieve 4A. The reactants were stirred at room temperature for 2 hours. The molecular sieve was filtered and washed with DCM (50 mL). The DCM layer was washed with water (200 mL), dried with Magnesium sulfate, and concentrated under reduced pressure. The residue was purified by column chromatography, and the title compound (12.0 g) was obtained as a white solid. MS (ESI, m/z): [M+H]⁺ = 183.2.

### Step 3) Preparation of 4-fluoro-2-formylbenzoic acid

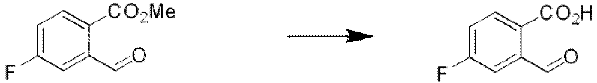

Methyl 4-fluoro-2-formylbenzoate (12.0 g, 65.9 mmol) solution in THF (50 mL) at room temperature and lithium hydroxide (1+) solution (13.8 g, 329 mmol) in H₂O (50 mL) were added. After stirring for 2 hours, the reactants were concentrated under reduced pressure to dry the THF. After cooling to 0°C, the reactants were acidified with 1M HCl and the pH was adjusted to 4. The reactants were extracted with ethyl acetate (50 mL x 2). The mixed ethyl acetate layer was dried with Magnesium sulfate and concentrated under reduced pressure to obtain white crystals (10.0 g). MS (ESI, m/z): [M+H]⁺ = 169.2.

### Step 4) Preparation of 6-fluoro-1,2-dihydrophthalazine-1-one

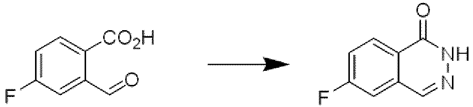

NH₂NH₂ monohydrate (2.02 mg, 40.3 mmol) was added to 4-fluoro-2-formylbenzoic acid (6.78 g, 40.3 mmol) solution in MeOH (50 mL) at room temperature. After stirring for 30 minutes, the reactants were heated to 80°C for 2 hours. The reactants were cooled to room temperature and concentrated under reduced pressure. The residue was poured into water (150 ml) and extracted with ethyl acetate (150 mL x 2). The mixed ethyl acetate layer was dried with Magnesium sulfate and concentrated under reduced pressure to obtain white crystals (5.5 g). MS (ESI, m/z): [M+H]⁺ = 165.3.

### Step 5) Preparation of 3-(6-fluoro-1-oxopthalazine-2(1H)-day)piperidine-2,6-dione

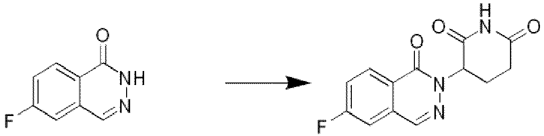

Sodium 2-methylpropane-2-oleate (175 mg, 0.914 mmol) was added to 6-fluoro-1,2-dihydroptalazin-1-one (100 mg, 0.609 mmol) solution in DMF (2 mL) at 0°C. After stirring for 30 minutes, 3-bromoperidin-2,6-dione (90 mg, 0.471 mmol) was added to the reaction mixture, and stirred for 6 hours at room temperature. The reaction mixture was poured into water (20 mL) and extracted with ethyl acetate (20 mL x 2). The bound ethyl acetate was dried with Magnesium sulfate, concentrated under reduced pressure. The residue was purified by column chromatography, and the title compound (110.0 mg) was obtained as white crystals. MS (ESI, m/z): [M+H]⁺ = 276.6.
1H NMR (400 MHz, DMSO-d6) δ 11.11 (s, 1H), 8.50 (s, 1H), 8.38 (dd, J=8.86, 5.44 Hz, 1H), 7.72 - 7.89 (m, 2H), 5.70 - 5.90 (m, 11H), 2.56 - 2.70 (m, 2H), 2.11 - 2.25 (m, 1H)

### Step 6) Preparation of 4-((2-(2-(2,6-dioxopiperidin-3-yl)-1-oxo-1,2-dihydroptalazine-6-yl)amino)butanoic acid

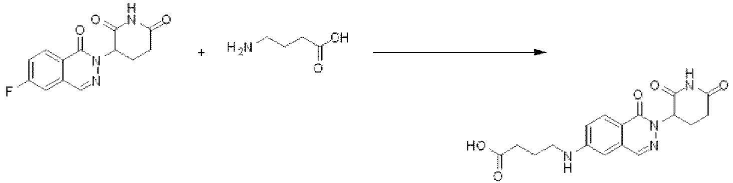

Ethylbis (propane-2-yl)amine (4.0 equivalent) was added to 3-(6-fluoro-1-oxo-1,2-dihydroptalazine-2-yl)piperidine-2,6-dione (300 mg, 1.09 mmol) and 4-aminobutanoic acid (225 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the title compound (250 mg). MS (ESI, m/z): [M+H]⁺ = 359.4

### Step 7) Preparation of 3-{6-[(4-{4-[(4-{2-[(2S)-2-{[5-(1-methyl-1H-pyrazole-4-yl)-1H-[1,2,3]triazolo[4,5-b]pyrazine-1-yl]methyl}morpholine-4-day]pyrimidinine-5-yl}phenyl)methyl]piperazine-1-yl}-4-oxobutyl)amino]-1-oxo-1,2-dihydropthalazine-2-yl}piperidine-2,6-dione (ICT-0000922)

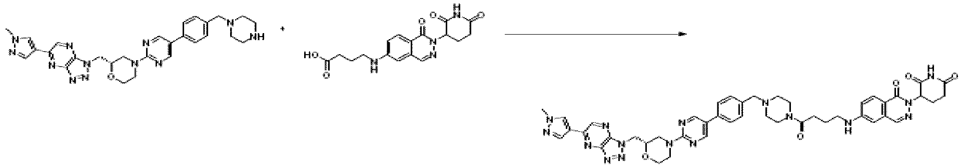

DMF (2. 0 ml) of (2S)-2-{[5-(1-methyl-1H-pyrazole-4-yl)-1H-[1,2,3]triazolo[4,5-b]pyrazine-1-yl]methyl}-4-(5-{4-[(piperazine-1-yl)methyl]phenyl}pyrimidine-2-yl)morpholine (17 mg, 0.03 mmol) and 4-{[2-(2,6-dioxopiperidin-3-yl)-1-oxo-1,2-dihydroptalazin-6-yl}amino}butanoic acid (13.2 mg, 0.04 mmol), followed by ethylbis (propane-2-yl)amine (5.0 equivalent). The reactants were stirred at room temperature for 6 hours. The reactants were poured into the reactants. It was extracted with ethyl acetate, dried with Magnesium sulfate, and concentrated under reduced pressure. The residues were purified by MeOH/DCM (0-20%) column chromatography to obtain the title compound (17 mg). MS (ESI, m/z): [M+H]⁺ = 894.1.
¹H NMR (500 MHz, DMSO-d6) δ ppm 10.91 (s, 1 H) 9.14 (s, 1 H) 8.65 - 8.70 (m, 2 H) 8.58 (s, 1 H) 8.21 - 8.29 (m, 1 H) 8.11 (s, 1 H) 7.82 - 7.87 (m, 1 H) 7.54 (m, J=8.09 Hz, 2 H) 7.27 - 7.34 (m, 2 H) 7.01 (dd, J=8.93, 2.06 Hz, 1 H) 6.86 (t, J=5.26 Hz, 1 H) 6.69 (d, J=1.98 Hz, 1 H) 5.65 (dd, J=12.05, 5.04 Hz, 1 H) 4.79 - 4.91 (m, 2 H) 4.66 (d, J=12.36 Hz, 1 H) 4.31 (d, J=13.28 Hz, 1 H) 4.12 - 4.19 (m, 1 H) 3.82 - 3.90 (m, 4 H) 3.34 - 3.47 (m, 6 H) 2.99 - 3.13 (m, 4 H) 2.78 - 2.88 (m, 1 H) 2.46 - 2.59 (m, 2 H) 2.36 (t, J=7.02 Hz, 1 H) 2.26 (d, J=14.19 Hz, 4 H) 1.94 - 2.03 (m, 1 H) 1.83 - 1.94 (m, 1 H) 1.75 (t, J=6.87 Hz, 1 H) 1.30 (t, J=6.71 Hz, 1 H) 1.14 - 1.21 (m, 1 H)

### Example B-2 : 3-{6-[(6-{4-[(4-{2-[(2S)-2-{[5-(1-methyl-1H-pyrazole-4-yl)-1H-[1,2,3]triazolo[4,5-b]pyrazine-1-yl]methyl}morpholine-4-day]pyrimidine-5-yl}phenyl)methyl]piperazine-1-yl}-6-oxohexyl)amino]-1-oxo-1,2-dihydrophthalazine-2-yl}piperidine-2,6-dione (ICT-0000923) synthesis

### Step 1) Preparation of 6-{[2-(2,6-dioxopiperidin-3-yl)-1-oxo-1,2-dihydroptalazine-6-yl]amino} Hexanoic acid

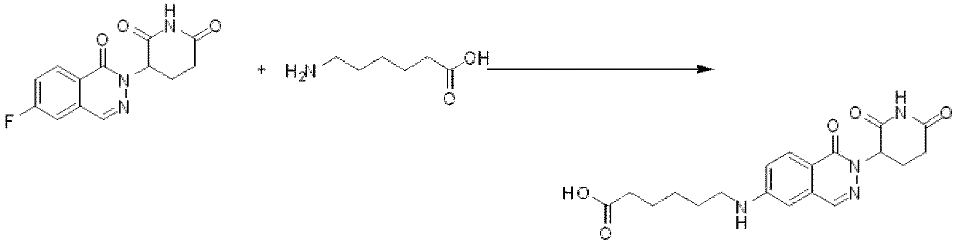

Ethylbis(propane-2-yl)amine (4.0 equivalent) was added to 3-(6-fluoro-1-oxo-1,2-dihydroptalazin-2-yl)piperidine-2,6-dione (300 mg, 1.09 mmol) and 6-aminohexanoic acid (286 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the title compound (270 mg). MS (ESI, m/z): [M+H]⁺ = 387.4

### Step 2) Preparation of 3-{6-[(6-{4-[(4-{2-[(2S)-2-{[5-(1-methyl-1H-pyrazole-4-yl)-1H-[1,2,3]triazolo[4,5-b]pyrazine-1-yl]methyl}morpholine-4-il]pyrimidine-5-il}phenyl)methyl]piperazine-1-yl}-6-oxohexyl)amino]-1-oxo-1,2-dihydropthalazine-2-yl}piperidine-2,6-dione

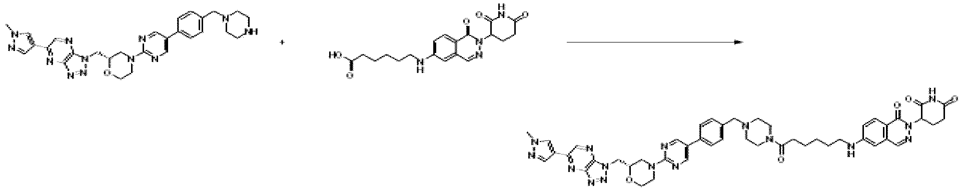

DMF (2.0ml) of (2S)-2-{[5-(1-methyl-1H-pyrazole-4-yl)-1H-[1,[1,2,3]triazolo[4,5-b]pyrazine-1-yl]methyl}-4-(5-{4-[(piperazine-1-yl)methyl]phenyl}pyrimidine-2-yl)morpholine (30 mg, 0.05 mmol) and 6-{[2-(2,6-dioxopiperidine-3-yl)-1-oxo-1,2-dihydropthalazine-6-yl]amino}hexanoic acid (23.1 mg, 0.06 mmol) was added, followed by ethylbis (propane-2-yl)amine (5.0 equivalent). The reactants were stirred at room temperature for 6 hours. The reactants were poured into the water. It was extracted with ethyl acetate, dried with Magnesium sulfate, and concentrated under reduced pressure. The residues were purified by MeOH/DCM (0-20%) column chromatography to obtain the title compound (20 mg). MS (ESI, m/z): [M+H]⁺ = 922.6.
1H NMR (400 MHz, DMSO-d6) δ ppm 10.98 (s, 1 H) 9.21 (s, 1 H) 8.74 (s, 2 H) 8.65 (s, 1 H) 8.31 (s, 1 H) 8.18 (s, 1 H) 7.91 (d, J=8.68 Hz, 1 H) 7.61 (d, J=8.19 Hz, 2 H) 7.37 (d, J=8.07 Hz, 2 H) 7.07 (dd, J=8.74, 2.26 Hz, 1 H) 6.84 - 6.92 (m, 1 H) 6.75 (d, J=2.20 Hz, 1 H) 5.66 - 5.77 (m, 1 H) 4.85 - 5.00 (m, 2 H) 4.73 (d, J=12.23 Hz, 1 H) 4.38 (d, J=13.45 Hz, 1 H) 4.22 (d, J=4.28 Hz, 1 H) 3.88 - 3.99 (m, 4 H) 3.49 (s, 2 H) 3.36 - 3.47 (m, 4 H) 3.05 - 3.18 (m, 2 H) 2.83 - 2.98 (m, 1 H) 2.61 (br. s., 1 H) 2.55 (d, J=8.07 Hz, 1 H) 2.25 - 2.40 (m, 4 H) 2.01 - 2.10 (m, 2 H) 1.88 - 2.01 (m, 2 H) 1.74 (br. s., 2 H) 1.47 - 1.66 (m, 2 H) 1.31 - 1.47 (m, 2 H) 1.14 - 1.31 (m, 2 H)

### Example B-3: Preparation of 3-(6-((5-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)5-oxopentyl)amino)1-oxopthalazine-2(1H)-yl)piperidine-2,6-dione (ICT-0000924)

### Step 1) Preparation of 5-((2-(2-(2,6-dioxopiperidin-3-yl)-1-oxo-1,2-dihydroptalazine-6-yl)amino)pentanoic acid

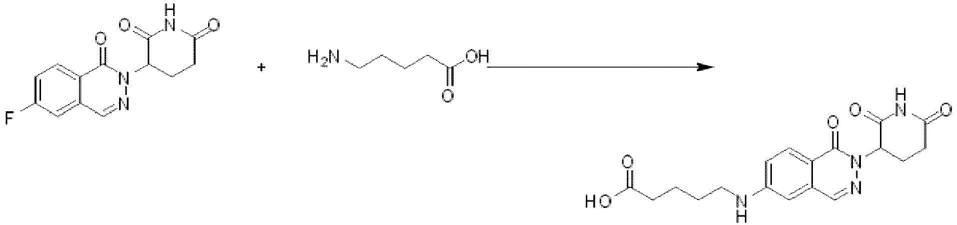

Ethylbis (propane-2-yl)amine (4.0 equivalent) was added to 3-(6-fluoro-1-oxo-1,2-dihydroptalazine-2-yl) and 5-aminopentanoic acid (245 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the heading compound (250 mg). MS (ESI, m/z): [M+H]⁺ = 373.4

### Step 2) Preparation of 3-(6-((5-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)5-oxophtyl)amino)1-oxopthalazine-2(1H)-yl)piperidine-2,6-dione (ICT-0000924)

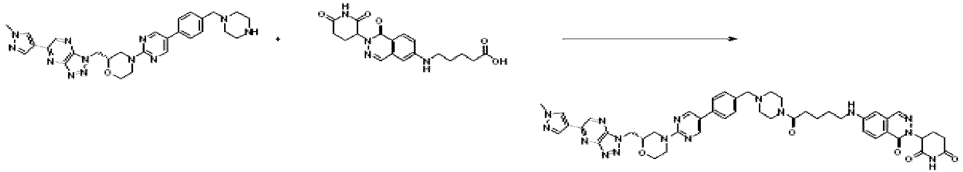

DMF (2. 0 ml) in (2S)-2-{[5-(1-methyl-1H-pyrazole-4-yl)-1H-[1,2,3]triazolo[4,5-b]pyrazin-1-yl]methyl}-4-(5-{4-[(piperazine-1-yl)methyl]phenyl}pyrimidine-2-yl)morpholine (30 mg, 0.05 mmol) and 5-((2-(2-(2,6-dioxopiperidin-3-yl)-1-oxo-1,2-dihydroptalazine-6-yl)amino)pentanoic acid (28.1 mg, 0.06 mmol), followed by ethylbis (propane-2-yl)amine (5 equivalent). The reactant was stirred at room temperature for 6 hours. The reactant was poured into the water. It was extracted with ethyl acetate, dried with Magnesium sulfate, and concentrated under reduced pressure. The residue was purified by MeOH/DCM (0-20%) column chromatography to obtain the title compound (23 mg). MS (ESI, m/z): [M+H]⁺ = 908.1.
1H NMR (400 MHz, DMSO-d6) δ ppm 10.98 (s, 1 H) 9.21 (s, 1 H) 8.74 (s, 2 H) 8.64 (s, 1 H) 8.31 (s, 1 H) 8.18 (s, 1 H) 7.91 (d, J=8.93 Hz, 1 H) 7.61 (m, J=8.19 Hz, 2 H) 7.38 (m, J=8.19 Hz, 2 H) 7.04 - 7.11 (m, 1 H) 6.87 - 6.93 (m, 1 H) 6.76 (d, J=2.08 Hz, 1 H) 5.74 (dd, J=11.55, 7.15 Hz, 1 H) 4.85 - 5.00 (m, 2 H) 4.73 (d, J=11.98 Hz, 1 H) 4.38 (d, J=12.72 Hz, 1 H) 4.22 (br. s., 1 H) 3.87 - 3.98 (m, 4 H) 3.50 (s, 2 H) 3.44 (br. s., 4 H) 3.05 - 3.19 (m, 2 H) 2.85 - 2.97 (m, 2 H) 2.59 (d, J=19.93 Hz, 1 H) 2.35 (br. s., 4 H) 2.30 (br. s., 3 H) 2.07 (dd, J=11.92, 4.22 Hz, 1 H) 1.89 - 2.01 (m, 2 H) 1.61 (br. s., 2 H) 1.37 (t, J=5.93 Hz, 2 H)

### Example B-4: Preparation of 3-(6-((7-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)7-oxoheptyl)amino)1-oxopthalazine-2(1H)-yl)piperidine-2,6-dione (ICT-0000925) synthesis

### Step 1) Preparation of 7-((2-(2-(2,6-dioxopiperidin-3-yl)-1-oxo-1,2-dihydroptalazine-6-yl)amino)heptanoic acid

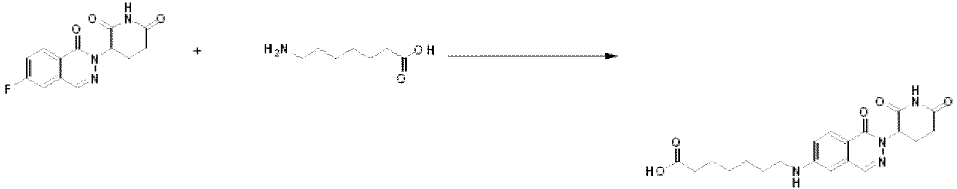

Ethylbis (propane-2-yl)amine (4.0 equivalent) was added to 3-(6-fluoro-1-oxo-1,2-dihydropetthalazine-2-yl) and 7-aminoheptanoic acid (260 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the heading compound (250 mg). MS (ESI, m/z): [M+H]⁺ = 401.4

### Step 2) Preparation of 3-(6-((7-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-il)benzyl)piperazine-1-yl)7-oxoheptyl)amino)1-oxopthalazine-2(1H)-yl)piperidine-2,6-dione (ICT-0000925)

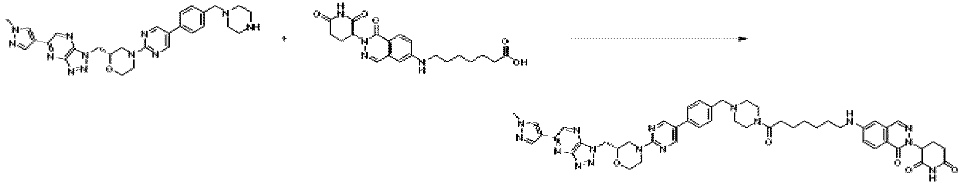

DMF (2.) 0 ml) in (2S)-2-{[5-(1-methyl-1H-pyrazole-4-yl)-1H-[1,2,3]triazolo[4,5-b]pyrazin-1-yl]methyl}-4-(5-{4-[(piperazine-1-yl)methyl]phenyl}pyrimidine-2-yl)morpholine (30 mg, 0.05 mmol) and 7-((2-(2-(2-(2-(2-(dioxopiperidine-3-yl)1-oxo-1,2-dihydropthalazine-6-yl)amino)heptanoic acid (32.1 mg, 0.06 mmol) HATU (41.3 mg, 0.11 mmol), followed by ethylbis (propane-2-yl)amine (5 equivalent). The reactant was stirred at room temperature for 6 hours. Pour the reactant into the reactant. It was extracted with ethyl acetate, dried with Magnesium sulfate, and concentrated under reduced pressure. The residues were purified by MeOH/DCM (0-20%) column chromatography to obtain the title compound (19 mg). MS (ESI, m/z): [M+H]⁺ = 936.8.
1H NMR (400 MHz, DMSO-d6) δ ppm10.98 (s, 1 H) 9.21 (s, 1 H) 8.74 (s, 2 H) 8.64 (s, 1 H) 8.31 (s, 1 H) 8.18 (s, 1 H) 7.91 (d, J=8.93 Hz, 1 H) 7.61 (m, J=8.07 Hz, 2 H) 7.38 (m, J=8.07 Hz, 2 H) 7.07 (dd, J=8.86, 2.14 Hz, 1 H) 6.88 (t, J=4.77 Hz, 1 H) 6.75 (d, J=1.83 Hz, 1 H) 5.72 (dd, J=11.92, 5.32 Hz, 1 H) 4.85 - 4.99 (m, 2 H) 4.73 (d, J=12.84 Hz, 1 H) 4.38 (d, J=13.45 Hz, 1 H) 4.22 (d, J=4.77 Hz, 1 H) 3.88 - 3.97 (m, 4 H) 3.50 (s, 2 H) 3.44 (br. s., 4 H) 3.05 - 3.17 (m, 2 H) 2.84 - 2.96 (m, 2 H) 2.61 (br. s., 1 H) 2.56 (br. s., 1 H) 2.36 (br. s., 2 H) 2.24 - 2.33 (m, 4 H) 2.01 - 2.10 (m, 1 H) 1.90 - 2.01 (m, 2 H) 1.55 - 1.65 (m, 2 H) 1.45 - 1.55 (m, 2 H) 1.31 - 1.43 (m, 4 H)

### Example B-5: Preparation of 3-(6-((2-(2-(2-(2-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)-2-oxoethoxy)ethoxy)ethyl)amino)-1-oxopthalazine-2(1H)-yl)piperidine-2,6-dione (ICT-0000935)

### Step 1) Preparation of 2-(2-(2-((2-(2-(2-(2,6-dioxopiperidin-3-yl)-1-oxo-1,2-dihydroptalazine-6-yl)amino)ethoxy)ethoxy)ethoxy)acetate

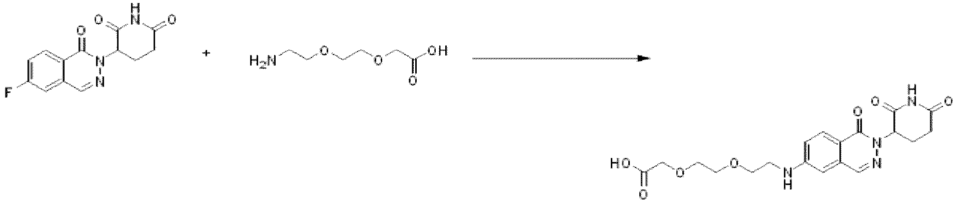

Ethylbis (propane-2-yl)amine (4.0 equivalent) was added to 3-(6-fluoro-1-oxo-1,2-dihydrophthalazine-2-yl)piperidine-2,6-dione (300 mg, 1.09 mmol) and 2-(2-(2-aminoethoxy)ethoxy-acetic acid (285 mg, 2.18 mmol) solutions in NMP (2.0 ml). The reactants were stirred at 130°C for 48 hours. After the end of the reaction, the reaction mixture was concentrated under reduced pressure. The residues were purified by MeOH/DCM (0-20%) column chromatography. Subjective compound (240 mg) was obtained. MS (ESI, m/z): [M+H]⁺ = 419.4

### Step 2) Preparation of 3-(6-((2-(2-(2-(2-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)-2-oxoethoxy)ethoxy)ethyl)amino)-1-oxopthalazine-2(1H)-yl)piperidine-2,6-dione (ICT-0000935)

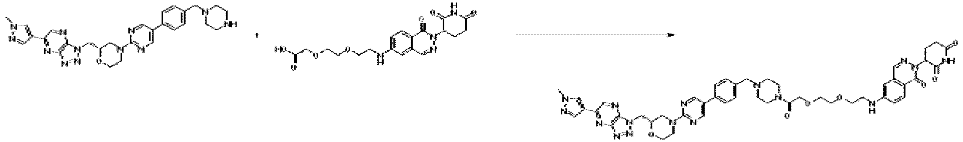

HATU (41.3 mg, 0.11 mmol) is added to the solution of (2S)-2-{[5-(1-methyl-1H-pyrazol-4-yl)-1H-[1,2,3]triazolo[4,5-b]pyrazin-1-yl]methyl}-4-(5-{4-[(piperazine-1-yl)methyl]phenyl}pyrimidine-2-yl)morpholine (30 mg, 0.05 mmol) and 2-(2-(2-(2-(2-(2-(2-(2,6-dioxopiperidin-3-yl)-1-oxo-1,2-dihydroplopthalazine-6-yl)amino)ethoxy)ethoxy)acetic acid (43.1 mg, 0.06 mmol), Ethylbis (propane-2-yl)amine (5 equivalent) was then added. The reactants were stirred at room temperature for 6 hours. The reactants were poured into water, extracted with ethyl acetate, dried with Magnesium sulfate, and concentrated under reduced pressure. The residues were purified by MeOH/DCM (0-20%) column chromatography to obtain the title compound (35 mg). MS (ESI, m/z): [M+H]⁺ = 954.1.
1H NMR (400 MHz, DMSO-d6) δ ppm10.99 (s, 1 H) 9.21 (s, 1 H) 8.79 (br. s., 2 H) 8.65 (s, 1 H) 8.31 (s, 1 H) 8.17 (s, 1 H) 7.92 (d, J=8.93 Hz, 1 H) 7.78 (br. s., 2 H) 7.57 (br. s., 2 H) 7.12 (dd, J=8.93, 2.20 Hz, 1 H) 6.93 (br. s., 1 H) 6.82 (s, 1 H) 5.73 (d, J=10.88 Hz, 1 H) 4.86 - 5.00 (m, 2 H) 4.73 (d, J=12.23 Hz, 1 H) 4.39 (d, J=12.59 Hz, 3 H) 4.21 (br. s., 3 H) 3.88 - 3.98 (m, 4 H) 3.56 - 3.66 (m, 6 H) 3.13 (br. s., 2 H) 2.84 - 2.98 (m, 2 H) 2.59 (d, J=18.10 Hz, 2 H) 2.08 (d, J=4.28 Hz, 2 H) 1.91 - 2.02 (m, 2 H) 1.75 (s, 2 H) 1.46 (br. s., 2 H) 1.34 - 1.42 (m, 3 H)

### Example B-6: Preparation of 3-[6-(12-{4-[(4-{2-[(2S)-2-{[5-(1-methyl-1H-pyrazole-4-yl)-1H-[1,2,3]triazolo[4,5-b]pyrazine-1-yl]methyl}morpholine-4-day]pyrimidine-5-yl}phenyl)methyl]piperazine-1-yl}-12-oxo-4,7,10-trioxa-1-azadodecane-1-yl)-1-oxo-1,2-dihydroptalazine-2-yl]piperidine-2,6-dione (ICT-0000938)

### Step 1) Preparation of 2-{2-[2-(2-{[2-(2,6-dioxopiperidin-3-yl)-1-oxo-1,2-dihydroptalazin-6-yl]amino}ethoxy)ethoxy]ethoxy]ethoxy}acetic acid

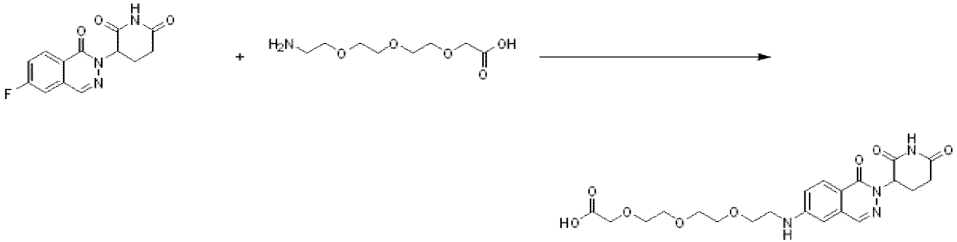

Ethylbis (propane-2-yl)amine (4.0 equivalent) was added to 3-(6-fluoro-1-oxo-1,2-dihydroptallazine-2-yl)piperidine-2,6-dione (300 mg, 1.09 mmol) and 2-{2-[2-(2-aminoethoxy)ethoxy]ethoxy}acetic acid (410 mg, 2.18 mmol) in NMP (2.0 ml). The reactants were stirred at 130°C for 48 hours. After the end of the reaction, the reaction mixture was concentrated under reduced pressure. The residues were purified by MeOH/DCM (0-20%) column chromatography. Title compound (250 mg) was obtained. MS (ESI, m/z): [M+H]⁺ = 463.4

### Step 2) Preparation of 3-[6-(12-{4-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-yl}phenyl)methyl]piperazine-1-yl}-12-oxo-4,7,10-trioxa-1-azadodecane-1-yl)-1-oxo-1,2-dihydroptalazine-2-yl]piperidine-2,6-dione (ICT-0000938)

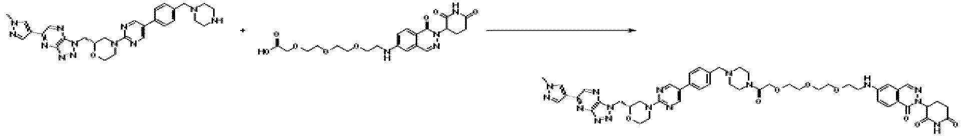

HATU (41.3 mg, 0.11 mmol) is added to a solution of (2S)-2-{[5-(1-methyl-1H-pyrazol-4-yl)-1H-[1,2,3]triazolo[4,5-b]pyrazine-1-yl]methyl}-4-(5-{4-[(piperazine-1-yl)methyl]phenyl}pyrimidin-2-yl)morpholine (30 mg, 0.05 mmol) and 2-{2-[2-{[2-(2,6-dioxopiperidin-3-yl)-1-oxo-1,2-dihydrophoptalazine-6-yl]amino}ethoxy)ethoxy]ethoxy}acetic acid (27.1 mg, 0.06 mmol), Ethylbis (propane-2-yl)amine (5 equivalent) was then added. The reactants were stirred at room temperature for 6 hours. The reactants were poured into water, extracted with ethyl acetate, dried with Magnesium sulfate, and concentrated under reduced pressure. The residues were purified by MeOH/DCM (0-20%) column chromatography to obtain the title compound (20 mg). MS (ESI, m/z): [M+H]⁺ = 998.2.
1H NMR (400 MHz, DMSO-d6) δ ppm 10.99 (s, 1 H) 9.21 (s, 1 H) 8.79 (br. s., 2 H) 8.65 (s, 1 H) 8.31 (s, 1 H) 8.16 (s, 1 H) 7.91 (d, J=8.80 Hz, 1 H) 7.78 (br. s., 2 H) 7.57 (br. s., 2 H) 7.08 - 7.14 (m, 1 H) 6.92 (br. s., 1 H) 6.80 (s, 1 H) 5.72 (dd, J=11.37, 4.89 Hz, 1 H) 4.85 - 5.00 (m, 2 H) 4.73 (d, J=12.10 Hz, 1 H) 4.39 (d, J=11.62 Hz, 3 H) 4.15 - 4.21 (m, 3 H) 3.88 - 3.97 (m, 4 H) 3.44 - 3.67 (m, 15 H) 3.10 (d, J=12.84 Hz, 3 H) 2.78 - 2.98 (m, 2 H) 2.59 (d, J=17.97 Hz, 2 H) 1.88 - 2.09 (m, 2 H) 1.33 - 1.41 (m, 3 H)

### Example B-7: Preparation of 3-[6-(15-{4-[(4-{2-[(2S)-2-{[5-(1-methyl-1H-pyrazole-4-yl)-1H-[1,2,3]triazolo[4,5-b]pyrazine-1-yl]methyl}morpholine-4-il]pyrimidine-5-yl}phenyl)methyl]piperazine-1-yl}-15-oxo-4,7,10,13-tetraoxa-1-azpentadecan-1-yl)-1-oxo-1,2-dihydroptalazine-2-yl]piperidin-2,6-dione (ICT-0000939) synthesis

### Step 1) Preparation of 14-{[2-(2,6-dioxopiperidin-3-yl)-1-oxo-1,2-dihydroptalazine-6-yl]amino}-3,6,9,12-tetratetradecanoic acid

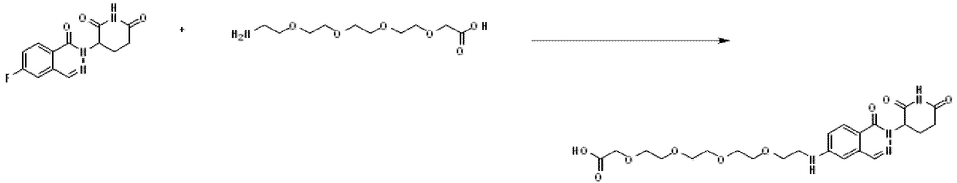

Ethylbis (propane-2-yl)amine (4.0 equivalent) was added to 3-(6-fluoro-1-oxo-1,2-dihydroptallazine-2-yl)piperidine-2,6-dione (300 mg, 1.09 mmol) and 14-amino-3,6,9,12-tetratetradecanoic acid (548 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the title compound (270 mg). MS (ESI, m/z): [M+H]⁺ = 507.4

### Step 2) Preparation of 3-[6-(15-{4-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-yl}phenyl)methyl]piperazine-1-yl}-15-oxo-4,7,10,13-tetraoxa-1-azpentadecane-1-yl)-1-oxo-1,2-dihydroptalazine-2-yl]piperidine-2,6-dione (ICT-0000939)

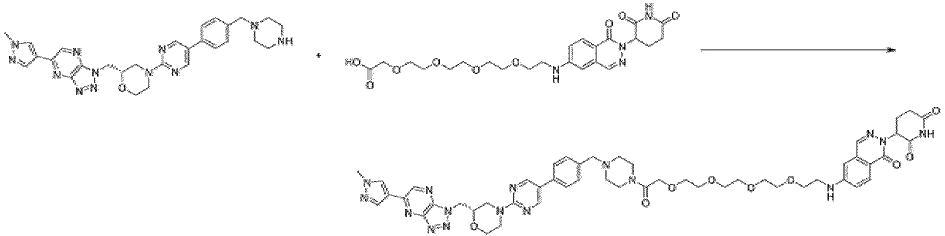

DMF (2.0ml) of (2S)-2-{[5-(1-methyl-1H-pyrazole-4-yl)-1H-[1,[1,2,3]triazolo[4,5-b]pyrazine-1-yl]methyl}-4-(5-{4-[(piperazine-1-yl)methyl]phenyl}pyrimidin-2-yl)morpholine (30 mg, 0.05 mmol) and 14-{[2-(2,6-dioxopiperidin-3-yl)-1-oxo-1,2-dihydroptalazin-6-yl]amino}-3,6,9,12-tetraoxatetradecanoic acid (20.5 mg, 0.06 mmol) with HATU (41.3 mg, 0.11 mmol), followed by ethylbis (propane-2-yl)amine (5 equivalent). The reactants were stirred at room temperature for 6 hours. The reactants were poured into water. It was extracted with ethyl acetate, dried with Magnesium sulfate, and concentrated under reduced pressure. The residues were purified by MeOH/DCM (0-20%) column chromatography to obtain the title compound (20 mg). MS (ESI, m/z): [M+H]⁺ = 1042.8.
1H NMR (400 MHz, DMSO-d6) δ ppm 10.99 (s, 1 H) 9.21 (s, 1 H) 8.79 (br. s., 2 H) 8.65 (s, 1 H) 8.31 (s, 1 H) 8.17 (s, 1 H) 7.91 (d, J=8.93 Hz, 1 H) 7.78 (br. s., 2 H) 7.58 (br. s., 2 H) 7.11 (dd, J=8.93, 2.20 Hz, 1 H) 6.93 (br. s., 1 H) 6.80 (d, J=2.08 Hz, 1 H) 5.67 - 5.77 (m, 1 H) 4.85 - 5.00 (m, 2 H) 4.73 (d, J=12.84 Hz, 2 H) 4.39 (d, J=12.59 Hz, 3 H) 4.09 - 4.21 (m, 3 H) 3.86-4.00 (m, 4 H) 3.44 - 3.66 (m, 12 H) 3.12 (dd, J=17.97, 12.59 Hz, 3 H) 2.83 - 3.00 (m, 2 H) 2.56 - 2.62 (m, 2 H) 1.88 - 2.11 (m, 6 H) 1.45 (br. s., 2 H) 1.37 (t, J=5.87 Hz, 3 H)

### Example B-8: Preparation of 3-(8-((2-(2-(3-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl})benzyl)piperazine-1-yl)-3-oxopropoxy)ethoxy)ethyl)amino)-1-oxopthalazine-2-(1H)-yl]piperidine-2,6-dione (ICT-0000940)

### Step 1) Preparation of methyl 2-fluoro-6-methylbenzoate

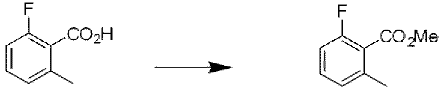

K₂CO₃ (44.8 g, 324 mmol) was added to a solution of 2-fluoro-6-methylbenzoic acid (10 g, 64.9 mmol) in acetone (200 ml) at room temperature. After stirring for 30 minutes, the reactants were treated with iodomethane (46 g, 324 mmol) and heated to 60°C for 6 hours. After cooling, the reactants were filtered and concentrated under reduced pressure. The product (11.2 g) was used for the next reaction without additional purification. MS (ESI, m/z): [M+H]⁺ = 168.3.

### Step 2) Preparation of methyl 2-(bromomethyl)-6-fluorobenzoate

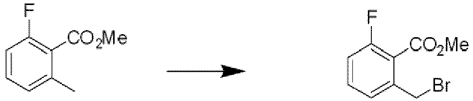

In a solution of methyl 2-fluoro-6-methylbenzoate (21.2 g, 126 mmol) in ClCH₂CH₂Cl (250 ml), 1-bromophilolidin-2,5-dione (24.7 g, 139 mmol) was added, followed by benzoyl benzene carboperoxoate (1.53 g, 6.30 mmol) at room temperature. The reactants were refluxed heated for 16 hours. At the end of the reaction, the redness disappeared. After cooling, the reactants were washed with water, dried with Magnesium sulfate, and concentrated under reduced pressure. The crude product (35 g, 112%) was used for the next reaction without additional purification. MS (ESI, m/z): [M+H]⁺ = 247.9.

### Step 3) Synthesis of methyl 2-fluoro-6-formylbenzoate

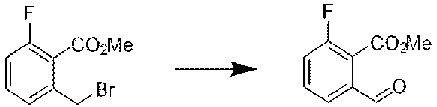

NMO (24.9 g, 212 mmol) was added to a solution of methyl 2-(bromomethyl)-6-fluorobenzoate (35 g, 142 mmol) in DCM (280 mL) at room temperature. The reactants were stirred at room temperature for 4 hours. The DCM layer was washed with water (200 mL), dried with Magnesium sulfate, and concentrated under reduced pressure. The residue was purified by column chromatography, and the title compound (11.52 g) was obtained as a white solid. MS (ESI, m/z): [M+H]⁺ = 183.1.

### Step 4) Preparation of 2-fluoro-6-formylbenzoic acid

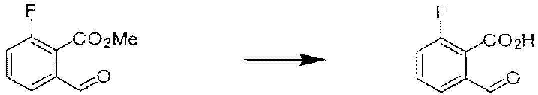

Methyl 2-fluoro-6-formylbenzoate (11.5 g, 63.2 mmol) solution in THF (82 mL) at room temperature and lithium hydroxide (1+) solution (7.57 g, 180 mmol) in H2O (41 mL) were added to the solution THF. After stirring for 4 hours, the reactants were concentrated under reduced pressure and THF was dried. After cooling to 0°C, the reactants were acidified to 1M HCl and the pH was adjusted to 4. The reactants were extracted with ethyl acetate (100 mL x 2). The mixed ethyl acetate layer was dried with Magnesium sulfate, concentrated under reduced pressure, white crystals (10.4 g) were obtained MS (ESI, m/z): [M+H]⁺ = 168.8

### Step 5) Preparation of 8-Fluoroptalazine-1(2H)-one

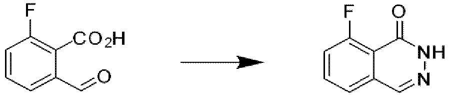

NH₂NH₂ monohydrate (3.72 mg, 61.9 mmol) was added to 2-fluoro-6-formylbenzoic acid (10.4 g, 61.9 mmol) solution in MeOH (120 mL) at room temperature, and stirred at room temperature for 16 hours. The white precipitation was filtered and washed with MeOH. The obtained white solids were triturated to 100 ml EA, filtered, and white crystals (3.05 g, 30%) were obtained. MS (ESI, m/z): [M+H]⁺ = 165.1.

### Step 6) Preparation of 3-(8-fluoro-1-oxopthalazine-2(1H)-yl)piperidine-2,6-dione

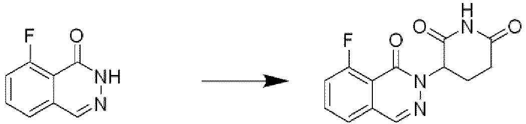

NaH (60%, 53.6 mg, 1.34 mmol) was added to 8-fluoroptalazine-1(2H)-one (200 mg, 1.22 mmol) solution in DMF (5 mL) at 0°C and stirred for 30 minutes. 3-bromoperidin-2,6-dione (468 mg, 2.44 mmol) was added to the solution and stirred at room temperature for 6 hours. The reactants were terminated with water and extracted with ethyl acetate (20 mL x2). The bound ethyl acetate was dried with Magnesium sulfate and concentrated under reduced pressure. The residues were purified by column chromatography. The title compound (70.0 mg, 20.8%) was obtained as white crystals. MS (ESI, m/z): [M+H]⁺ = 276.1.
1H NMR (400 MHz, DMSO-d6) δppm 11.06 (s, 1H), 8.48 (s, 1H), 7.99 (ddd, J = 4.6, 7.2, 8.2 Hz, 1H), 7.80 (d, J = 7.2 Hz, 1H), 7.67 (dd, J = 8.2, 11.4 Hz, 1H), 5.77 (dd, J=5.3, 12.0 Hz, 1H), 2.99-2.77 (m, 1H), 2.68-2.51 (m, 2H), 2.23-2.02 (m, 1H)

### Step 7) Preparation of 3-[2-(2-{[3-(2,6-dioxopiperidin-3-yl)-4-oxo-3,4-dihydroptalazine-5-yl]amino}ethoxy)ethoxy]propanoic acid

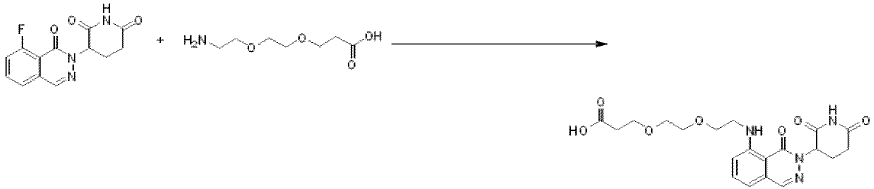

Ethylbis (propane-2-yl)amine (4.0 equivalent) was added to 3-(8-fluoro-1-oxo-1,2-dihydroptallazine-2-yl)piperidine-2,6-dione (300 mg, 1.45 mmol) and 3-[2-(2-aminoethoxy)ethoxy]propanoic acid (257 mg, 1.45 mmol) solutions in NMP (2.0 ml). The reactants were stirred at 130°C for 48 hours. After the end of the reaction, the reaction mixture was concentrated under reduced pressure. The residues were purified by MeOH/DCM (0-20%) column chromatography. Subjective compound (290 mg) was obtained. MS (ESI, m/z): [M+H]⁺ = 433.4

### Step 8) Preparation of 3-(8-((2-(2-(3-(4-(4-(2-(2-(2-((S)-2-((5-(1-methyl-1H-pyrazole-4-yl)-1H-[1,2,3]triazolo[4,5-b]pyrazine-1-yl)methyl)morpholino)pyrimidine-5-yl})benzyl)piperazine-1-yl)-3-oxopropoxy)ethoxy)ethyl)amino)1-oxopthalazine-2-(1H)-yl]piperidin-2,6-dione (ICT-0000940)

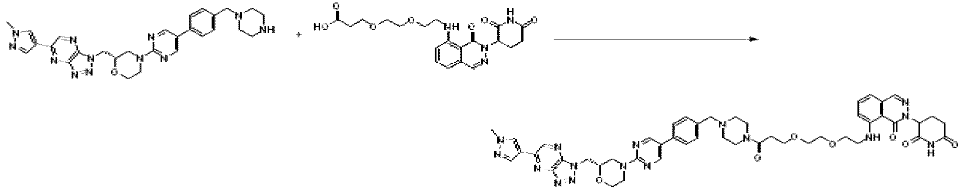

HATU (38.0 mg, 0.10 mmol) is added to the solution of (2S)-2-{[5-(1-methyl-1H-pyrazole-4-yl)-1H-[1,2,3]triazolo[4,5-b]pyrazin-1-yl]methyl}-4-(5-{4-[(piperazine-1-yl)methyl]phenyl}pyrimidine-2-yl)morpholine (27.6 mg, 0.05 mmol) and 3-[2-(2-{[3-(2,6-dioxopiperidin-3-yl)-4-oxo-3,4-dihydroptalazin-5-yl]amino}ethoxy)ethoxy]propanoic acid (25.9 mg, 0.06 mmol), Ethylbis (propane-2-yl)amine (5 equivalent) was then added. The reactants were stirred at room temperature for 6 hours. The reactants were poured into water, extracted with ethyl acetate, dried with Magnesium sulfate, and concentrated under reduced pressure. The residues were purified by MeOH/DCM (0-20%) column chromatography to obtain the title compound (25 mg). MS (ESI, m/z): [M+H]⁺ = 968.1.
¹H NMR (400 MHz, DMSO-d6) δppm 10.96 (s, 1 H) 9.14 (s, 1 H) 8.71 - 8.79 (m, 1 H) 8.67 (s, 2 H) 8.57 (s, 1 H) 8.24 (s, 1 H) 8.15 (s, 1 H) 7.51 - 7.61 (m, 2 H) 7.29 (d, J=7.93 Hz, 2 H) 6.84 (d, J=7.48 Hz, 2 H) 5.64 (d, J=7.32 Hz, 1 H) 4.79 - 4.93 (m, 2 H) 4.66 (d, J=12.36 Hz, 1 H) 4.31 (d, J=12.97 Hz, 1 H) 4.15 (br. s., 1 H) 3.52 - 3.62 (m, 4 H) 3.39 - 3.52 (m, 4 H) 3.36 (br. s., 4 H) 2.98 - 3.11 (m, 3 H) 2.78 - 2.88 (m, 1 H) 2.39 - 2.58 (m, 5 H) 2.25 (d, J=16.63 Hz, 2 H) 1.98 - 2.05 (m, 1 H) 1.83 - 1.93 (m, 1 H) 1.12 - 1.24 (m, 6 H)

**Example B-9: Preparation of 3-[8-(12-{4-[(4-{2-[(2S)-2-{[5-(1-methyl-1H-pyrazole-4-yl)-1H-[1,2,3]triazolo[4,5-b]pyrazine-1-yl]methyl}morpholine-4-day]pyrimidine-5-yl}phenyl)methyl]piperazine-1-il}-12-oxo-4,7,10-trioxa-1-azadodecane-1-yl)-1-oxo-1,2-dihydroptalazine-2-yl]piperidine-2,6-dione (ICT-0000942)**

### Step 1) Preparation of 2-{2-[2-(2-{[3-(2,6-dioxopiperidin-3-yl)-4-oxo-3,4-dihydroptalazin-5-yl}amino}ethoxy)ethoxy]ethoxy}ethoxy}acetate

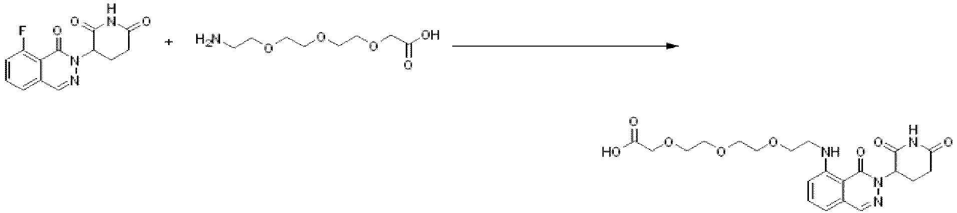

Ethylbis (propane-2-yl)amine (4.0 equivalent) was added to 3-(8-fluoro-1-oxo-1,2-dihydroptallazine-2-yl)piperidine-2,6-dione (399 mg, 1.45 mmol) and 2-{2-[2-(2-(2-aminoethoxy)ethoxy}acetic acid (300 mg, 1.45 mmol) in NMP (2.0 ml). The reactants were stirred at 130°C for 48 hours. After the reaction was completed, the reaction mixture was concentrated under reduced pressure. The residues were purified by MeOH/DCM (0-20%) column chromatography. Subjective compound (290 mg) was obtained. MS (ESI, m/z): [M+H]⁺ = 463.5

### Step 2) Preparation of 3-[8-(12-{4-[(4-{2-[(2S)-2-{[5-(1-methyl-1H-pyrazole-4-yl)-1H-[1,2,3]triazolo[4,5-b]pyrazine-1-yl]methyl}morpholine-4-il]pyrimidine-5-yl}phenyl)methyl]piperazine-1-yl}-12-oxo-4,7,10-trioxa-1-azadodecane-1-yl)-1-oxo-1,2-dihydroptalazine-2-yl]piperidin-2,6-dione (ICT-0000942)

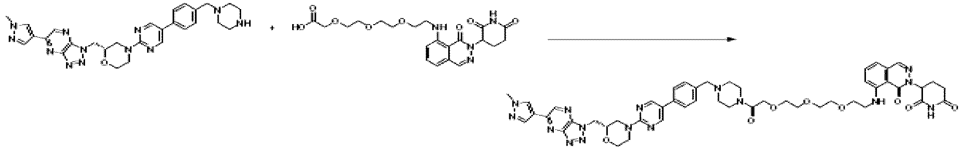

HATU (38.0 mg, 0.10 mmol) is added to (2S)-2-{[5-(1-methyl-1H-pyrazole-4-yl)-1H-[1,2,3]triazolo[4,5-b]pyrazin-1-yl]methyl}-4-(5-{4-[(piperazine-1-yl)methyl]phenyl}pyrimidin-2-yl)morpholine (27.6 mg, 0.05 mmol) and 2-{2-[2-{[3-(2,6-dioxopiperidin-3-yl)-4-oxo-3,4-dihydroptalazine-5-yl]amino}ethoxy)ethoxy]ethoxy}acetic acid (27.7 mg, 0.06 mmol), Ethylbis (propane-2-yl)amine (5 equivalent) was then added. The reactants were stirred at room temperature for 6 hours. The reactants were poured into water, extracted with ethyl acetate, dried with Magnesium sulfate, and concentrated under reduced pressure. The residues were purified by MeOH/DCM (0-20%) column chromatography to obtain the title compound (26 mg). MS (ESI, m/z): [M+H]⁺ = 998.1.
1H NMR (400 MHz, DMSO-d6) δppm 10.96 (s, 1 H) 9.14 (s, 1 H) 8.72 - 8.78 (m, 1 H) 8.64 - 8.69 (m, 2 H) 8.58 (s, 1 H) 8.24 (s, 1 H) 8.13 - 8.16 (m, 1 H) 7.51 - 7.60 (m, 2 H) 7.29 (d, J=7.93 Hz, 2 H) 6.80 - 6.87 (m, 2 H) 4.79 - 4.91 (m, 2 H) 4.65 (d, J=12.97 Hz, 1 H) 4.31 (d, J=13.12 Hz, 1 H) 4.04 (s, 2 H) 3.97 - 4.02 (m, 1 H) 3.82- 3.90 (m, 4 H) 3.52 - 3.63 (m, 4 H) 3.44 - 3.52 (m, 4 H) 3.29 - 3.36 (m, 4 H) 2.99 - 3.09 (m, 2 H) 2.78 - 2.88 (m, 1 H) 2.56 (br. s., 1 H) 2.52 (br. s., 1 H) 2.29 (br. s., 2 H) 2.24 (br. s., 1 H) 1.82 - 1.94 (m, 2 H) 1.30 (t, J=6.56 Hz, 2 H) 1.11 - 1.25 (m, 6 H)

### Example B-10: Preparation of 3-[8-(16-{4-[(4-{2-[(2S)-2-{[5-(1-methyl-1H-pyrazole-4-yl)-1H-[1,2,3]triazolo[4,5-b]pyrazine-1-yl]methyl}morpholine-4-day]pyrimidine-5-yl}phenyl)methyl]piperazine-1-yl}-16-oxo-4,7,10,13-tetraoxa-1-azahexadecane-1-yl)-1-oxo-1,2-dihydroptalazine-2-yl]piperidine-2,6-dione (ICT-0000946)

### Step 1) Preparation of 2-{2-[2-(2-{[3-(2,6-dioxopiperidin-3-yl)-4-oxo-3,4-dihydroptalazin-5-yl}amino}ethoxy)ethoxy]ethoxy}ethoxy}acetate

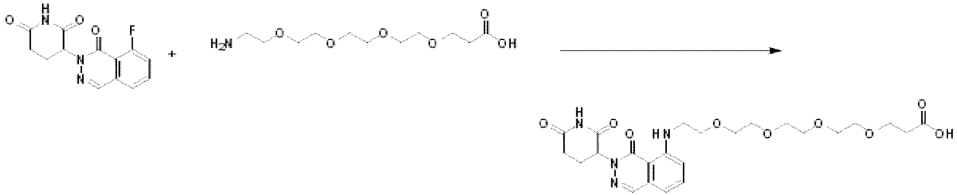

Ethylbis (propane-2-yl)amine (4.0 equivalent) was added to 3-(8-fluoro-1-oxo-1,2-dihydroptallazine-2-yl)piperidine-2,6-dione (399 mg, 1.45 mmol) and 2-{2-[2-(2-(2-aminoethoxy)ethoxy}acetic acid (300 mg, 1.45 mmol) in NMP (2.0 ml). The reactants were stirred at 130°C for 48 hours. After the reaction was completed, the reaction mixture was concentrated under reduced pressure. The residues were purified by MeOH/DCM (0-20%) column chromatography. Subjective compound (290 mg) was obtained. MS (ESI, m/z): [M+H]⁺ = 463.5

### Step 2) Preparation of 3-[8-(16-{4-[(4-{2-[(2S)-2-{[5-(1-methyl-1H-pyrazole-4-yl)-1H-[1,2,3]triazolo[4,5-b]pyrazine-1-yl]methyl}morpholine-4-day]pyrimidine-5-day}phenyl)methyl]piperazine-1-yl}-16-oxo-4,7,10,13-tetraoxa-1-azahexadecane-1-yl)-1-oxo-1,2-dihydroptalazine-2-yl]piperidine-2,6-dione (ICT-0000946)

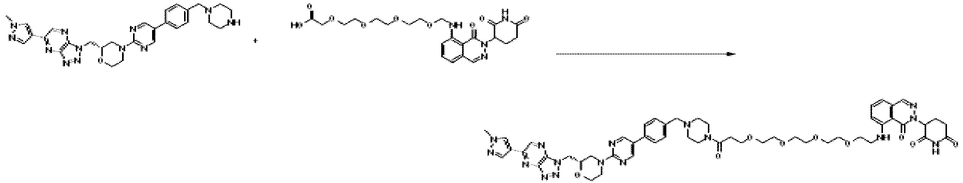

HATU (38.0 mg, 0.10 mmol) is added to the solution of (2S)-2-{[5-(1-methyl-1H-pyrazole-4-yl)-1H-[1,2,3]triazolo[4,5-b]pyrazin-1-yl]methyl}-4-(5-{4-[(piperazine-1-yl)methyl]phenyl}pyrimidin-2-yl)morpholine (27.6 mg, 0.05 mmol) and 2-{2-[2-{[3-(2,6-dioxopiperidine-3-yl)-4-oxo-3,4-dihydropetalazine-5-yl]amino}ethoxy)ethoxy]ethoxy}acetic acid (27.7 mg, 0.06 mmol), Ethylbis (propane-2-yl)amine (5 equivalent) was then added. The reactants were stirred at room temperature for 6 hours. The reactants were poured into water, extracted with ethyl acetate, dried with Magnesium sulfate, and concentrated under reduced pressure. The residues were purified by MeOH/DCM (0-20%) column chromatography to obtain the title compound (26 mg). MS (ESI, m/z): [M+H]⁺ = 998.1. 1H NMR (400 MHz, DMSO-d 6)

### Example B-11: Preparation of 3-{8-[(5-{4-[(4-{2-[(2S)-2-{[5-(1-methyl-1H-pyrazole-4-yl)-1H-[1,2,3]triazolo[4,5-b]pyrazine-1-yl]methyl}morpholine-4-il]pyrimidine-5-yl}phenyl)methyl]piperazine-1-yl}-5-oxopentyl)amino]-1-oxo-1,2-dihydropthalazine-2-yl}piperidine-2,6-dione (ICT-0000959)

### Step 1) Preparation of 5-{[3-(2,6-dioxopiperidin-3-yl)-4-oxo-3,4-dihydroptalazine-5-yl]amino}pentanoic acid

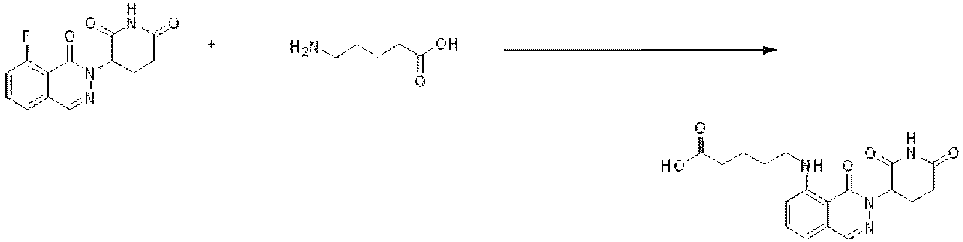

Ethylbis (propane-2-yl)amine (4.0 equivalent) was added to 3-(8-fluoro-1-oxopthalazine-2(1H)-yl) and 5-aminopentanoic acid (255 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the title compound (300 mg). MS (ESI, m/z): [M+H]⁺ = 373.4

### Step 2) Preparation of 3-{8-[(5-{4-[(4-{2-[(2S)-2-{[5-(1-methyl-1H-pyrazole-4-yl)-1H-[1,2,3]triazolo[4,5-b]pyrazine-1-yl]methyl}morpholine-4-il]pyrimidine-5-yl}phenyl)methyl]piperazine-1-yl}-5-oxopentyl)amino]-1-oxo-1,2-dihydroptalazine-2-yl}piperidine-2,6-dione (ICT-0000959)

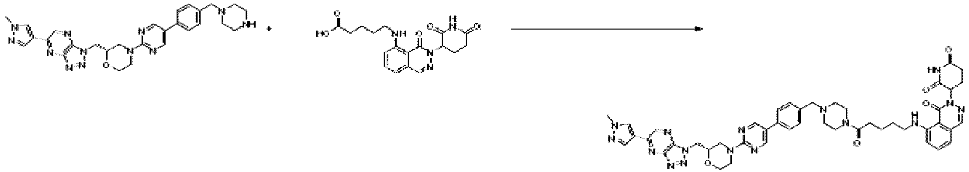

DMF (2. 0 ml) in (2S)-2-{[5-(1-methyl-1H-pyrazole-4-yl)-1H-[1,2,3]triazolo[4,5-b]pyrazin-1-yl]methyl}-4-(5-{4-[(piperazine-1-yl)methyl]phenyl}pyrimidine-2-yl)morpholine (30 mg, 0.05 mmol) and 5-{[3-(2,6-dioxopiperidine-3-yl)-4-oxo-3,4-dihydroptalazin-5-yl}amino}pentanoic acid (22.2 mg, 0.06 mmol), followed by ethylbis (propane-2-yl)amine (5 equivalent). The reactant was stirred at room temperature for 6 hours. The reactant was poured into the water. It was extracted with ethyl acetate, dried with Magnesium sulfate, and concentrated under reduced pressure. The residues were purified by MeOH/DCM (0-20%) column chromatography to obtain the title compound (20 mg). MS (ESI, m/z): [M+H]⁺ = 908.8.
1H NMR (400 MHz, DMSO-d6) δppm 11.04 (s, 1 H) 9.21 (s, 1 H) 8.81 (s, 2 H) 8.72 (br. s., 1 H) 8.65 (s, 1 H) 8.31 (s, 1 H) 8.24 (s, 1 H) 7.79 (d, J=7.46 Hz, 2 H) 7.66 (t, J=7.95 Hz, 1 H) 7.56 (d, J=8.44 Hz, 2 H) 6.92 (d, J=7.21 Hz, 1 H) 6.89 (d, J=8.80 Hz, 1 H) 5.69 (br. s., 1 H) 4.82 - 4.99 (m, 2 H) 4.73 (d, J=12.96 Hz, 1 H) 4.42 (br. s., 2 H) 4.38 (br. s., 2 H) 4.22 (br. s., 1 H) 4.07 (br. s., 1 H) 3.87 - 4.00 (m, 4 H) 3.23 (m, 4 H) 3.02 - 3.20 (m, 4 H) 2.77 - 3.01 (m, 2 H) 2.52 - 2.68 (m, 2 H) 2.33 (br. s., 4 H) 2.09 (s, 1 H) 1.64 (br. s., 2 H) 1.23 (s, 2 H)

### Example B-12: Preparation of 3-{8-[(7-{4-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-yl}phenyl)methyl]piperazine-1-yl}-7-oxoheptyl)amino]-1-oxo-1,2-dihydrophthalazine-2-yl}piperidine-2,6-dione (ICT-0000960)

### Step 1) Preparation of 7-{[3-(2,6-dioxopiperidin-3-yl)-4-oxo-3,4-dihydroptalazine-5-yl]amino} heptanoic acid

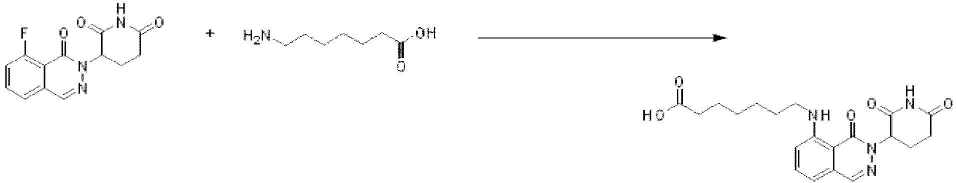

Ethylbis (propane-2-yl)amine (4.0 equivalent) was added to 3-(8-fluoro-1-oxo-1,2-dihydroptalazine-2-yl) and 4-aminobutanoic acid (317 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the heading compound (300 mg). MS (ESI, m/z): [M+H]⁺ = 401.4

### Step 2) Preparation of 3-{8-[(7-{4-[(4-{2-[(2S)-2-{[5-(1-methyl-1H-pyrazole-4-yl)-1H-[1,2,3]triazolo[4,5-b]pyrazine-1-yl]methyl}morpholine-4-il]pyrimidine-5-yl}phenyl)methyl]piperazine-1-yl}-7-oxohepty)amino]-1-oxo-1,2-dihydropthalazine-2-yl}piperidine-2,6-dione (ICT-0000960)

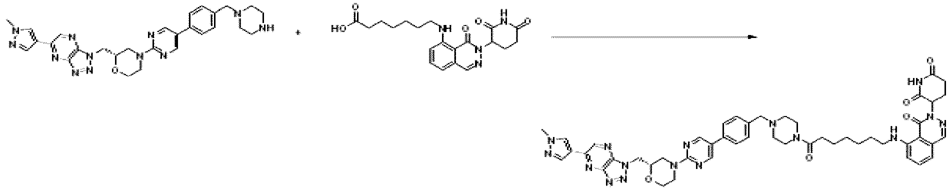

DMF (2. 0 ml) of (2S)-2-{[5-(1-methyl-1H-pyrazole-4-yl)-1H-[1,2,3]triazolo[4,5-b]pyrazin-1-yl]methyl}-4-(5-{4-[(piperazine-1-yl)methyl]phenyl}pyrimidine-2-yl)morpholine (30 mg, 0.05 mmol) and 7-{[3-(2,6-dioxopiperidin-3-yl)-4-oxo-3,4-dihydroptalazine-5-yl]amino}amino}, followed by ethylbis (propane-2-yl)amine (5 equivalent). It was extracted with ethyl acetate, dried with Magnesium sulfate, and concentrated under reduced pressure. The residues were purified by MeOH/DCM (0-20%) column chromatography to obtain the title compound (25 mg). MS (ESI, m/z): [M+H]⁺ = 936.1
1H NMR (400 MHz, DMSO-d6) δppm 11.03 (s, 1 H) 9.21 (s, 1 H) 8.71 (s, 1 H) 8.75 (s, 2 H) 8.65 (s, 1 H) 8.31 (s, 1 H) 8.23 (s, 1 H) 7.79 (br. s., 1 H) 7.54 - 7.70 (m, 3 H) 7.39 (br. s., 1 H) 6.87 (d, J=8.56 Hz, 1 H) 6.91 (d, J=7.46 Hz, 1 H) 5.69 (br. s., 1 H) 4.85 - 4.99 (m, 2 H) 4.73 (d, J=12.23 Hz, 1 H) 4.38 (d, J=14.06 Hz, 2 H) 4.21 (br. s., 1 H) 3.88 - 3.98 (m, 4 H) 3.50 (br. s., 2 H) 3.43 (br. s., 4 H) 3.04 - 3.23 (m, 4 H) 2.80 - 2.96 (m, 2 H) 2.55 - 2.69 (m, 2 H) 2.33 (s, 2 H) 1.62 (br. s., 2 H) 1.50 (br. s., 2H) 1.40 (br. s., 2 H) 1.34 (br. s., 2 H) 1.14 - 1.28 (m, 4 H)

### Example B-13: Preparation of 3-[8-({2-[2-(3-{4-[(4-{2-[(2S)-2-{[5-(1-methyl-1H-pyrazole-4-yl)-1H-[1,2,3]triazolo[4,5-b]pyrazine-1-yl]methyl}morpholine-4-il]pyrimidine-5-il}phenyl)methyl]piperazine-1-yl}-3-oxoproxi)ethoxy]ethyl}amino)1-oxo-1,2-dihydroptalazine-2-yl]piperidin-2,6-dione (ICT-0000967)

### Step 1) Preparation of 17-((3-(2,6-dioxopiperidin-3-yl)-4-oxo-3,4-dihydroptalazine-5-yl)amino)-3,6,9,12,15-pentaoxaheptadecanoic acid

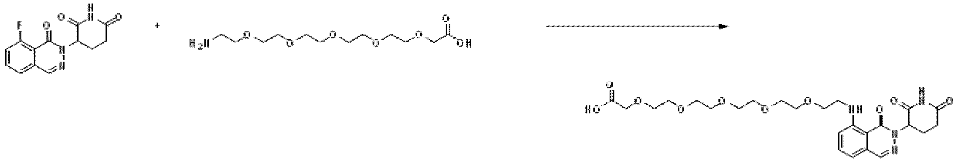

Ethylbis (propane-2-yl)amine (4.0 equivalent) was added to 3-(8-fluoro-1-oxopthalazine-2(1H)-yl)piperidine-2,6-dione (300 mg, 1.45 mmol) and 17-amino-3,6,9,12,15-pentaheptadecanic acid (327 mg, 1.45 mmol) solutions in NMP (2.0 ml). The reactants were stirred at 130°C for 48 hours. After the end of the reaction, the reaction mixture was concentrated under reduced pressure. The residues were purified by MeOH/DCM (0-20%) column chromatography to obtain the heading compound (310 mg). MS (ESI, m/z): [M+H]⁺ = 551.1

### Step 2) Preparation of 3-(8-((17-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)17-oxo-3,6,9,12,15-pentaheptacel)amino)-1-oxopthalazine-2(1H)-yl)piperidine-2,6-dione (ICT-0000967)

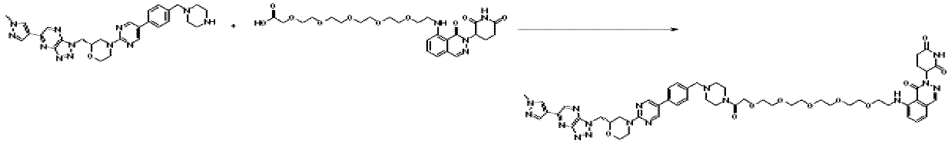

DMF (2.0ml) of (2S)-2-{[5-(1-methyl-1H-pyrazole-4-yl)-1H-[1,[1,2,3]triazolo[4,5-b]pyrazine-1-yl]methyl}-4-(5-{4-[(piperazine-1-yl)methyl]phenyl}pyrimidin-2-yl)morpholine (30.1 mg, 0.05 mmol) and 17-{[3-(2,6-dioxopiperidin-3-yl)-4-oxo-3,4-dihydroptalazine-5-yl]amino}-3,6,9,12,15-pentaheptaceanoic acid (30.0 mg, 0.05 mmol) with HATU (41.4 mg, 0.10 mmol), followed by ethylbis (propane-2-yl)amine (5 equivalent). The reactants were stirred at room temperature for 6 hours. The reactants were poured into water. It was extracted with ethyl acetate, dried with Magnesium sulfate, and concentrated under reduced pressure. The residues were purified by MeOH/DCM (0-20%) column chromatography to obtain the title compound (25 mg). MS (ESI, m/z): [M+H]⁺ = 1086.1.
1H NMR (400 MHz, DMSO-d6) δppm 10.97 (s, 1 H) 9.14 (s, 1 H) 8.69 (s, 2 H) 8.64 (t, J=4.88 Hz, 1 H) 8.58 (s, 1 H) 8.24 (s, 1 H) 8.16 (s, 1 H) 7.52 - 7.61 (m, 2 H) 7.29 (d, J=7.93 Hz, 2 H) 6.84 (d, J=7.32 Hz, 1 H) 6.81 (d, J=8.54 Hz, 1 H) 5.63 (d, J=7.48 Hz, 1 H) 4.79 - 4.92 (m, 2 H) 4.66 (d, J=12.66 Hz, 1 H) 4.31 (d, J=13.12 Hz, 1 H) 4.06 - 4.19 (m, 1 H) 3.82 - 3.90 (m, 4 H) 3.51 - 3.61 (m, 1 H) 3.38 - 3.50 (m, 3 H) 3.36 (br. s., 2 H) 2.98 - 3.18 (m, 5 H) 2.76 - 2.88 (m, 1 H) 2.45 - 2.59 (m, 4 H) 2.29 (br. s., 1 H) 2.25 (br. s., 3 H) 1.98 - 2.07 (m, 1 H) 1.82 - 1.94 (m, 1 H) 1.57 (quin, J=6.98 Hz, 2 H) 1.48 (quin, J=7.36 Hz, 2 H) 1.29 - 1.40 (m, 2 H) 1.13 - 1.24 (m, 11 H)

### Example B-14: Preparation of 3-(8-((21-(4-(4-(2-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)21-oxo-3,6,9,12,15,18-hexaoxahennicosyl)amino)-1-oxopthalazine-2(1H)-yl)piperidin-2,6-dione (ICT-0000968)

### Step 1) Preparation of 1-((3-(2,6-dioxopiperidine-3-yl)4-oxo-3,4-dihydroptalazine-5-yl)amino)-3,6,9,12,15,18-hexaoxahenicoic acid-21-oic acid

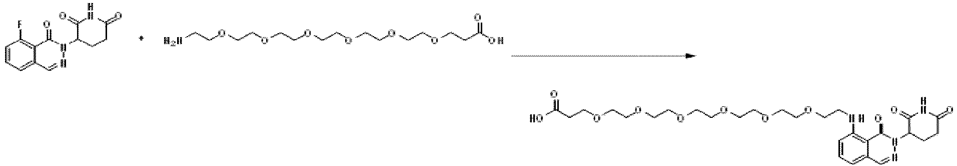

Ethylbis (propane-2-yl)amine (4.0 equivalent) was added to 3-(8-fluoro-1-oxopthalazine-2(1H)-yl)piperidine-2,6-dione (8-fluoro-1-oxopthalazine-2(1H)-yl)piperidine-2,6-dione (300 mg, 1.45 mmol) and 1-amino-3,6,9,12,15,18-hexaoxaheniconic acid-21-ohic acid (327 mg, 1.45 mmol) in NMP (2.0 ml). The reactants were stirred at 130°C for 48 hours. After the end of the reaction, the reaction mixture was concentrated under reduced pressure. The residues were purified by MeOH/DCM (0-20%) column chromatography. Subjective compound (310 mg) was obtained. MS (ESI, m/z): [M+H]⁺ = 609.6

### Step 2) Preparation of 3-(8-((21-(4-(4-(2-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)-21-oxo-3,6,9,12,15,18-hexaoxahenicosyl)amino)-1-oxopthalazine-2(1H)-yl)piperidine-2,6-dione (ICT-0000968)

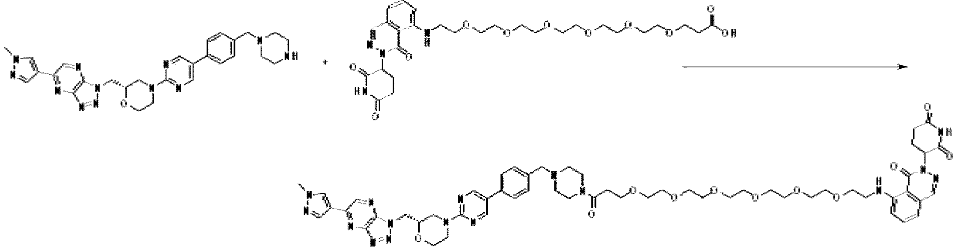

DMF (2.0ml) in (2S)-2-{[5-(1-methyl-1H-pyrazole-4-yl)-1H-[1,[1,2,3]Triazolo[4,5-b]pyrazine-1-yl]methyl}-4-(5-{4-[(piperazine-1-yl)methyl]phenyl}pyrimidine-2-yl)morpholine (31.8 mg, 0.05 mmol) and 1-((3-(2,6-dioxopiperidin-3-yl)4-oxo-3,4-dihydrophthalazine-5-yl)amino)3,6,9,12,15,18-hexaoxaheniconic acid-21-ohic acid (35.0 mg, 0.05 mmol) HATU (43.7 mg, 0.10 mmol) was added, followed by ethylbis (propane-2-yl)amine (5 equivalent). The reactants were stirred at room temperature for 6 hours. The reactants were poured into the water. It was extracted with ethyl acetate, dried with Magnesium sulfate, and concentrated under reduced pressure. The residues were purified by MeOH/DCM (0-20%) column chromatography to obtain the title compound (25 mg). MS (ESI, m/z): [M+H]⁺ = 1144.1.
1H NMR (400 MHz, DMSO-d6) δppm 10.97 (s, 1 H) 9.14 (s, 1 H) 8.64 - 8.70 (m, 2 H) 8.57 (s, 1 H) 8.24 (s, 1 H) 8.13 - 8.17 (m, 1 H) 7.52 - 7.60 (m, 3 H) 7.31 (d, J=8.09 Hz, 2 H) 6.81 - 6.88 (m, 2 H) 5.62 (br. s., 1 H) 4.79 - 4.92 (m, 2 H) 4.66 (d, J=13.43 Hz, 2 H) 4.31 (d, J=12.36 Hz, 1 H) 4.15 (br. s., 2 H) 3.81 - 3.90 (m, 4 H) 3.59 (t, J=5.19 Hz, 2 H) 3.32 - 3.55 (m, 8 H) 2.99 - 3.09 (m, 2 H) 2.77 - 2.88 (m, 2 H) 2.57 (br. s., 1 H) 2.46 - 2.55 (m, 6 H) 2.30 (br. s., 2 H) 2.24 (br. s., 1 H) 1.81 - 1.93 (m, 5 H) 1.68 (br. s., 2 H) 1.38 (br. s., 3 H) 1.17 (s, 7 H)

### Example B-15: Preparation of 3-(8-((4-(4-(4-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)4-oxobutyl)amino)-1-oxopthalazine-2(1H)-yl)piperidine-2,6-dione (ICT-0000969)

### Step 1) Preparation of 4-((3-(2,6-dioxopiperidin-3-yl)-4-oxo-3,4-dihydroptalazine-5-yl)amino)butanoic acid

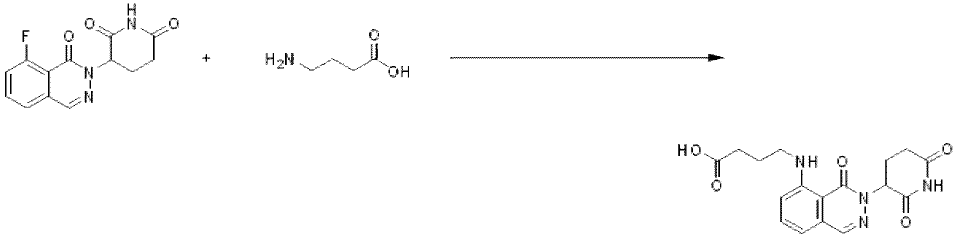

Ethylbis (propane-2-yl)amine (4.0 equivalent) was added to a solution of 3-(8-fluoro-1-oxopthalazine-2(1H)-yl)piperidine-2,6-dione 3-(8-fluoro-1-oxopthalazine-2(1H)-day) and 4-aminobutanoic acid (127 mg, 1.45 mmol) in NMP (2.0 ml). Ethyl bis(propane-2-yl)amine (4.0 equivalent) was added to a solution of 3-(8-fluoro-1-oxopthalazine-2(1H)-day) piperidine-2,6-dione 3-(8-fluoro-1-oxopthalazine-2(1H)-day. Subjective compound (310 mg) was obtained. MS (ESI, m/z): [M+H]⁺ = 359.6

### Step 2) Preparation of 3-(8-((4-(4-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)4-oxobutyl)amino)1-oxopthalazine-2(1H)-yl)piperidine-2,6-dione (ICT-0000969)

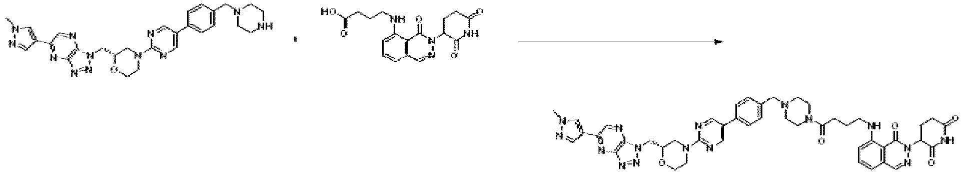

DMF (2. 0 ml) in (2S)-2-{[5-(1-methyl-1H-pyrazole-4-yl)-1H-[1,2,3]triazolo[4,5-b]pyrazin-1-yl]methyl}-4-(5-{4-[(piperazine-1-yl)methyl]phenyl}pyrimidine-2-yl)morpholine (31.8 mg, 0.05 mmol) and 4-((3-(2,6-dioxopiperidine-3-yl-4-oxo-3,4-dihydroptalazin-5-yl)amino)butanoic acid (25.0 mg, 0.10 mmol), followed by ethylbis (propane-2-yl)amine (5 equivalent). It was extracted with ethyl acetate, dried with Magnesium sulfate, and concentrated under reduced pressure. The residues were purified by MeOH/DCM (0-20%) column chromatography to obtain the title compound (25 mg). MS (ESI, m/z): [M+H]⁺ = 893.9
1H NMR (400 MHz, DMSO-d6) δppm 10.97 (s, 1 H) 9.14 (s, 1 H) 8.64 - 8.70 (m, 2 H) 8.57 (s, 1 H) 8.24 (s, 1 H) 8.13 - 8.17 (m, 1 H) 7.52 - 7.60 (m, 3 H) 7.31 (d, J=8.09 Hz, 2 H) 6.81 - 6.88 (m, 2 H) 5.62 (br. s., 1 H) 4.79 - 4.92 (m, 2 H) 4.66 (d, J=13.43 Hz, 2 H) 4.31 (d, J=12.36 Hz, 1 H) 4.15 (br. s., 2 H) 3.81 - 3.90 (m, 4 H) 3.59 (t, J=5.19 Hz, 2 H) 3.32 - 3.55 (m, 8 H) 2.99 - 3.09 (m, 2 H) 2.77 - 2.88 (m, 2 H) 2.57 (br. s., 1 H) 2.46 - 2.55 (m, 6 H) 2.30 (br. s., 2 H) 2.24 (br. s., 1 H) 1.81 - 1.93 (m, 5 H) 1.68 (br. s., 2 H) 1.38 (br. s., 3 H) 1.17 (s, 7 H)

### Example B-16: Preparation of 3-(8-((6-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)6-oxohexyl)amino)1-oxopthalazine-2(1H)-yl)piperidine-2,6-dione (ICT-0000970)

### Step 1) Preparation of 6-((3-(2,6-dioxopiperidine-3-yl)-4-oxo-3,4-dihydroptalazine-5-yl)amino)hexanoic acid

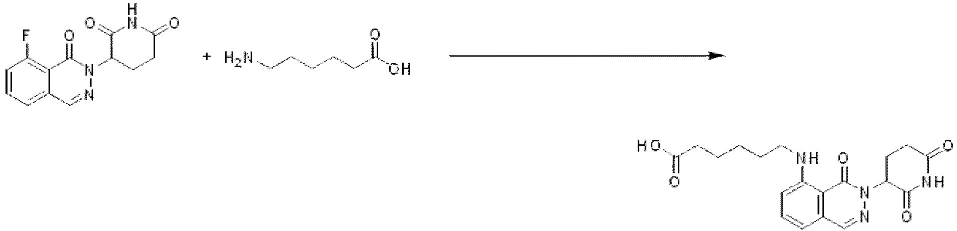

Ethylbis (propane-2-yl)amine (4.0 equivalent) was added to a solution of 3-(8-fluoro-1-oxopthalazine-2(1H)-yl)piperidine-2,6-dione (8-fluoro-1-oxopthalazine-2(1H)-day) and 6-aminohexanoic acid (153 mg, 1.45 mmol) in NMP (2.0 ml). Ethylbis (propane-2-yl)amine (4.0 equivalent) was added to a solution of 3-(8-fluoro-1-oxopthalazine-2(1H)-day) piperidin-2,6-dione 3-(8-fluoro-1-oxopthalazine-2(1H)-day. Subjective compound (310 mg) was obtained. MS (ESI, m/z): [M+H]⁺ = 387.6

### Step 2) Preparation of 3-(8-((6-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)6-oxohexyl)amino)1-oxopthalazine-2(1H)-yl)piperidine-2,6-dione (ICT-0000970)

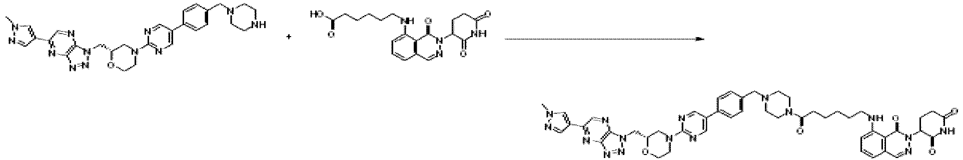

DMF (2.0ml) of (2S)-2-{[5-(1-methyl-1H-pyrazol-4-yl)-1H-[1,[1,2,3]triazolo[4,5-b]pyrazine-1-yl]methyl}-4-(5-{4-[(piperazine-1-yl)methyl]phenyl}pyrimidine-2-yl)morpholine (31.8 mg, 0.05 mmol) and 6-((3-(2,6-dioxopiperidine-3-yl)4-oxo-3,4-dihydroptalazin-5-yl)amino)hexanoic acid (29.0 mg, 0.05 mmol) solution with HATU (43.7 mg, 0.10 mmol), followed by ethylbis (propane-2-yl)amine (5 equivalent). The reactants were stirred at room temperature for 6 hours. The reactants were poured into water. It was extracted with ethyl acetate, dried with Magnesium sulfate, and concentrated under reduced pressure. The residues were purified by MeOH/DCM (0-20%) column chromatography to obtain the title compound (25 mg). MS (ESI, m/z): [M+H]⁺ = 922.3.
1H NMR (400 MHz, DMSO-d6) δppm 10.97 (s, 1 H) 9.14 (s, 1 H) 8.64 - 8.70 (m, 2 H) 8.57 (s, 1 H) 8.24 (s, 1 H) 8.13 - 8.17 (m, 1 H) 7.52 - 7.60 (m, 3 H) 7.31 (d, J=8.09 Hz, 2 H) 6.81 - 6.88 (m, 2 H) 5.62 (br. s., 1 H) 4.79 - 4.92 (m, 2 H) 4.66 (d, J=13.43 Hz, 2 H) 4.31 (d, J=12.36 Hz, 1 H) 4.15 (br. s., 2 H) 3.81 - 3.90 (m, 4 H) 3.59 (t, J=5.19 Hz, 2 H) 3.32 - 3.55 (m, 8 H) 2.99 - 3.09 (m, 2 H) 2.77 - 2.88 (m, 2 H) 2.57 (br. s., 1 H) 2.46 - 2.55 (m, 10 H) 2.30 (br. s., 2 H) 2.24 (br. s., 1 H) 1.81 - 1.93 (m, 5 H) 1.68 (br. s., 2 H) 1.38 (br. s., 3 H) 1.17 (s, 7 H)

### Example B-17: Preparation of 3-(6-(4-((1-(4-(1-(2-((S)-2-((5-(1-methyl-1H-pyrazole-4-yl)-1H-[1,2,3]triazolo[4,5-b]pyrazine-1-yl)methyl)morpholino)pyrimidine-5-il)benzyl)piperidine-4-il)methyl)piperazine-1-yl)piperazine-1-yl)1-oxopthalazine-2(1H)-yl)piperidine-2,6-dione (ICT-0000978)

### Step 1) Preparation of tert-butyl 4-((4-(2-(2,6-dioxopiperidin-3-yl)-1-oxo-1,2-dihydroptalazine-6-yl)piperazine-1-yl)methyl)piperidine-1-carboxylate

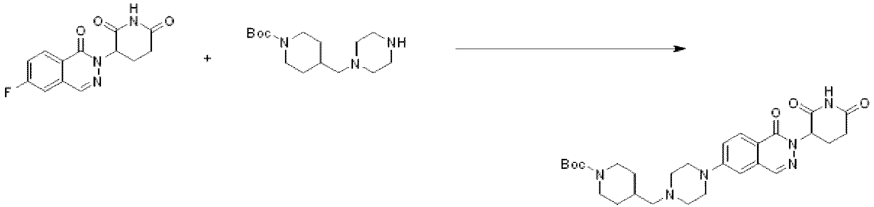

Ethylbis (propane-2-yl)amine (4.0 equivalent) was added to 3-(6-fluoro-1-oxopthalazine-2(1H)-yl) piperidine-2,6-dione (300 mg, 1.09 mmol) and tert-butyl 4-(piperazine-1-monomethyl)piperidine-1-carboxylate (600 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the heading compound (400 mg). MS (ESI, m/z): [M+H]⁺ = 539.4

### Step 2) Preparation of 3-(1-oxo-6-(4-(piperidine-4-ylmethyl)piperazine-1-yl)phthalazine-2(1H)-yl)piperidine-2,6-dione

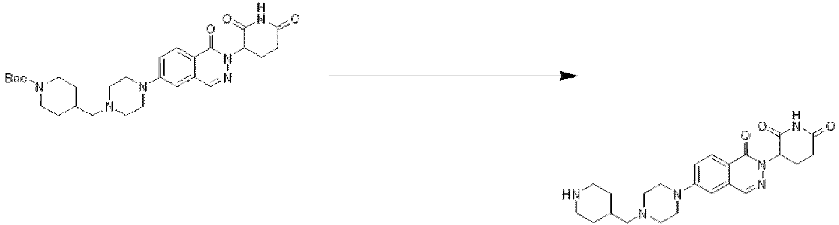

TFA (10 ml) was added to tert-butyl 4-((4-(2-(2,6-dioxopiperidine-3-yl)-1-oxo-1,2-dihydroptalazine-6-yl)piperazine-1-yl)methyl)piperidine-1-carboxylate (400 mg, 1.52 mmol) solution in DCM (30 ml). The reactants were stirred at room temperature for 1 hour. After the reaction was finished, the reaction mixture was concentrated under reduced pressure. The residues were purified by MeOH/DCM (0-20%) column chromatography to obtain the heading compound (400 mg). MS (ESI, m/z): [M+H]⁺ = 439.4

### Step 3) Preparation of 3-(6-(4-((1-(4-(2-((S)-2-((5-(1-methyl-1H-pyrazole-4-yl)-1H-[1,2,3]triazolo[4,5-b]pyrazine-1-yl)methyl)morpholino)pyrimidine-5-il)benzyl)piperidine-4-il)methyl)piperazine-1-yl)1-oxopthalazine-2(1H)-yl)piperidine-2,6-dione (ICT-0000978)

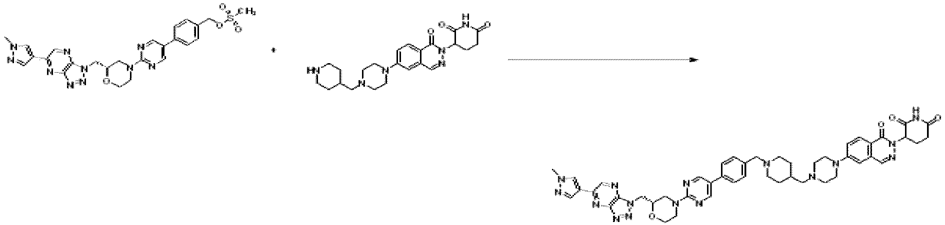

Potassium carbonate (6 mg, 0.02 mmol) was added to (S)-4-(2-(2-((5-(1-(1-methyl-1H-pyrazole-4-yl)-1H-[1,2,3]triazolo[4,5-b]pyrazine-1-yl)methyl) morpholino) pyrimidine-5-yl) benzyl methanesulfonate (12 mg, 0.02 mmol) and 3-(1-oxo-6-(4-(piperidine-4-yl)piperazine-1-yl)phthalazine-2(1H)-yl)piperidine-2,6-dione (9.5 mg, 0.02 mmol) in DMF (2.0 ml). The reactants were stirred at 60°C for 2 hours. The reactants were poured into water. It was extracted with ethyl acetate, dried with Magnesium sulfate, and concentrated under reduced pressure. The residue was purified by MeOH/DCM (0-20%) column chromatography to obtain the title compound (5 mg). MS (ESI, m/z): [M+H]⁺ = 906.8.
1H NMR (400 MHz, DMSO-d6) δ ppm 10.98 (s, 1 H) 9.21 (s, 1 H) 8.74 (s, 2 H) 8.64 (s, 1 H) 8.31 (s, 1 H) 8.18 (s, 1 H) 7.91 (d, J=8.93 Hz, 1 H) 7.61 (m, J=8.19 Hz, 2 H) 7.38 (m, J=8.19 Hz, 2 H) 7.04 - 7.11 (m, 1 H) 6.87 - 6.93 (m, 1 H) 5.74 (dd, J=11.55, 7.15 Hz, 1 H) 4.85 - 5.00 (m, 2 H) 4.73 (d, J=11.98 Hz, 1 H) 4.38 (d, J=12.72 Hz, 1 H) 4.22 (br. s., 1 H) 3.87 - 3.98 (m, 4 H) 3.50 (s, 2 H) 3.44 (br. s., 4 H) 3.05 - 3.19 (m, 2 H) 2.85 - 2.97 (m, 2 H) 2.59 (d, J=19.93 Hz, 1 H) 2.35 (br. s., 4 H) 2.30 (br. s., 4 H) 2.07 (dd, J=11.92, 4.22 Hz, 1 H) 1.89 - 2.01 (m, 2 H) 1.61 (br. s., 2 H) 1.37 (t, J=5.93 Hz, 2 H)

### Example B-18: Preparation of 3-(4-methyl-8-((2-(4-(4-(4-(2-((S)-2-((5-(1H-pyrazol-4-yl)-1H-[1,2,3]triazolo[4,5-b]pyrazine-1-yl)methyl)morpho)pyrimidine-5-yl)benzyl)piperazine-1-yl)2-oxoethyl)amino)1-oxophthalazine-2(1H)-yl)piperidine-2,6-dione (ICT-0000983) synthesis

### Step 1) Preparation of methyl 2-acetyl-6-fluorobenzoate

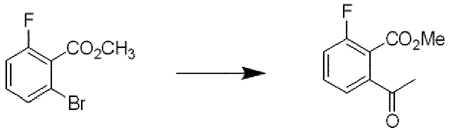

Tetrakis (triphenylphosphine)-palladium (0) (557 mg, 0.48 mmol) was added to a solution of methyl 2-acetyl-6-fluorobenzoate (1.12 g, 4.81 mmol) and tributyl (1-ethoxyvinyl) tin (1.91 g, 5.29 mmol) in toluene (20 ml) and stirred at 100°C for 16 hours. After cooling, 5 ml 1N-HCl was added and stirred for 1 hour. The organic layer was dried with Magnesium sulfate and concentrated under reduced pressure. The residues were purified by silica column chromatography, and the title compound (820 mg) was obtained MS (ESI, m/z): [M+H]⁺ = 196.8

### Step 2) Preparation of 2-Acetyl-6-Fluorobenzoic Acid

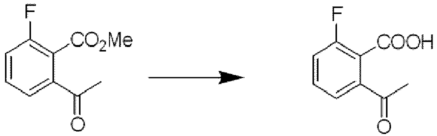

LiOH (494 mg, 20.6 mmol) was added to a solution of methyl 2-acetyl-6-fluorobenzoate (810 mg, 4.13 mmol) in THF (20 ml) and water (10 ml), and stirred at room temperature for 20 hours. The solution was acidified with 1N-HCl until the pH was about 3. 100 ml EA was added, the organic layer was dried with Magnesium sulfate, and concentrated under reduced pressure. MS (ESI, m/z): [M+H]⁺ = 183.8

### Step 3) Preparation of 8-Fluoro-4-methylphthalazine-1(2H)-one

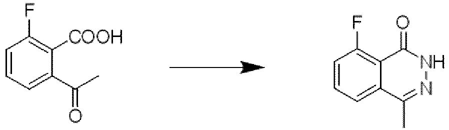

Hydrazine monohydrate (261 mg, 5.20 mmol) was added to a 2-acetyl-6-fluorobenzoic acid (790 mg, 4.34 mmol) solution in methanol (23 mL) and stirred at room temperature for 16 hours. MS (ESI, m/z): [M+H]⁺ = 179.1

### Step 4) Preparation of 3-(8-fluoro-4-methyl-1-oxopthalazine-2(1H)-yl)piperidine-2,6-dione

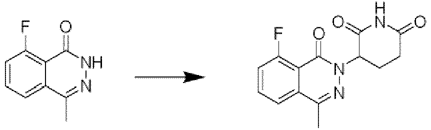

t-BuONa (17.9 mg, 0.185 mmol) was added to 8-fluoro-4-methylphthalazine-1(2H)-one (30 mg, 0.168 mmol) solution in DMF (1 mL) at 0°C at 0°C, and stirred at 0°C for 20 minutes. 3-bromoperidin-2,6-dione (32.3 mg, 0.168 mmol) was added to the solution and stirred at room temperature for 1 hour. The title compound (2 mg) was obtained as a solid. MS (ESI, m/z): [M+H]⁺ = 289.8

1H NMR (400 MHz, DMSO-d6) δ11.05 (s, 1H), 8.04-7.94 (m, 1H), 7.79 (d, J = 7.9 Hz, 1H), 7.69 (dd, J = 7.9, 11.3 Hz, 1H), 5.71 (br dd, J = 4.9, 12.1 Hz, 1H), 3.0 - 2.83 (m, 1H), 2.64 - 2.56 (m, 2H), 2.55 (s, 3H), 2.17 - 2.05 (m, 1H).

### Step 5) Preparation of (3-(2,6-dioxopiperidin-3-yl)-1-methyl-4-oxo-3,4-dihydroptalazin-5-yl)glycine synthesis

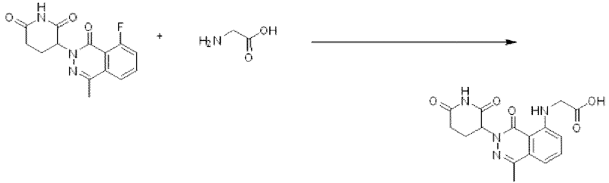

Ethylbis (propane-2-yl)amine (4.0 equivalent) was added to 3-(8-fluoro-4-methyl-1-oxopthalazine-2(1H)-yl) and glycine (125 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the title compound (250 mg). MS (ESI, m/z): [M+H]⁺ = 345.4

### Step 6) Preparation of 3-(4-methyl-8-((2-(4-(4-(2-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)2-oxoethyl)amino)1-oxopthalazine-2(1H)-yl)piperidine-2,6-dione (ICT-0000983)

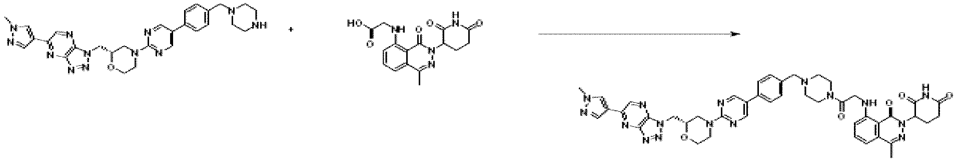

HATU (41.3 mg, 0.11 mmol) was added to (S)-2-((5-(1-(1-methyl-1H-pyrazole-4-yl)-1H-[1,2,3]triazolo[4,5-b]pyrazine-1-yl)methyl)methyl)4-(5-(4-(piperazine-1-yl)phenyl)pyrimidine-2-yl)morpholine (30 mg, 0.05 mmol) and (3-(2,6-dioxopiperidine-3-yl)1-methyl-4-oxo-3,4-dihydroptalazin-5-yl)glycine (20.6 mg, 0.06 mmol) in DMF (2.0 ml), followed by ethylbis (propane-2-yl)amine (5 equivalent). The reactants were stirred at room temperature for 6 hours. The reactants were poured into the water. It was extracted with ethyl acetate, dried with Magnesium sulfate, and concentrated under reduced pressure. The residues were purified by MeOH/DCM (0-20%) column chromatography to obtain the title compound (17 mg). MS (ESI, m/z): [M+H]⁺ = 879.8
1H NMR (400 MHz, DMSO-d6) δppm 10.94 (s, 1 H) 9.36 (br. s., 1 H) 1 9.14 (s, 1 H) 8.73 (br. s., 2 H) 8.58 (s, 1 H) 8.24 (s, 1 H) 7.65 - 7.77 (m, 2 H) 7.62 (t, J=7.78 Hz, 1 H) 7.42 - 7.56 (m, 2 H) 6.78 - 6.93 (m, 1 H) 4.80 - 4.92 (m, 2 H) 4.67 (d, J=12.51 Hz, 1 H) 4.33 (d, J=12.97 Hz, 2 H) 4.15 (br. s., 1 H) 3.82 - 3.90 (m, 4 H) 3.55 (dq, J=10.59, 6.54 Hz, 2 H) 3.43 (t, J=10.45 Hz, 2 H) 3.00 - 3.12 (m, 4 H) 2.78 - 2.89 (m, 1 H) 2.47 - 2.59 (m, 3 H) 2.01 (d, J=5.04 Hz, 1 H) 1.89 (d, J=17.09 Hz, 2 H) 1.38 (d, J=6.87 Hz, 2 H) 1.09 - 1.23 (m, 6 H)

### Example B-19: Preparation of 3-(6-(4-(2-(4-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)-2-oxoethyl)piperazine-1-day)-1-oxopthalazine-2(1H)-yl)piperidine-2,6-dione (ICT-0001109)

### Step 1) Preparation of tert-butyl 4-(2-(2,6-dioxopiperidine-3-yl)-1-oxo-1,2-dihydroptalazine-6-yl)piperazine-1-carboxylate

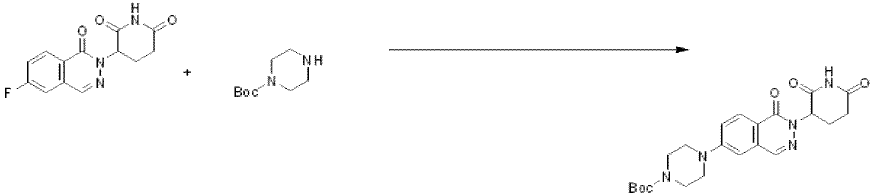

Ethylbis (propane-2-yl)amine (4.0 equivalent) was added to 3-(6-fluoro-1-oxopthalazine-2(1H)-yl)piperidine-2,6-dione (300 mg, 1.04 mmol) and tert-butyl piperazine-1-carboxylate (386 mg, 2.07 mmol) solutions in NMP (3.0 ml). The reactants were stirred at 130°C for 48 hours. After the reaction was completed, the reaction mixture was concentrated under reduced pressure. The residues were purified by MeOH/DCM (0-20%) column chromatography to obtain the heading compound (350 mg). MS (ESI, m/z): [M+H]⁺ = 442.4

### Step 2) Preparation of 3-(1-oxo-6-(piperazine-1-yl)phthalazine-2(1H)-yl)piperidine-2,6-dione

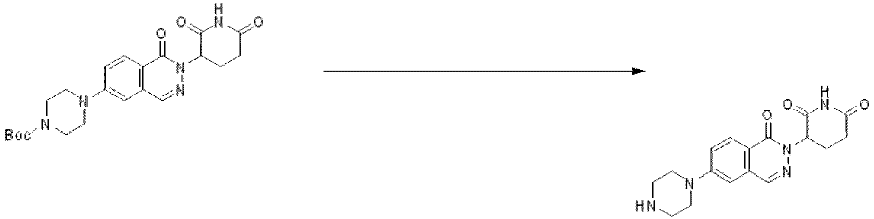

TFA (10 ml) was added to tert-butyl 4-(2-(2,6-dioxopiperidine-3-yl)-1-oxo-1,2-dihydroptalazine-6-yl) solution in DCM (30 ml). The reactants were stirred at room temperature for 1 hour. After the reaction was completed, the reaction mixture was concentrated under reduced pressure. The residues were purified by MeOH/DCM (0-20%) column chromatography to obtain the heading compound (200 mg). MS (ESI, m/z): [M+H]⁺ = 342.4

### Step 3) Preparation of tert-butyl 2-(4-(2-(2,6-dioxopiperidin-3-yl)-1-oxo-1,2-dihydroptalazine-6-yl)piperazine-1-yl)acetate

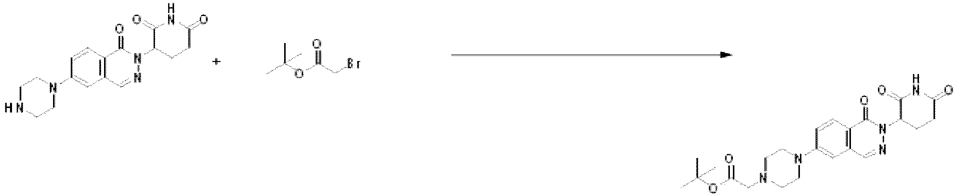

Ethylbis (propane-2-yl)amine (2.0 equivalent) was added to 3-(1-oxo-6-(piperazine-1-yl)phthalazine-2(1H)-yl)piperidine-2,6-dione (200 mg, 0.56 mmol) and tert-butyl 2-bromoacetate (121 mg, 0.62 mmol) solutions in ACN (10.0 ml). The reactants were stirred at room temperature for 5 hours. After the reaction was completed, the reaction mixture was concentrated under reduced pressure. The residues were purified by MeOH/DCM (0-20%) column chromatography to obtain the title compound (210 mg). MS (ESI, m/z): [M+H]⁺ = 456.4

### Step 4) Preparation of 2-(4-(2-(2,6-dioxopiperidin-3-yl)-1-oxo-1,2-dihydroptalazine-6-yl)piperazine-1-yl)acetic acid

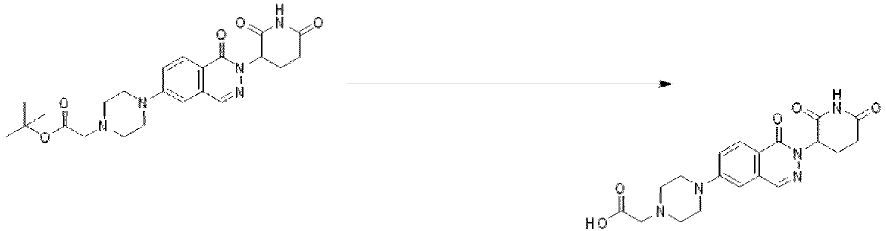

TFA (1 ml) was added to tert-butyl 2-(4-(2-(2,6-dioxopiperidine-3-yl)-1-oxo-1,2-dihydroptalazine-6-yl) acetate (30 mg, 0.06 mmol) in DCM (3 ml). The reactants were stirred at room temperature for 1 hour. After the reaction was completed, the reaction mixture was concentrated under reduced pressure. The residues were purified by MeOH/DCM (0-20%) column chromatography to obtain the heading compound (15 mg). MS (ESI, m/z): [M+H]⁺ = 400.4

### Step 5) Preparation of 3-(6-(4-(2-(4-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)-2-oxoethyl)piperazine-1-yl)-1-oxopthalazine-2(1H)-yl)piperidine-2,6-dione (ICT-0001109)

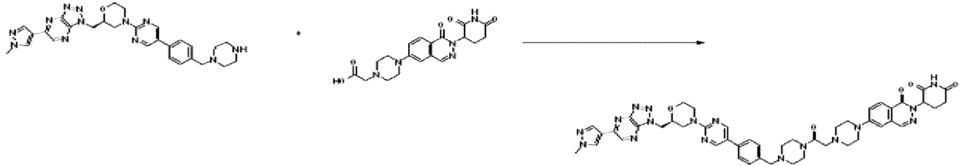

DMF (2. HATU (20.6 mg, 0.06 mmol) was added to the (S)-2-((5-(1-(1-methyl-1H-pyrazole-4-yl)-1H-[1,2,3]triazolo[4,5-b]pyrazine-1-yl)methyl)methyl)4-(5-(4-(piperazine-1-yl)phenyl-pyrimidine-2-yl)morpholine (15 mg, 0.03 mmol) and 2-(4-(2-(2-(2,6-dioxopiperidine-3-yl)1-oxo-1,2-dihydroptalazine-6-yl)piperazine-1-yl)piperazine-1-yl) acetic acid (12.3 mg, 0.06 mmol), followed by ethylbis (propane-2-yl)amine (5 equivalent). The reactants were stirred at room temperature for 6 hours. The reactants were poured into the water. It was extracted with ethyl acetate, dried with Magnesium sulfate, and concentrated under reduced pressure. The residues were purified by MeOH/DCM (0-20%) column chromatography to obtain the title compound (8 mg). MS (ESI, m/z): [M+H]⁺ = 935.1.
1H NMR (400 MHz, DMSO-d6) δ ppm 10.98 (s, 1 H) 9.21 (s, 1 H) 8.74 (s, 2 H) 8.64 (s, 1 H) 8.31 (s, 1 H) 8.18 (s, 1 H) 7.91 (d, J=8.93 Hz, 1 H) 7.61 (m, J=8.19 Hz, 2 H) 7.38 (m, J=8.19 Hz, 2 H) 7.04 - 7.11 (m, 1 H) 6.87 - 6.93 (m, 1 H) 6.76 (d, J=2.08 Hz, 1 H) 4.85 - 5.00 (m, 2 H) 4.73 (d, J=11.98 Hz, 1 H) 4.38 (d, J=12.72 Hz, 1 H) 4.22 (br. s., 1 H) 3.87 - 3.98 (m, 4 H) 3.50 (s, 2 H) 3.44 (br. s., 4 H) 3.05 - 3.19 (m, 2 H) 2.85 - 2.97 (m, 2 H) 2.59 (d, J=19.93 Hz, 2 H) 2.35 (br. s., 4 H) 2.30 (br. s., 4 H) 2.07 (dd, J=11.92, 4.22 Hz, 1 H) 1.89 - 2.01 (m, 2 H) 1.61 (br. s., 2 H) 1.37 (t, J=5.93 Hz, 2 H)

### Example B-20: Preparation of 3-((3-fluoro-4-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)phenyl)amino)piperidine-2,6-dione (ICT-0001403)

### Step 1) Preparation of (S)-4-(5-(4-((4-(2-(2-fluoro-4-nitrophenyl)piperazine-1-yl)methyl)phenyl)pyrimidine-2-yl)-2-((5-(1-methyl-1H-pyrazol-4-yl)-1H-[1,2,3]triazolo[4,5-b]pyrazine-1-yl)methyl)morpholine

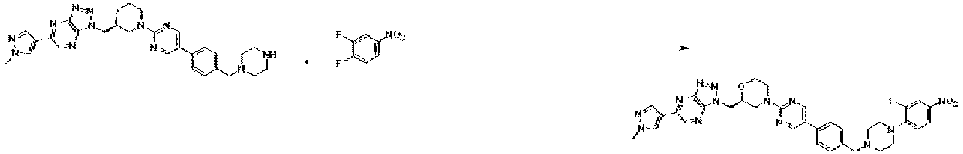

Ethylbis (propane-2-yl)amine (35.1 mg, 0.27 mmol) was added to (S)-2-((5-(1-(1-methyl-1H-pyrazole-4-yl)-1H-[1,2,3]triazolo[4,5-b]pyrazine-1-yl)methyl)methyl)-4-(5-(4-(piperazine-1-yl)phenyl-2-yl)pyrimidin-2-yl)morpholine (87.5 mg, 0.18 mmol) and 2,4-difluoro-1-nitrobenzene (57.6 mg, 0.36 mmol) in DMF (2 ml). The reactants were stirred at 120°C for 2 hours. After cooling to room temperature, the reactants were poured into water and extracted with ethyl acetate. The organic layer was dried with Magnesium sulfate, concentrated under reduced pressure, and purified by column chromatography to obtain the title compound (95 mg). MS (ESI, m/z): [M+H]⁺ = 692.6.

### Step 2) Preparation of (S)-3-fluoro-4-(4-(4-(2-(2-((5-(1-methyl-1H-pyrazole-4-yl)-1H-[1,2,3]triazolo[4,5-b]pyrazine-1-yl)methyl)morpholino)pyrimidine-5-il)benzyl)piperazine-1-yl)aniline

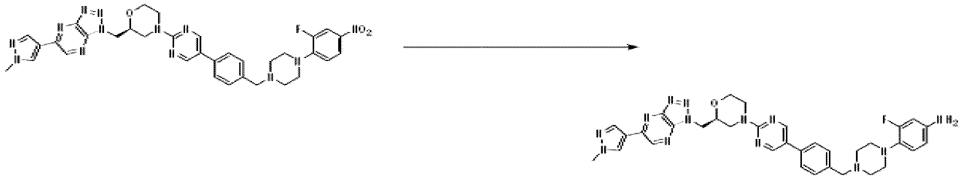

2 mL saturated NH₄Cl was added to (S)-4-(5-(4-((4-(4-(2-(2-fluoro-4-nitrophenyl)piperazine-1-yl)methyl)phenyl)pyrimidine-2-yl)-2-((5-(1-(1-methyl-1H-pyrazole-4-yl)-1H-[1,2,3]triazolo[4,5-b]pyrazine-1-yl)methyl)morpholine (85 mg, 0.12 mmol) and iron (34.3 mg, 0.62 µmol) in a solution. The reactants were stirred at 100°C for 2 hours. After cooling to room temperature, the reactants were filtered, the filtrate was poured into water. Ethyl acetate. The organic layer was dried with Magnesium sulfate, concentrated under reduced pressure, and purified by column chromatography to obtain the title compound (69 mg). MS (ESI, m/z): [M+H]⁺ = 662.6.

### Step 3) Preparation of 3-((3-fluoro-4-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)phenyl)amino)piperidine-2,6-dione (ICT-0001403)

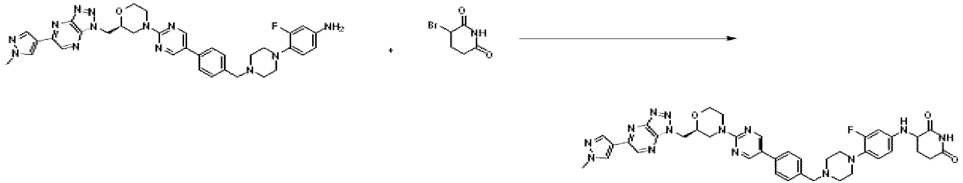

Sodium bicarbonate (27.9 mg, 0.33 mmol) was added to 3-fluoro-4-{(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-yl}phenyl)methyl]piperazine-1-yl}aniline (120 mg, 0.18 mmol) and 3-bromomorphidine-2,6-dione (69.6 mg, 0.36 mmol) solution. Reactants were stirred at 60°C for 12 hours. The reactants were filtered, the filtrate was poured into water and extracted with ethyl acetate. The organic layer was dried with Magnesium sulfate, concentrated under reduced pressure, and purified by column chromatography to obtain the title compound (121 mg). MS (ESI, m/z): [M+H]⁺ = 773.9.
1H NMR (400 MHz, DMSO-d6) δppm 10.71 (s, 1 H) 9.14 (s, 1 H) 8.68 (s, 2 H) 8.58 (s, 1 H) 8.25 (s, 1 H) 7.55 (m, J=8.09 Hz, 2 H) 7.33 (m, J=8.09 Hz, 2 H) 6.76 (t, J=9.31 Hz, 1 H) 6.43 (dd, J=14.95, 2.29 Hz, 1 H) 6.35 (d, J=8.54 Hz, 1 H) 5.74 (d, J=7.78 Hz, 1 H) 4.79 - 4.93 (m, 2 H) 4.66 (d, J=12.36 Hz, 1 H) 4.31 (d, J=12.97 Hz, 1 H) 4.09 - 4.25 (m, 1 H) 3.79 - 3.89 (m, 4 H) 3.37 - 3.55 (m, 3 H) 3.00 - 3.10 (m, 2 H) 2.80 (br. s., 2 H) 2.61 - 2.71 (m, 1 H) 2.51 (d, J=4.43 Hz, 1 H) 2.47 (s, 2 H) 1.97 - 2.05 (m, 1 H) 1.77 (dd, J=12.13, 4.50 Hz, 1 H)

### Example B-21: Preparation of 3-(((2-fluoro-4-(4-(2-(2-(2-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-il)acetyl)piperazine-1-il)phenyl)amino)piperidine-2,6-dione (ICT-0001439)

### Step 1) Preparation of tert-butyl(S)-2-(4-(4-(2-(2-(2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)acetate

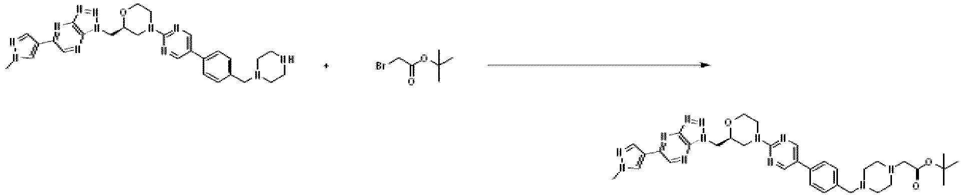

tert-butyl 2-bromoacetate (150 mg, 0.62 mmol), ethylbis (propane-2-yl)amine (2.0 equivalent) were added to (S)-2-((5-(1-(1-methyl-1H-pyrazole-4-yl)-1H-[1,2,3]triazolo[4,5-b]pyrazine-1-yl)methyl)methyl)4-(5-(4-(piperazine-1-yl)phenyl-pyrimidin-2-yl)morpholine (300 mg, 0.62 mmol) in ACN (10.0 ml). tert-butyl 2-bromoacetate (150 mg, 0.62 mmol) and ethylbis(propane-2-yl)amine (2.0 equivalent) were added to the solution of (S)-2-(5-(1-(1-methyl-1-yl)phenyl-pyrizine-2-yl)morpholine (300 mg, 0.62 mmol). Subjective compound (210 mg) was obtained. MS (ESI, m/z): [M+H]⁺ = 667.7

### Step 2) Preparation of (S)-2-(4-(4-(2-(2-((5-(1-(1-methyl-1H-pyrazole-4-yl)-1H-[1,2,3]triazolo[4,5-b]pyrazin-1-yl)methyl)morpholino)pyrimidine-5-yl)benzyl)piperazine-1-il)acetic acid

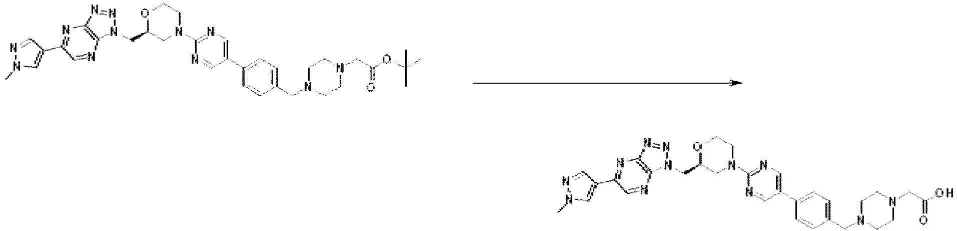

TFA (1 ml) was added to tert-butyl(S)-2-(4-(4-(2-(2-((5-(1-(1H-pyrazole-4-yl)-1H-[1,2,3]triazolo[4,5-b]pyrazine-1-yl)methyl)morpholino)pyrimidine-5-yl)benzyl)piperazine-1-yl)acetate (200 mg, 0.45 mmol) in a solution. The reactants were stirred at room temperature for 1 hour. After the reaction was completed, the reaction mixture was concentrated under reduced pressure. The residues were purified by MeOH/DCM (0-20%) column chromatography. Subjective compound (150 mg) was obtained. MS (ESI, m/z): [M+H]⁺ = 611.4

### Step 3) Preparation of 3-((3-fluoro-4-(4-(2-(4-(4-(2-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-il)acetyl)piperazine-1-il)phenyl)amino)piperidine-2,6-dione (ICT-0001439)

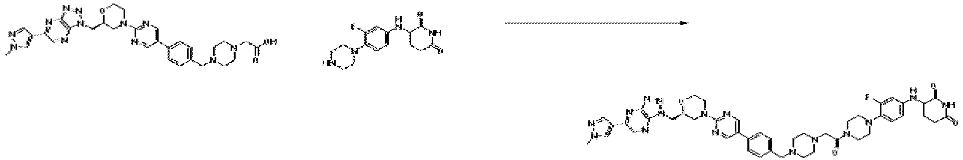

HATU (74.4 mg, 0.16 mmol) is added to the (S)-2-(4-(2-(2-((5-(5-(1-methyl-1H-pyrazole-4-yl)-1H-[1,2,3]triazolo[4,5-b]pyrazin-1-yl)methyl)morpholino)pyrimidine-5-yl)benzyl)piperazine-1-yl)acetic acid (100 mg, 0.16 mmol) and 3 3-((3-fluoro-4-(piperazine-1-yl)phenyl)amino)piperidine-2,6-dione (50.2 mg, 0.16 mmol), followed by ethylbis (propane-2-yl)amine (31.7 mg, 0.22 mmol). The reactants were stirred for 6 hours. The reactants were poured into water, extracted with ethyl acetate, dried with Magnesium sulfate, and concentrated under reduced pressure. The residue was purified with MeOH/DCM (0-10%) MPLC to obtain the title compound (79 mg). MS (ESI, m/z): [M+H]⁺ = 900.1.
1H NMR (400 MHz, DMSO-d6) δ ppm 10.80 (s, 1 H) 9.21 (s, 1 H) 8.75 (s, 2 H) 8.65 (s, 1 H) 8.32 (s, 1 H) 7.60 (m, J=7.93 Hz, 2 H) 7.37 (m, J=8.09 Hz, 2 H) 6.78 - 6.88 (m, 1 H) 6.53 (dd, J=14.80, 2.29 Hz, 1 H) 6.44 (d, J=8.70 Hz, 1 H) 5.88 (d, J=7.78 Hz, 1 H) 4.86 - 4.99 (m, 2 H) 4.73 (d, J=12.21 Hz, 1 H) 4.38 (d, J=13.12 Hz, 1 H) 3.89 - 3.97 (m, 4 H) 3.66 (br. s., 1 H) 3.56 (br. s., 2 H) 3.45 - 3.54 (m, 3 H) 3.17 (br. s., 1 H) 3.04 - 3.14 (m, 2 H) 2.86 (br. s., 2 H) 2.78 (br. s., 2 H) 2.74 (s, 1 H) 2.59 (br. s., 1 H) 2.42 (br. s., 4 H) 2.37 (br. s., 2 H)

### Example B-22: Preparation of 3-((3-fluoro-4-(4-(2-(4-(4-(4-(2-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-il)acetyl)piperazine-1-il)phenyl)amino)piperidine-2,6-dione (ICT-0001379)

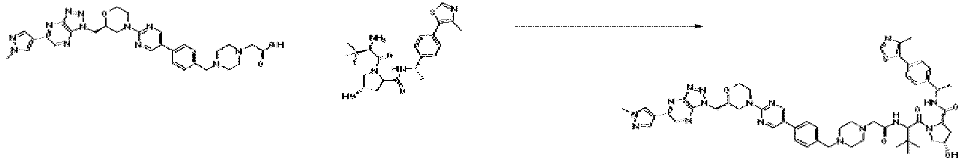

HATU (74.4 mg, 0.16 mmol) in (S)-2-(4-(2-(2-(2-((5-(1-(1-methyl-1H-pyrazole-4-yl)-1H-[1,2,3]triazolo[4,5-b]pyrazin-1-yl)methyl)morpholino)pyrimidine-5-yl)benzyl)piperazine-1-yl)acetic acid (100 mg, 0.16 mmol) and (2R,4S)-1-((R)-2-amino-3,3-dimethylbutanoyl)-4-hydroxy-N- ((S)-1-(4-(4-methylthiazole-5-yl)phenyl)ethyl)pyrrolidine-2-carboxamide (80.2 mg, 0.16 mmol) 0.2 mmol), followed by ethylbis (propane-2-yl)amine (31.7 mg, 0.22 mmol). The reactants were stirred for 6 hours. The reactants were poured into water, extracted with ethyl acetate, dried with Magnesium sulfate, and concentrated under reduced pressure. The residue was purified with MeOH/DCM (0-10%) MPLC to obtain the title compound (50 mg). MS (ESI, m/z): [M+H]⁺ = 1038.1.
1H NMR (400 MHz, DMSO-d6) δppm 9.14 (s, 1 H) 8.92 (s, 1 H) 8.65 - 8.74 (m, 2 H) 8.58 (s, 1 H)8.24 (s, 1 H) 7.54 (d, J=7.93 Hz, 2 H) 7.35 - 7.40 (m, 2 H) 7.22 - 7.35 (m, 4 H) 5.05 (d, J=3.20 Hz, 1 H) 4.79 - 4.94 (m, 3 H) 4.66 (d, J=12.66 Hz, 1 H) 4.40 - 4.49 (m, 1 H) 4.26 - 4.40 (m, 2 H) 4.21 (br. s., 1 H) 3.82 - 3.89 (m, 4 H) 3.37 -3.55 (m, 4 H) 2.95 - 3.09 (m, 2 H) 2.37 - 2.40 (m, 4 H) 1.91 - 2.05 (m, 1 H) 1.31 (d, J=7.02 Hz, 3 H) 0.80 - 0.94 (m, 10 H)

**[Table 1] In vitro degradation analysis and cell viability analysis of C-MET PROTAC compounds (see Experimental Examples 1 to 3 described below)**

| ICT-CODE | STRUCTURE | In vitro cell viability assay (nM) | | | | | | | |
|---|---|---|---|---|---|---|---|---|---|
| | | MKN-45 | | Hs746T | | SNU-638 | | EBC-1 | |
| | | DC₅₀ | IC₅₀ | DC₅₀ | IC₅₀ | DC₅₀ | IC₅₀ | DC₅₀ | IC₅₀ |
| ICT-0000922 | 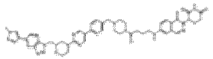 | A | A | - | - | - | A | - | - |
| ICT-0000923 | 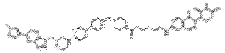 | A | A | - | - | - | A | - | - |
| ICT-0000924 | 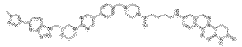 | A | A | - | - | - | A | - | - |
| ICT-0000925 | 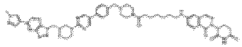 | A | A | - | | - | A | - | - |
| ICT-00009.35 | 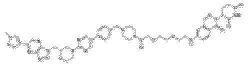 | B | B | - | - | - | - | - | - |
| ICT-0000938 | 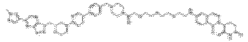 | C | B | - | - | - | - | - | - |
| ICT-0000939 | 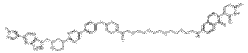 | C | B | - | - | - | - | - | - |
| ICT-0000940 | 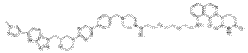 | B | B | - | - | - | - | - | - |
| ICT-0000942 | 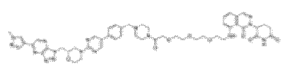 | - | A | - | - | - | | - | - |
| ICT-0000946 | 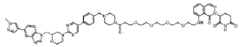 | - | B | - | - | - | - | - | - |
| ICT-0000959 | 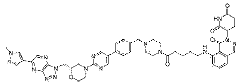 | A | A | - | - | - | - | - | - |
| ICT-0000960 | 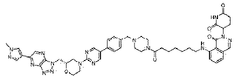 | B | A | - | - | - | - | - | - |
| ICT-0000967 | 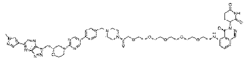 | - | B | - | - | - | - | - | - |
| ICT-0000968 | 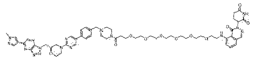 | - | B | - | - | - | - | - | - |
| ICT-0000969 | 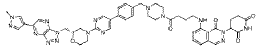 | A | A | - | - | - | - | - | - |
| ICT-0000970 | 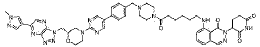 | - | A | - | - | - | - | - | - |
| ICT-0000978 | 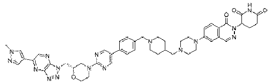 | B | A | - | - | - | - | - | - |
| ICT-0000983 | 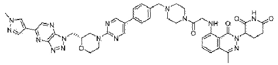 | A | A | - | - | - | - | - | - |
| ICT-0001109 | 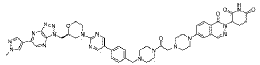 | A | A | - | - | - | - | - | - |
| ICT-0001403 | 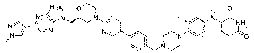 | - | - | - | - | - | A | - | A |
| ICT-0001439 | 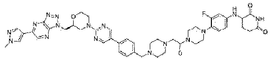 | - | - | - | - | A | A | A | A |
| ICT-0001379 | 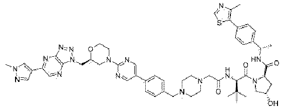 | - | - | - | - | A | A | A | A |

| | | | | | | | | | |
|---|---|---|---|---|---|---|---|---|---|
| A: IC₅₀ or DC₅₀ < 100 nM B: 100 nM < IC₅₀ or DC₅₀ < 1 uM C: 1 uM < IC₅₀ or DC₅₀ | | | | | | | | | |

### Example C: Preparation of Tepotinib-based cMET PROTAC

### Example C-1: Preparation of 3-(1-(3-(5-((1-(1-(1-(1-((1-((3-(2,6-dioxopiperidin-3-yl)-4-oxo-3,4-dihydroptalazine-5-yl)amino)-3,6,9,12-tetraoxapentadecan-15-oil)piperidine-4-yl)methoxy)pyrimidine-2-yl)benzyl)6-oxo-1,6-dihydropyridazine-3-yl)benzonitrile (ICT-0000907)

### Step 1) Preparation of tert-butyl 1-((3-(2,6-dioxopiperidin-3-yl)-4-oxo-3,4-dihydroptalazine-5-yl)amino)-3,6,9,12-tetraoxapentadecan-15-oate

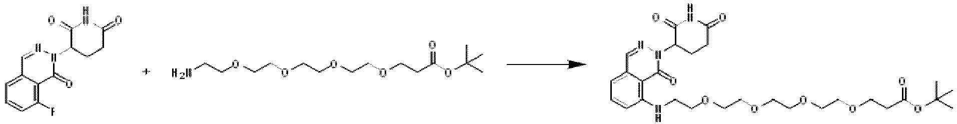

NMP (1. 5 mL) of 3-(8-fluoro-1-oxo-1,2-dihydroptallazine-2-yl)piperidine-2,6-dione (200 mg, 0.73 mmol), tert-butyl 1-amino-3,6,9,12-tetraoxapentadecane-15-oate (280 mg, 0.87 mmol), and DIPEA (0.38 ml, 2.18 mmol) solutions were stirred at 120°C for 20 hours. MS (ESI, m/z): [M+H]⁺= 577.2.

### Step 2) Preparation of 1-((3-(2,6-dioxopiperidin-3-yl)-4-oxo-3,4-dihydroptalazine-5-yl)amino)-3,6,9,12-tetraoxapentadecan-15-oic acid

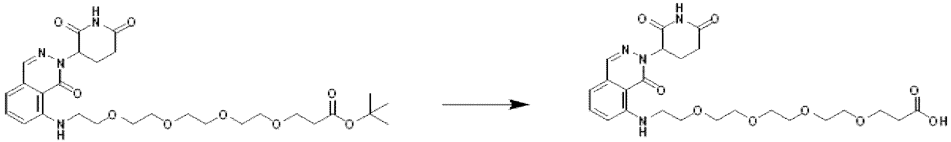

1 ml TFA solution was added to tert-butyl 1-((3-(2,6-dioxopiperidin-3-yl)-4-oxo-3,4-dihydroptalazine-5-yl)amino)-3,6,9,12-tetraoxapentadecan-15-oate (360 mg, 0.62 mmol) in DCM (5 mL) and stirred at room temperature for 2 hours. MS (ESI, m/z): [M+H]⁺= 521.2.

### Step 3) Preparation of 3-(1-(3-(5-((1-(1-(1-((3-(2,6-dioxopiperidine-3-yl)-4-oxo-3,4-dihydroptalazine-5-yl)amino)3,6,9,12-tetraoxapentadecan-15-oil)piperidine-4-yl)methoxy)pyrimidine-2-yl)benzyl)6-oxo-1,6-dihydropyridazine-3-yl)benzonitrile (ICT-0000907)

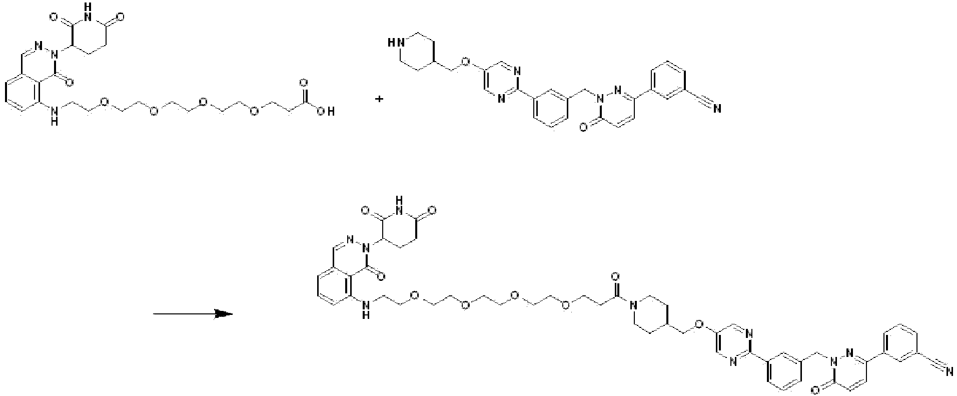

3-{6-oxo-1-[(3-{5-[(piperidine-4-yl)methoxy]pyrimidine-2-yl}phenyl)methyl]-1,in DMF (5 ml) at room temperature HATU (13.5 mmol, 0.06 mmol) was added to a solution of 6-dihydropyridazin-3-yl}benzonitrile (25 mg, 0.05 mmol) and 1-((3-(2,6-dioxopiperidine-3-yl)4-oxo-3,4-dihydroptalazine-5-yl)amino)-3,6,9,12-tetraoxapentadecan-15-osan (27.2 mmol, 0.05 mmol) solution, followed by ethylbis (propane-2-yl)amine (33.8 mg, 0.26 mmol). The reactant was stirred for 6 hours. It was extracted with ethyl acetate, dried with Magnesium sulfate, and concentrated under reduced pressure. The residue was purified with MeOH/DCM (0-20%) MPLC to obtain the title compound (51.3 mg). MS (ESI, m/z): [M+H]⁺= 982.1.
1H NMR (500 MHz, DMSO-d6) δ ppm 10.98 (s, 1 H) 8.63 - 8.68 (m, 2 H) 8.39 (s, 2 H) 8.15 - 8.28 (m, 2 H) 7.89 - 7.96 (m, 2 H) 7.73 (t, J=7.86 Hz, 1 H) 7.46 - 7.53 (m, 2 H) 7.17 (d, J=9.61 Hz, 1 H) 7.08 (dd, J=8.85, 1.98 Hz, 1 H) 6.89 (t, J=5.11 Hz, 1 H) 6.72 - 6.78 (m, 1 H) 5.67 - 5.77 (m, 1 H) 4.85 - 5.00 (m, 2 H) 4.73 (d, J=12.84 Hz, 2 H) 4.39 (d, J=12.59 Hz, 3 H) 4.09 - 4.21 (m, 3 H) 3.86 - 4.00 (m, 4 H) 3.44 - 3.66 (m, 8 H) 3.12 (dd, J=17.97, 12.59 Hz, 3 H) 2.83 - 3.00 (m, 2 H) 2.56 - 2.62 (m, 2 H) 1.88 - 2.11 (m, 6 H) 1.45 (br. s., 2 H)

### Example C-2: Preparation of 3-(1-(3-(5-((1-(1-(2-(4-((1-(2-(2-(2-(3-yl)-1-oxo-1,2-dihydrophthalazine-6-yl)piperidine-4-il)methyl)piperazine-1-yl)2-oxoethyl)piperidine-4-il)methoxy)pyrimidine-2-yl)benzonitrile (ICT-000975)

### Step 1) Preparation of tert-butyl 4-((1-((benzyloxy)carbonyl)piperidine-4-yl)methyl)piperazine-1-carboxylate

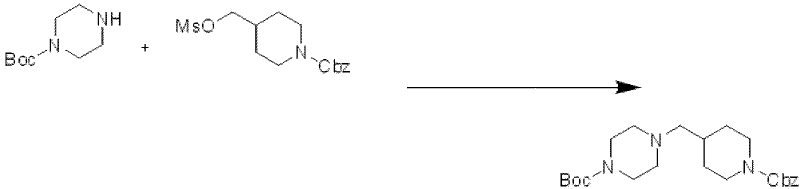

K₂CO₃ (1.41 g, 10.2 mmol) was added to tert-butylpiperazine-1-carboxylate (3 g, 10.2 mmol) and benzyl 4-((methylsulfonyl)oxymethyl)piperidine-1-carboxylate (4.5 g, 20.5 mmol) solutions in 30 ml DMF, and stirred at 85°C for 16 hours. After cooling, the reactant was poured into water (50 ml) and extracted with ethyl acetate. The extract was dried with Magnesium sulfate and concentrated under reduced pressure. The residue was purified by reversed-phase column chromatography. Benzyl 4-((1-(tert-butoxycarbonyl)piperidine-4-yl)methyl)piperazine-1-carboxylate (1.95 g) was obtained. MS (ESI, m/z): [M+H]⁺ = 418.2.

### Step 2) Preparation of tert-butyl 4-(piperidine-4-ylmethyl)piperazine-1-carboxylate

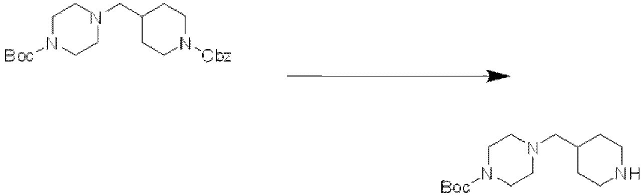

Pd/C (10 wt%, 50 mg) was added to tert-butyl 4-((1-(benzyloxy)carbonyl)piperidine-4-yl)methyl)piperazine-1-carboxylate (0.5 g, 1.2 mmol) solution in 10 ml MeOH and stirred at room temperature under a hydrogen atmosphere for 4 hours. The solution was filtered in a phase of cellite 545. The solvent was removed under reduced pressure, and tert-butyl 4-(piperazine-1-ylmethyl)piperidine-1-carboxylate (0.35 g) was obtained. MS (ESI, m/z): [M+H]⁺ = 284.0.

### Step 3) Preparation of tert-butyl 4-((1-(2-(2,6-dioxopiperidin-3-yl)-1-oxo-1,2-dihydroptalazin-6-yl)piperidine-4-yl)methyl)piperazine-1-carboxylate

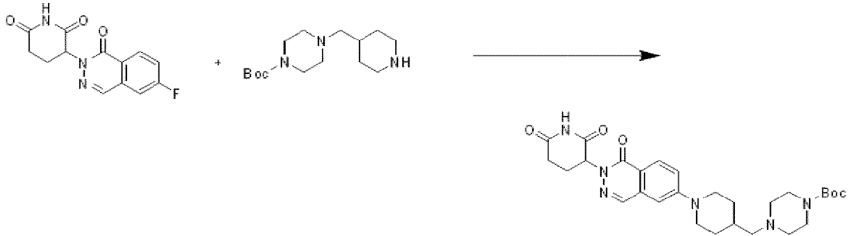

3-(6-fluoro-1-oxopthalazine-2(1H)-yl)piperidine-2,6-dione (1 g, 3.46 mmol), tert-butyl 4-(piperidine-4-yl)piperazine-1-carboxylate (1.96 g, 6.91 mmol), and DIPEA (1.81 ml, 10.4 mmol) solutions in NMP (10 mL) were stirred at 120°C for 20 hours. The reaction mixture was purified by reversed-phase column chromatography to obtain tert-butyl 4-((4-(2-(2,6-dioxopiperidin-3-yl)4-methyl-1-oxo-1,2-dihydroptalazin-6-yl)piperazine-1-yl)piperazine-1-carboxylate (910 mg). MS (ESI, m/z): [M+H]⁺ = 552.3.

### Step 4) Preparation of 3-(1-oxo-6-(4-(piperazine-1-ylmethyl)piperidine-1-yl)phthalazine-2(1H)-yl)piperidine-2,6-dione

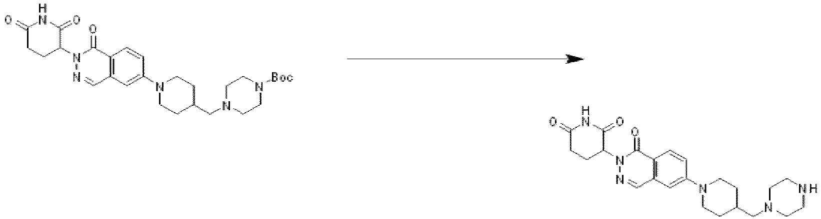

2 ml TFA was added to tert-butyl 4-((1-(2-(2-(2-(2-(2-(2-dioxopiperidine-3-yl)-1-oxo-1,2-dihydroptalazin-6-yl)piperidine-4-yl)methyl)piperazine-1-carboxylate (910 mg, 1.65 mmol) solution in 10 ml DCM, and stirred at room temperature for 2 hours. MS (ESI, m/z): [M+H]⁺ = 453.0.

### Step 5) Preparation of 3-(1-(1-(3-(5-((1-(2-(4-((1-(2-(2,6-dioxopiperidine-3-yl)-1-oxo-1,2-dihydropthalazine-6-yl)piperidine-4-il)methyl)piperazine-1-il)2-oxoethyl) piperidine-4-il)methoxy)pyrimidine-2-yl)benzonitrile (ICT-000975)

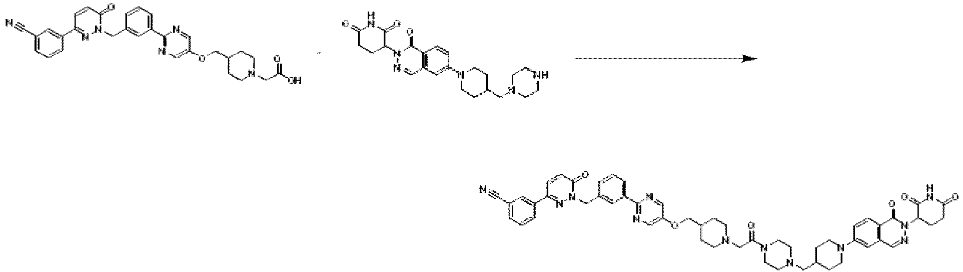

HATU (47.7 mg, 0.13 mmol) was added to 2-(((2-(3-((3-(3-cyanophenyl)-6-oxopridazine-1(6H)-yl)methyl)phenyl)pyrimidine-5-yl)oxy)methyl)piperidine-1-yl)acetic acid (30 mg, 0.06 mmol) and 3-(1-oxo-6-(4-(piperazine-1-yl)piperidine-1-yl)phthalazine-2(1H)-yl)piperidine-2,6-dione (24 mg, 0.06 mmol) in DMF (2.0 ml), followed by ethylbis (propane-2-yl)amine (3 equivalent). The reactants were stirred at room temperature for 6 hours. The reactants were poured into the water. It was extracted with ethyl acetate, dried with Magnesium sulfate, and concentrated under reduced pressure. The residues were purified by MeOH/DCM (0-20%) column chromatography to obtain the title compound (25 mg). MS (ESI, m/z): [M+H]⁺ = 972.1.
1H NMR (500 MHz, DMSO-d6) δ ppm 11.03 (s, 1 H) 8.65 - 8.70 (m, 3 H) 8.38 (s, 3 H) 8.22 - 8.30 (m, 3 H) 8.19 (d, J=9.78 Hz, 1 H) 8.12 (d, J=9.29 Hz, 1 H) 7.94 (d, J=7.58 Hz, 1 H) 7.73 (t, J=7.83 Hz, 1 H) 7.48 - 7.53 (m, 2 H) 7.17 (d, J=9.78 Hz, 1 H) 5.45 (s, 2 H) 4.10 (d, J=5.99 Hz, 1 H)

### Example C-3: Preparation of 3-(1-{[3-(5-{[1-(2-{4-[2-(2,6-dioxopiperidine-3-yl)-1-oxo-1,2-dihydrophthalazine-6-yl]piperazine-1-day}acetyl)piperidine-4-yl]methoxy}pyrimidine-2-yl)phenyl]methyl}-6-oxo-1,6-dihydropyridazine-3-yl)benzonitrile (ICT-000979) synthesis

### Step 1) Synthesis of tert-butyl 4-(2-(2,6-dioxopiperidine-3-yl)-1-oxo-1,2-dihydroptalazine-6-yl)piperazine-1-carboxylate

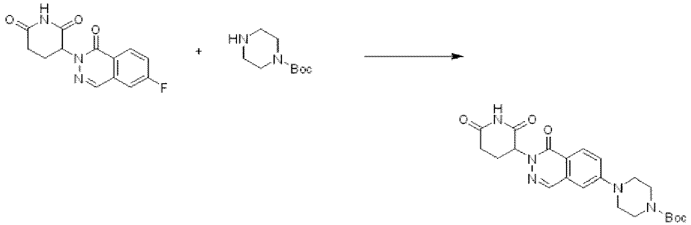

Ethylbis (propane-2-yl)amine (4.0 equivalent) was added to 3-(6-fluoro-1-oxopthalazine-2(1H)-yl)piperidine-2,6-dione (300 mg, 1.04 mmol) and tert-butyl piperazine-1-carboxylate (386 mg, 2.07 mmol) solutions in NMP (3.0 ml). The reactants were stirred at 130°C for 48 hours. After the reaction was completed, the reaction mixture was concentrated under reduced pressure. The residues were purified by MeOH/DCM (0-20%) column chromatography to obtain the heading compound (350 mg). MS (ESI, m/z): [M+H]⁺ = 442.4

### Step 2) Preparation of 3-(1-oxo-6-(piperazine-1-yl)phthalazine-2(1H)-yl)piperidine-2,6-dione

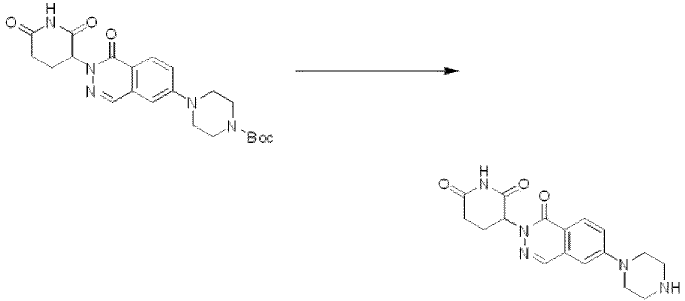

TFA (10.0 ml) was added to tert-butyl 4-(2-(2,6-dioxopiperidine-3-yl)-4-methyl-1-oxo-1,2-dihydroptalazin-6-yl) solution in DCM (30.0 ml). TFA (10.0 ml) was added to the tert-butyl 4-(2-(2,6-dioxopiperine-3-yl)-piperazine-1-carboxylate-6-yl solution in DCM (30.0 ml). The reactant was stirred at room temperature for 1 hour. After the end of the reaction, the reaction mixture was concentrated under reduced pressure. The residues were purified by MeOH/DCM (0-20%) column chromatography to obtain the title compound (200 mg). MS (ESI, m/z): [M+H]⁺ = 342.4

### Step 3) Preparation of tert-Butyl 2-(4-(2-(2,6-dioxopiperidin-3-yl)-1-oxo-1,2-dihydroptalazine-6-yl)piperazine-1-yl)acetate

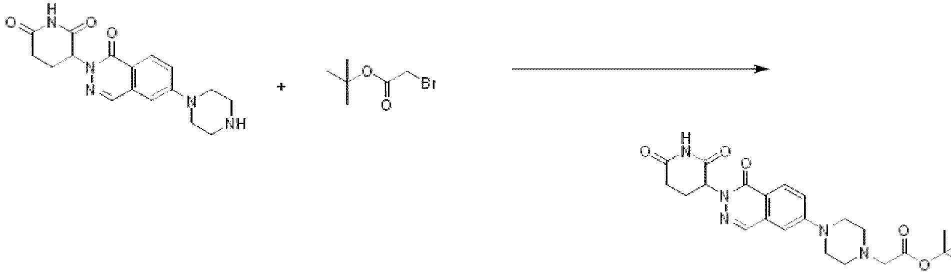

Ethylbis (propane-2-yl)amine (2.0 equivalent) was added to 3-(1-oxo-6-(piperazine-1-yl)phthalazine-2(1H)-yl) piperidine-2,6-dione (200 mg, 0.56 mmol) and tert-butyl 2-bromoacetate (121 mg, 0.62 mmol) solutions in ACN (10.0 ml). The reactants were stirred at room temperature for 5 hours. After the reaction was completed, the reaction mixture was concentrated under reduced pressure. The residue was purified by MeOH/DCM (0-20%) column chromatography, and the title compound (210 mg) was obtained MS (ESI, m/z): [M+H]⁺ = 456.4

### Step 4) Preparation of 2-(4-(2-(2,6-dioxopiperidin-3-yl)-1-oxo-1,2-dihydroptalazine-6-yl)piperazine-1-yl)acetic acid

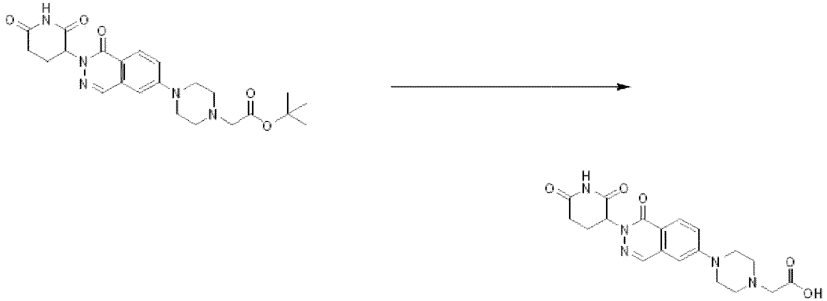

TFA (1.0 ml) was added to tert-butyl 2-(4-(2-(2,6-dioxopiperidine-3-yl)-1-oxo-1,2-dihydroptalazine-6-yl) acetate (30 mg, 0.06 mmol) solution in DCM (3.0 ml). The reactants were stirred at room temperature for 1 hour. After the reaction was completed, the reaction mixture was concentrated under reduced pressure. The residues were purified by MeOH/DCM (0-20%) column chromatography to obtain the title compound (15 mg). MS (ESI, m/z): [M+H]⁺ = 400.4

### Step 5) Preparation of 3-(1-{[3-(5-{[1-(2-{4-[2-(2,6-dioxopiperidin-3-yl)-1-oxo-1,2-dihydroptalazine-6-yl]piperazine-1-day}acetyl)piperidine-4-il]methoxy}pyrimidine-2-yl)phenyl]methyl}-6-oxo-1,6-dihydropyridazine-3-yl)benzonitrile (ICT-000979)

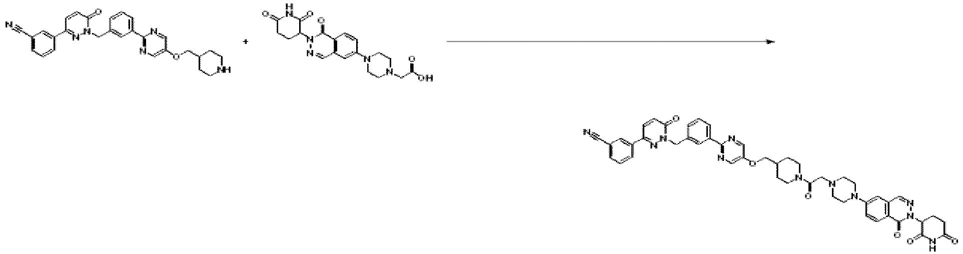

HATU (47.7 mg, 0.13 mmol) was added to a solution of 3-{6-oxo-1-[(piperidine-4-yl)methoxy]pyrimidin-2-yl}phenyl)methyl]-1,6-dihydropyridazin-3-yl}benzonitrile (30 mg, 0.06 mmol) and 2-{[3-(2,6-dioxopiperidine-3-yl)-1-methyl-4-oxo-3,4-dihydropthalazine-5-yl}acetic acid (20.6 mg, 0.06 mmol) in DMF (2.0 ml), followed by ethylbis (propane-2-yl)amine (3 equivalent). The reactants were stirred at room temperature for 6 hours. The reactants were poured into water. It was extracted with ethyl acetate, dried with Magnesium sulfate, and concentrated under reduced pressure. The residues were purified by MeOH/DCM (0-20%) column chromatography to obtain the title compound (40 mg). MS (ESI, m/z): [M+H]⁺ = 860.9.
1H NMR (500 MHz, DMSO-d6) δ ppm 11.01 (s, 1 H) 8.65 (s, 2 H) 8.38 (s, 2 H) 8.20 - 8.27 (m, 3 H) 8.18 (d, J=9.77 Hz, 1 H) 8.04 (d, J=9.16 Hz, 1 H) 7.93 (d, J=7.63 Hz, 1 H) 7.72 (t, J=7.86 Hz, 1 H) 7.44 - 7.53 (m, 3 H) 7.26 (s, 1 H) 7.17 (d, J=9.77 Hz, 1 H) 5.75 (dd, J=11.90, 4.73 Hz, 1 H) 5.44 (s, 2 H) 4.42 (d, J=12.21 Hz, 1 H) 4.06 - 4.16 (m, 3 H) 3.43 (br. s., 6 H) 3.12 (d, J=12.97 Hz, 1 H) 3.05 (t, J=11.98 Hz, 1 H) 2.86 - 2.98 (m, 1 H) 2.64 (br. s., 1 H) 2.60 (br. s., 6 H) 2.09 (d, J=5.04 Hz, 1 H) 1.96 (d, J=16.48 Hz, 1 H) 1.83 (br. s., 2 H) 1.32 - 1.40 (m, 1 H)

### Example C-4: Preparation of 3-(1-(3-(5-((1-(1-((1-((1-((3-(2,6-dioxopiperidin-3-yl)-1-methyl-4-oxo-3,4-dihydroptalazine-5-yl)glycil)piperidine-4-il)methyl)piperidine-4-il)methoxy)pyrimidine-2-il)benzyl)6-oxo-1,6-dihydropyridazine-3-yl)benzonitrile (ICT-0000980) synthesis

### Step 1) Preparation of 3-(6-oxo-1-(3-(5-((1-(piperidine-4-ylmethyl)piperidine-4-yl)methoxy)pyrimidine-2-yl)benzyl)1,6-dihydropyridazine-3-yl)benzonitrile

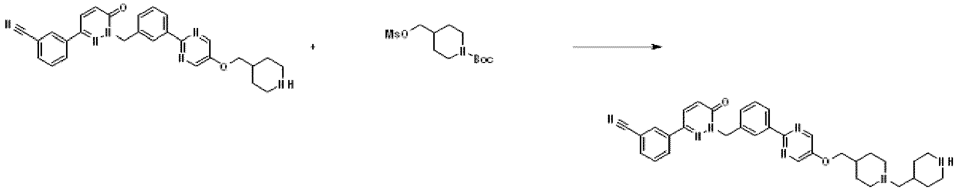

Ethylbis (81 mg, 0.63 mmol) was added to a solution of 3-{6-oxo-1-[(3-{5-[(piperidine-4-yl)methoxy]pyrimidin-2-yl}phenyl)methyl]-1,6-dihydropyridazin-3-yl} benzonitrile (250 mg, 0.52 mmol) and tert-butyl 4-[(methanesulfonyoxy)methyl]piperidine-1-carboxylate (184 mg, 0.62 mmol) in 1 ml of DMF at room temperature. The reactants were stirred at 60°C for 6 hours. The reactants were poured into water, extracted with ethyl acetate, dried with Magnesium sulfate, and concentrated under reduced pressure. The residue was purified with MeOH/ DCM (0-10%) MPLC to obtain a Boc-protected compound. The compound was treated with 4.0 M HCl of dioxane and stirred for 4 hours. After the reaction was completed, the reactants were concentrated under reduced pressure and vacuum-dried. The residue was dissolved in DCM, passed through NH-DM 1020, a Chromatorex pad, and the title compound (205 mg) was obtained. MS (ESI, m/z): [M+H]+= 576.7.
1H NMR (500 MHz, DMSO-d6) δppm 11.02 (s, 1 H) 8.60 - 8.71 (m, 2 H) 8.37 (s, 2 H) 8.13 - 8.28 (m, 4 H) 8.04 (d, J=9.05 Hz, 1 H) 7.93 (d, J=7.70 Hz, 1 H) 7.71 (t, J=7.89 Hz, 1 H) 7.42 - 7.54 (m, 3 H) 7.25 (d, J=2.08 Hz, 1 H) 7.16 (d, J=9.78 Hz, 1 H) 5.75 (dd, J=12.10, 4.89 Hz, 1 H) 5.44 (s, 2 H) 3.98 - 4.13 (m, 2 H) 3.37 - 3.47 (m, 4 H) 2.99 - 3.15 (m, 2 H) 2.85 - 2.98 (m, 1 H) 2.59 (br. s., 5 H) 1.99 (s, 1 H) 1.29 - 1.39 (m, 1 H) 1.11 - 1.25 (m, 2 H)

### Step 2) Preparation of 2-{[3-(2,6-dioxopiperidine-3-yl)-1-methyl-4-oxo-3,4-dihydroptalazine-5-yl]amino}acetic acid

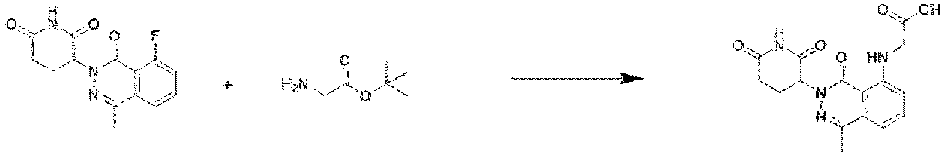

A solution of 3-(8-fluoro-4-methyl-1-oxo-1,2-dihydroptallazine-2-yl)piperidine-2,6-dione at room temperature was followed by ethylbis (propane-2-yl)amine (179 mg, 1.38 mmol). The reactants were heated to 110°C for 8 hours. The reactants were cooled to room temperature, poured into water (50 mL), and extracted with ethyl acetate (30 mL x 2). The mixed organic layer was dried with Magnesium sulfate and concentrated under reduced pressure. The residues were purified by column chromatography, intermediates were obtained, and treated with 50% TFA in CH₂Cl₂ (4 mL). The reactants were stirred for 2 hours, concentrated under reduced pressure, and subjected compounds (189 mg) were obtained. MS (ESI, m/z): [M+H]+=.345.2

### Step 3) Preparation of 3-(1-(3-(5-((1-((1-((3-(3-(2,6-dioxopiperidin-3-yl)-1-methyl-4-oxo-3,4-dihydroptalazine-5-yl)glysil)piperidine-4-yl)methyl)piperidine-4-yl)methoxy)pyrimidine-2-yl)benzyl)6-oxo-1,6-dihydropyridazine-3-yl)benzonitrile (ICT-0000980)

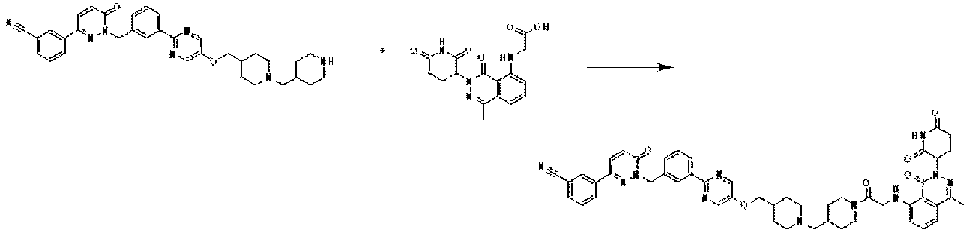

HATU (3-[6-oxo-1-({3-[5-({1-[(piperidine-4-yl)methyl]piperidine-4-yl}methoxy)pyrimidine-2-yl]phenyl}methyl)-1,6-dihydropyridazine-3-yl]benzonitrile (50 mg, 0.09 mmol) and 2-{[3-(2,6-dioxopiperidine-3-yl)-1-methyl-4-oxo-3,4-dihydroptalazin-5-yl]amino}acetic acid (29.2 mg, 0.09 mmol), followed by ethylbis (propane-2-yl)amine (33.7 mmol). The reactants were stirred for 6 hours. The reactants were poured into the water. It was extracted with ethyl acetate, dried with Magnesium sulfate, and concentrated under reduced pressure. The residue was purified with MeOH/DCM (0-20%) MPLC to obtain the title compound (34.2 mg). MS (ESI, m/z): [M+H]⁺ = 903.0.
1H NMR (500 MHz, DMSO-d6) δ ppm 11.00 (s, 1 H) 9.45 (br. s., 1 H) 8.65 (s, 2 H) 8.38 (s, 2 H) 8.15 - 8.29 (m, 3 H) 7.94 (d, J=7.70 Hz, 1 H) 7.61 -7.79 (m, 2 H) 7.45 - 7.53 (m, 2 H) 7.17 (d, J=9.78 Hz, 1 H) 6.94 (d, J=7.46 Hz, 2 H) 5.63 (br. s., 1 H) 5.45 (s, 2 H) 4.40 (d, J=12.35 Hz, 1 H) 4.08 (br. s., 4 H) 3.92 (d, J=13.82 Hz, 1 H) 3.02 (d, J=12.23 Hz, 1 H) 2.79 - 2.97 (m, 3 H) 2.54 - 2.71 (m, 4 H) 2.42 (s, 4 H) 2.01 - 2.18 (m, 3 H) 1.82 - 2.01 (m, 3 H) 1.77 (br. s., 6 H) 1.31 (br. s., 1 H) 1.08 - 1.27 (m, 4 H) 1.04 (d, J=6.11 Hz, 1 H) 0.94 (d, J=5.87 Hz, 1 H)

### Example C-5: Preparation of 3-(1-(3-(5-((1-(1-((3-(3-(2,6-dioxopiperidin-3-yl)-1-methyl-4-oxo-3,4-dihydrophthalazine-5-yl)glycil)piperidine-4-il)methoxy)pyrimidine-2-yl)benzyl)-6-oxo-1,6-dihydropyridazine-3-yl)benzonitrile (ICT-0000981)

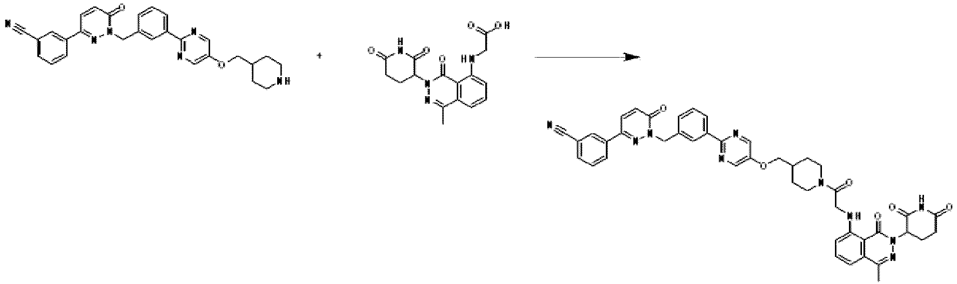

HATU (36.8 mg, 0.1 mmol) was added to 3-{6-oxo-1-[(piperidine-4-yl)methoxy]pyrimidin-2-yl}phenyl)methyl]-1,6-dihydropyridazine-3-yl}benzonitrile (41.6 mg, 0.09 mmol) and 2-{[3-(2,6-dioxopiperidine-3-yl)-1-methyl-4-oxo-3,4-dihydroptalazine-5-yl}amino}acetic acid (29.3 mg, 0.86 mmol), followed by ethylbis (propane-2-yl)amine (33.7 mg, 0.26 mmol) in DMF (5 ml) at room temperature. It was extracted with ethyl acetate, dried with Magnesium sulfate, and concentrated under reduced pressure. The residue was purified with MeOH/DCM (0-20%) MPLC to obtain the title compound (35.6 mg). MS (ESI, m/z): [M+H]⁺ = 805.9.
1H NMR (500 MHz, DMSO-d6) δ ppm 11.01 (s, 1 H) 9.47 (br. s., 1 H) 8.61 - 8.71 (m, 2 H) 8.33 - 8.43 (m, 2 H) 8.20 - 8.28 (m, 2 H) 8.18 (d, J=9.90 Hz, 1 H) 7.85 - 7.99 (m, 1 H) 7.72 (t, J=7.89 Hz, 1 H) 7.67 (t, J=8.13 Hz, 1 H) 7.41 -7.54 (m, 2 H) 7.17 (d, J=9.78 Hz, 1 H) 6.95 (dd, J=8.13, 3.12 Hz, 2 H) 5.45 (s, 2 H) 3.95 - 4.15 (m, 3 H) 3.10 (t, J=12.23 Hz, 1H) 2.82 - 2.98 (m, 1 H) 2.52 - 2.81 (m, 3 H) 2.42 (s, 3 H) 2.02 - 2.16 (m, 1 H) 1.11 - 1.29 (m, 1 H)

### Example C-6: Preparation of 3-(1-{[3-(5-{[1-(6-{[2-(2,6-dioxopiperidin-3-yl)-1-oxo-1,2-dihydropthalazine-6-yl]amino}hexanoyl)piperidine-4-il]methoxy}pyrimidine-2-yl)phenyl]methyl}-6-oxo-1,6-dihydropyridazine-3-yl)benzonitrile (ICT-0000984)

### Step 1) Preparation of 6-{[2-(2,6-dioxopiperidin-3-yl)-1-oxo-1,2-dihydroptalazine-6-yl]amino} hexanoic acid

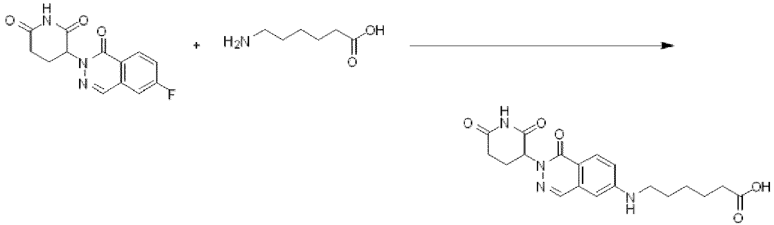

Ethylbis (propane-2-yl)amine (4.0 equivalent) was added to 3-(6-fluoro-1-oxo-1,2-dihydroptallazine-2-yl) and 6-aminohexanoic acid (286 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography, and the heading compound (270 mg) was obtained. MS (ESI, m/z): [M+H]⁺ = 387.4

### Step 2) Preparation of 3-(1-{[3-(5-{[1-(6-{[2-(2,6-dioxopiperidin-3-yl)-1-oxo-1,2-dihydroptalazine-6-yl]amino}hexanoyl)piperidine-4-il]methoxy}pyrimidine-2-yl)phenyl]methyl}-6-oxo-1,6-dihydropyridazine-3-yl)benzonitrile (ICT-0000984)

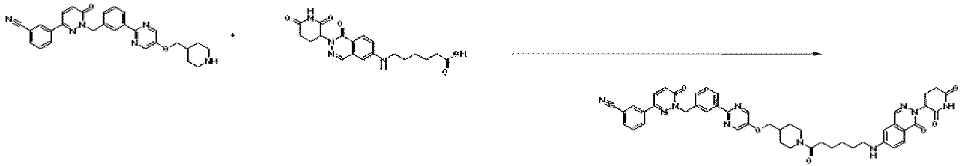

HATU (47.7 mg, 0.13 mmol) was added to 3-{6-oxo-1-[(piperidine-4-yl)methoxy]pyrimidin-2-yl}phenyl)methyl]-1,6-dihydropyridazine-3-yl}benzonitrile (30 mg, 0.06 mmol) and 6-{[2-(2,6-dioxopiperidine-3-yl)-1-oxo-1,2-dihydroptalazin-6-yl]amino}hexanoic acid (24.2 mg, 0.06 mmol), followed by ethylbis (propane-2-yl)amine (3 equivalent). The reactant was stirred at room temperature for 6 hours. The reactant was poured into the water. It was extracted with ethyl acetate, dried with Magnesium sulfate, and concentrated under reduced pressure. The residues were purified by MeOH/DCM (0-20%) column chromatography to obtain the title compound (20 mg). MS (ESI, m/z): [M+H]⁺ = 847.9.
1H NMR (500 MHz, DMSO-d6) δppm 10.98 (s, 1 H) 8.63 - 8.68 (m, 2 H) 8.39 (s, 2 H) 8.15 - 8.28 (m, 2 H) 7.89 - 7.96 (m, 2 H) 7.73 (t, J=7.86 Hz, 1 H) 7.46 - 7.53 (m, 2 H) 7.17 (d, J=9.61 Hz, 1 H) 7.08 (dd, J=8.85, 1.98 Hz, 1 H) 6.89 (t, J=5.11 Hz, 1 H) 6.72 - 6.78 (m, 1 H) 5.72 (dd, J=11.83, 4.65 Hz, 1 H) 5.45 (s, 2 H) 4.44 (d, J=14.04 Hz, 1 H) 4.06 (d, J=6.41 Hz, 2 H) 3.91 (d, J=13.89 Hz, 2 H) 3.62 (dq, J=10.49, 6.62 Hz, 2 H) 3.10 - 3.19 (m, 4 H) 3.02 (t, J=12.44 Hz, 2 H) 2.86 - 2.96 (m, 2 H) 2.30 - 2.37 (m, 2 H) 2.02 - 2.11 (m, 2 H) 1.80 (t, J=15.03 Hz, 2 H) 1.51 - 1.67 (m, 4 H) 1.36 - 1.45 (m, 2 H)

### Example C-7: Preparation of 3-(1-{[3-(5-{[1-(7-{[2-(2,6-dioxopiperidin-3-yl)-1-oxo-1,2-dihydropthalazine-6-yl]amino}heptanoyl)piperidine-4-il]methoxy}pyrimidine-2-yl)phenyl]methyl}-6-oxo-1,6-dihydropyridazine-3-yl)benzonitrile (ICT-0000985)

### Step 1) Preparation of 7-((2-(2-(2,6-dioxopiperidin-3-yl)-1-oxo-1,2-dihydroptalazine-6-yl)amino)heptanoic acid

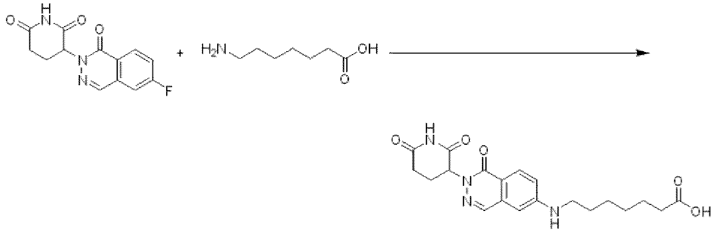

Ethylbis (propane-2-yl)amine (4.0 equivalent) was added to 3-(6-fluoro-1-oxo-1,2-dihydropetthalazine-2-yl) and 7-aminoheptanoic acid (260 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the heading compound (250 mg). MS (ESI, m/z): [M+H]⁺ = 401.4

### Step 2) Preparation of 3-(1-{[3-(5-{[1-(7-{[2-(2,6-dioxopiperidin-3-yl)-1-oxo-1,2-dihydrophthalazine-6-yl]amino}heptanoyl)piperidine-4-il]methoxy}pyrimidine-2-yl)phenyl]methyl}-6-oxo-1,6-dihydropyridazine-3-yl)benzonitrile (ICT-0000985)

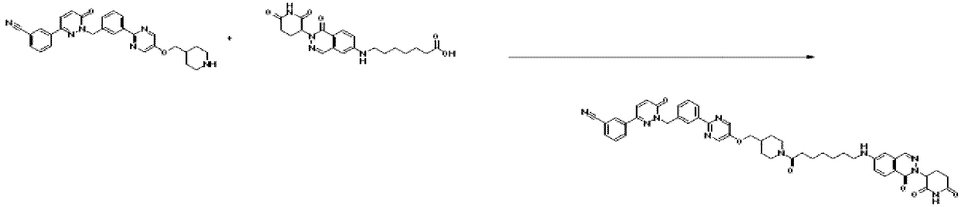

HATU (47.7 mg, 0.13 mmol) was added to 3-{6-oxo-1-[(piperidine-4-yl)methoxy]pyrimidin-2-yl}phenyl)methyl]-1,6-dihydropyridazin-3-yl} benzonitrile (30 mg, 0.06 mmol) and 7-((2-(2,6-dioxopiperidine-3-yl)1-oxo-1,2-dihydroptalazine-6-yl)amino)heptanoic acid (25.2 mg, 0.06 mmol), followed by ethylbis(propane-2-yl)amine (3 equivalent). The reactant was stirred at room temperature for 6 hours. The reactant was poured into the water. It was extracted with ethyl acetate, dried with Magnesium sulfate, and concentrated under reduced pressure. The residues were purified by MeOH/DCM (0-20%) column chromatography to obtain the title compound (30 mg). MS (ESI, m/z): [M+H]⁺ = 861.9.
1H NMR (500 MHz, DMSO-d6) δ ppm 10.99 (s, 1 H) 8.62 - 8.69 (m, 2 H) 8.39 (s, 2 H) 8.14 - 8.30 (m, 2 H) 7.86 - 7.97 (m, 2 H) 7.73 (t, J=7.93 Hz, 1 H) 7.44 - 7.54 (m, 2 H) 7.17 (d, J=9.61 Hz, 1 H) 7.08 (dd, J=8.93, 2.06 Hz, 1 H) 6.89 (br. s., 1 H) 6.73 - 6.80 (m, 1 H) 5.72 (dd, J=12.05, 5.04 Hz, 1 H) 5.45 (s, 2 H) ) 4.43 (d, J=12.66 Hz, 1 H) 4.07 (d, J=6.26 Hz, 2 H) 3.90 (d, J=13.12 Hz, 1 H) 3.62 (dq, J=10.47, 6.53 Hz, 4 H) 3.09 - 3.20 (m, 6 H) 3.02 (t, J=11.60 Hz, 2 H) 2.85 - 2.97 (m, 1 H) 2.32 (t, J=7.40 Hz, 2 H) 2.02 - 2.12 (m, 1 H) 1.88 - 2.01 (m, 2 H) 1.75 - 1.87 (m, 2 H) 1.57 - 1.64 (m, 2 H) 1.31 - 1.55 (m, 2 H)

### Example C-8: Preparation of 3-{1-[(3-{5-[(1-{2-[2-(2-{2-(2,6-dioxopiperidin-3-yl)-1-oxo-1,2-dihydroptalazin-6-yl}amino}ethoxy)ethoxy]acetyl}piperidine-4-il)methoxy]pyrimidine-2-yl}phenyl)methyl]-6-oxo-1,6-dihydropyridazine-3-yl}benzonitrile (ICT-0000986)

### Step 1) Preparation of 2-(2-(2-((2-(2-(2-(2,6-dioxopiperidin-3-yl)-1-oxo-1,2-dihydroptalazine-6-yl)amino)ethoxy)ethoxy)ethoxy)acetate

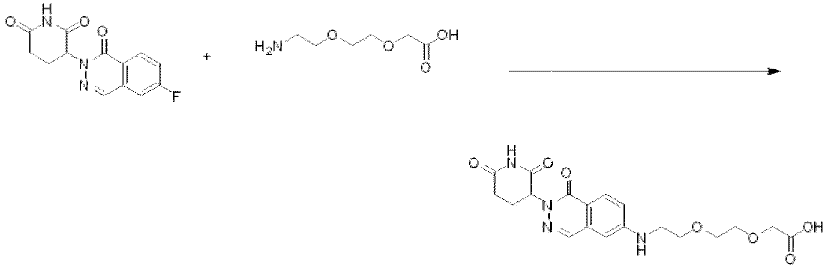

Ethylbis (propane-2-yl)amine (4.0 equivalent) was added to 3-(6-fluoro-1-oxo-1,2-dihydrophthalazine-2-yl)piperidine-2,6-dione (300 mg, 1.09 mmol) and 2-(2-(2-aminoethoxy)ethoxy-acetic acid (285 mg, 2.18 mmol) solutions in NMP (2.0 ml). The reactants were stirred at 130°C for 48 hours. After the end of the reaction, the reaction mixture was concentrated under reduced pressure. The residues were purified by MeOH/DCM (0-20%) column chromatography. Subjective compound (240 mg) was obtained MS (ESI, m/z): [M+H]⁺ = 419.4

### Step 2) Preparation of 3-{1-[(3-{5-[(1-{2-[2-(2-{[2-(2,6-dioxopiperidin-3-yl)-1-oxo-1,2-dihydropthalazine-6-yl]amino}ethoxy)ethoxy]acetyl}piperidine-4-il)methoxy]pyrimidine-2-yl}phenyl)methyl]-6-oxo-1,6-dihydropyridazine-3-yl}benzonitrile (ICT-0000986)

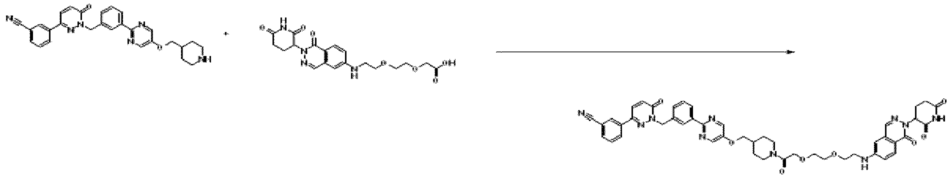

HATU (47.7 mg, 0.13 mmol) was added to 3-{6-oxo-1-[(piperidine-4-yl)methoxy]pyrimidin-2-yl}phenyl)methyl]-1,6-dihydropyridazine-3-yl} benzonitrile (30 mg, 0.06 mmol) and 2-(2-(2-((2-(2-(2-(2-(2,6-dioxopiperidine-3-yl)-1-oxo-1,2-dihydropthalazine-6-yl)amino)ethoxy)ethoxy) acetic acid (26.2 mg, 0.06 mmol), followed by ethylbis(propane-2-yl)amine (3 equivalent). The reactants were stirred at room temperature for 6 hours. It was extracted with ethyl acetate, dried with Magnesium sulfate, and concentrated under reduced pressure. The residues were purified by MeOH/DCM (0-20%) column chromatography to obtain the title compound (30 mg). MS (ESI, m/z): [M+H]⁺ = 879.9.
1H NMR (500 MHz, DMSO-d6) δ ppm 10.98 (s, 1 H) 8.63 - 8.68 (m, 2 H) 8.39 (s, 2 H) 8.15 - 8.28 (m, 2 H) 7.89 - 7.96 (m, 2 H) 7.73 (t, J=7.86 Hz, 1 H) 7.46 - 7.53 (m, 2 H) 7.17 (d, J=9.61 Hz, 1 H) 7.08 (dd, J=8.85, 1.98 Hz, 1 H) 6.89 (t, J=5.11 Hz, 1 H) 6.72 - 6.78 (m, 1 H) 5.73 (d, J=10.88 Hz, 1 H) 4.86 - 5.00 (m, 2 H) 4.73 (d, J=12.23 Hz, 1 H) 4.39 (d, J=12.59 Hz, 2 H) 4.21 (br. s., 2 H) 3.88 - 3.98 (m, 4 H) 3.56 - 3.66 (m, 6 H) 3.13 (br. s., 2 H) 2.84 - 2.98 (m, 2 H) 2.59 (d, J=18.10 Hz, 2 H) 2.08 (d, J=4.28 Hz, 2 H) 1.91 - 2.02 (m, 2 H) 1.75 (s, 2 H)

### Example C-9: Preparation of 3-(1-{[3-(5-{1-(2-{2-{2-[2-(2-{2-(2,6-dioxopiperidin-3-yl)-1-oxo-1,2-dihydropetalazine-6-yl}amino}ethoxy)ethoxy}ethoxy}acetyl)piperidine-4-il]methoxy}pyrimidine-2-yl)phenyl]methyl}6-oxo-1,6-dihydropyridazine-3-yl)benzonitrile (ICT-0000987)

### Step 1) Preparation of 2-{2-[2-(2-{[2-(2,6-dioxopiperidin-3-yl)-1-oxo-1,2-dihydroptalazin-6-yl]amino}ethoxy)ethoxy]ethoxy}ethoxy}ethoxy}acetate

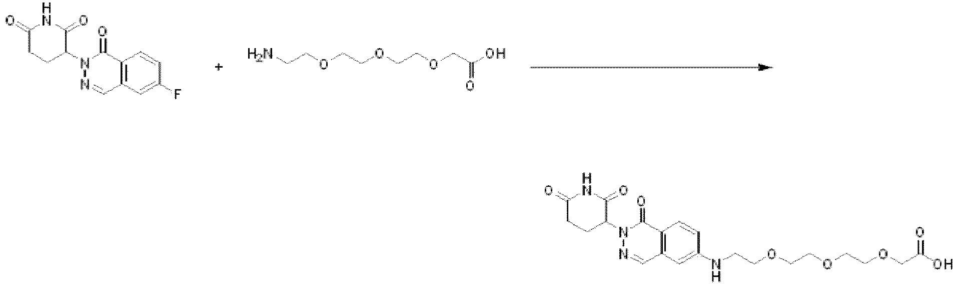

Ethylbis (propane-2-yl)amine (4.0 equivalent) was added to 3-(6-fluoro-1-oxo-1,2-dihydroptallazine-2-yl)piperidine-2,6-dione (300 mg, 1.09 mmol) and 2-{2-[2-(2-aminoethoxy)ethoxy]ethoxy}acetic acid (410 mg, 2.18 mmol) in NMP (2.0 ml). The reactants were stirred at 130°C for 48 hours. After the end of the reaction, the reaction mixture was concentrated under reduced pressure. The residues were purified by MeOH/DCM (0-20%) column chromatography. Subject compound (250 mg) MS (ESI, m/z): [M+H]⁺ = 463.4

### Step 2) Preparation of 3-(1-{[3-(5-{[1-(2-{2-{2-(2-{2-{2-(2,6-dioxopiperidine-3-yl)-1-oxo-1,2-dihydroptalazine-6-yl]amino}ethoxy)ethoxy}acetyl)piperidine-4-il]methoxy}pyrimidine-2-yl)phenyl]methyl}6-oxo-1,6-dihydropyridazine-3-yl)benzonitrile (ICT-0000987)

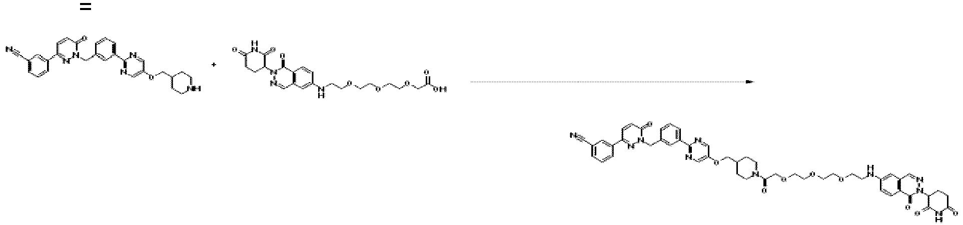

HATU (47.7 mg, 0.13 mmol) was added to 3-{6-oxo-1-[(piperidine-4-yl)methoxy]pyrimidin-2-yl}phenyl)methyl]-1,6-dihydropyridazin-3-yl} benzonitrile (30 mg, 0.06 mmol) and 2-{2-[2-(2-{[2-(2,6-dioxopiperidine-3-yl)-1-oxo-1,2-dihydrophoptalazin-6-yl]amino}ethoxy]ethoxy]ethoxy}acetic acid (29.2 mg, 0.06 mmol), followed by ethylbis(propane-2-yl)amine (3 equivalent). The reactants were stirred at room temperature for 6 hours. The reactants were poured into the water. It was extracted with ethyl acetate, dried with Magnesium sulfate, and concentrated under reduced pressure. The residues were purified by MeOH/DCM (0-20%) column chromatography to obtain the title compound (40 mg). MS (ESI, m/z): [M+H]⁺ = 823.9.
1H NMR (500 MHz, DMSO-d6) δ ppm 10.98 (s, 1 H) 8.63 - 8.68 (m, 2 H) 8.39 (s, 2 H) 8.15 - 8.28 (m, 2 H) 7.89 - 7.96 (m, 2 H) 7.73 (t, J=7.86 Hz, 1 H) 7.46 - 7.53 (m, 2 H) 7.17 (d, J=9.61 Hz, 1 H) 7.08 (dd, J=8.85, 1.98 Hz, 1 H) 6.89 (t, J=5.11 Hz, 1 H) 6.72 - 6.78 (m, 1 H) 5.72 (dd, J=11.37, 4.89 Hz, 1 H) 4.85 - 5.00 (m, 2 H) 4.73 (d, J=12.10 Hz, 1 H) 4.39 (d, J=11.62 Hz, 3 H) 4.15 - 4.21 (m, 3 H) 3.88 - 3.97 (m, 4 H) 3.44 - 3.67 (m, 8 H) 3.10 (d, J=12.84 Hz, 3 H) 2.78 - 2.98 (m, 2 H) 2.59 (d, J=17.97 Hz, 2 H) 1.88 - 2.09 (m, 2 H) 1.33 - 1.41 (m, 3 H)

### Example C-10: Preparation of 3-(1-{[3-(5-{[1-(14-{[2-(2,6-dioxopiperidin-3-yl)-1-oxo-1,2-dihydroptalazine-6-yl]amino}-3,6,9,12-tetradecanoyl)piperidin-4-yl]methoxy}pyrimidine-2-yl)phenyl]methyl}-6-oxo-1,6-dihydropyridazine-3-yl)benzonitrile (ICT-0000988)

### Step 1) Preparation of Synthesis 14-{[2-(2,6-dioxopiperidin-3-yl)-1-oxo-1,2-dihydroptalazine-6-yl]amino}-3,6,9,12-tetratetradecanoic acid

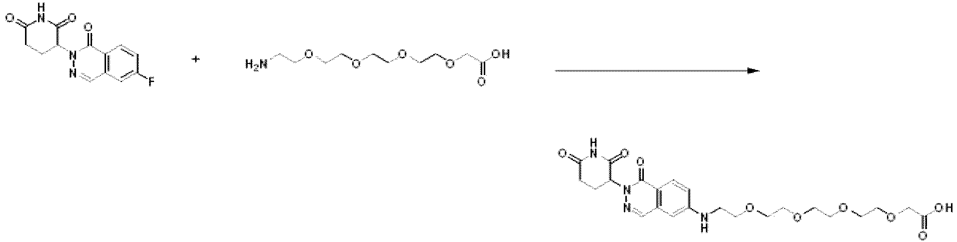

Ethylbis (propane-2-yl)amine (4.0 equivalent) was added to 3-(6-fluoro-1-oxo-1,2-dihydroptallazine-2-yl)piperidine-2,6-dione (300 mg, 1.09 mmol) and 14-amino-3,6,9,12-tetratetradecanoic acid (548 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the title compound (270 mg). MS (ESI, m/z): [M+H]⁺ = 507.4

### Step 2) Preparation of 3-(1-{[3-(5-{[1-(14-{[2-(2,6-dioxopiperidin-3-yl)-1-oxo-1,2-dihydroptalazine-6-yl]amino}-3,6,9,12-tetratetradecanoyl)piperidine-4-il]methoxy}pyrimidin-2-yl)phenyl]methyl}-6-oxo-1,6-dihydropyridazine-3-yl)benzonitrile (ICT-0000988)

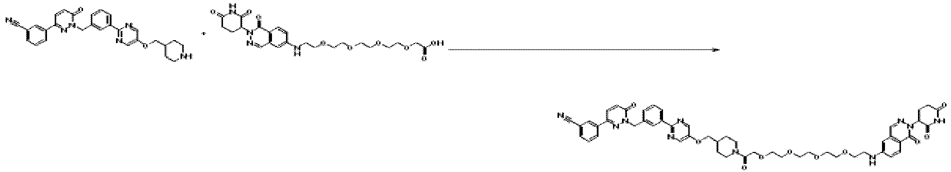

HATU (47.7 mg, 0.13 mmol) was added to 3-{6-oxo-1-[(piperidine-4-yl)methoxy]pyrimidine-2-yl}phenyl)methyl]-1,6-dihydropyridazin-3-yl} benzonitrile (30 mg, 0.06 mmol) and 6-{[2-(2,6-dioxopiperidine-3-yl)-1-oxo-1,2-dihydropedalazine-6-yl]amino}hexanoic acid (31.8 mg, 0.06 mmol), followed by ethylbis (propane-2-yl)amine (3 equivalent). The reactants were stirred at room temperature for 6 hours. The reactants were poured into water. It was extracted with ethyl acetate, dried with Magnesium sulfate, and concentrated under reduced pressure. The residues were purified by MeOH/DCM (0-20%) column chromatography to obtain the title compound (40 mg). MS (ESI, m/z): [M+H]⁺ = 968.3.
1H NMR (500 MHz, DMSO-d6) δ ppm 10.98 (s, 1 H) 8.63 - 8.68 (m, 2 H) 8.39 (s, 2 H) 8.15 - 8.28 (m, 2 H) 7.89 - 7.96 (m, 2 H) 7.73 (t, J=7.86 Hz, 1 H) 7.46 - 7.53 (m, 2 H) 7.17 (d, J=9.61 Hz, 1 H) 7.08 (dd, J=8.85, 1.98 Hz, 1 H) 6.89 (t, J=5.11 Hz, 1 H) 6.72 - 6.78 (m, 1 H) 5.67 - 5.77 (m, 1 H) 4.85 - 5.00 (m, 2 H) 4.73 (d, J=12.84 Hz, 2 H) 4.39 (d, J=12.59 Hz, 3 H) 4.09 - 4.21 (m, 3 H) 3.86 - 4.00 (m, 4 H) 3.44 - 3.66 (m, 8 H) 3.12 (dd, J=17.97, 12.59 Hz, 3 H) 2.83 - 3.00 (m, 2 H) 2.56 - 2.62 (m, 2 H) 1.88 - 2.11 (m, 6 H) 1.45 (br. s., 2 H)

### Example C-11: 3-(1-(3-(5-((1-(1-(4-((2-(2,6-dioxopiperidin-3-yl)-4-methyl-1-oxo-1,2-dihydrophthalazine-6-yl)amino)butanoyl)piperidine-4-il)methoxy)pyrimidin-2-yl)benzyl)-6-oxo-1,6-dihydropyridazine-3-yl)benzonitrile (ICT-0000989) synthesis

### Step 1) Preparation of 4-((2-(2-(2,6-dioxopiperidin-3-yl)-4-methyl-1-oxo-1,2-dihydroptalazin-6-yl)amino)butanoic acid

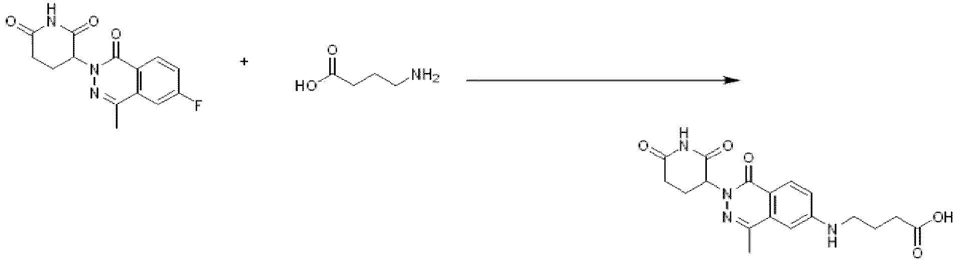

Ethylbis (propane-2-yl)amine (4.0 equivalent) was added to 3-(6-fluoro-4-methyl-1-oxopthalazine-2(1H)-yl)piperidine-2,6-dione (300 mg, 1. mmol) and 4-aminobutanoic acid (225 mg, 2.18 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the title compound (270 mg). MS (ESI, m/z): [M+H]⁺ = 373.4.

### Step 2) Preparation of 3-(1-(3-(5-((1-(4-((2-(2,6-dioxopiperidin-3-yl)-4-methyl-1-oxo-1,2-dihydropthalazine-6-yl)amino)butanoyl)piperidine-4-yl)methoxy)pyrimidine-2-yl)benzonitrile-6-oxo-1,6-dihydropyridazine-3-yl)benzonitrile (ICT-0000989)

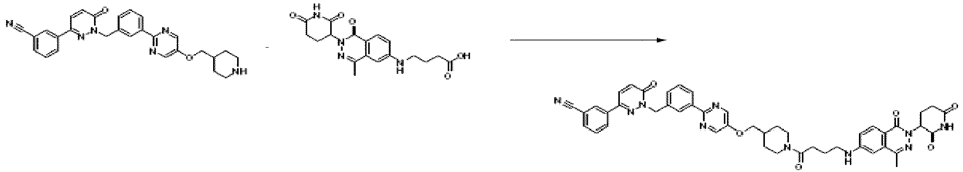

HATU (47.7 mg, 0.13 mmol) was added to 3-{6-oxo-1-[(piperidine-4-yl)methoxy]pyrimidin-2-yl}phenyl)methyl]-1,6-dihydropyridazine-3-yl} benzonitrile (30 mg, 0.06 mmol) and 4-((2-(2-(2-(2-(2-dioxopiperidine-3-yl)4-methyl-1-oxo-1,2-dihydrophthalazine-6-yl)amino)butanoic acid (30.8 mg, 0.06 mmol), followed by ethylbis (propane-2-yl)amine (3 equivalent). The reactant was stirred at room temperature for 6 hours. The reactant was poured into the water. It was extracted with ethyl acetate, dried with Magnesium sulfate, and concentrated under reduced pressure. The residues were purified by MeOH/DCM (0-20%) column chromatography to obtain the title compound (40 mg). MS (ESI, m/z): [M+H]⁺ = 833.9.
1H NMR (500 MHz, DMSO-d6) δ ppm 10.98 (s, 1 H) 8.63 - 8.68 (m, 2 H) 8.39 (s, 2 H) 8.15 - 8.28 (m, 2 H) 7.89 - 7.96 (m, 2 H) 7.73 (t, J=7.86 Hz, 1 H) 7.46 - 7.53 (m, 2 H) 7.17 (d, J=9.61 Hz, 1 H) 7.08 (dd, J=8.85, 1.98 Hz, 1 H) 6.89 (t, J=5.11 Hz, 1 H) 6.72 - 6.78 (m, 1 H) 5.72 (dd, J=11.83, 4.65 Hz, 1 H) 5.45 (s, 2 H) 4.44 (d, J=14.04 Hz, 1 H) 4.06 (d, J=6.41 Hz, 2 H) 3.91 (d, J=13.89 Hz, 2 H) 3.62 (dq, J=10.49, 6.62 Hz, 2 H) 3.10 - 3.19 (m, 4 H) 3.02 (t, J=12.44 Hz, 2 H) 2.86 - 2.96 (m, 2 H) 2.30 - 2.37 (m, 2 H) 2.02 - 2.11 (m, 3 H) 1.80 (t, J=15.03 Hz, 2 H) 1.51 - 1.67 (m, 2 H) 1.36 - 1.45 (m, 2 H)

### Example C-12: Preparation of 3-(1-(3-(5-((1-(1-(6-((2-(2,6-dioxopiperidin-3-yl)-4-methyl-1-oxo-1,2-dihydrophthalazine-6-yl)amino)hexanoyl)piperidine-4-il)methoxy)pyrimidine-2-yl)benzyl)6-oxo-1,6-dihydropyridazine-3-yl)benzonitrile (ICT-0000990) synthesis

### Step1) Preparation of 6-((2-(2,6-dioxopiperidin-3-yl)-4-methyl-1-oxo-1,2-dihydroptalazine-6-yl)amino)hexanoic acid

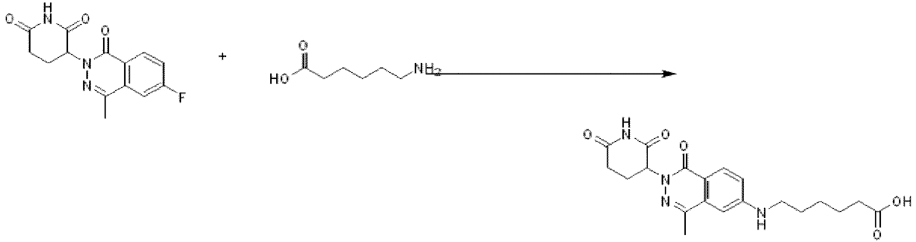

Ethylbis (propane-2-yl)amine (4.0 equivalent) was added to 3-(6-fluoro-4-methyl-1-oxopthalazine-2(1H)-yl)piperidine-2,6-dione (300 mg, 1. mmol) and 6-aminohexanoic acid (325 mg, 2.18 mmol) solutions in NMP (2.0 ml). The reactants were stirred at 130°C for 48 hours. After the end of the reaction, the reaction mixture was concentrated under reduced pressure. The residues were purified by MeOH/DCM (0-20%) column chromatography to obtain the heading compound (280 mg). MS (ESI, m/z): [M+H]⁺ = 401.4.

### Step 2) Preparation of 3-(1-(3-(5-((1-(6-((2-(2,6-dioxopiperidin-3-yl)-4-methyl-1-oxo-1,2-dihydropthalazine-6-yl)amino)hexanoyl)piperidine-4-yl)methoxy)pyrimidine-2-yl)benzyl)benzonitrile (ICT-0000990)

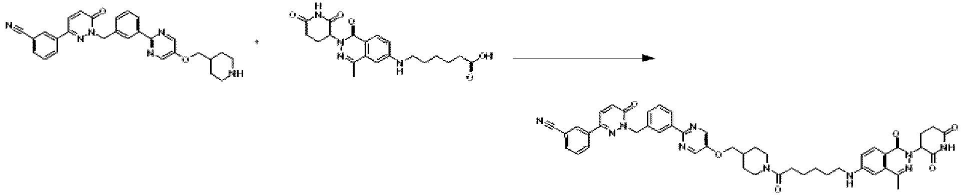

HATU (47.7 mg, 0.13 mmol) was added to 3-(6-oxo-1-(3-(5-(piperidine-4-ylmethoxy)pyrimidine-2-yl)benzyl)-1,6-dihydropyridazine-3-yl) benzonitrile (30 mg, 0.06 mmol) and 6-((2-(2-(2,6-dioxopiperidine-3-yl)4-methyl-1-oxo-1,2-dihydropthalazine-6-yl)amino)hexanoic acid (38.8 mg, 0.13 mmol), followed by ethylbis (propane-2-yl)amine (3 equivalent). The reactants were stirred at room temperature for 6 hours. The reactants were poured into the water. It was extracted with ethyl acetate, dried with Magnesium sulfate, and concentrated under reduced pressure. The residue was purified by MeOH/DCM (0-20%) column chromatography to obtain the title compound (40 mg). MS (ESI, m/z): [M+H]⁺ = 861.9.
1H NMR (500 MHz, DMSO-d6) δ ppm 10.98 (s, 1 H) 8.63 - 8.68 (m, 2 H) 8.39 (s, 2 H) 8.15 - 8.28 (m, 2 H) 7.89 - 7.96 (m, 2 H) 7.73 (t, J=7.86 Hz, 1 H) 7.46 - 7.53 (m, 2 H) 7.17 (d, J=9.61 Hz, 1 H) 7.08 (dd, J=8.85, 1.98 Hz, 1 H) 6.89 (t, J=5.11 Hz, 1 H) 6.72 - 6.78 (m, 1 H) 5.72 (dd, J=11.83, 4.65 Hz, 1 H) 5.45 (s, 2 H) 4.44 (d, J=14.04 Hz, 1 H) 4.06 (d, J=6.41 Hz, 2 H) 3.91 (d, J=13.89 Hz, 2 H) 3.62 (dq, J=10.49, 6.62 Hz, 2 H) 3.10 - 3.19 (m, 4 H) 3.02 (t, J=12.44 Hz, 2 H) 2.86 - 2.96 (m, 2 H) 2.30 - 2.37 (m, 2 H) 2.02 - 2.11 (m, 2 H) 1.95 - 2.00 (s, 3H) 1.80 (t, J=15.03 Hz, 2 H) 1.51 - 1.67 (m, 4 H) 1.36 - 1.45 (m, 2 H)

### Example C-13: Preparation of 3-(1-(3-(5-((1-(2-(2-(2-(2-(2-(2-(2-(2-(2,6-dioxopiperidin-3-yl)-4-methyl-1-oxo-1,2-dihydrophthalazine-6-yl)amino)ethoxy)ethoxy)acetyl)piperidine-4-il)methoxy)pyrimidine-2-yl)benzyl)6-oxo-1,6-dihydropyridazine-3-yl)benzonitrile (ICT-0001087)

### Step 1) Preparation of 2-(2-(2-((2-(2-(2-(2,6-dioxopiperidin-3-yl)-4-methyl-1-oxo-1,2-dihydroptalazine-6-yl)amino)ethoxy)ethoxy)ethoxy)acetic acid

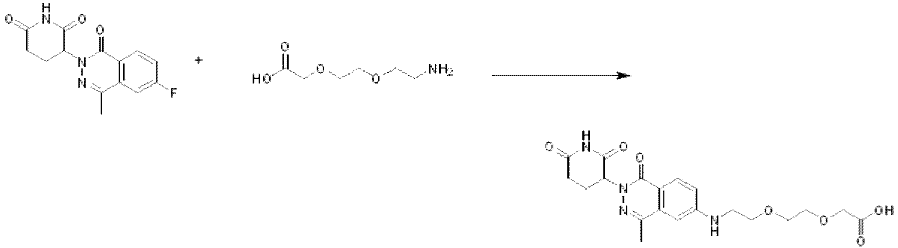

Ethylbis (propane-2-yl)amine (4.0 equivalent) was added to 3-(6-fluoro-4-methyl-1-oxopthalazine-2(1H)-yl) piperidine-2,6-dione (300 mg, 1. mmol) and 2-(2-(2-aminoethoxy)ethoxy) acetic acid (425 mg, 2.18 mmol) solutions in NMP (2.0 ml). The reactants were stirred at 130°C for 48 hours. After the end of the reaction, the reaction mixture was concentrated under reduced pressure. The residues were purified by MeOH/DCM (0-20%) column chromatography. Subjective compound (270 mg) was obtained. MS (ESI, m/z): [M+H]⁺ = 433.4.

### Step 2) Preparation of 3-(1-(3-(5-((1-(2-(2-(2-(2-(2-(2-(2-(2-(2,6-dioxopiperidine-3-yl)-4-methyl-1-oxo-1,2-dihydroptalazine-6-yl)amino)ethoxy)ethoxy)acetyl)piperidine-4-il)methoxy)pyrimidine-2-yl)benzyl)6-oxo-1,6-dihydropyridazine-3-yl)benzonitrile (ICT-0001087)

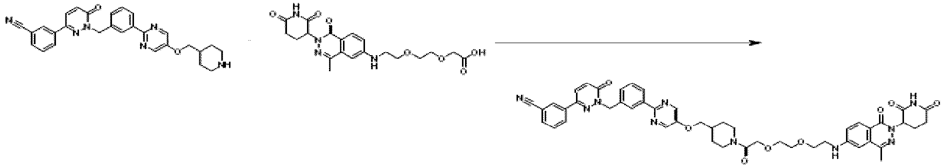

HATU (47.7 mg, 0.13 mmol) was added to 3-(6-oxo-1-(3-(5-(piperidine-4-ylmethoxy)pyrimidine-2-yl)benzyl)1,6-dihydropyridazine-3-yl) benzonitrile (30 mg, 0.06 mmol) and 2-(2-(2-((2-(2-(2-(2-(2,6-dioxopiperidine-3-yl)4-methyl-1-oxo-1,2-dihydropthalazine-6-yl)amino)ethoxy)ethoxy) acetic acid (58.8 mg, 0.06 mmol), followed by ethylbis (propane-2-yl)amine (3 equivalent). It was extracted with ethyl acetate, dried with Magnesium sulfate, and concentrated under reduced pressure. The residues were purified by MeOH/DCM (0-20%) column chromatography to obtain the title compound (40 mg). MS (ESI, m/z): [M+H]⁺ = 893.9.
1H NMR (500 MHz, DMSO-d6) δ ppm 10.93 (s, 1 H) 8.60 - 8.66 (m, 2 H) 8.37 (d, J=5.34 Hz, 2 H) 8.20 - 8.29 (m, 3 H) 8.17 (d, J=9.77 Hz, 1 H) 7.94 (t, J=9.46 Hz, 2 H) 7.71 (t, J=7.86 Hz, 1 H) 7.45 - 7.52 (m, 2 H) 7.09 - 7.18 (m, 2 H) 6.88 (t, J=5.42 Hz, 1 H) 6.72 - 6.79 (m, 1 H) 5.60 - 5.69 (m, 1 H) 5.44 (s, 2 H) 4.37 (d, J=12.36 Hz, 1 H) 4.10 - 4.21 (m, 2 H) 4.04 (d, J=6.26 Hz, 2 H) 3.80 (d, J=13.43 Hz, 1 H) 3.55 - 3.68 (m, 8 H) 3.35 - 3.44 (m, 2 H) 3.10 - 3.19 (m, 2 H) 2.82 - 3.02 (m, 2 H) 2.52 - 2.63 (m, 3 H) 2.41 (s, 3 H) 1.98 - 2.09 (m, 2 H) 1.77 (br. s., 2 H) 1.25 (q, J=7.12 Hz, 17 H)

### Example C-14: Preparation of 3-(1-(3-(5-((1-(2-(2-(2-(2-(2-(2-(2-(2-(2-(2-(2-(2-(2,6-dioxopiperidin-3-yl)-4-methyl-1-oxo-1,2-dihydroptalazine-6-yl)amino)ethoxy)ethoxy)ethoxy)ethoxy)acetyl)piperidine-4-il)methoxy)pyrimidine-2-yl)benzyl)6-oxo-1,6-dihydropyridazine-3-yl)benzonitrile (ICT-0001088)

### Step 1) Preparation of 2-(2-(2-(2-(2-(2-(2-(2-(2-(2,6-dioxopiperidin-3-yl)4-methyl-1-oxo-1,2-dihydroptalazine-6-yl)amino)ethoxy)ethoxy)ethoxy)ethoxy)ethoxy)acetic acid

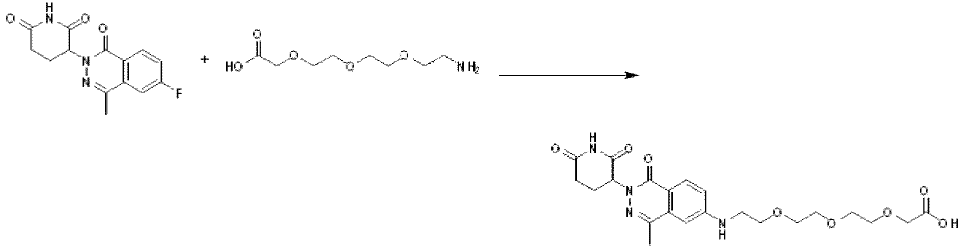

Ethyl bis (propane-2-yl)amine (4.0 equivalent) was added to 3-(6-fluoro-4-methyl-1-oxopthalazine-2(1H)-yl)piperidine-2,6-dione (300 mg, 1. mmol) and 2-(2-(2-(2-(2-aminoethoxy)ethoxy)ethoxy)acetic acid (455 mg, 2.18 mmol) solutions in NMP (2.0 ml). The reactants were stirred at 130°C for 48 hours. After the end of the reaction, the reaction mixture was concentrated under reduced pressure. The residues were purified by MeOH/DCM (0-20%) column chromatography. Subjective compound (290 mg) was obtained. MS (ESI, m/z): [M+H]⁺ = 477.4.

### Step 2) Preparation of 3-(1-(3-(5-((1-(2-(2-(2-(2-(2-(2-(2-(2-(2-(2-(2-(dioxopiperidin-3-yl)-4-methyl-1-oxo-1,2-dihydroptalazine-6-yl)amino)ethoxy)ethoxy)acetyl)piperidine-4-yl)methoxy)pyrimidine-2-yl)benzonitrile (ICT-0001088)

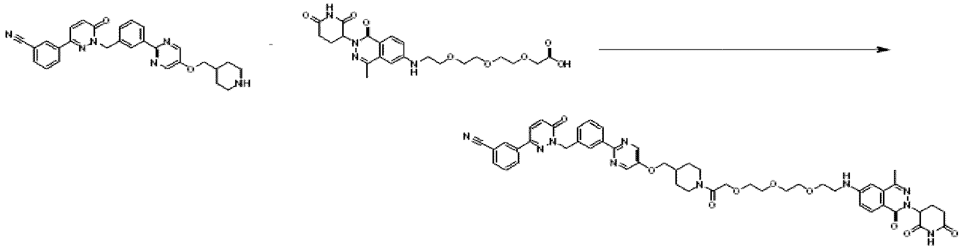

HATU (47.7 mg, 0.13 mmol) was added to the 3-(6-oxo-1-(3-(5-(piperidine-4-ylmethoxy)pyrimidin-2-yl)benzyl)1,6-dihydropyridazine-3-yl) benzonitrile (30 mg, 0.06 mmol) and 2-(2-(2-(2-((2-(2-(2-(2-(2,6-dioxopiperidin-3-yl)-4-methyl-1-oxo-1,2-dihydrophostalamin-6-yl)amino)ethoxy)ethoxy)ethoxy)acetic acid (63.8 mg, 0.06 mmol), followed by ethylbis(propane-2-yl)amine (3 equivalent). The reactant was stirred at room temperature for 6 hours. The reactant was poured into the water. It was extracted with ethyl acetate, dried with Magnesium sulfate, and concentrated under reduced pressure. The residues were purified by MeOH/DCM (0-20%) column chromatography to obtain the title compound (50 mg). MS (ESI, m/z): [M+H]⁺ = 837.9.
1H NMR (500 MHz, DMSO-d6) δ ppm 10.95 (s, 1 H) 8.58 - 8.65 (m, 2 H) 8.35 - 8.39 (m, 2 H) 8.20 - 8.27 (m, 2 H) 8.15 - 8.19 (m, 1 H) 7.90 - 7.96 (m, 2 H) 7.71 (t, J=7.86 Hz, 1 H) 7.45 - 7.52 (m, 2 H) 7.16 (d, J=9.77 Hz, 1 H) 7.10 (dd, J=8.77, 2.06 Hz, 1 H) 6.87 (t, J=5.42 Hz, 1 H) 6.68 - 6.77 (m, 1 H) 5.65 (d, J=7.17 Hz, 1 H) 5.44 (s, 2 H) 4.36 (d, J=12.66 Hz, 1 H) 4.13 - 4.19 (m, 1 H) 4.07 - 4.13 (m, 1 H) 4.04 (d, J=6.10 Hz, 2 H) 3.82 (d, J=12.51 Hz, 1 H) 3.62 (t, J=5.49 Hz, 3 H) 3.51 - 3.58 (m, 8 H) 3.35 - 3.41 (m, 2 H) 3.11 - 3.18 (m, 1 H) 2.83 - 3.05 (m, 2 H) 2.54 - 2.62 (m, 2 H) 2.38 - 2.44 (s, 3 H) 1.93 - 2.07 (m, 2 H) 1.78 (d, J=11.60 Hz, 2 H)

### Example C-15: Preparation of 3-(1-(3-(5-((1-(1-(1-(14-((2-(2,6-dioxopiperidin-3-yl)-4-methyl-1-oxo-1,2-dihydropetalazine-6-yl)amino)-3,6,9,12-tetradecanoyl)piperidine-4-yl)methoxy)pyrimidine-2-yl)benzonitrile-6-oxo-1,6-dihydropyridazine-3-yl)benzonitrile (ICT-0001089)

### Step 1) Preparation of 14-((2-(2,6-dioxopiperidin-3-yl)4-methyl-1-oxo-1,2-dihydroptalazine-6-yl)amino)-3,6,9,12-tetratetradecanoic acid

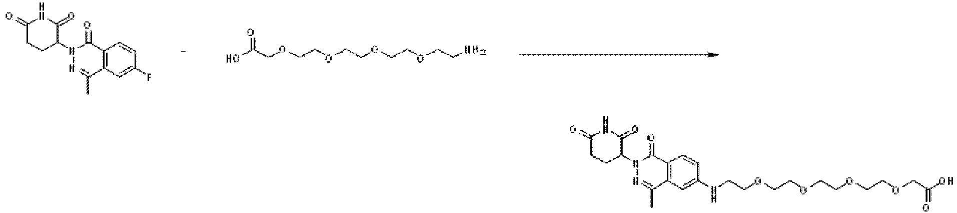

Ethylbis (propane-2-yl)amine (4.0 equivalent) was added to 3-(6-fluoro-4-methyl-1-oxopthalazine-2(1H)-yl)piperidine-2,6-dione (300 mg, 1. mmol) and 14-amino-3,6,9,12-tetratetradecanoic acid (485 mg, 2.18 mmol) solutions in NMP (2.0 ml). The reactants were stirred at 130°C for 48 hours. After the end of the reaction, the reaction mixture was concentrated under reduced pressure. The residues were purified by MeOH/DCM (0-20%) column chromatography to obtain the title compound (290 mg). MS (ESI, m/z): [M+H]⁺ = 521.4.

### Step 2) Preparation of of 3-(1-(3-(5-((1-(14-((2-(2,6-dioxopiperidine-3-yl)-4-methyl-1-oxo-1,2-dihydrophthalazine-6-yl)amino)3,6,9,12-tetratetradecanoyl)piperidine-4-yl)methoxy)pyrimidin-2-yl)benzyl)6-oxo-1,6-dihydropyridazine-3-yl)benzonitrile (ICT-0001089)

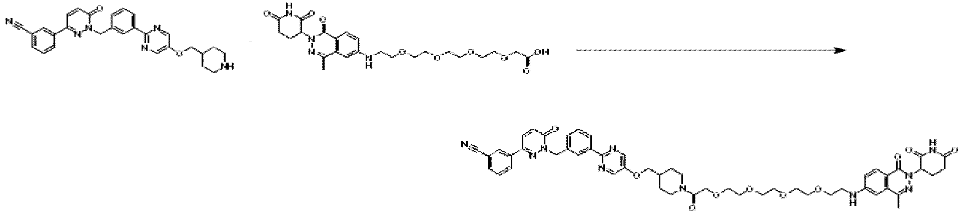

HATU (47.7 mg, 0.13 mmol) was added to 3-(6-oxo-1-(3-(5-(piperidine-4-ylmethoxy)pyrimidin-2-yl)benzyl)1,6-dihydropyridazin-3-yl)benzonitrile (30 mg, 0.06 mmol) and 14-((2-(2-(2,6-dioxopiperidine-3-yl)4-methyl-1-oxo-1,2-dihydroplopthalazine-6-yl)amino)3,6,9,12-tetraoxadenoic acid (68.8 mg, 0.06 mmol), followed by ethylbis (propane-2-yl)amine (3 equivalent). The reactant was stirred at room temperature for 6 hours. The reactant was poured into the water. It was extracted with ethyl acetate, dried with Magnesium sulfate, and concentrated under reduced pressure. The residues were purified by MeOH/DCM (0-20%) column chromatography to obtain the title compound (50 mg). MS (ESI, m/z): [M+H]⁺ = 837.9.
1H NMR (500 MHz, DMSO-d6) δ ppm 10.95 (s, 1 H) 8.58 - 8.65 (m, 2 H) 8.35 - 8.39 (m, 2 H) 8.20 - 8.27 (m, 2 H) 8.15 - 8.19 (m, 1 H) 7.90 - 7.96 (m, 2 H) 7.71 (t, J=7.86 Hz, 1 H) 7.45 - 7.52 (m, 2 H) 7.16 (d, J=9.77 Hz, 1 H) 7.10 (dd, J=8.77, 2.06 Hz, 1 H) 6.87 (t, J=5.42 Hz, 1 H) 6.68 - 6.77 (m, 1 H) 5.65 (d, J=7.17 Hz, 1 H) 5.44 (s, 2 H) 4.36 (d, J=12.66 Hz, 1 H) 4.13 - 4.19 (m, 1 H) 4.07 - 4.13 (m, 1 H) 4.04 (d, J=6.10 Hz, 2 H) 3.82 (d, J=12.51 Hz, 1 H) 3.62 (t, J=5.49 Hz, 3 H) 3.51 - 3.58 (m, 8 H) 3.35 - 3.41 (m, 2 H) 3.11 - 3.18 (m, 1 H) 2.83 - 3.05 (m, 2 H) 2.54 - 2.62 (m, 6 H) 2.38 - 2.44 (s, 3 H) 1.93 - 2.07 (m, 2 H) 1.78 (d, J=11.60 Hz, 2 H)

### Example C-16: Preparation of 3-(1-(3-(5-((1-(1-(1-(1-((2-(2,6-dioxopiperidin-3-yl)-4-methyl-1-oxo-1,2-dihydropetalazine-6-yl)amino)-3,6,9,12-tetraoxapentadecan-15-oil)piperidine-4-yl)methoxy)pyrimidine-2-yl)benzyl)6-oxo-1,6-dihydropyridazine-3-yl)benzonitrile (ICT-0001091)

### Step 1) Preparation of 1-((2-(2-(2,6-dioxopiperidin-3-yl)-4-methyl-1-oxo-1,2-dihydropetalazine-6-yl)amino)-3,6,9,12-tetraoxapentadecan-15-oic acid

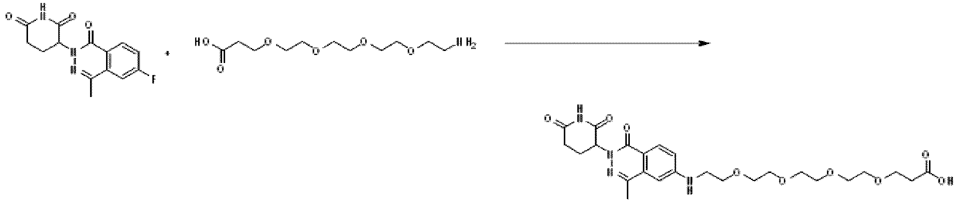

Ethylbis (propane-2-yl)amine (4.0 equivalent) was added to 3-(6-fluoro-4-methyl-1-oxopthalazine-2(1H)-yl)piperidine-2,6-dione (300 mg, 1. mmol) and 1-amino-3,6,9,12-tetraoxapentadecan-15-oic acid (515 mg, 2.18 mmol) in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the title compound (300 mg). MS (ESI, m/z): [M+H]⁺ = 535.4.

### Step 2) Preparation of 3-(1-(3-(5-((1-(1-(1-(2-(2,6-dioxopiperidin-3-yl)-4-methyl-1-oxo-1,2-dihydropthalazine-6-yl)amino)3,6,9,12-tetraoxapentadecan-15-oil)piperidine-4-yl)methoxy)pyrimidine-2-yl)benzyl)6-oxo-1,6-dihydropyridazine-3-yl)benzonitrile (ICT-0001091)

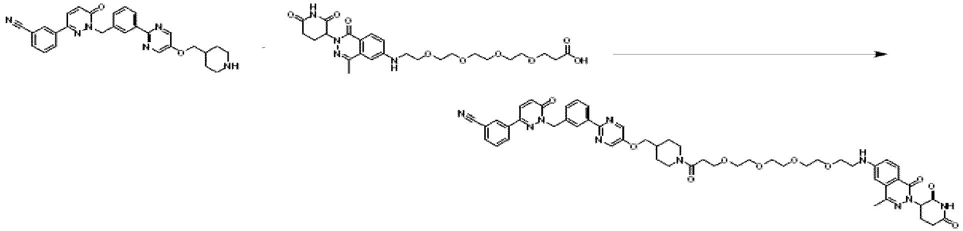

HATU (47.7 mg, 0.13 mmol) was added to 3-(6-oxo-1-(3-(5-(piperidine-4-ylmethoxy)pyrimidine-2-yl)benzyl)-1,6-dihydropyridazin-3-yl) benzonitrile (30 mg, 0.06 mmol) and 1-((2-(2-(2,6-dioxopiperidine-3-yl)4-methyl-1-oxo-1,2-dihydroptalazine-6-yl)amino)-3,6,9,12-tetraoxapentadecan-15-osan (71.8 mg, 0.06 mmol), followed by ethylbis (propane-2-yl)amine (3 equivalent). The reactants were stirred at room temperature for 6 hours. The reactants were poured into water, extracted with ethyl acetate, dried with Magnesium sulfate, and concentrated under reduced pressure. The residues were purified by MeOH/DCM (0-20%) column chromatography to obtain the title compound (50 mg). MS (ESI, m/z): [M+H]+ = 996.1.
1H NMR (500 MHz, DMSO-d6) δ ppm 10.95 (s, 1 H) 8.61 - 8.68 (m, 2 H) 8.38 (br. s., 2 H) 8.23 (t, J=8.39 Hz, 2 H) 8.17 (d, J=9.77 Hz, 1 H) 7.89 - 7.97 (m, 2 H) 7.71 (t, J=7.86 Hz, 1 H) 7.42 - 7.53 (m, 2 H) 7.16 (d, J=9.77 Hz, 1 H) 7.07 - 7.14 (m, 1 H) 6.87 (t, J=5.42 Hz, 1 H) 6.70 - 6.79 (m, 1 H) 5.65 (d, J=6.71 Hz, 1 H) 5.44 (s, 2 H) 4.36 (d, J=12.51 Hz, 1 H) 4.13 - 4.19 (m, 1 H) 4.07 - 4.13 (m, 1 H) 3.98 - 4.07 (m, 2 H) 3.77 - 3.89 (m, 1 H) 3.58 - 3.65 (m, 2 H) 3.45 - 3.58 (m, 12 H) 3.35 - 3.41 (m, 2 H) 2.84 - 3.04 (m, 2 H) 2.52 - 2.66 (m, 3 H) 2.39 - 2.44 (m, 3 H) 2.00 - 2.07 (m, 2 H) 1.96 (d, J=17.40 Hz, 1 H) 1.78 (d, J=11.60 Hz, 2 H)

### Example C-17: Preparation of 3-(1-(3-(5-((1-(4-((4-(4-(2-(2,6-dioxopiperidin-3-yl)-4-methyl-1-oxo-1,2-dihydropthalazine-6-yl)piperazine-1-il)methyl)piperidine-1-carbonyl)piperidine-4-il)methoxy)pyrimidine-2-yl)benzyl)6-oxo-1,6-dihydropyridazine-3-yl)benzonitrile (ICT-0001096) synthesis

### Step 1) Preparation of benzyl 4-((1-(tert-butoxycarbonyl)piperidine-4-yl)methyl)piperazine-1-carboxylate

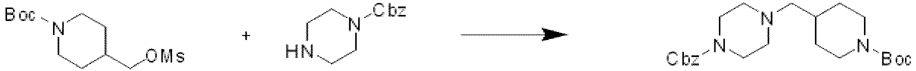

K₂CO₃ (1.41 g, 10.2 mmol) was added to tert-butyl 4-(((methylsulfonyl)oxymethyl)piperidine-1-carboxylate (3 g, 10.2 mmol) and benzyl piperazine-1-carboxylate (4.5 g, 20.5 mmol) solutions in 30 ml DMF, and stirred at 85°C for 16 hours. After cooling, the reactant (50 ml) was poured into water and extracted with ethyl acetate. The extract was dried with Magnesium sulfate and concentrated under reduced pressure. The residue was purified by reversed-phase column chromatography to obtain benzyl 4-(((1-(tert-butoxycarbonyl)piperidine-4-yl)methyl)piperazine-1-carboxylate (1.95 g). MS (ESI, m/z): [M+H]⁺ = 418.2.

### Step 2) Preparation of tert-butyl 4-(piperazine-1-ylmethyl)piperidine-1-carboxylate

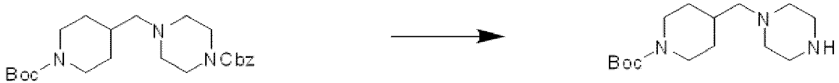

Pd/C (10 wt%, 50 mg) was added to benzyl 4-((1-(tert-butoxycarbonyl)piperidine-4-yl)methyl)piperazine-1-carboxylate (0.5 g, 1.2 mmol) solution in 10 ml MeOH and stirred at room temperature under a hydrogen atmosphere for 4 hours. The solution was filtered in Cellite 545 phase. The solvent was removed under reduced pressure, and tert-butyl 4-(piperazine-1-monomethyl)piperidine-1-carboxylate (0.35 g) was obtained. MS (ESI, m/z): [M+H]⁺ = 284.0.

### Step 3) Preparation of tert-butyl 4-((4-(2-(2,6-dioxopiperidine-3-yl)-4-methyl-1-oxo-1,2-dihydroptalazine-6-yl)piperazine-1-yl)methyl)piperidine-1-carboxylate

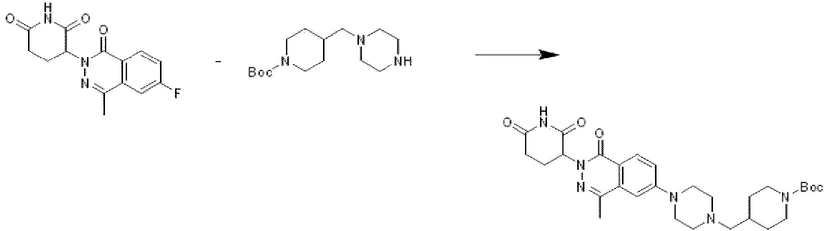

3-(6-fluoro-4-methyl-1-oxopthalazine-2(1H)-yl)piperidine-2,6-dione (1 g, 3.46 mmol), tert-butyl 4-(piperazine-1-monomethyl)piperidine-1-carboxylate (1.96 g, 6.91 mmol), and DIPEA (1.81 ml, 10.4 mmol) solution in NMP (10 mL) were stirred at 120°C for 20 hours. The reaction mixture was purified by reversed-phase column chromatography to obtain tert-butyl 4-((4-(2-(2-(2,6-dioxopiperidin-3-yl)4-methyl-1-oxo-1,2-dihydroptalazine-6-yl)piperazine-1-yl)methylperidine-1-carboxylate (910 mg). MS (ESI, m/z): [M+H]⁺ = 552.3.

### Step 4) Preparation of 3-(4-methyl-1-oxo-6-(4-(piperidine-4-ylmethyl)piperazine-1-yl)phthalazine-2(1H)-yl)piperidine-2,6-dione

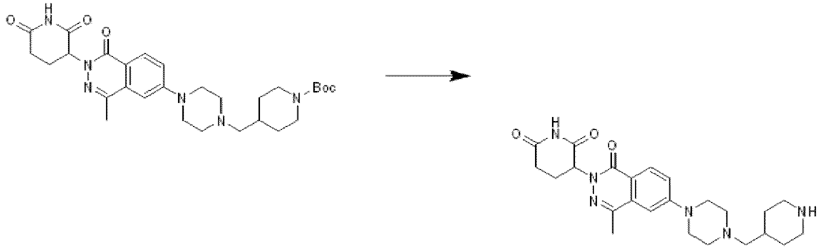

2 ml TFA was added to tert-butyl 4-((4-(2-(2,6-dioxopiperidine-3-yl)-4-methyl-1-oxo-1,2-dihydroptalazin-6-yl)piperazine-1-yl)piperazine-1-yl)methyl)piperidine-1-carboxylate (910 mg, 1.65 mmol) solution in 10 ml DCM and stirred at room temperature for 2 hours. MS (ESI, m/z): [M+H]⁺ = 453.0.

### Step 5) Preparation of 3-(1-(3-(5-((1-(4-((4-(2-(2-(2-(2,6-dioxopiperidine-3-yl)-4-methyl-1-oxo-1,2-dihydroptalazine-6-yl)piperazine-1-yl)methyl-piperidine-1-carbonyl)piperidine-4-yl)methoxy)pyrimidine-2-yl)benzyl)6-oxo-1,6-dihydropyridazin-3-yl)benzonitrile (ICT-0001096)

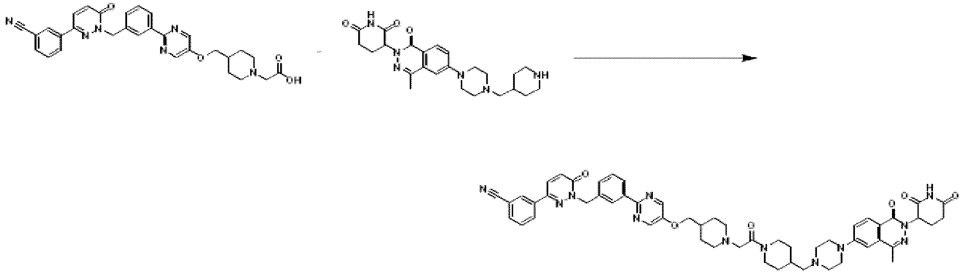

HATU (47.7 mg, 0.13 mmol) was added to 2-(((2-(3-((3-(3-(3-cyanophenyl)-6-oxopridazine-1(6H)-yl)methyl) (6H)-yl)methyl)phenyl)pyrimidine-5-yl)oxy)methyl)piperidine-1-yl)acetic acid (30 mg, 0.06 mmol) and 3-(4-methyl-1-oxo-6-(4-(piperidine-4-ylmethyl)piperazine-1-yl)phthalazine-2(1H)-yl)piperidine-2,6-dione (24 mg, 0.06 mmol) in DMF (2.0 ml), followed by ethylbis (propane-2-yl)amine (3 equivalent). The reactants were stirred at room temperature for 6 hours. It was extracted with ethyl acetate, dried with Magnesium sulfate, and concentrated under reduced pressure. The residues were purified by MeOH/DCM (0-20%) column chromatography to obtain the title compound (25 mg). MS (ESI, m/z): [M+H]⁺ = 972.1.
1H NMR (500 MHz, DMSO-d6) δ ppm 10.97 (s, 1 H) 8.60 - 8.70 (m, 2 H) 8.39 (s, 1 H) 8.37 (s, 1 H) 8.20 - 8.26 (m, 2 H) 8.17 (d, J=9.77 Hz, 1 H) 8.06 (d, J=9.00 Hz, 1 H) 7.92 (d, J=7.63 Hz, 1 H) 7.71 (t, J=7.86 Hz, 1 H) 7.46 - 7.53 (m, 3 H) 7.16 (d, J=9.77 Hz, 1 H) 7.07 (s, 1 H) 5.68 (dd, J=11.75, 4.73 Hz, 1 H) 5.44 (s, 2 H) 4.34 (d, J=12.21 Hz, 1 H) 4.00 - 4.08 (m, 3 H) 3.43 (br. s., 5 H) 2.80 - 3.03 (m, 3 H) 2.53 - 2.67 (m, 4 H) 2.43 - 2.49 (m, 4 H) 2.19 (d, J=6.56 Hz, 2 H) 1.91 - 2.13 (m, 4 H) 1.77 (br. s., 7 H) 1.36 - 1.45 (m, 1 H) 1.32 (d, J=10.68 Hz, 2 H) 1.23 (s, 1 H) 1.08 (d, J=11.44 Hz, 1 H) 0.92 (d, J=10.83 Hz, 1 H)

### Example C-18: Preparation of 3-(1-(3-(5-((1-(2-(4-(3-(2,6-dioxopiperidin-3-yl)-1-methyl-4-oxo-3,4-dihydropetalazine-6-yl)piperazine-1-yl)2-oxoethyl)piperidine-4-il)methoxy)pyrimidine-2-yl)benzyl)6-oxo-1,6-dihydropyridazine-3-yl)benzonitrile (ICT-0001115) synthesis

### Step 1) Preparation of tert-butyl 4-(3-(2,6-dioxopiperidin-3-yl)-1-methyl-4-oxo-3,4-dihydroptalazine-6-yl)piperazine-1-carboxylate

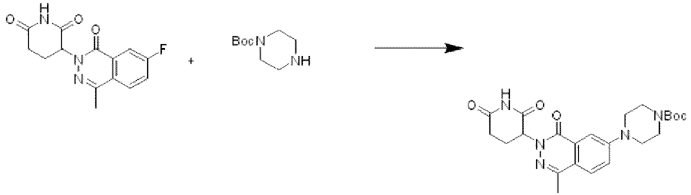

3-(7-fluoro-4-methyl-1-oxopthalazine-2(1H)-yl)piperidine-2,6-dione (1 g, 3.46 mmol), tert-butyl piperazine-1-carboxylate (1.26 g, 6.91 mmol), and DIPEA (1.81 ml, 10.4 mmol) solutions in NMP (10 mL) were stirred at 120°C for 20 hours. The reaction mixture was purified by reversed-phase column chromatography to obtain tert-butyl 4-((4-(2-(2,6-dioxopiperine-3-yl)-4-methyl-1-oxo-1,2-dihydroptalazine-6-yl)piperazine-1-yl)methyl)piperidine-1-carboxylate (910 mg). MS (ESI, m/z): [M+H]⁺ = 456.3.

### Step 2) Preparation of 3-(4-methyl-1-oxo-7-(piperazine-1-yl)phthalazine-2(1H)-yl)piperidine-2,6-dione

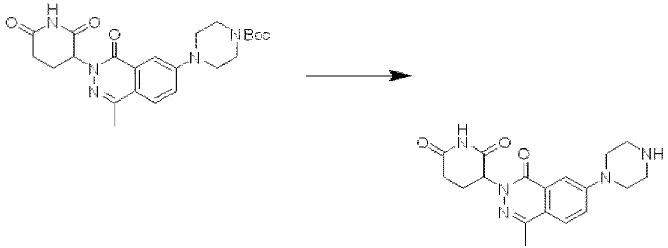

2 ml TFA was added to tert-butyl 4-(2-(2,6-dioxopiperidine-3-yl)-4-methyl-1-oxo-1,2-dihydroptalazine-6-yl) solution in 10 ml DCM, and stirred at room temperature for 2 hours. MS (ESI, m/z): [M+H]⁺ = 356.3.

### Step 3) Preparation of 3-(1-(3-(5-((1-(2-(4-(3-(2-(3-(2,6-dioxopiperidine-3-yl)-1-methyl-4-oxo-3,4-dihydropthalazine-6-yl)piperazine-1-il)-2-oxoethyl)piperidine-4-il)methoxy)pyrimidine-2-yl)benzyl)6-oxo-1,6-dihydropyridazine-3-yl)benzonitrile (ICT-0001115)

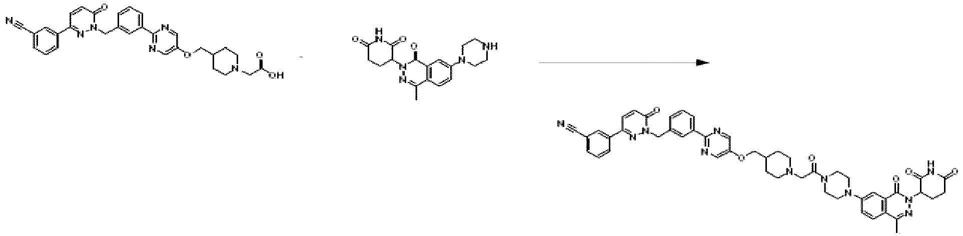

HATU (47.7 mg, 0.13 mmol) was added to 2-(((2-(3-((3-(3-cyanophenyl)-6-oxopridazine-1(6H)-yl)methyl)phenyl)pyrimidine-5-yl)oxy)methyl)piperidine-1-yl) acetic acid (30 mg, 0.06 mmol) and 3-(4-methyl-1-oxo-7-(piperazine-1-yl)phthalazine-2,6-dione (20 mg, 0.06 mmol) in DMF (2.0 ml), followed by ethylbis (propane-2-yl)amine (3 equivalent). The reactants were stirred at room temperature for 6 hours. The reactants were poured into the water. It was extracted with ethyl acetate, dried with Magnesium sulfate, and concentrated under reduced pressure. The residues were purified by MeOH/DCM (0-20%) column chromatography to obtain the title compound (20 mg). MS (ESI, m/z): [M+H]⁺ = 874.9.
1H NMR (500 MHz, DMSO-d6) δ ppm 10.93 (s, 1 H) 8.53 - 8.61 (m, 2 H) 8.26 - 8.35 (m, 2 H) 8.13 - 8.20 (m, 2 H) 8.08 - 8.13 (m, 1 H) 7.86 (d, J=7.70 Hz, 1 H) 7.74 (d, J=8.93 Hz, 1 H) 7.64 (t, J=7.89 Hz, 1 H) 7.55 (dd, J=8.99, 2.51 Hz, 1 H) 7.48 (d, J=2.45 Hz, 1 H) 7.35 - 7.45 (m, 2 H) 7.05 - 7.13 (m, 1 H) 5.60 - 5.72 (m, 2 H) 5.37 (s, 2 H) 3.97 (d, J=5.87 Hz, 2 H) 3.70 (br. s., 1 H) 3.57 (br. s., 1 H) 3.40 (br. s., 2 H) 3.07 - 3.19 (m, 2 H) 2.78 - 2.89 (m, 2 H) 2.46 - 2.58 (m, 2 H) 2.36 - 2.42 (m, 3 H) 1.92 - 2.05 (m, 2 H) 1.71 (d, J=9.29 Hz, 2 H) 1.19 - 1.32 (m, 2 H) 1.06 (d, J=6.60 Hz, 1 H)

### Example C-19: Preparation of 3-(1-(4-(5-((1-(2-(2-(2-(2-(2-(2,6-dioxopiperidin-3-yl)-4-methyl-1-oxo-1,2-dihydropetalazine-6-yl)piperazine-1-il)acetyl)piperidine-4-il)methoxy)pyrimidine-2-il)benzyl)6-oxo-1,6-dihydropyridazine-3-yl)benzonitrile (ICT-001173)

### Step 1) Preparation of tert-butyl 4-(2-(2,6-dioxopiperidin-3-yl)-4-methyl-1-oxo-1,2-dihydroptalazine-6-yl)piperazine-1-carboxylate

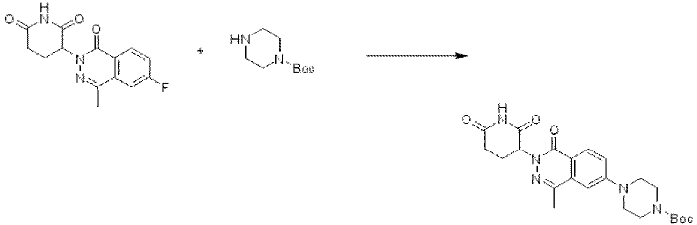

Ethylbis (propane-2-yl)amine (4.0 equivalent) was added to (3.0 ml) 3-(6-fluoro-4-methyl-1-oxopthalazine-2(1H)-yl) piperidine-2,6-dione (300 mg, 1.04 mmol) and tert-butylpiperazine-1-carboxylate (386 mg, 2.07 mmol) solutions in NMP. The reactants were stirred at 130°C for 48 hours. After the reaction was completed, the reaction mixture was concentrated under reduced pressure. The residues were purified by MeOH/DCM (0-20%) column chromatography to obtain the title compound (350 mg). MS (ESI, m/z): [M+H]⁺ = 456.4

### Step 2) Preparation of 3-(4-methyl-1-oxo-6-(piperazine-1-yl)phthalazine-2(1H)-yl)piperidine-2,6-dione

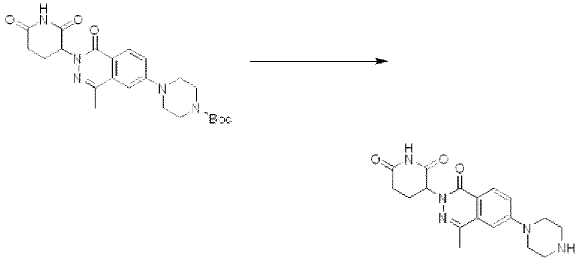

TFA (10.0 ml) was added to tert-butyl 4-(2-(2,6-dioxopiperidine-3-yl)-4-methyl-1-oxo-1,2-dihydroptalazine-6-yl) solution in DCM (30.0 ml). TFA (10.0 ml) was added to the solution of piperazine-1-carboxylate (300 mg, 0.71 mmol). The reactants were stirred at room temperature for 1 hour. After the end of the reaction, the reaction mixture was concentrated under reduced pressure. The residues were purified by MeOH/DCM (0-20%) column chromatography to obtain the title compound (200 mg). MS (ESI, m/z): [M+H]⁺ = 356.4

### Step 3) Preparation of tert-butyl 2-(4-(2-(2,6-dioxopiperidin-3-yl)-4-methyl-1-oxo-1,2-dihydroptalazine-6-yl)piperazine-1-yl)acetate

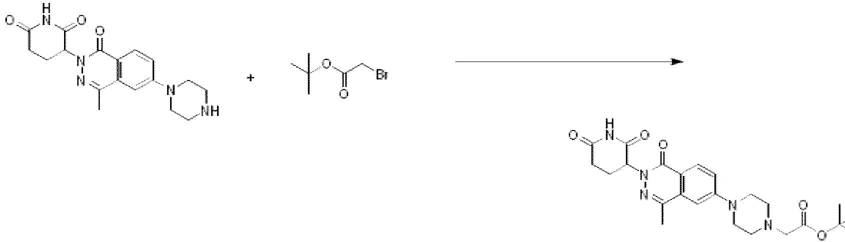

Ethylbis (propane-2-yl)amine (2.0 equivalent) was added to 3-(4-methyl-1-oxo-6-(piperazine-1-yl)phthalazine-2(1H)-yl)piperidine-2,6-dione (200 mg, 0.56 mmol) and tert-butyl 2-bromoacetate (131 mg, 0.62 mmol) solutions in ACN (10.0 ml). The reactants were stirred at room temperature for 5 hours. After the reaction was completed, the reaction mixture was concentrated under reduced pressure. The residues were purified by MeOH/DCM (0-20%) column chromatography, and the headline compound (210 mg) was obtained MS (ESI, m/z): [M+H]⁺ = 470.4

### Step 4) Preparation of 2-(4-(2-(2,6-dioxopiperidin-3-yl)-4-methyl-1-oxo-1,2-dihydroptalazine-6-yl)piperazine-1-yl)acetic acid

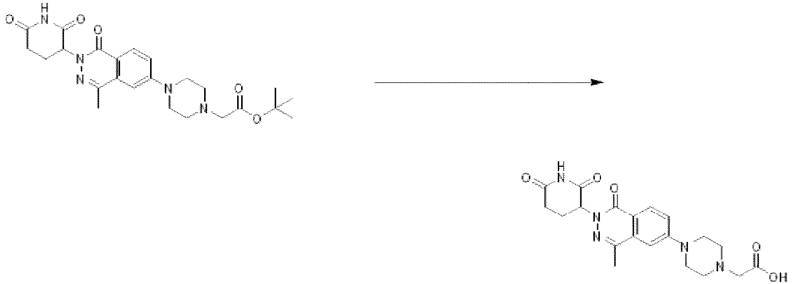

TFA (1.0 ml) was added to tert-butyl 2-(4-(2-(2,6-dioxopiperidin-3-yl)-4-methyl-1-oxo-1,2-dihydroptallazine-6-yl)piperazine-1-yl) acetate (30 mg, 0.06 mmol) in DCM (3.0 ml). The reactants were stirred at room temperature for 1 hour. After the reaction was completed, the reaction mixture was concentrated under reduced pressure. The residues were purified by MeOH/DCM (0-20%) column chromatography to obtain the title compound (15 mg). MS (ESI, m/z): [M+H]⁺ = 414.4

### Step 5) Preparation of 3-(1-(4-(5-((1-(2-(4-(4-(2-(2-(2-(2-(2,6-dioxopiperidin-3-yl)-4-methyl-1-oxo-1,2-dihydropthalazine-6-yl)piperazine-1-il)acetyl)piperidine-4-il)methoxy)pyrimidine-2-yl)benzyl)-6-oxo-1,6-dihydropyridazine-3-yl)benzonitrile (ICT-001173)

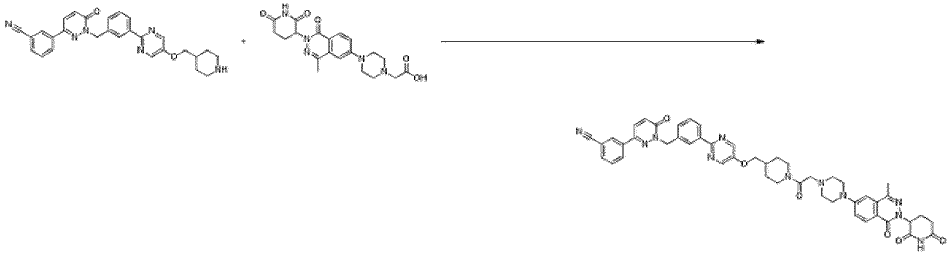

HATU (47.7 mg, 0.13 mmol) was added to 3-{6-oxo-1-[(3-{5-[(piperidine-4-yl)methoxy]pyrimidin-2-yl}phenyl)methyl]-1,6-dihydropyridazine-3-yl} benzonitrile (30 mg, 0.06 mmol) and 2-(4-(2-(2-(2,6-dioxopiperidine-3-yl)4-methyl-1-oxo-1,2-dihydroptalazin-6-yl)piperazine-1-yl)acetic acid (20.6 mg, 0.06 mmol) in DMF (2.0 ml), followed by ethylbis (propane-2-yl)amine (3 equivalent). The reactants were stirred at room temperature for 6 hours. The reactants were poured into water, extracted with ethyl acetate, dried with Magnesium sulfate, and concentrated under reduced pressure. The residue was purified by MeOH/DCM (0-20%) column chromatography to obtain the title compound (40 mg). MS (ESI, m/z): [M+H]+ = 874.9.
1H NMR (500 MHz, DMSO-d6) δ ppm 10.91 (s, 1 H) 8.54 - 8.62 (m, 2 H) 8.31 (s, 2 H) 8.13 - 8.20 (m, 2 H) 8.10 (d, J=9.77 Hz, 1 H) 8.00 (d, J=8.85 Hz, 1 H) 7.86 (d, J=7.63 Hz, 1 H) 7.64 (t, J=7.86 Hz, 1 H) 7.37 - 7.49 (m, 3 H) 7.09 (d, J=9.77 Hz, 1 H) 7.02 (br. s., 1 H) 5.61 (d, J=6.56 Hz, 1 H) 5.37 (s, 2 H) 4.35 (d, J=12.36 Hz, 1 H) 3.60 - 4.24 (m, 1 H) 4.01 (d, J=6.10 Hz, 2 H) (3.42 - 3.52 (m, 2 H) 3.39 (br. s., 2 H) 3.33 - 3.37 (m, 1 H) 3.12 (s, 1 H) 2.94 - 3.04 (m, 1 H) 2.79 - 2.90 (m, 1 H) 2.45 - 2.64 (m, 8 H) 2.38 - 2.44 (s, 3 H) 1.94 - 2.04 (m, 1 H) 1.81 - 1.94 (m, 2 H) 1.76 (br. s., 2 H)

### Example C-20: Preparation of 3-(1-(3-(5-((1-(2-(2-(2-(2-(2-(2,6-dioxopiperidin-3-yl)-4-methyl-1-oxo-1,2-dihydroptalazine-6-yl)piperazine-1-il)acetyl)piperidine-4-il)methoxy)pyrimidine-2-yl)benzyl)6-oxo-1,6-dihydropyridazine-3-yl)benzonitrile (ICT-001174)

### Step 1) Preparation of tert-butyl 3-(4-(2-(2,6-dioxopiperidin-3-yl)-4-methyl-1-oxo-1,2-dihydroptalazine-6-yl)piperazine-1-yl)propanoate

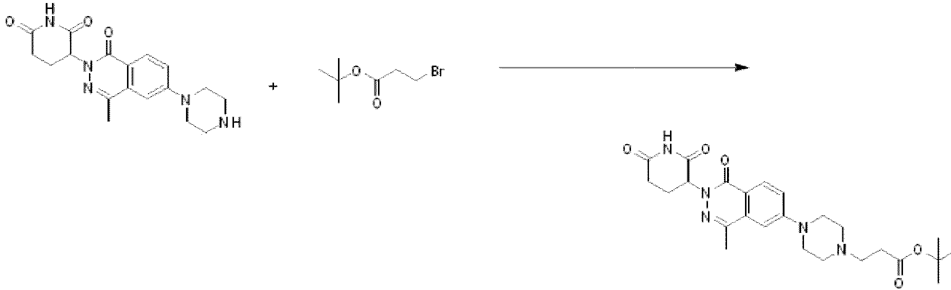

Ethylbis (propane-2-yl)amine (2.0 equivalent) was added to 3-(4-methyl-1-oxo-6-(piperazine-1-yl)phthalazine-2(1H)-yl)piperidine-2,6-dione (200 mg, 0.56 mmol) and tert-butyl 3-bromopropanoate (140 mg, 0.62 mmol) solutions in ACN (10.0 ml). The reactants were stirred at room temperature for 5 hours. After the end of the reaction, the reaction mixture was concentrated under reduced pressure. The residues were purified by MeOH/DCM (0-20%) column chromatography to obtain the heading compound (210 mg) MS (ESI, m/z): [M+H]⁺ = 484.4

### Step 2) Preparation of 3-(4-(2-(2,6-dioxopiperidin-3-yl)-4-methyl-1-oxo-1,2-dihydroptalazine-6-yl)piperazine-1-yl)profanoic acid

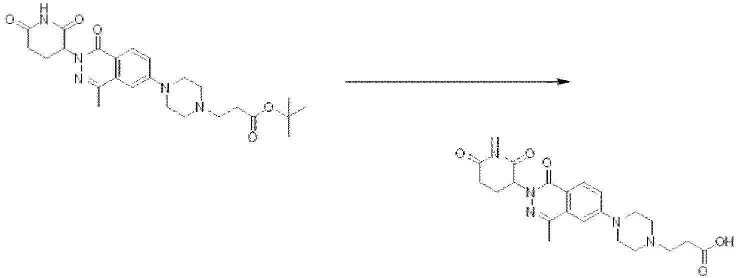

TFA (1.0 ml) was added to tert-butyl 3-(4-(2-(2,6-dioxopiperidin-3-yl)-4-methyl-1-oxo-1,2-dihydroptalazine-6-yl)piperazine-1-yl) propanoate (40 mg, 0.08 mmol) in DCM (3.0 ml). The reactants were stirred at room temperature for 1 hour. After the reaction was completed, the reaction mixture was concentrated under reduced pressure. The residues were purified by MeOH/DCM (0-20%) column chromatography to obtain the title compound (25 mg). MS (ESI, m/z): [M+H]⁺ = 428.4

### Step 3) Preparation of 3-(1-(3-(5-((1-(3-(4-(4-(2-(2,6-dioxopiperidine-3-yl)-4-methyl-1-oxo-1,2-dihydropthalazine-6-yl)piperazine-1-il)propanoyl-4-il)methoxy)pyrimidine-2-yl)benzyl)6-oxo-1,6-dihydropyridazine-3-yl)benzonitrile (ICT-001174)

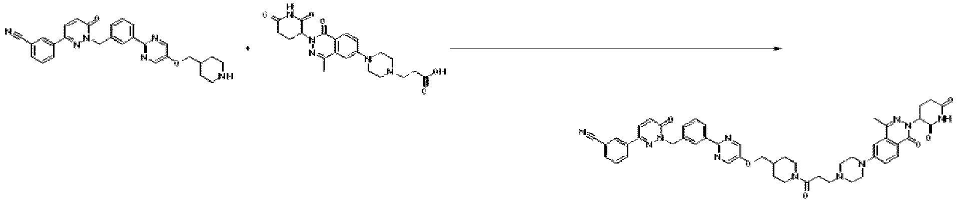

HATU (47.7 mg, 0.13 mmol) was added to 3-{6-oxo-1-[(3-{5-[(piperidine-4-yl)methoxy]pyrimidin-2-yl}phenyl)methyl]-1,6-dihydropyridazine-3-yl} benzonitrile (30 mg, 0.06 mmol) and 3-(4-(4-(2-(2-(2,6-dioxopiperidine-3-yl)4-methyl-1-oxo-1,2-dihydroptalazine-6-yl)piperazine-1-yl)propanoic acid (20.6 mg, 0.06 mmol) in DMF (2.0 ml), followed by ethylbis (propane-2-yl)amine (3 equivalent). The reactants were stirred at room temperature for 6 hours. The reactants were poured into water, extracted with ethyl acetate, dried with Magnesium sulfate, and concentrated under reduced pressure. The residue was purified by MeOH/DCM (0-20%) column chromatography to obtain the title compound (40 mg). MS (ESI, m/z): [M+H]+ = 889.1.
1H NMR (500 MHz, DMSO-d6) δ 10.98 (s, 1 H) 8.61 - 8.68 (m, 2 H) 8.38 (s, 2 H) 8.24 (t, J=8.24 Hz, 2 H) 8.18 (d, J=9.77 Hz, 1 H) 8.07 (d, J=9.00 Hz, 1 H) 7.90 - 7.97 (m, 1 H) 7.72 (t, J=7.86 Hz, 1 H) 7.44 - 7.54 (m, 3 H) 7.17 (d, J=9.77 Hz, 1 H) 7.09 (s, 1 H) 5.68 (d, J=6.71 Hz, 1 H) 5.45 (s, 2 H) 4.44 (d, J=13.73 Hz, 1 H) 4.08 (d, J=6.10 Hz, 2 H) 3.98 (d, J=13.28 Hz, 1 H) 3.37 - 3.48 (m, 4 H) 3.06 (t, J=12.74 Hz, 1 H) 2.85 - 2.96 (m, 2 H) 2.52 - 2.67 (m, 12 H) 2.38 - 2.44 (s, 3 H) 2.03 - 2.13 (m, 1 H) 1.90 - 2.00 (m, 1 H) 1.84 (d, J=12.36 Hz, 1 H) 1.78 (br. s., 1 H)

### Example C-21: Preparation of 3-(1-(3-(5-((1-(2-(4-(2-(2-(2,6-dioxopiperidin-3-yl)-4-methyl-1-oxo-1,2-dihydropetthalazine-6-yl)piperazine-1-il)acetyl)piperidine-4-il)methoxy)pyrimidine-2-yl)benzyl)6-oxo-1,6-dihydropyridazine-3-yl)benzonitrile (ICT-001175)

### Step 1) Preparation of tert-butyl 4-(4-(2-(2,6-dioxopiperidin-3-yl)-4-methyl-1-oxo-1,2-dihydroptalazine-6-yl)piperazine-1-yl)butanoate

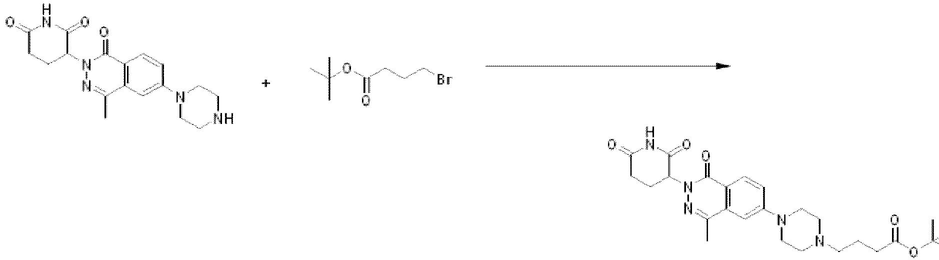

Ethylbis (propane-2-yl)amine (2.0 equivalent) was added to 3-(4-methyl-1-oxo-6-(piperazine-1-yl)phthalazine-2(1H)-yl)piperidine-2,6-dione (200 mg, 0.56 mmol) and tert-butyl 4-bromobutanoate (145 mg, 0.62 mmol) solutions in ACN (10.0 ml). The reactants were stirred at room temperature for 5 hours. After the reaction was completed, the reaction mixture was concentrated under reduced pressure. The residues were purified by MeOH/DCM (0-20%) column chromatography to obtain the heading compound (210 mg) MS (ESI, m/z): [M+H]⁺ = 498.4

### Step 2) Preparation of 4-(4-(2-(2,6-dioxopiperidin-3-yl)-4-methyl-1-oxo-1,2-dihydroptalazine-6-yl)piperazine-1-yl)butanoic acid

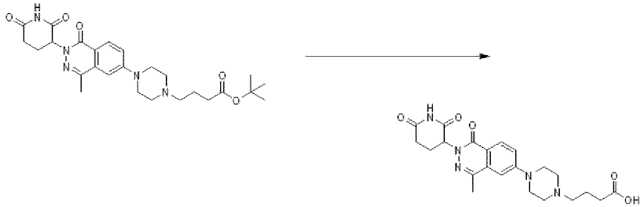

TFA (1.0 ml) was added to tert-butyl 4-(4-(2-(2,6-dioxopiperidine-3-yl)-4-methyl-1-oxo-1,2-dihydroptalazine-6-yl)piperazine-1-yl)butanoate (40 mg, 0.08 mmol) in DCM (3.0 ml). The reactants were stirred at room temperature for 1 hour. After the reaction was completed, the reaction mixture was concentrated under reduced pressure. The residues were purified by MeOH/DCM (0-20%) column chromatography to obtain the title compound (25 mg). MS (ESI, m/z): [M+H]⁺ = 442.4

### Step 3) Preparation of 3-(1-(3-(5-((1-(4-(4-(2-(2,6-dioxopiperidine-3-yl)-4-methyl-1-oxo-1,2-dihydropthalazine-6-yl)piperazine-1-yl)butanoyl)piperidine-4-il)methoxy)pyrimidine-2-yl)benzyl)6-oxo-1,6-dihydropyridazine-3-yl)benzonitrile (ICT-001175)

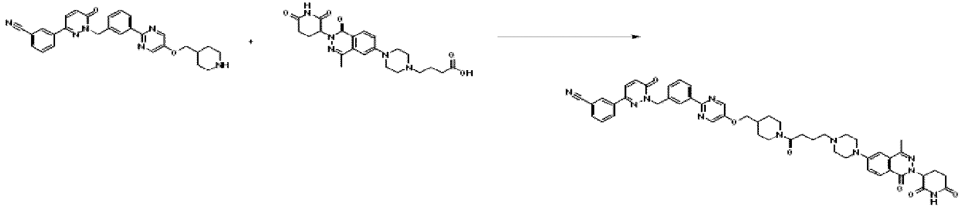

HATU (47.7 mg, 0.13 mmol) was added to DMF (2.0 ml) of 3-(6-oxo-1-(3-(5-(piperidine-4-ylmethoxy)pyrimidin-2-yl)benzyl)-1,6-dihydropyridazine-3-yl) benzonitrile (30 mg, 0.06 mmol) and 4-(4-(2-(2-(2-(2-(2-(2-dioxopiperidine-3-yl)4-methyl-1-oxo-1,2-dihydroptalazine-6-yl)piperazine-1-yl)butanoic acid (22.6 mg, 0.06 mmol) solution, followed by ethylbis (propane-2-yl)amine (3 equivalent). The reactants were stirred at room temperature for 6 hours. The reactants were poured into water, extracted with ethyl acetate, dried with Magnesium sulfate, and concentrated under reduced pressure. The residue was purified by MeOH/DCM (0-20%) column chromatography to obtain the title compound (40 mg). MS (ESI, m/z): [M+H]+ = 903.1.
1H NMR (500 MHz, DMSO-d6) δ ppm 10.91 (s, 1 H) 8.54 - 8.60 (m, 2 H) 8.31 (br. s., 2 H) 8.13 - 8.20 (m, 2 H) 8.11 (d, J=9.77 Hz, 1 H) 7.99 (d, J=9.00 Hz, 1 H) 7.86 (d, J=7.48 Hz, 1 H) 7.65 (t, J=7.86 Hz, 1 H) 7.37 - 7.47 (m, 3 H) 7.10 (d, J=9.77 Hz, 1 H) 7.01 (br. s., 1 H) 5.61 (d, J=6.87 Hz, 1 H) 5.38 (s, 2 H) 4.38 (d, J=12.51 Hz, 1 H) 4.00 (d, J=6.10 Hz, 2 H) 3.87 (d, J=12.36 Hz, 1 H) 3.37 (br. s., 3 H) 2.97 (t, J=11.98 Hz, 1 H) 2.76 - 2.89 (m, 1 H) 2.46 - 2.59 (m, 6 H) 2.41 (s, 4 H) 2.30 (t, J=6.79 Hz, 3 H) 1.94 - 2.04 (m, 3 H) 1.83 - 1.94 (m, 3 H) 1.61 - 1.79 (m, 4 H)

### Example C-22: Preparation of 3-(1-(3-(5-((1-(5-(5-(5-(5-(4-(2-(2,6-dioxopiperidin-3-yl)-4-methyl-1-oxo-1,2-dihydrophthalazine-6-yl)piperazine-1-il)pentanoyl)piperidine-4-il)methoxy)pyrimidin-2-yl)benzyl)-6-oxo-1,6-dihydropyridazine-3-yl)benzonitrile (ICT-001176)

### Step 1) Preparation of tert-butyl 5-(4-(2-(2,6-dioxopiperidine-3-yl)-4-methyl-1-oxo-1,2-dihydroptalazine-6-yl)piperazine-1-yl)pentanoate

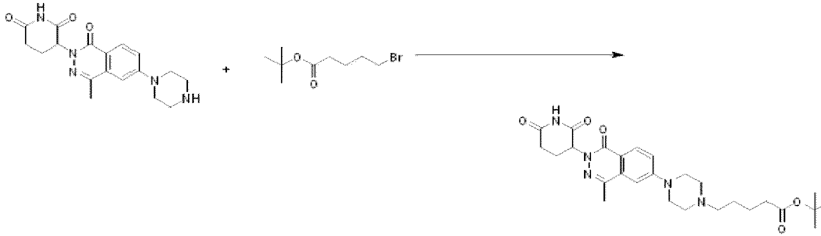

Ethylbis (propane-2-yl)amine (2.0 equivalent) was added to 3-(4-methyl-1-oxo-6-(piperazine-1-yl)phthalazine-2(1H)-yl) piperidine-2,6-dione (200 mg, 0.56 mmol) and tert-butyl 5-bromopentanoate (145 mg, 0.62 mmol) solutions in ACN (10.0 ml). The reactants were stirred at room temperature for 5 hours. After the reaction was completed, the reaction mixture was concentrated under reduced pressure. The residues were purified by MeOH/DCM (0-20%) column chromatography to obtain the title compound (250 mg) MS (ESI, m/z): [M+H]⁺ = 512.4

### Step 2) Preparation of 5-(4-(2-(2,6-dioxopiperidin-3-yl)-4-methyl-1-oxo-1,2-dihydroptalazine-6-yl)piperazine-1-yl)pentanoic acid

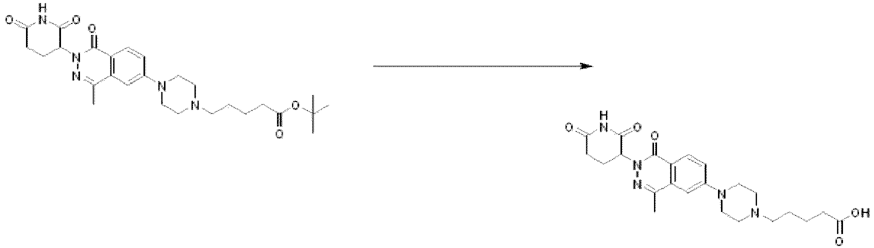

TFA (1.0 ml) was added to tert-butyl 5-(4-(2-(2,6-dioxopiperidine-3-yl)-4-methyl-1-oxo-1,2-dihydroptalazine-6-yl)piperazine-1-yl) pentanoate (40 mg, 0.08 mmol) in DCM (3.0 ml). The reactants were stirred at room temperature for 1 hour. After the reaction was completed, the reaction mixture was concentrated under reduced pressure. The residues were purified by MeOH/DCM (0-20%) column chromatography to obtain the title compound (28 mg). MS (ESI, m/z): [M+H]⁺ = 456.4

### Step 3) Preparation of 3-(1-(3-(5-((1-(5-(1-(5-(4-(2-(2,6-dioxopiperidine-3-yl)-4-methyl-1-oxo-1,2-dihydropthalazine-6-yl)piperazine-1-il)pentanoyl)piperidine-4-il)methoxy)pyrimidine-2-yl)benzyl)6-oxo-1,6-dihydropyridazine-3-yl)benzonitrile (ICT-001176)

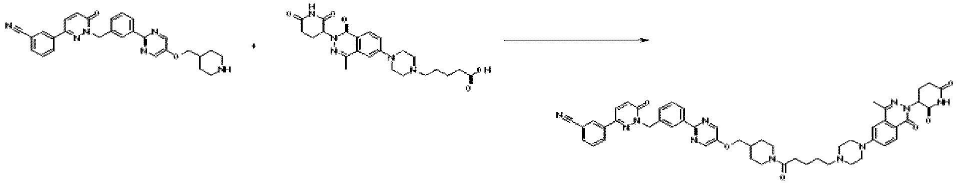

HATU (47.7 mg, 0.13 mmol) was added to 3-(6-oxo-1-(3-(5-(piperidine-4-ylmethoxy)pyrimidin-2-yl)benzyl)-1,6-dihydropyridazin-3-yl) benzonitrile (30 mg, 0.06 mmol) and 5-(4-(4-(2-(2-(2-(2-(2-dioxopiperidine-3-yl)4-methyl-1-oxo-1,2-dihydropthalazine-6-yl)piperazine-1-yl)pentanoic acid (23 mg, 0.06 mmol) in a solution, followed by ethylbis (propane-2-yl)amine (3 equivalent). The reactants were stirred at room temperature for 6 hours. The reactants were poured into water, extracted with ethyl acetate, dried with Magnesium sulfate, and concentrated under reduced pressure. The residue was purified by MeOH/DCM (0-20%) column chromatography to obtain the title compound (35 mg). MS (ESI, m/z): [M+H]⁺ = 917.1.
1H NMR (500 MHz, DMSO-d6) δ ppm 10.91 (s, 1 H) 8.53 - 8.61 (m, 2 H) 8.31 (br. s., 2 H) 8.17 (t, J=8.93 Hz, 2 H) 8.11 (d, J=9.77 Hz, 1 H) 8.02 (d, J=8.70 Hz, 1 H) 7.86 (d, J=7.78 Hz, 1 H) 7.65 (t, J=7.86 Hz, 1 H) 7.37 - 7.50 (m, 3 H) 7.01 - 7.11 (m, 2 H) 5.62 (d, J=7.32 Hz, 1 H) 5.37 (s, 2 H) 4.37 (d, J=12.82 Hz, 1 H) 4.00 (d, J=6.26 Hz, 2 H) 3.86 (d, J=12.97 Hz, 1 H) 3.49 - 3.58 (m, 1 H) 3.44 (br. s., 1 H) 3.31 - 3.39 (m, 2 H) 3.06 (dd, J=7.25, 4.20 Hz, 1 H) 2.97 (t, J=12.21 Hz, 1 H) 2.77 - 2.90 (m, 1 H) 2.46 - 2.58 (m, 6 H) 2.42 (br. s., 5 H) 2.30 (br. s., 2 H) 1.95 - 2.05 (m, 2 H) 1.67 - 1.80 (m, 2 H) 1.48 (br. s., 2 H)1.11 - 1.26 (m, 4 H)

### Example C-23: Preparation of 3-(1-{[3-(5-{[1-(2-{4-[2-(2,6-dioxopiperidin-3-yl)-4-methyl-1-oxo-1,2-dihydropthalazine-6-yl]piperazine-1-yl}-2-oxoethyl)piperidine-4-yl]methoxy}pyrimidine-2-yl)phenyl]methyl}-6-oxo-1,6-dihydropyridazine-3-yl)benzonitrile (ICT-0001177)

### Step 1) Preparation of tert-butyl 2-(4-(((2-(3-((3-(3-cyanophenyl)-6-oxopyridazin-1(6H)-yl)methyl)phenyl)pyrimidine-5-il)oxy)methyl)piperidine-1-il)acetate

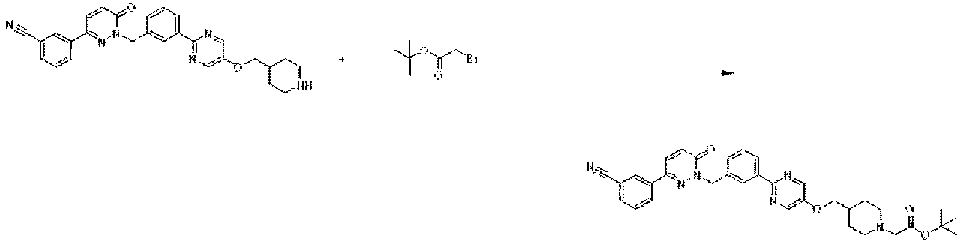

Ethylbis (propane-2-yl)amine (2.0 equivalent) was added to 3-(6-oxo-1-(3-(5-(piperidine-4-ylmethoxy)pyrimidin-2-yl)benzyl)benzonitrile (200 mg, 0.42 mmol) and tert-butyl 2-bromoacetate (82.0 mg, 0.42 mmol) in ACN (5 ml). The reactants were stirred at room temperature for 5 hours. After the reaction was completed, the reaction mixture was concentrated under reduced pressure. The residues were purified by MeOH/DCM (0-20%) column chromatography. Subject compound (210 mg) MS (ESI, m/z): [M+H]⁺ = 593.7

### Step 2) Preparation of 2-[4-({[2-(3-{[3-(3-cyanophenyl)-6-oxo-1,6-dihydropyridazine-1-yl]methyl}phenyl)pyrimidine-5-il]oxy}methyl)piperidine-1-il]acetic acid

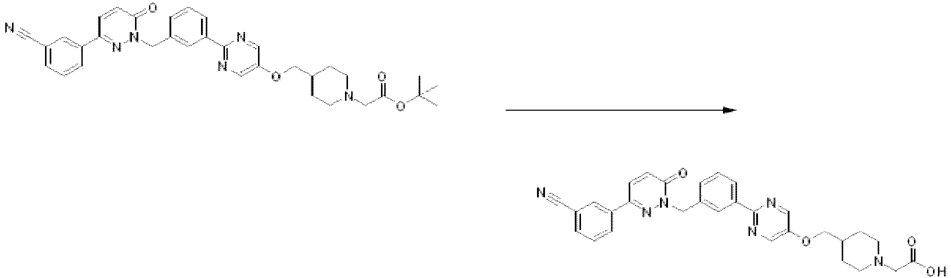

TFA (3ml) was added to tert-butyl 2-(4-(((2-(3-((3-(3-(3-(3-cyanophenyl)-6-oxopridazine-1(6H)-yl)methyl)phenyl)pyrimidine-5-yl)oxy)methyl)piperidine-1-yl)acetate (200 mg, 0.38 mmol) in DCM (9ml). The reactants were stirred at room temperature for 1 hour. After the reaction was completed, the reaction mixture was concentrated under reduced pressure. The residues were purified by MeOH/DCM (0-20%) column chromatography to obtain the title compound (130 mg) MS (ESI, m/z): [M+H]⁺ = 537.6

### Step 3) Preparation of 3-(1-{[3-(5-{[1-(2-{4-[2-(2,6-dioxopiperidin-3-yl)-4-methyl-1-oxo-1,2-dihydropetalazine-6-yl]piperazine-1-yl}-2-oxoethyl)piperidine-4-yl]methoxy}pyrimidine-2-yl)phenyl]methyl}-6-oxo-1,6-dihydropyridazine-3-yl)benzonitrile (ICT-0001177)

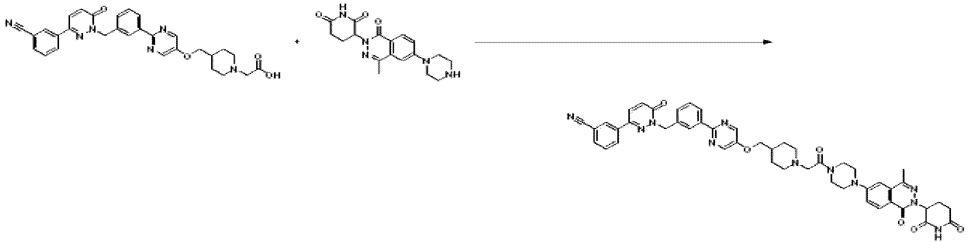

HATU (42.5 mg, 0.12 mmol) was added to 2-[4-({[2-(3-{3-(3-cyanophenyl)-6-oxo-1,6-dihydropyridazin-1-yl]methyl}phenyl)pyrimidine-5-yl]oxy}methyl)piperidine-1-yl]acetic acid (30 mg, 0.056 mmol) and 3-[4-methyl-1-oxo-6-(piperazine-1-yl)-1,2-dihydroptalazin-2-yl]piperidine-2,6-dione (19.9 mg, 0.05 mmol) in DMF (2.0 ml), followed by ethylbis (propane-2-yl)amine (3 equivalent). The reactants were stirred at room temperature for 6 hours. The reactants were poured into the water. It was extracted with ethyl acetate, dried with Magnesium sulfate, and concentrated under reduced pressure. The residue was purified by MeOH/DCM (0-20%) column chromatography to obtain the title compound (20 mg). MS (ESI, m/z): [M+H]⁺ = 874.9.
1H NMR (500 MHz, DMSO-d6) δppm 10.91 (s, 1 H) 8.57 (s, 2 H) 8.31 (s, 2 H) 8.13 - 8.20 (m, 2 H) 8.11 (d, J=9.77 Hz, 1 H) 8.02 (d, J=9.00 Hz, 1 H) 7.86 (d, J=7.63 Hz, 1 H) 7.65 (t, J=7.86 Hz, 1 H) 7.36 - 7.48 (m, 3 H) 7.09 (d, J=9.77 Hz, 1 H) 7.05 (d, J=1.98 Hz, 1 H) 5.62 (d, J=7.17 Hz, 1 H) 5.37 (s, 2 H) 3.98 (d, J=5.80 Hz, 2 H) 3.70 (br. s., 2 H) 3.57 (br. s., 2 H) 3.46 (br. s., 2 H) 3.39 (br. s., 2 H) 2.77 - 2.89 (m, 3 H) 2.48 - 2.56 (m, 2 H) 1.94 - 2.04 (m, 4 H) 1.71 (d, J=9.77 Hz, 4 H) 1.26 (d, J=10.68 Hz, 2 H) 1.16 (s, 3 H)

### Example C-24: Preparation of 3-(1-(3-(5-((1-(2-(4-(4-(3-(2,6-dioxopiperidine-3-yl)-1-methyl-4-oxo-3,4-dihydropetalazine-6-yl)piperazine-1-il)methyl-1-il)-2-oxoethyl)piperidine-4-il)methoxy)pyrimidine-2-yl)benzonitrile (ICT-0001195)

### Step 1) Preparation of 3-(4-methyl-1-oxo-7-(4-(piperidine-4-ylmethyl)piperazine-1-yl)phthalazine-2(1H)-yl)piperidine-2,6-dione

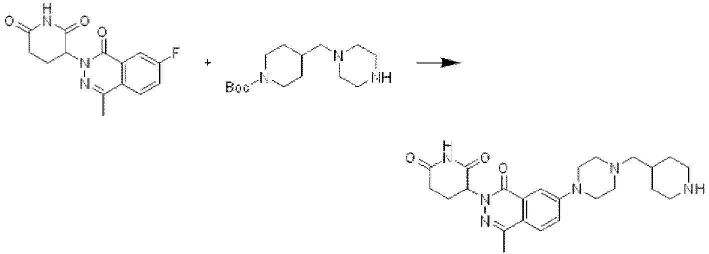

Ethyl bis(propane-2-yl)amine (1.34 g, 10.4 mmol) was added to 3-(7-fluoro-4-methyl-1-oxopthalazine-2(1H)-yl)piperidine-2,6-dione (0.5 g, 1.73 mmol) and tert-butyl 4-(piperazine-1-monomethyl)piperidine-1-carboxylate (980 mg, 3.46 mmol) in 5 ml of DMA at room temperature. The reactants were heated at 120°C for 16 hours. The reactants were poured into water, extracted with ethyl acetate, dried with Magnesium sulfate, and concentrated under reduced pressure. The residue was purified with MeOH/DCM (0-10%) MPLC. The protected compound was obtained, treated with (5 mL) 50% TFA in DCM, and stirred for 5 hours. After the reaction was completed, the reactants were concentrated under reduced pressure and vacuum-dried. The residue was dissolved in DCM, and the title compound was obtained by passing it through a Chromatorex pad with NH-DM 1020 (550 mg). MS (ESI, m/z): [M+H]⁺ = 453.6.

### Step 2) Preparation of 3-(1-(3-(5-((1-(2-(4-((4-(3-(2,6-dioxopiperidine-3-yl)-1-methyl-4-oxo-3-yl)piperazine-1-yl)methyl-1-il)methyl)piperidine-1-il)2-oxoethyl)piperidine-4-il)methoxy)pyrimidine-2-yl)benzonitrile (ICT-0001195)

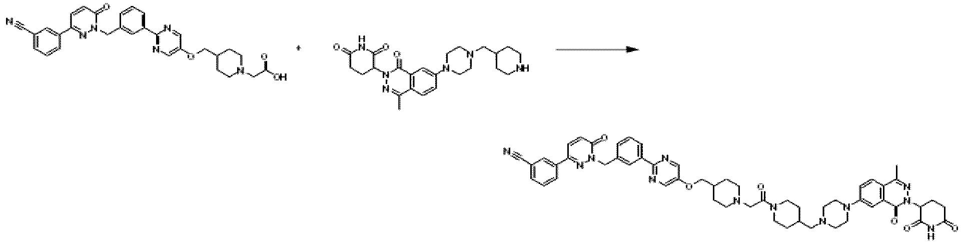

K₂CO₃ (68.1 mg, 0.5 mmol) was added to 5-chloro-N2-[5-methyl-4-(piperidine-4-yl)2-(propane-2-oxy)phenyl]-N4-[2-(propane-2-sulfonyl)phenyl]pyrimidine-2,4-diamine (47.2 mg, 0.1 mmol) and 3-{7-[4-(2-chloroacetyl)piperazine-1-yl]-4-methyl-1-oxo-1,2-dihydroptalazin-2-yl}piperidine-2,6-dione (42.6 mg, 0.1 mmol) in DMF (5 ml). The reactants were stirred for 4 hours at room temperature. The reactants were poured into the water. Ethyl acetate. The organic layer was dried with Magnesium sulfate, concentrated under reduced pressure, and purified by column chromatography to obtain the title compound (38.2 mg). MS (ESI, m/z): [M+H]⁺ = [972.2],
1H NMR (500 MHz, DMSO-d6) δ ppm 11.02 (s, 1 H) 8.67 (s, 1 H) 8.50 (s, 2 H) 8.38 (s, 1 H) 8.11 - 8.29 (m, 2 H) 7.83 - 7.99 (m, 1 H) 7.73 (t, J=7.95 Hz, 1 H) 7.46 - 7.56 (m, 1 H) 7.38 - 7.45 (m, 4 H) 7.31 (d, J=8.07 Hz, 1 H) 7.17 (d, J=9.66 Hz, 1 H) 5.71 (br. s., 1 H) 5.45 (s, 1 H) 4.37 (d, J=13.20 Hz, 1 H) 4.19 - 4.29 (m, 1 H) 4.05 - 4.19 (m, 2 H) 3.71 (d, J=12.96 Hz, 2 H) 3.44 (br. s., 1 H) 2.86 - 3.13 (m, 3 H) 2.78 (br. s., 2 H) 2.56 - 2.72 (m, 1 H) 2.53 (s, 1 H) 2.43 (d, J=6.85 Hz, 1 H) 2.06 - 2.12 (m, 1 H) 1.96 (d, J=10.27 Hz, 2 H) 1.84 (d, J=11.25 Hz, 1 H) 1.67 (d, J=11.49 Hz, 1 H)

### Example C-25 : Preparation of 3-(1-{[3-(5-{[1-(3-{4-[2-(2,6-dioxopiperidin-3-yl)-1-oxo-1,2-dihydropthalazine-6-yl]piperazine-1-yl}propanoyl)piperidine-4-il]methoxy}pyrimidin-2-yl)phenyl]methyl}-6-oxo-1,6-dihydropyridazine-3-yl)benzonitrile (ICT-0001199)

### Step 1) Preparation of tert-butyl 3-(4-(2-(2,6-dioxopiperidin-3-yl)-1-oxo-1,2-dihydroptalazine-6-yl)piperazine-1-yl)propanoate

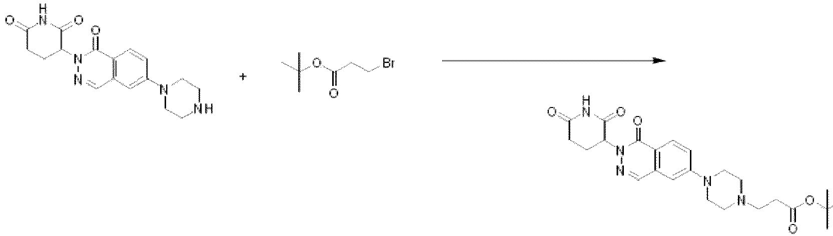

Ethylbis (propane-2-yl)amine (2.0 equivalent) was added to a solution of 3-[4-methyl-1-oxo-6-(piperazine-1-yl)-1,2-dihydropetalazine-2-yl]piperidine-2,6-dione (200 mg, 0.56 mmol) and tert-butyl 3-bromopropanoate (153 mg, 0.73 mmol) in a CAN (10 ml). The reactants were stirred at room temperature for 5 hours. After the end of the reaction, the reaction mixture was concentrated under reduced pressure. The residues were purified by MeOH/DCM (0-20%) column chromatography to obtain the heading compound (150 mg) MS (ESI, m/z): [M+H]⁺ = 484.4

### Step 2) Preparation of 3-{4-[2-(2,6-dioxopiperidin-3-yl)-1-oxo-1,2-dihydroptalazine-6-yl]piperazine-1-yl} Propanoic acid

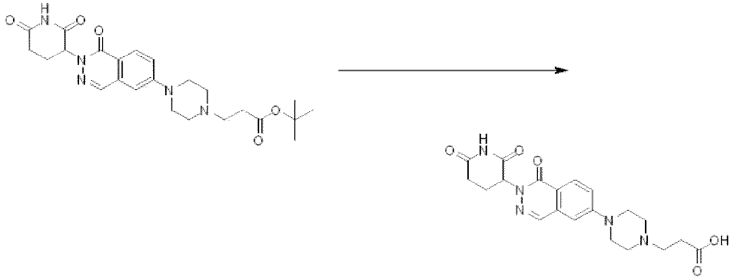

TFA (5 ml) was added to tert-butyl tert-butyl 3-(4-(2-(2,6-dioxopiperidine-3-yl)-1-oxo-1,2-dihydroptalazine-6-yl)piperazine-1-yl) propanoate (150 mg, 0.32 mmol) solution in DCM (15 ml). The reactants were stirred at room temperature for 1 hour. After the reaction was completed, the reaction mixture was concentrated under reduced pressure. The residues were purified by MeOH/DCM (0-20%) column chromatography to obtain the title compound (100 mg) MS (ESI, m/z): [M+H]⁺ = 414.4

### Step 3) Preparation of 3-(1-{[3-(5-{[1-(3-{4-[2-(2,6-dioxopiperidine-3-yl)-1-oxo-1,2-dihydropthalazine-6-yl]piperazine-1-day}profanoyl)piperidine-4-il]methoxy}pyrimidine-2-yl)phenyl]methyl}6-oxo-1,6-dihydropyridazine-3-yl)benzonitrile (ICT-0001199)

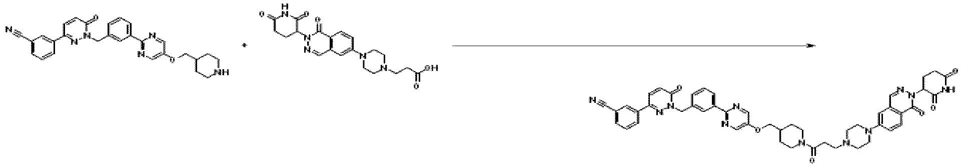

HATU (47.7 mg, 0.13 mmol) was added to 3-{6-oxo-1-[(3-{5-[(piperidine-4-yl)methoxy]pyrimidin-2-yl}phenyl)methyl]-1,6-dihydropyridazine-3-yl} benzonitrile (30 mg, 0.063 mmol) and 3-{4-[2-(2,6-dioxopiperidine-3-yl)-1-oxo-1,2-dihydroptalazine-6-yl]piperazine-1-yl}piperazine-1-yl}propanoic acid (26.0 mg, 0.13 mmol), followed by ethylbis (propane-2-yl)amine (3 equivalent). The reactants were stirred at room temperature for 6 hours. The reactants were poured into the water. It was extracted with ethyl acetate, dried with Magnesium sulfate, and concentrated under reduced pressure. The residues were purified by MeOH/DCM (0-20%) column chromatography to obtain the title compound (43 mg). MS (ESI, m/z): [M+H]⁺ = 875.1.
1H NMR (500 MHz, DMSO-d6) δppm 11.01 (s, 1 H) 8.66 (s, 2 H) 8.39 (s, 2 H) 8.21 - 8.29 (m, 3 H) 8.18 (d, J=9.92 Hz, 1 H) 8.05 (d, J=8.39 Hz, 1 H) 7.94 (d, J=7.93 Hz, 1 H) 7.73 (t, J=7.86 Hz, 1 H) 7.44 - 7.56 (m, 3 H) 7.28 (br. s., 1 H) 7.17 (d, J=9.77 Hz, 1 H) 5.72 - 5.79 (m, 1 H) 5.45 (s, 2 H) 4.44 (d, J=12.97 Hz, 1 H) 4.08 (d, J=6.10 Hz, 2 H) 3.97 (d, J=12.97 Hz, 1 H) 3.62 (dq, J=10.49, 6.52 Hz, 8 H) 3.07 - 3.19 (m, 8 H) 2.83 - 2.97 (m, 2 H) 2.04 - 2.11 (m, 3 H) 1.84 (d, J=12.82 Hz, 1 H) 1.78 (br. s., 1 H)

### Example C-26 : Preparation of 3-(1-{[3-(5-{1-(4-{4-[2-(2,6-dioxopiperidin-3-yl)-1-oxo-1,2-dihydropthalazine-6-yl]piperazine-1-day}butanoyl)piperidine-4-il]methoxy}pyrimidine-2-yl)phenyl]methyl}-6-oxo-1,6-dihydropyridazine-3-yl)benzonitrile (ICT-001200)

### Step 1) Preparation of tert-butyl 4-(4-(2-(2,6-dioxopiperidin-3-yl)-1-oxo-1,2-dihydroptalazine-6-yl)piperazine-1-yl)butanoate

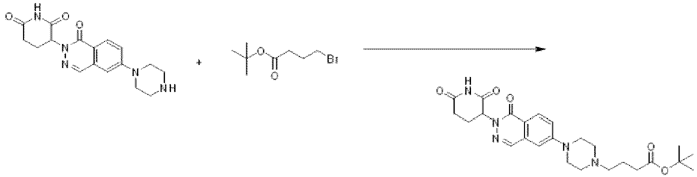

Ethylbis (propane-2-yl)amine (4.0 equivalent) was added to 3-(1-oxo-6-(piperazine-1-yl)phthalazine-2(1H)-yl) piperidine-2,6-dione (200 mg, 0.56 mmol) and tert-butyl 4-bromobutanoate (188 mg, 0.84 mmol) solutions in NMP (2.0 ml). The reactants were stirred at 130°C for 48 hours. After the end of the reaction, the reaction mixture was concentrated under reduced pressure. The residues were purified by MeOH/DCM (0-20%) column chromatography to obtain the heading compound (200 mg) MS (ESI, m/z): [M+H]⁺ = 484.6

### Step 2) Preparation of 4-{4-[2-(2,6-dioxopiperidin-3-yl)-1-oxo-1,2-dihydroptalazine-6-yl]piperazine-1-day} Butanoic acid

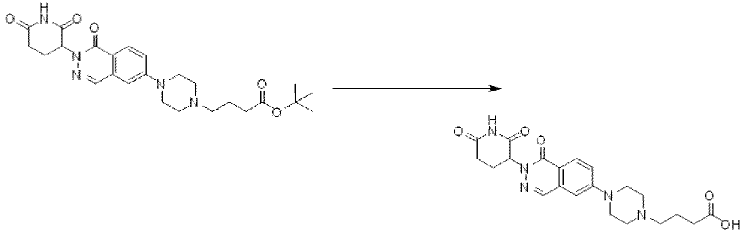

TFA (5 ml) was added to tert-butyl 4-(4-(2-(2,6-dioxopiperidine-3-yl)-1-oxo-1,2-dihydroptalazine-6-yl)piperazine-1-yl)butanoate (200 mg, 0.41 mmol) solution in DCM (15 ml). The reactants were stirred at room temperature for 1 hour. After the end of the reaction, the reaction mixture was concentrated under reduced pressure. The residues were purified by MeOH/DCM (0-20%) column chromatography to obtain the heading compound (150 mg) MS (ESI, m/z): [M+H]⁺ = 428.4

### Step 3) Preparation of 3-(1-{[3-(5-{[1-(4-{4-[2-(2,6-dioxopiperidine-3-yl)-1-oxo-1,2-dihydropthalazine-6-yl]piperazine-1-day}butanoyl)piperidine-4-il]methoxy}pyrimidine-2-yl)phenyl]methyl}6-oxo-1,6-dihydropyridazine-3-yl)benzonitrile (ICT-001200)

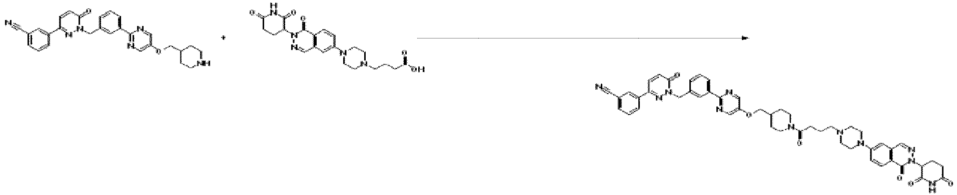

HATU (47.7 mg, 0.13 mmol) was added to 3-{6-oxo-1-[(3-{5-[(piperidine-4-yl)methoxy]pyrimidin-2-yl}phenyl)methyl]-1,6-dihydropyridazin-3-yl} benzonitrile (30 mg, 0.06 mmol) and 4-{4-[2-(2,6-dioxopiperidine-3-yl)-1-oxo-1,2-dihydroptalazine-6-yl]piperazine-1-yl}piperazine-1-yl}butanoic acid (26.8 mg, 0.06 mmol), followed by ethylbis (propane-2-yl)amine (3 equivalent). The reactants were stirred at room temperature for 6 hours. The reactants were poured into water. It was extracted with ethyl acetate, dried with Magnesium sulfate, and concentrated under reduced pressure. The residue was purified by MeOH/DCM (0-20%) column chromatography to obtain the title compound (40 mg). MS (ESI, m/z): [M+H]⁺ = 889.1.
1H NMR (500 MHz, DMSO-d6) δppm 10.94 (s, 1 H) 8.55 - 8.61 (m, 2 H) 8.31 (s, 2 H) 8.13 - 8.21 (m, 3 H) 8.11 (d, J=9.92 Hz, 1 H) 7.98 (d, J=7.93 Hz, 1 H) 7.86 (d, J=7.63 Hz, 1 H) 7.65 (t, J=7.93 Hz, 1 H) 7.40 - 7.46 (m, 3 H) 7.21 (br. s., 1 H) 7.10 (d, J=9.77 Hz, 1 H) 5.63 - 5.73 (m, 1 H) 5.38 (s, 2 H) 4.37 (d, J=12.05 Hz, 1 H) 4.00 (d, J=6.26 Hz, 2 H) 3.86 (d, J=13.89 Hz, 1 H) 3.54 (dd, J=10.45, 6.33 Hz, 3 H) 3.03 - 3.12 (m, 3 H) 2.97 (t, J=12.59 Hz, 1 H) 2.79 - 2.90 (m, 1 H) 2.47 - 2.58 (m, 4 H) 2.29 (br. s., 3 H) 1.95 - 2.04 (m, 4 H) 1.68 - 1.81 (m, 2 H) 1.47 (br. s., 4 H)

### Example C-27 : Preparation of 3-(1-{[3-(5-{[1-(5-{4-[2-(2,6-dioxopiperidin-3-yl)-1-oxo-1,2-dihydropetalazine-6-yl]piperazine-1-yl}pentanoyl)piperidine-4-il]methoxy}pyrimidine-2-yl)phenyl]methyl}6-oxo-1,6-dihydropyridazine-3-yl)benzonitrile (ICT-0001201)

### Step 1) Preparation of tert-butyl 5-(4-(2-(2,6-dioxopiperidin-3-yl)-1-oxo-1,2-dihydroptalazine-6-yl)piperazine-1-yl)pentanoate

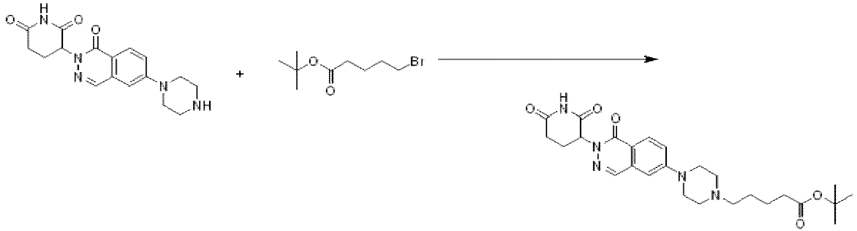

Ethylbis (propane-2-yl)amine (4.0 equivalent) was added to 3-(6-fluoro-1-oxo-1,2-dihydrophthalazine-2-yl)piperidine-2,6-dione (200 mg, 0.56 mmol) and tert-butyl 5-bromopentanoate (200 mg, 0.84 mmol) solutions in NMP (2.0 ml). The reactants were stirred at 130°C for 48 hours. After the reaction was completed, the reaction mixture was concentrated under reduced pressure. The residues were purified by MeOH/DCM (0-20%) column chromatography to obtain the heading compound (200 mg) MS (ESI, m/z): [M+H]⁺ = 512.6

### Step 2) 3-(1-oxo-6-(piperazine-1-yl)phthalazine-2(1H)-yl)piperidine-2,6-dione

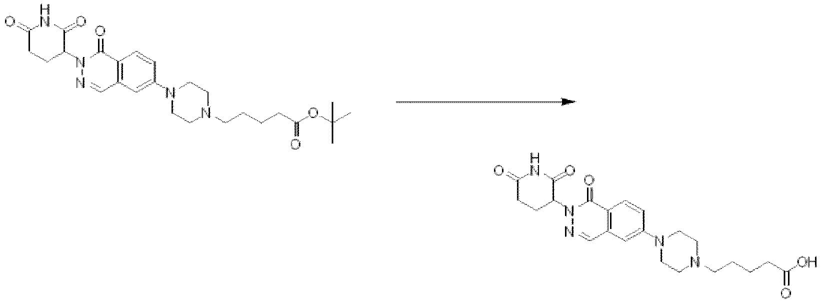

TFA (5 ml) was added to tert-butyl 5-(4-(2-(2,6-dioxopiperidine-3-yl)-1-oxo-1,2-dihydroptalazine-6-yl) solution in DCM (15 ml). TFA (5 ml) was added to the solution of tert-butyl 5-(4-(2-(2,6-dioxopiperidine-3-yl)-1-oxo-1,2-dihydroptalazine-6-yl. The reactants were stirred at room temperature for 1 hour. After the reaction was completed, the reaction mixture was concentrated under reduced pressure. The residues were purified by MeOH/DCM (0-20%) column chromatography to obtain the title compound (150 mg) MS (ESI, m/z): [M+H]⁺ = 442.5

### Step 3) Preparation of 3-(1-{[3-(5-{[1-(5-{4-[2-(2,6-dioxopiperidin-3-yl)-1-oxo-1,2-dihydropthalazine-6-yl]piperazine-1-day}pentanoyl)piperidine-4-il]methoxy}pyrimidine-2-yl)phenyl]methyl}6-oxo-1,6-dihydropyridazine-3-yl)benzonitrile (ICT-0001201)

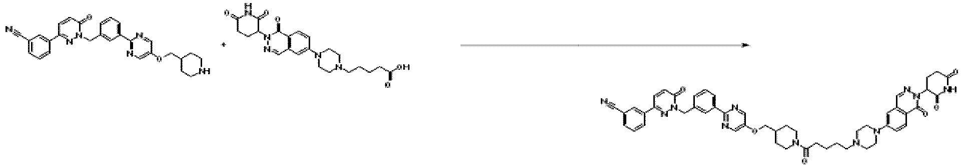

HATU (47.7 mg, 0.13 mmol) was added to 3-{6-oxo-1-[(3-{5-[(piperidine-4-yl)methoxy]pyrimidin-2-yl}phenyl)methyl]-1,6-dihydropyridazin-3-yl} benzonitrile (30 mg, 0.06 mmol) and 5-{4-[2-(2,6-dioxopiperidine-3-yl)-1-oxo-1,2-dihydropthalazine-6-yl]piperazine-1-yl}pentanoic acid (27.7 mg, 0.13 mmol), followed by ethylbis (propane-2-yl)amine (3 equivalent). The reactants were stirred at room temperature for 6 hours. The reactants were poured into water, extracted with acetate, dried with Magnesium sulfate, and concentrated under reduced pressure. The residue was purified by MeOH/DCM (0-20%) column chromatography to obtain the title compound (42 mg). MS (ESI, m/z): [M+H]⁺ = 903.0.
1H NMR (500 MHz, DMSO-d6) δ ppm 11.01 (s, 1 H) 8.59 - 8.73 (m, 2 H) 8.38 (s, 2 H) 8.21 - 8.30 (m, 3 H) 8.18 (d, J=9.77 Hz, 1 H) 8.06 (d, J=8.24 Hz, 1 H) 7.94 (d, J=7.63 Hz, 1 H) 7.72 (t, J=7.86 Hz, 1 H) 7.45 - 7.56 (m, 3 H) 7.29 (br. s., 1 H) 7.17 (d, J=9.77 Hz, 1 H) 5.76 (dd, J=12.21, 4.88 Hz, 1 H) 5.45 (s, 2 H) 4.45 (d, J=12.82 Hz, 1 H) 4.08 (d, J=6.26 Hz, 2 H) 3.93 (d, J=12.21 Hz, 1 H) 3.62 (dq, J=10.57, 6.55 Hz, 4 H) 3.09 - 3.19 (m, 4 H) 3.05 (t, J=11.75 Hz, 1 H) 2.87 - 2.97 (m, 1 H) 2.54 - 2.65 (m, 4 H) 2.38 (d, J=12.05 Hz, 3 H) 2.03 - 2.13 (m, 3 H) 1.84 (d, J=12.21 Hz, 2 H) 1.78 (br. s., 2 H) 1.26 - 1.32 (m, 3 H)

### Example C-28: Preparation of 3-(1-(3-(5-((1-(2-(4-(4-(2-(2-(2-(2-(2-(2,6-dioxopiperidine-3-yl)-7-fluoro-4-methyl-1-oxo-1,2-dihydrophthalazine-6-yl)piperazine-1-il)methyl)piperidine-1-il)-2-oxoethyl)piperidine-4-yl)methoxy)pyrimidine-2-yl)benzyl)6-oxo-1,6-dihydropyridazine-3-yl)benzonitrile (ICT-0001204)

### Step 1) Preparation of 3-(7-fluoro-4-methyl-1-oxo-6-(4-(piperidine-4-ylmethyl)piperazine-1-yl)phthalazine-2(1H)-yl)piperidine-2,6-dione and 3-(6-fluoro-4-methyl-1-oxo-7-(4-(piperidine-4-ylmethyl)piperazine-1-yl)phthalazine-2(1H)-yl)piperidine-2,6-dione

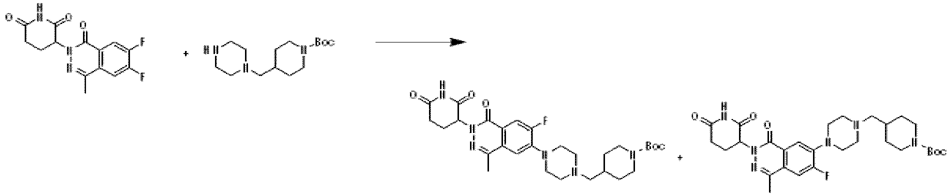

tert-butyl 4-(piperazine-1-yl)piperidine-1-carboxylate (92 mg, 325 µmol) was added to 3-(6,7-difluoro-4-methyl-1-oxopthalazine-2(1H)-yl)piperidine-2,6-dione (100 mg, 325 µmol) solution in DMA (1 mL) at ambient temperature, followed by ethyl bis(propane-2-yl)amine (46.3 mg, 358 µmol). The reactants were heated at 120°C for 16 hours. After the end of the reaction, the reactants were poured into water and extracted with ethyl acetate (25 mL x 2). The organic layer was separated. The crude product was obtained by drying, filtering, and concentrating with Magnesium sulfate, which was purified with MPLC (1 to 5% MeOH in CH₂Cl₂). Boc-protected compounds were obtained, treated with 50% TFA in DCM (5 mL), and stirred for 5 hours. After the reaction was completed, the reactants were concentrated under reduced pressure and vacuum-dried. The residue was dissolved in DCM, passed through an NH-DM 1020, Chromatorex pad to obtain a mixture of regio isomers, and purified with a 0-80% acetonitrile C-18 column in H2O, and the two compounds were obtained in a ratio of 3:5. Less polar compounds were conferred as 3-(7-fluoro-4-methyl-1-oxo-6-(4-(piperidine-4-ylmethyl)piperazine-1-yl)phthalazine-2(1H)-yl)piperidine-2,6-dione, and more polar compounds were given as 3-(6-fluoro-1-oxo-7-(4-(piperidine-4-ylmethyl)piperazine-1-yl)phthalazine-2(1H)-yl)piperidine-2,6-dione. MS (ESI, m/z): [M+H]⁺ = 471.3, and 471.2

### Step 2) Preparation of 3-(1-(3-(5-((1-(2-(4-(4-(2-(2,6-dioxopiperidine-3-yl)-7-fluoro-4-methyl-1-oxo-1,2-dihydropthalazine-6-yl)piperazine-1-il)methyl)piperidine-1-day)-2-oxoethyl)piperidine-4-il)methoxy)pyrimidine-2-yl)benzonitrile (ICT-0001204)

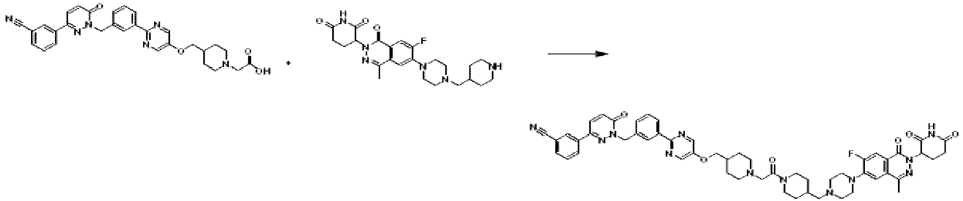

HATU (36.8 mg, 0.09 mmol) is added to 2-[4-({[2-(3-{[3-(3-cyanophenyl)-6-oxo-1,6-dihydropyridazin-1-yl]methyl}phenyl)pyrimidine-5-yl]oxy}methyl)piperidine-1-yl]acetic acid (46.6 mg, 0.09 mmol) and 3-(7-fluoro-4-methyl-1-oxo-6-(4-piperidine-4-ylmethyl)piperazine-1-yl)piperazine-2,6-dione (40.8 mg, 0.09 mmol)-yl) in DMF (5 ml) at room temperature, followed by ethylbis (propane-2-yl)amine (33.7 mg, 0.1 mmol). The reactants were stirred for 6 hours. The reactants were poured into water, extracted with ethyl acetate, dried with Magnesium sulfate, and concentrated under reduced pressure. The residue was purified with MeOH/DCM (0-20%) MPLC to obtain the title compound (48.2 mg). MS (ESI, m/z): [M+H]⁺ = 990.2.
1H NMR (500 MHz, DMSO-d6) δ ppm 10.95 - 11.03 (m, 1 H) 8.63 - 8.71 (m, 2 H) 8.38 (d, J=5.65 Hz, 2 H) 8.20 - 8.27 (m, 2 H) 8.11 - 8.20 (m, 1 H) 7.93 (d, J=7.78 Hz, 1 H) 7.86 (d, J=12.97 Hz, 1 H) 7.72 (t, J=7.93 Hz, 1 H) 7.45 - 7.54 (m, 2 H) 7.29 (d, J=8.09 Hz, 1 H) 7.16 (d, J=9.77 Hz, 1 H) 5.68 - 5.77 (m, 1 H) 5.45 (s, 2 H) 4.36 (d, J=11.44 Hz, 1 H) 4.09 (d, J=5.34 Hz, 2 H) 3.28 (br. s., 4 H) 2.97 - 3.05 (m, 1 H) 2.86 - 2.97 (m, 1 H) 2.60 - 2.66 (m, 1 H) 2.54 - 2.60 (m, 5 H) 2.40 - 2.48 (m, 1 H) 2.23 (br. s., 1 H) 2.03 - 2.12 (m, 1 H) 1.87 (br. s., 3 H) 1.66 - 1.84 (m, 3 H) 1.53 (br. s., 1 H) 1.39 (s, 2 H) 1.11 (d, J=11.44 Hz, 1 H) 0.96 (d, J=8.54 Hz, 1 H)

### Example C-29: Preparation of 2-(4-(((2-(3-((3-(3-(3-(3-cyanophenyl)-6-oxopyridazine-1(6H)-yl)methyl)phenyl)pyrimidine-5-yl)oxy)methyl)piperidine-1-yl)N-(5-(2-(2,6-dioxopiperidin-3-yl)-1-oxo-1,2-dihydroptalazine-6-yl)pent-4-phospho-1-yl)acetamide (ICT-0001205)

### Step 1) Preparation of 3-(6-(5-aminopent-1-phosphorus-1-yl)-1-oxopthalazine-2(1H)-day)piperidine-2,6-dione

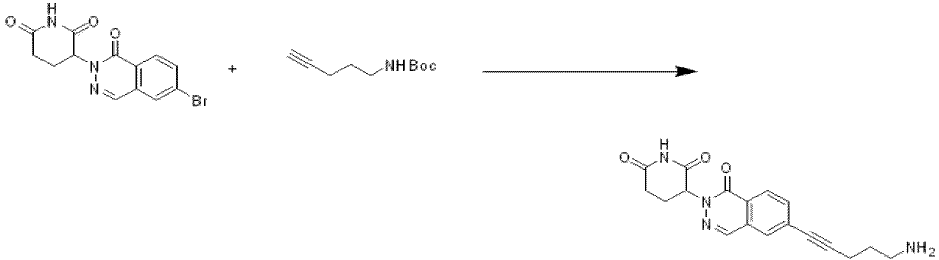

Cul (113 mg, 0.6 mmol) and Pd(PPh₃)₂Cl₂ (418 mg, 0.6 mmol) were added to 3-(6-bromo-1-oxo-1,2-dihydrophoptalazin-2-ylphorapazine-2-yl) and t-butylpent-4-phosphorus-1-ylcarbamate (1.2 g, 6.54 mmol) solutions in DMF (5 mL), followed by TEA (1.81 g, 17.8 mmol). The mixture was irradiated at 80°C in a microwave reactor for 1.5 hours. The reaction mixture was diluted with water and extracted with ethyl acetate. The organic layer was washed with brine solution, dried with Na₂SO₄, and concentrated. The resulting residues were purified by column chromatography, treated with 50% TFA in DCM (5 mL), and stirred for 5 hours. After the deprotection reaction was completed, the reactants were concentrated under reduced pressure and vacuum-dried. The residue was dissolved in DCM (5 mL) and passed through a Chromatorex pad with NH-DM 1020, a Chromatorex pad, to obtain the title compound as an off-white oil (1.59 g). MS (ESI, m/z): [M+H]⁺ = 339.1.

### Step 2) Preparation of 2-(4-(((2-(3-(3-(3-(3-(3-cyanophenyl)-6-oxopyridazine-1(6H)-yl)methyl)phenyl)pyrimidine-5-il)oxy)methyl)piperidine-1-il)-N-(5-(2-(2-(2,6-dioxopiperidine-3-yl)1-oxo-1,2-dihydroptalazine-6-yl)pent-4-phosphorus-1-yl)acetamide (ICT-0001205)

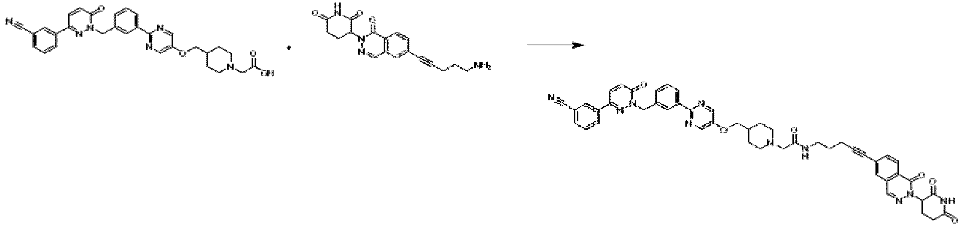

HATU (36.3 mg, 0.09 mmol) is added to 2-[4-({[2-(3-{[3-(3-cyanophenyl)-6-oxo-1,6-dihydropyridazine-1-yl]methyl}phenyl)pyrimidine-5-yl]oxy}methyl)piperidine-1-yl]acetic acid (46.6 mg, 0.09 mmol) and 3-(6-(5-aminopent-1-phosphorus-1-yl)-1-oxopthalazine-2(1H)-day)piperidine-2,6-dione (29.4 mg, 0.09 mmol) in DMF (5 ml) at room temperature, followed by ethylbis (propane-2-yl)amine (33.7 mg, 0.26 mmol). The reactants were stirred for 6 hours. The reactants were poured into water, extracted with ethyl acetate, dried with Magnesium sulfate, and concentrated under reduced pressure. The residue was purified with MeOH/DCM (0-20%) MPLC to obtain the title compound (45 mg). MS (ESI, m/z): [M+H]⁺ = 857.9.
1H NMR (500 MHz, DMSO-d6) δ ppm 11.05 (s, 1 H) 8.60 - 8.74 (m, 2 H) 8.44 (s, 1 H) 8.35 - 8.42 (m, 2 H) 8.11 - 8.29 (m, 4 H) 8.01 (s, 1 H) 7.90 - 7.98 (m, 1 H) 7.84 (dd, J=8.32, 1.30 Hz, 1 H) 7.72 (t, J=7.86 Hz, 1 H) 7.43 - 7.54 (m, 2 H) 7.16 (d, J=9.77 Hz, 1 H) 5.80 (dd, J=12.13, 5.26 Hz, 1 H) 5.45 (s, 2 H) 4.07 (d, J=5.49 Hz, 1 H) 3.29 (br. s., 1 H) 2.87 - 2.98 (m, 2 H) 2.58 - 2.66 (m, 1 H) 2.52 - 2.57 (m, 2 H) 2.08 - 2.17 (m, 1 H) 1.85 (br. s., 2 H) 1.77 (quin, J=6.98 Hz, 2 H) 1.39 (s, 1 H)

### Example C-30: Preparation of 2-(4-(((2-(3-((3-(3-(3-(3-cyanophenyl)-6-oxopridazine-1(6H)-yl)methyl)phenyl)pyrimidine-5-il)oxy)methyl)piperidine-1-yl)N-(5-(2-(2,6-dioxopiperidin-3-yl)-1-oxo-1,2-dihydroptalazine-6-yl)pentyl)acetamide (ICT-0001206)

### Step 1) Preparation of 3-(6-(5-aminopentyl)-1-oxopthalazine-2(1H)-yl)piperidine-2,6-dione

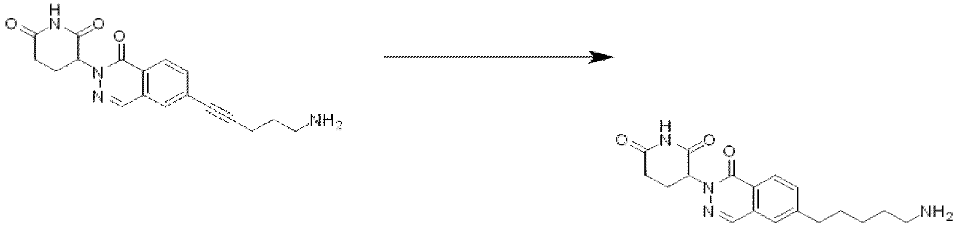

10% Pd/C (10 mg) was added to 3-(6-(5-aminopent-1-phosphorus-1-yl)-1-oxotalasine-2(1H)-yl)piperidine-2,6-dione (0.1 g, 0.3 mmol) solution in MeOH (5 mL) at ambient temperature. The reaction was carried out by attaching an H₂ balloon. After stirring the reactants at room temperature for 5 hours, the reactants were filtered and vacuum-dried to obtain the subject compound (85 mg). MS (ESI, m/z): [M+H]⁺ = 343.4.

### Step 2) Preparation of 2-(4-(((2-(3-((3-(3-(3-cyanophenyl)-6-oxopridazine-1(6H)-yl)methyl)phenyl)pyrimidine-5-il)oxy)methyl)piperidine-1-yl)N-(5-(2-(2,6-dioxopiperidine-3-yl)1-oxo-1,2-dihydropthalazine-6-yl)pentyl)acetamide (ICT-0001206)

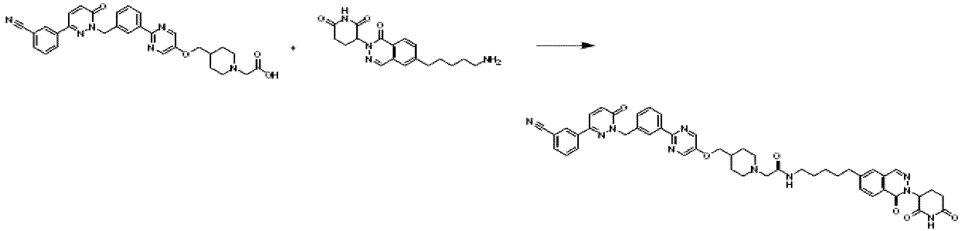

HATU (36.3 mg, 0.09 mmol) is added to 2-(4-(((2-(3-((3-(3-(3-cyanophenyl)-6-oxopridazine-1(6H)-yl)methyl)phenyl)pyrimidine-5-yl)oxy)methyl)piperidine-1-yl) acetic acid (46.6 mg, 0.09 mmol) and 3-(6-(5-aminopentyl)-1-oxopthalazine-2(1H)-yl)piperidine-2,6-dione (28.7 mg, 0.09 mmol) in DMF (5 ml) at room temperature, followed by ethylbis (propane-2-yl)amine (33.7 mg, 0.26 mmol). The reactants were stirred for 6 hours. The reactants were poured into water, extracted with ethyl acetate, dried with Magnesium sulfate, and concentrated under reduced pressure. The residue was purified with MeOH/DCM (0-20%) MPLC to obtain the title compound (38 mg). MS (ESI, m/z): [M+H]⁺ = 861.9.
1H NMR (500 MHz, DMSO-d6) δ ppm 11.05 (s, 1 H) 8.59 - 8.72 (m, 2 H) 8.33 - 8.46 (m, 3 H) 8.12 - 8.28 (m, 4 H) 7.87 - 8.00 (m, 1 H) 7.68 - 7.82 (m, 3 H) 7.43 - 7.57 (m, 2 H) 7.17 (d, J=9.77 Hz, 1 H) 5.81 (dd, J=11.98, 5.26 Hz, 1 H) 5.45 (s, 2 H) 4.07 (d, J=4.58 Hz, 1 H) 3.12 (d, J=6.10 Hz, 1 H) 2.85 - 2.99 (m, 1 H) 2.80 (t, J=7.48 Hz, 2 H) 2.53 - 2.67 (m, 2 H) 2.05 - 2.16 (m, 1 H) 1.68 (quin, J=7.48 Hz, 2 H) 1.48 (quin, J=7.13 Hz, 2 H) 1.39 (s, 1 H) 1.31 (d, J=6.56 Hz, 2 H)

### Example C-31: Preparation of 2-(4-(((2-(3-((3-(3-(3-(3-cyanophenyl)-6-oxopyridazine-1(6H)-yl)methyl)phenyl)pyrimidine-5-il)oxy)methyl)piperidine-1-yl)N-(4-(4-(2-(2,6-dioxopiperidine-3-yl)-4-methyl-1-oxo-1,2dihydroptalazine-6-yl)piperazine-1-yl)phenyl)acetamide (ICT-0001207)

### Step 1) Preparation of 3-(6-(4-(4-aminophenyl)piperazine-1-yl)-4-methyl-1-oxopthalazine-2(1H)-yl)piperidine-2,6-dione

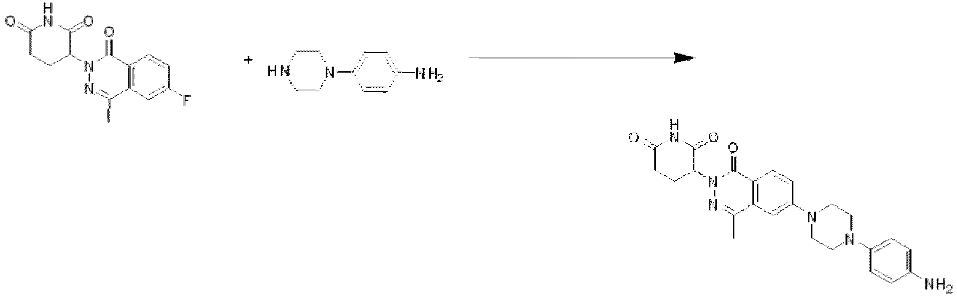

Ethylbis (propane-2-yl)amine (670 mg, 5.19 mmol) was added to a solution of 3-(6-fluoro-4-methyl-1-oxopthalazine-2(1H)-yl)piperidine-2,6-dione (0.5 g, 1.73 mmol) 4-(piperazine-1-yl)aniline (306 mg, 1.73 mmol) in DMA (2 ml) at room temperature. The reactants were stirred at 160°C for 16 hours. The reactants were poured into water, extracted with ethyl acetate, dried with Magnesium sulfate, and concentrated under reduced pressure. The residues were purified with MeOH/DCM (0-10%) MPLC. Subjective compound (621 mg). MS (ESI, m/z): [M+H]⁺ = 447.6.

### Step 2) 2-(4-(((2-(3-((3-(3-(3-cyanophenyl)-6-oxopyridazine-1(6H)-yl)methyl)phenyl)pyrimidine-5-yl)oxy)methyl)piperidine-1-yl)-N-(4-(4-(2-(2,6-dioxopiperidin-3-yl)-4-methyl-1-oxo-1,2-dihydroptalazine-6-yl)piperazine-1-yl)phenyl)acetamide (ICT-0001207)

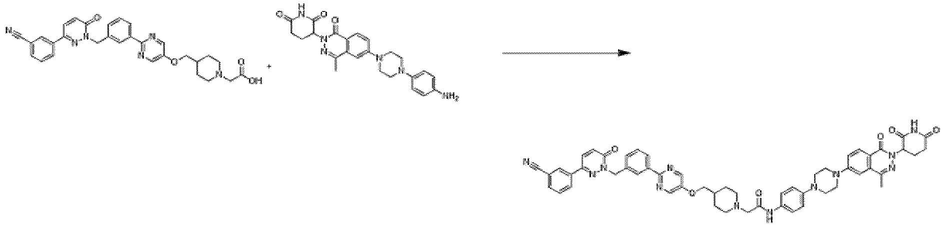

HATU (39 mg, 0.10 mmol) is added to a solution of 2-[4-({[2-(3-{3-(3-cyanophenyl)-6-oxo-1,6-dihydropyridazine-1-yl]methyl}phenyl)pyrimidine-5-yl]oxy}methyl)piperidine-1-yl]acetic acid (50 mg, 0.09 mmol) and 3-{6-[4-(4-aminophenyl)piperazine-1-yl]-4-methyl-1-oxo-1,2-dihydroptalazin-2-yl}piperidine-2,6-dione (41.6 mg, 0.09 mmol) in DMF (2 ml) at room temperature, followed by ethylbis (propane-2-yl)amine (21.4 mg, 0.18 mmol). The reactants were stirred for 6 hours. The reactants were poured into water, extracted with ethyl acetate, dried with Magnesium sulfate, and concentrated under reduced pressure. The residue was purified with MeOH/DCM (0-20%) MPLC to obtain the title compound (52 mg). MS (ESI, m/z): [M+H]⁺ = 966.1.
1H NMR (500 MHz, DMSO-d6) δ ppm 10.97 (s, 1 H) 8.62 - 8.67 (m, 2 H) 8.33 - 8.41 (m, 2 H) 8.21 - 8.29 (m, 2 H) 8.06 - 8.21 (m, 2 H) 7.93 (d, J=7.78 Hz, 1 H) 7.72 (t, J=7.86 Hz, 1 H) 7.57 (d, J=8.39 Hz, 1 H) 7.45 - 7.53 (m, 4 H) 7.12 - 7.20 (m, 2 H) 7.00 (d, J=8.54 Hz, 2 H) 5.69 (d, J=7.48 Hz, 1 H) 5.45 (s, 2 H) 4.01 - 4.15 (m, 1 H) 3.61 (br. s., 3 H) 3.28 (br. s., 4 H) 2.82 - 3.01 (m, 1 H) 2.52 - 2.66 (m, 2 H) 1.30 - 1.43 (m, 1 H) 1.14 - 1.28 (m, 1 H)

### Example C-32: Preparation of 3-(1-(3-(5-((1-(1-(2-(4-((1-(2-(2-(1-(2,6-dioxopiperidine-3-yl)-4-methyl-1-oxo-1,2-dihydrophthalazine-6-yl)piperidine-4-il)methyl)piperazine-1-il)acetyl)piperidine-4-il)methoxy)pyrimidine-2-yl)benzyl)6-oxo-1,6-dihydropyridazine-3-yl)benzonitrile (ICT-0001213)

### Step 1) Preparation of 3-(1-(3-(5-((1-(2-chloroacetyl)piperidine-4-yl)methoxy)pyrimidine-2-yl)benzyl)-6-oxo-1,6-dihydropyridazine-3-yl)benzonitrile

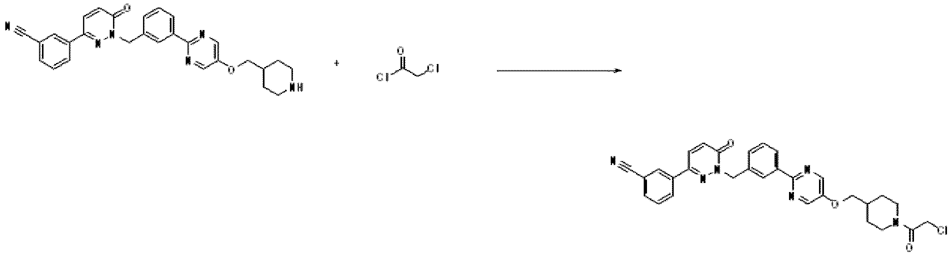

3-(6-oxo-1-(3-(5-(piperidine-4-ylmethoxy)pyrimidine-2-yl)benzyl)-1 in DCM (3mL) at 0°C Chloroacetyl chloride (0.784 mmol) was added to a 6-dihydropyridazine-3-yl) benzonitrile (0.522 mmol) and DIPEA (1.04 mmol) solution, and stirred at 0°C for 1 hour. The reaction mixture was concentrated. The reaction mixture was purified by reversed-phase column chromatography (water (0.1% FA) / ACN (0.1% FA) = 95/1 to 0/100 gradient). The corresponding fraction was lyophilized and 143 mg of 3-(1-(3-(5-((1-(2-chloroacety)piperidine-4-yl)methoxy)pyrimidine-2-yl)benzyl)-6-oxo-1, 6-dihydropyridazine-3-yl) benzonitrile. MS (ESI, m/z): [M+H]⁺ = 555.2

### Step 2) Preparation of 3-(1-(3-(5-((1-(2-(4-((1-(2-(4-(1-(2-(2,6-dioxopiperidine-3-yl)-4-methyl-1-oxo-1,2-dihydrophthalazine-6-yl)piperidine-4-il)methyl)piperazine-1-il)acetyl)piperidine-4-il)methoxy)pyrimidine-2-yl)benzyl)6-oxo-1,6-dihydropyridazine-3-yl)benzonitrile (ICT-0001213)

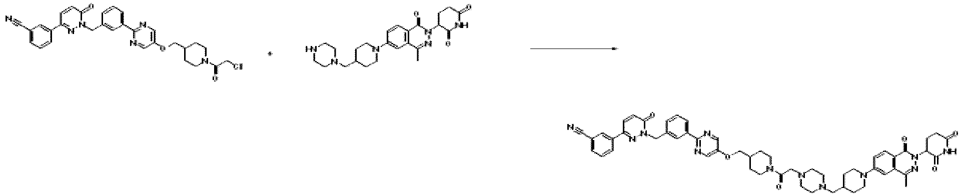

3-(1-(3-(5-((1-(1-(2-chloroacety)piperidine-4-yl)methoxy)pyrimidine-2-yl)benzyl)-6-oxo-1,in 0.6 ml DMSO DIPEA (98 µmol) was added to 6-dihydropyridazine-3-yl) benzonitrile (20 mg, 0.02 mmol) and 3-(4-(4-methyl-1-yl)piperidine-1-(4-(piperazine-1-yl)piperidine-2,6-dione (1H)-yl)piperidine-2,6-dione (15 mg, 0.02 mmol) solutions and stirred at 80°C for 16 hours. The reaction mixture was purified with reversed-phase column chromatography (water (0.1% FA) / ACN (0.1% FA) = 95/1 to 0/100 gradient). The corresponding fraction was lyophilized. The obtained product was amino silica Purified by column chromatography (DCM/MeOH, 100/0 to 97/3, gradient), 4 mg of 3-(1-(1-(5-((1-(2-(2-(4-(1-(2-(2-(2-(2-(2-(2-(2-(2-(dioxopiperine-3-yl)4-methyl-1-oxo-1,2-dihydropthalazine-6-yl)piperidine-4-il)methyl)piperazine-1-il)acetyl)piperidine-4-il)methoxy)pyrimidine-2-yl)benzyl)6-oxo-1,6-dihydropyridazine-3-yl) benzonitrile. MS (ESI, m/z): [M+H]⁺= 971.8.
1H NMR (500 MHz, DMSO-d6) δ ppm 10.97 (s, 1H), 8.65 (s, 2H), 8.39 (s, 1H), 8.37 (s, 1H), 8.24 (d, J = 8.1 Hz, 1H), 8.23 (s, 1H), 8.17 (d, J = 8.1 Hz, 1H), 8.03 (m, 1H) 7.93 (d, J = 7.8 Hz, 1H), 7.71 (t, J = 7.9 Hz, 1H), 7.49 (s, 1H), 7.46 (m, 2H), 7.16 (d, J = 9.8 Hz, 1H), 7.02 (s, 1H), 5.68 (m, 1H), 5.44 (s, 2H), 4.38 (d, J = 12.5 Hz, 1H), 4.08 (s, 2H), 4.02 (d, J = 12.5 Hz, 2H), 3.28 (m, 4H), 2.98 (m, 2H), 2.90 (m, 3H), 2.59 (m, 2H), 2.47 (s, 3H), 2.38 (brd, 6H), 2.10 (brd, 2H), 1.77 (m, 3H), 1.3 (m, 1H), 1.23 (m, 2H), 1.10 (m, 6H)

### Example C-33: Preparation of 3-(1-(3-(5-((1-(2-(4-(3-(2,6-dioxopiperidin-3-yl)-1-methyl-4-oxo-3,4-dihydropetalazine-6-yl)piperazine-1-il)acetyl)piperidine-4-il)methoxy)pyrimidine-2-yl)benzyl)6-oxo-1,6-dihydropyridazine-3-yl)benzonitrile (ICT-0001214)

### Step 1) Preparation of tert-butyl 2-(4-(3-(2,6-dioxopiperidin-3-yl)-1-methyl-4-oxo-3,4-dihydroptalazine-6-yl)piperazine-1-yl)acetate

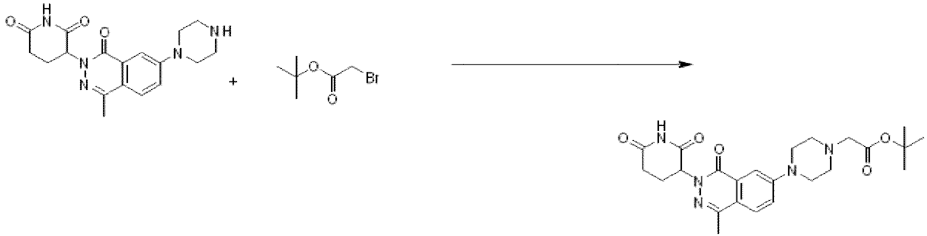

Ethylbis (propane-2-yl)amine (2.0 equivalent) was added to 3-(4-methyl-1-oxo-7-(piperazine-1-yl)phthalazine-2(1H)-yl) piperidine-2,6-dione (200 mg, 0.56 mmol) and tert-butyl 2-bromoacetate (143 mg, 0.73 mmol) solutions in ACN (10 ml). The reactants were stirred at room temperature for 5 hours. After the reaction was completed, the reaction mixture was concentrated under reduced pressure. The residues were purified by MeOH/DCM (0-20%) column chromatography to obtain the heading compound (150 mg) MS (ESI, m/z): [M+H]⁺ = 470.4

### Step 2) Preparation of 2-(4-(3-(2,6-dioxopiperidin-3-yl)-1-methyl-4-oxo-3,4-dihydroptalazine-6-yl)piperazine-1-yl)acetic acid

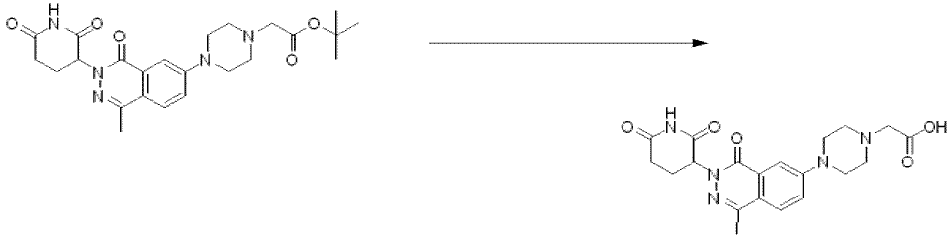

TFA (5 ml) was added to tert-butyl 2-(4-(3-(2,6-dioxopiperidine-3-yl)-1-methyl-4-oxo-3,4-dihydroptalazin-6-yl)piperazine-1-yl) acetate (150 mg, 0.32 mmol) in DCM (15 ml). The reactants were stirred at room temperature for 1 hour. After the end of the reaction, the reaction mixture was concentrated under reduced pressure. The residues were purified by MeOH/DCM (0-20%) column chromatography to obtain the title compound (100 mg) MS (ESI, m/z): [M+H]⁺ = 414.4

### Step 3) Preparation of 3-(1-(3-(5-((1-(2-(4-(3-(2-(3-(2,6-dioxopiperidine-3-yl)-1-methyl-4-oxo-3,4-dihydropthalazine-6-yl)piperazine-1-il)acetyl)piperidine-4-il)methoxy)pyrimidine-2-yl)benzyl)6-oxo-1,6-dihydropyridazine-3-yl)benzonitrile (ICT-0001214)

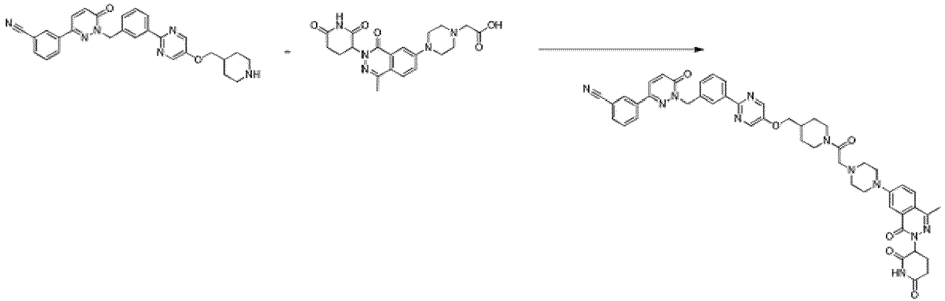

HATU (47.7 mg, 0.13 mmol) was added to 3-(6-oxo-1-(3-(5-(piperidine-4-ylmethoxy)pyrimidine-2-yl)benzyl)-1,6-dihydropyridazine-3-yl) benzonitrile (30 mg, 0.06 mmol) and 2-(4-(4-(3-(2,6-dioxopiperidine-3-yl)1-methyl-4-oxo-3,4-dihydroptalazine-6-yl)piperazine-1-yl)acetic acid (26.0 mg, 0.06 mmol), followed by ethylbis (propane-2-yl)amine (3 equivalent). The reactants were stirred at room temperature for 6 hours. The reactants were poured into water, extracted with ethyl acetate, dried with Magnesium sulfate, and concentrated under reduced pressure. The residue was purified by MeOH/DCM (0-20%) column chromatography to obtain the title compound (33 mg). MS (ESI, m/z): [M+H]+ = 875.1.
1H NMR (500 MHz, DMSO-d6) δ ppm 10.98 (s, 1 H) 8.65 (s, 2 H) 8.38 (br. s., 2 H) 8.20 - 8.28 (m, 2 H) 8.17 (d, J=9.77 Hz, 1 H) 7.93 (d, J=7.63 Hz, 1 H) 7.78 (d, J=8.85 Hz, 1 H) 7.71 (t, J=7.86 Hz, 1 H) 7.59 (d, J=8.54 Hz, 1 H) 7.54 (br. s., 1 H) 7.45 - 7.50 (m, 2 H) 7.16 (d, J=9.77 Hz, 1 H) 5.70 (br. s., 1 H) 5.44 (s, 2 H) 4.41 (d, J=12.82 Hz, 1 H) 4.08 (d, J=6.26 Hz, 3 H) 3.62 (dd, J=10.38, 6.56 Hz, 1 H) 3.41 (br. s., 4 H) 3.09 - 3.19 (m, 2 H) 3.05 (t, J=12.05 Hz, 1 H) 2.86 - 2.96 (m, 1 H) 2.54 - 2.66 (m, 6 H) 2.45 (s, 3 H) 2.07 (dd, J=8.85, 4.88 Hz, 2 H) 1.95 - 2.05 (m, 2 H) 1.81 (br. s., 2 H)

### Example C-34: Preparation of 3-(1-(3-(5-((1-(1-(3-(3-(3-(2,6-dioxopiperidin-3-yl)-1-methyl-4-oxo-3,4-dihydrophthalazine-6-yl)piperazine-1-il)propanoyl)piperidine-4-il)methoxy)pyrimidin-2-yl)benzyl)6-oxo-1,6-dihydropyridazine-3-yl)benzonitrile (ICT-0001215)

### Step 1) Preparation of tert-butyl 3-(4-(3-(2,6-dioxopiperidin-3-yl)-1-methyl-4-oxo-3,4-dihydroptalazine-6-yl)piperazine-1-yl)propanoate

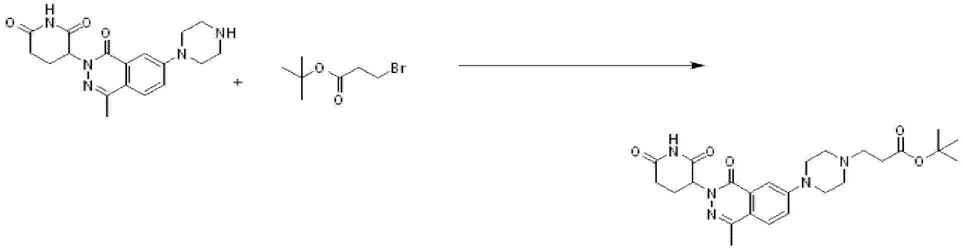

Ethylbis (propane-2-yl)amine (2.0 equivalent) was added to 3-(4-methyl-1-oxo-7-(piperazine-1-yl)phthalazine-2(1H)-yl)piperidine-2,6-dione (200 mg, 0.56 mmol) and tert-butyl 3-bromopropanoate (143 mg, 0.73 mmol) solution in ACN (10 ml). The reactants were stirred at room temperature for 5 hours. After the reaction was completed, the reaction mixture was concentrated under reduced pressure. The residues were purified by MeOH/DCM (0-20%) column chromatography to obtain the title compound (160 mg) MS (ESI, m/z): [M+H]⁺ = 484.4

### Step 2) Preparation of 3-(4-(3-(2,6-dioxopiperidin-3-yl)-1-methyl-4-oxo-3,4-dihydroptalazine-6-yl)piperazine-1-yl)propanoic acid

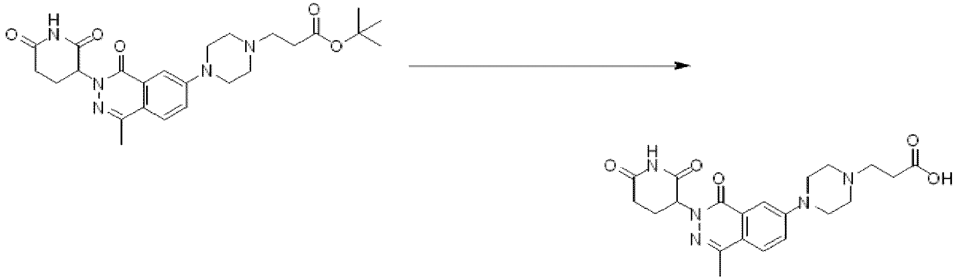

TFA (5 ml) was added to tert-butyl 3-(4-(3-(2,6-dioxopiperidine-3-yl)-1-methyl-4-oxo-3,4-dihydroptalazine-6-yl)piperazine-1-yl) propanoate (160 mg, 0.32 mmol) solution in DCM (15 ml). The reactants were stirred at room temperature for 1 hour. After the end of the reaction, the reaction mixture was concentrated under reduced pressure. The residues were purified by MeOH/DCM (0-20%) column chromatography to obtain the heading compound (100 mg) MS (ESI, m/z): [M+H]⁺ = 428.4

### Step 3) Preparation of 3-(1-(3-(5-((1-(3-(3-(4-(3-(3-(3-(2,6-dioxopiperidine-3-yl)-1-methyl-4-oxo-3,4-dihydropthalazine-6-yl)piperazine-1-il)propinoyl)piperidine-4-il)methoxy)pyrimidine-2-yl)benzyl)6-oxo-1,6-dihydropyridazine-3-yl)benzonitrile (ICT-0001215)

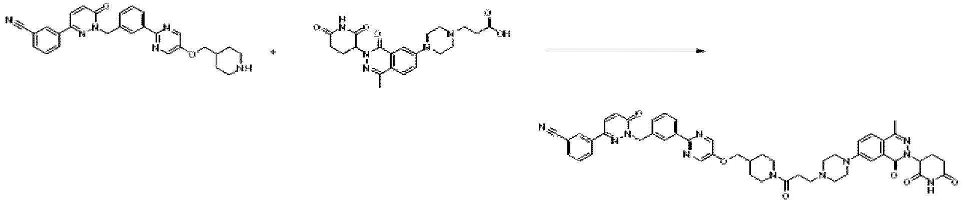

HATU (47.7 mg, 0.13 mmol) was added to 3-(6-oxo-1-(3-(5-(piperidine-4-ylmethoxy)pyrimidin-2-yl)benzyl)-1,6-dihydropyridazin-3-yl) benzonitrile (30 mg, 0.06 mmol) and 3-(4-(4-(2,6-dioxopiperidine-3-yl)1-methyl-4-oxo-3,4-dihydroptalazine-6-yl)piperazine-1-yl)propanoic acid (28.0 mg, 0.06 mmol) in DMF (2.0 ml), followed by ethylbis (propane-2-yl)amine (3 equivalent). The reactants were stirred at room temperature for 6 hours. The reactants were poured into water, extracted with ethyl acetate, dried with Magnesium sulfate, and concentrated under reduced pressure. The residue was purified by MeOH/DCM (0-20%) column chromatography to obtain the title compound (33 mg). MS (ESI, m/z): [M+H]+ = 875.1.
1H NMR (500 MHz, DMSO-d6) δ ppm 10.98 (s, 1 H) 8.65 (s, 2 H) 8.38 (br. s., 2 H) 8.20 - 8.28 (m, 2 H) 8.17 (d, J=9.77 Hz, 1 H) 7.93 (d, J=7.63 Hz, 1 H) 7.78 (d, J=8.85 Hz, 1 H) 7.71 (t, J=7.86 Hz, 1 H) 7.59 (d, J=8.54 Hz, 1 H) 7.54 (br. s., 1 H) 7.45 - 7.50 (m, 2 H) 7.16 (d, J=9.77 Hz, 1 H) 5.70 (br. s., 1 H) 5.44 (s, 2 H) 4.41 (d, J=12.82 Hz, 1 H) 4.08 (d, J=6.26 Hz, 3 H) 3.62 (dd, J=10.38, 6.56 Hz, 1 H) 3.41 (br. s., 4 H) 3.09 - 3.19 (m, 2 H) 3.05 (t, J=12.05 Hz, 1 H) 2.86 - 2.96 (m, 1 H) 2.54 - 2.66 (m, 10 H) 2.45 (s, 3 H) 2.07 (dd, J=8.85, 4.88 Hz, 2 H) 1.95 - 2.05 (m, 2 H) 1.81 (br. s., 2 H)

### Example C-35: Preparation of 3-(1-(3-(5-((1-(4-(4-(3-(2,6-dioxopiperidin-3-yl)-1-methyl-4-oxo-3,4-dihydroptalazine-6-yl)piperazine-1-il)butanoyl)piperidine-4-il)methoxy)pyrimidine-2-yl)benzyl)6-oxo-1,6-dihydropyridazine-3-yl)benzonitrile (ICT-0001216)

### Step 1) Preparation of tert-butyl 4-(4-(3-(2,6-dioxopiperidin-3-yl)-1-methyl-4-oxo-3,4-dihydroptalazine-6-yl)piperazine-1-yl)butanoate

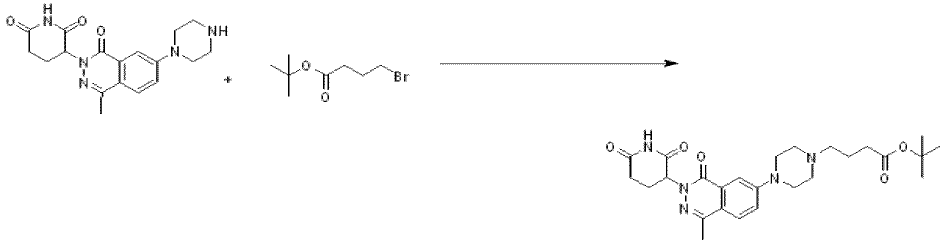

Ethylbis (propane-2-yl)amine (2.0 equivalent) was added to 3-(4-methyl-1-oxo-7-(piperazine-1-yl)phthalazine-2(1H)-yl)piperidine-2,6-dione (200 mg, 0.56 mmol) and tert-butyl 4-bromobutanoate (153 mg, 0.73 mmol) solutions in ACN (10 ml). The reactants were stirred at room temperature for 5 hours. After the reaction was completed, the reaction mixture was concentrated under reduced pressure. The residues were purified by MeOH/DCM (0-20%) column chromatography to obtain the heading compound (165 mg) MS (ESI, m/z): [M+H]⁺ = 498.4

### Step 2) Preparation of 4-(4-(3-(2,6-dioxopiperidin-3-yl)-1-methyl-4-oxo-3,4-dihydroptalazine-6-yl)piperazine-1-yl)butanoic acid

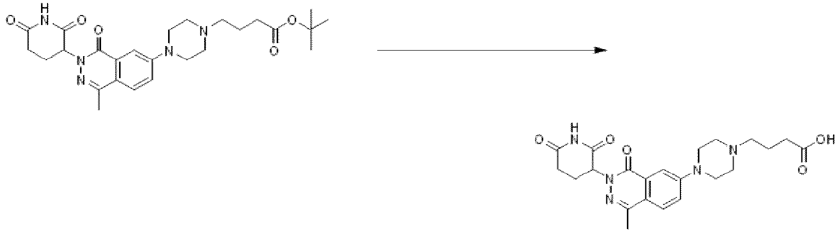

TFA (5 ml) was added to tert-butyl 4-(4-(3-(2,6-dioxopiperidine-3-yl)-1-methyl-4-oxo-3,4-dihydroptalazine-6-yl)piperazine-1-yl)butanoate (165 mg, 0.32 mmol) in DCM (15 ml). The reactants were stirred at room temperature for 1 hour. After the reaction was completed, the reaction mixture was concentrated under reduced pressure. The residues were purified by MeOH/DCM (0-20%) column chromatography to obtain the heading compound (100 mg) MS (ESI, m/z): [M+H]⁺ = 442.4

### Step 3) Preparation of 3-(1-(3-(5-((1-(4-(4-(3-(3-(2,6-dioxopiperidine-3-yl)-1-methyl-4-oxo-3,4-dihydropthalazine-6-yl)piperazine-1-il)butanoyl)piperidine-4-il)methoxy)pyrimidine-2-yl)benzyl)-6-oxo-1,6-dihydropyridazine-3-yl)benzonitrile (ICT-0001216)

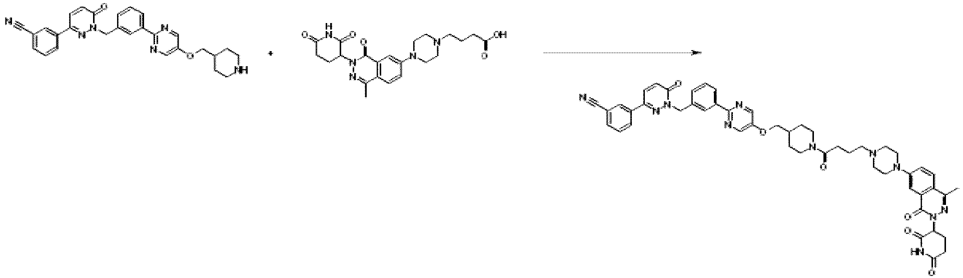

HATU (47.7 mg, 0.13 mmol) was added to 3-(6-oxo-1-(3-(5-(piperidine-4-ylmethoxy)pyrimidine-2-yl)benzyl)-1,6-dihydropyridazine-3-yl) benzonitrile (30 mg, 0.06 mmol) and 4-(4-(4-(3-(2,6-dioxopiperidine-3-yl)1-methyl-4-oxo-3,4-dihydropthalazine-6-yl)piperazine-1-yl)butanoic acid (30.0 mg, 0.06 mmol) in DMF (2.0 ml), followed by ethylbis (propane-2-yl)amine (3 equivalent). The reactants were stirred at room temperature for 6 hours. The reactants were poured into water, extracted with ethyl acetate, dried with Magnesium sulfate, and concentrated under reduced pressure. The residue was purified by MeOH/DCM (0-20%) column chromatography to obtain the title compound (33 mg). MS (ESI, m/z): [M+H]+ = 889.1.
1H NMR (500 MHz, DMSO-d6) δ ppm 10.98 (s, 1 H) 8.65 (s, 2 H) 8.38 (br. s., 2 H) 8.20 - 8.28 (m, 2 H) 8.17 (d, J=9.77 Hz, 1 H) 7.93 (d, J=7.63 Hz, 1 H) 7.78 (d, J=8.85 Hz, 1 H) 7.71 (t, J=7.86 Hz, 1 H) 7.59 (d, J=8.54 Hz, 1 H) 7.54 (br. s., 1 H) 7.45 - 7.50 (m, 2 H) 7.16 (d, J=9.77 Hz, 1 H) 5.70 (br. s., 1 H) 5.44 (s, 2 H) 4.41 (d, J=12.82 Hz, 1 H) 4.08 (d, J=6.26 Hz, 3 H) 3.62 (dd, J=10.38, 6.56 Hz, 1 H) 3.41 (br. s., 4 H) 3.09 - 3.19 (m, 2 H) 3.05 (t, J=12.05 Hz, 1 H) 2.86 - 2.94 (m, 1 H) 2.54 - 2.66 (m, 8 H) 2.45 (s, 3 H) 2.08 (dd, J=8.85, 4.88 Hz, 2 H) 1.95 - 2.02 (m, 2 H) 1.83 (br. s., 2 H)

### Example C-36: Preparation of 3-(1-(3-(5-((1-(5-(5-(5-(4-(3-(2,6-dioxopiperidin-3-yl)-1-methyl-4-oxo-3,4-dihydropetalazine-6-yl)piperazine-1-il)pentanoyl)piperidine-4-il)methoxy)pyrimidine-2-yl)benzyl)6-oxo-1,6-dihydropyridazine-3-yl)benzonitrile (ICT-0001217)

### Step 1) Preparation of tert-butyl 5-(4-(3-(2,6-dioxopiperidin-3-yl)-1-methyl-4-oxo-3,4-dihydroptalazine-6-yl)piperazine-1-yl)pentanoate

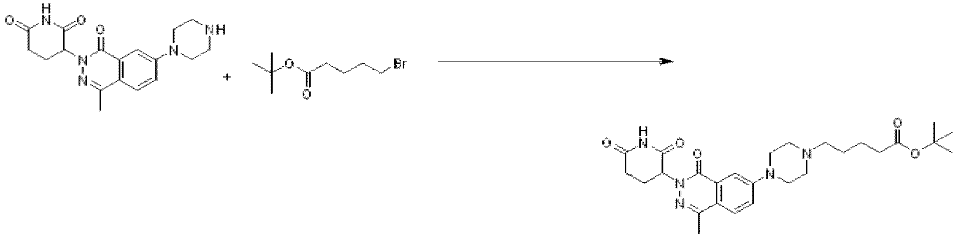

Ethylbis (propane-2-yl)amine (2.0 equivalent) was added to 3-(4-methyl-1-oxo-7-(piperazine-1-yl)phthalazine-2,6-dione (200 mg, 0.56 mmol) and tert-butyl 5-bromopentanoate (173 mg, 0.73 mmol) solutions in ACN (10 ml). The reactants were stirred at room temperature for 5 hours. After the reaction was completed, the reaction mixture was concentrated under reduced pressure. The residues were purified by MeOH/DCM (0-20%) column chromatography, and the heading compound was obtained (170 mg) MS (ESI, m/z): [M+H]⁺ = 512.4

### Step 2) Preparation of 5-(4-(3-(2,6-dioxopiperidin-3-yl)-1-methyl-4-oxo-3,4-dihydroptalazine-6-yl)piperazine-1-yl)pentanoic acid

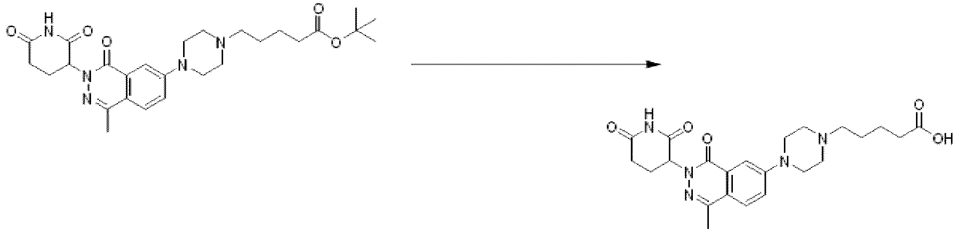

TFA (5 ml) was added to tert-butyl 5-(4-(3-(2,6-dioxopiperidine-3-yl)-1-methyl-4-oxo-3,4-dihydroptalazine-6-yl)piperazine-1-yl) pentanoate (165 mg, 0.32 mmol) in DCM (15 ml). The reactants were stirred at room temperature for 1 hour. After the reaction was completed, the reaction mixture was concentrated under reduced pressure. The residues were purified by MeOH/DCM (0-20%) column chromatography to obtain the heading compound (100 mg) MS (ESI, m/z): [M+H]⁺ = 456.4

### Step 3) Preparation of 3-(1-(3-(5-((1-(5-(1-(5-(4-(3-(2,6-dioxopiperidine-3-yl)-1-methyl-4-oxo-3,4-dihydroptalazine-6-yl)piperazine-1-il)pentanoyl)piperidine-4-il)methoxy)pyrimidine-2-yl)benzyl)6-oxo-1,6-dihydropyridazine-3-yl)benzonitrile (ICT-0001217)

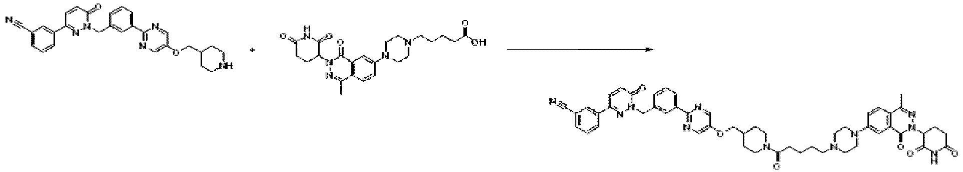

HATU (47.7 mg, 0.13 mmol) was added to 3-(6-oxo-1-(3-(5-(piperidine-4-ylmethoxy)pyrimidin-2-yl)benzyl)-1,6-dihydropyridazine-3-yl) benzonitrile (30 mg, 0.06 mmol) and 5-(4-(4-(2,6-dioxopiperidine-3-yl)1-methyl-4-oxo-3,4-dihydropthalazine-6-yl)piperazine-1-yl)piperazine-1-yl)pentanoic acid (32.0 mg, 0.06 mmol) in a solution of DMF (2.0 ml), followed by ethylbis (propane-2-yl)amine (3 equivalent). The reactants were stirred at room temperature for 6 hours. The reactants were poured into water, extracted with ethyl acetate, dried with Magnesium sulfate, and concentrated under reduced pressure. The residue was purified by MeOH/DCM (0-20%) column chromatography to obtain the title compound (33 mg). MS (ESI, m/z): [M+H]+ = 917.1.
1H NMR (500 MHz, DMSO-d6) δ ppm 10.96 (s, 1 H) 8.58 - 8.67 (m, 2 H) 8.34 - 8.41 (m, 2 H) 8.23 (t, J=8.62 Hz, 2 H) 8.17 (d, J=9.77 Hz, 1 H) 7.93 (d, J=7.78 Hz, 1 H) 7.74 - 7.81 (m, 1 H) 7.71 (t, J=7.86 Hz, 1 H) 7.59 (d, J=10.07 Hz, 1 H) 7.54 (br. s., 1 H) 7.44 - 7.51 (m, 2 H) 7.16 (d, J=9.77 Hz, 1 H) 5.69 (d, J=5.49 Hz, 1 H) 5.44 (s, 2 H) 4.44 (d, J=12.36 Hz, 1 H) 4.07(d, J=6.26 Hz, 2 H) 3.93 (d, J=12.66 Hz, 1 H) 3.42 - 3.54 (m, 2 H) 3.37 (br. s., 2 H) 3.04 (t, J=12.13 Hz, 1 H) 2.87 - 2.96 (m, 2 H) 2.52 - 2.66 (m, 6 H) 2.41 - 2.46 (m, 6 H) 2.36 (br. s., 3 H) 2.01 - 2.12 (m, 2 H) 1.74 - 1.88 (m, 2 H) 1.54 (br. s., 4 H)

### Example C-37: Preparation of 3-(1-(3-(5-((1-(2-(4-(4-(2-(2-(2-(2-(2-(2,6-dioxopiperidine-3-yl)-4-methyl-1-oxo-1,2-dihydrophthalazine-6-yl)piperazine-1-carbonyl)piperidine-1-il)acetyl)piperidine-4-il)methoxy)pyrimidine-2-yl)benzyl)6-oxo-1,6-dihydropyridazine-3-yl)benzonitrile (ICT-0001218)

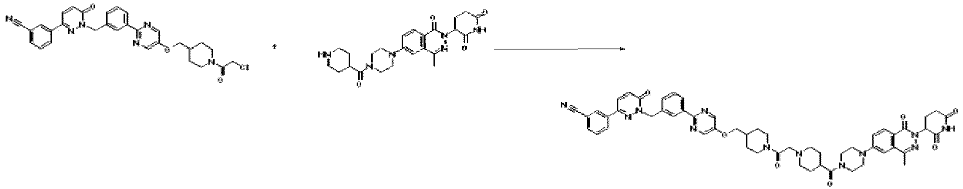

3-(1-(3-(5-(((1-(2-chloroacetyl)piperidine-4-yl)methoxy)pyrimidine-2-yl)benzyl)-6-oxo-1,in 0.6 ml DMSO DIPEA (123 µmol) was added to 6-dihydropyridazine-3-yl) benzonitrile (24.7 µmol) and 3-(4-methyl-1-oxo-6-(4-(piperidine-4-carbonyl)piperazine-1-yl)phthalazine-2(1H)-yl)piperidine-2,6-dione (24.7 µmol) solutions and stirred at 80°C for 16 hours. The reaction mixture was purified with reversed-phase column chromatography (water (0.1% FA) / ACN (0.1% FA) = 95/1 to 0/100 gradient). The corresponding fraction was lyophilized. The obtained products were processed by amino silica column chromatography (DCM/MeOH, 100/0 to 97/3, gradient) and obtained 13 mg of 3-(1-(3-(5-((1-(2-(4-(4-(2-(2-(2-(2-(2-(2-(2-(2,6-dioxopiperidine-3-yl)4-methyl-1-oxo-1,2-dihydroptalazine-6-yl)piperazine-1-carbonyl)piperidine-1-il)acetyl)piperidine-4-yl)methoxy)pyrimidine-2-yl)benzyl)6-oxo-1,6-dihydropyridazine-3-yl) benzonitrile. MS (ESI, m/z): [M+H]⁺ = 985.8
1H NMR (500 MHz, DMSO-d6) δ ppm 10.96 (s, 1H), 8.64 (s, 2H), 8.38 (s, 1H), 8.37 (s, 1H), 8.23 (m, 2H), 8.17 (d, J = 9.8 Hz, 1H), 8.08 (d, J = 9 Hz, 1H), 7.93 (d, J = 7.6 Hz, 1H), 7.71 (t, J = 7.9 Hz, 1H), 7.48 (m, 3H), 7.16 (d, J = 7.9 Hz, 1H), 7.09 (s, 1H), 5.68 (m, 1H), 5.44 (s, 2H), 4.38 (d, J = 12.5 Hz, 1H), 4.14 (d, J = 12.5 Hz, 1H), 4.08 (d, J = 6.6 Hz, 2H), 3.7 (brd, 2H), 3.63 (brd, 2H), 3.49 (brd, 2H), 3.45 (brd, 2H), 3.37 (m, 2H), 2.99 (m, 2H), 2.88 (m, 3H), 2.63 (brd, 2H), 2.58 (m, 2H), 2.48 (s, 3H), 2.07 (m, 5H), 1.81 (m, 2H), 1.62 (m, 4H)

### Example C-38: Preparation of 3-(1-(3-(5-((1-(2-(4-((1-(2-(1-(2-(2,6-dioxopiperidine-3-yl)-4-methyl-1-oxo-1,2-dihydrophthalazine-6-yl)piperidine-4-il)amino)piperidine-1-il)acetyl)piperidine-4-il)methoxy)pyrimidine-2-yl)benzyl)6-oxo-1,6-dihydropyridazine-3-yl)benzonitrile (ICT-0001219)

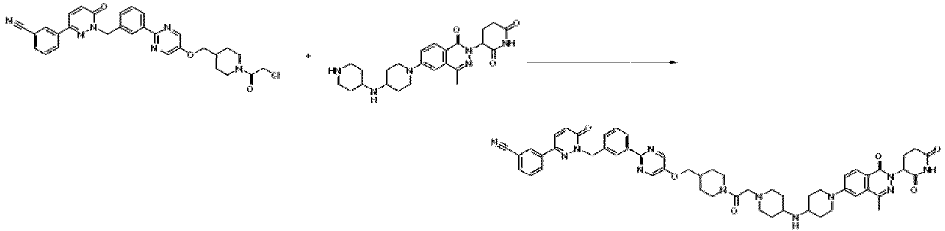

3-(1-(3-(5-((1-(1-(2-chloroacety)piperidine-4-yl)methoxy)pyrimidine-2-yl)benzyl)-6-oxo-1,in 0.6 ml DMSO DIPEA (123 µmol) was added to 6-dihydropyridazine-3-yl) benzonitrile (24.7 µmol) and 3-(4-methyl-1-oxo-6-(4-(piperidine-4-ylamino)piperidine-1-yl)phthalazine-2(1H)-yl)piperidine-2,6-dione (24.7 µmol) solutions, and stirred at 80°C for 16 hours. The reaction mixture was purified with reversed-phase column chromatography (water (0.1% FA) / ACN (0.1% FA) = 95/1 to 0/100 gradient). The corresponding fraction was lyophilized. The obtained products were processed by amino silica column chromatography (DCM/MeOH, 100/0 to 97/3, gradient) and 15 mg of 3-(1-(3-(5-((1-(2-(4-((1-(2-(2-(2-(2-(2-(2-(2,6-dioxopiperidine-3-yl)4-methyl-1-oxo-1,2-dihydropthalazine-6-yl)piperidine-4-yl)amino)piperidine-1-il)acetyl)piperidine-4-il)methoxy)pyrimidine-2-yl)benzyl)6-oxo-1,6-dihydropyridazine-3-yl) benzonitrile. MS (ESI, m/z): [M+H]⁺ = 971.8

1H NMR (500 MHz, DMSO-d6) δ ppm 10.97 (s, 1H), 8.65 (s, 2H), 8.39 (s, 1H), 8.37 (s, 1H), 8.23 (m, 2H), 8.17 (d, J = 9.8 Hz, 1H), 8.03 (d, J = 9 Hz, 1H), 7.93 (d, J = 7.6 Hz, 1H), 7.71 (t, J = 7.9 Hz, 1H), 7.48 (m, 3H), 7.16 (d, J = 9.8 Hz, 1H), 7.03 (s, 1H), 5.68 (m, 1H), 5.44 (s, 2H), 4.38 (d, J = 12.5 Hz, 1H), 4.12 (d, J = 12.5 Hz, 1H), 4.07 (d, J = 6.6 Hz, 2H), 3.97 (d, J = 12.7 Hz, 2H), 3.36 (m, 2H), 3.26 (d, J = 13 Hz, 2H), 2.97 (m, 5H), 2.88 (m, 2H), 2.80 (brd, 2H), 2.58 (m, 3H), 2.46 (s, 3H), 2.05 (m, 4H), 1.89 (m, 2H), 1.80 (brd, 3H) 1.28 (m, 6H).

### Example C-39: Preparation of 3-(1-(3-(5-((1-(2-(4-(2-(2-(2-(2,6-dioxopiperidine-3-yl)-1-oxo-1,2-dihydropthalazine-6-yl)piperazine-1-yl)-2-oxoethyl)piperidine-4-il)methoxy)pyrimidine-2-yl)benzyl)-6-oxo-1,6-dihydropyridazine-3-yl)benzonitrile (ICT-0001221)

### Step 1) Preparation of tert-butyl 2-(4-(((2-(3-((3-(3-cyanopronyl)-6-oxopyridazine-1(6H)-yl)methyl)phenyl)pyrimidine-5-il)oxy)methyl)piperidine-1-day)acetate

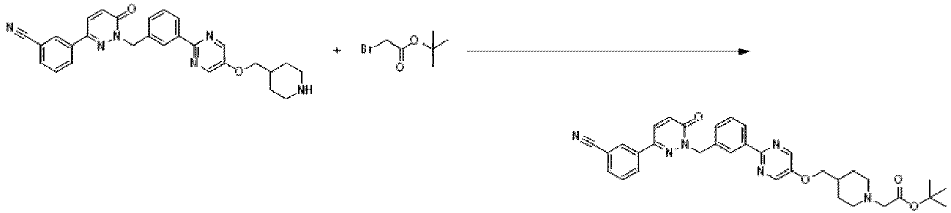

Ethylbis (propane-2-yl)amine (4.0 equivalent) was added to 3-(6-oxo-1-(3-(5-(piperidine-4-ylmethoxy)pyrimidin-2-yl)benzyl)-1,6-dihydropyridazine-3-yl)benzonitrile (200 mg, 0.42 mmol) and tert-butyl 2-bromoacetate (160 mg, 0.84 mmol) in a solution of NMP (2.0 ml). The reactants were stirred at 130°C for 48 hours. After the end of the reaction, the reaction mixture was concentrated under reduced pressure. The residues were purified by MeOH/DCM (0-20%) column chromatography. Subjective compound (150 mg) MS (ESI, m/z): [M+H]⁺ = 593.7

### Step 2) Preparation of 2-(4-(((2-(3-((3-(3-(3-cyanophenyl)-6-oxopyridazin-1(6H)-yl)methyl)phenyl)pyrimidine-5-il)oxy)methyl)piperidine-1-day)acetic acid

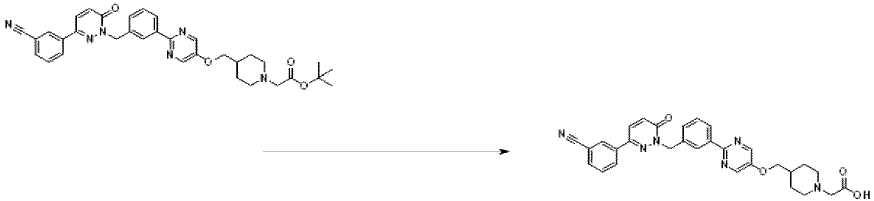

TFA (5 ml) was added to tert-butyl 2-(((2-(3-((3-(3-(3-(3-(3-(3-cyanophenyl)-6-oxopyridazine-1(6H)-yl)methyl)phenyl)pyrimidine-5-yl)oxy)methyl)piperidine-1-yl)acetate (150 mg, 0.31 mmol) in DCM (15 ml). The reactants were stirred at room temperature for 1 hour. After the reaction was finished, the reaction mixture was concentrated under reduced pressure. The residues were purified by MeOH/DCM (0-20%) column chromatography to obtain the heading compound (100 mg) MS (ESI, m/z): [M+H]⁺ = 537.6

### Step 3) Preparation of 3-(1-(3-(5-((1-(2-(4-(2-(2-(2-(2,6-dioxopiperidine-3-yl)-1-oxo-1,2-dihydropetalazine-6-yl)piperazine-1-il)2-oxoethyl)piperidine-4-il)methoxy)pyrimidine-2-yl)benzyl)6-oxo-1,6-dihydropyridazine-3-yl)benzonitrile (ICT-0001221)

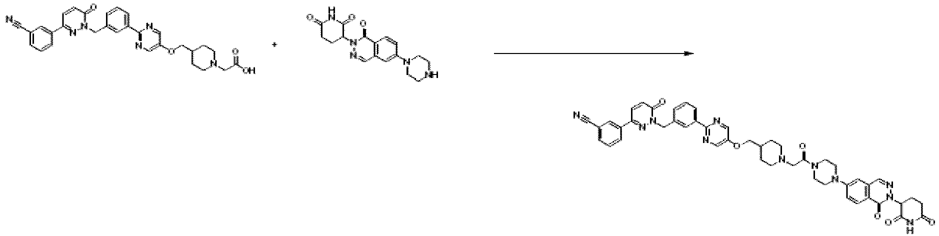

HATU (56.7 mg, 0.15 mmol) was added to 2-(4-(((2-(3-((3-(3-cyanophenyl)-6-oxopridazine-1(6H)-yl)methyl)phenyl)pyrimidine-5-yl)oxy)methyl)piperidine-1-yl)acetic acid (40 mg, 0.08 mmol) and 3-(1-oxo-6-(piperazine-1-yl)phthalazine-2,6-dione (1H)-yl)piperidine-2,6-dione (25.4 mg, 0.08 mmol) in DMF (2.0 ml), followed by ethylbis (propane-2-yl)amine (3 equivalent). The reactant was stirred at room temperature for 6 hours. The reactant was poured into water. It was extracted with ethyl acetate, dried with Magnesium sulfate, and concentrated under reduced pressure. The residue was purified by MeOH/DCM (0-20%) column chromatography to obtain the title compound (30 mg). MS (ESI, m/z): [M+H]⁺ = 860.9.
1H NMR (500 MHz, DMSO-d6) δ ppm 11.02 (s, 1 H) 8.66 (s, 2 H) 8.39 (s, 2 H) 8.13 - 8.30 (m, 4 H) 8.08 (d, J=8.85 Hz, 1 H) 7.94 (d, J=7.63 Hz, 1 H) 7.73 (t, J=7.86 Hz, 1 H) 7.45 - 7.58 (m, 3 H) 7.29 (d, J=1.83 Hz, 1 H) 7.17 (d, J=9.77 Hz, 1 H) 5.76 (dd, J=11.75, 5.04 Hz, 1 H) 5.45 (s, 2 H) 4.08 (br. s., 2 H) 3.57 - 3.75 (m, 6 H) 3.54 (br. s., 2 H) 3.42 - 3.51 (m, 2 H) 3.10 - 3.20 (m, 6 H) 2.86 - 2.97 (m, 2 H) 2.55 - 2.65 (m, 2 H) 1.26 - 1.31 (m, 3 H)

### Example C-40: Preparation of 3-(1-(3-(5-((1-(1-(3-(3-(3-(2-(2,6-dioxopiperidine-3-yl)-1-oxo-1,2-dihydropetalazine-6-yl)piperazine-1-yl)-3-oxopropyl)piperidine-4-il)methoxy)pyrimidine-2-yl)benzyl)6-oxo-1,6-dihydropyridazine-3-yl)benzonitrile (ICT-0001223)

### Step 1) Preparation of tert-Butyl 3-(4-(((2-(3-((3-(3-(3-cyanophenyl)-6-oxopyridazine-1(6H)-yl)methyl)phenyl)pyrimidine-5-il)oxy)methyl)piperidine-1-il)propanoate

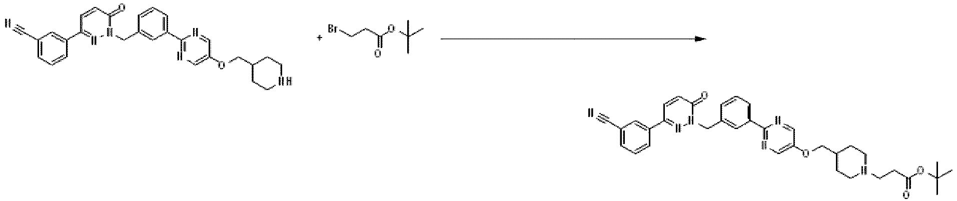

Ethylbis (propane-2-yl)amine (4.0 equivalent) was added to 3-(6-oxo-1-(3-(5-(piperidine-4-ylmethoxy)pyrimidine-2-yl)benzyl)benzonitrile (200 mg, 0.42 mmol) and tert-butyl 3-bromopropanoate (131 mg, 0.63 mmol) in NMP (2.0 ml). The reactants were stirred at 130°C for 48 hours. After the end of the reaction, the reaction mixture was concentrated under reduced pressure. The residues were purified by MeOH/DCM (0-20%) column chromatography. Subjective compound (100 mg) MS (ESI, m/z): [M+H]⁺ = 607.7

### Step 2) Preparation of 3-(4-(((2-(3-((3-(3-(3-cyanophenyl)-6-oxopyridazine-1(6H)-yl)methyl)phenyl)pyrimidine-5-il)oxy)methyl)piperidine-1-day)propanoic acid

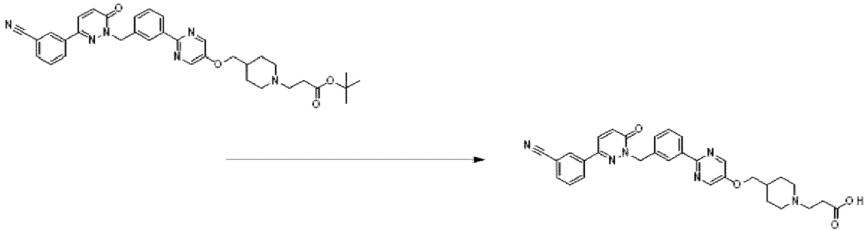

TFA (5 ml) was added to tert-butyl 3-(4-(((2-(3-((3-(3-(3-(3-cyanophenyl)-6-oxopyridazine-1(6H)-yl)methyl)phenyl)pyrimidine-5-yl)oxy)methyl)piperidine-1-yl)propanoate (150 mg, 0.25 mmol) in DCM. The reactants were stirred at room temperature for 1 hour. After the reaction was completed, the reaction mixture was concentrated under reduced pressure. The residue was purified by MeOH/DCM (0-20%) column chromatography to obtain the heading compound (100 mg) MS (ESI, m/z): [M+H]⁺ = 551.6

### Step 3) Preparation of 3-(1-(3-(5-((1-(3-(1-(3-(4-(2-(2,6-dioxopiperidine-3-yl)-1-oxo-1,2-dihydropthalazine-6-yl)piperazine-1-yl)-3-oxopropyl)piperidine-4-il)methoxy)pyrimidine-2-yl)benzyl)-6-oxo-1,6-dihydropyridazine-3-yl)benzonitrile (ICT-0001223)

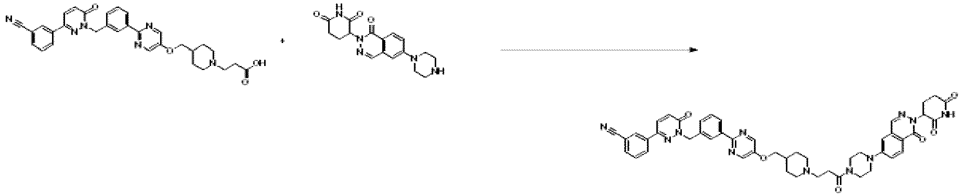

HATU (55.2 mg, 0.15 mmol) was added to 3-(4-((2-(3-((3-(3-cyanophenyl)-6-oxopridazine-1(6H)-yl)methyl)phenyl)pyrimidine-5-yl)oxy)methyl)piperidine-1-yl)propanoic acid (40 mg, 0.07 mmol) and 3-(1-oxo-6-(piperazine-1-yl)phthalazine-2,6-dione (1H)-yl)piperidine-2,6-dione (24.8 mg, 0.07 mmol) in DMF (2 ml), followed by ethylbis (propane-2-yl)amine (3 equivalent). The reactants were stirred at room temperature for 6 hours. The reactants were poured into water. It was extracted with ethyl acetate, dried with Magnesium sulfate, and concentrated under reduced pressure. The residue was purified by MeOH/DCM (0-20%) column chromatography to obtain the title compound (35 mg). MS (ESI, m/z): [M+H]⁺ = 875.0.
1H NMR (500 MHz, DMSO-d6) δ ppm 10.95 (s, 1 H) 8.58 (s, 2 H) 8.32 (s, 2 H) 8.14 - 8.23 (m, 3 H) 8.11 (d, J=9.77 Hz, 1 H) 8.00 (d, J=9.00 Hz, 1 H) 7.87 (d, J=7.63 Hz, 1 H) 7.66 (t, J=7.86 Hz, 1 H) 7.39 - 7.48 (m, 3 H) 7.21 (s, 1 H) 7.10 (d, J=9.61 Hz, 1 H) 5.69 (dd, J=11.60, 5.04 Hz, 1 H) 5.38 (s, 2 H) 4.00 (br. s., 2 H) 3.59 (br. s., 4 H) 3.45 (br. s., 2 H) 3.38 (br. s., 3 H) 2.80 - 2.89 (m, 2 H) 2.47 - 2.59 (m, 3 H) 2.00 - 2.06 (m, 2 H) 1.82 - 1.93 (m, 2 H) 1.80 (br. s., 1 H) 1.40 (d, J=6.87 Hz, 2 H) 1.17 (s, 4 H)

### Example C-41: Preparation of 3-(1-{[3-(5-{[1-(3-{4-[2-(2,6-dioxopiperidin-3-yl)-4-methyl-1-oxo-1,2-dihydropthalazine-6-yl]piperazine-1-yl}-3-oxopropyl-4-yl]methoxy}pyrimidine-2-yl)phenyl]methyl}-6-oxo-1,6-dihydropyridazine-3-yl)benzonitrile (ICT-0001224)

### Step 1) Preparation of tert-butyl 4-(2-(2,6-dioxopiperidin-3-yl)-4-methyl-1-oxo-1,2-dihydroptalazine-6-yl)piperazine-1-carboxylate

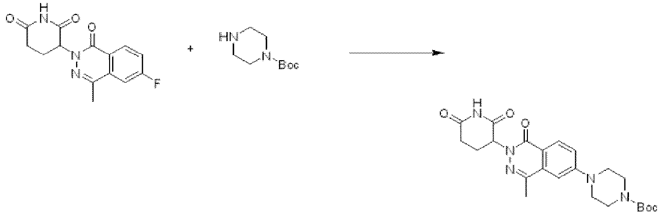

Ethylbis (propane-2-yl)amine (4.0 equivalent) was added to 3-(6-fluoro-4-methyl-1-oxopthalazine-2(1H)-yl) piperidine-2,6-dione (300 mg, 1.04 mmol) and tert-butyl piperazine-1-carboxylate (386 mg, 2.07 mmol) solutions in NMP (3.0 ml). The reactants were stirred at 130°C for 48 hours. After the reaction was completed, the reaction mixture was concentrated under reduced pressure. The residues were purified by MeOH/DCM (0-20%) column chromatography to obtain the heading compound (350 mg) MS (ESI, m/z): [M+H]⁺ = 456.4

### Step 2) Preparation of 3-(4-methyl-1-oxo-6-(piperazine-1-yl)phthalazine-2(1H)-yl)piperidine-2,6-dione

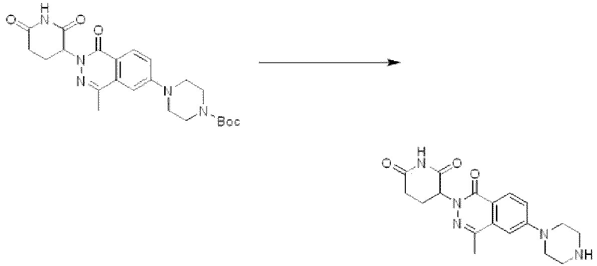

TFA (10 ml) was added to tert-butyl 4-(2-(2,6-dioxopiperidine-3-yl)-4-methyl-1-oxo-1,2-dihydroptalazine-6-yl) solution in DCM (30 ml). The reactants were stirred at room temperature for 1 hour. After the end of the reaction, the reaction mixture was concentrated under reduced pressure. The residues were purified by MeOH/DCM (0-20%) column chromatography to obtain the heading compound (200 mg) MS (ESI, m/z): [M+H]⁺ = 356.4

### Step 3) Preparation of 3-(1-(3-(5-((1-(3-(3-(4-(2-(2,6-dioxopiperidine-3-yl)-4-methyl-1-oxo-1,2-dihydropthalazine-6-yl)piperazine-1-yl)-3-oxopropyl)piperidine-4-il)methoxy)pyrimidine-2-yl)benzyl)-6-oxo-1,6-dihydropyridazine-3-yl)benzonitrile (ICT-0001224)

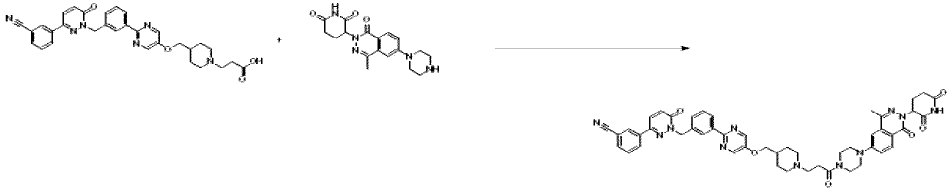

HATU (55.2 mg, 0.15 mmol) was added to 3-(4-(((2-(3-((3-(3-cyanophenyl)-6-oxopyridazin-1(6H)-yl)methyl)phenyl)pyrimidine-5-yl)oxy)methyl)piperidine-1-yl)propanoic acid (30 mg, 0.06 mmol) and 3-(4-methyl-1-oxo-6-(piperazine-1-yl)phthalazine-2(1H)-yl)piperidine-2,6-dione (25.8 mg, 0.06 mmol) in DMF (2.0 ml), followed by ethylbis (propane-2-yl)amine (3 equivalent). The reactants were stirred at room temperature for 6 hours. The reactants were poured into water. It was extracted with ethyl acetate, dried with Magnesium sulfate, and concentrated under reduced pressure. The residue was purified by MeOH/DCM (0-20%) column chromatography to obtain the title compound (20 mg). MS (ESI, m/z): [M+H]⁺ = 889.1.
1H NMR (500 MHz, DMSO-d6) δ ppm 10.98 (s, 1 H) 8.66 (s, 2 H) 8.39 (s, 2 H) 8.21 - 8.29 (m, 2 H) 8.19 (d, J=9.77 Hz, 1 H) 8.06 - 8.14 (m, 1 H) 7.94 (d, J=7.78 Hz, 1 H) 7.73 (t, J=7.86 Hz, 1 H) 7.44 - 7.58 (m, 3 H) 7.17 (d, J=9.77 Hz, 1 H) 7.11 (s, 1 H) 5.67 - 5.73 (m, 1 H) 5.45 (s, 2 H) 4.08 (br. s., 1 H) 3.67 (br. s., 4 H) 3.44 - 3.58 (m, 4 H) 3.12 (br. s., 1 H) 3.08 (s, 1 H) 2.87 - 2.98 (m, 2 H) 2.75 (br. s., 1 H) 2.70 (br. s., 1 H) 2.54 - 2.67 (m, 11 H) 2.03 - 2.09 (m, 1 H) 1.90 - 2.00 (m, 1 H) 1.24 (s, 2 H)

### Example C-42: Preparation of 3-(1-(3-(5-((1-(3-(1-(3-(4-(3-(2,6-dioxopiperidin-3-yl)-1-methyl-4-oxo-3,4-dihydropetthalazine-6-yl)piperazine-1-yl)-3-oxopropyl)piperidine-4-yl)methoxy)pyrimidine-2-yl)benzyl)6-oxo-1,6-dihydropyridazine-3-yl)benzonitrile (ICT-0001225)

### Step 1) Preparation of tert-butyl 4-(3-(2,6-dioxopiperidin-3-yl)-1-methyl-4-oxo-3,4-dihydroptalazine-6-yl)piperazine-1-carboxylate

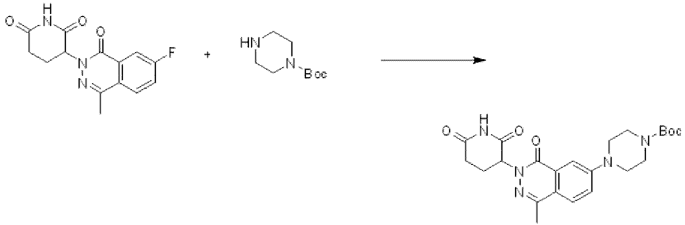

Ethylbis (propane-2-yl)amine (4.0 equivalent) was added to 3-(7-fluoro-4-methyl-1-oxopthalazine-2(1H)-yl)piperidine-2,6-dione (300 mg, 1.04 mmol) and tert-butylpiperazine-1-carboxylate (400 mg, 2.07 mmol) solutions in NMP (3 ml). The reactants were stirred at 130°C for 48 hours. After the end of the reaction, the reaction mixture was concentrated under reduced pressure. The residues were purified by MeOH/DCM (0-20%) column chromatography to obtain the title compound (350 mg) MS (ESI, m/z): [M+H]⁺ = 456.4

### Step 2) Preparation of 3-(4-methyl-1-oxo-7-(piperazine-1-yl)phthalazine-2(1H)-yl)piperidine-2,6-dione

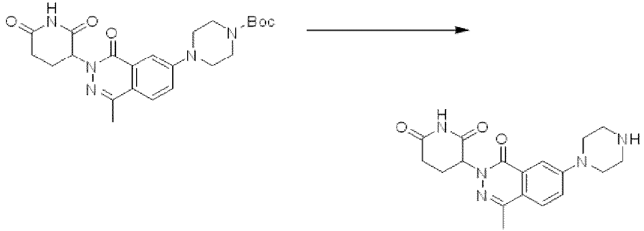

TFA (10 ml) was added to tert-butyl 4-(3-(2,6-dioxopiperidine-3-yl)-1-methyl-4-oxo-3,4-dihydroptalazin-6-yl) solution in DCM (30 ml). TFA (10 ml) was added to the tert-butyl 4-(3,6-dioxopiperidine-3-yl)-piperazine-1-carboxylate-6-yl. The reactants were stirred at room temperature for 1 hour. After the reaction was completed, the reaction mixture was concentrated under reduced pressure. The residues were purified by MeOH/DCM (0-20%) column chromatography to obtain the title compound (200 mg) MS (ESI, m/z): [M+H]⁺ = 356.4

### Step 3) Preparation of 3-(1-(3-(5-((1-(3-(3-(3-(4-(3-(3-(2,6-dioxopiperidine-3-yl)-1-methyl-4-oxo-3,4-dihydroptalazine-6-yl)piperazine-1-yl)-3-oxopropyl)piperidine-4-il)methoxy)pyrimidine-2-yl)benzyl)-6-oxo-1,6-dihydropyridazine-3-yl)benzonitrile (ICT-0001225)

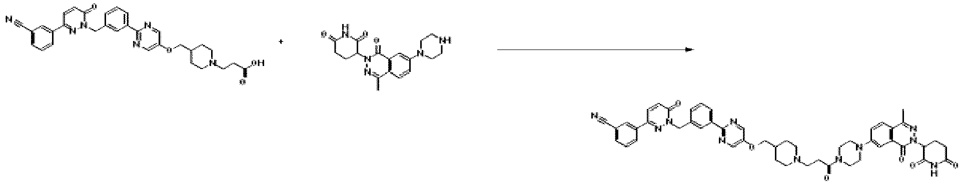

HATU (55.2 mg, 0.15 mmol) was added to 3-(4-((2-(3-((3-(3-cyanophenyl)-6-oxopyridazin-1(6H)-yl)methyl)phenyl)pyrimidine-5-yl)oxy)methyl)piperidine-1-yl)propanoic acid (40 mg, 0.07 mmol) and 3-(4-methyl-1-oxo-7-(piperazine-1-yl)phthalazine-2,6-(1H)-yl)piperidine-2,6-dione (25.8 mg, 0.07 mmol) in DMF (2.0 ml), followed by ethylbis (propane-2-yl)amine (3 equivalent). The reactants were stirred at room temperature for 6 hours. The reactants were poured into water. It was extracted with ethyl acetate, dried with Magnesium sulfate, and concentrated under reduced pressure. The residue was purified by MeOH/DCM (0-20%) column chromatography to obtain the title compound (30 mg). MS (ESI, m/z): [M+H]⁺ = 889.1.
1H NMR (500 MHz, DMSO-d6) δ ppm 11.00 (s, 1 H) 8.62 - 8.67 (m, 2 H) 8.39 (s, 2 H) 8.21 - 8.28 (m, 2 H) 8.19 (d, J=9.77 Hz, 1 H) 7.94 (d, J=7.63 Hz, 1 H) 7.83 (d, J=9.16 Hz, 1 H) 7.73 (t, J=7.86 Hz, 1 H) 7.63 (dd, J=9.00, 2.29 Hz, 1 H) 7.55 (d, J=2.29 Hz, 1 H) 7.47 - 7.53 (m, 2 H) 7.17 (d, J=9.77 Hz, 1 H) 5.71 (d, J=5.95 Hz, 1 H) 5.45 (s, 2 H) 4.09 (d, J=6.10 Hz, 2 H) 3.67 (br. s., 4 H) 3.49 (br. s., 2 H) 3.43 (br. s., 1 H) 2.87 - 2.97 (m, 1 H) 2.71 - 2.81 (m, 2 H) 2.54 - 2.64 (m, 8 H) 2.47 (s, 3 H) 2.04 - 2.11 (m, 1 H) 1.89 (d, J=12.82 Hz, 1 H) 1.49 (d, J=11.29 Hz, 1 H) 1.25 (br. s., 4 H)

### Example C-43: Preparation of 3-(1-(3-(5-((1-(1-(4-(4-(4-(2-(2,6-dioxopiperidin-3-yl)-1-oxo-1,2-dihydropthalazine-6-yl)piperazine-1-yl)-4-oxobutyl)piperidine-4-il)methoxy)pyrimidin-2-yl)benzyl)6-oxo-1,6-dihydropyridazine-3-yl)benzonitrile (ICT-0001226)

### Step 1) Preparation of tert-Butyl 4-(4-(((2-(3-((3-(3-cyanophenyl)-6-oxopridazine-1(6H)-yl)methyl)phenyl)pyrimidine-5-il)oxy)methyl)piperidine-1-day)butanoate

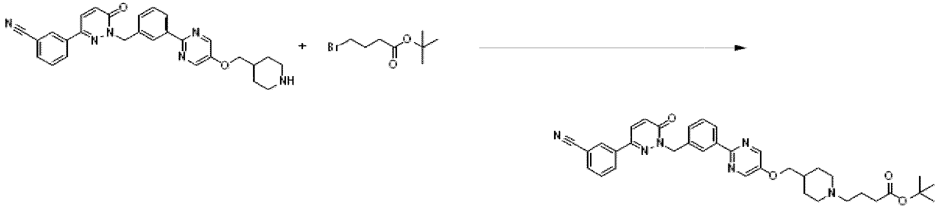

Ethylbis (propane-2-yl)amine (4.0 equivalent) was added to 3-(6-oxo-1-(3-(5-(piperidine-4-ylmethoxy)pyrimidin-2-yl)benzyl)benzonitrile (200 mg, 0.42 mmol) and tert-butyl 4-bromobutanoate (280 mg, 1.25 mmol) in NMP (2.0 ml). The reactants were stirred at 130°C for 48 hours. After the end of the reaction, the reaction mixture was concentrated under reduced pressure. The residues were purified by MeOH/DCM (0-20%) column chromatography. Subjective compound (100 mg) MS (ESI, m/z): [M+H]⁺ = 621.7

### Step 2) Preparation of 4-(4-(((2-(3-((3-(3-(3-cyanophenyl)-6-oxopyridazine-1(6H)-yl)methyl)phenyl)pyrimidine-5-il)oxy)methyl)piperidine-1-il)butanoic acid

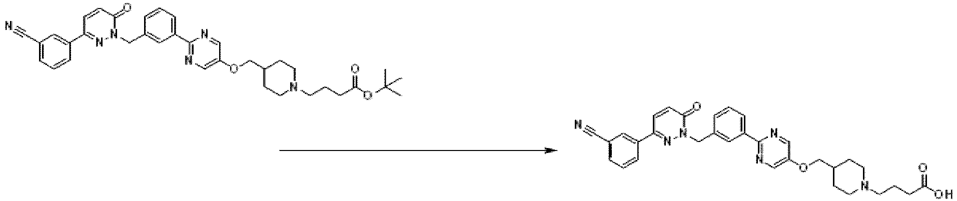

TFA (5 ml) was added to tert-butyl 4-(4-(((2-(3-((3-(3-(3-(3-cyanophenyl)-6-oxopyridazin-1(6H)-yl)methyl)phenyl)pyrimidine-5-yl)oxy)methyl)piperidine-1-yl)butanoate (150 mg, 0.25 mmol) solution. The reactants were stirred at room temperature for 1 hour. After the reaction was finished, the reaction mixture was concentrated under reduced pressure. The residues were purified by MeOH/DCM (0-20%) column chromatography to obtain the heading compound (100 mg) MS (ESI, m/z): [M+H]⁺ = 565.6

### Step 3) Preparation of 3-(1-(3-(5-((1-(4-(4-(2-(2,6-dioxopiperidine-3-yl)-1-oxo-1,2-dihydropetalazine-6-yl)piperazine-1-il)-4-oxobutyl)piperidine-4-il)methoxy)pyrimidine-2-yl)benzyl)-6-oxo-1,6-dihydropyridazine-3-yl)benzonitrile (ICT-0001226)

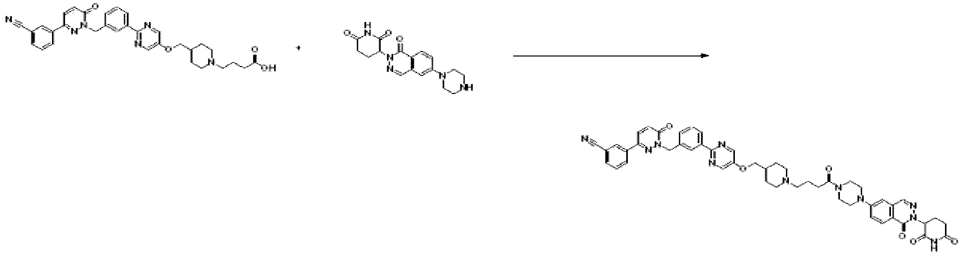

HATU (54.0 mg, 0.14 mmol) was added to 4-(((2-(3-((3-(3-(3-cyanophenyl)-6-oxopyridazine-1(6H)-yl)methyl)phenyl)pyrimidine-5-yl)oxy)methyl)piperidine-1-yl)butanoic acid (40 mg, 0.07 mmol) and 3-(1-oxo-6-(piperazine-1-yl)phthalazine-2,6-dione (1H)-yl)piperidine-2,6-dione (24.2 mg, 0.07 mmol) in DMF (2.0 ml), followed by ethylbis (propane-2-yl)amine (3.0 equivalent). The reactants were stirred at room temperature for 6 hours. It was extracted with ethyl acetate, dried with Magnesium sulfate, and concentrated under reduced pressure. The residues were purified by MeOH/DCM (0-20%) column chromatography to obtain the title compound (50 mg). MS (ESI, m/z): [M+H]⁺ = 889.0.
1H NMR (500 MHz, DMSO-d6) δ ppm 11.01 (s, 1 H) 8.65 (s, 2 H) 8.39 (s, 2 H) 8.22 - 8.29 (m, 3 H) 8.19 (d, J=9.77 Hz, 1 H) 8.06 (d, J=8.85 Hz, 1 H) 7.94 (d, J=7.93 Hz, 1 H) 7.73 (t, J=7.86 Hz, 1 H) 7.45 - 7.53 (m, 3 H) 7.27 (d, J=2.29 Hz, 1 H) 7.17 (d, J=9.77 Hz, 1 H) 5.70 - 5.81 (m, 1 H) 5.45 (s, 2 H) 4.05 (d, J=5.80 Hz, 2 H) 3.59 - 3.69 (m, 4 H) 3.35 - 3.47 (m, 6 H) 2.85 - 2.98 (m, 2 H) 2.53 - 2.65 (m, 2 H) 2.35 - 2.43 (m, 5 H) 1.90 - 2.02 (m, 2 H) 1.69 - 1.82 (m, 2 H) 1.18 (br. s., 4 H)

### Example C-44: Preparation of 3-(1-(3-(5-((1-(4-(4-(2-(2,6-dioxopiperidin-3-yl)-4-methyl-1-oxo-1,2-dihydropetalazine-6-yl)piperazine-1-yl)4-oxobutyl)piperidine-4-yl)methoxy)pyrimidine-2-yl)benzyl)6-oxo-1,6-dihydropyridazine-3-yl)benzonitrile (ICT-0001227)

### Step 1) Preparation of 3-(1-(3-(5-((1-(4-(4-(2-(2,6-dioxopiperidine-3-yl)-4-methyl-1-oxo-1,2-dihydropthalazine-6-yl)piperazine-1-yl)-4-oxobutyl)piperidine-4-il)methoxy)pyrimidine-2-yl)benzyl)benzonitrile (ICT-0001227)

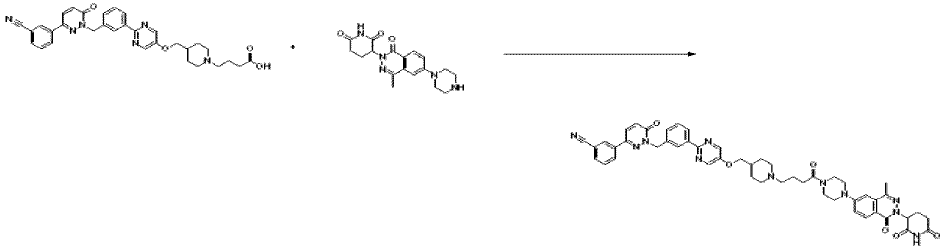

HATU (54.0 mg, 0.14 mmol) was added to 4-(4-(((2-(3-((3-(3-cyanophenyl)-6-oxopyridase-1(6H)-yl)methyl)phenyl)pyrimidine-5-yl)oxy)methyl)piperidine-1-yl)butanoic acid (40 mg, 0.07 mmol) and 3-(4-methyl-1-oxo-6-(piperazine-1-yl)phthalazine-2(1H)-yl)piperidine-2,6-dione (25.2 mg, 0.07 mmol) in DMF (2.0 ml), followed by ethylbis (propane-2-yl)amine (3.0 equivalent). The reactant was stirred at room temperature for 6 hours. It was extracted with ethyl acetate, dried with Magnesium sulfate, and concentrated under reduced pressure. The residues were purified by MeOH/DCM (0-20%) column chromatography to obtain the title compound (50 mg). MS (ESI, m/z): [M+H]⁺ = 903.0.
1H NMR (500 MHz, DMSO-d6) δ ppm 10.91 (s, 1 H) 8.57 (s, 2 H) 8.31 (s, 2 H) 8.27 (d, J=8.24 Hz, 1 H) 8.14 - 8.21 (m, 2 H) 8.02 (d, J=9.00 Hz, 1 H) 7.87 (d, J=7.78 Hz, 1 H) 7.65 (t, J=7.93 Hz, 1 H) 7.40 - 7.46 (m, 3 H) 7.10 (d, J=9.77 Hz, 1 H) 7.02 (s, 1 H) 5.62 (d, J=7.02 Hz, 1 H) 5.38 (s, 2 H) 4.00 (d, J=6.10 Hz, 2 H) 3.57 (br. s., 4 H) 3.44 (br. s., 2 H) 3.39 (br. s., 2 H) 2.78 - 2.89 (m, 2 H) 2.46 - 2.60 (m, 7 H) 2.37 (t, J=6.94 Hz, 2 H) 1.97 - 2.04 (m, 2 H) 1.71 - 1.93 (m, 2 H) 1.39 (d, J=10.99 Hz, 1 H) 1.16 (br. s., 6 H)

### Example C-45 : Preparation of 3-(1-(3-(5-((1-(4-(4-(3-(2,6-dioxopiperidin-3-yl)-1-methyl-4-oxo-3,4-dihydropetalazine-6-ylpiperazine-1-yl)piperazine-4-oxobutyl)piperidine-4-yl)methoxy)pyrimidine-2-yl)benzyl)6-oxo-1,6-dihydropyridazine-3-yl)benzonitrile (ICT-0001228)

### Step 1) Preparation of 3-(1-(3-(5-((1-(4-(4-(3-(3-(3-(2,6-dioxopiperidin-3-yl)-1-methyl-4-oxo-3,4-dihydropthalazine-6-yl)piperazine-1-yl)-4-oxobutyl)piperidine-4-il)methoxy)pyrimidine-2-yl)benzyl)-6-oxo-1,6-dihydropyridazine-3-yl)benzonitrile (ICT-0001228)

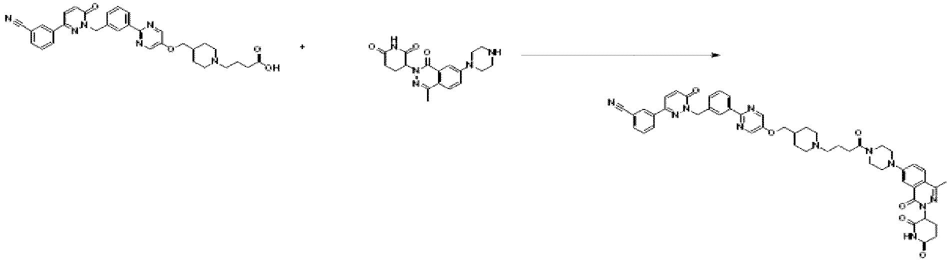

HATU (54.0 mg, 0.14 mmol) was added to 4-(((2-(3-((3-(3-(3-(3-(3-cyanophenyl)6-oxopyridase-1(6H)-yl)methyl)phenyl)pyrimidine-5-yl)oxy)methyl)piperidine-1-yl)butanoic acid (40 mg, 0.07 mmol) and 3-(4-methyl-1-oxo-7-(piperazine-1-yl)phthalazine-2,6-dione (1H)-yl)piperidine-2,6-dione (25.2 mg, 0.07 mmol) in DMF (2.0 ml), followed by ethylbis (propane-2-yl)amine (3.0 equivalent). The reactant was stirred at room temperature for 6 hours. The reactant was poured into water. It was extracted with ethyl acetate, dried with Magnesium sulfate, and concentrated under reduced pressure. The residues were purified by MeOH/DCM (0-20%) column chromatography to obtain the title compound (50 mg). MS (ESI, m/z): [M+H]⁺ = 903.0.
1H NMR (500 MHz, DMSO-d6) δ ppm 10.92 (s, 1 H) 8.57 (s, 2 H) 8.31 (s, 2 H) 8.14 - 8.20 (m, 2 H) 8.11 (d, J=9.77 Hz, 1 H) 7.87 (d, J=7.78 Hz, 1 H) 7.74 (d, J=9.00 Hz, 1 H) 7.65 (t, J=7.78 Hz, 1 H) 7.51 - 7.56 (m, 1 H) 7.45 - 7.49 (m, 1 H) 7.39 - 7.45 (m, 2 H) 7.10 (d, J=9.77 Hz, 1 H) 5.63 (d, J=6.87 Hz, 1 H) 5.38 (s, 2 H) 3.99 (d, J=5.80 Hz, 2 H) 3.58 (br. s., 4 H) 3.39 (br. s., 3 H) 3.34 (br. s., 3 H) 2.78 - 2.90 (m, 1 H) 2.45 - 2.59 (m, 4 H) 2.33 - 2.41 (m, 10 H) 1.95 - 2.05 (m, 2 H) 1.84 - 1.95 (m, 1 H) 1.66 - 1.80 (m, 2 H)

### Example C-46: Preparation of 3-(1-(3-(5-((1-(2-(4-(((2R)-4-(2-(2-(2,6-dioxopiperidin-3-yl)-4-methyl-1-oxo-1,2-dihydrophthalazine-6-yl)morpholine-2-il)methyl)piperazine-1-il)acetyl)piperidine-4-il)methoxy)pyrimidine-2-yl)benzyl)6-oxo-1,6-dihydropyridazine-3-yl)benzonitrile (ICT-0001229)

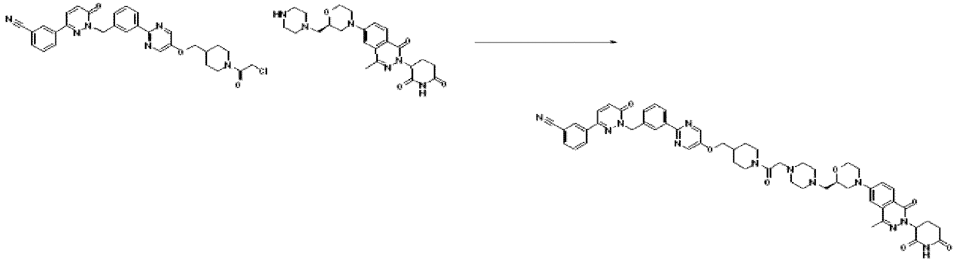

3-(1-(3-(5-(((1-(2-chloroacetyl)piperidine-4-yl)methoxy)pyrimidine-2-yl)benzyl)6-oxo-1,in 0.6 ml DMSO DIPEA (98 µmol) was added to 6-dihydropyridazine-3-yl) benzonitrile (18 µmol) and 3-(4-methyl-1-oxo-6-((R)-2-(piperazine-1-ylmethyl)morpholino)phthalazine-2(1H)-yl)piperidine-2,6-dione (18 µmol) and stirred at 80°C for 16 hours. The reaction mixture was purified with reversed-phase column chromatography (water (0.1% FA) / ACN (0.1% FA) = 95/1 to 0/100 gradient). The corresponding fraction was lyophilized. The obtained product was processed with amino silica column chromatography (DCM/ MeOH, 100/0 to 97/3, gradient) and obtained 5.6 mg of 3-(1-(3-(5-((1-(2-(2-(4-((2R)-4-(2-(2,6-dioxopiperidine-3-yl)-4-methyl-1-oxo-1,2-dihydroptalazin-6-yl)morpholine-2-yl)methyl)piperazine-1-il)acetyl)piperidine-4-il)methoxy)pyrimidine-2-yl)benzyl)6-oxo-1,6-dihydropyridazine-3-yl) benzonitrile. MS (ESI, m/z): [M+H]⁺ = 973.8
1H NMR (500 MHz, DMSO-d6) δ ppm 10.98 (s, 1H), 8.65 (s, 2H), 8.38 (s, 1H), 8.37 (s, 1H), 8.23 (m, 2H), 8.17 (d, J = 9.8 Hz, 1H), 8.08 (d, J = 9 Hz, 1H), 7.93 (d, J = 7.6 Hz, 1H), 7.71 (t, J = 7.9 Hz, 1H), 7.48 (m, 3H), 7.16 (d, J = 9.8 Hz, 1H), 7.07 (s, 1H), 5.68 (m, 1H), 5.44 (s, 2H), 5.32 (s, 2H), 4.38 (d, J = 12.5 Hz, 1H), 4.09 - 4.03 (m, 2H), 3.97 (m, 1H), 3.87 (m, 2H), 3.71 (m, 2H), 3.60 (m, 2H) 3.53 - 3.47 (m, 2H), 3.40 - 3.35 (m, 2H), 2.98 (m, 2H), 2.88 (m, 2H), 2.65 - 2.55 (m, 3H), 2.48 (s, 3H), 2.45 (brd, 2H), 2.06 (m, 2H), 1.80 (m, 2H), 1.38 - 1.10 (brd, 4H)

### Example C-47: Preparation of 3-(1-(3-(5-((1-(2-(4-(4-(4-(3-(2,6-dioxopiperidine-3-yl)-7-fluoro-1-methyl-4-oxo-3,4-dihydrophthalazine-6-yl)piperazine-1-il)methyl)piperidine-1-il)-2-oxoethyl)piperidine-4-yl)methoxy)pyrimidine-2-yl)benzyl)6-oxo-1,6-dihydropyridazine-3-yl)benzonitrile (ICT-0001231)

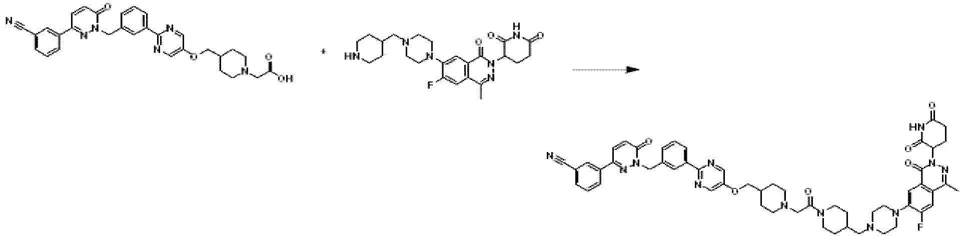

HATU (34.0 mg, 0.12 mmol) is added to the solution of 2-(4-(((2-(3-(3-(3-cyanophenyl)-6-oxopridazine-1(6H)-yl)methyl)phenyl)pyrimidine-5-yl)oxy)methyl)piperidine-1-yl) acetic acid (46.6 mg, 0.09 mmol) and 3-(6-fluoro-4-methyl-1-oxo-7-(4-(piperidine-4-yl)piperazine-1-(4-(1H)-yl)piperazine-2,6-dione (40.9 mg, 0.09 mmol) in DMF (2 ml) at room temperature, followed by ethylbis (propane-2-yl)amine (33.7 mg, 0.26 mmol). The reactants were stirred for 6 hours. The reactants were poured into water, extracted with ethyl acetate, dried with Magnesium sulfate, and concentrated under reduced pressure. The residue was purified with MeOH/DCM (0-20%) MPLC to obtain the title compound (43 mg). MS (ESI, m/z): [M+H]⁺ = 990.0
1H NMR (500 MHz, DMSO-d6) δ ppm 11.02 (s, 1 H) 9.43 (br. s., 1 H) 8.58 - 8.80 (m, 2 H) 8.34 - 8.46 (m, 2 H) 8.11 - 8.28 (m, 3 H) 7.94 (d, J=7.63 Hz, 1 H) 7.73 (t, J=7.86 Hz, 2 H) 7.46 -7.54 (m, 2 H) 7.17 (d, J=9.77 Hz, 1 H) 5.70 (br. s., 1 H) 5.45 (s, 2 H) 4.38 (d, J=12.66 Hz, 1 H) 4.30 (br. s., 1 H) 4.25 (br. s., 1 H) 4.06 - 4.14 (m, 2 H) 3.76 (br. s., 1 H) 3.65 (br. s., 1 H) 3.54 (br. s., 2 H) 2.99 - 3.14 (m, 3 H) 2.83 - 2.99 (m, 2 H) 2.73 (s, 1 H) 2.54 - 2.66 (m, 3 H) 2.48 (br. s., 3 H) 2.22 (br. s., 1 H) 2.04 - 2.16 (m, 2 H) 1.89 - 2.04 (m, 2 H) 1.84 (br. s., 2 H) 1.70 (br. s., 2 H) 1.07 - 1.31 (m, 2 H) 1.01 (br. s., 1 H)

### Example C-48: Preparation of 3-(1-(3-(5-((1-(2-(3-(1-(2-(3-(4-(2-(2,6-dioxopiperidin-3-yl)-1-oxo-1,2-dihydroptalazine-6-yl)piperazine-1-il)azetidine-1-il)2-oxoethyl)piperidine-4-yl)methoxy)pyrimidine-2-yl)benzyl)6-oxo-1,6-dihydropyridazine-3-yl)benzonitrile (ICT-0001232)

### Step 1) S Preparation of benzyl 4-(1-(tert-butoxycarbonyl)azetidine-3-yl)piperazine-1-carboxylate

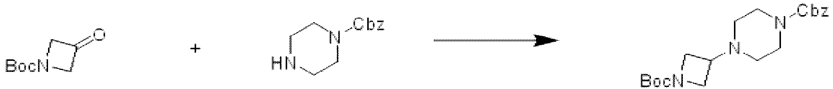

Sodium cyanoborohydride (4.07 g, 102 mmol) was added to tert-butyl 3-oxoazetidine-1-carboxylate (12.8 g, 74.9 mmol) and benzyl piperazine-1-carboxylate (15 g, 68.1 mmol) solutions in methanol (300 ml) and AcOH (6 ml), and stirred at room temperature for 16 hours. The reactants were poured into water, extracted with ethyl acetate, dried with Magnesium sulfate, and concentrated under reduced pressure. The residues were purified with HX/EA (20~90%) MPLC to obtain the title compound (22 g). MS (ESI, m/z): [M+H]⁺ = 376.0.

### Step 2) Preparation of tert-butyl 3-(piperazine-1-yl)azetidine-1-carboxylate

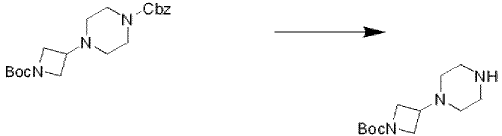

Pd/C (10 wt%, 50 mg) was added to benzyl 4-(1-(tert-butoxycarbonyl)azetidine-3-yl)piperazine-1-carboxylate (1 g, 2.66 mmol) solution in MeOH (10 ml) and stirred at room temperature under a hydrogen atmosphere for 5 hours. The solution was filtered in a cellite 545 phase. The solvent was removed under reduced pressure, and the title compound was obtained (0.7 g). MS (ESI, m/z): [M+H]⁺ = 242.0.

### Step 3) Preparation of tert-butyl 3-(4-(2-(2,6-dioxopiperidine-3-yl)-1-oxo-1,2-dihydroptalazine-6-yl)piperazine-1-yl)azetidine-1-carboxylate

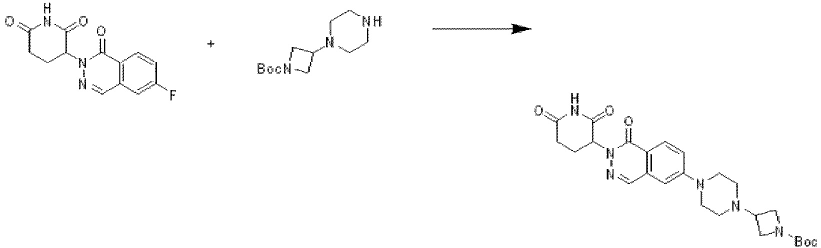

3-(6-fluoro-1-oxopthalazine-2(1H)-yl) piperidine-2,6-dione (642 mg, 2.4 mmol) and tert-butyl 3-(piperazine-1-yl)azetidine-1-carboxylate (643 mg, 2.66 mmol), and DIPEA (0.93 ml, 5.33 mmol) solutions in NMP (15 mL) were stirred at 120°C for 20 hours. MS (ESI, m/z): [M+H]⁺ = 497.3.

### Step 4) Preparation of 3-(6-(4-(azetidine-3-yl)piperazine-1-il)-1-oxopthalazine-2(1H)-yl)piperidine-2,6-dione

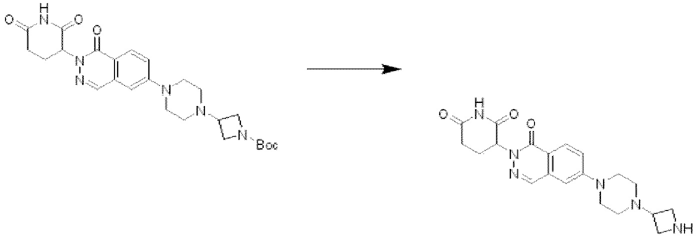

TFA (0.3 ml) was added to tert-butyl 3-(4-(2-(2,6-dioxopiperidine-3-yl)-1-oxo-1,2-dihydroptalazine-6-yl) piperazine-1-yl)azetidine-1-carboxylate (180 mg, 0.4 mmol) solution in DCM (1 ml) and stirred at room temperature for 2 hours. After the end of the reaction, the reactants were concentrated under reduced pressure and vacuum-dried. The residues were dissolved in DCM, NH-DM 1020, and the title compound was obtained by passing through a Chromatorex pad, which was used in the next step without further purification (140 mg). MS (ESI, m/z): [M+H]⁺ = 397.3.

### Step 5) Preparation of 3-(1-(1-(3-(5-((1-(2-(3-(3-(4-(2-(2,6-dioxopiperidine-3-yl)-1-oxo-1,2-dihydrophthalazine-6-yl)piperazine-1-day)azetidine-1-il)2-oxoethyl)piperidine-4-il)methoxy)pyrimidine-2-yl)benzyl)6-oxo-1,6-dihydropyridazine-3-yl)benzonitrile (ICT-0001232)

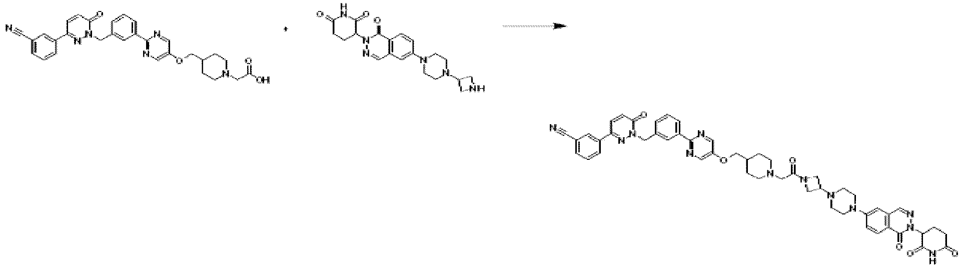

HATU (36.6 mg, 0.09 mmol) and 3-{6-{4-({[2-(3-{3-(3-cyanophenyl)-6-oxo-1,6-dihydropyridazin-1-yl]methyl}phenyl)pyrimidine-5-yl]oxy}methyl)piperidine-1-yl]acetic acid (46.6 mg, 0.09 mmol) and 3-{6-[4-(azetidine-3-yl)piperazine-1-yl]-1-oxo-1,2-dihydroptalazine-2-yl}piperidine-2,6-dione (34.4 mg, 0.09 mmol) in DMF (2 ml) at room temperature, followed by ethylbis (propane-2-yl)amine (33.7 mg, 0.261 µmol). The reactants were stirred for 6 hours. The reactants were poured into water, extracted with ethyl acetate, dried with Magnesium sulfate, and concentrated under reduced pressure. The residue was purified with MeOH/DCM (0-20%) MPLC to obtain the title compound (30 mg). MS (ESI, m/z): [M+H]⁺ = 916.0.
1H NMR (500 MHz, DMSO-d6) δ ppm 11.01 (s, 1 H) 8.66 (s, 2 H) 8.38 (s, 2 H) 8.21 - 8.28 (m, 3 H) 8.11 - 8.21 (m, 1 H) 8.06 (d, J=9.00 Hz, 1 H) 7.88 - 7.96 (m, 1 H) 7.72 (t, J=7.86 Hz, 1 H) 7.46 - 7.57 (m, 3 H) 7.28 (s, 1 H) 7.17 (d, J=9.77 Hz, 1 H) 5.75 (dd, J=11.44, 5.19 Hz, 1 H) 5.45 (s, 2 H) 4.18 - 4.26 (m, 1 H) 4.09 (br. s., 2 H) 4.04 (t, J=8.77 Hz, 1 H) 3.88 (br. s., 1 H) 3.50 (s, 1 H) 3.45 (br. s., 4 H) 3.27 (br. s., 1 H) 2.86 - 2.97 (m, 1 H) 2.61 (d, J=17.85 Hz, 1 H) 2.54 (br. s., 3 H) 1.39 (s, 1 H)

### Example C-49: Preparation of 3-(1-(3-(5-((1-(2-(4-(1-(3-(2,6-dioxopiperidine-3-yl)-1-methyl-4-oxo-3,4-dihydropthalazine-6-yl)piperidine-4-yl)piperazine-1-yl)2-oxoethyl)piperidine-4-il)methoxy)pyrimidine-2-yl)benzyl)6-oxo-1,6-dihydropyridine-3-yl)benzonitrile (ICT-0001233)

### Step 1) Preparation of benzyl 4-(1-(3-(2,6-dioxopiperidine-3-yl)-1-methyl-4-oxo-3,4-dihydroptalazine-6-yl)piperidine-4-yl)piperazine-1-carboxylate

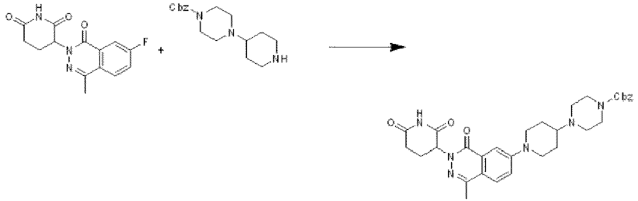

Ethylbis (670 mg, 5.12 mmol) was added to a solution of 3-(7-fluoro-4-methyl-1-oxofthalazine-2(1H)-yl) piperidine-2,6-dione (0.5 g, 1.73 mmol) and benzyl 4-(piperidine-4-yl)piperazine-1-carboxylate (0.53 g, 1.73 mmol) in solvent DMF (3 ml) at room temperature. The reactants were stirred at 130°C for 16 hours. After cooling, the reactants were poured into water, extracted with ethyl acetate, dried with Magnesium sulfate, and concentrated under reduced pressure. The resulting residues were purified with 100% ethyl acetate MPLC. The title compound was obtained (678 mg) as a white foam. MS (ESI, m/z): [M+H]⁺ = 573.2.

### Step 2) Preparation of 3-(4-methyl-1-oxo-7-(4-(piperazine-1-yl)piperidine-1-yl)phthalazine-2(1H)-yl)piperidine-2,6-dione

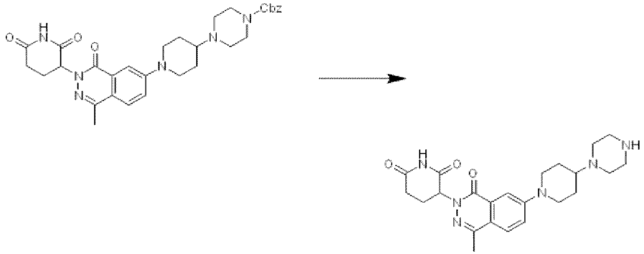

Pd/C 10% (100 mg) was added to a solution of benzyl 4-(1-(3-(2,6-dioxopiperidine-3-yl)1-methyl-4-oxo-3,4-dihydroptalazine-6-yl)piperidine-4-yl)piperazine-4-yl)piperazine-1-carboxylate (1 g, 1.75 mmol) at room temperature. The reaction was carried out by attaching an H2 balloon and stirred for 16 hours at room temperature. After cooling, the reactants were filtered, the filtrate was concentrated, and the title compound was obtained as a white solid (590 mg). MS (ESI, m/z): [M+H]⁺ = 439.5.

### Step 3) Preparation of 3-(1-(3-(5-((1-(2-(4-(1-(1-(3-(2,6-dioxopiperidine-3-yl)-1-methyl-4-oxo-3,4-dihydropthalazine-6-yl)piperidine-4-yl)piperazine-1-il)2-oxoethyl)piperidine-4-il)methoxy)pyrimidine-2-yl)benzyl)6-oxo-1,6-dihydropyridazine-3-yl)benzonitrile (ICT-0001233)

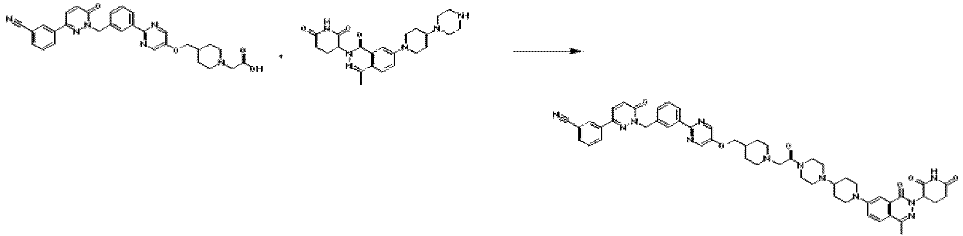

2-[4-({[2-(3-{3-(3-cyanophenyl)-6-oxo-1,{[3-(3-cyanophenyl)-6-oxo-1,in DMF (2ml) at room temperature HATU (36.3 mg, 0.1 mmol) was added to a solution of 6-dihydropyridazine-1-yl]methyl}phenymidine-5-yl]oxy}methyl)piperidine-1-yl]acetic acid (46.6 mg, 0.086 mmol) and 3-{4-methyl-1-oxo-7-[4-(piperazine-1-yl)piperidine-1-yl]-1,2-dihydropthalazine-2-yl}piperidine-2,6-dione (31.8 mg, 0.086 mmol) solution, followed by ethylbis (propane-2-yl)amine (33.7 mg. 0.26 mmol). The reactant was stirred for 6 hours. The reactant was poured into the water. It was extracted with ethyl acetate, dried with Magnesium sulfate, and concentrated under reduced pressure. The residue was purified with MeOH/DCM (0-20%) MPLC to obtain the title compound (53.2 mg). MS (ESI, m/z): [M+H]⁺ = 958.2
1H NMR (500 MHz, DMSO-d6) δ ppm 10.98 (s, 1 H) 8.66 (s, 2 H) 8.38 (d, J=1.68 Hz, 2 H) 8.21 - 8.28 (m, 2 H) 8.12 - 8.21 (m, 1 H) 7.90 - 7.97 (m, 1 H) 7.78 (d, J=9.00 Hz, 1 H) 7.72 (t, J=7.86 Hz, 1 H) 7.61 (d, J=8.70 Hz, 1 H) 7.55 (br. s., 1 H) 7.46 - 7.53 (m, 2 H) 7.17 (d, J=9.77 Hz, 1 H) 5.68 (d, J=5.95 Hz, 1 H) 5.45 (s, 2 H) 4.10 (br. s., 1 H) 3.47 - 3.56 (m, 2 H) 2.85 - 3.02 (m, 3 H) 2.52 - 2.66 (m, 4 H) 2.46 (s, 4 H) 2.02 - 2.12 (m, 1 H) 1.39 (s, 1 H)

### Example C-50: Preparation of 3-(1-(3-(5-((1-(1-(2-(4-((1-(3-(2,6-dioxopiperidine-3-yl)-1-methyl-4-oxo-3,4-dihydropthalazine-6-yl)piperidine-4-yl)methyl)piperazine-1-yl)-2-oxoethyl)piperidine-4-il)methoxy)pyrimidine-2-yl)benzyl)6-oxo-1,6-dihydropyridazine-3-yl)benzonitrile (ICT-0001234)

### Step 1) Preparation of 3-(4-methyl-1-oxo-7-(4-(piperazine-1-ylmethyl)piperidine-1-yl)phthalazine-2(1H)-yl)piperidine-2,6-dione

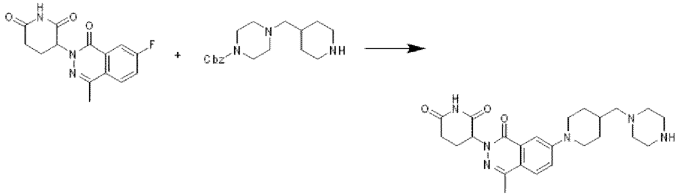

Ethylbis (propane-2-yl)amine (1.34 g, 10.4 mmol) was added to 3-(7-fluoro-4-methyl-1-oxopthalazine-2(1H)-yl)piperidine-2,6-dione (1.0 g, 3.46 mmol) and benzyl 4-(piperidine-4-ylmethyl)piperazine-1-carboxylate (1.1 g, 3.46 mmol) solutions in DMA (5 ml) at room temperature. The reactants were heated at 120°C for 16 hours. The reactants were poured into water, extracted with ethyl acetate, dried with Magnesium sulfate, and concentrated under reduced pressure. The residues were purified with MeOH/DCM (0-10%) MPLC. Protected compound was obtained. The CBZ-protected compound was dissolved in MeOH (0.5 mL), 10% Pd/C (100 mg) was added, and stirred under a hydrogen atmosphere for 5 hours. The reactants are filtered, concentrated under reduced pressure, and subjected compounds (1.21 g). MS (ESI, m/z): [M+H]⁺ = 453.6.

### Step 2) Preparation of 3-(1-(1-(3-(5-((1-(2-(4-((1-(3-(2,6-dioxopiperidin-3-yl)-1-methyl-4-oxo-3,4-dihydroptalazine-6-yl)piperidine-4-il)methyl)piperazine-1-day)2-oxoethyl)piperidine-4-il)methoxy)pyrimidine-2-yl)benzyl)6-oxo-1,6-dihydropyridazine-3-yl)benzonitrile (ICT-0001234)

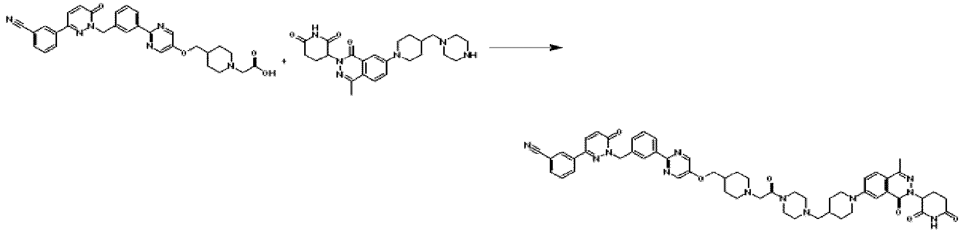

HATU (36.3 mg, 0.09 mmol) is added to 2-(4-(((2-(3-((3-(3-(3-cyanophenyl)-6-oxopridazine-1(6H)-yl)methyl)phenyl)pyrimidine-5-yloxy)methyl)piperidine-1-yl) acetic acid (46 mg, 0.09 mmol) and 3-(4-(4-methyl-1-oxo-7-(4-(piperazine-1-yl)piperidine-1-yl)phthalazine-2(1H)-yl)piperidine-2,6-dione (39 mg, 0.09 mmol) in DMF (2 ml) at room temperature, followed by ethylbis (propane-2-yl)amine (33.7 mg, 0.26 mmol). The reactants were stirred for 6 hours. The reactants were poured into water, extracted with ethyl acetate, dried with Magnesium sulfate, and concentrated under reduced pressure. The residue was purified with MeOH/DCM (0-20%) MPLC to obtain the title compound (43.2 mg). MS (ESI, m/z): [M+H]⁺ = 972.2.
1H NMR (500 MHz, DMSO-d6) δ ppm 10.97 (s, 1 H) 8.65 (s, 2 H) 8.36 - 8.40 (m, 2 H) 8.24 (s, 1 H) 8.19 (s, 1 H) 8.12 - 8.14 (m, 1 H) 7.96 (d, J=6.56 Hz, 1 H) 7.93 (d, J=7.93 Hz, 1 H) 7.74 (s, 1 H) 7.56 (d, J=6.10 Hz, 1 H) 7.50 (s, 2 H) 7.17 - 7.19 (m, 1 H) 7.07 (br. s., 1 H) 6.52 (s, 2 H) 5.45 (s, 2 H) 5.34 (br. s., 1 H) 4.08 (br. s., 1 H) 3.94 - 4.01 (m, 1 H) 2.88 - 2.96 (m, 2 H) 2.53 - 2.66 (m, 3 H) 2.42 - 2.47 (m, 6 H) 2.20 (br. s., 1 H) 1.77 - 1.88 (m, 1 H) 1.34 - 1.42 (m, 1 H) 1.17 - 1.23 (m, 10 H)

### Example C-51: Preparation of 3-(1-(3-(5-((1-(1-(2-(3-(1-(2-(3-((4-(2-(2,6-dioxopiperidin-3-yl)-4-methyl-1-oxo-1,2-dihydropthalazine-6-yl)piperazine-1-il)methyl)azetidine-1-yl)-2-oxoethyl)piperidine-4-yl)methoxy)pyrimidine-2-yl)benzyl)6-oxo-1,6-dihydropyridazine-3-yl)benzonitrile (ICT-0001235)

### Step 1) Preparation of tert-butyl 3-((methylsulfonyl)oxy)methyl)azetidine-1-carboxylate

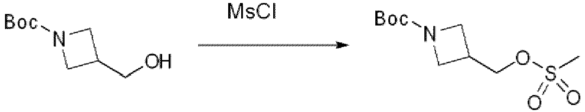

Methanesulfonyl chloride (2.69 g, 23.5 mmol) was slowly added to tert-butyl 3-(hydroxymethyl)azetidine-1-carboxylate (4 g, 21.4 mmol) and DIPEA (5.58 ml, 32 mmol) solutions in DCM (20 ml) at 0°C. After the end of the reaction, the solvent was removed under reduced pressure. The residue was dissolved in EA (150 ml), washed with 150 ml water and 150 ml brine solution. The organic layer was dried with Magnesium sulfate, concentrated, and the title compound was obtained (5.8 g, red oil). MS (ESI, m/z): [M+H]⁺ = 266.1.

### Step 2) Preparation of benzyl 4-((1-(tert-butoxycarbonyl)azetidine-3-yl)methyl)piperazine-1-carboxylate

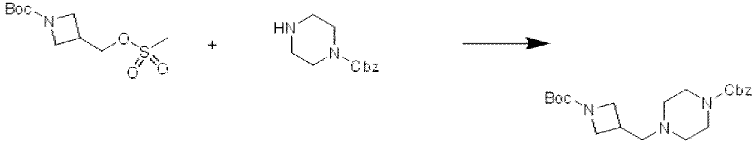

Potassium carbonate (6.04 g, 43.7 mmol) was slowly added to tert-butyl 3-((methylsulfonyl)methyl)azetidine-1-carboxylate (5.8 g, 21.9 mmol) and benzyl piperazine-1-carboxylate (7.22 g, 32.8 mmol) solution in DMF (50 ml) and stirred at 85°C for 17 hours. The reaction mixture was diluted with 200 ml EA and washed with water and brine solution. The organic layer was dried with Magnesium sulfate and concentrated. The residues were purified by reversed-phase column chromatography to obtain the subjective compound (3.87 g). MS (ESI, m/z): [M+H]⁺ = 390.1

### Step 3) Preparation of tert-butyl 3-(piperazine-1-monomethyl)azetidine-1-carboxylate

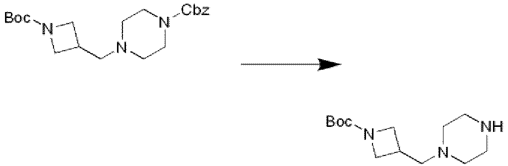

Benzyl 4-((1-(tert-butoxycarbonyl)azetidine-3-yl)methyl)piperazine-1-carboxylate (2.45 g, 6.29 mmol) solution in MeOH (60 ml) was added to Pd/C (300 mg 10 wt%) and stirred under a hydrogen atmosphere at room temperature for 17 hours. The solution was filtered on cellite 545, the solvent was removed under reduced pressure, and the title compound was obtained (1.56 g). MS (ESI, m/z): [M+H]⁺ = 256.0.

### Step 4) Preparation of tert-butyl 3-((4-(2-(2,6-dioxopiperidin-3-yl)-4-methyl-1-oxo-1,2-dihydroptalazine-6-yl)piperazine-1-yl)methyl)azetidine-1-carboxylate

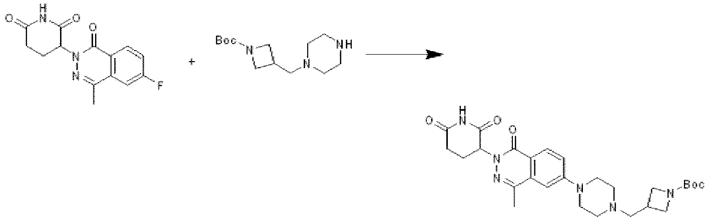

3-(6-fluoro-4-methyl-1-oxopthalazine-2(1H)-yl)piperidine-2,6-dione (200 mg, 0.69 mmol), tert-butyl 3-(piperazine-1-monomethyl)azetidine-1-carboxylate (265 mg, 1.04 mmol), and DIPEA (0.241 ml, 1.38 mmol) solutions were stirred at 120°C for 20 hours. MS (ESI, m/z): [M+H]⁺ = 525.1

### Step 5) Preparation of 3-(6-(4-(azetidine-3-ylmethyl)piperazine-1-yl)-4-methyl-1-oxopthalazine-2(1H)-yl)piperidine-2,6-dione

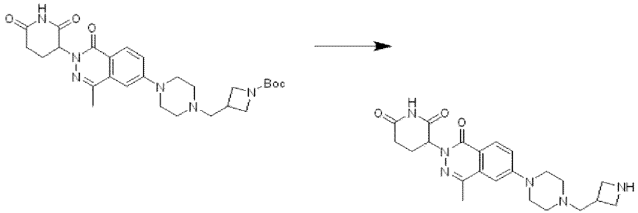

TFA (0.2 ml) was added to tert-butyl 3-((4-(2-(2,6-dioxopiperidin-3-yl)-4-methyl-1-oxo-1,2-dihydroptalazin-6-yl)piperazine-1-yl)methyl)azetidine-1-carboxylate (20 mg, 0.038 mmol) in DCM (1 ml) and stirred at room temperature for 2 hours. After the end of the reaction, the reactants were concentrated under reduced pressure and vacuum-dried. The residues were dissolved in DCM, passed through NH-DM 1020, Chromatorex pads to obtain the heading compounds, which were used for the next step without further purification. MS (ESI, m/z): [M+H]⁺ = 425.1.

### Step 6) Preparation of 3-(1-(1-(3-(5-((1-(2-(3-((4-(2-(2,6-dioxopiperidine-3-yl)-4-methyl-1-oxo-1,2-dihydropetthalazine-6-yl)piperazine-1-il)methyl)azetidin-1-il)-2-oxoethyl)piperidine-4-il)methoxy)pyrimidine-2-yl)benzyl)6-oxo-1,6-dihydropyridazine-3-yl)benzonitrile (ICT-0001235)

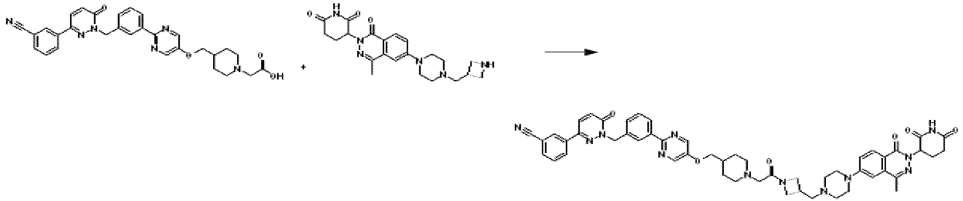

HATU (36.3 mg, 0.1 mmol) is added to 2-(4-(((2-(3-((3-(3-(3-(3-cyanophenyl)-6-oxopridazine-1(6H)-yl)methyl)phenyl)pyrimidine-5-yl)oxy)methyl)piperidine-1-yl) acetic acid (46.6 mg, 0.08 mmol) and 3-(6-(4-(azetidine-3-yl)piperazine-1-yl)4-methyl-1-oxopthalazine-2(1H)-yl)piperidine-2,6-dione (36.9 mg, 0.08 µmol) in DMF (2 ml) at room temperature, followed by ethylbis (propane-2-yl)amine (33.7 mg, 0.26 mmol). The reactants were stirred for 6 hours. The reactants were poured into water, extracted with ethyl acetate, dried with Magnesium sulfate, and concentrated under reduced pressure. The residue was purified with MeOH/DCM (0-20%) MPLC to obtain the title compound (37 mg). MS (ESI, m/z): [M+H]⁺ = 944.1.
1H NMR (500 MHz, DMSO-d6) δ ppm 10.97 (s, 1 H) 8.65 (s, 2 H) 8.36 - 8.40 (m, 2 H) 8.24 (s, 1 H) 8.19 (s, 1 H) 8.12 - 8.14 (m, 1 H) 7.96 (d, J=6.56 Hz, 1 H) 7.93 (d, J=7.93 Hz, 1 H) 7.74 (s, 1 H) 7.56 (d, J=6.10 Hz, 1 H) 7.50 (s, 2 H) 7.17 - 7.19 (m, 1 H) 7.07 (br. s., 1 H) 6.52 (s, 2 H) 5.45 (s, 2 H) 5.34 (br. s., 1 H) 4.08 (br. s., 1 H) 3.94 - 4.01 (m, 1 H) 2.88 - 2.96 (m, 2 H) 2.53 - 2.66 (m, 3 H) 2.42 - 2.47 (m, 2 H) 2.20 (br. s., 1 H) 1.77 - 1.88 (m, 1 H) 1.34 - 1.42 (m, 1 H) 1.17 - 1.23 (m, 10 H)

### Example C-52: Preparation of 3-(1-(3-(5-((1-(1-(2-(4-((1-(2-(2-(2-(3-yl)-4-methyl-1-oxo-1,2-dihydrophthalazine-6-yl)piperidine-4-il)methyl)piperazine-1-yl)2-oxoethyl)piperidine-4-il)methoxy)pyrimidine-2-yl)benzyl)6-oxo-1,6-dihydropyridazine-3-yl)benzonitrile (ICT-0001236)

### Step 1) Preparation of 3-(4-methyl-1-oxo-6-(4-(piperazine-1-ylmethyl)piperidine-1-yl)phthalazine-2(1H)-yl)piperidine-2,6-dione

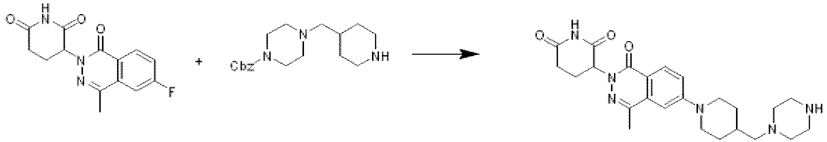

Ethylbis (1.34 g, 10.4 mmol) was added to 3-(6-fluoro-4-methyl-1-oxopthalazine-2(1H)-yl)piperidine-2,6-dione (1.0 g, 3.46 mmol) and benzyl 4-(piperidine-4-ylmethyl)piperazine-1-carboxylate (1.1 g, 3.46 mmol) in DMA (5 ml) at room temperature. The reactants were heated at 120°C for 16 hours. The reactants were poured into water, extracted with ethyl acetate, dried with Magnesium sulfate, and concentrated under reduced pressure. The residue was purified with MeOH/DCM (0-10%) MPLC. Protected compound was obtained. The CBZ-protected compound was dissolved in MeOH (0.5 mL), 10% Pd/C (100 mg) was added, and stirred under a hydrogen atmosphere for 5 hours. The reactants were filtered, concentrated under reduced pressure, and subjected compounds were obtained (1.1 g). MS (ESI, m/z): [M+H]⁺ = 453.6.

### Step 2) Preparation of 3-(1-(3-(5-((1-(1-(2-(4-((1-(2-(2-(dioxopiperidine-3-yl)-4-methyl-1-oxo-1,2-dihydropthalazine-6-yl)piperidine-4-yl)methylperazine-1-yl)2-oxoethyl)piperidine-4-il)methoxy)pyrimidine-2-yl)benzonitrile (ICT-0001236)

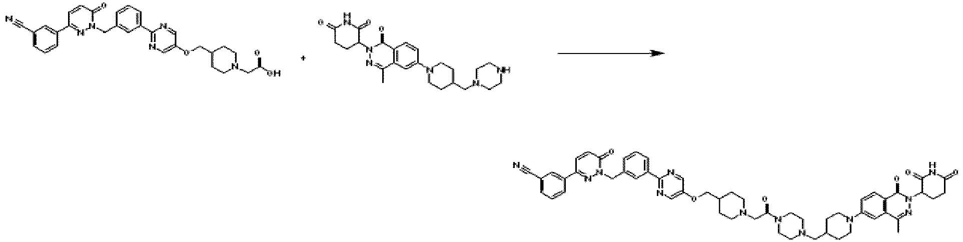

HATU (36.3 mg, 0.09 mmol) is added to 2-(4-(((2-(3-((3-(3-(3-cyanophenyl)-6-oxopridazine-1(6H)-yl)methyl)phenyl)pyrimidine-5-yloxy)methyl)piperidine-1-yl) acetic acid (46 mg, 0.09 mmol) and 3-(4-methyl-1-oxo-6-(4-(piperazine-1-yl)piperidine-1-yl)phthalazine-2(1H)-yl)piperidine-2,6-dione (39 mg, 0.09 mmol) in DMF (2 ml) at room temperature, followed by ethylbis (propane-2-yl)amine (33.7 mg, 0.26 mmol). The reactants were stirred for 6 hours. The reactants were poured into water, extracted with ethyl acetate, dried with Magnesium sulfate, and concentrated under reduced pressure. The residue was purified with MeOH/DCM (0-20%) MPLC to obtain the title compound (40.2 mg). MS (ESI, m/z): [M+H]⁺ = 972.2
1H NMR (500 MHz, DMSO-d6) δ ppm 10.95 (s, 1 H) 8.64 (s, 2 H) 8.38 (d, J=4.73 Hz, 2 H) 8.20 - 8.26 (m, 3 H) 8.17 (d, J=9.77 Hz, 1 H) 8.04 (d, J=9.00 Hz, 1 H) 7.93 (d, J=7.78 Hz, 1 H) 7.72 (t, J=7.86 Hz, 1 H) 7.47 - 7.51 (m, 2 H) 7.16 (d, J=9.77 Hz, 1 H) 7.04 (s, 1 H) 5.44 (s, 2 H) 4.05 (d, J=5.80 Hz, 2 H) 3.55 (br. s., 1 H) 3.09 - 3.15 (m, 2 H) 2.77 - 2.97 (m, 5 H) 2.76 (s, 1 H) 2.52 - 2.65 (m, 4 H) 2.43 - 2.49 (m, 5 H) 2.36 (br. s., 2 H) 2.29 (br. s., 1 H) 2.16 (d, J=6.41 Hz, 1 H) 1.95 - 2.06 (m, 1 H) 1.73 - 1.86 (m, 4 H) 1.39 (s, 1 H) 1.30 (d, J=11.14 Hz, 1 H) 1.15 - 1.26 (m, 1 H)

### Example C-53: Preparation of 3-(1-(3-(5-((1-(2-(4-(4-(2-(2-(2-(2-(2-(2-(2-yl)-4-methyl-1-oxo-1,2-dihydropthalazine-6-yl)piperazine-1-il)piperidine-1-il)piperidine-1-yl)2-oxoethyl)piperidine-4-il)methoxy)pyrimidine-2-yl)benzyl)6-oxo-1,6-dihydropyridazine-3-yl)benzonitrile (ICT-0001237)

### Step 1) Preparation of tert-butyl 4-(4-(2-(2,6-dioxopiperidine-3-yl)-4-methyl-1-oxo-1,2-dihydroptalazine-6-yl)piperazine-1-yl)piperidine-1-carboxylate

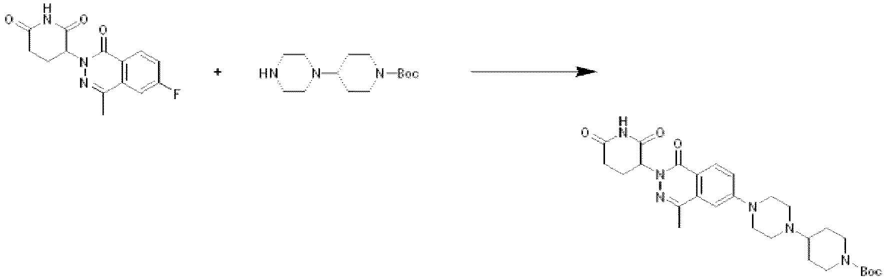

Ethylbis (propane-2-yl)amine (1.34 g, 10.4 mmol) was added to 3-(6-fluoro-4-methyl-1-oxopthalazine-2(1H)-yl)piperidine-2,6-dione (1.0 g, 3.46 mmol) and tert-butyl 4-(piperazine-1-yl)piperidine-1-carboxylate (1.0 g, 3.46 mmol) solutions in DMA (5 ml) at room temperature. The reactants were heated at 120°C for 16 hours. The reactants were poured into water, extracted with ethyl acetate, dried with Magnesium sulfate, and concentrated under reduced pressure. The residues were purified with MeOH/DCM (0-10%) MPLC. Subjective compound (1.3 g) was obtained. MS (ESI, m/z): [M+H]⁺ = 539.6

### Step 2) Preparation of 3-(4-methyl-1-oxo-6-(4-(piperidine-4-yl)piperazine-1-yl)phthalazine-2(1H)-yl)piperidine-2,6-dione

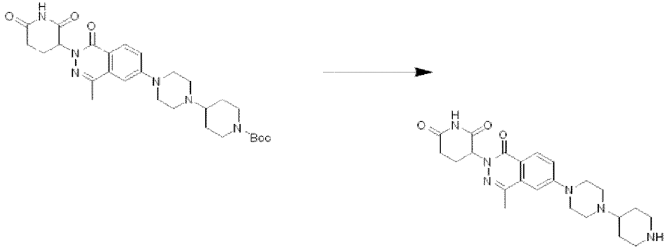

TFA (1 ml) was added to tert-butyl 4-(4-(2-(2,6-dioxopiperidine-3-yl)-4-methyl-1-oxo-1,2-dihydroptalazin-6-yl)piperazine-1-yl)piperidine-1-carboxylate (200 mg, 0.5 mmol) solution in DCM (3 ml) and stirred at room temperature for 2 hours. After the end of the reaction, the reactants were concentrated under reduced pressure and vacuum-dried. The residues were dissolved in DCM, and the title compound was obtained by passing NH-DM 1020, Chromatorex pads, and used for the next step without further purification. MS (ESI, m/z): [M+H]⁺ = 439.1.

### Step 3) Preparation of 3-(1-(3-(5-((1-(2-(4-(4-(2-(2-(2-(2-(2-(2-(dioxopiperidine-3-yl)-4-methyl-1-oxo-1,2-dihydropthalazine-6-yl)piperazine-1-il)piperidine-1-il)piperidine-1-il)2-oxoethyl)piperidine-4-il)methoxy)pyrimidine-2-yl)benzyl)6-oxo-1,6-dihydropyridazine-3-yl)benzonitrile (ICT-0001237)

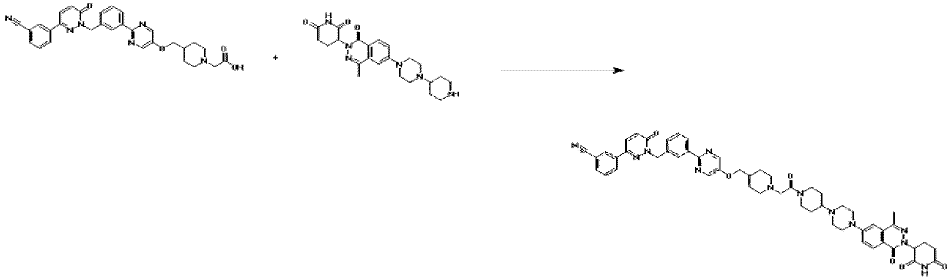

HATU (36.3 mg, 0.09 mmol) is added to the solution of 2-(4-(((2-(3-(3-(3-(3-cyanophenyl)-6-oxpyridazine-1(6H)-yl)methyl)phenyl)pyrimidine-5-yl)oxy)methyl)piperidine-1-day)acetic acid (46 mg, 0.09 mmol) 3-(4-methyl-1-oxo-6-(4-(piperidine-4-yl)piperazine-1-yl)phthalazine-2(1H)-yl)piperidine-2,6-dione (38 mg, 0.09 mmol) in DMF (2 ml) at room temperature, followed by ethylbis (propane-2-yl)amine (33.7 mg, 0.26 mmol). The reactants were stirred for 6 hours. The reactants were poured into water, extracted with ethyl acetate, dried with Magnesium sulfate, and concentrated under reduced pressure. The residue was purified with MeOH/DCM (0-20%) MPLC to obtain the title compound (40.2 mg). MS (ESI, m/z): [M+H]⁺ = 958.2
1H NMR (500 MHz, DMSO-d6) δ ppm 10.98 (s, 1 H) 8.66 (s, 2 H) 8.38 (s, 2 H) 8.11 - 8.28 (m, 3 H) 8.08 (d, J=8.70 Hz, 1 H) 7.94 (d, J=7.78 Hz, 1 H) 7.72 (t, J=7.86 Hz, 1 H) 7.45 - 7.55 (m, 3 H) 7.17 (d, J=9.77 Hz, 1 H) 7.09 (br. s., 1 H) 6.54 (s, 1 H) 5.68 (d, J=7.17 Hz, 1 H) 5.45 (s, 2 H) 4.10 (br. s., 1 H) 2.69 (br. s., 1 H) 2.53 - 2.66 (m, 1 H) 2.36 (s, 1 H) 1.20 (d, J=6.41 Hz, 1 H)

### Example C-54: Preparation of N-(3-(2-(4-(((2-(2-(3-((3-(3-(3-(3-cyanophenyl)-6-oxopyridazine-1(6H)-yl)methyl)phenyl)pyrimidine-5-il)oxy)methyl)piperidine-1-il)acetamido)propyl)-1-(3-(2,6-dioxopiperidin-3-yl)1-methyl-4-oxo-3,4-dihydroptalazine-6-yl)piperidine-4-carboxamide (ICT-0001238)

### Step 1) Preparation of 1-(3-(2,6-dioxopiperidine-3-yl)-1-methyl-4-oxo-3,4-dihydroptalazine-6-yl)piperidine-4-carboxylic acid

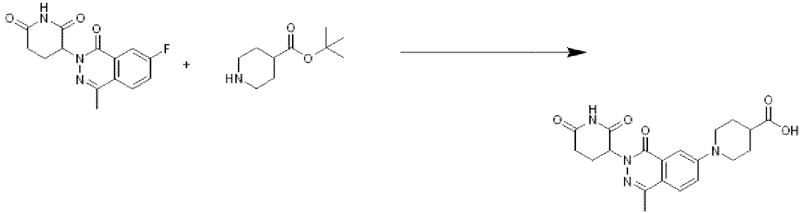

3-(7-fluoro-4-methyl-1-oxopthalazine-2(1H)-yl)piperidine-2,6-dione (200 mg, 0.69 mmol), tert-butylpiperidine-4-carboxylate (230 mg, 1.04 mmol), and DIPEA (0.24 ml, 1.38 mmol) solutions in NMP (2 mL) were stirred at 120°C for 20 hours. The reaction mixture was purified by reversed-phase column chromatography to obtain the protected compound (284 mg), dissolved in DCM (4 ml), TFA (1 ml) was added, and stirred at room temperature for 2 hours. The reactants were vacuum-concentrated. The residues were purified with MeOH/DCM (0-20%) MPLC. Subjective compound (250 mg). MS (ESI, m/z): [M+H]⁺ = 399.0.

### Step 2) Preparation of N-(3-aminopropyl)-1-(3-(2,6-dioxopiperidine-3-yl)-1-methyl-4-oxo-3,4-dihydroptalazine-6-yl)piperidine-4-carboxamide

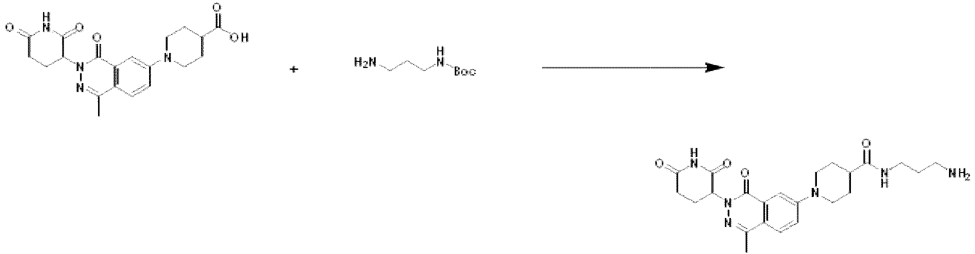

HATU (105 mg, 0.27 mmol) was added to a solution of 1-(3-(2,6-dioxopiperidine-3-yl)-1-methyl-4-oxo-3,4-dihydroptalazine-6-yl) and tert-butyl (3-aminopropyl)carbamate (43.7 mg, 0.25 mmol) in DMF (1 mL) at room temperature, followed by ethyl bis (propane-2-yl)amine (97.3 mg, 0.75 mmol). The reactants were stirred for 6 hours. The reactants were poured into water, extracted with ethyl acetate. It was dried with Magnesium sulfate and concentrated under reduced pressure. The residue was purified with DCM/MeOH (0~10%) MPLC to obtain the title compound (102 mg). MS (ESI, m/z): [M+H]⁺ = 455.7

### Step 3) Preparation of N-(3-(2-(4-(((2-(3-((3-(3-(3-(3-cyanophenyl)-6-oxopridazine-1(6H)-yl)methyl)phenyl)pyrimidine-5-yl)oxy)methyl)piperidine-1-il)acetamide)propyl)-1-(3-(2,6-dioxopiperidin-3-yl)1-methyl-4-oxo-3,4-dihydroptalazine-6-yl)piperidine-4-carboxamide (ICT-0001238)

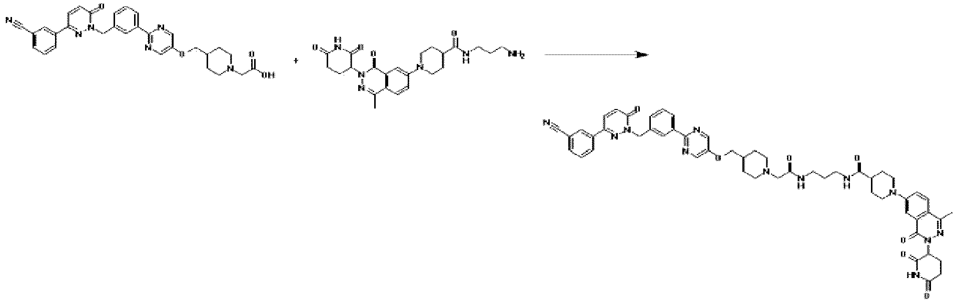

HATU (36.6 mg, 0.09 mmol) and N-(3-aminopropyl)-1-[3-(2-(3-{3-(3-cyanophenyl)-6-oxo-1,6-dihydropyridazin-1-yl]methyl}phenyl)pyrimidine-5-yl]oxy}methyl)piperidine-1-yl]acetic acid (46.6 mg, 0.09 mmol) and N-(3-aminopropyl)-1-[3-(2,6-dioxopiperidine-3-yl)-1-methyl-4-oxo-3,4-dihydropthalazine-6-yl]piperidine-4-carboxydamide (39.5 mg, 0.09 mmol) in DMF (2 ml) at room temperature, followed by ethylbis (propane-2-yl)amine (33.7 mg, 0.26 mmol). The reactants were stirred for 6 hours. The reactants were poured into water, extracted with ethyl acetate, dried with Magnesium sulfate, and concentrated under reduced pressure. The residue was purified with MeOH/ DCM (0-20%) MPLC to obtain the title compound (51 mg). MS (ESI, m/z): [M+H]⁺ = 974.1
1H NMR (500 MHz, DMSO-d6) δ ppm 10.97 (s, 1 H) 8.63 - 8.67 (m, 2 H) 8.38 (br. s., 2 H) 8.13 - 8.27 (m, 3 H) 8.05 (d, J=9.00 Hz, 1 H) 7.84 - 7.96 (m, 2 H) 7.72 (t, J=7.86 Hz, 1 H) 7.46 - 7.52 (m, 2 H) 7.17 (d, J=9.77 Hz, 1 H) 7.03 - 7.09 (m, 1 H) 5.68 (d, J=7.32 Hz, 1 H) 5.45 (s, 2 H) 4.00 - 4.14 (m, 3 H) 3.45 - 3.63 (m, 2 H) 3.03 - 3.19 (m, 4 H) 2.83 - 3.03 (m, 4 H) 2.53 - 2.69 (m, 2 H) 2.34 - 2.49 (m, 4 H) 1.99 - 2.11 (m, 1 H) 1.95 (br. s., 1 H) 1.89 (br. s., 1 H) 1.74 - 1.85 (m, 2 H) 1.50 - 1.71 (m, 5 H) 1.33 - 1.43 (m, 1 H)

### Example C-55: Preparation of 3-(1-(3-(5-((1-(2-(4-((1-(3-(2-(4-(1-(3-(2,6-dioxopiperidine-3-yl)-1-methyl-4-oxo-3,4-dihydropthalazine-6-yl)piperidine-4-yl)methyl)piperazine-1-il)acetyl)piperidine-4-il)methoxy)pyrimidine-2-yl)benzonitrile-6-oxo-1,6-dihydropyridazine-3-yl)benzonitrile (ICT-0001239)

### Step 1) Preparation of 3-(4-methyl-1-oxo-7-(4-(piperazine-1-ylmethyl)piperidine-1-yl)phthalazine-2(1H)-yl)piperidine-2,6-dione

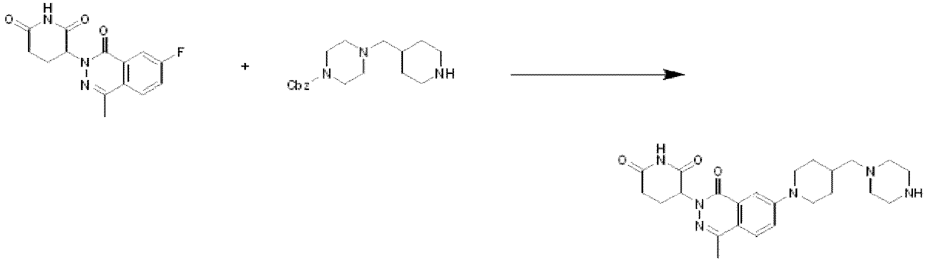

Ethylbis (2.68 g, 20.7 mmol) was added to 3-(6-fluoro-4-methyl-1-oxo-1,2-dihydroptalazine-2-yl) piperidine-2,6-dione (2.0 g, 6.91 mmol) and 4-(piperazine-1-yl)aniline (2.63 g, 8.3 mmol) solutions in DMA (5 ml) at room temperature. The reactants were stirred at 130°C for 16 hours. The reactants were poured into water, extracted with ethyl acetate, dried with Magnesium sulfate, and concentrated under reduced pressure. The residues were purified with MPLC. CBZ-protected compound was obtained. The compound was dissolved in MeOH (10 ml) and 10% Pd/C (100 mg) was added. The reactants were stirred under an H₂ balloon for 5 hours. The reactants were filtered, concentrated under reduced pressure, and the title compound was obtained (2.15 g). MS (ESI, m/z): [M+H]⁺ = 453.2.

### Step 2) Preparation of 3-(1-(3-(5-((1-(2-(4-((1-(3-(2-(4-(1-(3-(2,6-dioxopiperidine-3-yl)-1-methyl-4-oxo-3,4-dihydropetthalazine-6-yl)piperidine-4-il)methyl)piperazine-1-il)acetyl)piperidine-4-il)methoxy)pyrimidine-2-yl)benzyl)6-oxo-1,6-dihydropyridazine-3-yl)benzonitrile (ICT-0001239)

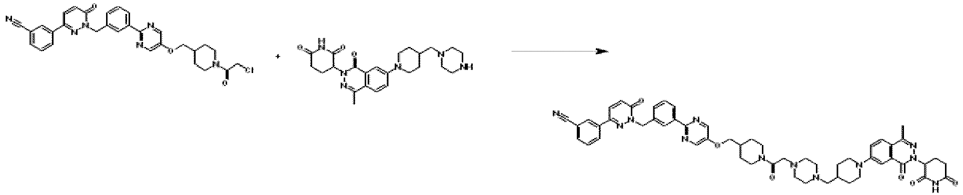

Triethylamine (18.2 mg, 0.18 mmol) was added to 3-(1-(3-(5-((1-(1-(2-chloroacetyl)piperidine-4-yl)methoxy)pyrimidin-2-yl)benzyl)benzonitrile (50 mg, 0.09 mmol) and 3-(4-methyl-1-iodine-7-(4-(piperazine-1-yl)piperidine-1-(1H)-yl)piperidine-2,6-dione (40.8 mg, 0.09 mmol) solution in DMF (1 mL). The reactants were stirred at 45°C for 4 hours. The reactants were poured into water. Ethyl acetate. The organic layer was dried with Magnesium sulfate, concentrated under reduced pressure, and purified by column chromatography to obtain the title compound (45.2 mg). MS (ESI, m/z): [M+H]⁺ = 972.1
1H NMR (500 MHz, DMSO-d6) δ ppm 10.98 (s, 1 H) 8.65 (s, 2 H) 8.39 (s, 1 H) 8.37 (s, 1 H) 8.21 - 8.26 (m, 2 H) 8.13 - 8.20 (m, 2 H) 7.93 (d, J=7.63 Hz, 1 H) 7.68 - 7.77 (m, 2 H) 7.54 (d, J=9.00 Hz, 1 H) 7.45 - 7.51 (m, 2 H) 7.16 (d, J=9.77 Hz, 1 H) 5.69 (d, J=6.10 Hz, 1 H) 5.44 (s, 1 H) 4.38 (d, J=12.21 Hz, 1 H) 4.08 (t, J=5.49 Hz, 2 H) 3.96 (d, J=12.05 Hz, 1 H) 3.27 (d, J=13.12 Hz, 1 H) 2.98 (d, J=12.97 Hz, 1 H) 2.83 - 2.94 (m, 2 H) 2.53 - 2.65 (m, 3 H) 2.39 - 2.47 (m, 4 H) 2.37 (br. s., 1 H) 2.02 - 2.12 (m, 3 H) 1.71 - 1.84 (m, 4 H) 1.31 (d, J=16.17 Hz, 1 H) 1.06 - 1.23 (m, 2 H) -0.03 - 0.03 (m, 2 H)

### Example C-56: Preparation of 3-(1-(3-(5-((1-((4-(4-(2-(2,6-dioxopiperidin-3-yl)-4-methyl-1-oxo-1,2-dihydropthalazine-6-yl)piperazine-1-yl)phenyl)glycyl)piperidine-4-il)methoxy)pyrimidine-2-yl)benzyl)6-oxo-1,6-dihydropyridazine-3-yl)benzonitrile (ICT-0001240)

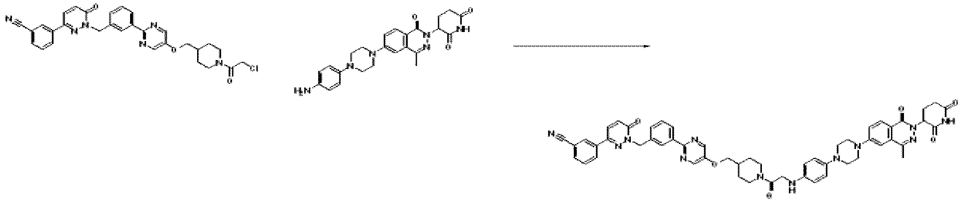

3-(1-(3-(5-(((1-(2-chloroacetyl)piperidine-4-yl)methoxy)pyrimidine-2-yl)benzyl)-6-oxo-1,in 0.6 ml DMSO DIPEA (98 µmol) was added to 6-dihydropyridazine-3-yl) benzonitrile (18 µmol) and 3-(6-(4-(4-aminophenyl)piperazine-1-yl)-4-methyl-1-oxopthalazine-2(1H)-yl)piperidine-2,6-dione (18 µmol) solutions, and stirred at 90°C for 16 hours. The reaction mixture was purified with reversed-phase column chromatography (water (0.1% FA) / ACN (0.1% FA) = 95/1 to 0/100 gradient). The corresponding fraction was lyophilized. The obtained product was used by amino silica column chromatography (DCM/ MeOH, 100/0 to 97/3, gradient) and obtained 1.8 mg of 3-(1-(3-(5-((1-((4-(4-(2-(2,6-diodexperidine-3-yl)-4-methyl-1-oxo-1,2-dihydropthalazine-6-yl)piperazine-1-yl)phenyl)glycy)piperidine-4-il)methoxy)pyrimidine-2-yl)benzonitrile-6-oxo-1,6-dihydropyridazine-3-yl)benzonitrile. MS (ESI, m/z): [M+H]⁺ = 965.8
1H NMR (500 MHz, DMSO-d6) δ ppm 10.98 (s, 1H), 8.65 (s, 2H), 8.38 (s, 2H), 8.24 (m, 2H), 8.18 (d, J = 9.8 Hz, 1H), 8.08 (d, J = 9 Hz, 1H), 7.93 (d, J = 7.6 Hz, 1H), 7.71 (t, J = 7.9 Hz, 1H), 7.58 (m, 1H), 7.49 (brd, 2H), 7.26 (d, J = 9.8 Hz, 1H), 7.1 (d, J = 9.6 Hz, 2H), 6.84 (d, J = 9.6 Hz, 2H), 6.64 (m, 1H), 6.62 (m, 1H), 5.69 (m, 1H), 5.44 (s, 2H), 5.32 (s, 2H), 4.48 - 4.31 (d, J = 12.5 Hz, 1H), 4.08 - 4.03 (m, 2H), 3.86 (m, 1H), 3.72 (m, 1H), 3.58 - 3.47 (m, 4H), 3.40 - 3.35 (m, 2H), 3.00 (m, 2H), 2.91 (m, 1H), 2.65 - 2.55 (m, 2H), 2.48 (s, 3H), 2.08 (m, 2H), 2.00 - 1.83 (m, 3H), 1.38 - 1.10 (brd, 2H)

### Example C-57: Preparation of 3-(1-(3-(5-((1-(5-(5-(5-(4-(2-(2,6-dioxopiperidin-3-yl)-4-methyl-1-oxo-1,2-dihydroptalazine-6-yl)piperazine-1-yl)piperazine-5-oxopentyl)piperidine-4-il)methoxy)pyrimidine-2-yl)benzyl)-6-oxo-1,6-dihydropyridazine-3-yl)benzonitrile (ICT-0001250)

### Step1) Preparation of tert-butyl 5-(4-(((2-(3-((3-(3-(3-cyanophenyl)-6-oxophiridazine-1(6H)-yl)methyl)phenyl)pyrimidine-5-yl)oxy)methyl)piperidine-1-yl)pentanoate

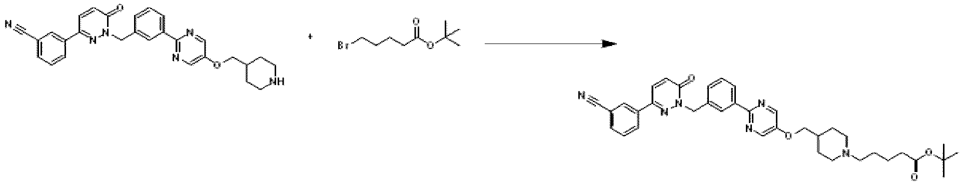

Triethylamine (3 equivalent) was added to 3-(6-oxo-1-(3-(5-(piperidine-4-ylmethoxy)pyrimidin-2-yl)benzyl)benzyl)-1,6-dihydropyridazin-3-yl) benzonitrile (100 mg, 2.09 mmol) and tert-butyl 5-bromopentanoate (390 mg, 1.46 mmol) solutions in DCM (25 ml). The reactants were stirred overnight at room temperature. The organic layer was dried, concentrated under reduced pressure, and purified by MeOH/DCM (0-20%) column chromatography to obtain the heading compound (133 mg). MS (ESI, m/z): [M+H]⁺ = 635.7

### Step 2) Preparation of 5-(4-(((2-(3-((3-(3-(3-cyanophenyl)-6-oxopyridazine-1(6H)-yl)methyl)phenyl)pyrimidine-5-il)oxy)methyl)piperidine-1-day)pentanoic acid

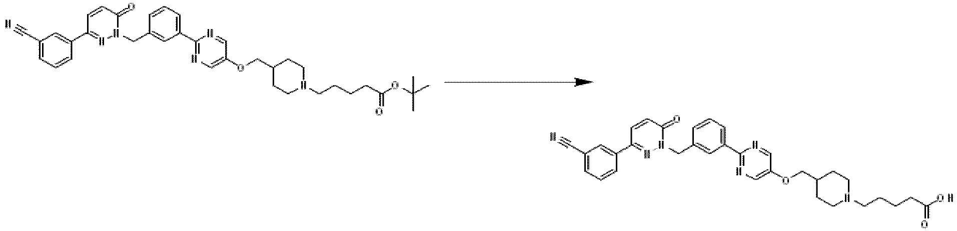

TFA (2 ml) was added to tert-butyl 5-(4-(((2-(3-((3-(3-(3-(3-(3-cyanophenyl)-6-oxopyridazine-1(6H)-yl)methyl)phenyl)pyrimidine-5-yl)oxy)methyl)piperidine-1-yl)pentanoate (133 mg, 2.10 mmol) in a solution, and the reactant was stirred at room temperature for 1 hour. After the end of the reaction, the reaction mixture was concentrated under reduced pressure. The residue was purified by MeOH/DCM (0-20%) column chromatography to obtain the title compound (121 mg). MS (ESI, m/z): [M+H]⁺ = 579.6

### Step 3) Preparation of 3-(1-(3-(5-((1-(5-(5-(4-(2-(2,6-dioxopiperidine-3-yl)-4-methyl-1-oxo-1,2-dihydropthalazine-6-yl)piperazine-1-yl)5-oxopentyl)piperidine-4-il)methoxy)pyrimidine-2-yl)benzyl)benzonitrile (ICT-0001250)

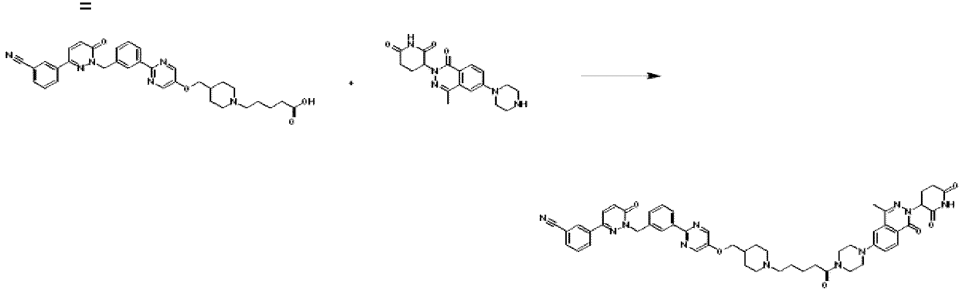

Ethylbis (propane-2-yl)amine (3-equivalent) was added to 5-(4-(((2-(3-(3-(3-(3-(3-cyanophenyl)-6-oxopyridazin-1(6H)-yl)methyl)phenyl)pyrimidine-5-yl)oxy)methyl)piperidine-1-yl)pentanoic acid (23 mg, 0.04 mmol) and 3-(4-methyl-1-oxo-6-(piperazine-1-yl)phthalazine-2(1H)-yl)piperidine-2,6-dione (14.1 mg, 0.04 mmol) and HATU (22.7 mg, 0.06 mmol) solutions in DCM (1 ml). The reactants were stirred overnight at room temperature. It was extracted with methylene chloride. The organic layer was dried with Magnesium sulfate, concentrated under reduced pressure, and purified by MeOH/DCM (0-20%) column chromatography to obtain the title compound (24.5 mg). MS (ESI, m/z): [M+H]⁺ = 917.1
1H NMR (500 MHz, DMSO-d6) δ ppm 10.91 (s, 1 H) 8.56 (s, 2 H) 8.31 (s, 2 H) 8.19 (d, J=8.39 Hz, 2 H) 8.11 (d, J=9.77 Hz, 1 H) 8.00 - 8.07 (m, 1 H) 7.86 (d, J=7.48 Hz, 1 H) 7.65 (t, J=7.93 Hz, 1 H) 7.39 - 7.44 (m, 3 H) 7.09 (d, J=9.77 Hz, 1 H) 7.01 (s, 1 H) 5.56 - 5.68 (m, 1 H) 5.38 (s, 2 H) 3.97 (d, J=5.95 Hz, 2 H) 3.43 (br. s., 4 H) 3.38 (br. s., 2 H) 3.14 - 3.26 (m, 2 H) 3.00 (br. s., 2 H) 2.83 (d, J=13.43 Hz, 2 H) 2.70 (br. s., 2 H) 2.47 - 2.59 (m, 4 H) 2.40 - 2.44 (m, 2 H) 2.30 (t, J=7.02 Hz, 2 H) 1.96 - 2.04 (m, 4 H) 1.78 - 1.93 (m, 2 H) 1.68 (br. s., 2 H) 1.54 (br. s., 2 H)

### Example C-58: Preparation of 3-(1-(3-(5-((1-(5-(5-(5-(4-(3-(2,6-dioxopiperidin-3-yl)-1-methyl-4-oxo-3,4-dihydroptalazine-6-yl)piperazine-1-yl)piperazine-5-oxopentyl)piperidine-4-yl)methoxy)pyrimidine-2-yl)benzyl)6-oxo-1,6-dihydropyridazine-3-yl)benzonitrile (ICT-0001251)

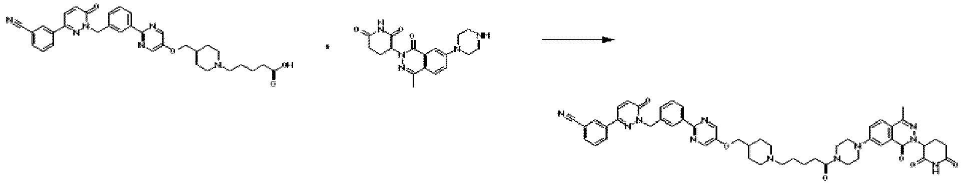

Ethylbis (propane-2-yl)amine (3 equivalents) were added to 5-(4-(((2-(3-(3-(3-(3-(3-cyanophenyl)-6-oxopyridazin-1(6H)-yl)methyl)phenyl)pyrimidine-5-yl)oxy)methyl)piperidine-1-yl)pentanoic acid (23 mg, 0.04 mmol) and 3-(4-methyl-1-oxo-7-(piperazine-1-yl)phthalazine-2(1H)-yl)piperidine-2,6-dione (14.1 mg, 0.04 mmol) and HATU (22.7 mg, 0.06 mmol) solutions in DCM (1 ml). The reactants were stirred overnight at room temperature. It was extracted with methylene chloride. The organic layer was dried with Magnesium sulfate, concentrated under reduced pressure, and purified by MeOH/DCM (0-20%) column chromatography to obtain the title compound (25.9 mg). MS (ESI, m/z): [M+H]⁺ =917.1
1H NMR (500 MHz, DMSO-d6) δ ppm 10.93 (s, 1 H) 8.54 - 8.58 (m, 2 H) 8.29 - 8.33 (m, 2 H) 8.14 - 8.19 (m, 2 H) 8.11 (d, J=9.77 Hz, 1 H) 7.86 (d, J=7.78 Hz, 1 H) 7.73 (d, J=9.00 Hz, 1 H) 7.65 (t, J=7.86 Hz, 1 H) 7.53 (dd, J=9.00, 2.59 Hz, 1 H) 7.46 (d, J=2.44 Hz, 1 H) 7.39 - 7.44 (m, 2 H) 7.09 (d, J=9.77 Hz, 1 H) 5.64 (d, J=6.41 Hz, 1 H) 5.38 (s, 2 H) 3.95 - 4.05 (m, 2 H) 3.46 - 3.59 (m, 4 H) 3.37 (br. s., 2 H) 3.03 (q, J=7.22 Hz, 2 H) 2.80 - 2.90 (m, 3 H) 2.46 - 2.58 (m, 2 H) 2.39 (s, 3 H) 2.29 (t, J=7.10 Hz, 1 H) 1.82 - 1.94 (m, 5 H) 1.67 (br. s., 2 H) 1.56 - 1.64 (m, 2 H) 1.33 - 1.52 (m, 6 H)

### Example C-59: Preparation of 3-(1-(3-(5-((1-(5-(5-(4-(2-(2,6-dioxopiperidin-3-yl)-1-oxo-1,2-dihydropthalazine-6-yl)piperazine-1-yl)-5-oxopentyl)piperidine-4-il)methoxy)pyrimidine-2-yl)benzyl)-6-oxo-1,6-dihydropyridazine-3-yl)benzonitrile (ICT-0001252)

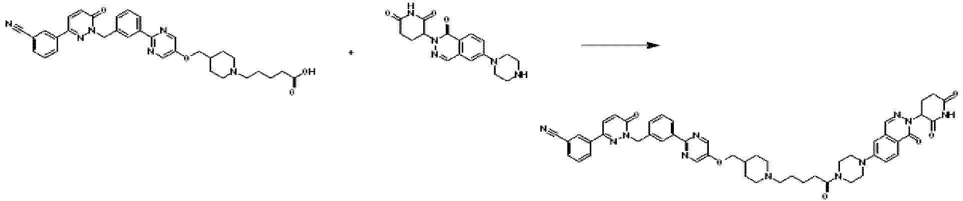

Ethylbis (propane-2-yl)amine (3 equivalents) were added to 5-(4-((2-(3-(3-(3-(3-cyanophenyl)-6-oxpyridazin-1(6H)-yl)methyl)phenyl)pyrimidine-5-yl)oxy)methyl)piperidine-1-yl)pentanoic acid (121 mg, 0.21 mmol), 3-(1-oxo-6-(piperazine-1-yl)phthalazine-2(1H)-yl)piperidine-2,6-dione (72 mg, 0.21 mmol) and HATU (160 mg, 0.32 mmol) solutions in DCM (10 ml). It was extracted with methylene chloride. The organic layer was dried with Magnesium sulfate, concentrated under reduced pressure, and purified by MeOH/DCM (0-20%) column chromatography to obtain the title compound (60 mg). MS (ESI, m/z): [M+H]⁺ =903.1
1H NMR (500 MHz, DMSO-d6) δ ppm 10.96 (s, 1 H) 8.56 (s, 2 H) 8.31 (d, J=7.48 Hz, 2 H) 8.13 - 8.23 (m, 3 H) 8.10 (d, J=9.77 Hz, 1 H) 7.99 (d, J=9.00 Hz, 1 H) 7.86 (d, J=7.78 Hz, 1 H) 7.64 (t, J=7.86 Hz, 1 H) 7.37 - 7.46 (m, 3 H) 7.14 - 7.19 (m, 1 H) 7.09 (d, J=9.77 Hz, 1 H) 5.69 (dd, J=11.90, 5.04 Hz, 1 H) 5.38 (s, 2 H) 3.98 (d, J=5.95 Hz, 2 H) 3.41 (br. s., 2 H) 3.35 (br. s., 2 H) 3.26 (d, J=9.16 Hz, 2 H) 2.96 - 3.08 (m, 2 H) 2.80 - 2.91 (m, 2 H) 2.78 (br. s., 2 H) 2.46 - 2.61 (m, 3 H) 2.31 (t, J=7.10 Hz, 2 H) 1.99 - 2.06 (m, 2 H) 1.80 - 1.98 (m, 4 H) 1.52 - 1.61 (m, 2 H) 1.35 - 1.51 (m, 4 H)

### Example C-60: Preparation of 3-(1-(3-(5-((1-(2-(3-(1-(2-(3-((4-(2-(2,6-dioxopiperidine-3-yl)-4-methyl-1-oxo-1,2-dihydropthalazine-6-yl)piperazine-1-il)methylazetidin-1-il)acetyl)piperidine-4-il)methoxy)pyrimidine-2-yl)benzyl)6-oxo-1,6-dihydropyridazine-3-yl)benzonitrile (ICT-0001253)

### Step 1) Preparation of 3-(1-(3-(5-((1-(2-chloroacetyl)piperidine-4-yl)methoxy)pyrimidine-2-yl)benzyl)-6-oxo-1,6-dihydropyridazine-3-yl)benzonitrile

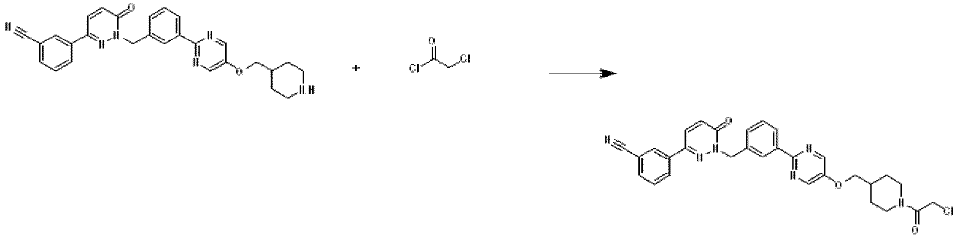

Triethylamine (0.62 ml, 8.36 mmol) was added to 3-{6-oxo-1-[(3-{5-[(piperidine-yl)methoxy]pyrimidine-2-yl}phenyl)methyl]-1,6-dihydropyridazin-3-yl}benzonitrile (2 g, 4.18 mmol), and 2-chloroacetyl chloride (0.71 g, 6.28 mmol) in CH2Cl2 (50 mL) at 0°C, warmed to room temperature, and stirred for 1 hour. After the end of the reaction, the reactant was poured into water, extracted with DCM, dried with Magnesium sulfate, and concentrated under reduced pressure. The residue was purified with HX/EA (20~90%) MPLC to obtain the title compound (37 mg). MS (ESI, m/z): [M+H]⁺ = 556.1

### Step 2) Preparation of 3-(1-(3-(5-((1-(2-(3-(3-((4-(2,6-dioxopiperidine-3-yl)-4-methyl-1-oxo-1,2-dihydrophthalazine-6-yl)piperazine-1-il)methylazetidin-1-il)acetyl)piperidine-4-il)methoxy)pyrimidine-2-yl)benzonitrile (ICT-0001253)

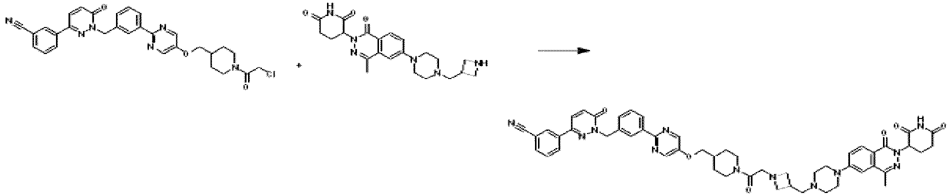

Ethylbis (45 ul, 0.26 mmol) was added to 3-(1-(3-(5-((1-(1-(1-(2-chloroacetyl)piperidine-4-yl)methoxy)pyrimidin-2-yl)benzonitrile)-6-oxo-1,6-dihydropyridazin-3-yl) benzonitrile (50 mg, 0.09 mmol) and 3-(6-(4-(4-(azzetidine-3-yl)piperazine-1-yl)piperazine-1(1H)-yl)piperidine-2,6-dione (40 mg, 0.09 mmol) in DMF (1 ml) at room temperature. The reactant was stirred for 6 hours. It was extracted with ethyl acetate, dried with Magnesium sulfate, and concentrated under reduced pressure. The residue was purified with DCM/MeOH (0-10%) MPLC to obtain the title compound. MS (ESI, m/z): [M+H]⁺ = 944.1
1H NMR (500 MHz, DMSO-d6) δ ppm 10.95 (s, 1 H) 8.65 (s, 1 H) 8.67 (s, 1 H) 8.33 - 8.40 (m, 2 H) 8.31 (s, 1 H) 8.23 (br. s., 2 H) 8.11 - 8.20 (m, 1 H) 7.97 - 8.09 (m, 1 H) 7.88 - 7.94 (m, 1 H) 7.66 - 7.75 (m, 1 H) 7.45 - 7.50 (m, 2 H) 7.13 - 7.18 (m, 1 H) 5.66 (br. s., 1 H) 5.39 - 5.46 (m, 2 H) 4.38 (br. s., 1 H) 4.07 (br. s., 3 H) 3.76 (br. s., 1 H) 3.63 (br. s., 1 H) 3.44 -3.53 (m, 4 H) 3.41 (br. s., 6 H) 3.02 (d, J=13.58 Hz, 2 H) 2.89 (br. s., 1 H) 2.73 - 2.83 (m, 1 H) 2.62 (d, J=14.19 Hz, 2 H) 2.55 (d, J=8.85 Hz, 4 H) 2.15 (d,J=12.82 Hz, 1 H) 2.07 (br. s., 2 H) 1.95 - 2.04 (m, 2 H) 1.86 - 1.95 (m, 2 H) 1.82 (br. s., 2 H)

### Example C-61: Preparation of 3-(1-(3-(5-((1-(1-(2-((6R)-6-((4-(2-(2,6-dioxopiperidin-3-yl)-4-methyl-1-oxo-1,2-dihydropthalazine-6-yl)piperazine-1-yl)methyl)1,3-oxajinan-3-il)acetyl)piperidine-4-yl)methoxy)pyrimidine-2-yl)benzyl)6-oxo-1,6-dihydropyridazine-3-yl)benzonitrile (ICT-0001254)

### Step 1) Preparation of tert-butyl(2S)-2-{[(4-methylbenzenesulfonyl)oxy]methyl}morpholine-4-carboxylate

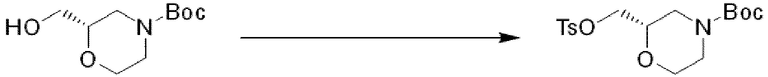

4-methylbenzene-1-sulfonyl chloride (9.65 g, 50.6 mmol), N,N-dimethylpyridine-4-amine (0.84 g, 6.9 mmol) and triethylamine (3.0 equivalent) were added to tert-butyl (2S)-2-(hydroxymethyl)morpholine-4-carboxylate (10 g, 46 mmol) solution in DCM (500 ml). Subjective compound (17 g). MS (ESI, m/z): [M+H]⁺ = 372.4.

### Step 2) tert-butyl(2R)-2-((4-(2-(2,6-dioxopiperidin-3-yl)-4-methyl-1-oxo-1,2-dihydroptalazine-6-yl)piperazine-1-yl)methyl)morpholine-4-carboxylate synthesis

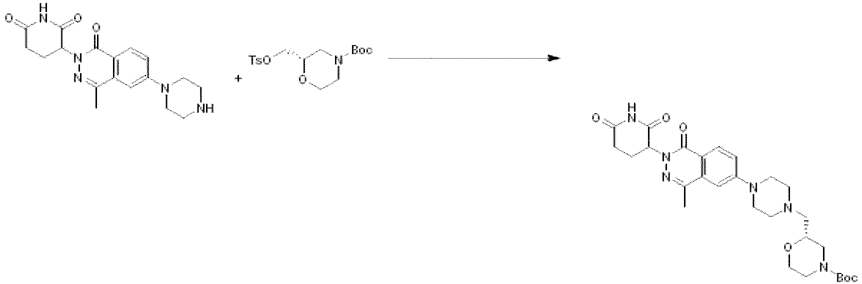

Potassium carbonate (6.04 g, 43.7 mmol) was slowly added to 3-(4-methyl-1-oxo-6-(piperazine-1-yl)phthalazine-2(1H)-yl)piperidine-2,6-dione (5.8 g, 21.9 mmol) and tert-butyl(S)-2-((tosyloxy)methyl)morpholine-4-carboxylate (7.22 g, 32.8 mmol) solution in DMF (50 ml), and stirred at 85°C for 17 hours. The reaction mixture was diluted with 200 ml EA and washed with water and brine solution. The organic layer was dried with Magnesium sulfate. It was concentrated. The residues were purified by reversed-phase column chromatography to obtain a heading compound (3.87 g). MS (ESI, m/z): [M+H]⁺ = 555.6.

### Step 3) Preparation of 3-(4-methyl-6-(4-(((S)-morpholine-2-yl)methyl)piperazine-1-yl)-1-oxopthalazine-2(1H)-yl)piperidine-2,6-dione

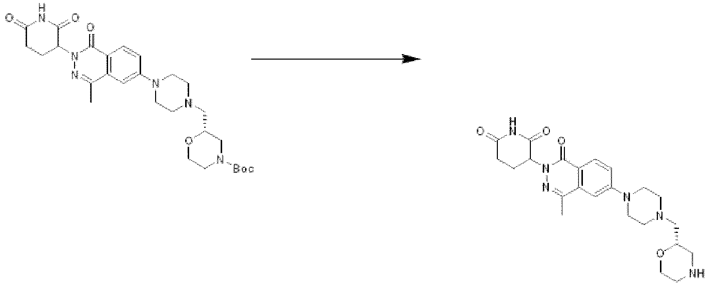

TFA (1 ml) was added to tert-butyl (2R)-2-((4-(2-(2,6-dioxopiperidin-3-yl)-4-methyl-1-oxo-1,2-dihydroptalazine-6-yl)piperazine-1-yl)methyl)morpholine-4-carboxylate (200 mg, 0.5 mmol) solution in DCM (3 ml) and stirred at room temperature for 2 hours. After the end of the reaction, the reactants were concentrated under reduced pressure and vacuum-dried. The residue was dissolved in DCM, and the subject compound was obtained by passing NH-DM 1020, Chromatorex pads, and used for the next step without further purification. MS (ESI, m/z): [M+H]⁺ = 455.6.

### Step 4) Preparation of 3-(1-(3-(5-((1-(2-((6R)-6-((4-(2-(2,6-dioxopiperidine-3-yl)-4-methyl-1-oxo-1,2-dihydropetthalazine-6-yl)piperazine-1-yl)methyl)1,3-oxajinan-3-il)acetyl)piperidine-4-yl)methoxy)pyrimidine-2-yl)benzyl)6-oxo-1,6-dihydropyridazine-3-yl)benzonitrile (ICT-0001254)

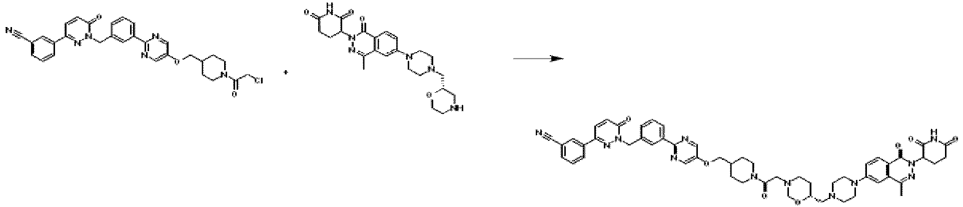

Ethylbis (45 ul, 0.09 mmol) was added to 3-(1-(3-(5-((1-(1-(1-(2-chloroacetyl)piperidine-4-yl)methoxy)pyrimidin-2-yl)benzyl)benzonitrile (50 mg, 0.09 mmol) and 3-(4-methyl-6-(4-((S)-morpholine-2-yl)methyl)piperazine-1-yl)1-oxophthalazine-2(1H)-yl)piperidine-2,6-dione (40 mg, 0.09 mmol) in DMF (1 ml) at room temperature. The reactant was stirred for 6 hours. It was extracted with ethyl acetate, dried with Magnesium sulfate, and concentrated under reduced pressure. The residue was purified with DCM/MeOH (0-10%) MPLC to obtain the title compound. MS (ESI, m/z): [M+H]⁺ = 974.1
1H NMR (500 MHz, DMSO-d6) δ ppm 10.95 (s, 1 H) 8.65 (s, 1 H) 8.67 (s, 1 H) 8.33 - 8.40 (m, 2 H) 8.31 (s, 1 H) 8.23 (br. s., 2 H) 8.11 - 8.20 (m, 1 H) 7.97 - 8.09 (m, 1 H) 7.88 - 7.94 (m, 1 H) 7.66 - 7.75 (m, 1 H) 7.45 - 7.50 (m, 2 H) 7.13 - 7.18 (m, 1 H) 5.66 (br. s., 1 H) 5.39 - 5.46 (m, 2 H) 4.38 (br. s., 1 H) 4.07 (br. s., 3 H) 3.76 (br. s., 1 H) 3.63 (br. s., 1 H) 3.44 -3.53 (m, 4 H) 3.41 (br. s., 6 H) 3.02 (d, J=13.58 Hz, 2 H) 2.89 (br. s., 1 H) 2.73 - 2.83 (m, 1 H) 2.62 (d, J=14.19 Hz, 4 H) 2.55 (d, J=8.85 Hz, 4 H) 2.15 (d,J=12.82 Hz, 1 H) 2.07 (br. s., 2 H) 1.95 - 2.04 (m, 2 H) 1.86 - 1.95 (m, 2 H) 1.82 (br. s., 2 H)

### Example C-62: Preparation of 3-(1-(3-(5-((1-(2-(2-(4-(4-(2-(2,6-dioxopiperidin-3-yl)-4-methyl-1-oxo-1,2-dihydrophthalazine-6-yl)piperazine-1-il)piperidine-1-il)acetyl)piperidine-4-il)methoxy)pyrimidine-2-yl)benzonitrile-6-oxo-1,6-dihydropyridazine-3-yl)benzonitrile (ICT-0001255)

### Step 1) Preparation of 3-(1-(3-(5-((1-(2-(4-(4-(2-(2-(2,6-dioxopiperidin-3-yl)-4-methyl-1-oxo-1,2-dihydroptalazine-6-yl)piperazine-1-il)piperidine-1-il)acetyl)piperidine-4-il)methoxy)pyrimidine-2-yl)benzyl)6-oxo-1,6-dihydropyridazine-3-yl)benzonitrile (ICT-0001255)

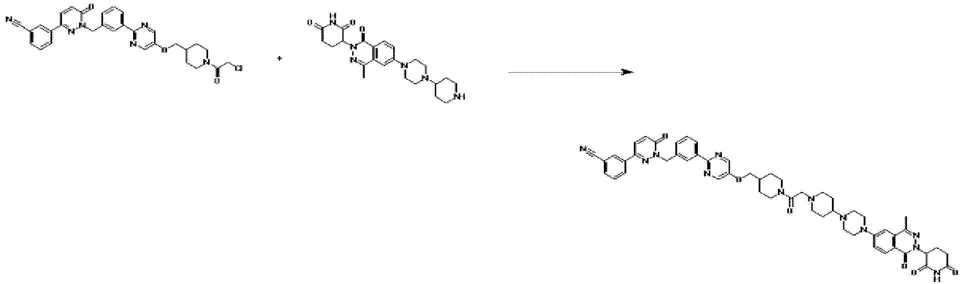

Ethylbis (45 ul, 0.26 mmol) was added to 3-(1-(3-(5-((1-(1-(1-(2-chloroacetyl)piperidine-4-yl)methoxy)pyrimidin-2-yl)benzyl)6-oxo-1,6-dihydropyridazine-3-yl) benzonitrile (50 mg, 0.09 mmol) and 3-(4-(4-methyl-1-oxo-6-(4-(piperidine-4-yl)piperazine-1-yl)phthalazine-2(1H)-yl)piperidine-2,6-dione (38 mg, 0.09 mmol) in DMF (1 ml) at room temperature. The reactants were stirred for 6 hours. The reactants were poured into water, extracted with ethyl acetate, dried with Magnesium sulfate, and concentrated under reduced pressure. The residue was purified with DCM/MeOH (0-10%) MPLC to obtain the title compound. MS (ESI, m/z): [M+H]+ = 958.2
1H NMR (500 MHz, DMSO-d6) δ ppm 10.95 (s, 1 H) 8.62 - 8.68 (m, 2 H) 8.35 (s, 1 H) 8.39 (s, 1 H) 8.22 (d, J=7.17 Hz, 2 H) 8.11 - 8.19 (m, 1 H) 8.03 (d,J=9.00 Hz, 1 H) 7.92 (d, J=7.93 Hz, 1 H) 7.65 - 7.75 (m, 1 H) 7.39 - 7.52 (m, 3 H) 7.10 - 7.19 (m, 1 H) 7.00 - 7.09 (m, 1 H) 5.67 (d, J=7.02 Hz, 1 H) 5.39 -5.47 (m, 2 H) 4.38 (d, J=13.58 Hz, 1 H) 4.09 (d, J=6.41 Hz, 3 H) 2.92 - 3.05 (m, 2 H) 2.82 - 2.92 (m, 6 H) 2.55 - 2.66 (m, 8 H) 2.20 (br. s., 2 H) 2.07 (br. s., 2 H) 1.93 - 2.04 (m, 2 H) 1.80 (br. s., 4 H) 1.42 (br. s., 2 H) 1.34 (d, J=9.31 Hz, 2 H) 1.24 (br. s., 2 H)

### Example C-63: Preparation of 3-(1-(3-(5-((1-(1-(2-(4-(4-(4-(2-(2,6-dioxopiperidin-3-yl)-4-methyl-1-oxo-1,2-dihydrophthalazine-6-yl)piperazine-1-il)methyl)piperidine-1-il)acetyl)piperidine-4-il)methoxy)pyrimidine-2-yl)benzyl)6-oxo-1,6-dihydropyridazine-3-yl)benzonitrile (ICT-0001256)

### Step 1) Preparation of 3-(1-(3-(5-((1-(2-(4-(4-(2-(2,6-dioxopiperidin-3-yl)-4-methyl-1-oxo-1,2-dihydroptalazin-6-yl)piperazine-1-il)methyl)piperidine-1-il)acetyl)piperidine-4-il)methoxy)pyrimidine-2-yl)benzonitrile (ICT-0001256)

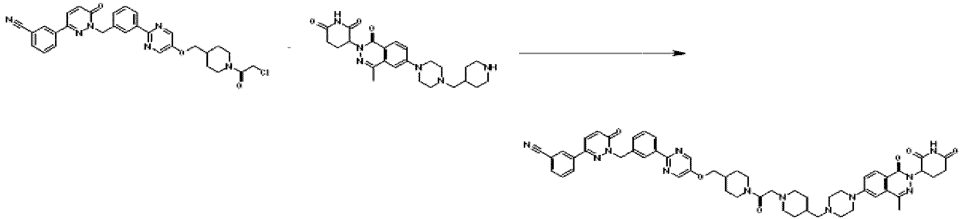

Ethylbis (45 ul, 0.26 mmol) was added to 3-(1-(3-(5-((1-(1-(2-chloroacetyl)piperidine-4-yl)methoxy)pyrimidin-2-yl)benzyl)benzonitrile (50 mg, 0.09 mmol) and 3-(4-(4-methyl-1-oxo-6-(4-(piperidine-4-ylmethyl)piperazine-1-(1H)-yl)piperidine-2,6-dione (40 mg, 0.09 mmol) in DMF (1 ml) at room temperature. The reactants were stirred for 6 hours. The reactants were poured into water, extracted with ethyl acetate, dried with Magnesium sulfate, and concentrated under reduced pressure. The residue was purified with DCM/MeOH (0-10%) MPLC to obtain the title compound. MS (ESI, m/z): [M+H]+ = 972.2
1H NMR (500 MHz, DMSO-d6) δ ppm 10.95 (s, 1 H) 8.62 - 8.68 (m, 2 H) 8.35 (s, 1 H) 8.39 (s, 1 H) 8.22 (d, J=7.17 Hz, 2 H) 8.11 - 8.19 (m, 1 H) 8.03 (d,J=9.00 Hz, 1 H) 7.92 (d, J=7.93 Hz, 1 H) 7.65 - 7.75 (m, 1 H) 7.39 - 7.52 (m, 3 H) 7.10 - 7.19 (m, 1 H) 7.00 - 7.09 (m, 1 H) 5.67 (d, J=7.02 Hz, 1 H) 5.39 -5.47 (m, 2 H) 4.38 (d, J=13.58 Hz, 1 H) 4.09 (d, J=6.41 Hz, 3 H) 2.92 - 3.05 (m, 2 H) 2.82 - 2.92 (m, 6 H) 2.55 - 2.66 (m, 8 H) 2.20 (br. s., 2 H) 2.07 (br. s., 4 H) 1.93 - 2.04 (m, 2 H) 1.80 (br. s., 4 H) 1.42 (br. s., 2 H) 1.34 (d, J=9.31 Hz, 2 H) 1.24 (br. s., 2 H)

### Example C-64: Preparation of 3-(1-(3-(5-((1-(2-(1-(1-(1-(4-(2-(2,6-dioxopiperidine-3-yl)-4-methyl-1-oxo-1,2-dihydropthalazine-6-yl)piperazine-1-carbonyl)azetidine-3-il)acetyl)piperidine-4-il)methoxy)pyrimidine-2-yl)benzyl)6-oxo-1,6-dihydropyridazine-3-yl)benzonitrile (ICT-0001257)

### Step 1) Preparation of 3-(1-(3-(5-((1-(2-(3-(3-(4-(2-(2,6-dioxopiperidine-3-yl)-4-methyl-1-oxo-1,2-dihydrophthalazine-6-yl)piperazine-1-carbonyl)azetidine-1-il)acetyl)piperidine-4-il)methoxy)pyrimidine-2-yl)benzyl)6-oxo-1,6-dihydropyridazine-3-yl)benzonitrile (ICT-0001257)

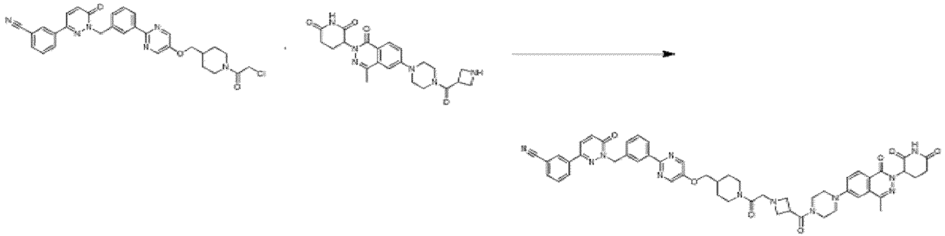

Ethylbis (45 ul, 0.26 mmol) was added to 3-(1-(3-(5-((1-(1-(2-chloroacetyl)piperidine-4-yl)methoxy)pyrimidine-2-yl)benzyl)benzonitrile (50 mg, 0.09 mmol) and 3-(6-(4-(azetidine-3-carbonyl)piperazine-1-yl)4-methyl-1-oxopthalazine-2(1H)-yl)piperidine-2,6-dione (40 mg, 0.09 mmol) in DMF (1 ml) at room temperature. The reactants were stirred for 6 hours. It was extracted with ethyl acetate, dried with Magnesium sulfate, and concentrated under reduced pressure. The residue was purified with DCM/MeOH (0-10%) MPLC to obtain the title compound. MS (ESI, m/z): [M+H]+ = 958.2.
1H NMR (500 MHz, DMSO-d6) δ ppm 10.95 (s, 1 H) 8.62 - 8.68 (m, 2 H) 8.35 (s, 1 H) 8.39 (s, 1 H) 8.22 (d, J=7.17 Hz, 2 H) 8.11 - 8.19 (m, 1 H) 8.03 (d,J=9.00 Hz, 1 H) 7.92 (d, J=7.93 Hz, 1 H) 7.65 - 7.75 (m, 1 H) 7.39 - 7.52 (m, 3 H) 7.10 - 7.19 (m, 1 H) 7.00 - 7.09 (m, 1 H) 5.67 (d, J=7.02 Hz, 1 H) 5.39 -5.47 (m, 2 H) 4.38 (d, J=13.58 Hz, 1 H) 4.09 (d, J=6.41 Hz, 3 H) 2.92 - 3.05 (m, 2 H) 2.82 - 2.92 (m, 6 H) 2.55 - 2.66 (m, 8 H) 2.20 (br. s., 2 H) 2.07 (br. s., 2 H) 1.93 - 2.04 (m, 2 H) 1.80 (br. s., 4 H) 1.42 (br. s., 2 H) 1.34 (d, J=9.31 Hz, 2 H) 1.24 (br. s., 2 H)

### Example C-65: Preparation of 3-(1-(3-(5-((1-(1-(2-((2R)-2-((4-(2-(2,6-dioxopiperidin-3-yl)-4-methyl-1-oxo-1,2-dihydrophthalazine-6-yl)piperazine-1-yl)methyl)morpholino)-2-oxoethyl)piperidine-4-il)methoxy)pyrimidine-2-yl)benzyl)6-oxo-1,6-dihydropyridazine-3-yl)benzonitrile (ICT-0001258)

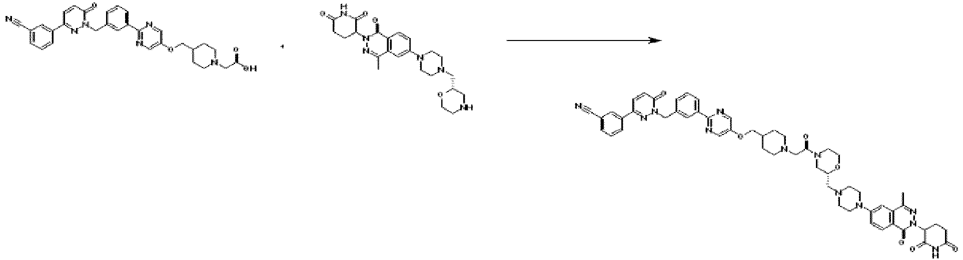

Ethylbis (propane-2-yl)amine (3-equivalent) was added to 2-(4-(((2-(3-(3-(3-(3-(3-(3-oxopridazine-1 (6H)-yl)methyl)phenyl)pyrimidine-5-yl)oxy)methyl)piperidine-1-yl)acetic acid (121 mg, 0.21 mmol) and 3-(4-methyl-6-(4-((S)-morpholine-2-yl)methyl)piperazine-1-yl)1-oxopthalazine-2(1H)-yl)piperidine-2,6-dione (70 mg, 0.21 mmol) and HATU (160 mg, 0.32 mmol) solutions in DCM (10 ml). The reactants were stirred overnight at room temperature. It was extracted with methylene chloride. The organic layer was dried with Magnesium sulfate, concentrated under reduced pressure, and purified by MeOH/DCM (0-20%) column chromatography to obtain the title compound (60 mg). MS (ESI, m/z): [M+H]⁺ =974.1
1H NMR (500 MHz, DMSO-d6) δ ppm 10.95 (d, J=6.26 Hz, 1 H) 8.63 (d, J=5.65 Hz, 2 H) 8.32 - 8.42 (m, 2 H) 8.12 - 8.30 (m, 2 H) 8.05 (dd, J=14.57, 8.62 Hz, 1 H) 7.86 - 7.98 (m, 1 H) 7.67 - 7.76 (m, 1 H) 7.60 - 7.67 (m, 1 H) 7.55 (br. s., 1 H) 7.39 - 7.52 (m, 2 H) 7.15 (dd, J=9.92, 5.49 Hz, 1 H) 7.06 (d, J=19.68 Hz, 1 H) 5.67 (br. s., 1 H) 5.44 (br. s., 2 H) 4.13 (br. s., 1 H) 4.05 (d, J=4.73 Hz, 2 H) 3.98 (d, J=10.99 Hz, 1 H) 3.84 (br. s., 2 H) 2.86 (d, J=10.53 Hz, 4 H) 2.53 - 2.68 (m, 6 H) 2.01 (br. s., 7 H) 1.93 - 2.04 (m, 2 H) 1.80 (br. s., 4 H) 1.77 (br. s., 3 H) 1.42 (br. s., 2 H)

### Example C-66: Preparation of 3-(1-(3-(5-((1-((4-(4-(3-(2,6-dioxopiperidin-3-yl)-1-methyl-4-oxo-3,4-dihydropthalazine-6-yl)piperazine-1-yl)phenyl)glycy)piperidine-4-il)methoxy)pyrimidine-2-yl)benzyl)6-oxo-1,6-dihydropyridazine-3-yl)benzonitrile (ICT-0001262)

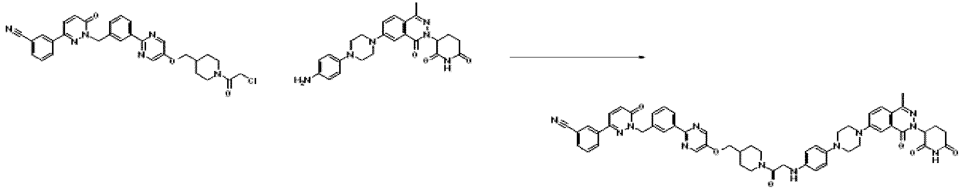

3-(1-(3-(5-((1-(1-(2-chloroacety)piperidine-4-yl)methoxy)pyrimidine-2-yl)benzyl)-6-oxo-1,in 0.6 ml DMSO DIPEA (200 µmol) was added to 6-dihydropyridazine-3-yl) benzonitrile (36 µmol) and 3-(7-(4-(4-aminophenyl)piperazine-1-yl)-4-methyl-1-oxopthalazine-2(1H)-yl)piperidine-2,6-dione (36 µmol) solutions and stirred at 90 °C for 16 hours. The reaction mixture was purified with reversed-phase column chromatography (water (0.1% FA) / ACN (0.1% FA) = 95/1 to 0/100 gradient). The corresponding fraction was lyophilized. The obtained product was used by amino silica column chromatography (DCM/ MeOH, 100/0 to 97/3, gradient), and 4.5 mg of 3-(1-(3-(5-((1-(4-(4-(4-(3-(3-(2,6-dioxopiperidine-3-yl)-1-methyl-4-oxo-3,4-dihydropthalazine-6-yl)piperazine-1-yl)phenyl)glycy)piperidine-4-il)methoxy)pyrimidine-2-yl)benzyl)6-oxo-1,6-dihydropyridazine-3-yl)benzonitrile. MS (ESI, m/z): [M+H]⁺ = 965.8
1H NMR (500 MHz, DMSO-d6) δ ppm 10.99 (s, 1H), 8.65 (s, 2H), 8.38 (m, 2H), 8.24 (m, 2H), 8.17 (d, J = 9.8 Hz, 1H), 7.94 (d, J = 9 Hz, 1H), 7.80 (d, J = 7.6 Hz, 1H), 7.72 (t, J = 7.9 Hz, 1H), 7.58 (m, 1H), 7.49 (brd, 2H), 7.26 (d, J = 9.8 Hz, 1H), 7.17 (d, J = 9.6 Hz, 2H), 6.84 (d, J = 9.6 Hz, 2H), 6.64 (m, 1H), 6.54 (s, 1H), 5.71 (m, 1H), 5.44 (s, 2H), 5.32 (s, 2H), 4.58 (m, 1H), 4.45 (m, 1H), 4.10 - 4.03 (m, 2H), 3.86 (m, 1H), 3.72 (m, 1H), 3.58 - 3.47 (m, 2H), 3.40 - 3.35 (m, 2H), 3.40 - 3.37 (m, 2H), 3.00 (brd, 2H), 2.91 (m, 1H), 2.65 - 2.55 (m, 2H), 2.47 (s, 3H), 2.08 (m, 2H), 1.63 (m, 2H), 1.38 - 1.10 (brd, 2H)

### Example C-67: Preparation of 3-(1-(3-(5-((1-(2-(4-(1-(3-(2-(4-(1-(3-(2,6-dioxopiperidin-3-yl)-1-methyl-4-oxo-3,4-dihydrophthalazine-6-yl)piperidine-4-il)piperazine-1-il)acetyl)piperidine-4-il)methoxy)pyrimidine-2-yl)benzyl)6-oxo-1,6-dihydropyridazine-3-yl)benzonitrile (ICT-0001270)

### Step 1) Preparation of benzyl 4-(1-(3-(2,6-dioxopiperidine-3-yl)-1-methyl-4-oxo-3,4-dihydroptalazine-6-yl)piperidine-4-yl)piperazine-1-carboxylate

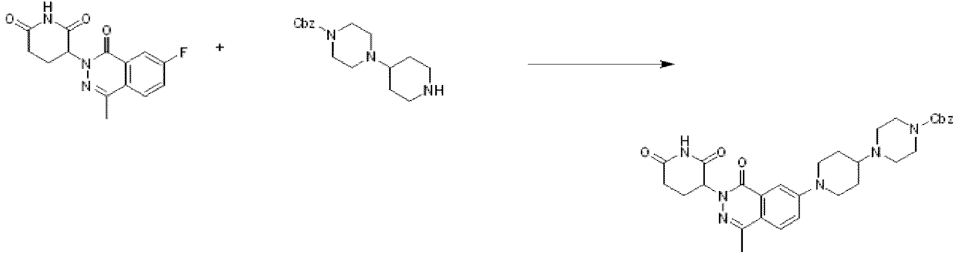

Ethylbis (propane-2-yl)amine (670 mg, 5.19 mmol) was added to 3-(7-fluoro-4-methyl-1-oxo-1,2-dihydroptalazin-2-yl) piperidine-2,6-dione (0.5 g, 1.73 mmol) and benzyl 4-(piperidine-4-yl)piperazine-1-carboxylate (524 mg, 1.73 mmol) solutions in DMA (3 mL) at room temperature. The reactants were stirred at 130°C for 16 hours. After cooling, the reactants were poured into water, extracted with ethyl acetate, dried with Magnesium sulfate, and concentrated under reduced pressure. The resulting residues were purified with 100% ethyl acetate MPLC. Subjective compound (678 mg) was obtained. MS (ESI, m/z): [M+H]⁺ = 573.3.

### Step 2) Preparation of 3-(4-methyl-1-oxo-7-(4-(piperazine-1-yl)piperidine-1-yl)phthalazine-2(1H)-yl)piperidine-2,6-dione

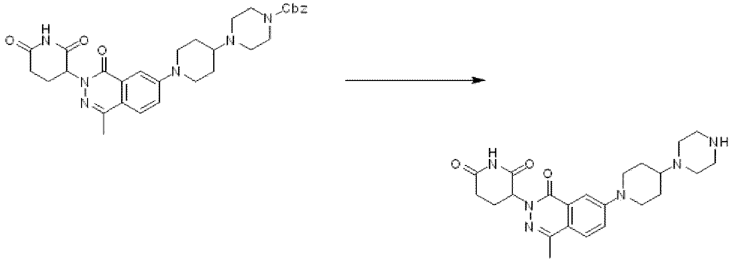

Pd/C 10% (500 mg) was added to a solution of benzyl 4-{1-[3-(2,6-dioxopiperidine-3-yl)-1-methyl-4-oxo-3,4-dihydroptallazine-6-yl]piperidine-4-yl}piperazine-1-carboxylate (1 g, 1.75 mmol) in ethanol (10 mL) at room temperature. The reaction was carried out by attaching an H2 balloon and stirred for 16 hours at room temperature. After cooling, the reactants were filtered, the filtrate was concentrated, the title compound was obtained (780 mg), which was used for the next step without additional purification. MS (ESI, m/z): [M+H]⁺ = 389.5.

### Step 3) Preparation of 3-(1-(3-(5-((1-(1-(2-(4-(1-(3-(2,6-dioxopiperidine-3-yl)-1-methyl-4-oxo-3,4-dihydropthalazine-6-yl)piperidine-4-il)piperazine-1-il)acetyl)piperidine-4-il)methoxy)pyrimidine-2-yl)benzyl)6-oxo-1,6-dihydropyridazine-3-yl)benzonitrile (ICT-0001270)

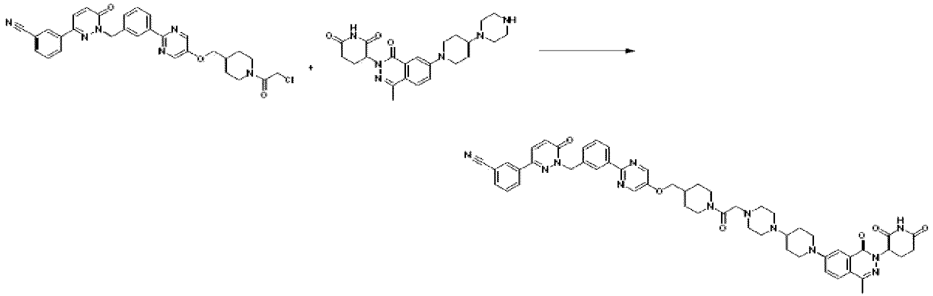

Triethylamine was added to 3-(1-{[3-(5-{[1-(2-chloroacetyl)piperidine-4-yl]methoxy}pyrimidin-2-yl)phenyl]methyl}-6-oxo-1,6-dihydropyridazine-3-yl)benzonitrile (50 mg, 90.1 µmol) and 3-{4-methyl-1-ioxo-7-[4-(piperazine-1-yl)piperidine-1-yl]-1,2-dihydropthalazine-2-yl}piperidine-2,6-dione (39.5 mg, 90.1 µmol) in DMF (5 ml). The reactants were stirred at 45°C for 4 hours. The reactants were poured into water and extracted with ethyl acetate. The organic layer was dried with Magnesium sulfate. Concentrated under reduced pressure, purified by column chromatography, and subjected compounds were obtained (49 mg). MS (ESI, m/z): [M+H]⁺ = 957.1
1H NMR (500 MHz, DMSO-d6) δ ppm 10.99 (s, 1 H) 8.61 - 8.71 (m, 2 H) 8.35 - 8.41 (m, 2 H) 8.21 - 8.27 (m, 2 H) 8.12 - 8.21 (m, 2 H) 7.91 - 7.96 (m, 1 H) 7.68 - 7.75 (m, 2 H) 7.46 - 7.53 (m, 3 H) 7.12 - 7.19 (m, 1 H) 5.40 - 5.46 (m, 2 H) 4.38 (d, J=12.36 Hz, 1 H) 4.09 (d, J=11.29 Hz, 3 H) 3.89 - 3.99 (m, 1 H) 3.47 - 3.70 (m, 3 H) 3.26 (d, J=13.43 Hz, 2 H) 2.81 - 3.04 (m, 6 H) 2.54 - 2.65 (m, 4 H) 2.42 - 2.47 (m, 5 H) 2.03 - 2.12 (m, 2 H) 1.75 - 1.88 (m, 4 H) 1.44 (br. s., 1 H) 1.39 (s, 2 H) 1.17 - 1.35 (m, 3 H) 1.14 (d, J=7.78 Hz, 1 H)

### Example C-68: Preparation of 3-(1-(3-(5-((1-(2-(4-(((2R)-4-(3-(2,6-dioxopiperidin-3-yl)-1-methyl-4-oxo-3,4-dihydrophthalazine-6-yl)morpholine-2-il)methyl)piperazine-1-il)acetyl)piperidine-4-il)methoxy)pyrimidine-2-yl)benzyl)6-oxo-1,6-dihydropyridazine-3-yl)benzonitrile (ICT-0001271)

### Step 1) Preparation of tert-butyl(2R)-2-((4-(3-(2,6-dioxopiperidin-3-yl)-1-methyl-4-oxo-3,4-dihydroptalazine-6-yl)piperazine-1-yl)methyl)morpholine-4-carboxylate

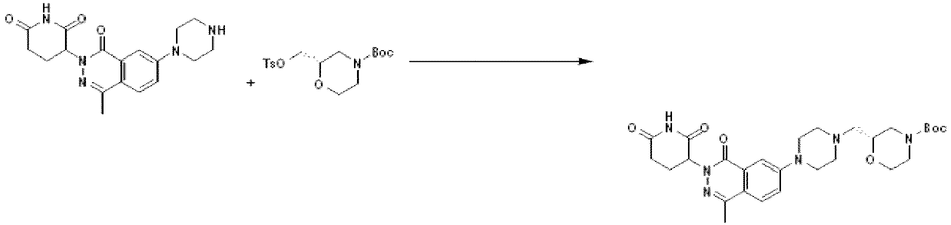

Potassium carbonate (6.04 g, 43.7 mmol) was slowly added to 3-(4-methyl-1-oxo-7-(piperazine-1-yl)phthalazine-2(1H)-yl)piperidine-2,6-dione (5.8 g, 21.9 mmol) and tert-butyl(S)-2-((tosyloxy)methyl)morpholine-4-carboxylate (7.22 g, 32.8 mmol) solution in DMF (50 ml), and stirred at 85°C for 17 hours. The reaction mixture was diluted with 200 ml EA and washed with water and brine solution. The organic layer was dried with Magnesium sulfate, It was concentrated. The residues were purified by reversed-phase column chromatography to obtain a heading compound (3.87 g). MS (ESI, m/z): [M+H]⁺ = 555.6.

### Step 2) Preparation of 3-(4-methyl-7-(4-(((S)-morpholine-2-yl)methyl)piperazine-1-yl)-1-oxopthalazine-2(1H)-yl)piperidine-2,6-dione

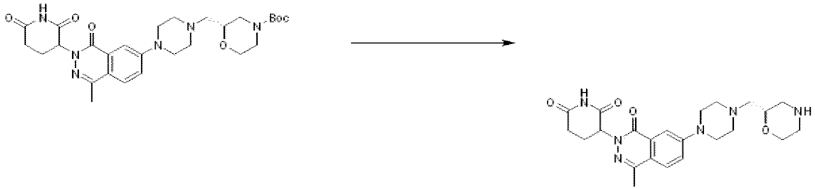

TFA (1 ml) was added to tert-butyl (2R)-2-((4-(2-(2,6-dioxopiperidin-3-yl)-4-methyl-1-oxo-1,2-dihydroptalazine-6-yl)piperazine-1-yl)methyl)morpholine-4-carboxylate (200 mg, 0.5 mmol) solution in DCM (3 ml) and stirred at room temperature for 2 hours. After the end of the reaction, the reactants were concentrated under reduced pressure and vacuum-dried. The residue was dissolved in DCM, and the subject compound was obtained by passing NH-DM 1020, Chromatorex pads, and used for the next step without further purification. MS (ESI, m/z): [M+H]⁺ = 455.6.

### Step 3) Preparation of 3-(1-(3-(5-((1-(2-(2-((2R)-4-(3-(2,6-dioxopiperidin-3-yl)-1-methyl-4-oxo-3,4-dihydrophthalazine-6-yl)morpholine-2-yl)methyl)piperazine-1-il)acetyl)piperidine-4-il)methoxy)pyrimidine-2-yl)benzonitrile (ICT-0001271)

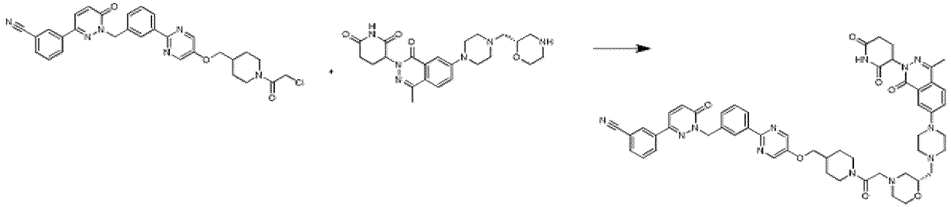

Ethylbis (45 ul, 0.26 mmol) was added to 3-(1-(3-(5-((1-(1-(2-chloroacetyl)piperidine-4-yl)methoxy)pyrimidine-2-yl)benzyl)benzonitrile (50 mg, 0.09 mmol) and 3-(4-methyl-7-(4-((S)-morpholine-2-yl)methyl)piperazine-1-yl)1-oxopthalazine-2(1H)-yl)piperidine-2,6-dione (40 mg, 0.09 mmol) in DMF (1 ml) at room temperature. The reactant was stirred for 6 hours. It was extracted with ethyl acetate, dried with Magnesium sulfate, and concentrated under reduced pressure. The residue was purified with DCM/MeOH (0-10%) MPLC to obtain the title compound. MS (ESI, m/z): [M+H]⁺ = 974.1
1H NMR (500 MHz, DMSO-d6) δ ppm 10.95 (s, 1 H) 8.65 (s, 1 H) 8.67 (s, 1 H) 8.33 - 8.40 (m, 2 H) 8.31 (s, 1 H) 8.23 (br. s., 2 H) 8.11 - 8.20 (m, 1 H) 7.97 - 8.09 (m, 1 H) 7.88 - 7.94 (m, 1 H) 7.66 - 7.75 (m, 1 H) 7.45 - 7.50 (m, 2 H) 7.13 - 7.18 (m, 1 H) 5.66 (br. s., 1 H) 5.39 - 5.46 (m, 2 H) 4.38 (br. s., 1 H) 4.07 (br. s., 3 H) 3.76 (br. s., 1 H) 3.63 (br. s., 1 H) 3.44 -3.53 (m, 4 H) 3.41 (br. s., 6 H) 3.02 (d, J=13.58 Hz, 2 H) 2.89 (br. s., 1 H) 2.73 - 2.83 (m, 1 H) 2.62 (d, J=14.19 Hz, 4 H) 2.55 (d, J=8.85 Hz, 4 H) 2.15 (d,J=12.82 Hz, 1 H) 2.07 (br. s., 2 H) 1.95 - 2.04 (m, 2 H) 1.86 - 1.95 (m, 2 H) 1.82 (br. s., 2 H)

### Example C-69: Preparation of 3-(1-(3-(5-((1-(2-(4-(4-(2-(2-(4-(2-(2,6-dioxopiperidine-3-yl)-7-fluoro-4-methyl-1-oxo-1,2-dihydroptalazine-6-yl)piperazine-1-il)methyl)piperidine-1-il)acetyl)piperidine-4-il)methoxy)pyrimidine-2-yl)benzyl)6-oxo-1,6-dihydropyridazine-3-yl)benzonitrile (ICT-0001272)

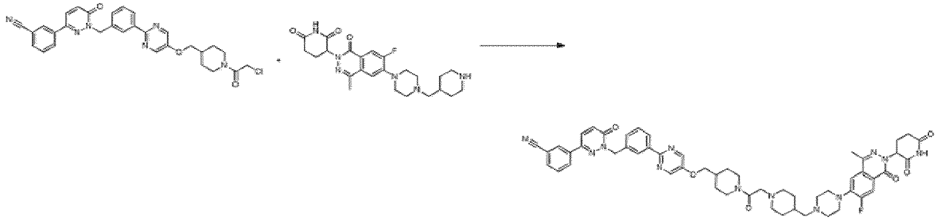

Triethylamine (18.2 mg, 180 µmol) was added to 3-(1-{[3-(5-{[1-(2-chloroacetyl)piperidine-4-yl]methoxy}pyrimidin-2-yl)phenyl]methyl}-6-oxo-1,6-dihydropyridazine-3-yl) benzonitrile (50 mg, 90.1 µmol) and 3-(7-fluoro-4-methyl-1-oxo-6-(4-(piperidine-4-yl)piperazine-1-(4-(piperidine-4-yl)piperazine-2(1H)-yl)piperidine-2,6-dione (42.4 mg, 90.1 µmol) in DMF (2 ml). The reactants were stirred at 45°C for 4 hours. The reactants were poured into water. Ethyl acetate. The organic layer was dried with Magnesium sulfate, concentrated under reduced pressure, purified by column chromatography, and the title compound was obtained (71 mg). MS (ESI, m/z): [M+H]⁺ = 990.1
1H NMR (500 MHz, DMSO-d6) δ ppm 12.04 (s, 1 H) 9.69 (s, 2 H) 9.44 (s, 1 H) 9.40 (s, 1 H) 9.27 (d, J=7.17 Hz, 2 H) 9.20 - 9.23 (m, 1 H) 8.96 (d, J=7.63 Hz, 1 H) 8.87 (d, J=12.97 Hz, 1 H) 8.75 (t, J=7.86 Hz, 1 H) 8.50 - 8.56 (m, 2 H) 8.29 (d, J=8.09 Hz, 1 H) 8.19 (d, J=9.61 Hz, 1 H) 6.75 (d, J=7.32 Hz, 1 H) 6.43 - 6.54 (m, 2 H) 5.43 (d, J=12.66 Hz, 1 H) 5.09 - 5.19 (m, 3 H) 4.24 - 4.38 (m, 4 H) 4.01 - 4.11 (m, 2 H) 3.90 - 4.01 (m, 1 H) 3.86 (br. s., 2 H) 3.57 - 3.70 (m, 3 H) 3.21 (d, J=7.02 Hz, 2 H) 2.98 - 3.17 (m, 3 H) 2.68 - 2.89 (m, 3 H) 2.38 (d, J=10.68 Hz, 1 H) 2.08 - 2.25 (m, 2 H) 1.04 (s, 2 H)

### Example C-70 : Preparation of 3-(1-{[3-(5-{[1-(2-{4-[2-(2,6-dioxopiperidin-3-yl)-1-oxo-1,2-dihydropetalazine-6-yl]piperazine-1-il}ethyl)piperidine-4-il]methoxy}pyrimidine-2-yl)phenyl]methyl}-6-oxo-1,6-dihydropyridazine-3-yl)benzonitrile (ICT-0001276)

### Step 1) Preparation of 3-(1-{[3-(5-{[1-(2-hydroxyethyl)piperidine-4-yl]methoxy}pyrimidin-2-yl)phenyl]methyl}-6-oxo-1,6-dihydropyridazine-3-yl)benzonitrile

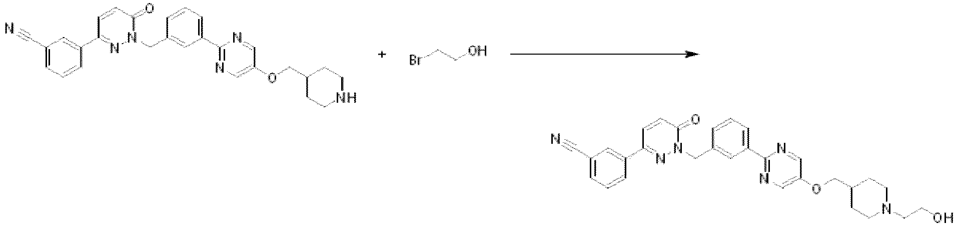

Cesium carbonate (4.0 equivalent) was added to 3-{6-oxo-1-[(3-{5-[(piperidine-4-yl)methoxy]pyrimidin-2-yl}phenyl)methyl]-1,6-dihydropyridazin-3-yl} benzonitrile (2.75 g, 5.75 mmol) and 2-bromoethane-1-ol (7.18 g, 57.5 mmol) solution in ACN (300 ml). Cesium carbonate (4.0 equivalent) was added to 3-{6-oxo-1-[(piperidine-4-yl)methoxy]pyrimidin-2-yl}phenyl)methyl]-1,6-dihydropyridazin-3-yl}benzonitrile (2.75 g, 5.75 mmol) and 2-bromoethane-1-ol (7.18 g, 57.5 mmol) solution. The reactants were stirred at 70°C for 12 hours. After the end of the reaction, the reaction mixture was concentrated under reduced pressure. The residues were purified by MeOH/DCM (0~5%) column chromatography. Subjective compound (2.5 g) MS (ESI, m/z): [M+H]⁺ = 523.6

### Step 2) Preparation of 2-[4-({[2-(3-{[3-(3-cyanophenyl)-6-oxo-1,6-dihydropyridazine-1-yl]methyl}phenyl)pyrimidine-5-il]oxy}methyl)piperidine-1-day]ethyl methanesulfonate

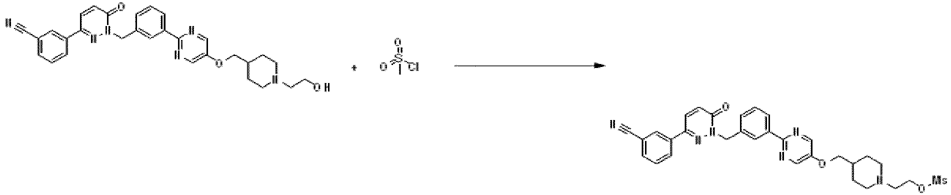

Triethylamine (4.0 equivalent) was added to 3-(1-{[3-(5-{[1-(2-hydroxyethyl)piperidine-4-yl]methoxy}pyrimidin-2-yl)phenyl]methyl}-6-oxo-1,6-dihydropyridazine-3-yl)benzonitrile (2.5 g, 4.78 mmol) and methanesulfonyl chloride (1.64 g, 14.4 mmol) in DCM (300 ml). Triethylamine (4.0 equivalent) was added to the solution of methanephonyl chloride (1.64 g, 14.4 mmol). The reactants were stirred at room temperature for 5 hours. After the end of the reaction, the reaction mixture was concentrated under reduced pressure. The residues were purified by MeOH/DCM (0~5%) column chromatography. Subjective compound (2.5 g) MS (ESI, m/z): [M+H]⁺ = 541.6

### Step 3) Preparation of 3-(1-{[3-(5-{[1-(2-{4-[2-(2,6-dioxopiperidin-3-yl)-1-oxo-1,2-dihydrophthalazine-6-yl]piperazine-1-day}ethyl)piperidine-4-day]methoxy}pyrimidin-2-yl)phenyl]methyl}-6-oxo-1,6-dihydropyridazine-3-yl)benzonitrile (ICT-0001276)

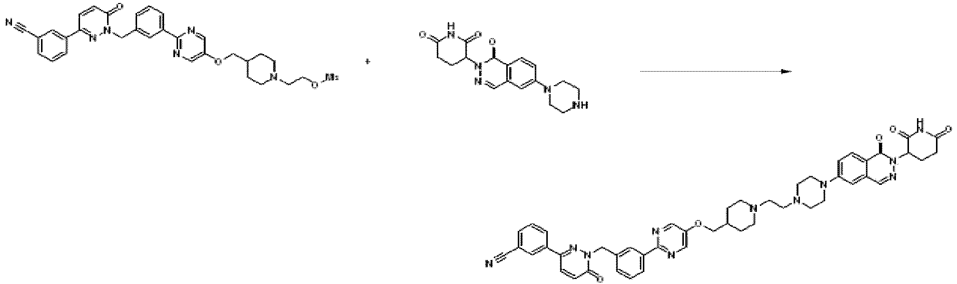

Dipotassium carbonate (27.6 mg, 0.2 mmol) was added to 2-[4-({[2-(3-{3-(3-cyanophenyl)-6-oxo-1,6-dihydropyridazin-1-yl]methyl}phenyl)pyrimidine-5-yl]oxy}methyl)piperidine-1-yl]ethyl methanesulfonate (40 mg, 0.07 mmol) and 3-[1-oxo-6-(piperazine-1-yl)-1,2-dihydroptalazine-2-yl]piperidine-2,6-dione (22.8 mg, 0.07 mmol) in DMF (2.0 ml), followed by potassium iodide (33.2 mg, 0.2 mmol). The reactants were stirred at 60°C for 5 hours. The reactants were poured into the water. It was extracted with ethyl acetate, dried with Magnesium sulfate, and concentrated under reduced pressure. The residue was purified by MeOH/DCM (0-20%) column chromatography to obtain the title compound (27 mg). MS (ESI, m/z): [M+H]⁺ = 847.0
1H NMR (500 MHz, DMSO-d6) δ ppm 10.96 (s, 1 H) 8.58 - 8.72 (m, 2 H) 8.38 (d, J=6.71 Hz, 2 H) 8.21 - 8.28 (m, 3 H) 8.18 (d, J=9.77 Hz, 1 H) 8.08 (d, J=9.00 Hz, 1 H) 7.93 (d, J=7.63 Hz, 1 H) 7.72 (t, J=7.86 Hz, 1 H) 7.43 - 7.55 (m, 3 H) 7.17 (d, J=9.77 Hz, 1 H) 7.08 - 7.15 (m, 1 H) 5.68 (dd, J=12.05, 5.49 Hz, 1 H) 5.45 (s, 2 H) 4.10 (d, J=5.04 Hz, 1 H) 3.47 (br. s., 3 H) 3.28 - 3.38 (m, 10 H) 2.85 - 2.96 (m, 1 H) 2.53 - 2.68 (m, 4 H) 2.48 - 2.52 (m, 4 H) 2.29 (s, 1 H) 2.02 - 2.12 (m, 2 H)

### Example C-71: Preparation of 3-(1-{[3-(5-{[1-(2-{4-[2-(2,6-dioxopiperidin-3-yl)-4-methyl-1-oxo-1,2-dihydrophthalazine-6-yl]piperazine-1-yl}ethyl)piperidine-4-il]methoxy}pyrimidine-2-yl)phenyl]methyl}6-oxo-1,6-dihydropyridazine-3-yl)benzonitrile (ICT-0001277)

### Step 1) Preparation of 3-(1-{[3-(5-{[1-(2-{4-[2-(2,6-dioxopiperidin-3-yl)-4-methyl-1-oxo-1,2-dihydropthalazine-6-yl]piperazine-1-il}ethyl)piperidine-4-il]methoxy}pyrimidine-2-yl)phenyl]methyl}-6-oxo-1,6-dihydropyridazine-3-yl)benzonitrile (ICT-0001277)

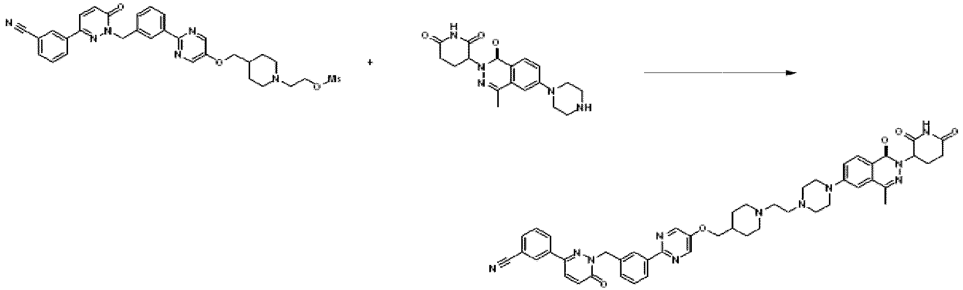

Dipotassium carbonate (27.6 mg, 0.2 mmol) was added to 2-[4-({[2-(3-{[3-(3-cyanophenyl)-6-oxo-1,6-dihydropyridazin-1-yl]methyl}phenyl)pyrimidine-5-yl]oxy}methyl)piperidine-1-yl]ethyl methanesulfonate (40 mg, 0.07 mmol) and 3-[4-methyl-1-oxo-6-(piperazine-1-yl)-1,2-dihydroptalazin-2-yl]piperidine-2,6-dione (23.7 mg, 0.07 mmol) in DMF (2.0 ml), followed by potassium iodide (33.2 mg, 0.2 mmol). The reactants were stirred at 60°C for 5 hours. The reactants were poured into the water. It was extracted with ethyl acetate, dried with Magnesium sulfate, and concentrated under reduced pressure. The residues were purified by column chromatography to obtain MeOH/DCM (0-20%) heading compound (30 mg). MS (ESI, m/z): [M+H]⁺ = 861.0
1H NMR (500 MHz, DMSO-d6) δ ppm 10.96 (s, 1 H) 8.58 - 8.72 (m, 2 H) 8.38 (d, J=6.71 Hz, 2 H) 8.21 - 8.28 (m, 2 H) 8.18 (d, J=9.77 Hz, 1 H) 8.08 (d, J=9.00 Hz, 1 H) 7.93 (d, J=7.63 Hz, 1 H) 7.72 (t, J=7.86 Hz, 1 H) 7.43 - 7.55 (m, 3 H) 7.17 (d, J=9.77 Hz, 1 H) 7.08 - 7.15 (m, 1 H) 5.68 (dd, J=12.05, 5.49 Hz, 1 H) 5.45 (s, 2 H) 4.10 (d, J=5.04 Hz, 1 H) 3.47 (br. s., 3 H) 3.28 - 3.38 (m, 10 H) 2.85 - 2.96 (m, 1 H) 2.53 - 2.68 (m, 5 H) 2.48 - 2.52 (m, 6 H) 2.29 (s, 1 H) 2.02 - 2.12 (m, 2 H)

### Example C-72: Preparation of 3-(1-{[3-(5-{[1-(2-{4-[3-(2,6-dioxopiperidin-3-yl)-1-methyl-4-oxo-3,4-dihydropthalazine-6-yl]piperazine-1-yl}ethyl)piperidine-4-day]methoxy}pyrimidine-2-yl)phenyl]methyl}6-oxo-1,6-dihydropyridazine-3-yl)benzonitrile (ICT-0001278)

### Step 1) Preparation of 3-(1-{[3-(5-{[1-(2-{4-[3-(2,6-dioxopiperidine-3-yl)-1-methyl-4-oxo-3,4-dihydropthalazine-6-yl]piperazine-1-il}ethyl)piperidine-4-il]methoxy}pyrimidine-2-yl)phenyl]methyl}-6-oxo-1,6-dihydropyridazine-3-yl)benzonitrile (ICT-0001278)

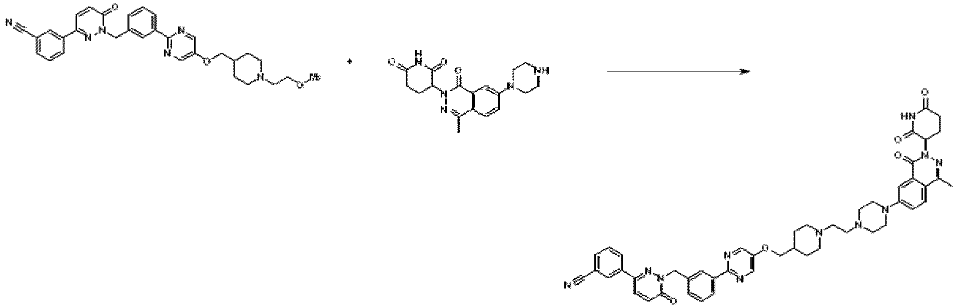

Dipotassium carbonate (27.6 mg, 0.2 mmol) was added to 2-[4-({[2-(3-{3-(3-cyanophenyl)-6-oxo-1,6-dihydropyridazin-1-yl]methyl}phenyl)pyrimidine-5-yl]oxy}methyl)piperidine-1-yl]ethyl methanesulfonate (40 mg, 0.07 mmol) and 3-[4-methyl-1-oxo-7-(piperazine-1-yl)-1,2-dihydroptalazine-2-yl]piperidine-2,6-dione (23.7 mg, 0.07 mmol) in DMF (2.0 ml), followed by potassium iodide (33.2 mg, 0.2 mmol). The reactants were stirred at 60°C for 5 hours. The reactants were poured into the water, It was extracted with ethyl acetate, dried with Magnesium sulfate, and concentrated under reduced pressure. The residues were purified by MeOH/DCM (0-20%) column chromatography to obtain the title compound (28 mg). MS (ESI, m/z): [M+H]⁺ = 861.0
1H NMR (500 MHz, DMSO-d6) δ ppm 10.94 (s, 1 H) 8.60 - 8.70 (m, 2 H) 8.35 (d, J=6.71 Hz, 2 H) 8.21 - 8.28 (m, 2 H) 8.18 (d, J=9.77 Hz, 1 H) 8.08 (d, J=9.00 Hz, 1 H) 7.93 (d, J=7.63 Hz, 1 H) 7.72 (t, J=7.86 Hz, 1 H) 7.43 - 7.55 (m, 3 H) 7.17 (d, J=9.77 Hz, 1 H) 7.08 - 7.15 (m, 1 H) 5.68 (dd, J=12.05, 5.49 Hz, 1 H) 5.40 (s, 2 H) 4.10 (d, J=5.04 Hz, 1 H) 3.47 (br. s., 4 H) 3.28 - 3.38 (m, 8 H) 2.85 - 2.96 (m, 2 H) 2.53 - 2.68 (m, 5 H) 2.47 - 2.52 (m, 4 H) 2.32 (s, 2 H) 2.02 - 2.12 (m, 2 H)

### Example C-73: Preparation of 3-(1-(4-(5-((1-(1-(5-(1-(5-(2-(2,6-dioxopiperidin-3-yl)-1-oxo-1,2-dihydroptalazine-5-yl)pentyl)piperidine-4-yl)methoxy)pyrimidine-2-yl)benzyl)6-oxo-1,6-dihydropyridazine-3-yl)benzonitrile (ICT-0001302)

### Step 1) Preparation of 3-(8-(5-hydroxypente-1-phosphorus-1-yl)-1-oxopthalazine-2(1H)-yl)piperidine-2,6-dione

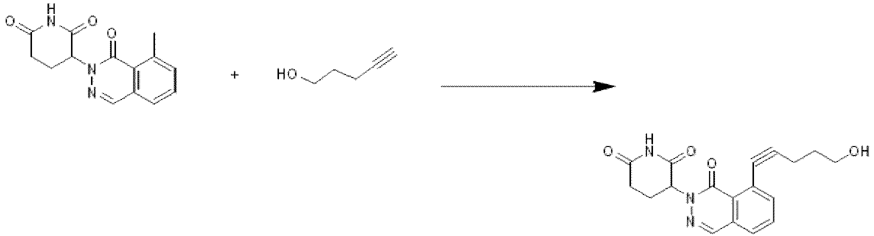

Copper iodide (I) (28 mg, 0.15 mmol) and Pd(PPh3)2Cl2 (104 mg, 0.15 mmol) were added to 3-(8-bromo-1-oxopthalazine-2(1H)-yl) solutions in DMF (5 mL) and pent-4-phosphorylase-1-ol (250 mg, 2.97 mmol) in a solution of copper iodide(I) (28 mg, 0.15 mmol) and Pd(PPh3)2Cl2 (104 mg, 0.15 mmol), followed by triethylamine (2 equivalent). The mixture was stirred at 100°C for 2 hours. The reactants were poured into the water and extracted with ethyl acetate. The organic layer was dried with Magnesium sulfate, Concentrated under reduced pressure and purified by MeOH/DCM (0-20%) column chromatography, the subject compound was obtained (540 mg). MS (ESI, m/z): [M+H]⁺ = 340.3

### Step 2) Preparation of 3-(8-(5-hydroxypentyl)-1-oxopthalazine-2(1H)-yl)piperidine-2,6-dione

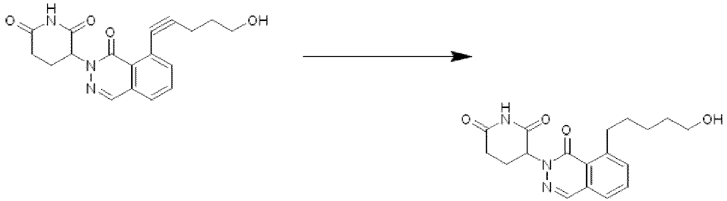

The mixture was stirred for 2 hours at room temperature in a nitrogen atmosphere in a solution of 3-(8-bromo-1-oxopthalazine-2(1H)-day) piperidine-2,6-dione (270 mg, 0.80 mmol) and palladium (7.53 mg, 0.071 mmol) in MeOH (10 ml). MS (ESI, m/z): [M+H]⁺ = 344.3

### Step3) Preparation of 5-(3-(2,6-dioxopiperidin-3-yl)-4-oxo-3,4-dihydroptalazine-5-yl)pentyl methanesulfonate

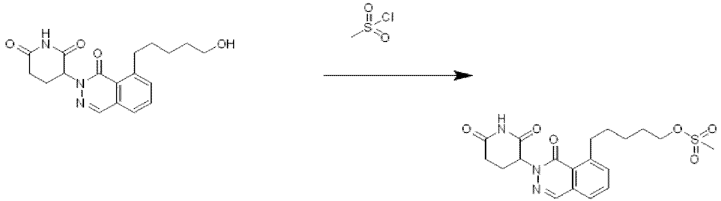

Ethylbis (propane-2-yl)amine (3 equivalents) was added to 3-(8-(5-hydroxypentyl)-1-oxotalamine-2(1H)-yl) piperidine-2,6-dione (403 mg, 0.79 mmol) and methanesulfonyl chloride (403 mg, 2.12 mmol) solutions in DCM (30 ml). The reactants were stirred at 0°C for 1 hour. The mixture was then stirred at room temperature for 1 hour. The reactants were poured into water and extracted with ethyl acetate. The organic layer was dried with Magnesium sulfate, concentrated under reduced pressure. Purified by column chromatography, the title compound was obtained (530 mg). MS (ESI, m/z): [M+H]⁺ = 422.3

### Step 4) Preparation of 3-(1-(4-(5-((1-(5-(2,6-dioxopiperidine-3-yl)-1-oxo-1,2-dihydrophthalazine-5-yl)pentyl)piperidine-4-yl)methoxy)pyrimidine-2-yl)benzyl)6-oxo-1,6-dihydropyridazine-3-yl)benzonitrile (ICT-0001302)

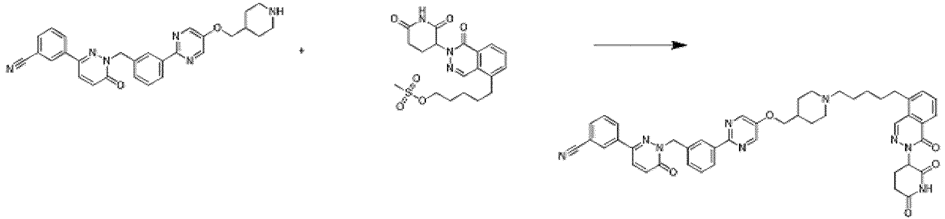

The mixture was stirred for 3 hours at 50°C in a solution of 3-(6-oxo-1-(3-(5-(piperidine-4-ylmethoxy)pyrimidin-2-yl)benzyl)-1,6-dihydropyridazin-3-yl) benzonitrile (114 mg, 0.24 mmol) and 5-(3-(2,6-dioxopiperidine-3-yl)4-oxo-3,4-dihydroptalazin-5-yl) pentyl methanesulfonate (100 mg, 0.24 mmol) and dipotassium carbonate (98 mg, 0.71 mmol) and potassium iodide (39 mg, 0.24 mmol) in DMF (3 mL). The reactants were poured into water and extracted with ethyl acetate. The organic layer was dried with Magnesium sulfate, concentrated under reduced pressure, and purified by MeOH/DCM (0-20%) column chromatography to obtain the title compound (53 mg). MS (ESI, m/z): [M+H]+ = 804.9
1H NMR (500 MHz, DMSO-d6) δ ppm 11.00 (s, 1 H) 8.55 - 8.58 (m, 2 H) 8.34 - 8.38 (m, 1 H) 8.31 (s, 2 H) 8.09 - 8.21 (m, 4 H) 7.87 (d, J=7.63 Hz, 1 H) 7.62 - 7.74 (m, 3 H) 7.42 (br. s., 2 H) 7.10 (d, J=9.92 Hz, 1 H) 5.69 - 5.77 (m, 1 H) 5.38 (s, 2 H) 3.96 (d, J=5.80 Hz, 2 H) 3.29 (br. s., 1 H) 2.69 - 2.83 (m, 4 H) 2.57 (br. s., 1 H) 2.46 - 2.55 (m, 1 H) 2.17 (t, J=6.94 Hz, 2 H) 1.79 (t, J=11.14 Hz, 2 H) 1.56 - 1.70 (m, 4 H) 1.28 - 1.43 (m, 4 H) 1.15 - 1.27 (m, 4 H)

### Example C-74: Preparation of 3-(1-(4-(5-((1-(1-(5-(1-(5-(2-(2,6-dioxopiperidin-3-yl)-1-oxo-1,2-dihydrophthalazine-5-il)pent-4-in-yl)piperidine-4-il)methoxy)pyrimidin-2-yl)benzyl)-6-oxo-1,6-dihydropyridazine-3-yl)benzonitrile (ICT-0001303)

### Step 1) Preparation of 5-(3-(2,6-dioxopiperidin-3-yl)-4-oxo-3,4-dihydroptalazine-5-yl)pent-4-phospho-1-yl methanesulfonate

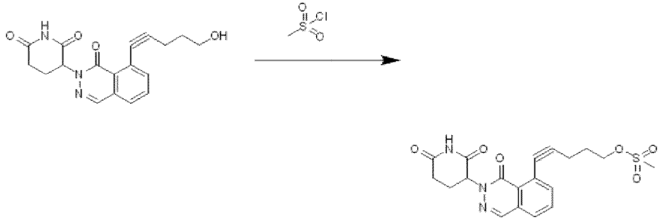

Ethylbis (propane-2-yl)amine (3 equivalent) was added to a solution of 3-(8-(5-hydroxypent-1-phosphorus-1-yl)-1-oxotalamine-2(1H)-yl) piperidine-2,6-dione (270 mg, 0.8 mmol) and methanesulfonyl chloride (273 mg, 2.39 mmol) in DCM (4.5 ml). The reactants were stirred at 0°C for 1 hour, and then the mixture was stirred at room temperature for 1 hour. The reactants were poured into the water and extracted with ethyl acetate. The organic layer was dried with Magnesium sulfate, concentrated under reduced pressure, Purified by MeOH/DCM (0-20%) column chromatography, the subject compound was obtained (325 mg). MS (ESI, m/z): [M+H]⁺ = 418.3

### Step 2) Preparation of 3-(1-(4-(5-((1-(5-(5-(2-(2,6-dioxopiperidine-3-yl)-1-oxo-1,2-dihydroptalazine-5-yl)pent-4-phosphorusine-4-yl)methoxy)pyrimidine-2-yl)benzonitrile (ICT-0001303)

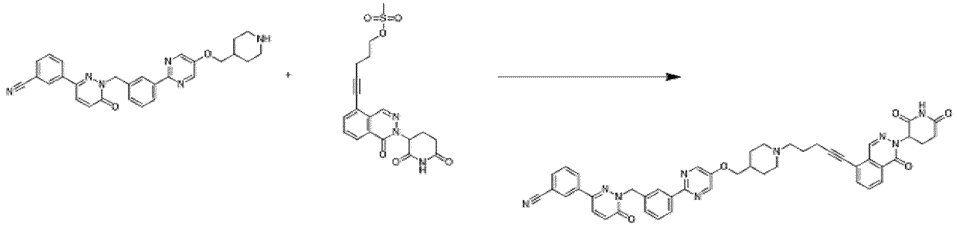

The mixture was stirred for 3 hours at 50°C in a solution of 3-(6-oxo-1-(3-(5-(piperidine-4-ylmethoxy)pyrimidin-2-yl)benzyl)-1,6-dihydropyridazine-3-yl) benzonitrile (115 mg, 0.24 mmol) and 5-(3-(2,6-dioxopiperidine-3-yl)4-oxo-3,4-dihydrophoptalazin-5-yl) pent-4-phosphorylase-1-yl methanesulfonate (100 mg, 0.24 mmol) and dipotassium carbonate (99 mg, 0.72 mmol) and potassium iodide (39 mg, 0.24 mmol) in DMF (3.0 ml). The reactants were poured into water and extracted with ethyl acetate. The organic layer was dried with Magnesium sulfate, concentrated under reduced pressure, and purified by MeOH/DCM (0-20%) column chromatography to obtain the title compound (34 mg). MS (ESI, m/z): [M+H]+ =800.4
1H NMR (500 MHz, DMSO-d6) δ ppm 11.00 (s, 1 H) 8.54 - 8.60 (m, 2 H) 8.38 (s, 1 H) 8.31 (br. s., 2 H) 8.09 - 8.21 (m, 4 H) 7.92 - 7.96 (m, 1 H) 7.87 (d, J=7.63 Hz, 1 H) 7.77 (d, J=8.85 Hz, 1 H) 7.59 - 7.68 (m, 1 H) 7.37 - 7.45 (m, 2 H) 7.10 (d, J=9.77 Hz, 1 H) 5.74 (dd, J=11.90, 5.19 Hz, 1 H) 5.38 (s, 2 H) 3.94 - 4.04 (m, 2 H) 3.32 - 3.56 (m, 2 H) 2.76 - 2.92 (m, 3 H) 2.46 - 2.59 (m, 4 H) 2.37 (t, J=6.71 Hz, 1 H) 2.00 - 2.10 (m, 1 H) 1.83 - 1.92 (m, 2 H) 1.64 - 1.75 (m, 4 H) 1.21 - 1.37 (m, 2 H)

### Example C-75: Preparation of 3-(1-(3-(5-((1-(1-(5-(5-(2-(2,6-dioxopiperidin-3-yl)-1-oxo-1,2-dihydropthalazine-6-yl)pentyl)piperidine-4-il)methoxy)pyrimidine-2-yl)benzyl)6-oxo-1,6-dihydropyridazine-3-yl)benzonitrile (ICT-0001304)

### Step1) Preparation of 3-(6-(5-hydroxypent-1-phosphoin-1-yl)-1-oxopthalazine-2(1H)-yl)piperidine-2,6-dione

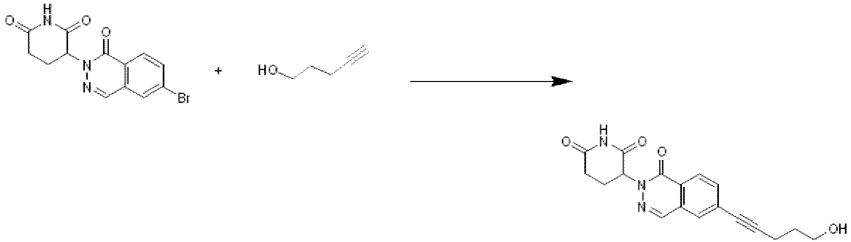

Copper iodide (I) (28 mg, 0.15 mmol) and Pd(PPh₃)₂Cl₂ (104 mg, 0.15 mmol) were added to 3-(6-bromo-1-oxopthalazine-2(1H)-yl) solutions of DMF (5 mL) and pent-4-phosphoryl-1-ol (250 mg, 2.97 mmol) in a solution of copper iodide (I) (28 mg, 0.15 mmol) and Pd (PPh3)2Cl2 (104 mg, 0.15 mmol), followed by triethylamine (2 equivalent). The mixture was stirred at 100°C for 2 hours. The reactant was poured into the water and extracted with ethyl acetate. The organic layer was dried with Magnesium sulfate. Concentrated under reduced pressure and purified by MeOH/DCM (0-20%) column chromatography, the title compound was obtained. (540 mg) MS (ESI, m/z): [M+H]⁺ = 340.3

### Step2) Preparation of 3-(6-(5-hydroxypentyl)-1-oxopthalazine-2(1H)-yl)piperidine-2,6-dione

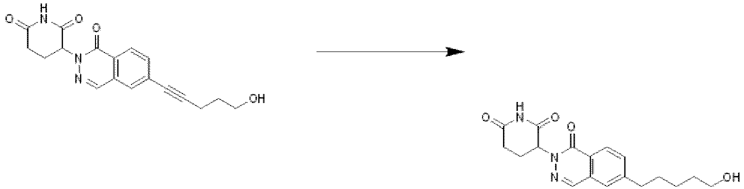

Palladium (7.53 mg, 0.071 mmol) was added to a solution of 3-(6-(5-hydroxypent-1-phosphorus-1-yl)-1-oxopthalazine-2(1H)-yl) in MeOH (10 ml), palladium (7.53 mg, 0.071 mmol) was added under a nitrogen atmosphere, and the mixture was stirred for 2 hours at room temperature. The solvent was evaporated and vacuum-dried (202 mg). MS (ESI, m/z): [M+H]⁺ = 344.3

### Step3) Preparation of 5-(2-(2,6-dioxopiperidin-3-yl)-1-oxo-1,2-dihydroptalazine-6-yl)pentyl methanesulfonate

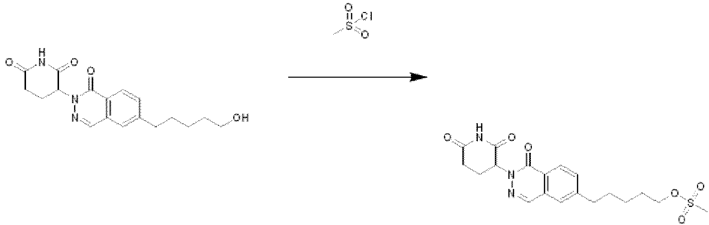

Ethylbis (propane-2-yl)amine (3 equivalents) was added to 3-(6-(5-hydroxypentyl)-1-oxotalamine-2(1H)-yl) piperidine-2,6-dione (403 mg, 0.79 mmol) and methanesulfonyl chloride (403 mg, 2.12 mmol) solutions in DCM (30 ml). The reactants were stirred at 0°C for 1 hour. The mixture was then stirred at room temperature for 1 hour. The reactants were poured into water and extracted with ethyl acetate. The organic layer was dried with Magnesium sulfate, concentrated under reduced pressure. Purified by column chromatography, the title compound was obtained (530 mg). MS (ESI, m/z): [M+H]⁺ = 422.3

### Step 4) Preparation of 3-(1-(3-(5-((1-(5-(1-(5-(2-(2,6-dioxopiperidin-3-yl)-1-oxo-1,2-dihydroptalazine-6-yl)pentyl)piperidine-4-yl)methoxy)pyrimidine-2-yl)benzonitrile (ICT-0001304)

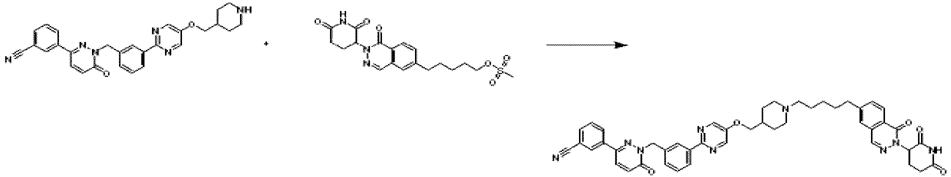

The mixture was stirred for 3 hours at 50°C in a solution of 3-(6-oxo-1-(3-(5-(piperidine-4-ylmethoxy)pyrimidin-2-yl)benzyl)-1,6-dihydropyridazin-3-yl) benzonitrile (114 mg, 0.24 mmol) and 5-(2-(2-(2,6-dioxopiperidine-3-yl)-1-oxo-1,2-dihydropthalazine-6-yl) pentyl methanesulfonate (100 mg, 0.24 mmol) and dipotassium carbonate (98 mg, 0.71 mmol) and potassium iodide (39 mg, 0.24 mmol) in DMF (3 mL). The reactants were poured into water and extracted with ethyl acetate. The organic layer was dried with Magnesium sulfate, concentrated under reduced pressure, and purified by MeOH/DCM (0-20%) column chromatography to obtain the title compound (53 mg). MS (ESI, m/z): [M+H]+ = 804.9
1H NMR (500 MHz, DMSO-d6) δ ppm 11.00 (s, 1 H) 8.55 - 8.58 (m, 2 H) 8.34 - 8.38 (m, 1 H) 8.31 (s, 2 H) 8.09 - 8.21 (m, 4 H) 7.87 (d, J=7.63 Hz, 1 H) 7.62 - 7.74 (m, 3 H) 7.42 (br. s., 2 H) 7.10 (d, J=9.92 Hz, 1 H) 5.69 - 5.77 (m, 1 H) 5.38 (s, 2 H) 3.96 (d, J=5.80 Hz, 2 H) 3.29 (br. s., 1 H) 2.69 - 2.83 (m, 4 H) 2.57 (br. s., 1 H) 2.46 - 2.55 (m, 1 H) 2.17 (t, J=6.94 Hz, 2 H) 1.79 (t, J=11.14 Hz, 2 H) 1.56 - 1.70 (m, 4 H) 1.28 - 1.43 (m, 4 H) 1.15 - 1.27 (m, 4 H)

### Example C-76: Preparation of 3-(1-(3-(5-((1-(1-(5-(2-(2,6-dioxopiperidin-3-yl)-1-oxo-1,2-dihydrophthalazine-6-yl)pent-4-phospho-1-yl)piperidine-4-il)methoxy)pyrimidine-2-yl)benzyl)-6-oxo-1,6-dihydropyridazine-3-yl)benzonitrile (ICT-0001305)

### Step 1) Preparation of 5-(2-(2,6-dioxopiperidine-3-yl)-1-oxo-1,2-dihydroptalazine-6-yl)pent-4-in-1-yl methanesulfonate

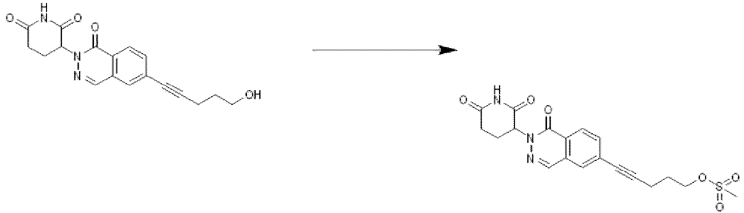

Ethylbis (propane-2-yl)amine (3 equivalent) was added to a solution of 3-(6-(5-hydroxypent-1-phosphoin-1-yl)-1-oxotalamine-2(1H)-yl) and methanesulfonyl chloride (273 mg, 2.39 mmol) in DCM (4.5 ml). The reactants were stirred at 0°C for 1 hour. Then, the mixture was stirred at room temperature for 1 hour. The reactants were poured into water and extracted with ethyl acetate. The organic layer was dried with Magnesium sulfate, concentrated under reduced pressure. Purified by MeOH/DCM (0-20%) column chromatography, the subject compound was obtained (325 mg). MS (ESI, m/z): [M+H]⁺ = 418.3

### Step 2) Preparation of 3-(1-(3-(5-((1-(5-(1-(5-(2-(2,6-dioxopiperidine-3-yl)-1-oxo-1,2-dihydropthalazine-6-yl)pent-4-phosphorus-1-il)piperidine-4-il)methoxy)pyrimidin-2-yl)benzonitrile-6-oxo-1,6-dihydropyridazine-3-yl)benzonitrile (ICT-0001305)

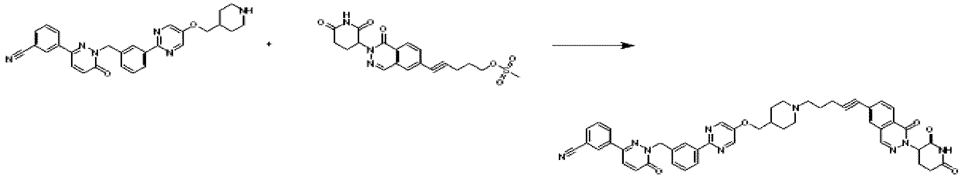

The mixture was stirred for 3 hours at 50°C for 3-(6-oxo-1-(3-(5-(piperidine-4-ylmethoxy)pyrimidin-2-yl)benzyl)-1,6-dihydropyridazin-3-yl) benzonitrile (115 mg, 0.24 mmol) and 5-(2-(2,6-dioxopiperidine-3-yl)-1-oxo-1,2-dihydroptalazine-6-yl) pent-4-phospho-1-yl methanesulfonate (100 mg, 0.24 mmol) and dipotassium carbonate (99 mg, 0.72 mmol) and potassium iodide (39 mg, 0.24 mmol) in DMF (3.0 ml). The reactants were poured into water and extracted with ethyl acetate. The organic layer was dried with Magnesium sulfate, concentrated under reduced pressure, and purified by MeOH/DCM (0-20%) column chromatography to obtain the title compound (34 mg). MS (ESI, m/z): [M+H]+ =800.4
1H NMR (500 MHz, DMSO-d6) δ ppm 11.00 (s, 1 H) 8.54 - 8.60 (m, 2 H) 8.38 (s, 1 H) 8.31 (br. s., 2 H) 8.09 - 8.21 (m, 4 H) 7.92 - 7.96 (m, 1 H) 7.87 (d, J=7.63 Hz, 1 H) 7.77 (d, J=8.85 Hz, 1 H) 7.59 - 7.68 (m, 1 H) 7.37 - 7.45 (m, 2 H) 7.10 (d, J=9.77 Hz, 1 H) 5.74 (dd, J=11.90, 5.19 Hz, 1 H) 5.38 (s, 2 H) 3.94 - 4.04 (m, 2 H) 3.32 - 3.56 (m, 2 H) 2.76 - 2.92 (m, 3 H) 2.46 - 2.59 (m, 4 H) 2.37 (t, J=6.71 Hz, 1 H) 2.00 - 2.10 (m, 1 H) 1.83 - 1.92 (m, 2 H) 1.64 - 1.75 (m, 4 H) 1.21 - 1.37 (m, 2 H)

### Example C-77: Preparation of 3-(1-(3-(5-((1-(2-(2-(2-(2-(2-(3-(2-(2-(2-(2-(2-(2-(2,6-dioxopiperidin-3-yl)-1-oxo-1,2-dihydroptalazine-5-yl)prop-2-phosphorus-1-il)oxy)ethoxy)ethoxy)ethyl)piperidine-4-il)methoxy)pyrimidine-2-yl)benzyl)6-oxo-1,6-dihydropyridazine-3-yl)benzonitrile (ICT-0001307)

### Step 1) Preparation of 3-(5-(3-(2-(2-(2-(2-(2-hydroxyethoxy)ethoxy)ethoxy)prop-1-phosphorus-1-yl)-1-oxopthalazine-2(1H)-day)piperidine-2,6-dione

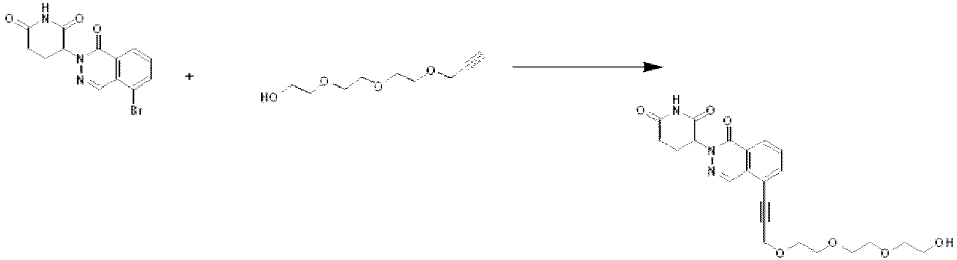

Copper iodide (I) (28 mg, 0.15 mmol) and Pd(PPh3)2Cl2 (104 mg, 0.15 mmol) were added to 3-(5-bromo-1-oxopthalazine-2(1H)-ylpride-2,6-dione (500 mg, 1.49 mmol) and 2-(2-(2-(prop-2-phosphoin-1-oxy)ethoxy)ethoxy)ethane-1-ol (250 mg, 2.97 mmol) in DMF (5 mL), followed by triethylamine (2 equivalent). The mixture was stirred at 100°C for 2 hours. The reactant was poured into the water. Ethyl acetate. The organic layer was dried with Magnesium sulfate, concentrated under reduced pressure, and purified by MeOH/DCM (0-20%) column chromatography to obtain the title compound. (540 mg) MS (ESI, m/z): [M+H]⁺ = 444.3

### Step 2) Preparation of 2-(2-(2-((3-(2-(2,6-dioxopiperidine-3-yl)-1-oxo-1,2-dihydroptalazine-5-yl)prop-2-phosphorus-1-yl)oxy)ethoxy)ethoxy)ethyl methanesulfonate

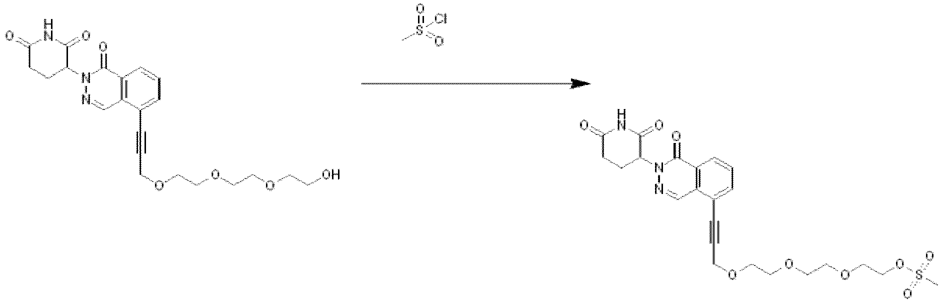

Ethyl bis(propane-2-yl)amine (3 equivalents) was added to 3-(5-(3-(2-(2-(2-(2-(2-(2-hydroxyethoxy)ethoxy)prop-1-phosphoin-1-yl)1-oxotalasine-2(1H)-yl)piperidine-2,6-dione (270 mg, 0.8 mmol) and methanesulfonyl chloride (273 mg, 2.39 mmol) solutions in DCM (4.5 ml). The reactants were stirred at 0°C for 1 hour. The mixture was then stirred at room temperature for 1 hour. The reactants were poured into water and extracted with ethyl acetate. The organic layer was dried with Magnesium sulfate. Concentrated under reduced pressure and purified by MeOH/DCM (0-20%) column chromatography to obtain a heading compound (325 mg). MS (ESI, m/z): [M+H]⁺ = 522.3

### Step 3) Preparation of 3-(1-(3-(5-((1-(2-(2-(2-(2-((3-(2-(2-(2-(2-(2-6-dioxopiperidine-3-yl)-1-oxo-1,2-dihydroptalazine-5-yl)prop-2-phosphorus-1-day)oxy)ethoxy)ethoxy)ethyl)piperidine-4-il)methoxy)pyrimidine-2-yl)benzyl)6-oxo-1,6-dihydropyridazine-3-yl)benzonitrile (ICT-0001307)

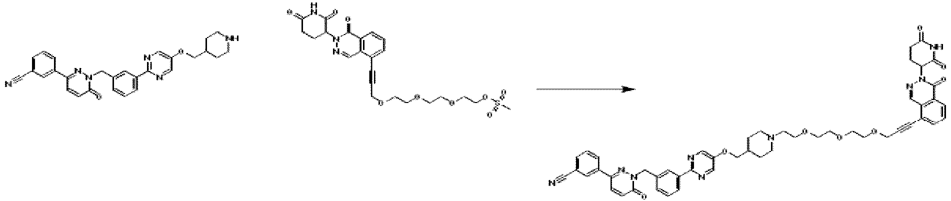

The mixture was stirred in a solution of 3-(6-oxo-1-(3-(5-(piperidine-4-ylmethoxy)pyrimidin-2-yl)benzyl)1,6-dihydropyridazin-3-yl) benzonitrile (115 mg, 0.24 mmol) and 2-(2-(2-((3-(2-(2-(2-(2-(2-(2-(2-(2-dioxopiperidin-3-yl)1-oxo-1,2-dihydroptalazine-5-yl)prop-2-phospho-1-yl)oxy)ethoxy)ethoxy)ethyl methanesulfonate (100 mg, 0.24 mmol) and dipotassium carbonate (99 mg, 0.72 mmol) and potassium iodide (39 mg, 0.24 mmol) in a solution of stirred for 3 hours at 50°C. Ethyl acetate. The organic layer was dried with Magnesium sulfate, concentrated under reduced pressure, and purified by MeOH/DCM (0-20%) column chromatography to obtain the heading compound (34 mg). MS (ESI, m/z): [M+H]+ = 905.4.
1H NMR (500 MHz, DMSO-d6) δ ppm 10.97 (s, 1H), 8.64 (s, 2 H) 8.38 (d, J=1.68 Hz, 2 H) 8.32 (s, 3 H) 8.20 - 8.27 (m, 2 H) 8.17 (d, J=9.77 Hz, 1 H) 7.93 (d, J=7.78 Hz, 1 H) 7.82 - 7.88 (m, 1 H) 7.72 (t, J=7.93 Hz, 1 H) 7.46 - 7.52 (m, 2 H) 7.29 (d, J=8.09 Hz, 1 H) 7.16 (d, J=9.61 Hz, 1 H) 5.66 - 5.76 (m, 1 H) 5.44 (s, 1 H) 4.04 (d, J=5.80 Hz, 1 H) 3.24 - 3.31 (m, 5 H) 2.84 - 2.96 (m, 4 H) 2.61 - 2.65 (m, 1 H) 2.42 (s, 3 H) 2.15 - 2.24 (m, 2 H) 2.03 - 2.11 (m, 1 H) 1.89 - 1.99 (m, 3 H) 1.82 (d, J=11.75 Hz, 1 H) 1.64 - 1.78 (m, 4 H) 1.20 - 1.36 (m, 3 H) 1.11 (d, J=11.44 Hz, 1 H)

### Example C-78: Preparation of 3-(1-(3-(5-((1-(1-(2-((2R)-2-((4-(2-(2,6-dioxopiperidin-3-yl)-4-methyl-1-oxo-1,2-dihydroptalazine-6-yl)piperazine-1-yl)methyl)morpholino)ethyl)piperidine-4-il)methoxy)pyrimidine-2-yl)benzyl)6-oxo-1,6-dihydropyridazine-3-yl)benzonitrile (ICT-0001315)

### Step 1) Preparation of 3-(1-(3-(5-((1-(2-((2R)-2-((4-(2-(2,6-dioxopiperidin-3-yl)-4-methyl-1-oxo-1,2-dihydroptalazine-6-yl)piperazine-1-day)methyl)morpholine)ethyl)piperidine-4-il)methoxy)pyrimidine-2-yl)benzonitrile (ICT-0001315)

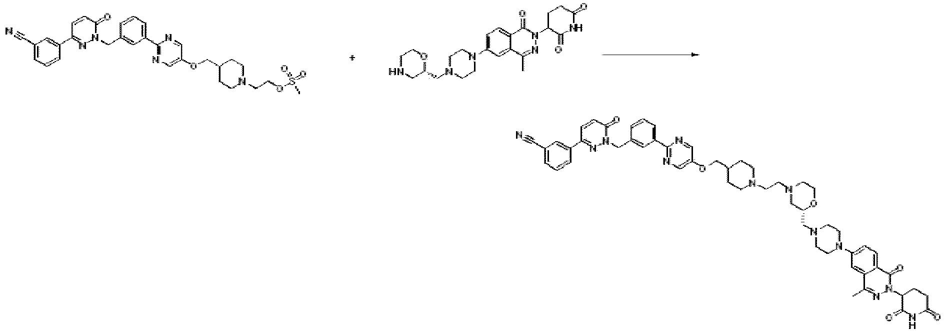

2-(4-(((2-(3-((3-(3-(3-(3-(3-oxopridazine-1(6H)-yl)methyl)phenyl)pyrimidine-5-yl)oxy)methyl)piperidine-1-yl)ethyl methanesulfonate (36 µmol) and 3-(4-methyl-6-(4-((S)-morpholine-2-yl)methyl)piperazine-1-yl)1-oxopthalazine-2(1H)-yl)piperidine-2,DIPEA (200 µmol) was added to a 6-dion (36 µmol) solution and stirred at 60°C for 16 hours. The reaction mixture was purified by reversed-phase column chromatography (water (0.1% FA) / ACN (0.1% FA) = 95/1 to 0/100 gradient). The corresponding fraction was lyophilized. The obtained products were lyophilized Purified by amino silica column chromatography (DCM/MeOH, 100/0 to 97/3, gradient), 4.5 mg of 3-(1-(3-(5-((1-(2-((2R)-2-((4-(2-(2-(dioxopiperidine-3-yl)-4-methyl-1-oxo-1,2-dihydropthalazine-6-yl)piperazine-1-yl)methylfolino)ethyl)piperidine-4-yl)methoxy)pyrimidine-2-yl)benzyl)6-oxo-1,6-dihydropyridazine-3-yl) benzonitrile. MS (ESI, m/z): [M+H]⁺ = 959.6.
1H NMR (500 MHz, DMSO-d6) δ ppm 10.97 (s, 1H), 8.64 (s, 2H), 8.38 (brd, 2H), 8.23 (m, 2H), 8.18 (d, J = 9.8 Hz, 1H), 8.05 (d, J = 9 Hz, 1H), 7.93 (d, J = 7.6 Hz, 1H), 7.72 (t, J = 7.9 Hz, 1H), 7.49 (m, 3H), 7.16 (d, J = 9.8 Hz, 1H), 7.07 (s, 1H), 5.67 (m, 1H), 5.44 (s, 2H), 4.06 (brd, 2H), 3.75 - 3.63 (m, 2H), 3.43 (m, 4H), 2.87 (m, 2H), 2.72 (brd, 1H), 2.64 - 2.55(brd, 6H), 2.48 (s, 3H), 2.40 (brd, 4H), 2.05 (m, 6H), 1.80 (brd, 4H), 1.23 (m, 5H)

### Example C-79: Preparation of 3-(1-(3-(5-((1-(2-(4-(2-(2-(4-(2-(2,6-dioxopiperidin-3-yl)-4-methyl-1-oxo-1,2-dihydrophthalazine-6-yl)piperazine-1-il)piperidine-1-il)ethyl)piperidine-4-il)methoxy)pyrimidine-2-yl)benzyl)6-oxo-1,6-dihydropyridazine-3-yl)benzonitrile (ICT-0001317)

### Step 1) Preparation of 3-(1-(3-(5-((1-(2-(4-(4-(2-(2,6-dioxopiperidine-3-yl)-4-methyl-1-oxo-1,2-dihydroptalazine-6-yl)piperazine-1-day)piperidine-1-day)ethyl)piperidine-4-il)methoxy)pyrimidine-2-yl)benzyl)6-oxo-1,6-dihydropyridazine-3-yl)benzonitrile (ICT-0001317)

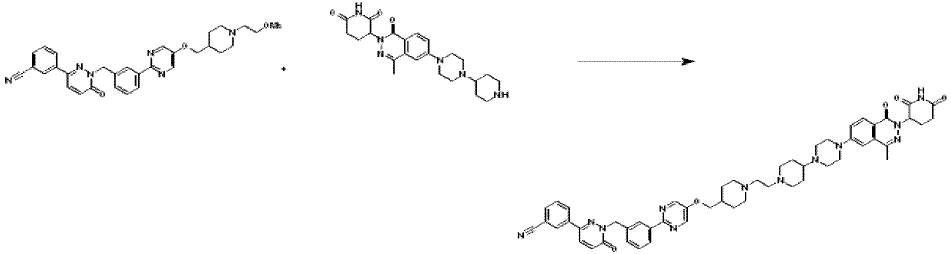

Triethylamine (9.1 mg, 90.1 µmol) was added to 2-(4-(((2-(3-(3-(3-(3-cyanophenyl)-6-oxopyridazin-1(6H)-yl)methyl)phenyl)pyrimidine-5-yl)oxy)methyl)piperidine-1-yl)ethyl methanesulfonate (50 mg, 90.1 µmol) and 3-{4-methyl-1-oxo-6-[4-(piperidine-4-yl)piperazine-1-yl]-1,2-dihydroptalazin-2-yl}piperidine-2,6-dione (42.4 mg, 90.1 µmol) in DMF (90 ml). The reactants were stirred at 45°C for 4 hours. The reactants were poured into water. Ethyl acetate. The organic layer was dried with Magnesium sulfate, concentrated under reduced pressure, purified by column chromatography, and the title compound was obtained (43 mg). MS (ESI, m/z): [M+H]⁺ = 944.1.
1H NMR (500 MHz, DMSO-d6) δ ppm 10.98 (s, 1 H) 8.66 (s, 3 H) 8.38 (s, 3 H) 8.24 (t, J=7.93 Hz, 2 H) 8.18 (d, J=9.77 Hz, 1 H) 7.94 (d, J=7.78 Hz, 1 H) 7.72 (t, J=7.93 Hz, 1 H) 7.46 - 7.53 (m, 3 H) 7.17 (d, J=9.77 Hz, 1 H) 5.45 (s, 2 H) 4.10 (d, J=4.73 Hz, 2 H) 2.84 - 2.98 (m, 3 H) 2.60 - 2.74 (m, 3 H) 2.52 - 2.60 (m, 2 H) 1.16 - 1.25 (m, 1 H)

### Example C-80: Preparation of 3-(1-(3-(5-((1-(2-(4-(4-(2-(2,6-dioxopiperidine-3-yl)-7-fluoro-4-methyl-1-oxo-1,2-dihydrophthalazine-6-yl)piperazine-1-il)methyl)piperidine-1-il)ethyl)piperidine-4-il)methoxy)pyrimidine-2-yl)benzyl)6-oxo-1,6-dihydropyridazine-3-yl)benzonitrile (ICT-0001318)

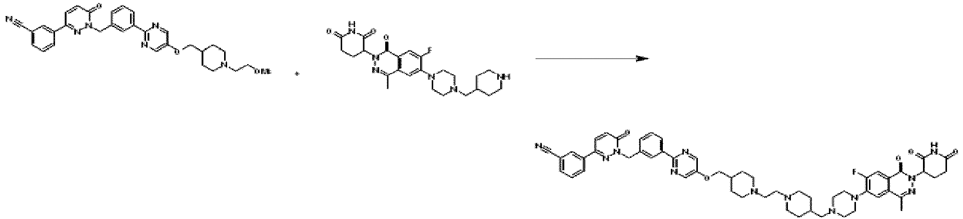

Triethylamine (10.1 mg, 100 µmol) was added to a solution of 2-(4-(((2-(3-((3-(3-(3-cyanophenyl)-6-oxopridazine-1(6H)-yl)methyl)phenyl-pyrimidine-5-yl)oxy)methyl)piperidine-1-yl)ethyl methanesulfonate (50 mg, 83.2 µmol) and 3-(7-fluoro-4-methyl-1-oxo-6-{4-[(piperidine-4-yl)methyl]piperazine-1-yl}-1,2-dihydroptalazine-2-yl)piperidine-2,6-dione (39.2 mg, 83.2 µmol). The reactants were stirred at 45°C for 4 hours. The reactants were poured into the water, Ethyl acetate. The organic layer was dried with Magnesium sulfate, concentrated under reduced pressure, purified by column chromatography, and the title compound was obtained (40 mg). MS (ESI, m/z): [M+H]⁺ = [976.1]
1H NMR (500 MHz, DMSO-d6) δ ppm 11.10 (s, 1 H) 8.58 - 8.62 (m, 2 H) 8.38 (s, 2 H) 8.22 - 8.27 (m, 2 H) 8.10 - 8.19 (m, 1 H) 8.03 - 8.09 (m, 1 H) 7.82 - 7.96 (m, 2 H) 7.68 - 7.77 (m, 1 H) 7.45 - 7.51 (m, 2 H) 7.16 (d, J=9.77 Hz, 1 H) 5.84 (d, J=7.32 Hz, 1 H) 5.41 - 5.47 (m, 2 H) 4.52 - 4.58 (m, 2 H) 4.01 (d, J=5.80 Hz, 1 H) 3.64 - 3.74 (m, 3 H) 3.62 (br. s., 3 H) 3.50 - 3.59 (m, 6 H) 3.15 - 3.22 (m, 1 H) 3.01 (br. s., 1 H) 2.89 - 2.99 (m, 1 H) 2.59 - 2.69 (m, 3 H) 2.57 (dd, J=12.74, 7.25 Hz, 1 H) 2.30 (s, 1 H) 2.10 - 2.20 (m, 1 H) 1.76 (d, J=11.29 Hz, 1 H) 1.35 (d, J=11.75 Hz, 1 H)

### Example C-81: Preparation of 3-(1-(3-(5-((1-(2-(4-(4-(2-(4-(2-(2,6-dioxopiperidin-3-yl)-4,7-dimethyl-1-oxo-1,2-dihydrophthalazine-6-yl)piperazine-1-il)methyl)piperidine-1-il)ethyl)piperidine-4-il)methoxy)pyrimidine-2-yl)benzyl)6-oxo-1,6-dihydropyridazine-3-yl)benzonitrile (ICT-0001319)

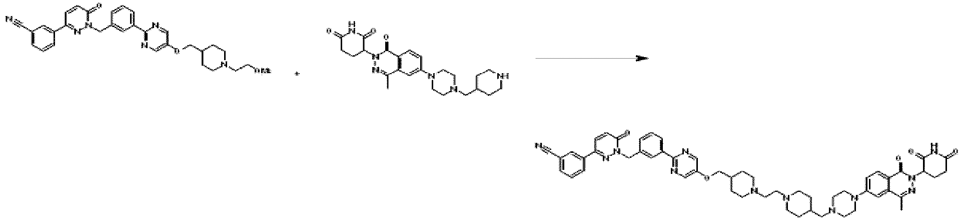

Triethylamine (10.1 mg, 100 µmol) was added to 2-(4-(((2-(3-((3-(3-(3-(3-cyanophenyl)-6-oxopyridazin-1(6H)-yl)methyl)phenyl)pyrimidine-5-yl)oxy)methyl)piperidine-1-yl)ethyl methanesulfonate (50 mg, 83.2 µmol) and 3-(4-methyl-1-oxo-6-(4-(piperidine-4-ylmethyl)piperazine-1-yl)phthalazine-2(1H)-yl)piperidine-2,6-dione (39.2 mg, 83.2 µmol) in DMF (2 ml). The reactants were stirred at 45°C for 4 hours. The reactants were poured into water. Ethyl acetate. The organic layer was dried with Magnesium sulfate, concentrated under reduced pressure, purified by column chromatography, and the title compound was obtained (40 mg). MS (ESI, m/z): [M+H]+ = 958.2
1H NMR (500 MHz, DMSO-d6) δ ppm 10.95 (s, 1 H) 8.64 (s, 2 H) 8.35 - 8.42 (m, 2 H) 8.20 - 8.28 (m, 2 H) 8.13 - 8.20 (m, 1 H) 8.04 (d, J=9.00 Hz, 1 H) 7.93 (d, J=7.63 Hz, 1 H) 7.72 (t, J=7.86 Hz, 1 H) 7.41 - 7.54 (m, 3 H) 7.16 (d, J=9.77 Hz, 1 H) 7.04 (s, 1H) 5.62 - 5.74 (m, 1 H) 5.44 (s, 2 H) 4.04 (d, J=5.95 Hz, 4 H) 2.84 - 2.97 (m, 5 H) 2.52 - 2.65 (m, 6 H) 2.47 (s, 3 H) 2.43 (br. s., 4 H) 2.36 (br. s., 1 H) 2.14 (d, J=6.41 Hz, 1 H) 1.94 - 2.10 (m, 3 H) 1.68 - 1.86 (m, 5 H) 1.31 (d, J=10.53 Hz, 3 H)

### Example C-82: Preparation of 3-(1-(3-(5-((1-(1-(2-(4-(4-(3-(2,6-dioxopiperidin-3-yl)-1-methyl-4-oxo-3,4-dihydropthalazine-6-yl)piperazine-1-il)methyl)piperidine-1-il)ethyl)piperidine-4-il)methoxy)pyrimidine-2-yl)benzonitrile-6-oxo-1,6-dihydropyridazine-3-yl)benzonitrile (ICT-0001321)

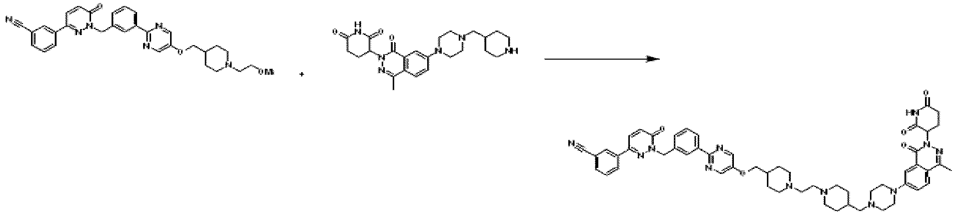

Triethylamine (10.1 mg, 100 µmol) was added to 2-(4-(((2-(3-((3-(3-(3-cyanophenyl)-6-oxopridazine-1(6H)-yl)methyl)phenyl)pyrimidine-5-yl)oxy)methyl)piperidine-1-yl)ethyl methanesulfonate (50 mg, 83.2 µmol) and 3-(4-methyl-1-oxo-7-(4-(piperidine-4-yl)piperazine-1-(4-(piperidine-4-yl)piperazine-2(1H)-yl)piperidine-2,6-dione (39.2 mg, 83.2 µmol) in DMF (2 ml). The reactants were stirred at 45°C for 4 hours. The reactants were poured into the water. Ethyl acetate. The organic layer was dried with Magnesium sulfate, concentrated under reduced pressure, purified by column chromatography, and the title compound was obtained (40 mg). MS (ESI, m/z): [M+H]+ = 958.2
1H NMR (500 MHz, DMSO-d6) δ ppm 10.96 (s, 1 H) 8.64 (s, 2 H) 8.35 - 8.42 (m, 2 H) 8.20 - 8.28 (m, 2 H) 8.12 - 8.20 (m, 1 H) 7.93 (d, J=7.78 Hz, 1 H) 7.69 - 7.79 (m, 2 H) 7.57 (dd, J=9.08, 2.52 Hz, 1 H) 7.44 - 7.54 (m, 3H) 7.16 (d, J=9.77 Hz, 1 H) 5.62 - 5.74 (m, 1 H) 5.44 (s, 2 H) 4.06 (d, J=5.80 Hz, 2 H) 3.99 (d, J=12.51 Hz, 2 H) 3.34 (br. s., 1 H) 2.91 (t, J=11.52 Hz, 4 H) 2.54 - 2.64 (m, 6 H) 2.45 (s, 4 H) 2.15 (d, J=4.73 Hz, 1 H) 1.79 (br. s., 4 H) 1.37 (d, J=11.75 Hz, 1 H)1.17 (d, J=12.21 Hz, 1 H)

### Example C-83: Preparation of 3-(1-(3-(5-((1-(2-(3-(1-(2-(3-(4-(2-(2,6-dioxopiperidin-3-yl)-4-methyl-1-oxo-1,2-dihydropthalazine-6-yl)piperazine-1-il)azetidine-1-il)ethyl)piperidine-4-il)methoxy)pyrimidine-2-yl)benzyl)6-oxo-1,6-dihydropyridazine-3-yl)benzonitrile (ICT-0001325)

### Step 1) Preparation of tert-butyl 3-(4-(2-(2,6-dioxopiperidin-3-yl)-4-methyl-1-oxo-1,2-dihydroptalazine-6-yl)piperazine-1-yl)azetidine-1-carboxylate

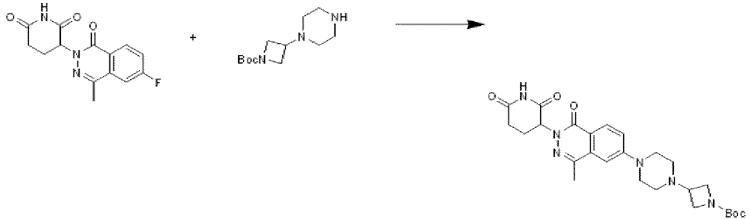

3-(6-fluoro-4-methyl-1-oxopthalazine-2(1H)-yl)piperidine-2,6-dione (642 mg, 2.4 mmol), tert-butyl 3-(piperazine-1-yl)azetidine-1-carboxylate (643 mg, 2.66 mmol), and DIPEA (0.93 ml, 5.33 mmol) solutions in NMP (15 mL) were stirred at 120°C for 20 hours. MS (ESI, m/z): [M+H]⁺ = 511.3.

### Step 2) Preparation of 3-(6-(4-(azetidine-3-yl)piperazine-1-yl)-4-methyl-1-oxopthalazine-2(1H)-yl)piperidine-2,6-dione

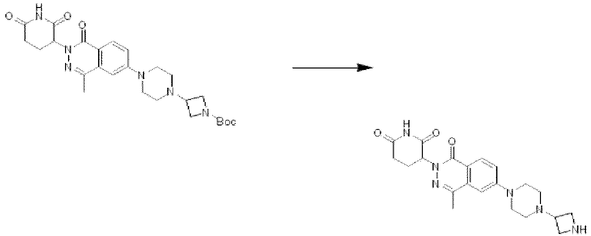

TFA (0.3 ml) was added to tert-butyl 3-(4-(2-(2,6-dioxopiperidin-3-yl)-4-methyl-1-oxo-1,2-dihydroptalazin-6-yl)piperazine-1-yl)azetidine-1-carboxylate (180 mg, 0.4 mmol) in DCM (1 ml) and stirred at room temperature for 2 hours. After the end of the reaction, the reactants were concentrated under reduced pressure and vacuum-dried. The residues were dissolved in DCM, NH-DM 1020, passed through a Chromatorex pad to obtain the heading compound, and used in the next step without further purification (140 mg). MS (ESI, m/z): [M+H]⁺ = 411.3.

### Step 3) Preparation of 3-(1-(3-(5-((1-(2-(3-(3-(3-(4-(2-(2,6-dioxopiperidin-3-yl)-4-methyl-1-oxo-1,2-dihydroptalazine-6-yl)piperazine-1-day)azetidine-1-il)ethyl)piperidine-4-il)methoxy)pyrimidine-2-yl)benzyl)6-oxo-1,6-dihydropyridazine-3-yl)benzonitrile (ICT-0001325)

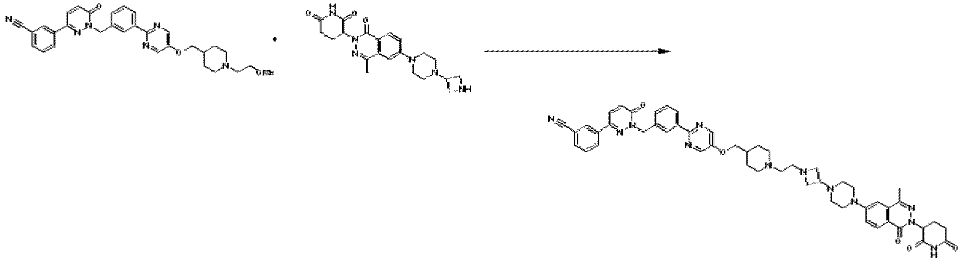

Triethylamine (10.4 mg, 102 µmol) was added to a solution of 2-[4-({[2-(3-{[3-(3-cyanophenyl)-6-oxo-1,6-dihydropyridazin-1-yl]methyl}phenyl)pyrimidine-5-yl]oxy}methyl)piperidine-1-yl]ethylbenzenesulfonate (50 mg, 75.4 µmol) and 3-{6-[4-(azetidine-3-yl)piperazine-1-yl]-4-methyl-1-oxo-1,2-dihydroptalazine-2-yl}piperidine-2,6-dione (382 mg, 75.42 µmol) in a solution. The reactants were stirred at 45°C for 4 hours. The reactants were poured into the water. Ethyl acetate. The organic layer was dried with Magnesium sulfate, concentrated under reduced pressure, and purified by column chromatography to obtain the title compound (61 mg). MS (ESI, m/z): [M+H]⁺ = 916.1
1H NMR (500 MHz, DMSO-d6) δ ppm 10.98 (s, 1 H) 8.64 (s, 2 H) 8.38 (s, 2 H) 8.15 - 8.28 (m, 4 H) 8.07 (d, J=8.85 Hz, 1 H) 7.94 (d, J=7.63 Hz, 1 H) 7.72 (t, J=7.86 Hz, 1 H) 7.47 - 7.52 (m, 3 H) 7.17 (d, J=9.77 Hz, 1 H) 5.44 (s, 2 H) 4.04 (d, J=5.65 Hz, 2 H) 3.41 - 3.51 (m, 2 H) 3.33 (br. s., 1 H) 2.92 (d, J=17.24 Hz, 3 H) 2.54 - 2.66 (m, 4 H) 2.39 - 2.48 (m, 4 H) 2.36 (br. s., 1 H) 2.30 (br. s., 1 H) 1.97 (t, J=11.06 Hz, 1 H) 1.75 (d, J=12.05 Hz, 2 H) 1.31 (d, J=10.68 Hz, 1 H) 1.15 - 1.27 (m, 1 H) 0.99 (d, J=6.26 Hz, 1 H)

### Example C-84: Preparation of 3-(1-(3-(5-((1-(2-(4-(4-(3-(2-(2-(4-(3-(2,6-dioxopiperidine-3-yl)-7-fluoro-1-methyl-4-oxo-3,4-dihydropthalazine-6-yl)piperazine-1-il)methyl)piperidine-1-il)ethyl)piperidine-4-il)methoxy)pyrimidine-2-yl)benzyl)6-oxo-1,6-dihydropyridazine-3-yl)benzonitrile (ICT-0001326)

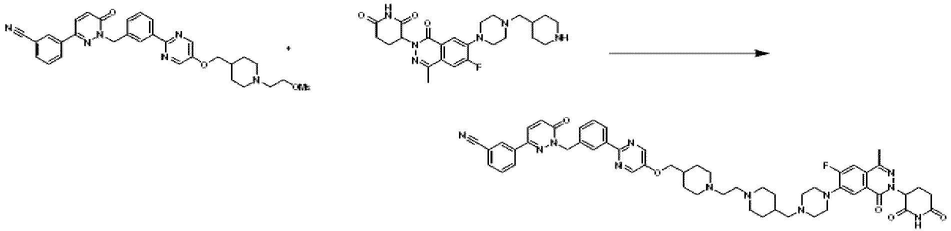

Triethylamine (15.3 mg, 151 µmol) was added to a solution of 2-[4-({[2-(3-{[3-(3-cyanophenyl)-6-oxo-1,6-dihydropyridazin-1-yl]methyl}phenyl)pyrimidine-5-yl]oxy}methyl)piperidine-1-yl]ethylbenzenesulfonate (50 mg, 75.4 µmol) and 3-{6-[4-(azetidine-3-yl)piperazine-1-yl]-4-methyl-1-oxo-1,2-dihydroptalazine-2-yl}piperidine-2,6-dione (39 mg, 83.0 µmol) in DMF (2 ml). The reactants were stirred at 45°C for 4 hours. The reactants were poured into the water. Ethyl acetate. The organic layer was dried with Magnesium sulfate, concentrated under reduced pressure, and purified by column chromatography to obtain the title compound (56 mg). MS (ESI, m/z): [M+H]⁺ = 976.1
1H NMR (500 MHz, DMSO-d6) δ ppm 11.01 (s, 1 H) 8.64 (s, 2 H) 8.38 (s, 2 H) 8.21 - 8.27 (m, 2 H) 8.15 - 8.20 (m, 1 H) 7.94 (d, J=7.78 Hz, 1 H) 7.67 - 7.78 (m, 3 H) 7.47 - 7.51 (m, 2 H) 7.17 (d, J=9.77 Hz, 1 H) 5.70 (br. s., 1 H) 5.44 (s, 2 H) 4.05 (d, J=5.80 Hz, 2 H) 3.30 - 3.41 (m, 7 H) 2.99 (br. s., 3 H) 2.87 - 2.96 (m, 2 H) 2.63 (br. s., 1 H) 2.59 (br. s., 2 H) 2.54 (br. s., 2 H) 2.47 (s, 3 H) 2.36 (br. s., 1 H) 2.20 (d, J=7.02 Hz, 2 H) 2.08 (d, J=11.60 Hz, 2 H) 1.77 (d, J=12.97 Hz, 3 H) 1.71 (br. s., 1 H) 1.58 (br. s., 1 H) 1.34 (d, J=10.53 Hz, 2 H) 1.12 - 1.25 (m, 5 H)

### Example C-85: Preparation of 3-(1-(3-(5-((1-(1-(4-((2,6-dioxopiperidine-3-yl)amino)-2-fluorophenyl)piperidine-4-yl)methoxy)pyrimidine-2-yl)benzyl)6-oxo-1,6-dihydropyridazine-3-yl)benzonitrile (ICT-0001383)

### Step 1) Preparation of 3-(1-(3-(5-((1-(2-fluoro-4-nitrophenyl)piperidine-4-yl)methoxy)pyrimidine-2-yl)benzyl)-6-oxo-1,6-dihydropyridazine-3-yl)benzonitrile

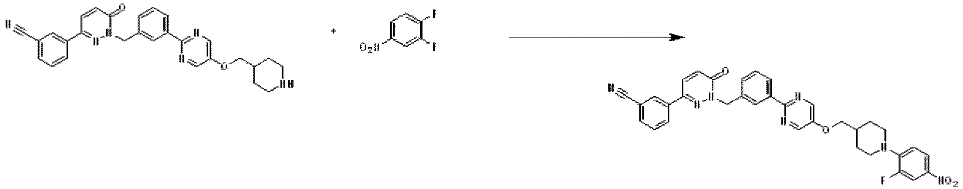

Ethylbis (propane-2-yl)amine (35.5 mg, 274 µmol) was added to a solution of 3-{6-oxo-1-[(piperidine-4-yl)methoxy]pyrimidin-2-yl}phenyl)methyl]-1,6-dihydropyridazin-3-yl}benzonitrile (87.5 mg, 183 µmol) and 1,2-difluoro-4-nitrobenzene (29.1 mg, 183 µmol) in DMF (2 ml). Ethyl bis (propane-2-yl)amine (35.5 mg, 274 µmol) was added to a solution of 3-{6-oxo-1-[(piperidine-4-yl)methoxy]pyrimidin-2-yl}phenyl)methyl]-1,6-dihydropyridazin-3-yl}benzonitrile (87.5 mg, 183 µmol) and 1,2-difluoro-4-nitrobenzene (29.1 mg, 183 µmol) in a solution. The reactants were stirred at 120°C for 2 hours. After cooling to room temperature, the reactants were poured into water and extracted with ethyl acetate. The organic layer was dried with Magnesium sulfate, concentrated under reduced pressure. Purified by column chromatography, the title compound was obtained (110 mg). MS (ESI, m/z): [M+H]⁺ = 618.2.

### Step 2) Preparation of 3-(1-(3-(5-((1-(4-amino-2-fluorophenyl)piperidine-4-yl)methoxy)pyrimidine-2-yl)benzyl)-6-oxo-1,6-dihydropyridazine-3-yl)benzonitrile

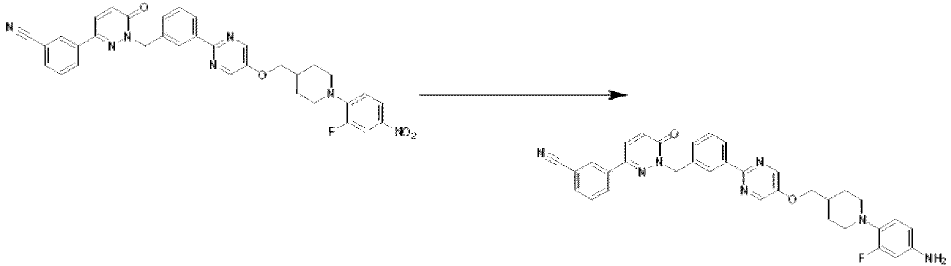

2 mL of saturated NH4Cl was added to 3-(1-{[3-(5-{[1-(2-fluoro-4-nitrophenyl)piperidine-4-yl]methoxy}pyrimidin-2-yl)phenyl]methyl}-6-oxo-1,6-dihydropyridazine-3-yl)benzonitrile (100 mg, 162 µmol) and iron (45.2 mg, 810 µmol) solutions in THF (2 ml). The reactants were stirred at 100°C for 2 hours. After cooling, the reactants were filtered to room temperature, the filtrate was poured into water, and extracted with ethyl acetate. The organic layer was dried with Magnesium sulfate. Concentrated under reduced pressure, purified by column chromatography, the title compound was obtained (85 mg). MS (ESI, m/z): [M+H]⁺ = 588.4
1H NMR (500 MHz, DMSO-d6) δ ppm 10.72 (s, 1 H) 8.58 - 8.63 (m, 3 H) 8.32 (br. s., 3 H) 8.15 - 8.21 (m, 3 H) 8.11 (d, J=9.77 Hz, 1 H) 7.87 (d, J=7.63 Hz, 1 H) 7.66 (t, J=7.86 Hz, 1 H) 7.39 - 7.44 (m, 2 H) 7.10 (d, J=9.77 Hz, 1 H) 6.79 (t, J=9.23 Hz, 1 H) 6.44 (d, J=14.95 Hz, 1 H) 6.35 (d, J=9.00 Hz, 1 H) 5.74 (d, J=7.78 Hz, 1 H) 5.38 (s, 2 H) 4.15 - 4.24 (m, 1 H) 4.04 (d, J=5.95 Hz, 2 H) 3.10 (d, J=11.14 Hz, 2 H) 2.62 - 2.72 (m, 1 H) 2.50 - 2.55 (m, 2 H) 1.98 - 2.05 (m, 1 H) 1.77 - 1.81 (m, 2 H) 1.36 - 1.47 (m, 2 H)

### Step 3) Preparation of 3-(1-(3-(5-((1-((1-(1-(2,6-dioxopiperidine-3-yl)-1H-1,2,3-triazole-4-yl)methyl)piperidine-4-yl)methoxy)pyrimidine-2-yl)benzyl)6-oxo-1,6-dihydropyridazine-3-yl)benzonitrile (ICT-0001383)

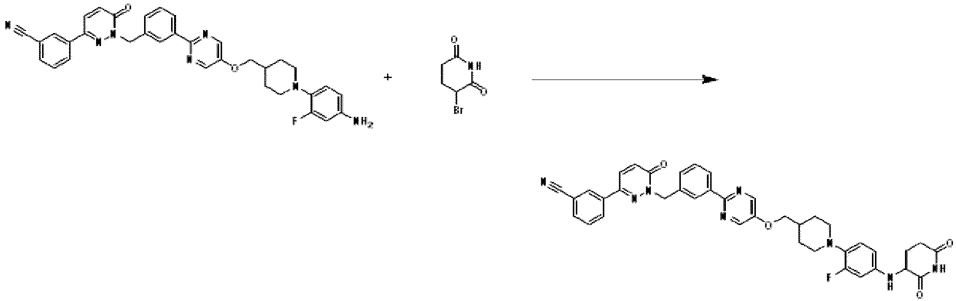

Sodium bicarbonate (27.9 mg, 332 µmol) was added to a solution of 3-(1-{3-(5-{[1-(4-amino-2-fluorophenyl)piperidine-4-yl]methoxy}pyrimidin-2-yl)phenyl]methyl}-6-oxo-1,6-dihydropyridazine-3-yl) benzonitrile (65 mg, 1114 µmol) and 3-bromomorpheridin-2,6-dione (42.5 mg, 221 µmol) in a solution. The reactants were stirred at 60°C for 12 hours. The reactants were filtered, the filtrate was poured into water and extracted with ethyl acetate. The organic layer was dried with Magnesium sulfate. Concentrated under reduced pressure, purified by column chromatography, the subject compound was obtained (56 mg). MS (ESI, m/z): [M+H]⁺ = 699.7,

### Example C-86: Preparation of 3-{1-[(3-{5-[(1-{[1-(2,6-dioxopiperidine-3-yl)-1H-1,2,3-triazole-4-yl]methyl}piperidine-4-il)methoxy]pyrimidine-2-yl}phenyl)methyl]-6-oxo-1,6-dihydropyridazine-3-yl}benzonitrile ICT-0001384)

### Step 1) Preparation of 3-{1-[(3-{5-[(1-{[1-(2,6-dioxopiperidin-3-yl)-1H-1,2,3-triazole-4-yl]methyl}piperidine-4-il)methoxy]pyrimidine-2-yl}phenyl)methyl]-6-oxo-1,6-dihydropyridazine-3-yl}benzonitrile ICT-0001384)

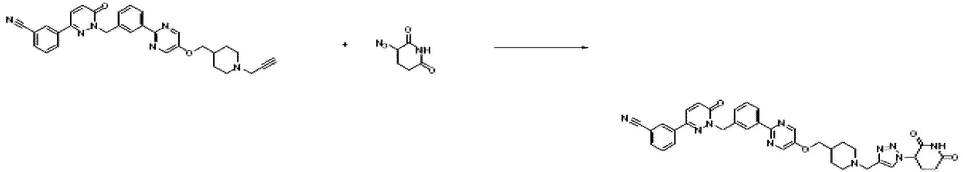

Sodium ascorbate and copper sulfate (2+) were added to a solution of 3-(6-oxo-1-{[3-(5-{1-(prop-2-phospho-1-yl)piperidine-4-yl]methoxy}pyrimidine-2-yl)phenyl]methyl}-1,6-dihydropyridazin-3-yl) benzonitrile (57 mg, 0.1 mmol) and 3-azidopiperidine-2,6-dione (17 mg, 0.1 mmol) in THF/H2O (5 mL/ 5 mL) at 25°C. The reactants were stirred for 2 hours. The reactants were poured into 50 mL water, extracted with EA (50 mL x2), Dried with Magnesium sulfate. The reaction mixture was loaded into silica and separated into MPLC (DCM/MeOH 0%-> 10%). MS (ESI, m/z): [M+H]⁺ = 588.4
1H NMR (500 MHz, DMSO-d6) δ ppm 11.14 (s, 1 H) 8.57 (s, 3 H) 8.31 (s, 3 H) 8.11 (d, J=9.77 Hz, 1 H) 7.87 (d, J=7.63 Hz, 1 H) 7.65 (t, J=7.93 Hz, 1 H) 7.37 - 7.46 (m, 3 H) 7.10 (d, J=9.77 Hz, 1 H) 5.72 (dd, J=12.36, 4.73 Hz, 1 H) 5.37 (s, 2 H) 3.98 (d, J=5.49 Hz, 3 H) 2.55 - 2.64 (m, 1 H) 2.01 - 2.08 (m, 1 H) 1.82 - 1.94 (m, 2 H) 1.71 (d, J=9.31 Hz, 1 H) 1.17 (s, 2 H)

### Example C-87: Preparation of 3-(1-(3-(5-(4-((1-(4-((1-((2,6-dioxopiperidin-3-yl)amino)-2-fluorophenyl)piperidine-4-il)methyl)piperazine-1-il)pyrimidine-2-yl)benzyl)6-oxo-1,6-dihydropyridazine-3-yl)benzonitrile (ICT-0001402)

### Step 1) Preparation of 3-(6-oxo-1-(3-(5-(4-(piperidine-4-ylmethyl)piperazine-1-yl)pyrimidine-2-yl)benzyl)-1,6-dihydropyridazine-3-yl)benzonitrile

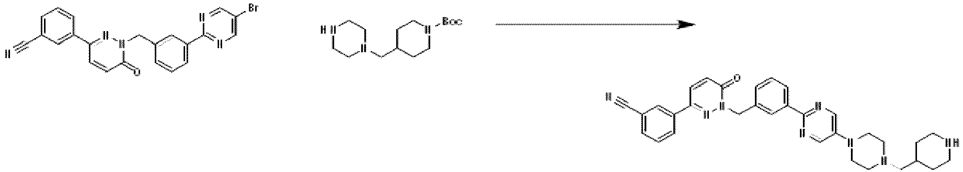

Tris (1,5-diphenylpenta-1,4-diene-3-one) dipalladium (144 mg, 158 µmol) and [5-(diphenylphosphony)9,9-dimethyl-9H-xanthenene-4-yl]diphenylphospan (91.2 mg, 158 µmol) were added to a solution of 3-(1,5-diphenylphosphony)-9,9-dimethyl-9H-xantene-4-yl]diphenylphospan (91.2 mg, 158 µmol) in a solution of toluene (10 ml), followed by K₂CO₃ (528 mg, 3.94 mmol). The reactants were stirred at 110°C for 5 hours. After cooling to room temperature, the reactants were concentrated under reduced pressure and purified by column chromatography to obtain BOC-protected compounds. The compound was treated with 4.0 M HCl in dioxane (5 mL) and stirred for 5 hours. The reactants were concentrated under reduced pressure, passed through a basic silica pad, and the subject compound was obtained (521 mg). MS (ESI, m/z): [M+H]⁺ = 547.3.

### Step 2) Preparation of 3-(1-(3-(5-(4-((1-(2-fluoro-4-nitrophenyl)piperidine-4-yl)methyl)piperazine-1-il)pyrimidine-2-yl)benzyl)-6-oxo-1,6-dihydropyridazine-3-yl)benzonitrile

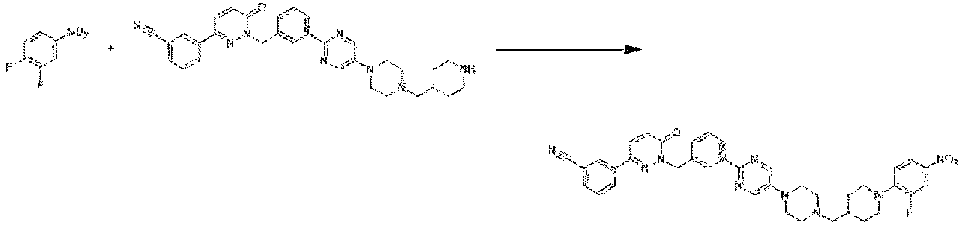

Ethylbis (propane-2-yl)amine (47.3 mg, 366 µmol) was added to a solution of 3-(6-oxo-1-{{5-{4-[(piperidine-4-yl)methyl]piperazine-1-yl}pyrimidine-2-yl)phenyl]methyl}-1,6-dihydropyridazin-3-yl)benzonitrile (100 mg, 183 µmol) and 1,2-difluoro-4-nitrobenzene (29.1 mg, 1839 µmol) in acetonitrile (2 ml). Ethylbis (propane-2-yl)amine (47.3 mg, 366 µmol) was added to a solution of 3-(6-oxo-1-{5-{4-[(piperidine-4-yl)methyl]piperazine-1-yl}pyridine-2-yl}pyridine-2-yl}, after cooling to room temperature, and purified by column chromatography to obtain the heading compound (110 mg). MS (ESI, m/z): [M+H]⁺ = 686.7.

### Step 3) Preparation of 3-(1-(3-(5-(4-((1-(4-amino-2-fluorophenyl)piperidine-4-yl)methyl)piperazine-1-il)pyrimidine-2-yl)benzyl)-6-oxo-1,6-dihydropyridazine-3-yl)benzonitrile

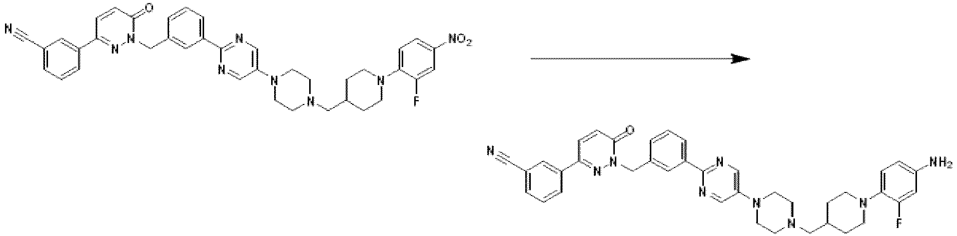

2 mL of saturated NH4Cl was added to a solution of 3-(1-(3-(5-(4-((1-(2-fluoro-4-nitrophenyl)piperidine-4-yl)methyl)piperazine-1-il)pyrimidine-2-yl)benzyl)-6-oxo-1,6-dihydropyridazine-3-yl) benzonitrile (100 mg, 146 µmol) and iron (40.7 mg, 729 µmol) in a solution. The reactants were stirred at 100°C for 2 hours. After cooling to room temperature, the reactants were filtered, the filtrate was poured into water, and extracted with ethyl acetate. The organic layer was dried with Magnesium sulfate. Concentrated under reduced pressure, purified by column chromatography, the title compound was obtained (79 mg). MS (ESI, m/z): [M+H]⁺ = 656.7.

### Step 4) Preparation of 3-(1-(3-(5-(4-((1-(4-(1-(4-((2,6-dioxopiperidin-3-yl)amino)-2-fluorophenyl)piperidine-4-yl)methyl)piperazine-1-il)pyrimidine-2-yl)benzyl)-6-oxo-1,6-dihydropyridazine-3-yl)benzonitrile (ICT-0001402)

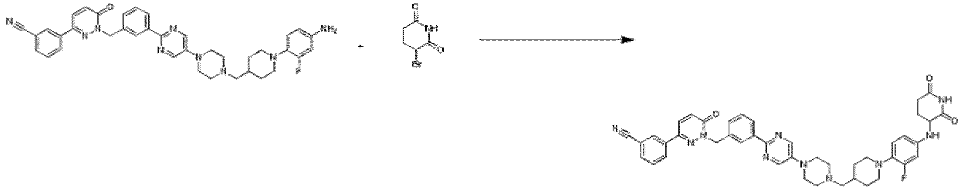

Ethylbis (propane-2-yl)amine (47.3 mg, 366 µmol) was added to 3-[1-({3-[5-(4-{[1-(2-fluoro-4-nitrophenyl)piperidine-4-yl]methyl}piperazine-1-yl)pyrimidin-2-yl]phenyl}methyl)-6-oxo-1,6-dihydropyridazine-3-yl]benzonitrile (100 mg, 146 µmol) and 3-bromomorphidine-2,6-dione solution in acetonitrile (2 ml). The reactants were stirred at 100°C for 2 hours. After cooling to room temperature, the reactants were concentrated under reduced pressure, purified by column chromatography. Subjective compound (110 mg) was obtained. MS (ESI, m/z): [M+H]⁺ = 767.9.
1H NMR (500 MHz, DMSO-d6) δ ppm 10.72 (s, 1 H) 8.52 (s, 3 H) 8.32 (s, 2 H) 8.28 (s, 1 H) 8.18 (d, J=8.09 Hz, 1 H) 8.09 - 8.14 (m, 3 H) 7.87 (d, J=7.78 Hz, 1 H) 7.65 (t, J=7.93 Hz, 1 H) 7.36 - 7.40 (m, 2 H) 7.10 (d, J=9.61 Hz, 1 H) 6.76 (t, J=9.31 Hz, 1 H) 6.40 - 6.48 (m, 1 H) 6.35 (d, J=8.70 Hz, 1 H) 5.72 (d, J=7.78 Hz, 1 H) 5.37 (s, 3 H) 4.15 - 4.23 (m, 1 H) 2.99 - 3.09 (m, 2 H) 2.58 - 2.73 (m, 1 H) 2.48 - 2.53 (m, 3 H) 2.17 (br. s., 1 H) 1.96 - 2.06 (m, 1 H) 1.82 - 1.92 (m, 1 H) 1.68 - 1.79 (m, 3 H) 1.15 - 1.25 (m, 3 H) 1.11 (t, J=7.17 Hz, 1 H)

### Example C-88: Preparation of 3-(1-(3-(5-((1-(1-((1-((1-(4-((2,6-dioxopiperidin-3-yl)amino)-2-fluorophenyl)azetidine-3-yl)methyl)piperidine-4-il)methoxy)pyrimidine-2-yl)benzyl)6-oxo-1,6-dihydropyridazine-3-yl)benzonitrile (ICT-0001425)

### Step 1) Preparation of 3-(1-(3-(5-((1-(azetidine-3-ylmethyl)piperidine-4-yl)methoxy)pyrimidine-2-yl)benzyl)-6-oxo-1,6-dihydropyridazine-3-yl)benzonitrile

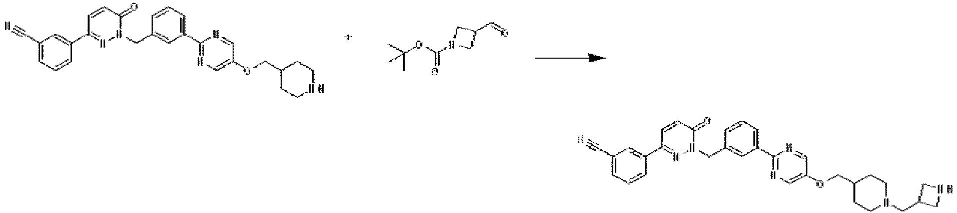

Bis(acetyloxy)boranuidil acetate (355 mg, 1.88 mmol) was added in small portions to 3-{6-oxo-1-[(piperidine-4-yl)methoxy]pyrimidin-2-yl}phenyl)methyl]-1,6-dihydropyridazine-3-yl} benzonitrile (300 mg, 625 µmol) and tert-butyl 3-formylazetidine-1-carboxylate (116 mg, 627 µmol) in DCM (5 ml) at room temperature. Bis(acetyloxy)boranudyl acetate (355 mg, 1.88 mmol) was added in small portions to 3-{6-oxo-1-methoxy]pyrimidin-2-yl}phenyl)methyl]-1,6-dihydropyridazin-3-benzonitrile (300 mg, 625 µmol) and tert-butyl 3-formylazetidine-1-carboxylate (116 mg, 627 µmol) in a solution at room temperature. The reactants were stirred for 6 hours. The reactants were neutralized with saturated NaHCO₃ to adjust the pH to ~8. The reactants were poured into the water. DCM, dried with Magnesium sulfate, and concentrated under reduced pressure. The residues were purified with MeOH/DCM (0~10%) MPLC, Boc-protected compound was obtained (421 mg), treated with 50% TFA in DCM (2 mL), and stirred for 5 hours. After the reaction was completed, the reactants were concentrated under reduced pressure and vacuum-dried. The residue was dissolved in DCM, and the heading compound was obtained by passing NH-DM 1020, a Chromatorex pad. MS (ESI, m/z): [M+H]⁺ = 548.6.

### Step 2) Preparation of 3-(1-(3-(5-((1-((1-(1-(1-(2-fluoro-4-nitrophenyl)azetidin-3-yl)methyl)piperidine-4-yl)methoxy)pyrimidine-2-yl)benzyl)6-oxo-1,6-dihydropyridazine-3-yl)benzonitrile

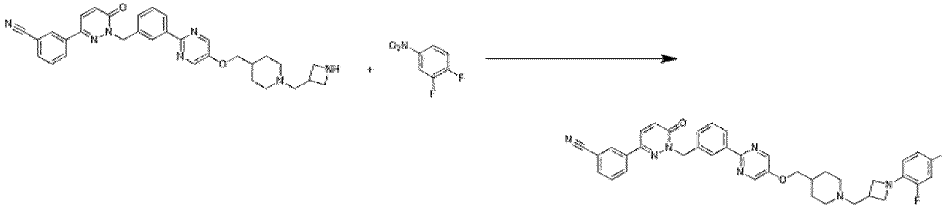

Ethylbis (propane-2-yl)amine was added to 3-(1-(3-(5-((1-(1-(azetidine-3-ylmethyl]piperidine-4-yl)methoxy)pyrimidine-2-yl)benzyl)-6-oxo-1,6-dihydropyridazin-3-yl]benzonitrile (200 mg, 365 µmol) and 1,2-difluoro-4-nitrobenzene (69.7 mg, 438 µmol) in a solution at room temperature. The reactants were warmed to 65°C and stirred for 3 hours. The reactants were transferred to a fractionating funnel, washed with water (50 ml), and extracted with ethyl acetate (50 ml x 2). The bound organic layer was dried and vacuum-concentrated with magnesium sulphate anhydrous. The residue was purified by column chromatography to obtain the compound (156 mg). MS (ESI, m/z): [M+H]⁺ = 687.3.

### Step 3) Preparation of 3-(1-(3-(5-((1-((1-(4-amino-2-fluorophenyl))azetidine-3-yl)methyl)piperidine-4-yl)methoxy)pyrimidine-2-yl)benzyl)6-oxo-1,6-dihydropyridazine-3-yl)benzonitrile

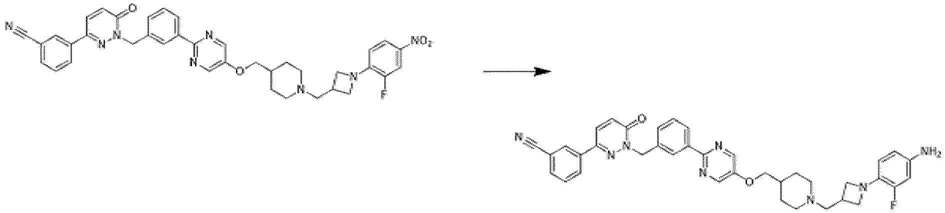

Saturated NH4Cl (3 mL) and iron (18.3 mg, 328 µmol) were added to a 3-(1-(5-((1-(1-(1-(2-fluoro-4-nitrophenyl)azetidine-3-yl)methyl)piperidine-4-yl)methoxy)pyrimidine-2-yl)benzyl)-6-oxo-1,6-dihydropyridazine-3-day} benzonitrile (45 mg, 328 µmol) in THF (3 mL) at room temperature. The reactants were warmed to 80°C and stirred for 3 hours. The organic layer was extracted with ethyl acetate (10 ml, twice). The bound organic layer was dried with magnesium sulfate anhydrous. Vacuum concentrated. The residue was purified by column chromatography to obtain the compound (39 mg). MS (ESI, m/z): [M+H]⁺ = 657.3.

### Step 4) Preparation of 3-(1-(3-(5-((1-((1-((1-(4-((2,6-dioxopiperidin-3-yl)amino)-2-fluorophenyl)azetidine-3-il)methyl)piperidine-4-il)methoxy)pyrimidine-2-yl)benzyl)6-oxo-1,6-dihydropyridazine-3-yl)benzonitrile (ICT-0001425)

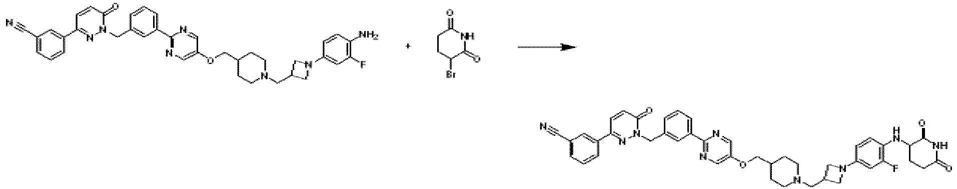

3-bromoperidin-2,6-dione was added to a solution of 3-{1-[(3-{5-[(1-{[1-(2-fluoro-4-nitrophenyl)azetidine-3-yl]methyl}piperidine-4-yl)methoxy]pyrimidine-2-yl}phenyl)methyl]-6-oxo-1,6-dihydropyridazine-3-yl}benzonitrile (45 mg, 68.5 µmol) and then sodium bicarbonate (11.5 mg, 137 µmol). The reactant was warmed to 80°C, stirred for 12 hours. The reactant was poured into water (20 mL). Extracted with ethyl acetate (20 mL x 2). The bound organic layer was dried with magnesium sulfate anhydrous and vacuum-concentrated. The residue was purified by column chromatography to obtain the compound (48 mg). MS (ESI, m/z): [M+H]⁺ = 768.9
1H NMR (500 MHz, DMSO-d6) δ ppm 10.77 (s, 1 H) 8.61 - 8.68 (m, 3 H) 8.39 (br. s., 2 H) 8.21 - 8.28 (m, 3 H) 8.18 (d, J=9.77 Hz, 1 H) 7.94 (d, J=7.78 Hz, 1 H) 7.72 (t, J=7.93 Hz, 1 H) 7.46 - 7.51 (m, 2 H) 7.17 (d, J=9.77 Hz, 1 H) 6.44 - 6.54 (m, 1 H) 6.32 - 6.44 (m, 2 H) 5.55 (d, J=7.48 Hz, 1 H) 5.45 (s, 2 H) 4.16 - 4.24 (m, 1 H) 4.05 (d, J=6.56 Hz, 2 H) 3.85 (t, J=6.64 Hz, 2 H) 2.86 (br. s., 2 H) 2.67 - 2.77 (m, 1 H) 2.54 - 2.63 (m, 2 H) 2.04 - 2.13 (m, 1 H) 1.88 - 1.99 (m, 2 H) 1.71 - 1.88 (m, 4 H) 1.32 (d, J=9.16 Hz, 2 H) 1.15 - 1.21 (m, 2 H)

### Example C-89: Preparation of 3-(1-(3-(5-((1-(1-(2-(4-((2,6-dioxopiperidine-3-yl)amino)-2-fluorophenyl)piperazine-1-il)ethyl)piperidine-4-il)methoxy)pyrimidine-2-yl)benzyl)6-oxo-1,6-dihydropyridazine-3-yl)benzonitrile (ICT-0001433)

### Step 1) Preparation of 3-(1-(3-(5-((1-(2-(4-(4-((2,6-dioxopiperidine-3-yl)amino)2-fluorophenyl)piperazine-1-il)ethyl)piperidine-4-il)methoxy)pyrimidine-2-yl)benzyl)6-oxo-1,6-dihydropyridazine-3-yl)benzonitrile (ICT-0001433)

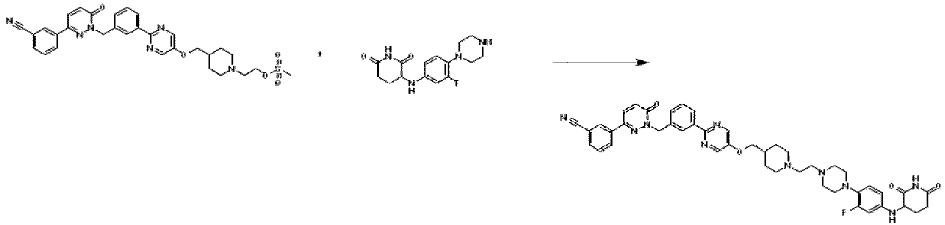

K2CO3 (51.3 mg, 3698 µmol) was added to 2-[4-({[2-(3-{3-(3-cyanophenyl)-6-oxo-1,6-dihydropyridazin-1-yl]methyl}phenyl)pyrimidine-5-yl]oxy}methyl)piperidine-1-yl]ethyl 4-methylbenzene-1-sulfonate (100 mg, 148 µmol) and 3-{[3-fluoro-4-(piperazine-1-yl)phenyl]amino}piperidine-2,6-dione (45.3 mg, 148 µmol) in THF (5 mL) at room temperature. The reactants were warmed to 80°C and stirred for 3 hours. The organic layer was extracted with ethyl acetate (10 ml, 2). The bound organic layer was dried with anhydrous magnesium sulfate and vacuum-concentrated. The residue was purified by column chromatography to obtain the compound (78 mg). MS (ESI, m/z): [M+H]⁺ = 811.9
1H NMR (500 MHz, DMSO-d6) δ ppm 10.79 (s, 1 H) 8.67 (s, 2 H) 8.39 (s, 3 H) 8.25 (t, J=7.78 Hz, 3 H) 8.19 (d, J=9.77 Hz, 1 H) 7.94 (d, J=7.78 Hz, 1 H) 7.73 (t, J=7.93 Hz, 1 H) 7.44 - 7.53 (m, 3 H) 7.17 (d, J=9.77 Hz, 1 H) 6.84 (t, J=9.31 Hz, 1 H) 6.52 (dd, J=14.95, 1.98 Hz, 1 H) 6.43 (d, J=8.54 Hz, 1 H) 5.85 (d, J=7.63 Hz, 1 H) 5.45 (s, 2 H) 4.23 - 4.32 (m, 1 H) 4.11 (d, J=5.49 Hz, 2 H) 3.14 - 3.24 (m, 2 H) 3.10 (d, J=7.48 Hz, 1 H) 2.90 (br. s., 5 H) 2.66 (br. s., 3 H) 2.08 (dt, J=12.74, 4.39 Hz, 2 H) 1.80 - 2.01 (m, 5 H) 1.58 (br. s., 2 H) 1.24 (s, 1 H) 1.15 - 1.21 (m, 1 H)

### Example C-90: Preparation of 3-(1-(1-(3-(5-(3-((4-(4-(((2,6-dioxopiperidin-3-yl)amino)-2-fluorophenyl)piperazine-1-yl)methyl)phenyl)pyrimidine-2-yl)benzyl)6-oxo-1,6-dihydropyridazine-3-yl)benzonitrile (ICT-0001434)

### Step 1) Preparation of 3-(1-(3-(5-(3-(3-(hydroxymethyl)phenyl)pyrimidine-2-yl)benzyl)-6-oxo-1,6-dihydropyridazine-3-yl)benzonitrile

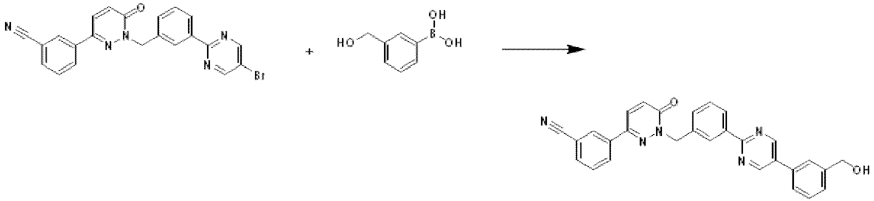

3-(1-{[3-(5-bromofirimidine-2-yl)phenyl]methyl}-6-oxo-1,6-dihydropyridazine-3-yl) benzonitrile (300 mg, 675 umol) and [3-(hydroxymethyl)phenyl]boronic acid (103 mg, 675 umol) were dissolved in dioxane (10 ml). Pd(PPh₃)₄ (39 mg, 33.8 umol) and 2M K₂CO₃ (233 mg, 1.69 mmol) were added to this solution. The reaction mixture was stirred in the MW reactor at 120°C for 2 hours. The mixture was transferred to a fractionating funnel, washed with water (50 ml), and extracted with ethyl acetate (50 ml, twice). The bound organic layer was dried with magnesium sulphate anhydrous and vacuum-concentrated. The residue was purified by column chromatography to obtain the compound. MS (ESI, m/z): [M+H]⁺ = 472.6.

### Step 2) Preparation of 3-(2-(3-((3-(3-(3-cyanophenyl)-6-oxopyridazine-1(6H)-yl)methyl)phenyl)pyrimidine-5-yl)benzyl methanesulfonate

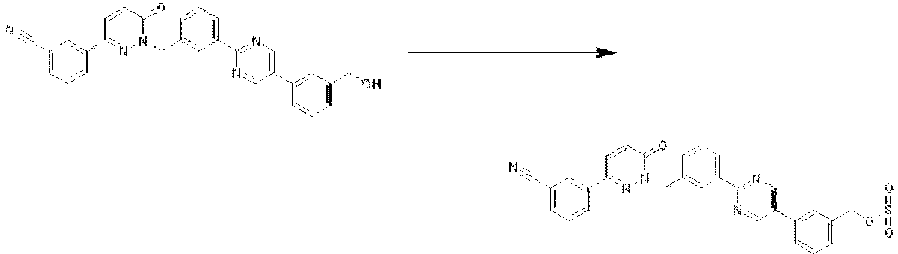

Methanesulfonyl chloride (107 mg, 931 umol) was added to a solution of 3-{1-[(3-{5-[3-(hydroxymethyl)phenyl]pyrimidin-2-yl}phenyl)methyl]-6-oxo-1,6-dihydropyridazin-3-yl}benzonitrile (366 mg, 776 umol) in DCM (10 mL) at 0°C. The reactants were warmed to room temperature and stirred for 1 hour. The reactants were transferred to a fractionating funnel, washed with water (50 ml), and extracted with ethyl acetate (50 ml, twice). The bound organic layer was dried with magnesium sulfate anhydrous. Vacuum concentrated. The residue was purified by column chromatography to obtain the compound (398 mg). MS (ESI, m/z): [M+H]⁺ = 571.0.

### Step 3) Preparation of 3-(1-(3-(5-(3-(3-((4-(4-((2,6-dioxopiperidin-3-yl)amino)2-fluorophenyl)piperazine-1-yl)methyl)phenyl)pyrimidine-2-yl)benzyl)6-oxo-1,6-dihydropyridazine-3-yl)benzonitrile (ICT-0001434)

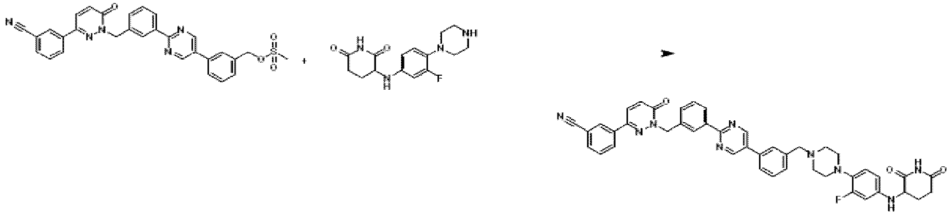

Triethylamine was added to a solution of {3-[2-(3-{[3-(3-cyanophenyl)-6-oxo-1,6-dihydropyridazin-1-yl]methyl}phenyl}phenyl)pyrimidine-5-yl]methylsulfurochloridate (45 mg, 78.9 µmol) and 3-{[3-fluoro-4-(piperazine-1-yl)phenyl]amino}piperidine-2,6-dione (24.2 mg, 78.9 µmol) in DCM (10 mL) at room temperature. The reactant was warmed to 45°C and stirred for 3 hours. The reactant was transferred to a fractionating funnel, washed with water (50 ml). DCM (50 ml, 2 times). The bound organic layer was dried with magnesium sulfate anhydrous and vacuum-concentrated. The residue was purified by column chromatography to obtain the compound (42 mg). MS (ESI, m/z): [M+H]⁺ = 760.8
1H NMR (500 MHz, DMSO-d6) δ ppm 10.79 (s, 1 H) 9.20 - 9.27 (m, 2 H) 8.54 (s, 1 H) 8.36 - 8.41 (m, 2 H) 8.26 (d, J=8.09 Hz, 1 H) 8.19 (d, J=9.77 Hz, 1 H) 7.92 - 7.97 (m, 2 H) 7.71 - 7.78 (m, 2 H) 7.59 - 7.65 (m, 1 H) 7.51 - 7.58 (m, 3 H) 7.45 - 7.47 (m, 1 H) 7.18 (d, J=9.77 Hz, 1 H) 6.51 (d, J=14.95 Hz, 1 H) 6.42 (d, J=8.70 Hz, 1 H) 5.81 (d, J=7.63 Hz, 1 H) 5.44 - 5.51 (m, 2 H) 4.21 - 4.30 (m, 1 H) 3.63 (s, 1 H) 2.89 (s, 3 H) 2.68 - 2.78 (m, 4 H) 2.54 - 2.60 (m, 3 H) 2.04 - 2.12 (m, 1 H) 1.85 (dd, J=12.13, 4.20 Hz, 1 H)

### Example C-91: Preparation of 3-(1-(3-(5-((1-(1-(2-(4-((2,6-dioxopiperidine-3-yl)amino)-3-fluorophenyl)piperazine-1-yl)-2-oxoethyl)piperidine-4-yl)methoxy)pyrimidine-2-yl)benzyl)6-oxo-1,6-dihydropyridazine-3-yl)benzonitrile (ICT-0001437)

### Step 1) Preparation of 3-(1-(3-(5-((1-(2-(4-(4-((2,6-dioxopiperidine-3-yl)amino)3-fluorophenyl)piperazine-1-il)-2-oxoethyl)piperidine-4-il)methoxy)pyrimidine-2-yl)benzyl)6-oxo-1,6-dihydropyridazine-3-yl)benzonitrile (ICT-0001437)

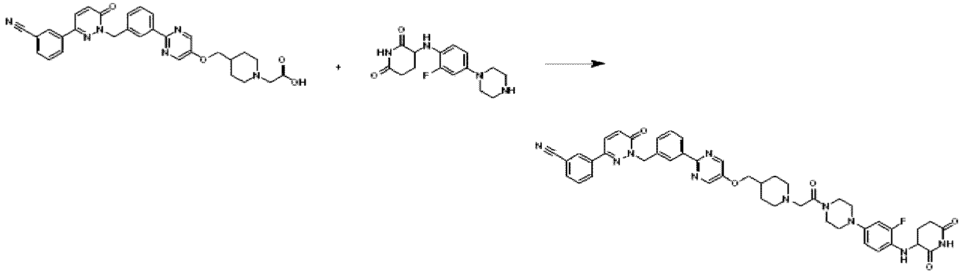

HATU (70.9 mg, 186 µmol) is added to 2-[4-({[2-(3-{3-(3-cyanophenyl)-6-oxo-1,6-dihydropyridazin-1-yl]methyl}phenyl)pyrimidine-5-yl]oxy}methyl)piperidine-1-yl]acetic acid (100 mg, 186 µmol) and 3-{[2-fluoro-4-(piperazine-1-yl)phenyl]amino}piperidine-2,6-dione (57.1 mg, 186 µmol) in DMF (1 mL) at room temperature, followed by ethylbis (propane-2-yl)amine (24.1 mg, 186 µmol). The reactants were stirred for 6 hours. The reactants were poured into water, extracted with ethyl acetate, dried with Magnesium sulfate, and concentrated under reduced pressure. The residue was purified with MeOH/DCM (0~10%) MPLC to obtain the title compound (130 mg). MS (ESI, m/z): [M+H]⁺ = 825.9
1H NMR (500 MHz, DMSO-d6) δ ppm 10.79 (s, 1 H) 8.62 - 8.67 (m, 2 H) 8.47 (d, J=3.81 Hz, 2 H) 8.38 (br. s., 1 H) 8.27 (d, J=8.55 Hz, 1 H) 8.23 (d, J=6.41 Hz, 1 H) 8.18 (d, J=9.77 Hz, 1 H) 7.91 - 7.97 (m, 2 H) 7.72 (t, J=7.86 Hz, 1 H) 7.47 - 7.51 (m, 2 H) 7.29 (dd, J=8.39, 4.27 Hz, 1 H) 7.16 (d, J=9.77 Hz, 1 H) 6.84 (t, J=9.31 Hz, 1 H) 6.50 - 6.56 (m, 1 H) 6.43 (d, J=8.85 Hz, 1 H) 5.89 (d, J=7.63 Hz, 1 H) 5.45 (s, 2 H) 4.21 - 4.32 (m, 1 H) 4.06 (d, J=5.95 Hz, 2 H) 3.65 (br. s., 4 H) 2.93 - 3.00 (m, 2 H) 2.87 (br. s., 2 H) 2.75 - 2.83 (m, 2 H) 2.53 - 2.62 (m, 1 H) 2.18 (br. s., 2 H) 2.03 - 2.11 (m, 1 H) 1.77 - 2.00 (m, 4 H) 1.33 - 1.41 (m, 2 H)

### Example C-92: Preparation of 3-(1-{[3-(5-{[1-(2-{4-[(4-{4-[(2,6-dioxopiperidin-3-yl)amino]-2-fluorophenyl}piperazine-1-yl)methyl]piperidine-1-yl}-2-oxoethyl)piperidine-4-yl]methoxy}pyrimidine-2-yl)phenyl]methyl}-6-oxo-1,6-dihydropyridazine-3-yl)benzonitrile (ICT-0001447)

### Step 1) Preparation of tert-butyl 4-{[4-(2-fluoro-4-nitrophenyl)piperazine-1-yl]methyl}piperidine-1-carboxylate

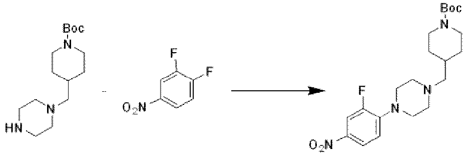

tert-butyl 4-[(piperazine-1-yl)methyl]piperidine-1-carboxylate (1.2 g, 4.2 mmol) and potassium carbonate (2.9 g, 21.2 mmol) were added to a solution of 1,2-difluoro-4-nitrobenzene (0.7 g, 4.2 mmol) in DMF (1 mL). The reactants were stirred at 80°C for 3 hours. After the end of the reaction, the reactants were poured into water, extracted with ethyl acetate (twice), dried with Magnesium sulfate, and concentrated under reduced pressure. The residue was purified by hexane/ethyl acetate (0-60%) column chromatography to obtain the title compound (1 g). MS (ESI, m/z): [M+H]+ = 423.5.

### Step 2) Preparation of tert-butyl 4-{[4-(4-amino-2-fluorophenyl)piperazine-1-yl]methyl}piperidine-1-carboxylate

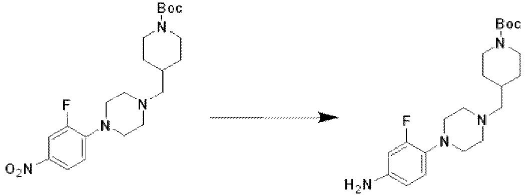

Iron powder (1.3 g, 23.7 mmol) was added to tert-butyl 4-{[4-(2-fluoro-4-nitrophenyl)piperazine-1-yl]methyl}piperidine-1-carboxylate (1 g, 2.4 mmol) in saturated NH4Cl/THF (1:1) (40 mL). The reactants were stirred at 80°C for 4 hours. After the reaction was completed, the reactants were filtered and concentrated under reduced pressure. The residue was purified by hexane/ethyl acetate (0-100%) column chromatography to obtain the title compound (0.82 g). MS (ESI, m/z): [M+H]+ = 393.5.

### Step 3) Preparation of tert-butyl 4-[(1-{4-[(2,6-dioxopiperidine-3-yl)amino]-2-fluorophenyl}piperidine-4-yl)methyl]piperazine-1-carboxylate

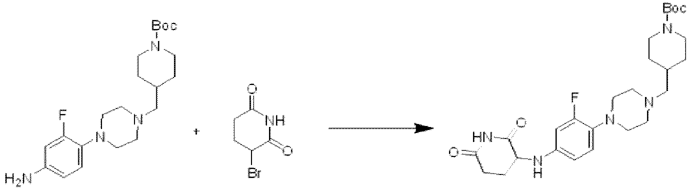

3-bromoperidin-2,6-dione (0.8 g, 4.2 mmol) and sodium bicarbonate (1.1 g, 12.5 mmol) were added to tert-butyl 4-{[1-(4-amino-2-fluorophenyl)piperidine-4-yl]methyl}piperazine-1-carboxylate (0.8 g, 2.1 mmol) in DMF (1.5 mL). The reactants were stirred at 85°C for 3 hours. After the end of the reaction, the reactants were poured into water, extracted with ethyl acetate (twice), dried with Magnesium sulfate, and concentrated under reduced pressure. The residue was purified by column chromatography to obtain a hexane/ethyl acetate (0~100%) heading compound (1 g). MS (ESI, m/z): [M+H]+ = 504.6.

### Step 4) 3-[(3-fluoro-4-{4-[(piperazine-1-yl)methyl]piperidine-1-yl}phenyl)amino]piperidine-2,6-dione

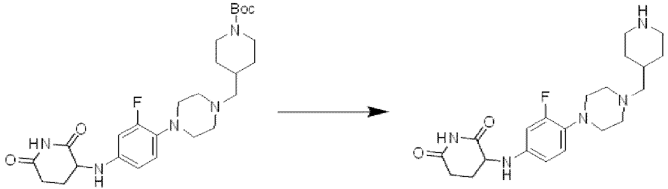

The reactants were stirred at room temperature for 30 min in a solution of tert-butyl 4-[(1-{4-[(2,6-dioxopiperidine-3-yl)amino]-2-fluorophenyl}piperidine-4-yl)methyl]piperazine-1-carboxylate (0.7 g, 1.3 mmol) in 30% TFA in DCM (3 mL). After the reaction was completed, it was concentrated under reduced pressure. The preparations are used in the next step without additional purification (0.9 g) ms (ESI, m/z): [M+H]+ = 404.5.

### Step 5) Preparation of 3-(1-{[3-(5-{[1-(2-{4-[(4-{4-[(2,6-dioxopiperidine-3-yl)amino]-2-fluorophenyl}piperazine-1-yl)methyl]piperidine-1-il}-2-oxoethyl)piperidine-4-il]methoxy}pyrimidine-2-yl)phenyl]methyl}6-oxo-1,6-dihydropyridazine-3-yl)benzonitrile (ICT-0001447)

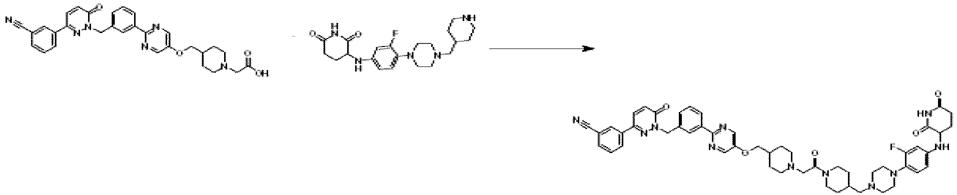

DMF (1. 5 mL) of 2-[4-({[2-(3-{3-{3-(3-cyanophenyl)-6-oxo-1,6-dihydropyridazin-1-yl]methyl}phenyl)pyrimidine-5-yl]oxy}methyl)piperidine-1-yl]acetic acid (75.3 mg, 140 µmol) in a solution of [(dimethylamino)({3H-[1,2,3]triazolo[4,5-b]pyridine-3-yloxy})methylidene]dimethylazanium (66 mg, 281 µmol), 3-[(3-fluoro-4-{4-[(piperidine-4-yl)methyl]piperazine-1-yl}phenyl)amino]piperidine-2,6-dione (56.6 mg, 140 µmol) and ethylbis (propane-2-yl)amine (181 mg, 140 µmol). The reactants were stirred at room temperature for 2 hours. After the reaction was finished, the reactants were poured into water, extracted with DCM (twice), dried with Magnesium sulfate, and concentrated under reduced pressure. The residue was purified by DCM/MeOH (0~5%) column chromatography to obtain the title compound (82 mg). MS (ESI, m/z): [M+H]+ = 923.1
1H NMR (500 MHz, DMSO-d6) δ ppm 10.79 (s, 1 H) 9.33 (br. s., 1 H) 8.63 - 8.68 (m, 2 H) 8.36 - 8.42 (m, 2 H) 8.21 - 8.27 (m, 2 H) 8.18 (d, J=9.77 Hz, 1 H) 7.94 (d, J=7.78 Hz, 1 H) 7.72 (t, J=7.86 Hz, 1 H) 7.47 - 7.51 (m, 2 H) 7.17 (d, J=9.77 Hz, 1 H) 6.83 (t, J=9.38 Hz, 1 H) 6.49 - 6.60 (m, 1 H) 6.28 - 6.48 (m, 1 H) 5.86 (d, J=7.48 Hz, 1 H) 5.45 (s, 2 H) 4.34 (d, J=12.97 Hz, 1 H) 4.23 - 4.30 (m, 1 H) 4.08 (br. s., 2 H) 3.59 (dt, J=13.08, 6.50 Hz, 4 H) 3.11 (q, J=7.32 Hz, 4 H) 2.92 - 2.96 (m, 1 H) 2.86 - 2.92 (m, 3 H) 2.68 - 2.78 (m, 2 H) 2.54 - 2.66 (m, 4 H) 2.06 - 2.11 (m, 1 H) 1.83 - 1.99 (m, 6 H) 1.75 - 1.81 (m, 2 H) 1.46 - 1.57 (m, 2 H) 1.39 (s, 1 H) 1.10 (d, J=11.29 Hz, 1 H) 0.95 (d, J=9.77 Hz, 1 H)

### Example C-93: Preparation of 3-(1-(1-(3-(5-(3-((4-(5-(2,6-dioxopiperidine-3-yl)pyridine-2-yl)piperazine-1-il)methyl)phenyl)pyrimidine-2-yl)benzyl)6-oxo-1,6-dihydropyridazine-3-yl)benzonitrile (ICT-0001449)

### Step 1) Preparation of 3-(1-(3-(5-(3-(3-((4-(5-(2,6-dioxopiperidine-3-yl)pyridine-2-yl)piperazine-1-il)methyl)phenyl)pyrimidine-2-yl)benzyl)6-oxo-1,6-dihydropyridazine-3-yl)benzonitrile (ICT-0001449)

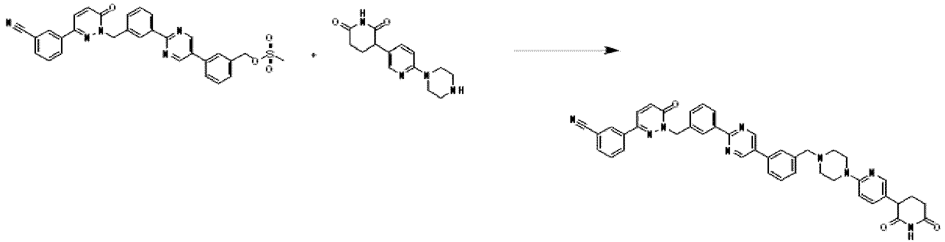

Triethylamine (12.0 mg, 118 µmol). The reactants were warmed to 65°C and stirred for 8 hours. The reactants were transferred to the fractionating funnel, washed with water (50 ml), and extracted with DCM (50 ml, twice). The bound organic layer was dried with anhydrous magnesium sulfate and vacuum-concentrated. The residues were purified by column chromatography to obtain the compound (42.3 mmol). MS (ESI, m/z): [M+H]⁺ = 728.8
1H NMR (500 MHz, DMSO-d6) δ ppm 10.82 (s, 1 H) 9.19 - 9.27 (m, 2 H) 8.54 (s, 1 H) 8.35 - 8.43 (m, 2 H) 8.26 (d, J=7.63 Hz, 1 H) 8.15 - 8.22 (m, 1 H) 7.90 - 7.98 (m, 2 H) 7.69 - 7.81 (m, 4 H) 7.45 - 7.63 (m, 4 H) 7.37 - 7.44 (m, 1 H) 7.15 - 7.21 (m, 1 H) 6.79 (d, J=8.85 Hz, 1 H) 5.49 (s, 2 H) 3.74 (dd, J=12.13, 4.81 Hz, 1 H) 3.63 (s, 1 H) 3.44 - 3.54 (m, 3 H) 2.90 (s, 1 H) 2.62 - 2.76 (m, 1 H) 2.11 - 2.24 (m, 1 H) 1.88 - 2.01 (m, 1 H)

### Example C-94: Preparation of 3-(1-(3-(5-(3-(3-((4-((4-(4-((4-((2,6-dioxopiperidin-3-yl)amino)-2-fluorophenyl)piperazine-1-il)methyl)piperidine-1-il)methyl)piperidine-1-il)methyl)phenyl)pyrimidine-2-yl)benzyl)-6-oxo-1,6-dihydropyridazine-3-yl)benzonitrile (ICT-0001450)

### Step 1) Preparation of 3-(1-(3-(5-(3-(3-((4-((4-(4-((2,6-dioxopiperidin-3-yl)amino)2-fluorophenyl)piperazine-1-il)methyl)piperidine-1-il)methyl)pyrimidine-2-yl)benzyl)6-oxo-1,6-dihydropyridazine-3-yl)benzonitrile (ICT-0001450)

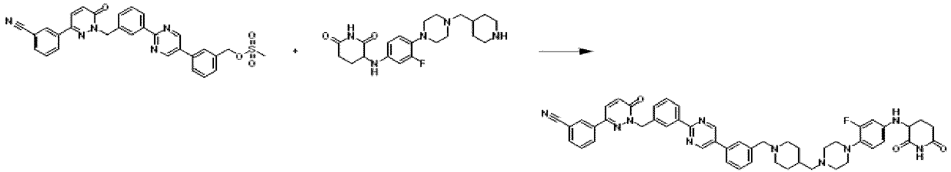

Triethylamine was added to a solution of {3-[2-(3-{[3-(3-cyanophenyl)-6-oxo-1,6-dihydropyridazin-1-yl]methyl}phenyl)pyrimidine-5-yl}methyl sulfurochloridate 3-(2-(3-((3-(3-(3-(3-cyanophenyl)-6-oxopyridazin-1(6H)-yl)methyl)phenyl)pyrimidine-5-yl)benzyl methanesulfonate (90 mg, 158 µmol) and 3-{[3-fluoro-4-(piperazine-1-yl)phenyl]amino}piperidine-2,6-dione (63.7 mg, 158 µmol) in a solution of triethylamine. They stirred for three hours. The reactants were transferred to the fractionating funnel, washed with water (50 ml), and extracted with DCM (50 ml, twice). The bound organic layer was dried with anhydrous magnesium sulfate and vacuum-concentrated. The residue was purified by column chromatography to obtain the compound (105 mg). MS (ESI, m/z): [M+H]⁺ = 858.0

1H NMR (500 MHz, DMSO-d6) δ ppm 10.79 (s, 1 H) 9.24 (s, 2 H) 8.54 (s, 1 H) 8.36 - 8.41 (m, 2 H) 8.26 (d, J=8.09 z, 1 H) 8.19 (d, J=9.77 Hz, 1 H) 7.94 (d, J=7.78 Hz, 1 H) 7.70 - 7.77 (m, 2 H) 7.50 - 7.61 (m, 3 H) 7.44 (br. s., 1 H) 7.18 (d, J=9.77 Hz, 1 H) 6.82 (t, J=9.31 z, 1 H) 6.51 (dd, J=15.11, 2.14 Hz, 1 H) 6.42 (d, J=8.70 Hz, 1 H) 5.81 (d, J=7.63 Hz, 1 H) 5.49 (s, 2 H) 4.25 (ddd, J=11.56, 7.21, 4.88 Hz, 1 H) 3.52 - 3.65 (m, 2 H) 2.89 (br. s., 1 H) 2.84 (br. s., 4 H) 2.68 - 2.79 (m, 2 H) 2.57 - 2.61 (m, 1 H) 2.55 (t, J=3.81 Hz, 1 H) 2.45 (br. s., 2 H) 2.17 (br. s., 2 H) 2.05 - 2.12 (m, 2H) 1.99 (s, 1 H) 1.80 - 1.92 (m, 2 H) 1.76 (dt, J=6.45, 3.26 Hz, 1 H) 1.53 (br. s., 1 H) 1.15 - 1.24 (m, 2 H).

### Example C-95: Preparation of 3-(1-{[3-(5-{[1-(2-{4-[(1-{4-[(2,6-dioxopiperidin-3-yl)amino]-2-fluorophenyl}piperidine-4-yl)methyl]piperazine-1-yl}-2-oxoethyl)piperidine-4-yl]methoxy}pyrimidine-2-yl)phenyl]methyl}-6-oxo-1,6-dihydropyridazine-3-yl)benzonitrile (ICT-0001468)

### Step 1) Preparation of benzyl 4-(hydroxymethyl)piperidine-1-carboxylate

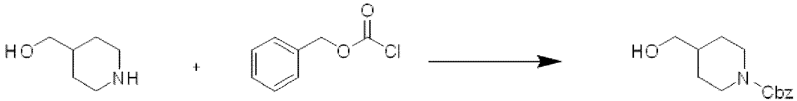

Triethylamine (13.2 g, 130 mmol) was added to the (piperidine-4-yl) methanol (5 g, 43.4 mmol) solution in DCM (100 mL). Benzyl carbonochloridate (11.1 g, 65.1 mmol) was added to the reaction mixture, and stirred for 3 hours at room temperature. After the reaction was completed, the reactants were poured into water, extracted with DCM (twice), dried with Magnesium sulfate, and concentrated under reduced pressure. The residue was purified with hexane/ethyl acetate (0-30%) column chromatography to obtain the title compound (5.4 g). MS (ESI, m/z): [M+H]+ = 250.3.

### Step 2) Preparation of benzyl 4-[(methanesulfonyoxy)methyl]piperidine-1-carboxylate

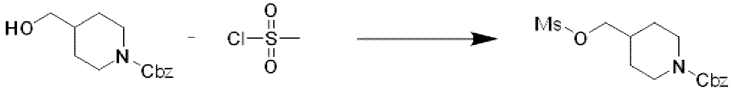

Triethylamine (4.4 g, 43.3 mmol) was added to benzyl 4-(hydroxymethyl)piperidine-1-carboxylate (5.4 g, 21.7 mmol) solution in DCM (70 mL) at room temperature and stirred for 20 min. In the reaction mixture, methanesulfonyl chloride diluted in 30 mL DCM (3 g, 26 mmol) was added dropwise. The reaction mixture was stirred for 30 minutes. After the reaction was completed, the reactants were poured into water, extracted with DCM (twice), dried with Magnesium sulfate, and concentrated under reduced pressure. The crude substance was used in the next step without additional tablets (7 g). MS (ESI, m/z): [M+H]+ = 328.4.

### Step 3) Preparation of tert-butyl 4-({1-[(benzyloxy)carbonyl]piperidine-4-yl}methyl) piperazine-1-carboxylate

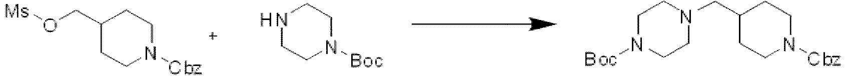

Tert-butyl piperazine-1-carboxylate (4 g, 21.4 mmol) was added to benzyl 4-[(methanesulfonyoxy)methyl]piperidine-1-carboxylate (7 g, 21.4 mmol) solution in DMF (20 mL). Dipotassium carbonate (14.8 g, 107 mmol) and sodium iodide (0.16 g, 1.07 mmol) were added to the reaction mixture. The reaction mixture was stirred at 95°C overnight. After the end of the reaction, the reactants were poured into water, extracted with ethyl acetate (twice), dried with Magnesium sulfate, and concentrated under reduced pressure. The crude substance was used in the next step without additional purification (9 g). MS (ESI, m/z): [M+H]+ = 418.6.

### Step 4) Preparation of tert-butyl 4-[(piperidine-4-yl)methyl]piperazine-1-carboxylate

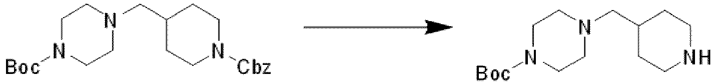

Pd/C (10 wt%) was added to tert-butyl 4-({1-[(benzyloxy)carbonyl]piperidine-4-yl}methyl)piperazine-1-carboxylate (9 g, 21.4 mmol) solution in ethanol (200 mL). The reactants were stirred overnight at room temperature under H₂ gas. After the reaction was completed, the reactants were filtered and concentrated under reduced pressure. The preparation substance is used in the next step without additional tablets (6 g). MS (ESI, m/z): [M+H]+ = 284.4.

### Step 5) Preparation of tert-butyl 4-{[1-(2-fluoro-4-nitrophenyl)piperidine-4-yl]methyl}piperazine-1-carboxylate

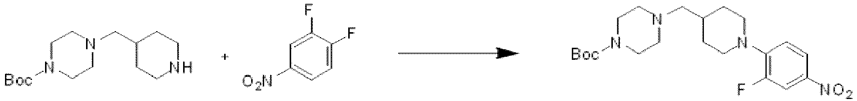

Tert-butyl 4-[(piperidine-4-yl)methyl]piperazine-1-carboxylate (6 g, 21.2 mmol) and dipotassium carbonate (14.6 g, 106 mmol) were added to a solution of 1,2-difluoro-4-nitrobenzene (3.37 g, 21.2 mmol) in DMF (15 mL). The reactants were stirred at 80°C for 3 hours. After the end of the reaction, the reactants were poured into water, extracted with ethyl acetate (twice), dried with Magnesium sulfate, and concentrated under reduced pressure. The residue was purified by hexane/ethyl acetate (0∼60%) column chromatography to obtain the title compound (7 g). MS (ESI, m/z): [M+H]+ = 423.5.

### Step 6) Preparation of tert-butyl 4-{[1-(4-amino-2-fluorophenyl)piperidine-4-yl]methyl}piperazine-1-carboxylate

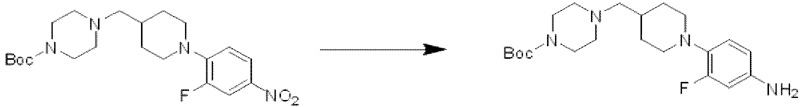

Iron powder (29.1 g, 521 mmol) was added to tert-butyl 4-{[4-(2-fluoro-4-nitrophenyl)piperazine-1-yl]methyl}piperidine-1-carboxylate (11 g, 26 mmol) in saturated NH4Cl/THF (1:1) (400 mL). The reactants were stirred at 80°C for 4 hours. After the reaction was completed, the reactants were filtered and concentrated under reduced pressure. The residue was purified by hexane/ethyl acetate (0∼100%) column chromatography to obtain the title compound (0.82 g). (ESI, m/z): [M+H]+ = 393.5.

### Step 7) Preparation of tert-butyl 4-[(1-{4-[(2,6-dioxopiperidin-3-yl)amino]-2-fluorophenyl}piperidine-4-yl)methyl]piperazine-1-carboxylate

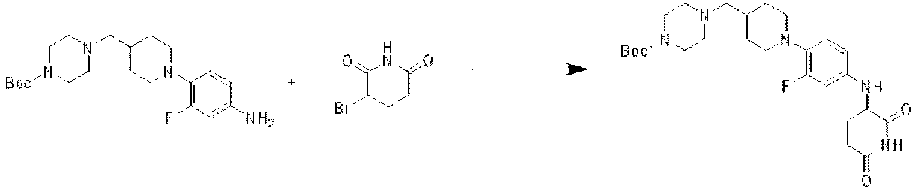

Tert-butyl 4-{[1-(4-amino-2-fluorophenyl)piperidine-4-yl]methyl}piperazine-1-carboxylate (1.2 g, 3.1 mmol), 3-bromoperiperidin-2,6-dione (1.2 g, 6.1 mmol) and sodium bicarbonate (1.28 g, 15.3 mmol) were added to the solution in DMF (1.5 mL). The reactants were stirred at 85°C for 6 hours. After the end of the reaction, the reactants were poured into water, extracted with ethyl acetate (twice), dried with Magnesium sulfate, and concentrated under reduced pressure. The residue was purified by hexane/ethyl acetate (0∼100%) column chromatography to obtain the title compound (1.3 g). MS (ESI, m/z): [M+H]+ = 504.6.

### Step 8) Preparation of 3-[(3-fluoro-4-{4-[(piperidine-4-yl)methyl]piperazine-1-yl}phenyl)amino]piperidine-2,6-dione

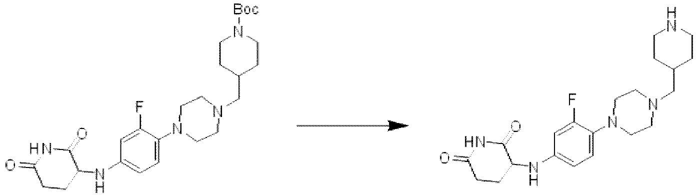

The reactants were stirred at room temperature for 1 hr in a solution of tert-butyl 4-[(4-{4-[(2,6-dioxopiperidine-3-yl)amino]-2-fluorophenyl}piperazine-1-yl)methyl]piperidine-1-carboxylate (1 g, 2 mmol) in 30% TFA in DCM (5 mL). After the end of the reaction, the reactants were poured into water, extracted with ethyl acetate (twice), dried with Magnesium sulfate, and concentrated under reduced pressure. The residue was purified by DCM/MeOH (5∼10%) amine column chromatography to obtain the title compound (0.5 g). MS (ESI, m/z): [M+H]+ = 404.5.

### Step 9) Preparation of 3-(1-{[3-(5-{[1-(2-{4-[(1-{4-[(2,6-dioxopiperidine-3-yl)amino]-2-fluorophenyl}piperidine-4-yl)methyl]piperazine-1-yl}-2-oxoethyl)piperidine-4-yl]methoxy}pyrimidine-2-yl)phenyl]methyl}-6-oxo-1,6-dihydropyridazine-3-yl)benzonitrile (ICT-0001468)

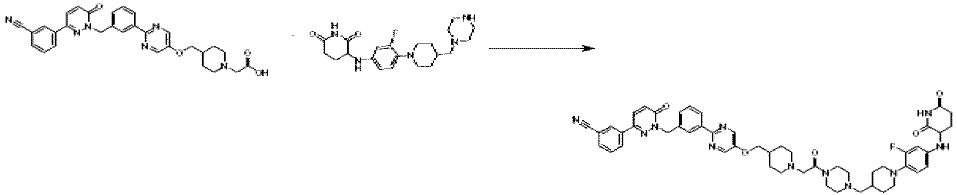

DMF (1. 5 mL) of [(dimethylamino)({[2-(3-{3-{3-(3-cyanophenyl)-6-oxo-1,6-dihydropyridazin-1-yl]methyl}phenyl)pyrimidine-5-yl]oxy}methyl)piperidine-1-yl]acetic acid (75.3 mg, 140 µmol) in a solution of [(dimethylamino)({3H-[1,2,3]triazolo[4,5-b]pyridine-3-yloxy})methylidene]dimethylazanium (66 mg, 281 µmol), 3-[(3-fluoro-4-{4-[(piperazine-1-yl)methyl]piperidine-1-yl}phenyl)amino]piperidine-2,6-dione (56.6 mg, 140 µmol) and ethylbis (propane-2-yl)amine (181 mg, 140 µmol). The reactants were stirred at room temperature for 2 hours. After the reaction was completed, the reactants were poured into water, extracted with DCM (twice), dried with Magnesium sulfate, and concentrated under reduced pressure. The residue was purified by DCM/MeOH (0~5%) column chromatography to obtain the title compound (82 mg). MS (ESI, m/z): [M+H]+ = 923.1
1H NMR (500 MHz, DMSO-d6) δ ppm 10.79 (s, 1 H) 8.58 - 8.63 (m, 2 H) 8.44 - 8.53 (m, 1 H) 8.32 (s, 2 H) 8.18 (t, J=7.93 Hz, 2 H) 8.12 (d, J=9.77 Hz, 1 H) 7.87 (d, J=7.48 Hz, 1 H) 7.66 (t, J=7.93 Hz, 1 H) 7.39 - 7.46 (m, 2 H) 7.10 (d, J=9.77 Hz, 1 H) 6.69 - 6.83 (m, 1 H) 6.40 - 6.49 (m, 1 H) 6.35 (d, J=7.78 Hz, 1 H) 5.69 - 5.81 (m, 1 H) 5.38 (s, 2 H) 4.72 (br. s., 1 H) 4.16 - 4.24 (m, 2 H) 4.03 (d, J=4.73 Hz, 2 H) 3.41 - 3.59 (m, 7 H) 3.01 - 3.13 (m, 5 H) 2.95 - 2.99 (m, 1 H) 2.83 - 2.90 (m, 1 H) 2.62 - 2.71 (m, 1 H) 2.54 - 2.60 (m, 1 H) 2.50 (dd, J=18.23, 3.89 Hz, 3 H) 2.29 (br. s., 1 H) 2.10 - 2.16 (m, 1 H) 10.72 (s, 1 H) 1.98 - 2.06 (m, 2 H) 1.83 - 1.95 (m, 6 H) 1.77 - 1.81 (m, 1 H)

### Example C-96: Preparation of 3-(1-(3-(5-((1-(2-(4-((2,6-dioxopiperidine-3-yl)amino)-2-fluorophenyl)piperazine-1-il)acetyl)piperidine-4-il)methoxy)pyrimidine-2-yl)benzyl)6-oxo-1,6-dihydropyridazine-3-yl)benzonitrile (ICT-0001494)

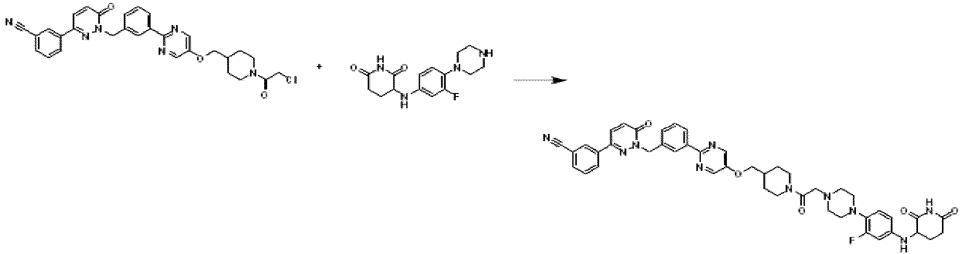

Ethylbis (propane-2-yl)amine (69.9 mg, 541 µmol) was added to 3-(1-{[3-(5-{[1-(2-chloroacetyl)piperidine-4-yl]methoxy}pyrimidin-2-yl)phenyl]methyl}-6-oxo-1,6-dihydropyridazine-3-yl)benzonitrile (100 mg, 180 µmol) and 3-{[2-fluoro-4-(piperazine-1-yl)phenyl]amino}piperidine-2,6-dione (55.2 mg, 180 µmol) in acetonitrile (10 mL) at room temperature. The reactant was warmed to 40°C and stirred for 3 hours. The reactants were transferred to the fractionation funnel. Washed with water (50 ml) and extracted with DCM (50 ml, 2 times). The bound organic layer was dried with magnesium sulfate anhydrous and vacuum-concentrated. The residue was purified by column chromatography to obtain the compound (89 mg). MS (ESI, m/z): [M+H]⁺ = 825.2
1H NMR (500 MHz, DMSO-d6) δ ppm 10.78 (s, 1 H) 8.65 (s, 3 H) 8.35 - 8.41 (m, 3 H) 8.21 - 8.28 (m, 3 H) 8.18 (d, J=9.77 Hz, 1 H) 7.93 (d, J=7.63 Hz, 1 H) 7.72 (t, J=7.93 Hz, 1 H) 7.45 - 7.51 (m, 2 H) 7.17 (d, J=9.77 Hz, 1 H) 6.82 (t, J=9.31 Hz, 1 H) 6.51 (dd, J=15.11, 2.14 Hz, 1 H) 6.41 (d, J=8.70 Hz, 1 H) 5.74 - 5.86 (m, 1 H) 5.45 (s, 2 H) 4.40 (d, J=12.51 Hz, 1 H) 4.21 - 4.32 (m, 1 H) 4.12 (d, J=13.28 Hz, 1 H) 4.08 (d, J=6.26 Hz, 2 H) 3.30 (d, J=13.28 Hz, 1 H) 2.99 - 3.12 (m, 2 H) 2.86 (br. s., 3 H) 2.68 - 2.77 (m, 1 H) 2.04 - 2.14 (m, 3 H) 1.99 (s, 1 H) 1.78 - 1.90 (m, 3 H) 1.33 (d, J=9.31 Hz, 1 H) 1.23 (s, 1 H) 1.10 - 1.20 (m, 2 H)

### Example C-97: Preparation of 3-(1-(1-(3-(5-(4-((1-(1-(2,6-dioxopiperidine-3-yl)-1-methyl-4-oxo-3,4-dihydropthalazine-6-yl)piperidine-4-il)methylpherozine-1-il)pyrimidine-2-yl)benzyl)6-oxo-1,6-dihydropyridazine-3-yl)benzonitrile (ICT-0001618)

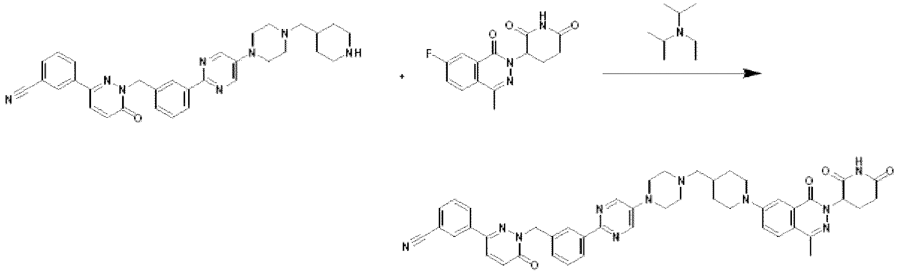

Ethylbis (70.g mg, 54 umol) was added to 3-(6-oxo-1-{3-(5-{4-[(piperidine-4-yl)methyl]piperazine-1-yl}pyrimidin-2-yl)phenyl]methyl}-1,6-dihydropyridazine-3-yl)benzonitrile (105 mg, 274 umol) and 3-(8-fluoro-4-methyl-1-oxo-1,2-dihydroptallazine-2-yl)piperidine-2,6-dione (79.4 mg, 274 umol) in NMP (5 ml), stirred for 2 hours at 110°C. After cooling to room temperature, the reactants were concentrated under pressure, Purified by column chromatography, 3-{1-[(3-{5-[4-({1-[3-(2,6-dioxopiperidin-3-yl)-1-methyl-4-oxo-3,4-dihydrophthalazine-6-yl]piperidine-4-yl}methyl)piperazine-1-il]pyrimidine-2-yl}phenyl)methyl]-6-oxo-1,6-dihydropyridazine-3-yl}benzonitrile was obtained as a white solid (142 mg). MS (ESI, m/z): [M+H]⁺ = 816.8

### Example C-98: Preparation of 3-(1-(3-(5-(4-((1-(2-(2,6-dioxopiperidin-3-yl)-1-oxo-1,2-dihydropthalazine-6-yl)piperidine-4-il)methyl)piperazine-1-il)pyrimidine-2-il)benzyl)6-oxo-1,6-dihydropyridazine-3-yl)benzonitrile (ICT-0001620)

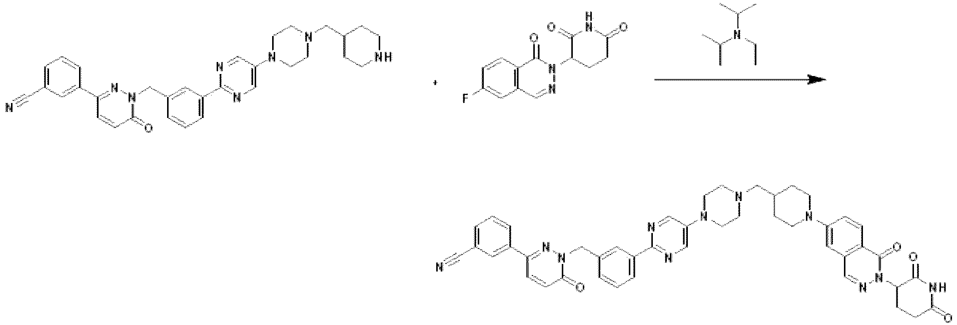

Ethylbis (70.g mg, 549 umol) was added to 3-(6-oxo-1-{[5-{4-[(piperidine-4-yl)methyl]piperazine-1-yl}pyrimidin-2-yl)phenyl]methyl}-1,6-dihydropyridazin-3-yl)benzonitrile (105 mg, 274 umol) and 3-(6-fluoro-1-oxopthalazine-2(1H)-yl)piperidine-2,6-dione (72.5 mg, 274 umol) in NMP (5 ml) and stirred for 2 hours at 110°C. After cooling to room temperature, the reactant was depressurized and concentrated. Purified by column chromatography, 3-(1-(3-(5-(4-((1-(2-(2,6-dioxopiperidine-3-yl)-1-oxo-1,2-dihydrophoptalazine-6-yl)piperidine-4-il)methyl)piperazine-1-day)pyrimidine-2-yl)benzonitrile-6-oxo-1,6-dihydropyridazine-3-yl) benzonitrile was obtained as a white solid (135 mg). MS (ESI, m/z): [M+H]⁺ = 802.65.

### Example C-99: Preparation of 3-(1-(3-(5-(4-((1-(3-(2,6-dioxopiperidin-3-yl)-4-oxo-3,4-dihydrophthalazine-6-yl)piperidine-4-il)methyl)piperazine-1-il)pyrimidine-2-il)benzyl)6-oxo-1,6-dihydropyridazine-3-yl)benzonitrile (ICT-0001642)

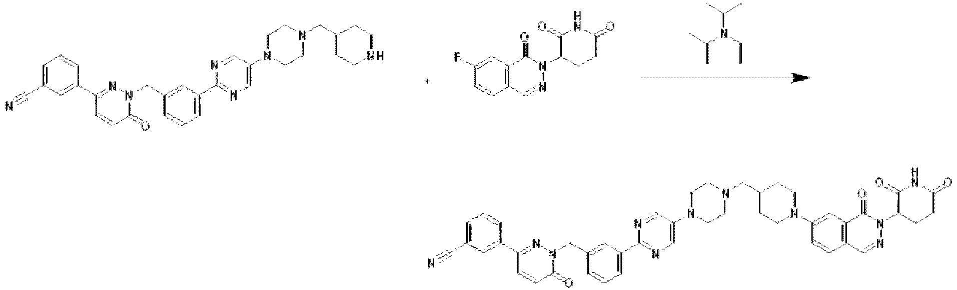

Ethylbis (70.g mg, 54 umol) was added to 3-(6-oxo-1-{[5-{4-[(piperidine-4-yl)methyl]piperazine-1-yl}pyrimidin-2-yl)phenyl]methyl}-1,6-dihydropyridazine-3-yl)benzonitrile (105 mg, 274 umol) and 3-(7-fluoro-1-oxophthalazine-2(1H)-day)piperidine-2,6-dione (72.5 mg, 274 umol) in NMP (5 ml), stirred for 2 hours at 110°C. After cooling to room temperature, the reactant was concentrated under reduced pressure, Purified by column chromatography, 3-{1-[(3-{5-[4-({1-[3-(2,6-dioxopiperidin-3-yl)-1-methyl-4-oxo-3,4-dihydropthalazine-6-yl]piperidine-4-yl}methyl)piperazine-1-il]pyrimidine-2-yl}phenyl)methyl]-6-oxo-1,6-dihydropyridazine-3-yl}benzonitrile was obtained as a white solid (107 mg). MS (ESI, m/z): [M+H]⁺ = 802.65.

### Example C-100: Synthetic 3-(1-(3-(5-(4-(3-(3-(4-(4-(2-(2,6-dioxopiperidin-3-yl)-1-oxo-1,2-dihydrophthalazine-6-yl)piperazine-1-yl)-2-hydroxypropyl)piperazine-1-yl)piperazine-1-yl)pyrimidine-2-yl)benzyl)-6-oxo-1,6-dihydropyridazine-3-yl)benzonitrile (ICT-0001745)

### Step 1) Preparation of 3-(1-(3-(5-(4-(oxyran-2-ylmethyl)piperazine-1-yl)pyrimidine-2-yl)benzyl)-6-oxo-1,6-dihydropyridazine-3-yl)benzonitrile

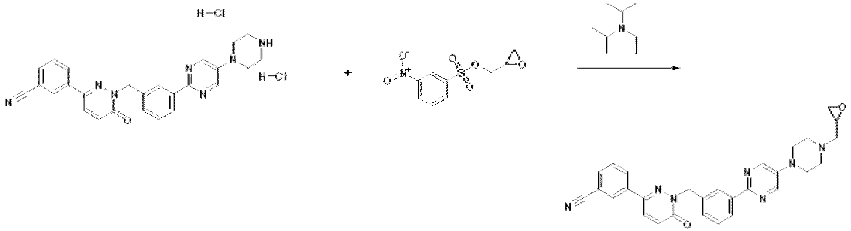

N-ethylbis (isopropyl)amine (1.93 g, 14.9 mmol) was added to m-[3-oxo-2-({m-[5-(1-piperazinyl)-2-pyrimidinyl]phenyl}methyl)-6-pyridazinyl]benzonitrile-hydrochloride (1/2) [2-hydrochloride] (1.95 g, 3.73 mmol) and 2-[(m-nitrophenylsulfonyloxy)methyl]oxyrane (968 mg, 3.73 mmol) in DMF (2 mL) at room temperature. The reactant was heated to 45°C and stirred for 5 hours. Extracted with ethyl acetate (30 mL x 2). The mixed organic layer was dried with Magnesium sulfate and concentrated under reduced pressure. The residue was purified with 100% ethyl acetate column chromatography to obtain the title compound (1.59 g). MS (ESI, m/z): [M+H]⁺ = 506.23.

### Step2) Preparation of 3-(1-(3-(5-(4-(3-(3-(4-(4-(2-(2,6-dioxopiperidin-3-yl)1-oxo-1,2-dihydropthalazine-6-yl)piperazine-1-yl)-2-hydroxypropyl)piperazine-1-il)pyrimidine-2-yl)benzyl)6-oxo-1,6-dihydropyridazine-3-yl)benzonitrile

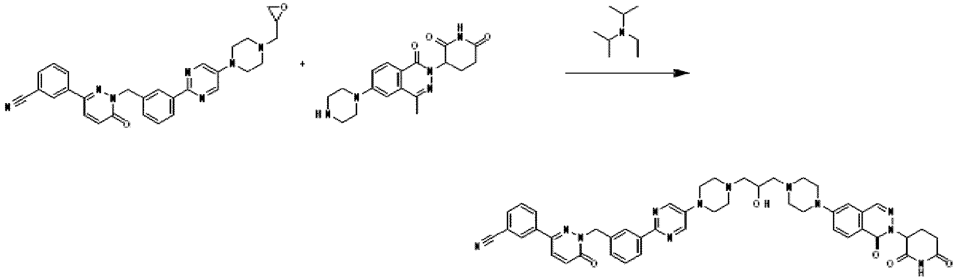

N-ethylbis(isopropyl)amine (128 mg, 989 umol) was added to the solution of m-(2-{[m-(5-{4-[(2-oxyranyl)methyl]-1-piperazinyl}-2-pyrimidinyl)phenyl]methyl}-3-oxo-6-pyridazinyl) benzonitrile (100 mg, 198 umol) and 3-[1-oxo-6-(1-piperazinyl)-2-phthalazine]-2,6-piperidione-hydrochloride (1/2) [2-hydrochloride] (81 mg, 198 umol) in DMF (2 mL) at room temperature. They stirred for five hours. After the end of the reaction, the reactant was poured into water (50 mL) and extracted with ethyl acetate (30 mL x 2). The mixed organic layer was dried with Magnesium sulfate and concentrated under reduced pressure. The residue was purified with 10% MeOH/ethyl acetate column chromatography to obtain the title compound (132 mg). MS (ESI, m/z): [M+H]⁺ = 847.28.

### Example C-101: Preparation of 3-(1-(3-(5-(4-(3-(3-(4-(4-(2-(2,6-dioxopiperidin-3-yl)-4-methyl-1-oxo-1,2-dihydropthalazine-6-yl)piperazine-1-yl)-2-hydroxypropyl)piperazine-1-yl)pyrimidine-2-yl)benzyl)-6-oxo-1,6-dihydropyridazine-3-yl)benzonitrile (ICT-0001704)

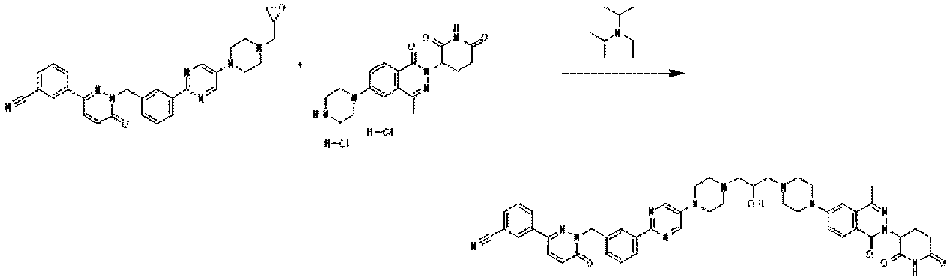

N-ethylbis(isopropyl)amine (192 mg, 1.48 mmol) was added to m-(2-{[m-(5-{4-[(2-oxyranyl)methyl]-1-piperazinyl}-2-pyrimidinyl)phenyl]methyl}-3-oxo-6-pyridazinyl) benzonitrile (150 mg, 297 umol) and 3-[4-methyl-1-oxo-6-(1-piperazinyl)-2-phthalazine]-2,6-piperidinedione-hydrochloride (1/1) (140 mg, 356 umol) in DMF (2 mL) at room temperature. They stirred for five hours. After the end of the reaction, the reactant was poured into water (50 mL) and extracted with ethyl acetate (30 mL x2). The mixed organic layer was dried with Magnesium sulfate and depressurized and concentrated. The residue was purified with 10% MeOH/ethyl acetate column chromatography to obtain the title compound (198 mg). MS (ESI, m/z): [M+H]⁺ = 861.41.

**[Table 2] c-MET PROTAC in vitro degradation assays and cell viability assays.**

| ICT-CODE | Structure | In vitro cell viability assay (nM) | | | | | | | |
|---|---|---|---|---|---|---|---|---|---|
| | | MKN-45 | | Hs746T | | SNU-638 | | EBC-1 | |
| | | DC₅₀ | IC₅₀ | DC₅₀ | IC₅₀ | DC₅₀ | IC₅₀ | DC₅₀ | IC₅₀ |
| ICT-0000907 | 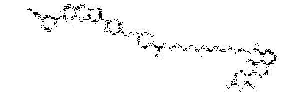 | B | A | | | | | | |
| ICT-0000975 | 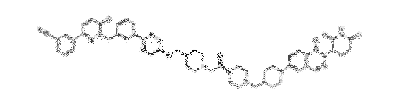 | B | A | | | | | | |
| ICT-0000979 | 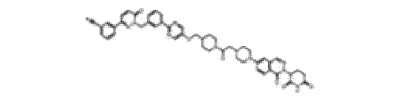 | A | A. | | | B | B | | |
| ICT-0000980 | 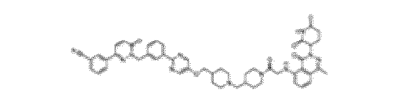 | A | A | | | C | B | | |
| ICT-0000981 | 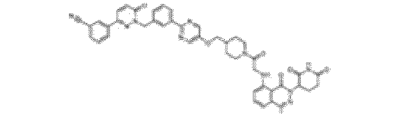 | C | A | | | | | | |
| ICT-0000984 | 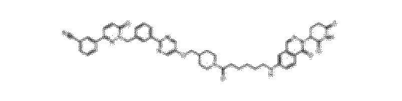 | B | A | | | | | | |
| ICT-0000985 | 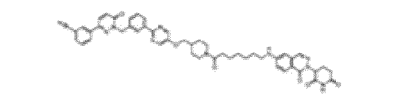 | A | A | | | | | | |
| ICT-0000986 | 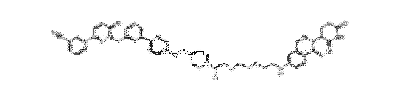 | A | A | | | | | | |
| ICT-0000987 | 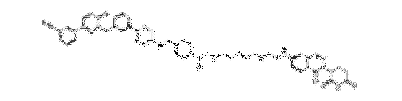 | B | A | | | | | | |
| ICT-0000988 | 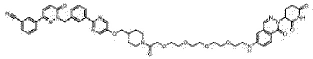 | A | B | | | | | | |
| ICT-0000989 | 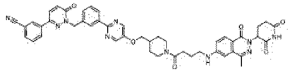 | B | A | | | | | | |
| ICT-0000990 | 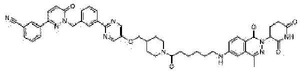 | B | B | | | | | | |
| ICT-0001087 | 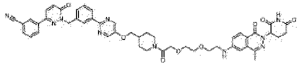 | A | A | | | | | | |
| ICT-0001088 | 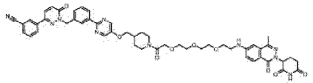 | A | B | | | | | | |
| ICT-0001089 | 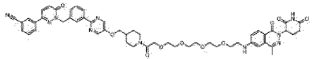 | B | B | | | | | | |
| ICT-0001091 | 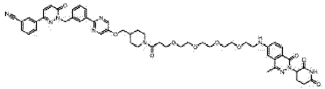 | A | A | | | | | | |
| ICT-0001096 | 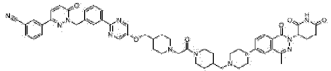 | A | A | | | | | | |
| ICT-0001115 | 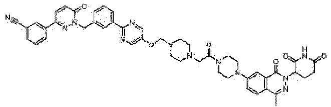 | A | A | | | | | | |
| ICT-0001173 | 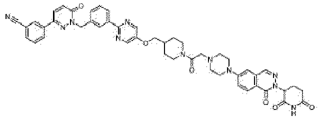 | A | A | | | | | | |
| ICT-0001174 | 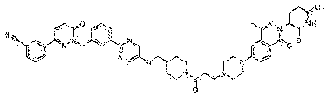 | A | A | | | | | | |
| ICT-0001175 | 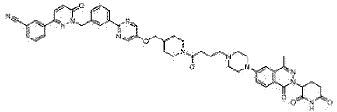 | A | A | | | | | | |
| ICT-0001176 | 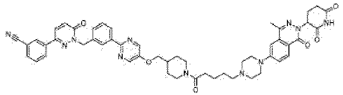 | A | A | | | | | | |
| ICT-0001177 | 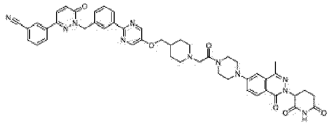 | A | A | | | | | | |
| ICT-0001195 | 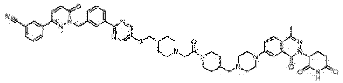 | A | A | | | | | | |
| ICT-0001199 | 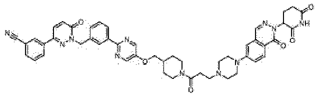 | A | A | | | | | | |
| ICT-0001200 | 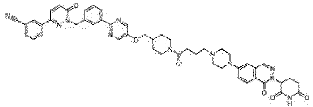 | A | A | | | | | | |
| ICT-0001201 | 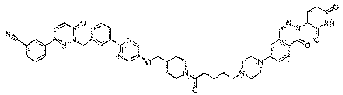 | A | A | | | | | | |
| ICT-0001204 | 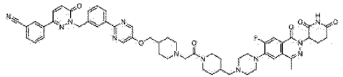 | A | A | | B | | | | A |
| ICT-0001205 | 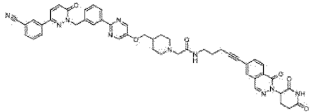 | A | A | | B | | | | |
| ICT-0001206 | 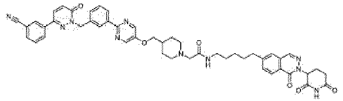 | A | A | | | | | | |
| ICT-0001207 | 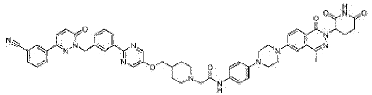 | A | A | A | B | A | A | A | A |
| ICT-0001213 | 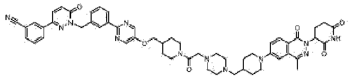 | A | A | | | | | | |
| ICT-0001214 | 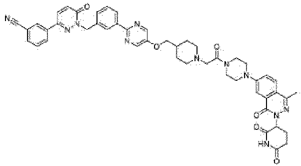 | A | A | | | | | | |
| ICT-0001215 | 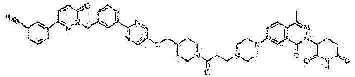 | A | A | | | A | A | A | A |
| ICT-0001216 | 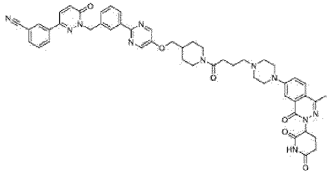 | A | A | | | | | | |
| ICT-0001217 | 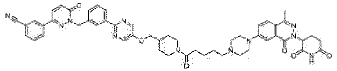 | A | A | | | | | | |
| ICT-0001218 | 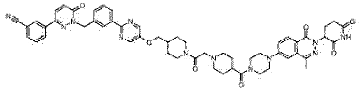 | A | A | | | A | A | | |
| ICT-0001219 | 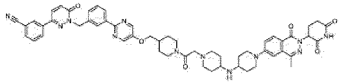 | A | A | | | | | | |
| ICT-0001221 | 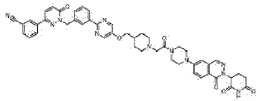 | A | A | | | | | | |
| ICT-0001223 | 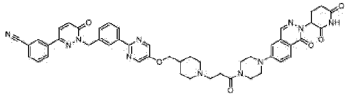 | A | A | | A | | | | |
| ICT-0001224 | 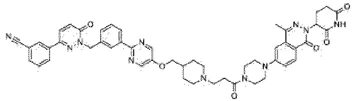 | A | A | | A | | | | |
| ICT-0001.225 | 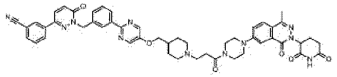 | A | A | | A | | | | |
| ICT-0001226 | 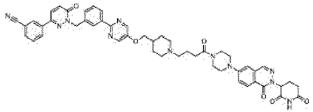 | B | A | | A | | | | |
| ICT-0001227 | 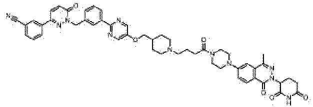 | B | A | | A | | | | |
| ICT-0001228 | 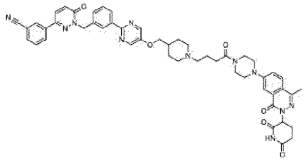 | A | A | | B | | | | |
| ICT-0001229 | 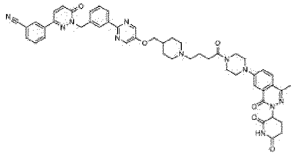 | A | A | | | | | | |
| ICT-0001231 | 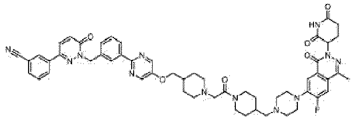 | | A | | | | | | |
| ICT-0001232 | 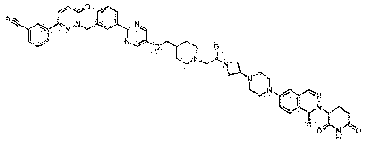 | A | A | | | A | | A | A |
| ICT-0001233 | 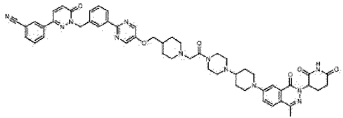 | A | A | | | | | | |
| ICT-0001234 | 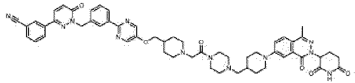 | C | A | | | | | | |
| ICT-0001235 | 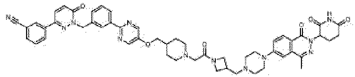 | A | A | | | | | | |
| ICT-0001236 | 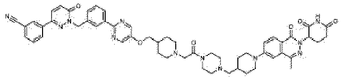 | A | A | | | | | | |
| ICT-0001237 | 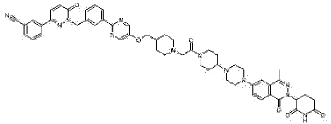 | A | A | | | | | | |
| ICT-0001238 | 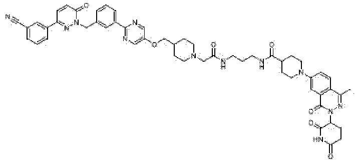 | C | A | | | | | | |
| ICT-0001239 | 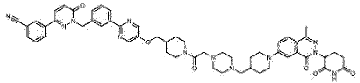 | A | A | | | | | | |
| ICT-0001240 | 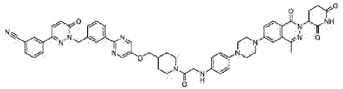 | B | A | | | | | | |
| ICT-0001250 | 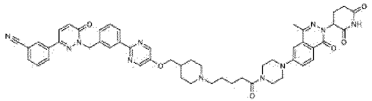 | B | A | | A | | | | |
| ICT-0001251 | 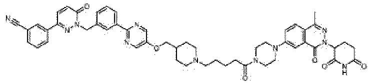 | A | A | | | | | | |
| ICT-0001252 | 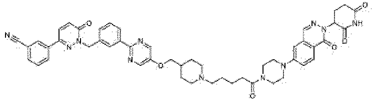 | A | A | | | | | | |
| ICT-0001253 | 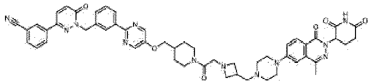 | A | A | | | | | | |
| ICT-0001254 | 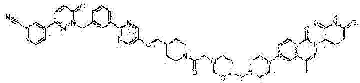 | B | A | | | | | | |
| ICT-0001255 | 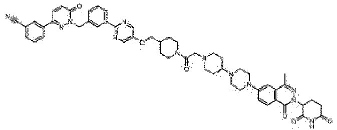 | A | A | | | | A | | |
| ICT-0001256 | 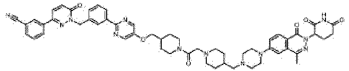 | A | A | | | | | | |
| ICT-0001257 | 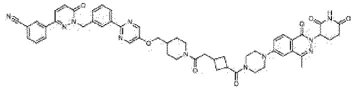 | A | A | | | | | B | A |
| ICT-0001258 | 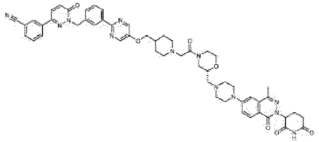 | A | A | | | | | | |
| ICT-0001262 | 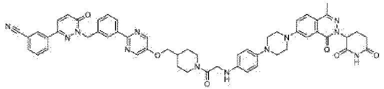 | A | A | | | | | | A |
| ICT-0001270 | 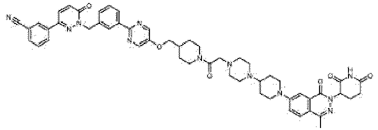 | A | A | | | | | | |
| ICT-0001271 | 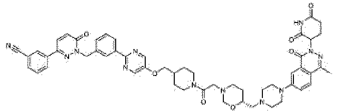 | A | A | | | | | | |
| ICT-0001272 | 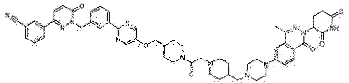 | A | A | | | | | | |
| ICT-0001276 | 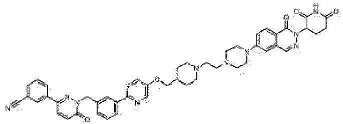 | A | A | | | | A | C | |
| ICT-0001277 | 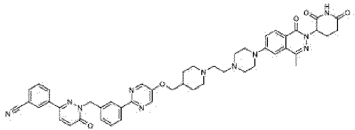 | A | A | A | A | A | A | A | A |
| ICT-0001278 | 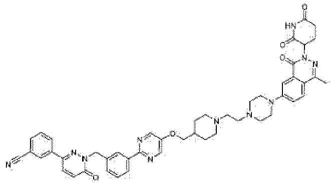 | B | A | | | | | C | |
| ICT-0001302 | 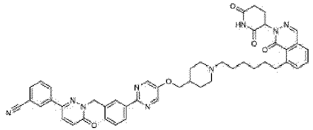 | | | | | A | A | | |
| ICT-0001303 | 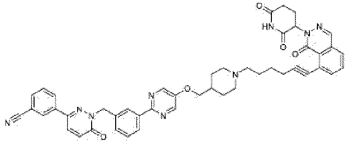 | | | | | B | A | | |
| ICT-0001304 | 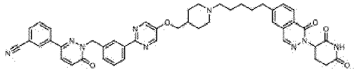 | | | | | | A | | |
| ICT-0001305 | 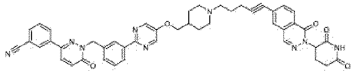 | | | | | | A | | |
| ICT-0001307 | 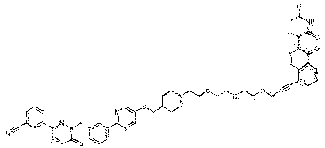 | | | | | | A | | |
| ICT-0001315 | 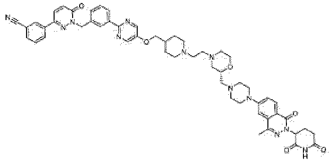 | | | | | | A | | |
| ICT-0001317 | 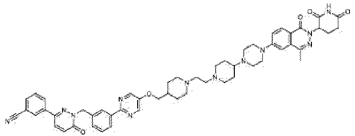 | | | | | | A | | |
| ICT-0001318 | 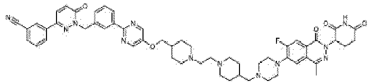 | | | | | | A | | |
| ICT-0001319 | 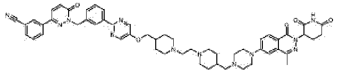 | | | | | | A | | |
| ICT-0001321 | 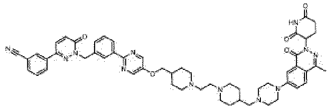 | | | | | | A | | |
| ICT-0001325 | 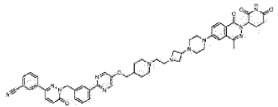 | | | | | C | A | | |
| ICT-0001326 | 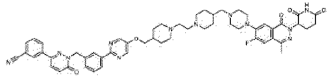 | | | | | C | B | | |
| ICT-0001383 | 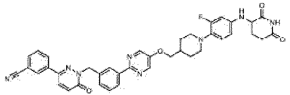 | | | | | A | A | A | A |
| ICT-0001384 | 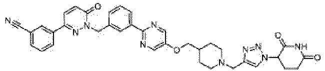 | | | | | C | B | A | A |
| ICT-0001402 | 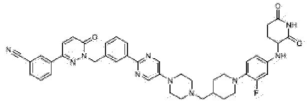 | | | A | A | A | A | A | A |
| ICT-0001425 | 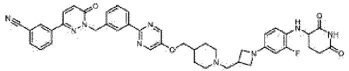 | | | | | A | A | A | A |
| ICT-0001433 | 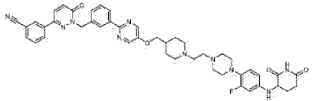 | | | | | A | A | A | A |
| ICT-0001434 | 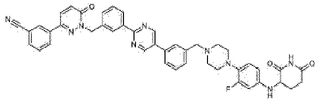 | | | | | C | A | A | A |
| ICT-0001437 | 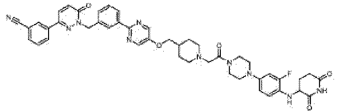 | | | | | A | A | A | A |
| ICT-0001447 | 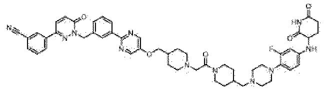 | | | | | B | A | A | A |
| ICT-0001449 | 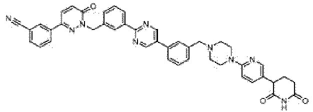 | | | | | C | A | A | A |
| ICT-0001450 | 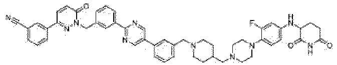 | | | | | B | A | A | A |
| ICT-0001468 | 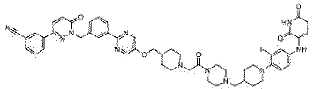 | | | | | B | A | A | A |
| ICT-0001494 | 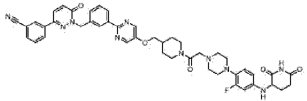 | | | | | A | A | A | A |

| | | | | | | | | | |
|---|---|---|---|---|---|---|---|---|---|
| A: IC₅₀ or DC₅₀ < 100 nM B: 100 nM < IC₅₀ or DC₅₀ < 1 uM C: 1 uM < IC₅₀ or DC₅₀ | | | | | | | | | |

### Example 1: Cell line culture and compound processing

c-MET overexpressing human gastric cancer cell lines [MKN45] and [SNU638] were cultured in RPMI medium with 10% fetal bovine serum, penicillin (100U/ml), and streptomycin (100mg/ml), and c-MET exon 14 skipping mutant human gastric cancer cell lines [Hs746T] were cultured in DMEM media. The c-MET overexpressing human lung cancer cell line EBC-1 was cultured in MEM media with the addition of 10% fetal bovine serum, penicillin (100U/ml), and streptomycin (100mg/ml). Normal cell lines WI-38 derived from human lung tissue were cultured in EMEM medium with the addition of 10% fetal bovine serum, penicillin (100U/ml), and streptomycin (100mg/ml). The compound synthesized in accordance with the present invention was prepared with a raw material solution of 10 mM dissolved in DMSO (dimethyl sulfoxide), and was sequentially diluted and processed.

### Example 2: Evaluation of cancer cell antiproliferative activity of cMET target ligand compounds

In order to observe the inhibitory activity on cell growth, cells were dispensed in a 96-well plate at a concentration of 5,000 cells/well, and then treated with the compound prepared at various concentrations for 72 hours. The viability of the cells was calculated using GraphicPad Prism 8.0 software by adding 10 ml per well of WST-8 (water soluble tetrazolium salt) sample, which reacts with dehydrogenase present in the electron transport system of mitochondria to produce formazon, incubated for 2 hours, and measured absorbance at 450 nm. The IC₅₀ value (concentration of the compound achieving 50% cell proliferation inhibition) is the average value of the measurement results. As shown in the experimental results, the compounds of the present invention showed the effect of inhibiting the proliferation of cancer cells. The results are shown in Tables 1 and 2 above.

### Example 3: Evaluation of c-MET protein resolution of PROTAC targeting cMET protein

In order to assess the resolution of the compound of the present invention for c-MET expressed in cells, cells were dispensed in a 12-well plate at a concentration of 2 × 105 cells/well, and then gastric cancer cell lines were treated with samples for 24 hours and lung cancer cell lines were treated for 48 hours. Cell lysates were collected using a scraper on ice, centrifuged at 4°C15,000 rpm for 30 min to obtain a protein-containing supernatant. Protein expression was then measured using the Simple Western Automated Western Blot System Abby instrument using 3 µg of protein for each sample. SDS and heat-induced denaturation proteins were separated by molecular weight, attached to capillary, reacted with anti-c-MET or anti-GAPDH antibodies, which are primary antibodies, and then reacted with HRP-conjugated secondary antibodies, and analyzed the protein expression signal with compass software. Protein expression was normalized based on the expression value of GAPDH, and protein development in each sample was measured as a percentage (%) based on 100% of samples treated with 0.1% DMSO carriers, and DC50 (concentration of a compound where 50% protein inhibition is achieved) was calculated from the change in protein expression amount due to compound treatment. The results are shown in Tables 1 and 2 above.

### [Industrial Applicability]

The TPD compound according to the present invention can be used usefully as an anticancer drug.

## Claims

1. A compound represented by the following Chemical Formula 1:
[Chemical Formula 1] cMET Targeted Protein-Binding Moiety (P)-{Linker (L)}p-E3 Ligase-Binding Moiety (E)
wherein,
E3 Ligase-Binding Moiety (E) is a compound represented by the following Chemical Formula 2; and
cMET Targeted Protein-Binding Moiety (P) is a compound represented with any of the following formulas 3 to 7; and
p is an integer of 0 or 1: 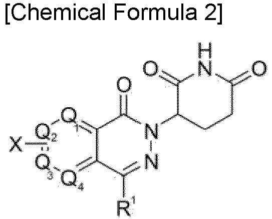 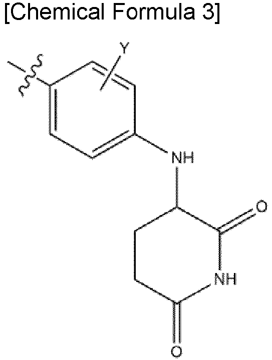 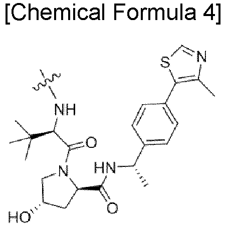 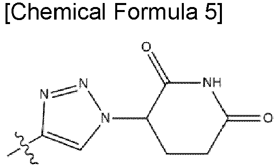 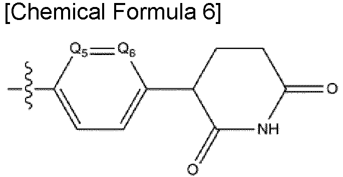 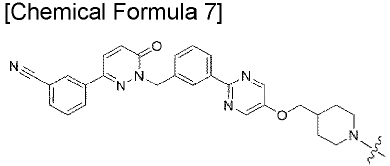 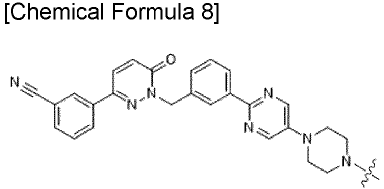 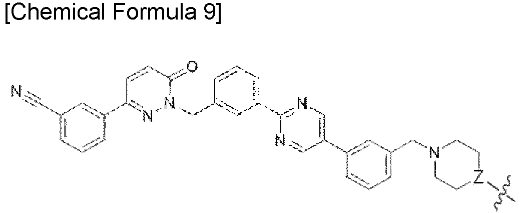 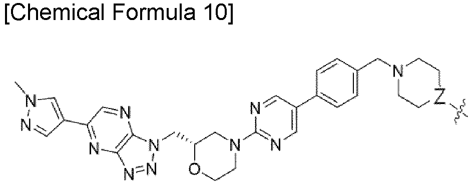 wherein,
X is hydrogen, halogen, amino, nitro, hydroxy, C1 to C6 straight or branched alkyl, or a 4 to 8 atom heterocyclic containing oxygen or nitrogen;
Q₁ to Q₄ are each independently C-F, C-Cl, C-H, C-X, or N, and at least any one of Q₁ to Q₄ is C-X;
Y is hydrogen, halogen, or a C1 to C6 a straight, branched or cyclic alkyloxy unsubstituted or substituted with halogen;
provided that when one of Q₅ and Q₆ is carbon atom, and the other is nitrogen atom,f
Z is carbon atom or nitrogen atom; and
R1 is hydrogen, nitro, amino, carbonyl, C1 to C6 straight, branched or cyclic alkyl, or C1 to C6 straight, branched, or cyclic alkyl substituted with halogen.

2. The compound of Claim 1, wherein one terminal of the linker (L) is
(i) connected to a structure in Chemical Formula 1 by substituting X in a structure of Chemical Formula 2;
(ii) connected to a structure in Chemical Formula 1 by bonding to nitrogen or oxygen atom of X in a structure of Chemical Formula 2;
(iii) directly connected to the benzene ring of the structure of the Chemical Formula 3;
(iv) directly connected to an amino group located at the terminal of the structure of Chemical Formula 4;
(v) directly connected to the triazole ring of the structure of the Chemical Formula 5; or
(vi) directly connected to the pyridine ring of the structure of Chemical Formula 6.

3. In Paragraph 2, the other terminal of the linker (L) is connected to a compound linked to piperidine or piperazine located at an end of any of the structures of Chemical Formula 7 to 10.

4. In paragraph 1, The compound of Claim 1, wherein
the linker (L) is 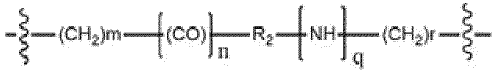 or 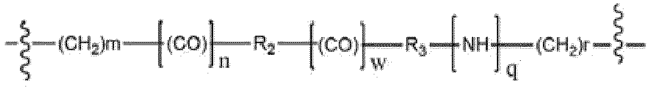 or 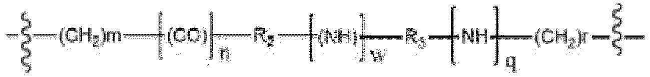 or 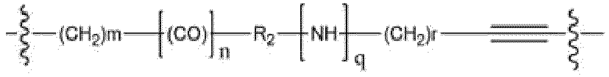 or 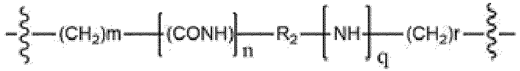 or 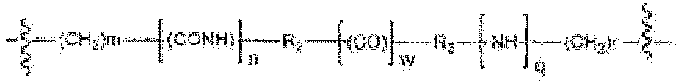 or 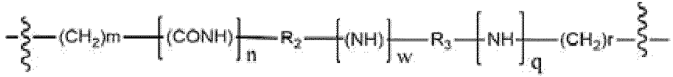 or 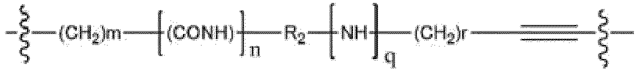 or
a structure selected from the group consisting of the following formulas: 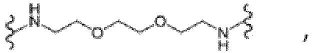 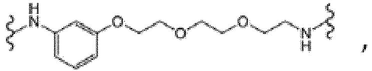 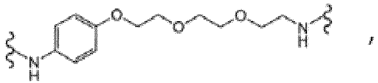 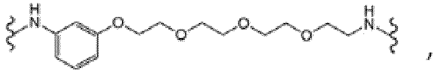 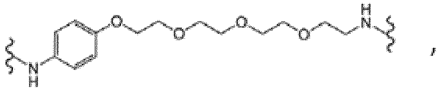 and 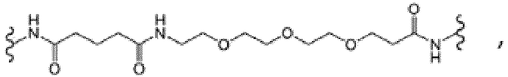
wherein, R₂ and R₃ are each independently -(CH₂)ₛ-, -(CH₂)ₜ-[O(C₂H₄)]ᵤ-, -O-(CH₂)-, - CH(OH)-piperazine, piperidine, azetidine, -(CH₂)ᵥ-piperidine, -(CH₂)ᵥ-piperazine, piperazine-(CH₂)ᵥ-piperidine, piperazine-(CH₂)ᵥ-morpholine, piperidine-(CH₂)ᵥ-morpholine, azetidine-(CH₂)ᵥ-piperazine, azetidine-(CH₂)ᵥ-piperidine, benzene-piperidine, benzene-piperazine, -(CO)-piperidine, -(CO)-piperazine, -(CO)-piperazine, benzene, triazole or pyrrolidine; and
m, n, q, r, s, t, u, v, and w are each independently an integer selected from 0 to 7.

5. A compound selected from the group consisting of the following compounds or a pharmaceutically acceptable salt thereof:
3-{6-[(4-{4-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-yl}phenyl)methyl]piperazine-1-yl}-4-oxobutyl)amino]-1-oxo-1,2-dihydropthalazine-2-yl}piperidine-2,6-dione;
3-{6-[(6-{4-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-yl}phenyl)methyl]piperazine-1-yl}-6-oxohexyl)amino]-1-oxo-1,2-dihydroptalazine-2-yl}piperidine-2,6-dione ;
3-(6-((5-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)5-oxophtyl)amino)1-oxopthalazine-2(1H)-yl)piperidine-2,6-dione;
3-(6-((7-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)7-oxoheptyl)amino)-1-oxopthalazine-2(1H)-yl)piperidine-2,6-dione;
3-(6-((2-(2-(2-(2-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)-2-oxoethoxy)ethoxy)ethyl)amino)-1-oxopthalazine-2(1H)-yl)piperidine-2,6-dione;
3-[6-(12-{4-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-yl}phenyl)methyl]piperazine-1-yl}-12-oxo-4,7,10-trioxa-1-azadodecane-1-yl)-1-oxo-1,2-dihydroptalazine-2-yl]piperidine-2,6-dione;
3-[6-(15-{4-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-day}phenyl)methyl]piperazine-1-yl}-15-oxo-4,7,10,13-tetraoxa-1-azpentadecan-1-yl)-1-oxo-1,2-dihydroptalazine-2-yl]piperidine-2,6-dione;
3-(8-((2-(2-(3-(4-(4-(2-((S)-2-((5-(1-methyl-1H-pyrazole-4-yl)-1H-[1,2,3]triazolo[4,5-b]pyrazine-1-yl)methyl)morpholino)pyrimidin-5-yl})benzyl)piperazine-1-yl)-3-oxopropoxy)ethoxy)ethyl)amino)-1-oxopthalazine-2-(1H)-yl]piperidine-2,6-dione;
3-[8-(12-{4-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-yl}phenyl)methyl]piperazine-1-yl}-12-oxo-4,7,10-trioxa-1-azadodecane-1-yl)-1-oxo-1,2-dihydroptalazine-2-yl]piperidine-2,6-dione;
3-[8-(16-{4-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-yl}phenyl)methyl]piperazine-1-yl}-16-oxo-4,7,10,13-tetraoxa-1-azahexadecane-1-yl)-1-oxo-1,2-dihydropetalazine-2-yl]piperidine-2,6-dione;
3-{8-[(5-{4-[(4-{2-[(2S)-2-{[5-(1-methyl-1H-pyrazole-4-yl)-1H-[1,2,3]triazolo[4,5-b]pyrazine-1-yl]methyl}morpholine-4-il]pyrimidine-5-yl}phenyl)methyl]piperazine-1-yl}-5-oxopentyl)amino]-1-oxo-1,2-dihydroptalazine-2-yl}piperidine-2,6-dione;
3-{8-[(7-{4-[(4-{2-[(2S)-2-{[5-(1-methyl-1H-pyrazole-4-yl)-1H-[1,2,3]triazolo[4,5-b]pyrazine-1-yl]methyl}morpholine-4-yl]pyrimidine-5-yl}phenyl)methyl]piperazine-1-yl}-7-oxohepty)amino]-1-oxo-1,2-dihydroptalazine-2-yl}piperidine-2,6-dione;
3-[8-({2-[2-(3-{4-[(4-{2-[(2S)-2-{[5-(1-methyl-1H-pyrazole-4-yl)-1H-[1,2,3]triazolo[4,5-b]pyrazine-1-yl]methyl}morpholine-4-day]pyrimidine-5-day}phenyl)methyl]piperazine-1-yl}-3-oxopropoxy)ethoxy]ethyl}amino)-1-oxo-1,2-dihydrophthalazine-2-yl]piperidine-2,6-dione;
3-(8-((21-(4-(4-(2-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)21-oxo-3,6,9,12,15,18-hexaoxahenicosyl)amino)-1-oxopthalazine-2(1H)-yl)piperidine-2,6-dione;
3-(8-((4-(4-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)4-oxobutyl)amino)1-oxopthalazine-2(1H)-yl)piperidine-2,6-dione;
3-(8-((6-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)6-oxohexyl)amino)-1-oxopthalazine-2(1H)-yl)piperidine-2,6-dione;
3-(6-(4-((1-(4-(2-((S)-2-((5-(1-methyl-1H-pyrazole-4-yl)-1H-[1,2,3]triazolo[4,5-b]pyrazine-1-yl)methyl)morpholino)pyrimidine-5-il)benzyl)piperidine-4-il)methyl)piperazine-1-yl)-1-oxopthalazine-2(1H)-yl)piperidine-2,6-dione;
3-(4-methyl-8-((2-(4-(4-(2-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)-2-oxoethyl)amino)1-oxopthalazine-2(1H)-yl)piperidine-2,6-dione;
3-(6-(4-(2-(4-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)-2-oxoethyl)piperazine-1-yl)-1-oxopthalazine-2(1H)-yl)piperidine-2,6-dione;
3-((3-fluoro-4-(4-(4-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-yl)phenyl)amino)piperidine-2,6-dione;
3-((2-fluoro-4-(4-(2-(4-(4-(2-(2-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-il)acetyl)piperazine-1-yl)phenyl)amino)piperidine-2,6-dione;
3-((3-fluoro-4-(4-(2-(4-(4-(2-(2-(2-((S)-2-((5-(1-methyl-1H-pyrazole-4-yl)-1H-[1,2,3]triazolo[4,5-b]pyrazine-1-yl)methyl)morpholino)pyrimidine-5-yl)benzyl)piperazine-1-day)acetyl)piperazine-1-yl)phenyl)amino)piperidine-2,6-dione;
3-(1-(3-(5-((1-(1-(1-(3-(3-(2,6-dioxopiperidine-3-yl)-4-oxo-3,4-dihydrophthalazine-5-yl)amino)3,6,9,12-tetraoxapentadecan-15-oil)piperidine-4-yl)methoxy)pyrimidine-2-yl)benzyl)6-oxo-1,6-dihydropyridazine-3-yl)benzonitrile;
3-(1-(3-(5-((1-(2-(4-((1-(1-(2-(2-(2,6-dioxopiperidine-3-yl)-1-oxo-1,2-dihydropthalazine-6-yl)piperidine-4-yl)methyl)piperazine-1-yl)2-oxoethyl)piperidine-4-yl)methoxy)pyrimidine-2-yl)benzyl)6-oxo-1,6-dihydropyridazine-3-yl)benzonitrile;
3-(1-{[3-(5-{[1-(2-{4-[2-(2,6-dioxopiperidine-3-yl)-1-oxo-1,2-dihydropthalazine-6-yl]piperazine-1-il}acetyl)piperidine-4-yl]methoxy}pyrimidine-2-yl)phenyl]methyl}-6-oxo-1,6-dihydropyridazine-3-yl)benzonitrile;
3-(1-(3-(5-((1-((1-((1-(3-(2,6-dioxopiperidine-3-yl)-1-methyl-4-oxo-3,4-dihydropthalazine-5-yl)glysil)piperidine-4-yl)methyl)piperidine-4-il)methoxy)pyrimidine-2-yl)benzyl)6-oxo-1,6-dihydropyridazine-3-yl)benzonitrile;
3-(1-(3-(5-((1-((3-(3-(2,6-dioxopiperidine-3-yl)-1-methyl-4-oxo-3,4-dihydroptalazin-5-yl)glysil)piperidine-4-yl)methoxy)pyrimidine-2-yl)benzyl)6-oxo-1,6-dihydropyridazine-3-yl)benzonitrile;
3-(1-{[3-(5-{[1-(6-{[2-(2,6-dioxopiperidine-3-yl)-1-oxo-1,2-dihydroptalazine-6-yl]amino}hexanoyl)piperidine-4-il]methoxy}pyrimidine-2-yl)phenyl]methyl}-6-oxo-1,6-dihydropyridazine-3-yl)benzonitrile;
3-(1-{[3-(5-{[1-(7-{[2-(2,6-dioxopiperidin-3-yl)-1-oxo-1,2-dihydropthalazine-6-yl]amino}heptanoyl)piperidine-4-il]methoxy}pyrimidine-2-yl)phenyl]methyl}-6-oxo-1,6-dihydropyridazine-3-yl)benzonitrile;
3-{1-[(3-{5-[(1-{2-[2-(2-{[2-(2,6-dioxopiperidine-3-yl)-1-oxo-1,2-dihydrophthalazine-6-yl]amino}ethoxy)ethoxy]acetyl}piperidine-4-yl)methoxy]pyrimidine-2-yl}phenyl)methyl]-6-oxo-1,6-dihydropyridazine-3-yl}benzonitrile;
3-(1-{[3-(5-{[1-(2-{2-{2-(2-{2-{2-(2,6-dioxopiperidine-3-yl)-1-oxo-1,2-dihydropetthalazine-6-yl]amino}ethoxy)ethoxy}ethoxy}piperidine-4-il]methoxy}pyrimidine-2-yl)phenyl]methyl}-6-oxo-1,6-dihydropyridazine-3-yl)benzonitrile;
3-(1-{[3-(5-{[1-(14-{[2-(2,6-dioxopiperidine-3-yl)-1-oxo-1,2-dihydroptalazine-6-yl]amino}-3,6,9,12-tetratetradecanoyl)piperidine-4-yl]methoxy}pyrimidine-2-yl)phenyl]methyl}-6-oxo-1,6-dihydropyridazine-3-yl)benzonitrile;
3-(1-(3-(5-((1-(4-(2-(2,6-dioxopiperidin-3-yl)-4-methyl-1-oxo-1,2-dihydropthalazine-6-yl)amino)butanoyl)piperidine-4-yl)methoxy)pyrimidine-2-yl)benzyl)6-oxo-1,6-dihydropyridazine-3-yl)benzonitrile;
3-(1-(3-(5-((1-(6-(2-(2,6-dioxopiperidine-3-yl)-4-methyl-1-oxo-1,2-dihydropthalazine-6-yl)amino)hexanoyl)piperidine-4-yl)methoxy)pyrimidine-2-yl)benzonitrile-6-oxo-1,6-dihydropyridazine-3-yl)benzonitrile;
3-(1-(3-(5-((1-(2-(2-(2-(2-(2-(2-(2-(2-(2,6-dioxopiperidin-3-yl)-4-methyl-1-oxo-1,2-dihydroptalazine-6-yl)amino)ethoxy)ethoxy)acetyl)piperidine-4-il)methoxy)pyrimidine-2-yl)benzyl)6-oxo-1,6-dihydropyridazine-3-yl)benzonitrile;
3-(1-(3-(5-((1-(2-(2-(2-(2-(2-(2-(2-(2-(2-(2,6-dioxopiperidine-3-yl)-4-methyl-1-oxo-1,2-dihydroptalazine-6-yl)amino)ethoxy)ethoxy)ethoxy)acetyl)piperidine-4-yl)methoxy)pyrimidine-2-yl)benzonitrile;
3-(1-(3-(5-((1-(1-(14-((2-(2,6-dioxopiperidine-3-yl)-4-methyl-1-oxo-1,2-dihydropthalazine-6-yl)amino)3,6,9,12-tetradedecanoyl)piperidine-4-yl)methoxy)pyrimidine-2-yl)benzyl)6-oxo-1,6-dihydropyridazine-3-yl)benzonitrile;
3-(1-(3-(5-((1-(1-(1-(2-(2,6-dioxopiperidine-3-yl)-4-methyl-1-oxo-1,2-dihydropthalazine-6-yl)amino)3,6,9,12-tetraoxapentadecane-15-oil)piperidine-4-yl)methoxy)pyrimidine-2-yl)benzyl)6-oxo-1,6-dihydropyridazine-3-yl)benzonitrile;
3-(1-(3-(5-((1-(4-((4-(2-(2,6-dioxopiperidine-3-yl)-4-methyl-1-oxo-1,2-dihydropthalazine-6-yl)piperazine-1-yl)methyl)piperidine-1-carbonyl)piperidine-4-il)methoxy)pyrimidine-2-yl)benzyl)6-oxo-1,6-dihydropyridazine-3-yl)benzonitrile;
3-(1-(3-(5-((1-(2-(4-(3-(2-(2,6-dioxopiperidine-3-yl)-1-methyl-4-oxo-3,4-dihydropthalazine-6-ylpiperazine-1-yl)-2-oxoethyl)piperidine-4-yl)methoxy)pyrimidine-2-yl)benzyl)6-oxo-1,6-dihydropyridazine-3-yl)benzonitrile;
3-(1-(4-(5-((1-(2-(2-(2-(2-(2-(2,6-dioxopiperidine-3-yl)-4-methyl-1-oxo-1,2-dihydroptalazine-6-yl)piperazine-1-yl)acetyl)piperidine-4-yl)methoxy)pyrimidine-2-yl)benzyl)6-oxo-1,6-dihydropyridazine-3-yl)benzonitrile;
3-(1-(3-(5-((1-(1-(2-(4-(2-(2,6-dioxopiperidine-3-yl)-4-methyl-1-oxo-1,2-dihydropthalazine-6-yl)piperazine-1-il)acetyl)piperidine-4-il)methoxy)pyrimidine-2-yl)benzyl)6-oxo-1,6-dihydropyridazine-3-yl)benzonitrile;
3-(1-(3-(5-((1-(1-(2-(4-(2-(2,6-dioxopiperidine-3-yl)-4-methyl-1-oxo-1,2-dihydropthalazine-6-yl)piperazine-1-il)acetyl)piperidine-4-il)methoxy)pyrimidine-2-yl)benzyl)6-oxo-1,6-dihydropyridazine-3-yl)benzonitrile;
3-(1-(3-(5-((1-(5-(5-(5-(2-(2,6-dioxopiperidine-3-yl)-4-methyl-1-oxo-1,2-dihydropthalazine-6-yl)piperazine-1-yl)pentanoyl)piperidine-4-il)methoxy)pyrimidine-2-yl)benzyl)6-oxo-1,6-dihydropyridazine-3-yl)benzonitrile;
3-(1-{[3-(5-{[1-(2-{4-[2-(2,6-dioxopiperidine-3-yl)-4-methyl-1-oxo-1,2-dihydropthalazine-6-yl]piperazine-1-yl}-2-oxoethyl)piperidine-4-yl]methoxy}pyrimidin-2-yl)phenyl]methyl}-6-oxo-1,6-dihydropyridazine-3-yl)benzonitrile;
3-(1-(3-(5-((1-(2-(4-((4-(3-(2,6-dioxopiperidine-3-yl)-1-methyl-4-oxo-3-yl)piperazine-1-yl)methyl-1-yl)methyl-1-yl)piperidine-1-yl)-2-oxoethyl)piperidine-4-il)methoxy)pyrimidine-2-yl)benzonitrile;
3-(1-{[3-(5-{[1-(3-{4-[2-(2,6-dioxopiperidine-3-yl)-1-oxo-1,2-dihydropthalazine-6-yl]piperazine-1-il}propanoyl)piperidine-4-il]methoxy}pyrimidine-2-yl)phenyl]methyl}-6-oxo-1,6-dihydropyridazine-3-yl)benzonitrile;
3-(1-{[3-(5-{[1-(4-{4-[2-(2,6-dioxopiperidine-3-yl)-1-oxo-1,2-dihydroptalazine-6-yl]piperazine-1-yl}butanoyl)piperidine-4-il]methoxy}pyrimidine-2-yl)phenyl]methyl}-6-oxo-1,6-dihydropyridazine-3-yl)benzonitrile;
3-(1-{[3-(5-{[1-(5-{4-[2-(2,6-dioxopiperidin-3-yl)-1-oxo-1,2-dihydropthalazine-6-yl]piperazine-1-yl}pentanoyl)piperidine-4-yl]methoxy}pyrimidine-2-yl)phenyl]methyl}-6-oxo-1,6-dihydropyridazine-3-yl)benzonitrile;
3-(1-(3-(5-((1-(2-(4-((4-(4-(2-(2,6-dioxopiperidine-3-yl)-7-fluoro-4-methyl-1-oxo-1,2-dihydrophthalazine-6-yl)piperazine-1-yl)methyl)piperidine-1-yl)-2-oxoethyl)piperidine-4-il)methoxy)pyrimidine-2-yl)benzonitrile;
2-(4-(((2-(3-((3-(3-(3-cyanophenyl)-6-oxopridazine-1(6H)-yl)methyl)phenyl)pyrimidine-5-il)oxy)methyl)piperidine-1-yl)N-(5-(2-(2,6-dioxopiperidin-3-yl)-1-oxo-1,2-dihydropthalazine-6-yl)pent-4-phosphorus-1-yl)acetamide;
2-(4-(((2-(3-(3-(3-(3-(3-(3-cyanophenyl)-6-oxopyridazine-1(6H)-yl)methyl)phenyl)pyrimidine-5-yl)oxy)methyl)piperidine-1-yl)-N-(5-(2-(2,6-dioxopiperidin-3-yl)1-oxo-1,2-dihydropthalazine-6-yl)pentyl)acetamide;
2-(4-(((2-(3-((3-(3-(3-cyanophenyl)-6-oxopyridazine-1(6H)-yl)methyl)phenyl)pyrimidine-5-yl)oxy)methyl)piperidine-1-yl)N-(4-(4-(2-(2,6-dioxopiperidin-3-yl)4-methyl-1-oxo-1,2-dihydroptalazine-6-yl)piperazine-1-yl)phenyl)acetamide;
3-(1-(3-(5-((1-(2-(4-((1-(2-(1-(2-(2,6-dioxopiperidin-3-yl)-4-methyl-1-oxo-1,2-dihydropthalazine-6-yl)piperidine-4-yl)methyl)piperazine-1-il)acetyl)piperidine-4-il)methoxy)pyrimidine-2-yl)benzyl)6-oxo-1,6-dihydropyridazine-3-yl)benzonitrile;
3-(1-(3-(5-((1-(1-(2-(4-(3-(2,6-dioxopiperidine-3-yl)-1-methyl-4-oxo-3,4-dihydropthalazine-6-yl)piperazine-1-il)acetyl)piperidine-4-yl)methoxy)pyrimidine-2-yl)benzyl)6-oxo-1,6-dihydropyridazine-3-yl)benzonitrile;
3-(1-(3-(5-((1-(3-(3-(3-(3-(3-(2,6-dioxopiperidine-3-yl)-1-methyl-4-oxo-3,4-dihydropthalazine-6-yl)piperazine-1-yl)propanoyl)piperidine-4-il)methoxy)pyrimidine-2-yl)benzyl)6-oxo-1,6-dihydropyridazine-3-yl)benzonitrile;
3-(1-(3-(5-((1-(1-(4-(4-(3-(2,6-dioxopiperidine-3-yl)-1-methyl-4-oxo-3,4-dihydropthalazine-6-yl)piperazine-1-yl)butanoyl)piperidine-4-il)methoxy)pyrimidine-2-yl)benzyl)6-oxo-1,6-dihydropyridazine-3-yl)benzonitrile;
3-(1-(3-(5-((1-(5-(5-(5-(3-(3-(2,6-dioxopiperidine-3-yl)-1-methyl-4-oxo-3,4-dihydropthalazine-6-yl)piperazine-1-yl)pentanoyl)piperidine-4-yl)methoxy)pyrimidine-2-yl)benzyl)-6-oxo-1,6-dihydropyridazine-3-yl)benzonitrile;
3-(1-(3-(5-((1-(2-(4-(4-(2-(2-(2-(2,6-dioxopiperidine-3-yl)-4-methyl-1-oxo-1,2-dihydroptalazine-6-yl)piperazine-1-carbonyl)piperidine-1-il)acetyl)piperidine-4-il)methoxy)pyrimidine-2-yl)benzyl)6-oxo-1,6-dihydropyridazine-3-yl)benzonitrile;
3-(1-(3-(5-((1-(2-(4-((1-(1-(2-(2-(2,6-dioxopiperidine-3-yl)-4-methyl-1-oxo-1,2-dihydropthalazine-6-yl)piperidine-4-il)amino)piperidine-1-il)acetyl)piperidine-4-il)methoxy)pyrimidine-2-yl)benzyl)6-oxo-1,6-dihydropyridazine-3-yl)benzonitrile;
3-(1-(3-(5-((1-(2-(4-(2-(2,6-dioxopiperidine-3-yl)-1-oxo-1,2-dihydroptalazine-6-yl)piperazine-1-yl)2-oxoethyl)piperidine-4-il)methoxy)pyrimidine-2-yl)benzyl)-6-oxo-1,6-dihydropyridazine-3-yl)benzonitrile;
3-(1-(3-(5-((1-(1-(3-(3-(4-(2-(2,6-dioxopiperidine-3-yl)-1-oxo-1,2-dihydropthalazine-6-yl)piperazine-1-yl)-3-oxopropyl)piperidine-4-yl)methoxy)pyrimidine-2-yl)benzyl)-6-oxo-1,6-dihydropyridazine-3-yl)benzonitrile;
3-(1-{[3-(5-{[1-(3-{4-[2-(2,6-dioxopiperidine-3-yl)-4-methyl-1-oxo-1,2-dihydropthalazine-6-yl]piperazine-1-yl}-3-oxopropyl)piperidine-4-yl]methoxy}pyrimidine-2-yl)phenyl]methyl}-6-oxo-1,6-dihydropyridazine-3-yl)benzonitrile;
3-(1-(3-(5-((1-(3-(3-(4-(3-(2,6-dioxopiperidine-3-yl)-1-methyl-4-oxo-3,4-dihydropthalazine-6-yl)piperazine-1-yl)-3-oxopropyl)piperidine-4-yl)methoxy)pyrimidine-2-yl)benzyl)-6-oxo-1,6-dihydropyridazine-3-yl)benzonitrile;
3-(1-(3-(5-((1-(4-(4-(2-(2,6-dioxopiperidine-3-yl)-1-oxo-1,2-dihydropthalazine-6-yl)piperazine-1-yl)4-oxobutyl)piperidine-4-yl)methoxy)pyrimidine-2-yl)benzyl)-6-oxo-1,6-dihydropyridazine-3-yl)benzonitrile;
3-(1-(3-(5-((1-(4-(4-(2-(2,6-dioxopiperidine-3-yl)-4-methyl-1-oxo-1,2-dihydropthalazine-6-yl)piperazine-1-yl)-4-oxobutyl)piperidine-4-yl)methoxy)pyrimidine-2-yl)benzyl)-6-oxo-1,6-dihydropyridazine-3-yl)benzonitrile;
3-(1-(3-(5-((1-(4-(4-(3-(2,6-dioxopiperidine-3-yl)-1-methyl-4-oxo-3,4-dihydrophthalazine-6-yl)piperazine-1-yl)-4-oxobutyl)piperidine-4-yl)methoxy)pyrimidine-2-yl)benzyl)-6-oxo-1,6-dihydropyridazine-3-yl)benzonitrile;
3-(1-(3-(5-((1-(2-(4-((2R)-4-(2-(2-(2,6-dioxopiperidine-3-yl)-4-methyl-1-oxo-1,2-dihydrophthalazine-6-yl)morpholine-2-yl)methyl)piperazine-1-il)acetyl)piperidine-4-yl)methoxy)pyrimidine-2-yl)benzonitrile 6-oxo-1,6-dihydropyridazine-3-yl)benzonitrile;
3-(1-(3-(5-((1-(2-(4-((4-(4-(3-(2,6-dioxopiperidine-3-yl)-7-fluoro-1-methyl-4-oxo-3,4-dihydropetalazine-6-yl)piperazine-1-il)methylpiperidine-1-il)-2-oxoethyl)piperidine-4-il)methoxy)pyrimidine-2-yl)benzonitrile;
3-(1-(3-(5-((1-(2-(3-(3-(4-(2-(2-(2,6-dioxopiperidine-3-yl)-1-oxo-1,2-dihydrophthalazine-6-yl)piperazine-1-il)azetidine-1-yl)2-oxoethyl)piperidine-4-yl)methoxy)pyrimidine-2-yl)benzyl)6-oxo-1,6-dihydropyridazine-3-yl)benzonitrile;
3-(1-(3-(5-((1-(2-(4-(1-(1-(3-(2-(1-(3-(2,6-dioxopiperidin-3-yl)-1-methyl-4-oxo-3,4-dihydropthalazine-6-yl)piperidine-4-yl)piperazine-1-yl)-2-oxoethyl)piperidine-4-yl)methoxy)pyrimidine-2-yl)benzyl)6-oxo-1,6-dihydropyridazine-3-yl)benzonitrile;
3-(1-(3-(5-((1-(2-(4-((1-(1-(2-(4-(1-(3-(2,6-dioxopiperidine-3-yl)-1-methyl-4-oxo-3,4-dihydropthalazine-6-yl)piperidine-4-yl)methyl)piperazine-1-yl)-2-oxoethyl)piperidine-4-yl)methoxy)pyrimidine-2-yl)benzonitrile;
3-(1-(3-(5-((1-(2-(3-((4-(2-(3-(4-(2-(2,6-dioxopiperidine-3-yl)-4-methyl-1-oxo-1,2-dihydropthalazine-6-yl)piperazine-1-yl)methylazetidin-1-yl)-2-oxoethyl)piperidine-4-il)methoxy)pyrimidine-2-yl)benzyl)6-oxo-1,6-dihydropyridazine-3-yl)benzonitrile;
3-(1-(3-(5-((1-(2-(4-((1-(2-(1-(2-(2,6-dioxopiperidine-3-yl)-4-methyl-1-oxo-1,2-dihydropthalazine-6-yl)piperidine-4-yl)methyl)piperazine-1-yl)2-oxoethyl)piperidine-4-il)methoxy)pyrimidine-2-yl)benzonitrile;
3-(1-(3-(5-((1-(1-(2-(4-(4-(2-(2-(2-(2,6-dioxopiperidine-3-yl)-4-methyl-1-oxo-1,2-dihydropthalazine-6-yl)piperazine-1-yl)piperidine-1-yl)piperidine-1-yl)2-oxoethyl)piperidine-4-yl)methoxy)pyrimidine-2-yl)benzyl)6-oxo-1,6-dihydropyridazine-3-yl)benzonitrile ;
N-(3-(2-(4-(((2-(3-((3-(3-(3-cyanophenyl)-6-oxopridazine-1(6H)-yl)methyl)phenyl)pyrimidine-5-yl)oxy)methyl)piperidine-1-yl)acetamido)propyl)-1-(3-(2,6-dioxopiperidine-3-yl)-1-methyl-4-oxo-3,4-dihydroptalazine-6-yl)piperidine-4-carboxamide;
3-(1-(3-(5-((1-(2-(4-((1-(1-(3-(2,6-dioxopiperidin-3-yl)-1-methyl-4-oxo-3,4-dihydropthalazine-6-yl)piperidine-4-il)methyl)piperazine-1-il)acetyl)piperidine-4-il)methoxy)pyrimidine-2-yl)benzyl)6-oxo-1,6-dihydropyridazine-3-yl)benzonitrile;
3-(1-(3-(5-((1-((4-(4-(2-(2,6-dioxopiperidine-3-yl)-4-methyl-1-oxo-1,2-dihydropthalazine-6-yl)piperazine-1-yl)phenyl)glycyl)piperidine-4-il)methoxy)pyrimidine-2-yl)benzyl)6-oxo-1,6-dihydropyridazine-3-yl)benzonitrile;
3-(1-(3-(5-((1-(5-(5-(5-(4-(2-(2,6-dioxopiperidine-3-yl)-4-methyl-1-oxo-1,2-dihydropthalazine-6-yl)piperazine-1-yl)-5-oxopentyl)piperidine-4-yl)methoxy)pyrimidine-2-yl)benzyl)6-oxo-1,6-dihydropyridazine-3-yl)benzonitrile;
3-(1-(3-(5-((1-(5-(5-(5-(3-(2,6-dioxopiperidine-3-yl)-1-methyl-4-oxo-3,4-dihydropthalazine-6-yl)piperazine-1-yl)-5-oxopentil)piperidine-4-yl)methoxy)pyrimidine-2-yl)benzyl)-6-oxo-1,6-dihydropyridazine-3-yl)benzonitrile;
3-(1-(3-(5-((1-(5-(5-(4-(2-(2,6-dioxopiperidin-3-yl)-1-oxo-1,2-dihydropthalazine-6-yl)piperazine-1-yl)5-oxopentyl)piperidine-4-yl)methoxy)pyrimidine-2-yl)benzyl)-6-oxo-1,6-dihydropyridazine-3-yl)benzonitrile;
3-(1-(3-(5-((1-(2-(3-((4-(2-(2,6-dioxopiperidine-3-yl)-4-methyl-1-oxo-1,2-dihydropthalazine-6-yl)piperazine-1-il)methylazetidin-1-il)acetyl)piperidine-4-il)methoxy)pyrimidine-2-yl)benzyl)6-oxo-1,6-dihydropyridazine-3-yl)benzonitrile;
3-(1-(3-(5-((1-(2-((6R)-6-((4-(2-(2,6-dioxopiperidin-3-yl)-4-methyl-1-oxo-1,2-dihydropthalazine-6-yl)piperazine-1-yl)methyl)1,3-oxajinan-3-yl)acetyl)piperidine-4-yl)methoxy)pyrimidine-2-yl)benzonitrile 6-oxo-1,6-dihydropyridazine-3-yl)benzonitrile;
3-(1-(3-(5-((1-(2-(4-(4-(2-(2-(2-(2,6-dioxopiperidine-3-yl)-4-methyl-1-oxo-1,2-dihydrophthalazine-6-yl)piperazine-1-il)piperidine-1-il)piperidine-1-il)acetyl)piperidine-4-il)methoxy)pyrimidine-2-yl)benzyl)6-oxo-1,6-dihydropyridazine-3-yl)benzonitrile;
3-(1-(3-(5-((1-(2-(4-(4-(2-(2-(2-(2-(2,6-dioxopiperidine-3-yl)-4-methyl-1-oxo-1,2-dihydropthalazine-6-yl)piperazine-1-il)methyl)piperidine-1-il)acetyl)piperidine-4-il)methoxy)pyrimidine-2-yl)benzyl)6-oxo-1,6-dihydropyridazine-3-yl)benzonitrile;
3-(1-(3-(5-((1-(2-(1-(1-(4-(2-(2-(2-(2,6-dioxopiperidine-3-yl)-4-methyl-1-oxo-1,2-dihydropthalazine-6-yl)piperazine-1-carbonyl)azetidine-3-il)acetyl)piperidine-4-yl)methoxy)pyrimidine-2-yl)benzyl)6-oxo-1,6-dihydropyridazine-3-yl)benzonitrile;
3-(1-(3-(5-((1-(2-((2R)-2-((4-(2-(2,6-dioxopiperidine-3-yl)-4-methyl-1-oxo-1,2-dihydropetalazine-6-yl)piperazine-1-yl)methyl)morpholino)2-oxoethyl)piperidine-4-yl)methoxy)pyrimidine-2-yl)benzonitrile;
3-(1-(3-(5-((1-((4-(4-(3-(2,6-dioxopiperidine-3-yl)-1-methyl-4-oxo-3,4-dihydropthalazine-6-yl)piperazine-1-yl)phenyl)glycy)piperidine-4-il)methoxy)pyrimidine-2-yl)benzyl)6-oxo-1,6-dihydropyridazine-3-yl)benzonitrile;
3-(1-(3-(5-((1-(2-(4-(1-(3-(2-(1-(3-(2,6-dioxopiperidine-3-yl)-1-methyl-4-oxo-3,4-dihydropthalazine-6-yl)piperidine-4-il)piperazine-1-il)acetyl)piperidine-4-il)methoxy)pyrimidine-2-yl)benzyl)6-oxo-1,6-dihydropyridazine-3-yl)benzonitrile;
3-(1-(3-(5-((1-(2-(4-((2R)-4-(3-(2,6-dioxopiperidine-3-yl)-1-methyl-4-oxo-3,4-dihydrophthalazine-6-yl)morpholine-2-yl)methyl)piperazine-1-il)acetyl)piperidine-4-il)methoxy)pyrimidine-2-yl)benzonitrile;
3-(1-(3-(5-((1-(2-(4-((4-(2-(2,6-dioxopiperidine-3-yl)-7-fluoro-4-methyl-1-oxo-1,2-dihydropthalazine-6-yl)piperazine-1-il)methyl)piperidine-1-il)acetyl)piperidine-4-il)methoxy)pyrimidine-2-yl)benzonitrile 6-oxo-1,6-dihydropyridazine-3-yl)benzonitrile;
3-(1-{[3-(5-{[1-(2-{4-[2-(2,6-dioxopiperidine-3-yl)-1-oxo-1,2-dihydroptalazine-6-yl]piperazine-1-il}ethyl)piperidine-4-yl]methoxy}pyrimidine-2-yl)phenyl]methyl}-6-oxo-1,6-dihydropyridazine-3-yl)benzonitrile;
3-(1-{[3-(5-{[1-(2-{4-[2-(2,6-dioxopiperidine-3-yl)-4-methyl-1-oxo-1,2-dihydropthalazine-6-yl]piperazine-1-il}ethyl)piperidine-4-il]methoxy}pyrimidin-2-yl)phenyl]methyl}-6-oxo-1,6-dihydropyridazine-3-yl)benzonitrile;
3-(1-{[3-(5-{[1-(2-{4-[3-(2,6-dioxopiperidin-3-yl)-1-methyl-4-oxo-3,4-dihydropthalazine-6-yl]piperazine-1-il}ethyl)piperidine-4-il]methoxy}pyrimidine-2-yl)phenyl]methyl}-6-oxo-1,6-dihydropyridazine-3-yl)benzonitrile;
3-(1-(4-(5-((1-(5-(5-(2-(2,6-dioxopiperidine-3-yl)-1-oxo-1,2-dihydroptalazine-5-yl)pentyl)piperidine-4-yl)methoxy)pyrimidin-2-yl)benzyl)6-oxo-1,6-dihydropyridazine-3-yl)benzonitrile;
3-(1-(4-(5-((1-(5-(5-(2,6-dioxopiperidine-3-yl)-1-oxo-1,2-dihydropetalazine-5-yl)pent-4-in-yl)piperidine-4-yl)methoxy)pyrimidine-2-yl)benzonitrile -6-oxo-1,6-dihydropyridazine-3-yl)benzonitrile;
3-(1-(3-(5-((1-(5-(5-(2-(2,6-dioxopiperidine-3-yl)-1-oxo-1,2-dihydrophthalazine-6-yl)pentyl)piperidine-4-yl)methoxy)pyrimidine-2-yl)benzyl)6-oxo-1,6-dihydropyridazine-3-yl)benzonitrile;
3-(1-(3-(5-((1-(5-(5-(2-(2,6-dioxopiperidine-3-yl)-1-oxo-1,2-dihydrophthalazine-6-yl)pent-4-phospho-1-yl)piperidine-4-yl)methoxy)pyrimidine-2-yl)benzyl)-6-oxo-1,6-dihydropyridazine-3-yl)benzonitrile;
3-(1-(3-(5-((1-(2-(2-(2-(2-(2-(3-(2-(2,6-dioxopiperidine-3-yl)-1-oxo-1,2-dihydropthalazine-5-yl)prop-2-phosphorus-1-yl)oxy)ethoxy)ethoxy)ethyl)piperidine-4-il)methoxy)pyrimidine-2-yl)benzyl)6-oxo-1,6-dihydropyridazine-3-yl)benzonitrile;
3-(1-(3-(5-((1-(2-((2R)-2-((4-(2-(2-(2-(dioxopiperidine-3-yl)-4-methyl-1-oxo-1,2-dihydroptalazine-6-yl)piperazine-1-yl)methyl)morphopolino)ethyl)piperidine-4-il)methoxy)pyrimidine-2-yl)benzyl)6-oxo-1,6-dihydropyridazine-3-yl)benzonitrile;
3-(1-(3-(5-((1-(1-(2-(4-(4-(2-(2-(2,6-dioxopiperidine-3-yl)-4-methyl-1-oxo-1,2-dihydropthalazine-6-yl)piperazine-1-il)piperidine-1-il)ethyl)piperidine-4-il)methoxy)pyrimidine-2-yl)benzyl)6-oxo-1,6-dihydropyridazine-3-yl)benzonitrile;
3-(1-(3-(5-((1-(2-(4-(4-(4-(2-(2,6-dioxopiperidine-3-yl)-7-fluoro-4-methyl-1-oxo-1,2-dihydrophthalazine-6-yl)piperazine-1-il)methyl)piperidine-1-il)ethyl)piperidine-4-il)methoxy)pyrimidine-2-yl)benzonitrile -6-oxo-1,6-dihydropyridazine-3-yl)benzonitrile;
3-(1-(3-(5-((1-(2-(4-((4-(4-(2-(2,6-dioxopiperidine-3-yl)-4,7-dimethyl-1-oxo-1,2-dihydrophthalazine-6-yl)piperazine-1-yl)methyl)piperidine-1-il)ethyl)piperidine-4-il)methoxy)pyrimidine-2-yl)benzonitrile 6-oxo-1,6-dihydropyridazine-3-yl)benzonitrile
3-(1-(3-(5-((1-(2-(4-((4-(3-(2,6-dioxopiperidine-3-yl)-1-methyl-4-oxo-3,4-dihydropthalazine-6-yl)piperazine-1-il)methyl)piperidine-1-il)ethyl)piperidine-4-il)methoxy)pyrimidine-2-yl)benzonitrile -6-oxo-1,6-dihydropyridazine-3-yl)benzonitrile;
3-(1-(3-(5-((1-(1-(2-(3-(4-(2-(2-(2,6-dioxopiperidine-3-yl)-4-methyl-1-oxo-1,2-dihydropthalazine-6-yl)piperazine-1-il)azetidine-1-il)ethyl)piperidine-4-il)methoxy)pyrimidine-2-yl)benzyl)6-oxo-1,6-dihydropyridazine-3-yl)benzonitrile;
3-(1-(3-(5-((1-(2-(4-((4-(3-(2,6-dioxopiperidine-3-yl)-7-fluoro-1-methyl-4-oxo-3,4-dihydroptalazine-6-yl)piperazine-1-il)methyl)piperidine-1-il)ethyl)piperidine-4-il)methoxy)pyrimidine-2-yl)benzonitrile;
3-(1-(3-(5-((1-(4-((2,6-dioxopiperidine-3-yl)amino)-2-fluorophenyl)piperidine-4-yl)methoxy)pyrimidine-2-yl)benzyl)6-oxo-1,6-dihydropyridazine-3-yl)benzonitrile;
3-{1-[(3-{5-[(1-{[1-(2,6-dioxopiperidine-3-yl)-1H-1,2,3-triazole-4-yl]methyl}piperidine-4-yl)methoxy]pyrimidine-2-yl}phenyl)methyl]-6-oxo-1,6-dihydropyridazine-3-yl}benzonitrile;
3-(1-(3-(5-(4-((1-(4-((2,6-dioxopiperidine-3-yl)amino)2-fluorophenyl)piperidine-4-yl)methyl)piperazine-1-il)pyrimidine-2-yl)benzyl)-6-oxo-1,6-dihydropyridazine-3-yl)benzonitrile;
3-(1-(3-(5-((1-((1-(1-((1-((2,6-dioxopiperidin-3-yl)amino)2-fluorophenyl)azetidine-3-yl)methyl)piperidine-4-yl)methoxy)pyrimidine-2-yl)benzyl)6-oxo-1,6-dihydropyridazine-3-yl)benzonitrile;
3-(1-(3-(5-((1-(2-(4-((4-((2,6-dioxopiperidine-3-yl)amino)2-fluorophenyl)piperazine-1-yl)ethyl)piperidine-4-yl)methoxy)pyrimidine-2-yl)benzyl)6-oxo-1,6-dihydropyridazine-3-yl)benzonitrile;
3-(1-(3-(5-(3-((4-((2,6-dioxopiperidin-3-yl)amino)2-(4-((2,6-dioxopiperidin-3-yl)amino)2-fluorophenyl)piperazine-1-yl)methyl)phenyl)pyrimidine-2-yl)benzyl)-6-oxo-1,6-dihydropyridazine-3-yl)benzonitrile;
3-(1-(3-(5-((1-(2-(4-((2,6-dioxopiperidine-3-yl)amino)-3-fluorophenyl)piperazine-1-yl)-2-oxoethyl)piperidine-4-yl)methoxy)pyrimidine-2-yl)benzonitrile;
3-(1-{[3-(5-{[1-(2-{4-[(4-{4-[(2,6-dioxopiperidine-3-yl)amino]-2-fluorophenyl}piperazine-1-yl)methyl]piperidine-1-yl}-2-oxoethyl)piperidine-4-il]methoxy}pyrimidine-2-yl)phenyl]methyl}-6-oxo-1,6-dihydropyridazine-3-yl)benzonitrile;
3-(1-(3-(5-(3-((4-(5-(2,6-dioxopiperidine-3-yl)pyridine-2-yl)piperazine-1-yl)methyl)phenyl)pyrimidine-2-yl)benzyl)6-oxo-1,6-dihydropyridazine-3-yl)benzonitrile;
3-(1-(3-(5-(3-((4-((4-((4-(4-((4-((2,6-dioxopiperidine-3-yl)amino)2-fluorophenyl)piperazine-1-il)methyl)piperidine-1-il)methyl)phenyl)pyrimidine-2-yl)benzyl)6-oxo-1,6-dihydropyridazine-3-yl)benzonitrile;
3-(1-{[3-(5-{[1-(2-{4-[(1-{4-[(2,6-dioxopiperidine-3-yl)amino]-2-fluorophenyl}piperidine-4-yl)methyl]piperazine-1-yl}-2-oxoethyl)piperidine-4-il]methoxy}pyrimidine-2-yl)phenyl]methyl}-6-oxo-1,6-dihydropyridazine-3-yl)benzonitrile;
3-(1-(3-(5-((1-(1-(2-(4-((4-((2,6-dioxopiperidin-3-yl)amino)2-fluorophenyl)piperazine-1-yl)acetyl)piperidine-4-yl)methoxy)pyrimidine-2-yl)benzyl)6-oxo-1,6-dihydropyridazine-3-yl)benzonitrile;
3-(1-(3-(5-(4-((1-(3-(3-(2,6-dioxopiperidine-3-yl)-1-methyl-4-oxo-3,4-dihydropthalazine-6-yl)piperidine-4-yl)methyl)piperazine-1-il)pyrimidine-2-yl)benzyl)6-oxo-1,6-dihydropyridazine-3-yl)benzonitrile;
3-(1-(3-(5-(4-((1-(2-(2,6-dioxopiperidine-3-yl)-1-oxo-1,2-dihydropthalazine-6-yl)piperidine-4-yl)methyl)piperazine-1-il)pyrimidine-2-yl)benzyl)6-oxo-1,6-dihydropyridazine-3-yl)benzonitrile;
3-(1-(3-(5-(4-((1-(3-(3-(2,6-dioxopiperidine-3-yl)-4-oxo-3,4-dihydrophthalazine-6-yl)piperidine-4-yl)methyl)piperazine-1-il)pyrimidine-2-yl)benzonitrile;
3-(1-(3-(5-(4-(3-(3-(4-(4-(2-(2,6-dioxopiperidine-3-yl)-1-oxo-1,2-dihydrophthalazine-6-yl)piperazine-1-yl)-2-hydroxypropyl)piperazine-1-day)pyrimidine-2-yl)benzyl)-6-oxo-1,6-dihydropyridazine-3-yl)benzonitrile; and
3-(1-(3-(5-(4-(3-(3-(4-(4-(2-(2,6-dioxopiperidin-3-yl)-4-methyl-1-oxo-1,2-dihydropthalazine-6-yl)piperazine-1-yl)-2-hydroxypropyl)piperazine-1-il)pyrimidine-2-yl)benzo)6-oxo-1,6-dihydropyridazine-3-yl)benzonitrile.

6. An anticancer composition comprising a compound of any of claims 1 to 5.
